# EUROPEAN PATENT APPLICATION

(11) **EP 4 667 578 A2**
(43) Date of publication of application: **24.12.2025**
(21) Application number: 25210112.6
(22) Date of filing: 02.04.2020
(51) Int. Cl.: C12N 15/864

(54) **GENE THERAPY FOR EYE PATHOLOGIES**

(30) Priority: 03.04.2019 US 201962828949 P; 03.06.2019 US 201962856533 P; 10.12.2019 US 201962946158 P
(62) Divisional of application: 20723243.0
(71) Applicant: REGENXBIO Inc., Rockville, MD 20850 (US)
(72) Inventor: DANOS, Olivier, New York, NY 10128 (US); VAN EVEREN, Sherri, Menlo Park, CA 94025 (US); YOO, Jesse I., Atlanta, GA 30306 (US); PATEL, Samir Maganbhai, Columbus, NJ 08022 (US); GHANEKAR, Avanti Arvind, St. Louis, MO 63018 (US); O'BERRY, Anthony Ray, Clarksburg, MD 20871 (US); IRWIN-PACK, Kim Rees, Milcreek, UT 84109 (US); CURTISS, Darin Thomas, Potomac, MD 20854 (US)
(74) Representative: Jones Day

(57) **Abstract**

Compositions and methods are described for the delivery of therapeutic products to the retina/vitreal humour in the eyes of human subjects to treat pathologies of the eye, involving, for example, recombinant viral vectors such as recombinant adeno-associated virus (rAAV) vectors.

## Description

### 1. CROSS-REFERENCE TO RELATED APPLICATIONS

This application claims the benefit of U.S. Provisional Patent Application Nos. 62/828,949, filed April 3, 2019, 62/856,533, filed June 3, 2019, and 62/946,158, filed December 10, 2019, which are incorporated by reference herein in their entireties.

### REFERENCE TO SEQUENCE LISTING SUBMITTED ELECTRONICALLY

This application incorporates by reference a Sequence Listing submitted with this application as text file entitled "12656-126-228_Sequence_Listing.txt" created on March 24, 2020 and having a size of 2,025,574 bytes.

### 2. INTRODUCTION

Compositions and methods are described for the delivery of therapeutic products (such as therapeutic proteins (for example, antibodies), therapeutic RNAs (for example, shRNAs, siRNAs, and miRNAs), and therapeutic aptamers) to the retina/vitreal humour in the eyes of human subjects to treat pathologies of the eye, involving, for example, recombinant viral vectors such as recombinant adeno-associated virus (rAAV) vectors.

### 3. BACKGROUND OF THE INVENTION

The human eye is a highly intricate and highly developed sensory organ, which is prone to a host of diseases and disorders. About 285 million people in the world are visually impaired, of whom 39 million are blind and 246 million have moderate to severe visual impairment (World Health Organization, 2012, "Global Data On Visual Impairments 2010," Geneva : World Health Organization). Some of the leading causes of blindness are cataract (47%), glaucoma (12%), age-related macular degeneration (AMD) (9%), and diabetic retinopathy (5%) (World Health Organization, 2007, "Global Initiative For The Elimination Of Avoidable Blindness: Action Plan 2006-2011," Geneva : World Health Organization).

An extensive number of ocular diseases and diseases with pathological manifestations in the eye can be traced to genetic alterations or protein dysregulations (Stone et al., 2017, Ophthalmology 124(9): 1314-1331). Recent advances in genomics and proteomics have made a huge impact in our understanding of disease mechanisms and/or genetic basis underlying such ocular diseases or manifestations. Gene therapy has been employed in treating certain eye diseases (*see, e.g.* International Patent Application No. PCT/US2017/027650 (International Publication No. WO 2017/181021 A1)).

There is a significant unmet medical need for therapies that specifically address the underlying genetic anomalies to treat ocular pathologies.

### 4. SUMMARY OF THE INVENTION

Compositions and methods are described for the delivery of therapeutic products (such as therapeutic proteins (for example, antibodies), therapeutic RNAs (for example, shRNAs, siRNAs, and miRNAs), and therapeutic aptamers) to the retina/vitreal humour in the eyes of human subjects to treat pathologies of the eye, involving, for example, recombinant viral vectors such as recombinant adeno-associated virus (rAAV) vectors. The therapeutic products can be, for example, therapeutic proteins (for example, antibodies), therapeutic RNAs (for example, shRNAs, siRNAs, and miRNAs), or therapeutic aptamers. In a specific embodiment, the therapeutic products is a human protein or an antibody against a human protein. Antibodies include, but are not limited to, monoclonal antibodies, polyclonal antibodies, recombinantly produced antibodies, human antibodies, humanized antibodies, chimeric antibodies, synthetic antibodies, tetrameric antibodies comprising two heavy chain and two light chain molecules, antibody light chain monomers, antibody heavy chain monomers, antibody light chain dimers, antibody heavy chain dimers, antibody light chain-heavy chain pairs, intrabodies, heteroconjugate antibodies, monovalent antibodies, antigen-binding fragments of full-length antibodies, and fusion proteins of the above. Such antigen-binding fragments include, but are not limited to, single-domain antibodies (variable domain of heavy chain antibodies (VHHs) or nanobodies), Fabs, F(ab')₂s. and scFvs (single-chain variable fragments). In certain embodiment, the therapeutic product (for example, a therapeutic protein) is post-translationally modified. In a specific embodiment, the post-translational modification is specific to the cell type, to which the therapeutic product (for example, a therapeutic protein) is delivered using a specific route as described herein. Delivery may be accomplished via gene therapy - *e.g.,* by administering a recombinant viral vector or a recombinant DNA expression construct (collectively, a "recombinant vector") encoding an therapeutic product to the suprachoroidal space, subretinal space (with vitrectomy, or without vitrectomy (*e.g.,* with a catheter through the suprachoroidal space, or via peripheral injection), intraretinal space, and/or outer surface of the sclera (*i.e*., juxtascleral administration) in the eye(s) of a human patient, to create a permanent depot in the eye that continuously supplies the therapeutic product *(e.g.,* a post-translationally modified therapeutic product).

In one aspect, provided herein is a method of subretinal administration without vitrectomy for treating a pathology of the eye, comprising administering to the subretinal space in the eye of a human subject in need of treatment a recombinant viral vector comprising a nucleotide sequence encoding a therapeutic product such that the therapeutic product is expressed and results in treatment of the pathology of the eye, wherein the method does not comprise performing a vitrectomy on the eye of said human patient. In certain embodiments, the administering step comprises administering to the subretinal space in the eye of said human subject the recombinant viral vector therapeutic product via the suprachoroidal space in the eye of said human subject. In certain embodiments, the administering step is by the use of a subretinal drug delivery device comprising a catheter that can be inserted and tunneled through the suprachoroidal space toward the posterior pole, where a small needle injects into the subretinal space. In certain embodiments, the administering step comprises inserting and tunneling the catheter of the subretinal drug delivery device through the suprachoroidal space.

In another aspect, provided herein is a method for treating a pathology of the eye, comprising administering to the subretinal space in the eye of a human subject in need of treatment a recombinant viral vector comprising a nucleotide sequence encoding a therapeutic product such that the therapeutic product is expressed and results in treatment of the pathology of the eye, wherein the method does not comprise performing a vitrectomy on the eye of said human patient. In certain embodiments, the administering step comprises administering to the subretinal space in the eye of said human subject the recombinant viral vector therapeutic product via the suprachoroidal space in the eye of said human subject. In certain embodiments, the administering step is by the use of a subretinal drug delivery device comprising a catheter that can be inserted and tunneled through the suprachoroidal space toward the posterior pole, where a small needle injects into the subretinal space. In certain embodiments, the administering step comprises inserting and tunneling the catheter of the subretinal drug delivery device through the suprachoroidal space.

In one aspect, provided herein is a method of subretinal administration with vitrectomy for treating a pathology of the eye, comprising administering to the subretinal space in the eye of a human subject in need of treatment a recombinant viral vector comprising a nucleotide sequence encoding a therapeutic product such that the therapeutic product is expressed and results in treatment of the pathology of the eye, wherein the method comprises performing a vitrectomy on the eye of said human patient. In certain embodiments, the vitrectomy is a partial vitrectomy.

In another aspect, provided herein is a method for treating a pathology of the eye, comprising administering to the subretinal space in the eye of a human subject in need of treatment a recombinant viral vector comprising a nucleotide sequence encoding a therapeutic product such that the therapeutic product is expressed and results in treatment of the pathology of the eye, wherein the method comprises performing a vitrectomy on the eye of said human patient. In certain embodiments, the vitrectomy is a partial vitrectomy.

In one aspect, provided herein is a method of suprachoroidal administration for treating a pathology of the eye, comprising administering to the suprachoroidal space in the eye of a human subject in need of treatment a recombinant viral vector comprising a nucleotide sequence encoding a therapeutic product such that the therapeutic product is expressed and results in treatment of the pathology of the eye. In certain embodiments, the administering step is by injecting the recombinant viral vector into the suprachoroidal space using a suprachoroidal drug delivery device. In certain embodiments, the suprachoroidal drug delivery device is a microinjector.

In another aspect, provided herein is a method for treating a pathology of the eye, comprising administering to the suprachoroidal space in the eye of a human subject in need of treatment a recombinant viral vector comprising a nucleotide sequence encoding a therapeutic product such that the therapeutic product is expressed and results in treatment of the pathology of the eye. In certain embodiments, the administering step is by injecting the recombinant viral vector into the suprachoroidal space using a suprachoroidal drug delivery device. In certain embodiments, the suprachoroidal drug delivery device is a microinjector.

In certain embodiments, delivery to the subretinal or suprachoroidal space can be performed using the methods and/or devices described and disclosed in International Publication Nos. WO 2016/042162, WO 2017/046358, WO 2017/158365, and WO 2017/158366, each of which is incorporated by reference in its entirety.

In one aspect, provided herein is a method of administration to the outer space of the sclera for treating a pathology of the eye, comprising administering to the outer surface of the sclera in the eye of a human subject in need of treatment a recombinant viral vector comprising a nucleotide sequence encoding a therapeutic product such that the therapeutic product is expressed and results in treatment of the pathology of the eye. In certain embodiments, the administering step is by the use of a juxtascleral drug delivery device that comprises a cannula whose tip can be inserted and kept in direct apposition to the scleral surface. In certain embodiments, the administering step comprises inserting and keeping the tip of the cannula in direct apposition to the scleral surface.

In another aspect, provided herein is a method for treating a pathology of the eye, comprising administering to the outer surface of the sclera in the eye of a human subject in need of treatment a recombinant viral vector comprising a nucleotide sequence encoding a therapeutic product such that the therapeutic product is expressed and results in treatment of the pathology of the eye. In certain embodiments, the administering step is by the use of a juxtascleral drug delivery device that comprises a cannula whose tip can be inserted and kept in direct apposition to the scleral surface. In certain embodiments, the administering step comprises inserting and keeping the tip of the cannula in direct apposition to the scleral surface

In certain embodiments, the therapeutic product is not an anti-human vascular endothelial growth factor (hVEGF) antibody.

In certain embodiments, the pathology of the eye is not associated with neovascular age-related macular degeneration (nAMD) (also known as the "wet," neovascular form of AMD ("WAMD" or "wet AMD")).

In certain embodiments, the therapeutic product is an anti-hVEGF antibody.

In certain embodiments, the pathology of the eye is associated with nAMD.

In certain embodiments, the pathology of the eye is associated with nAMD and the therapeutic product is an anti-hVEGF antibody.

In one aspect, provided herein is a method of subretinal administration accompanied by vitrectomy for treating a pathology of the eye, comprising administering to the subretinal space in the eye of a human subject in need of treatment a recombinant viral vector comprising a nucleotide sequence encoding a therapeutic product such that the therapeutic product is expressed and results in treatment of the pathology of the eye, wherein the method comprises performing a vitrectomy on the eye of said human patient, and wherein the therapeutic product is not anti-human vascular endothelial growth factor (hVEGF) antibody. In certain embodiments, the pathology of the eye is an ocular disease or a disease involving multiple organs including the eye. In certain embodiments, the vitrectomy is a partial vitrectomy.

In another aspect, provided herein is a method for treating a pathology of the eye, comprising administering to the subretinal space in the eye of a human subject in need of treatment a recombinant viral vector comprising a nucleotide sequence encoding a therapeutic product such that the therapeutic product is expressed and results in treatment of the pathology of the eye, wherein the method comprises performing a vitrectomy on the eye of said human patient, and wherein the therapeutic product is not anti-human vascular endothelial growth factor (hVEGF) antibody. In certain embodiments, the pathology of the eye is an ocular disease or a disease involving multiple organs including the eye. In certain embodiments, the vitrectomy is a partial vitrectomy.

In one aspect, provided herein is a method of subretinal administration for treating a pathology of the eye, comprising administering to the subretinal space peripheral to the optic disc, fovea and macula located in the back of the eye of a human subject in need of treatment a recombinant viral vector comprising a nucleotide sequence encoding a therapeutic product such that the therapeutic product is expressed and results in treatment of the pathology of the eye, wherein the method does not comprise performing a vitrectomy on the eye of said human patient. In certain embodiments, the injecting step is by transvitreal injection. In certain embodiments, the method of transvitreal administration results in uniform expression of the therapeutic product throughout the eye (*e.g.* the expression level at the site of injection varies by less than 5%, 10%, 20%, 30%, 40%, or 50% as compared to the expression level at other areas of the eye). In certain embodiments, the transvitreal injection comprises inserting a sharp needle into the sclera via the superior or inferior side of the eye and passing the sharp needle all the way through the vitreous to inject the recombinant viral vector to the subretinal space on the other side. In certain embodiments, a needle is inserted at the 2 or 10 o'clock position. In certain embodiments, the transvitreal injection comprises inserting a trochar into the sclera and inserting a cannula through the trochar and through the vitreous to inject the recombinant viral vector to the subretinal space on the other side. In certain embodiments, the therapeutic product is an anti-hVEGF antibody. In certain embodiments, the anti-hVEGF antibody is an anti-hVEGF antigen-binding fragment. In certain embodiments, the anti-hVEGF antigen-binding fragment is a Fab, F(ab')₂, or single chain variable fragment (scFv). In certain embodiments, the anti-hVEGF antibody comprises a heavy chain comprising the amino acid sequence of SEQ ID NO:2 or SEQ ID NO:4, and a light chain comprising the amino acid sequence of SEQ ID NO: 1, or SEQ ID NO:3. In certain embodiments, wherein the anti-hVEGF antibody comprises light chain CDRs 1-3 of SEQ ID NOs: 14-16 and heavy chain CDRs 1-3 of SEQ ID NOs: 17-19 or SEQ ID NOs:20, 18, and 21. In certain embodiments, wherein the pathology of the eye is associated with nAMD, dry age-related macular degeneration (dry AMD), retinal vein occlusion (RVO), diabetic macular edema (DME), or diabetic retinopathy (DR). In certain embodiments, the pathology of the eye is associated with nAMD.

In another aspect, provided herein is a method for treating a pathology of the eye, comprising administering to the subretinal space peripheral to the optic disc, fovea and macula located in the back of the eye of a human subject in need of treatment a recombinant viral vector comprising a nucleotide sequence encoding a therapeutic product such that the therapeutic product is expressed and results in treatment of the pathology of the eye, wherein the method does not comprise performing a vitrectomy on the eye of said human patient. In certain embodiments, the injecting step is by transvitreal injection. In certain embodiments, the method of transvitreal administration results in uniform expression of the therapeutic product throughout the eye (*e.g.* the expression level at the site of injection varies by less than 5%, 10%, 20%, 30%, 40%, or 50% as compared to the expression level at other areas of the eye). In certain embodiments, the transvitreal injection comprises inserting a sharp needle into the sclera via the superior or inferior side of the eye and passing the sharp needle all the way through the vitreous to inject the recombinant viral vector to the subretinal space on the other side. In certain embodiments, a needle is inserted at the 2 or 10 o'clock position. In certain embodiments, the transvitreal injection comprises inserting a trochar into the sclera and inserting a cannula through the trochar and through the vitreous to inject the recombinant viral vector to the subretinal space on the other side. In certain embodiments, the therapeutic product is an anti-hVEGF antibody. In certain embodiments, the anti-hVEGF antibody is an anti-hVEGF antigen-binding fragment. In certain embodiments, the anti-hVEGF antigen-binding fragment is a Fab, F(ab')₂, or single chain variable fragment (scFv). In certain embodiments, the anti-hVEGF antibody comprises a heavy chain comprising the amino acid sequence of SEQ ID NO:2 or SEQ ID NO:4, and a light chain comprising the amino acid sequence of SEQ ID NO: 1, or SEQ ID NO:3. In certain embodiments, wherein the anti-hVEGF antibody comprises light chain CDRs 1-3 of SEQ ID NOs:14-16 and heavy chain CDRs 1-3 of SEQ ID NOs:17-19 or SEQ ID NOs:20, 18, and 21. In certain embodiments, wherein the pathology of the eye is associated with nAMD, dry age-related macular degeneration (dry AMD), retinal vein occlusion (RVO), diabetic macular edema (DME), or diabetic retinopathy (DR). In certain embodiments, the pathology of the eye is associated with nAMD.

In certain embodiments of the methods described herein, (1) the pathology of the eye is associated with Batten-CLN1 and the therapeutic product is Palmitoyl-Protein Thioesterase 1 (PPT1); (2) the pathology of the eye is associated with Batten-CLN2 and the therapeutic product is Tripeptidyl-Peptidase 1 (TPP1); (3) the pathology of the eye is associated with Batten-CLN3 and the therapeutic product is Battenin (CLN3); (4) the pathology of the eye is associated with Batten-CLN6 and the therapeutic product is CLN6 Transmembrane ER Protein (CLN6); (5) the pathology of the eye is associated with Batten-CLN7 and the therapeutic product is Major Facilitator Superfamily Domain Containing 8 (MFSD8); (6) the pathology of the eye is associated with Usher's-Type 1 and the therapeutic product is Myosin VIIA (MYO7A); (7) the pathology of the eye is associated with Usher's-Type 1 and the therapeutic product is Cadherin Related 23 (CDH23); (8) the pathology of the eye is associated with Usher's-Type 2 and the therapeutic product is Protocadherin Related 15 (PCDH15); (9) the pathology of the eye is associated with Usher's-Type 2 and the therapeutic product is Usherin (USH2A); (10) the pathology of the eye is associated with Usher's-Type 3 and the therapeutic product is Clarin 1 (CLRN1); (11) the pathology of the eye is associated with Stargardt's and the therapeutic product is ATP Binding Cassette Subfamily A Member 4 (ABCA4); (12) the pathology of the eye is associated with Stargardt's and the therapeutic product is ELOVL Fatty Acid Elongase 4 (ELOVL4); (13) the pathology of the eye is associated with uveitis and the therapeutic product is an anti-Interleukin 6 (IL6) monoclonal antibody; (14) the pathology of the eye is associated with uveitis and the therapeutic product is an anti-TNF-alpha (TNF) monoclonal antibody; (15) the pathology of the eye is associated with diabetic macular edema (DME) and the therapeutic product is an anti-IL6 monoclonal antibody; (16) the pathology of the eye is associated with red-green color blindness and the therapeutic product is L opsin (OPN1LW); (17) the pathology of the eye is associated with red-green color blindness and the therapeutic product is M opsin (OPN1MW); (18) the pathology of the eye is associated with blue cone monochromacy and the therapeutic product is M opsin (OPN1MW); (19) the pathology of the eye is associated with Leber congenital amaurosis-1 (LCA 1) and the therapeutic product is Guanylate Cyclase 2D, Retinal (GUCY2D); (20) the pathology of the eye is associated with Leber congenital amaurosis-2 (LCA 2) and the therapeutic product is Retinoid Isomerohydrolase RPE65 (RPE65); (21) the pathology of the eye is associated with LCA 3 and the therapeutic product is Spermatogenesis Associated 7 (SPATA7); (22) the pathology of the eye is associated with Leber congenital amaurosis-4 (LCA 4) and the therapeutic product is Aryl Hydrocarbon Receptor Interacting Protein Like 1 (AIPL1); (23) the pathology of the eye is associated with Leber congenital amaurosis-5 (LCA 5) and the therapeutic product is Lebercilin (LCA5); (24) the pathology of the eye is associated with Leber congenital amaurosis-6 (LCA 6) and the therapeutic product is RPGR Interacting Protein 1 (RPGRIP1); (25) the pathology of the eye is associated with Leber congenital amaurosis-7 (LCA 7) and the therapeutic product is Cone-Rod Homeobox (CRX); (26) the pathology of the eye is associated with Leber congenital amaurosis-8 (LCA 8) and the therapeutic product is Crumbs Cell Polarity Complex Component 1 (CRB1) (also known as LCA8); (27) the pathology of the eye is associated with Leber congenital amaurosis-9 (LCA 9) and the therapeutic product is Nicotinamide Nucleotide Adenylyltransferase 1 (NMNAT1); (28) the pathology of the eye is associated with Leber congenital amaurosis-10 (LCA 10) and the therapeutic product is Centrosomal Protein 290 (CEP290); (29) the pathology of the eye is associated with Leber congenital amaurosis-11 (LCA 11) and the therapeutic product is Inosine Monophosphate Dehydrogenase 1 (IMPDH1); (30) the pathology of the eye is associated with Leber congenital amaurosis-12 (LCA 12) and the therapeutic product is Retinal Degeneration 3, GUCY2D regulator (RD3) ; (31) the pathology of the eye is associated with Leber congenital amaurosis-13 (LCA 13) and the therapeutic product is Retinol Dehydrogenase 12 (RDH12); (32) the pathology of the eye is associated with Leber congenital amaurosis-14 (LCA 14) and the therapeutic product is Lecithin Retinol Acyltransferase (LRAT); (33) the pathology of the eye is associated with Leber congenital amaurosis-15 (LCA 15) and the therapeutic product is Tubby Like Protein 1 (TULP1); (34) the pathology of the eye is associated with Leber congenital amaurosis-16 (LCA 16) and the therapeutic product is Potassium Voltage-Gated Channel Subfamily J Member 13 (KCNJ13); (35) the pathology of the eye is associated with Leber's hereditary optic neuropathy (LHON) and the therapeutic product is Mitochondrially Encoded NADH Dehydrogenase 1 (MT-ND1); (36) the pathology of the eye is associated with LHON and the therapeutic product is Mitochondrially Encoded NADH Dehydrogenase 4 (MT-ND4); (37) the pathology of the eye is associated with LHON and the therapeutic product is Mitochondrially Encoded NADH Dehydrogenase 6 (MT-ND6); (38) the pathology of the eye is associated with neuromyelitis optica (NMO) and the therapeutic product is an anti-complement antibody or an anti-complement aptamer, wherein the anti-complement monoclonal antibody or aptamer is an anti-complement C1 antibody or aptamer, an anti-complement C1q monoclonal antibody or aptamer, an anti-complement C1s monoclonal antibody or aptamer, an anti-complement C2 monoclonal antibody or aptamer, an anti-complement C2a monoclonal antibody or aptamer, an anti-complement C2b monoclonal antibody or aptamer, an anti-complement C3 monoclonal antibody or aptamer, an anti-complement C3a monoclonal antibody or aptamer, an anti-complement C3b monoclonal antibody or aptamer, an anti-complement C4 monoclonal antibody or aptamer, an anti-complement C4a monoclonal antibody or aptamer, an anti-complement C4b monoclonal antibody or aptamer, an anti-complement C5 monoclonal antibody or aptamer, an anti-complement C5a monoclonal antibody or aptamer, an anti-complement C5b monoclonal antibody or aptamer, an anti-complement C6 monoclonal antibody or aptamer, an anti-complement C7 monoclonal antibody or aptamer, an anti-complement C8 monoclonal antibody or aptamer, or an anti-complement C9 monoclonal antibody or aptamer, or preferably an anti-complement C5 antibody; (39) the pathology of the eye is associated with NMO and the therapeutic product is an anti-IL6 monoclonal antibody or aptamer; (40) the pathology of the eye is associated with uveitis and the therapeutic product is an anti-complement monoclonal antibody or aptamer, wherein the anti-complement monoclonal antibody or aptamer is an anti-complement C1 monoclonal antibody or aptamer, an anti-complement C1q monoclonal antibody or aptamer, an anti-complement C1s monoclonal antibody or aptamer, an anti-complement C2 monoclonal antibody or aptamer, an anti-complement C2a monoclonal antibody or aptamer, an anti-complement C2b monoclonal antibody or aptamer, an anti-complement C3 monoclonal antibody or aptamer, an anti-complement C3a monoclonal antibody or aptamer, an anti-complement C3b monoclonal antibody or aptamer, an anti-complement C4 monoclonal antibody or aptamer, an anti-complement C4a monoclonal antibody or aptamer, an anti-complement C4b monoclonal antibody or aptamer, an anti-complement C5 monoclonal antibody or aptamer, an anti-complement C5a monoclonal antibody or aptamer, an anti-complement C5b monoclonal antibody or aptamer, an anti-complement C6 monoclonal antibody or aptamer, an anti-complement C7 monoclonal antibody or aptamer, an anti-complement C8 monoclonal antibody or aptamer, or an anti-complement C9 monoclonal antibody or aptamers, or preferably an anti-complement C5 antibody; (41) the pathology of the eye is associated with uveitis and the therapeutic product is Angiotensin I Converting Enzyme (ACE); (42) the pathology of the eye is associated with uveitis and the therapeutic product is Interleukin 10 (IL10); (43) the pathology of the eye is associated with uveitis and the therapeutic product is an anti-TNF monoclonal antibody; (44) the pathology of the eye is associated with choroideremia and the therapeutic product is Rab Escort Protein 1 (CHM); (45) the pathology of the eye is associated with X-linked retinoschisis (XLRS) and the therapeutic product is Retinoschisin (RS1); (46) the pathology of the eye is associated with Bardet-Biedl syndrome 1 and the therapeutic product is Bardet-Biedl Syndrome 1 (BBS1); (47) the pathology of the eye is associated with Bardet-Biedl syndrome 2 and the therapeutic product is Bardet-Biedl Syndrome 2 (BBS2); (48) the pathology of the eye is associated with Bardet-Biedl syndrome 3 and the therapeutic product is ADP Ribosylation Factor Like GTPase 6 (ARL6) (also known as BBS3); (49) the pathology of the eye is associated with Bardet-Biedl syndrome 4 and the therapeutic product is Bardet-Biedl Syndrome 4 (BBS4); (50) the pathology of the eye is associated with Bardet-Biedl syndrome 5 and the therapeutic product is Bardet-Biedl Syndrome 5 (BBS5); (51) the pathology of the eye is associated with Bardet-Biedl syndrome 6 and the therapeutic product is McKusick-Kaufman Syndrome (MKKS), also known as BBS6; (52) the pathology of the eye is associated with Bardet-Biedl syndrome 7 and the therapeutic product is Bardet-Biedl Syndrome 7 (BBS7); (53) the pathology of the eye is associated with Bardet-Biedl syndrome 8 and the therapeutic product is Tetratricopeptide Repeat Domain 8 (TTC8), also known as BBS8; (54) the pathology of the eye is associated with Bardet-Biedl syndrome 9 and the therapeutic product is Bardet-Biedl Syndrome 9 (BBS9); (55) the pathology of the eye is associated with Bardet-Biedl syndrome 10 and the therapeutic product is Bardet-Biedl Syndrome 10 (BBS10); (56) the pathology of the eye is associated with Bardet-Biedl syndrome 11 and the therapeutic product is Tripartite Motif Containing 32 (TRIM32), also known as BBS11; (57) the pathology of the eye is associated with Bardet-Biedl syndrome 12 and the therapeutic product is Bardet-Biedl Syndrome 12 (BBS12); (58) the pathology of the eye is associated with Bardet-Biedl syndrome 13 and the therapeutic product is MKS Transition Zone Complex Subunit 1 (MKS1), also known as BBS13; (59) the pathology of the eye is associated with Bardet-Biedl syndrome 14 and the therapeutic product is Centrosomal Protein 290 (CEP290), also known as BBS14 and LCA10; (60) the pathology of the eye is associated with Bardet-Biedl syndrome 15 and the therapeutic product is WD Repeat Containing Planar Cell Polarity Effector (WDPCP), also known as BBS15; (61) the pathology of the eye is associated with Bardet-Biedl syndrome 16 and the therapeutic product is Serologically Defined Colon Cancer Antigen 8 (SDCCAG8), also known as BBS16; (62) the pathology of the eye is associated with Bardet-Biedl syndrome 17 and the therapeutic product is Leucine Zipper Transcription Factor Like 1 (LZTFL1), also known as BBS17; (63) the pathology of the eye is associated with Bardet-Biedl syndrome 18 and the therapeutic product is BBSome Interacting Protein 1 (BBIP1), also known as BBS18; (64) the pathology of the eye is associated with Bardet-Biedl syndrome 19 and the therapeutic product is Intraflagellar Transport 27 (IFT27), also known as BBS19; (65) the pathology of the eye is associated with cone dystrophy and the therapeutic product is Guanylate Cyclase Activator 1A (GUCA1A); (66) the pathology of the eye is associated with optic atrophy and the therapeutic product is OPA1 Mitochondrial Dynamin Like GTPase (OPA1); (67) the pathology of the eye is associated with retinitis pigmentosa 1 and the therapeutic product is RP1 Axonemal Microtubule Associated (RP1); (68) the pathology of the eye is associated with retinitis pigmentosa 2 and the therapeutic product is RP2 Activator of ARL3 GTPase (RP2); (69) the pathology of the eye is associated with retinitis pigmentosa 7 and the therapeutic product is Peripherin 2 (PRPH2); (70) the pathology of the eye is associated with retinitis pigmentosa 11 and the therapeutic product is Pre-mRNA Processing Factor 31(PRPF31); (71) the pathology of the eye is associated with retinitis pigmentosa 12 and the therapeutic product is Crumbs Cell Polarity Complex Component 1 (CRB1), also known as LCA8; (72) the pathology of the eye is associated with retinitis pigmentosa 13 and the therapeutic product is Pre-mRNA Processing Factor 8 (PRPF8); (73) the pathology of the eye is associated with retinitis pigmentosa 25 and the therapeutic product is Eyes Shut Homolog (EYS); (74) the pathology of the eye is associated with retinitis pigmentosa 28 and the therapeutic product is FAM161 Centrosomal Protein A (FAM161A); (75) the pathology of the eye is associated with retinitis pigmentosa 37 and the therapeutic product is Nuclear Receptor Subfamily 2 Group E Member 3 (NR2E3); (76) the pathology of the eye is associated with retinitis pigmentosa 38 and the therapeutic product is MER Proto-Oncogene, Tyrosine Kinase (MERTK); (77) the pathology of the eye is associated with retinitis pigmentosa 40 and the therapeutic product is Phosphodiesterase 6B (PDE6B); (78) the pathology of the eye is associated with retinitis pigmentosa 41 and the therapeutic product is Prominin 1 (PROM1); (79) the pathology of the eye is associated with retinitis pigmentosa 43 and the therapeutic product is Phosphodiesterase 6A (PDE6A); (80) the pathology of the eye is associated with retinitis pigmentosa 56 and the therapeutic product is Interphotoreceptor Matrix Proteoglycan 2 (IMPG2); (81) the pathology of the eye is associated with petinitis pigmentosa 62 and the therapeutic product is Male Germ Cell Associated Kinase (MAK); (82) the pathology of the eye is associated with retinitis pigmentosa 80 and the therapeutic product is Intraflagellar Transport 140 (IFT140); (83) the pathology of the eye is associated with dry AMD and the therapeutic product is an anti-complement monoclonal antibody or an anti-complement aptamer, wherein the anti-complement monoclonal antibody or an anti-complement aptamer is an anti-complement C1 monoclonal antibody or aptamer, an anti-complement C1q monoclonal antibody or aptamer, an anti-complement C1s monoclonal antibody or aptamer, an anti-complement C2 monoclonal antibody or aptamer, an anti-complement C2a monoclonal antibody or aptamer, an anti-complement C2b monoclonal antibody or aptamer, an anti-complement C3 monoclonal antibody or aptamer, an anti-complement C3a monoclonal antibody or aptamer, an anti-complement C3b monoclonal antibody or aptamer, an anti-complement C4 monoclonal antibody or aptamer, an anti-complement C4a monoclonal antibody or aptamer, an anti-complement C4b monoclonal antibody or aptamer, an anti-complement C5 monoclonal antibody or aptamer, an anti-complement C5a monoclonal antibody or aptamer, an anti-complement C5b monoclonal antibody or aptamer, an anti-complement C6 monoclonal antibody or aptamer, an anti-complement C7 monoclonal antibody or aptamer, an anti-complement C8 monoclonal antibody or aptamer, or an anti-complement C9 monoclonal antibody or aptamers, or preferably an anti-complement C5 antibody; (84) the pathology of the eye is associated with dry AMD and the therapeutic product is an anti-membrane attack complex (MAC) therapeutic product, preferably the anti-MAC therapeutic product is an anti-MAC monoclonal antibody, which is a monoclonal antibody against a human protein of the membrane attack complex, which is composed of four complement proteins C5b (SEQ ID NOs. 314-316), C6 (SEQ ID NO. 317), C7 (SEQ ID NO. 318), and C8 (SEQ ID NOs. 319-321); (85) the pathology of the eye is associated with dry AMD and the therapeutic product is HtrA Serine Peptidase 1 (HTRA1); (86) the pathology of the eye is associated with Best disease and the therapeutic product is Bestrophin 1 (BEST1); (87) the pathology of the eye is associated with dry AMD and the therapeutic product is a complement factor B antisense oligonucleotide; (88) the pathology of the eye is associated with dry AMD and the therapeutic product is an anti-beta-amyloid monoclonal antibody;(89) the pathology of the eye is associated with dry AMD and the therapeutic product is CD59 glycoprotein (CD59); (90) the pathology of the eye is associated with dry AMD and the therapeutic product is Channelrhodopsin-1 (ChR1), which includes the human homolog of ChR1; (91) the pathology of the eye is associated with dry AMD and the therapeutic product is Channelrhodopsin-2 (ChR2), which includes the human homolog of ChR2; (92) the pathology of the eye is associated with dry AMD and the therapeutic product is an anti-complement monoclonal antibody or an anti-complement aptamer, wherein the anti-complement monoclonal antibody or an anti-complement aptamer is an anti-complement C1 monoclonal antibody or aptamer, an anti-complement C1q monoclonal antibody or aptamer, an anti-complement C1s monoclonal antibody or aptamer, an anti-complement C2 monoclonal antibody or aptamer, an anti-complement C2a monoclonal antibody or aptamer, an anti-complement C2b monoclonal antibody or aptamer, an anti-complement C3 monoclonal antibody or aptamer, an anti-complement C3a monoclonal antibody or aptamer, an anti-complement C3b monoclonal antibody or aptamer, an anti-complement C4 monoclonal antibody or aptamer, an anti-complement C4a monoclonal antibody or aptamer, an anti-complement C4b monoclonal antibody or aptamer, an anti-complement C5 monoclonal antibody or aptamer, an anti-complement C5a monoclonal antibody or aptamer, an anti-complement C5b monoclonal antibody or aptamer, an anti-complement C6 monoclonal antibody or aptamer, an anti-complement C7 monoclonal antibody or aptamer, an anti-complement C8 monoclonal antibody or aptamer, or an anti-complement C9 monoclonal antibody or aptamers, or preferably an anti-complement C5 antibody; (93) the pathology of the eye is associated with dry AMD and the therapeutic product is anti-complement factor D therapeutic product, including but not limited to an anti-complement factor D monoclonal antibody, or an anti-complement factor D aptamer; (94) the pathology of the eye is associated with age-related retinal ganglion cell (RGC) degeneration and the therapeutic product is DnaJ heat shock protein family (Hsp40) member C3 (DNAJC3), also known as P58IPK; (95) the pathology of the eye is associated with blue cone monochromacy (BCM) and the therapeutic product is L opsin (OPN1LW); (96) the pathology of the eye is associated with glaucoma and the therapeutic product is beta-2 adrenoceptor siRNA; (97) the pathology of the eye is associated with glaucoma and the therapeutic product is Caspase-2 (CASP2); (98) the pathology of the eye is associated with glaucoma and the therapeutic product is Insulin Receptor Substrate 1 (IRS1); (99) the pathology of the eye is associated with glaucoma and the therapeutic product is HIF-1 Responsive Protein RTP801 (RTP801); (100) the pathology of the eye is associated with glaucoma and the therapeutic product is Transforming Growth Factor Beta 2 (TGFB2); (101) the pathology of the eye is associated with glaucoma and the therapeutic product is Brain Derived Neurotrophic Factor (BDNF); (102) the pathology of the eye is associated with glaucoma and the therapeutic product is Ciliary Neurotrophic Factor (CNTF); (103) the pathology of the eye is associated with glaucoma and the therapeutic product is Prostaglandin-Endoperoxide Synthase 2 (PTGS2); (104) the pathology of the eye is associated with glaucoma and the therapeutic product is Prostaglandin F Receptor (PTGFR) (when the pathology of the eye is associated with glaucoma, in a specific embodiment, a recombinant viral vector comprising a nucleotide sequence encoding PTGFR can be administered to the human subject in combination with a recombinant viral vector comprising a nucleotide sequence encoding PTGS2; in another specific embodiment, a recombinant viral vector comprising a nucleotide sequence encoding PTGFR and a nucleotide sequence encoding PTGS2 can be administered to the human subject); (105) the pathology of the eye is associated with glaucoma and the therapeutic product is a hyaluronidase, e.g. HYAL1, HYAL2, HYAL3, HYAL4, and HYAL5; (106) the pathology of the eye is associated with glaucoma and the therapeutic product is Pigment Epithelium-Derived Factor (PEDF); (107) the pathology of the eye is associated with glaucoma and the therapeutic product is Vascular Endothelial Growth Factor (VEGF); (108) the pathology of the eye is associated with glaucoma and the therapeutic product is Placental Growth Factor (PGF), wherein PGF can be used in combo with VEGF; (109) the pathology of the eye is associated with glaucoma (e.g., a congenital glaucoma or juvenile glaucoma) and the therapeutic product is Myocilin (MYOC); (110) the pathology of the eye is associated with NMO and the therapeutic product is an anti-complement C5 monoclonal antibody; (111) the pathology of the eye is associated with NMO and the therapeutic product is C-C Motif Chemokine Receptor 5 (CCR5) siRNA, CCR5 shRNA, siRNA or CCR5 miRNA (preferably, a CCR5 miRNA); (112) the pathology of the eye is associated with NMO and the therapeutic product is an anti-CD19 monoclonal antibody; (113) the pathology of the eye is associated with retinitis pigmentosa that is associated with rhodopsin mutations and the therapeutic product is Channelrhodopsin-1 (ChR1), which includes the human homolog of ChR1; (114) the pathology of the eye is associated with retinitis pigmentosa that is associated with rhodopsin mutations and the therapeutic product is Channelrhodopsin-2 (ChR2), which includes the human homolog of ChR2; (115) the pathology of the eye is associated with retinitis pigmentosa and the therapeutic product is Ciliary Neurotrophic Factor (CNTF); (116) the pathology of the eye is associated with autosomal recessive retinitis pigmentosa and the therapeutic product is Crumbs Cell Polarity Complex Component 1 (CRB1); (117) the pathology of the eye is associated with autosomal recessive retinitis pigmentosa and the therapeutic product is Crumbs Cell Polarity Complex Component 2 (CRB2); (118) the pathology of the eye is associated with retinitis pigmentosa and the therapeutic product is Histone Deacetylase 4 (HDAC4); (119) the pathology of the eye is associated with retinitis pigmentosa and the therapeutic product is Rhodopsin (RHO); (120) the pathology of the eye is associated with retinitis pigmentosa and the therapeutic product is Nerve Growth Factor (NGF); (121) the pathology of the eye is associated with retinitis pigmentosa and the therapeutic product is Nuclear Factor, Erythroid 2 Like 2 (NRF2); (122) the pathology of the eye is associated with retinitis pigmentosa and the therapeutic product is Pigment Epithelium-Derived Factor (PEDF); (123) the pathology of the eye is associated with retinitis pigmentosa and the therapeutic product is Glutathione S-Transferase PI 1 (GSTP1), also known as PI; (124) the pathology of the eye is associated with retinitis pigmentosa and the therapeutic product is Rod-Derived Cone Viability Factor (RDCVF); (125) the pathology of the eye is associated with retinitis pigmentosa and the therapeutic product is Rhodopsin (RHO); (126) the pathology of the eye is associated with retinitis pigmentosa and the therapeutic product is Retinaldehyde Binding Protein 1 (RLBP1); (127) the pathology of the eye is associated with Stargardt's disease and the therapeutic product is an anti-complement C5 aptamer; (128) the pathology of the eye is associated with uveitis and the therapeutic product is Double Homeobox 4 (DUX4); (129) the pathology of the eye is associated with uveitis and the therapeutic product is NLR Family Pyrin Domain Containing 3 (NLRP3); (130) the pathology of the eye is associated with uveitis and the therapeutic product is Spleen Associated Tyrosine Kinase (SYK); (131) the pathology of the eye is associated with uveitis and the therapeutic product is Adrenocorticotropic Hormone (ACTH); (132) the pathology of the eye is associated with uveitis and the therapeutic product is Caspase 1 (CASP1); (133) the pathology of the eye is associated with uveitis and the therapeutic product is anti-CD59 therapeutic product (such as an anti-CD59 therapeutic protein (for example, an anti-CD59 monoclonal antibody), or an anti-CD59 therapeutic RNA (for example, an anti-CD59 shRNA, anti-CD59 siRNA, or anti-CD59 miRNA), preferably an anti-CD59 monoclonal antibody); (134) the pathology of the eye is associated with uveitis and the therapeutic product is an anti-complement monoclonal antibody or an anti-complement aptamer, wherein the anti-complement monoclonal antibody or aptamer is an anti-complement C1 monoclonal antibody or aptamer, an anti-complement C1q monoclonal antibody or aptamer, an anti-complement C1s monoclonal antibody or aptamer, an anti-complement C2 monoclonal antibody or aptamer, an anti-complement C2a monoclonal antibody or aptamer, an anti-complement C2b monoclonal antibody or aptamer, an anti-complement C3 monoclonal antibody or aptamer, an anti-complement C3a monoclonal antibody or aptamer, an anti-complement C3b monoclonal antibody or aptamer, an anti-complement C4 monoclonal antibody or aptamer, an anti-complement C4a monoclonal antibody or aptamer, an anti-complement C4b monoclonal antibody or aptamer, an anti-complement C5 monoclonal antibody or aptamer, an anti-complement C5a monoclonal antibody or aptamer, an anti-complement C5b monoclonal antibody or aptamer, an anti-complement C6 monoclonal antibody or aptamer, an anti-complement C7 monoclonal antibody or aptamer, an anti-complement C8 monoclonal antibody or aptamer, or an anti-complement C9 monoclonal antibody or aptamers, or preferably an anti-complement C5 antibody; (135) the pathology of the eye is associated with corneal neovascularization and the therapeutic product is Insulin Receptor Substrate 1 (IRS1); (136) the pathology of the eye is associated with corneal neovascularization and the therapeutic product is NOTCH Regulated Ankyrin Repeat Protein (NRARP); (137) the pathology of the eye is associated with diabetic retinopathy and the therapeutic product is NOTCH Regulated Ankyrin Repeat Protein (NRARP); (138) the pathology of the eye is associated with diabetic retinopathy and the therapeutic product is Alpha-2-Antiplasmin (A2AP); (139) the pathology of the eye is associated with diabetic retinopathy and the therapeutic product is Plasminogen (PLG); (140) the pathology of the eye is associated with diabetic retinopathy and the therapeutic product can be a growth hormone; (141) the pathology of the eye is associated with diabetic retinopathy and the therapeutic product is Insulin Like Growth Factor 1 (IGF1), wherein IGF1 can be used in combo with growth hormone; (142) the pathology of the eye is associated with diabetic retinopathy and the therapeutic product is Interleukin 1 Beta (IL1B).(143) the pathology of the eye is associated with diabetic retinopathy and the therapeutic product is Angiotensin I Converting Enzyme 2 (ACE2), wherein ACE2 can be used in combo with IL1B; (144) the pathology of the eye is associated with diabetic retinopathy and the therapeutic product is IRS1; (145) the pathology of the eye is associated with diabetic retinopathy and the therapeutic product is an anti-integrin oligopeptide; (146) the pathology of the eye is associated with diabetic retinopathy and the therapeutic product is an anti-Placental Growth Factor (PGF) monoclonal antibody; (147) the pathology of the eye is associated with Graves' ophthalmopathy (also known as Graves' orbitopathy) and the therapeutic product is an anti-CD40 monoclonal antibody; (148) the pathology of the eye is associated with Graves' ophthalmopathy and the therapeutic product is an anti-Insulin-Like Growth Factor 1 Receptor (IGF1R) monoclonal antibody; (149) the pathology of the eye is associated with Graves' ophthalmopathy and the therapeutic product is an anti-Insulin-Like Growth Factor 2 Receptor (IGF2R) monoclonal antibody; (150) the pathology of the eye is associated with DME and the therapeutic product is an anti-integrin oligopeptide; (151) the pathology of the eye is associated with DME and the therapeutic product is an anti-Placental Growth Factor (PGF) monoclonal antibody; (152) the pathology of the eye is associated with DME and the therapeutic product is RTP801 siRNA; (153) the pathology of the eye is associated with multiple sclerosis (MS)-associated vision loss and the therapeutic product is ND1; (154) the pathology of the eye is associated with myopia and the therapeutic product is Matrix Metalloproteinase 2 (MMP2) RNAi; (155) the pathology of the eye is associated with X-linked recessive ocular albinism and the therapeutic product is G-Protein Coupled Receptor 143 (GPR143); (156) the pathology of the eye is associated with oculocutaneous albinism type 1 and the therapeutic product is Tyrosinase (TYR); (157) the pathology of the eye is associated with optic neuritis and the therapeutic product is Caspase 2 (CASP2); (158) the pathology of the eye is associated with optic neuritis and the therapeutic product is an anti-Leucine Rich Repeat And Ig Domain Containing Protein 1 (LINGO1) monoclonal antibody; or(159) the pathology of the eye is associated with polypoidal choroidal vasculopathy and the therapeutic product is anti-complement monoclonal antibody or an anti-complement aptamer, wherein the anti-complement monoclonal antibody or aptamer is an anti-complement C1 monoclonal antibody or aptamer, an anti-complement C1q monoclonal antibody/aptamer, an anti-complement C1s monoclonal antibody/aptamer, an anti-complement C2 monoclonal antibody/aptamer, an anti-complement C2a monoclonal antibody or aptamer, an anti-complement C2b monoclonal antibody or aptamer, an anti-complement C3 monoclonal antibody or aptamer, an anti-complement C3a monoclonal antibody or aptamer, an anti-complement C3b monoclonal antibody or aptamer, an anti-complement C4 monoclonal antibody or aptamer, an anti-complement C4a monoclonal antibody or aptamer, an anti-complement C4b monoclonal antibody or aptamer, an anti-complement C5 monoclonal antibody or aptamer, an anti-complement C5a monoclonal antibody or aptamer, an anti-complement C5b monoclonal antibody or aptamer, an anti-complement C6 monoclonal antibody or aptamer, an anti-complement C7 monoclonal antibody or aptamer, an anti-complement C8 monoclonal antibody or aptamer, or an anti-complement C9 monoclonal antibody or aptamers, or preferably an anti-complement C5 antibody.

In certain embodiments of the methods described herein, the pathology of the eye is associated with X-linked retinitis pigmentosa (XLRP) and the therapeutic product is Retinitis Pigmentosa GTPase Regulator (RPGR). In certain embodiments of any of the foregoing methods, the pathology of the eye is associated with achromatopsia (ACHM) and the therapeutic product is Cyclic Nucleotide Gated Channel Beta 3 (CNGB3). In certain embodiments of any of the foregoing methods, the pathology of the eye is associated with achromatopsia (for example, a CNGA3-linked achromatopsia) and the therapeutic product is Cyclic Nucleotide Gated Channel Alpha 3 (CNGA3). In certain embodiments of any of the foregoing methods, the pathology of the eye is associated with biallelic RPE65 mutation-associated retinal dystrophy and the therapeutic product is Retinoid Isomerohydrolase RPE65 (RPE65).

In certain embodiments of the methods described herein, the pathology of the eye is associated with (1) Batten-CLN1 and the therapeutic product is Palmitoyl-Protein Thioesterase 1 (PPT1); (2) Batten-CLN2 and the therapeutic product is Tripeptidyl-Peptidase 1 (TPP1); (3) Batten-CLN3 and the therapeutic product is Battenin (CLN3); (4) uveitis and the therapeutic product is an anti-Interleukin 6 (IL6) monoclonal antibody; (5) uveitis and the therapeutic product is an anti-TNF-alpha (TNF) monoclonal antibody; (6) diabetic macular edema (DME) and the therapeutic product is an anti-IL6 monoclonal antibody; (7) red-green color blindness and the therapeutic product is L opsin (OPN1LW); (8) red-green color blindness and the therapeutic product is M opsin (OPN1MW); (9) blue cone monochromacy and the therapeutic product is M opsin (OPN1MW); (10) Leber congenital amaurosis-1 (LCA 1) and the therapeutic product is Guanylate Cyclase 2D, Retinal (GUCY2D); (11) Leber congenital amaurosis-2 (LCA 2) and the therapeutic product is Retinoid Isomerohydrolase RPE65 (RPE65); (12) Leber congenital amaurosis-7 (LCA 7) and the therapeutic product is Cone-Rod Homeobox (CRX); (13) Leber congenital amaurosis-11 (LCA 11) and the therapeutic product is Inosine Monophosphate Dehydrogenase 1 (IMPDH1); (14) Leber congenital amaurosis-12 (LCA 12) and the therapeutic product is Retinal Degeneration 3, GUCY2D regulator (RD3); (15) Leber congenital amaurosis-13 (LCA 13) and the therapeutic product is Retinol Dehydrogenase 12 (RDH12); (16) Leber congenital amaurosis-15 (LCA 15) and the therapeutic product is Tubby Like Protein 1 (TULP1); (17) Leber congenital amaurosis-16 (LCA 16) and the therapeutic product is Potassium Voltage-Gated Channel Subfamily J Member 13 (KCNJ13); (18) Leber's hereditary optic neuropathy (LHON) and the therapeutic product is Mitochondrially Encoded NADH Dehydrogenase 1 (MT-ND1); (19) LHON and the therapeutic product is Mitochondrially Encoded NADH Dehydrogenase 4 (MT-ND4); (20) LHON and the therapeutic product is Mitochondrially Encoded NADH Dehydrogenase 6 (MT-ND6); (21) neuromyelitis optica (NMO) and the therapeutic product is an anti-complement monoclonal antibody or an anti-complement aptamer, wherein the anti-complement monoclonal antibody or aptamer is an anti-complement C1 monoclonal antibody or aptamer, an anti-complement C1q monoclonal antibody or aptamer, an anti-complement C1s monoclonal antibody or aptamer, an anti-complement C2 monoclonal antibody or aptamer, an anti-complement C2a monoclonal antibody or aptamer, an anti-complement C2b monoclonal antibody or aptamer, an anti-complement C3 monoclonal antibody or aptamer, an anti-complement C3a monoclonal antibody or aptamer, an anti-complement C3b monoclonal antibody or aptamer, an anti-complement C4 monoclonal antibody or aptamer, an anti-complement C4a monoclonal antibody or aptamer, an anti-complement C4b monoclonal antibody or aptamer, an anti-complement C5 monoclonal antibody or aptamer, an anti-complement C5a monoclonal antibody or aptamer, an anti-complement C5b monoclonal antibody or aptamer, an anti-complement C6 monoclonal antibody or aptamer, an anti-complement C7 monoclonal antibody or aptamer, an anti-complement C8 monoclonal antibody or aptamer, or an anti-complement C9 monoclonal antibody or aptamers, or preferably an anti-complement C5 antibody; (22) NMO and the therapeutic product is an anti-IL6 monoclonal antibody; (23) uveitis and the therapeutic product is an anti-complement C5 monoclonal antibody; (24) uveitis and the therapeutic product is Angiotensin I Converting Enzyme (ACE); (25) uveitis and the therapeutic product is Interleukin 10 (IL10); (26) uveitis and the therapeutic product is an anti-TNF monoclonal antibody; (27) X-linked retinoschisis (XLRS) and the therapeutic product is Retinoschisin (RS1); (28) Bardet-Biedl syndrome 1 and the therapeutic product is Bardet-Biedl Syndrome 1 (BBS1); (29) Bardet-Biedl syndrome 3 and the therapeutic product is ADP Ribosylation Factor Like GTPase 6 (ARL6); (30) Bardet-Biedl syndrome 5 and the therapeutic product is Bardet-Biedl Syndrome 5 (BBS5); (31) Bardet-Biedl syndrome 6 and the therapeutic product is McKusick-Kaufman Syndrome (MKKS); (32) Bardet-Biedl syndrome 10 and the therapeutic product is Bardet-Biedl Syndrome 10 (BBS10); (33) Bardet-Biedl syndrome 11 and the therapeutic product is Tripartite Motif Containing 32 (TRIM32); (34) Bardet-Biedl syndrome 13 and the therapeutic product is MKS Transition Zone Complex Subunit 1 (MKS1); (35) Bardet-Biedl syndrome 18 and the therapeutic product is BBSome Interacting Protein 1 (BBIP1); (36) Bardet-Biedl syndrome 19 and the therapeutic product is Intraflagellar Transport 27 (IFT27); (37) cone dystrophy and the therapeutic product is Guanylate Cyclase Activator 1A (GUCA1A); (38) retinitis pigmentosa 13 and the therapeutic product is Pre-mRNA Processing Factor 8 (PRPF8); (39) retinitis pigmentosa 37 and the therapeutic product is Nuclear Receptor Subfamily 2 Group E Member 3 (NR2E3); or (40) Best disease and the therapeutic product is Bestrophin 1 (BEST1).

In certain embodiments of the methods described herein, the pathology of the eye is associated with biallelic RPE65 mutation-associated retinal dystrophy and the therapeutic product is Retinoid Isomerohydrolase RPE65 (RPE65).

In certain embodiments of the methods described herein, the pathology of the eye is associated with (1) Batten-CLN2 and the therapeutic product is Tripeptidyl-Peptidase 1 (TPP1); (2) Usher's-Type 1 and the therapeutic product is Myosin VIIA (MYO7A); (3) Usher's-Type 1 and the therapeutic product is Cadherin Related 23 (CDH23); (4) Usher's-Type 2 and the therapeutic product is Protocadherin Related 15 (PCDH15); (5) Usher's-Type 2 and the therapeutic product is Usherin (USH2A); (6) Usher's-Type 3 and the therapeutic product is Clarin 1 (CLRN1); (7) Stargardt's and the therapeutic product is ATP Binding Cassette Subfamily A Member 4 (ABCA4); (8) Stargardt's and the therapeutic product is ELOVL Fatty Acid Elongase 4 (ELOVL4); (9) red-green color blindness and the therapeutic product is L opsin (OPN1LW); (10) red-green color blindness and the therapeutic product is M opsin (OPN1MW); (11) blue cone monochromacy and the therapeutic product is M opsin (OPN1MW); (12) Leber congenital amaurosis-1 (LCA 1) and the therapeutic product is Guanylate Cyclase 2D, Retinal (GUCY2D); (13) Leber congenital amaurosis-2 (LCA 2) and the therapeutic product is Retinoid Isomerohydrolase RPE65 (RPE65); (14) Leber congenital amaurosis-4 (LCA 4) and the therapeutic product is Aryl Hydrocarbon Receptor Interacting Protein Like 1 (AIPL1); (15) Leber congenital amaurosis-7 (LCA 7) and the therapeutic product is Cone-Rod Homeobox (CRX); (16) Leber congenital amaurosis-8 (LCA 8) and the therapeutic product is Crumbs Cell Polarity Complex Component 1 (CRB1); (17) Leber congenital amaurosis-9 (LCA 9) and the therapeutic product is Nicotinamide Nucleotide Adenylyltransferase 1 (NMNAT1); (18) Leber congenital amaurosis-10 (LCA 10) and the therapeutic product is Centrosomal Protein 290 (CEP290); (19) Leber congenital amaurosis-11 (LCA 11) and the therapeutic product is Inosine Monophosphate Dehydrogenase 1 (IMPDH1); (20) Leber congenital amaurosis-15 (LCA 15) and the therapeutic product is Tubby Like Protein 1 (TULP1); (21) LHON and the therapeutic product is Mitochondrially Encoded NADH Dehydrogenase 4 (MT-ND4); (22) LHON and the therapeutic product is Mitochondrially Encoded NADH Dehydrogenase 6 (MT-ND6); (23) choroideremia and the therapeutic product is Rab Escort Protein 1 (CHM); (24) X-linked retinoschisis (XLRS) and the therapeutic product is Retinoschisin (RS1); (25) Bardet-Biedl syndrome 1 and the therapeutic product is Bardet-Biedl Syndrome 1 (BBS1); (26) Bardet-Biedl syndrome 6 and the therapeutic product is McKusick-Kaufman Syndrome (MKKS); (27) Bardet-Biedl syndrome 10 and the therapeutic product is Bardet-Biedl Syndrome 10 (BBS10); (28) cone dystrophy and the therapeutic product is Guanylate Cyclase Activator 1A (GUCA1A); (29) optic atrophy and the therapeutic product is OPA1 Mitochondrial Dynamin Like GTPase (OPA1); (30) retinitis pigmentosa 1 and the therapeutic product is RP1 Axonemal Microtubule Associated (RP1); (31) retinitis pigmentosa 2 and the therapeutic product is RP2 Activator of ARL3 GTPase (RP2); (32) retinitis pigmentosa 7 and the therapeutic product is Peripherin 2 (PRPH2); (33) retinitis pigmentosa 11 and the therapeutic product is Pre-mRNA Processing Factor 31(PRPF31); (34) retinitis pigmentosa 13 and the therapeutic product is Pre-mRNA Processing Factor 8 (PRPF8); (35) retinitis pigmentosa 37 and the therapeutic product is Nuclear Receptor Subfamily 2 Group E Member 3 (NR2E3); (36) retinitis pigmentosa 38 and the therapeutic product is MER Proto-Oncogene, Tyrosine Kinase (MERTK); (37) retinitis pigmentosa 40 and the therapeutic product is Phosphodiesterase 6B (PDE6B); (38) retinitis pigmentosa 41 and the therapeutic product is Prominin 1 (PROM1); (39) retinitis pigmentosa 56 and the therapeutic product is Interphotoreceptor Matrix Proteoglycan 2 (IMPG2); (40) petinitis pigmentosa 62 and the therapeutic product is Male Germ Cell Associated Kinase (MAK); (41) retinitis pigmentosa 80 and the therapeutic product is Intraflagellar Transport 140 (IFT140); or (42) Best disease and the therapeutic product is Bestrophin 1 (BEST1).

In certain embodiments of the methods described herein, the pathology of the eye is associated with X-linked retinitis pigmentosa (XLRP) and the therapeutic product is Retinitis Pigmentosa GTPase Regulator (RPGR). In certain embodiments of any of the foregoing methods, the pathology of the eye is associated with achromatopsia and the therapeutic product is Cyclic Nucleotide Gated Channel Beta 3 (CNGB3); or achromatopsia (for example, a CNGA3-linked achromatopsia) and the therapeutic product is Cyclic Nucleotide Gated Channel Alpha 3 (CNGA3).

In certain embodiments of the method described herein, the recombinant viral vector further comprises a nucleotide sequence encoding a promoter or an enhancer-promoter, which nucleotide sequence encoding the promoter or enhancer-promoter is operably linked to the nucleotide sequence encoding the therapeutic product, and wherein the promoter or enhancer-promoter is a ubiquitous promoter/enhancer-promoter, eye-specific promoter/enhancer-promoter, or retina-specific promoter/enhancer-promoter.

In certain embodiments of the methods described herein, the recombinant viral vector further comprises a nucleotide sequence encoding a promoter or an enhancer-promoter, which nucleotide sequence encoding the promoter or enhancer-promoter is operably linked to the nucleotide sequence encoding the therapeutic product, and wherein the promoter or enhancer-promoter is: (1) a CAG promoter; (2) a CBA promoter; (3) a CMV promoter; (4) a 1.7-kb red cone opsin promoter (PR1.7 promoter); (5) a Rhodopsin Kinase (GRK1) photoreceptor-specific enhancer-promoter *(see, e.g.,* Young et al., 2003, Retinal Cell Biology; 44:4076-4085); (6) an hCARp promoter, which is a human cone arrestin promoter; (7) an hRKp, which is a rhodopsin kinase promoter; (8) a cone photoreceptor specific human arrestin 3 (ARR3) promoter; (9) a rhodopsin promoter; or (10) a U6 promoter (in particular when the therapeutic product is a small RNA such as shRNA and siRNA).

In certain embodiments of the methods described herein, the recombinant viral vector further comprises a nucleotide sequence encoding a cone-specific promoter, which nucleotide sequence encoding the cone-specific promoter is operably linked to the nucleotide sequence encoding the therapeutic product, and wherein: (1) the pathology of the eye is associated with red-green color blindness and the therapeutic product is L opsin (OPN1LW); (2) the pathology of the eye is associated with red-green color blindness and the therapeutic product is M opsin (OPN1MW); (3) the pathology of the eye is associated with blue cone monochromacy and the therapeutic product is M opsin (OPN1MW); (4) the pathology of the eye is associated with cone dystrophy and the therapeutic product is Guanylate Cyclase Activator 1A (GUCA1A); or (5) the pathology of the eye is associated with blue cone monochromacy (BCM) and the therapeutic product is L opsin (OPN1LW).

In certain embodiments of the methods described herein, the administering step delivers a therapeutically effective amount of the therapeutic product to the retina of said human subject.

In certain embodiments of the methods described herein, the therapeutically effective amount of the therapeutic product is produced by human retinal cells of said human subject.

In certain embodiments of the methods described herein, the therapeutically effective amount of the therapeutic product is produced by human photoreceptor cells, horizontal cells, bipolar cells, amacrine cells, retina ganglion cells, and/or retinal pigment epithelial cells in the external limiting membrane of said human subject.

In certain embodiments of the methods described herein, the human photoreceptor cells are cone cells and/or rod cells.

In certain embodiments of the methods described herein, the retina ganglion cells are midget cells, parasol cells, bistratified cells, giant retina ganglion cells, photosensitive ganglion cells, and/or Müller glia.

In certain embodiments of the methods described herein, the recombinant viral vector is an rAAV vector (e.g., an rAAV8, rAAV2, rAAV2tYF, or rAAV5 vector).

In certain embodiments of the methods described herein, wherein the recombinant viral vector is an rAAV8 vector.

In certain embodiments of the methods described herein, the method further comprises, after the administering step, a step of monitoring temperature of the surface of the eye using an infrared thermal camera. In a specific embodiment, the infrared thermal camera is an FLIR T530 infrared thermal camera. In a specific embodiment, the infrared thermal camera is an FLIR T420 infrared thermal camera. In a specific embodiment, the infrared thermal camera is an FLIR T440 infrared thermal camera. In a specific embodiment, the infrared thermal camera is an Fluke Ti400 infrared thermal camera. In a specific embodiment, the infrared thermal camera is an FLIRE60 infrared thermal camera. In a specific embodiment, the infrared resolution of the infrared thermal camera is equal to or greater than 75,000 pixels. In a specific embodiment, the thermal sensitivity of the infrared thermal camera is equal to or smaller than 0.05 °C at 30 °C. In a specific embodiment, the field of view (FOV) of the infrared thermal camera is equal to or lower than 25° × 25°.

In certain embodiments of the methods described herein, delivering to the eye comprises delivering to the retina, choroid, and/or vitreous humor of the eye.

In certain embodiments, the recombinant vector used for delivering the therapeutic product should have a tropism for cells of the eye, for example, human retinal cells, (*e.g.,* photoreceptor cells). Such vectors can include non-replicating recombinant adeno-associated virus vectors ("rAAV"), particularly those bearing an AAV8 capsid are preferred. However, other recombinant viral vectors may be used, including but not limited to recombinant lentiviral vectors, vaccinia viral vectors, or non-viral expression vectors referred to as "naked DNA" constructs. Preferably, the expression of therapeutic product should be controlled by appropriate expression control elements, for example, (1) a CAG promoter; (2) a CBA promoter; (3) a CMV promoter; (4) PR1.7 promoter; (5) a Rhodopsin Kinase (GRK1) photoreceptor-specific enhancer-promoter (6) an hCARp promoter; (7) an hRKp; (8) a cone photoreceptor specific human arrestin 3 (ARR3) promoter; (9) a rhodopsin promoter; or (10) a U6 promoter, and can include other expression control elements that enhance expression of the therapeutic product driven by the vector (*e.g*., introns such as the chicken β-actin intron, minute virus of mice (MVM) intron, human factor IX intron (*e.g.,* FIX truncated intron 1), β-globin splice donor/immunoglobulin heavy chain spice acceptor intron, adenovirus splice donor /immunoglobulin splice acceptor intron, SV40 late splice donor /splice acceptor (19S/16S) intron, and hybrid adenovirus splice donor/IgG splice acceptor intron and polyA signals such as the rabbit β-globin polyA signal, human growth hormone (hGH) polyA signal, SV40 late polyA signal, synthetic polyA (SPA) signal, and bovine growth hormone (bGH) polyA signal). *See, e.g.,* Powell and Rivera-Soto, 2015, Discov. Med., 19(102):49-57.

In certain embodiments of the method described herein, therapeutically effective doses of the recombinant vector are administered (1) to the subretinal space without vitrectomy (*e.g.,* via the suprachoroidal space or via peripheral injection), (2) to the suprachoroidal space, (3) to the outer space of the sclera (*i.e.,* juxtascleral administration), (4) to the subretinal space via vitrectomy, or (5) to the vitreous cavity, in a volume ranging from 50-100 µl or 100-500 µl, preferably 100-300 µl, and most preferably, 250 µl, depending on the administration method. In certain embodiments, therapeutically effective doses of the recombinant vector are administered suprachoroidally in a volume of 100 µl or less, for example, in a volume of 50-100 µl. In certain embodiments, therapeutically effective doses of the recombinant vector are administered to the outer surface of the sclera (*e.g.,* by a posterior juxtascleral depot procedure) in a volume of 500 µl or less, for example, in a volume of 10-20 µl, 20-50 µl, 50-100 µl, 100-200 µl, 200-300 µl, 300-400 µl, or 400-500 µl. In certain embodiments, therapeutically effective doses of the recombinant vector are administered to the subretinal space via peripheral injection, in a volume ranging from 50-100 µl or 100-500 µl, preferably 100-300 µl, and most preferably, 250 µl.

In certain embodiments, OptoKinetic Nystagmus (OKN) is assessed to measure visual acuity in patients. In certain embodiments, OKN can be performed using the methods and/or devices described and disclosed for example, in Cetinkaya et al., 2008, Eye, 22:77-81; Hyon et al., 2010, IOVS, 51(2): 752-757, Han et al., 2011, IOVS, 52(10): 7492-7497; Wester et al., 2007, IOVS, 48(10):4542-4548; Palmowski-Wolfe et al., 2019, J. AAPOS, 23(4): e49; Turuwhenua et al., Objective Assessment of Visual Performance Using Optokinetic Nystagmus in Young Children, October 2016, <anzctr.org.au/AnzctrAttachments/371914-OKN%?20protocol.pdf>; and Objective Acuity and Aier Eye Hospital Group Announce Strategic Cooperation Agreement, Cision PR Newswire, July 25, 2019, retrieved from the Internet <prnewswire.com/news-releases/objective-acuity-and-aier-eye-hospital-group-announce-a-strategic-cooperation-agreement-300891165.html>, each of which is incorporated by reference in its entirety.

Without being bound by theory, this visual acuity screening uses the principles of the OKN involuntary reflex to objectively assess whether a patient's eyes can follow a moving target. By using OKN, no verbal communication is needed between the tester and the patient. As such, OKN can be used to measure visual acuity in pre-verbal and/or non-verbal patients. In certain embodiments, OKN is used to measure visual acuity in patients that are 1 month old, 2 months old, 3 months old, 4 months old, 5 months old, 6 months old, 7 months old, 8 months old, 9 months old, 10 months old, 11 months old, 1 year old, 1.5 years old, 2 years old, 2.5 years old, 3 years old, 3.5 years old, 4 years old, 4.5 years old, or 5 years old. In certain embodiments, an iPad is used to measure visual acuity through detection of the OKN reflex when a patient is looking at movement on the iPad.

Without being bound by theory, this visual acuity screening uses the principles of the OKN involuntary reflex to objectively assess whether a patient's eyes can follow a moving target. By using OKN, no verbal communication is needed between the tester and the patient. As such, OKN can be used to measure visual acuity in pre-verbal and/or non-verbal patients. In certain embodiments, OKN is used to measure visual acuity in patients that are less than 1.5 months old, 2 months old, 3 months old, 4 months old, 5 months old, 6 months old, 7 months old, 8 months old, 9 months old, 10 months old, 11 months old, 1 year old, 1.5 years old, 2 years old, 2.5 years old, 3 years old, 3.5 years old, 4 years old, 4.5 years old, or 5 years old. In another specific embodiment, OKN is used to measure visual acuity in patients that are 1-2 months old, 2-3 months old, 3-4 months old, 4-5 months old, 5-6 months old, 6-7 months old, 7-8 months old, 8-9 months old, 9-10 months old, 10-11 months old, 11 months to 1 year old, 1-1.5 years old, 1.5-2 years old, 2-2.5 years old, 2.5-3 years old, 3-3.5 years old, 3.5-4 years old, 4-4.5 years old, or 4.5-5 years old. In another specific embodiment, OKN is used to measure visual acuity in patients that are 6 months to 5 years old. In certain embodiments, an iPad is used to measure visual acuity through detection of the OKN reflex when a patient is looking at movement on the iPad.

In certain embodiments, visual acuity is assessed in a patient presenting with Batten-CLN2-associated vision loss by measuring OKN before the patient has been treated with an AAV, preferably AAV8 or AAV9, encoding Tripeptidyl-Peptidase 1(TPP1). Specifically, the patient presenting with Batten-CLN2-associated vision loss is at the age, and/or within the age range described above. In certain embodiments, visual acuity assessed in a patient up to 5 years old presenting with Batten-CLN2-associated vision loss by measuring OKN after the patient has been treated with an AAV, preferably AAV8 or AAV9, encoding Tripeptidyl-Peptidase 1. In certain embodiments, a visual acuity assessment based on OKN determines that visual acuity does not decrease after treatment with AAV gene therapy. In certain embodiments, a visual acuity assessment based on OKN determines that visual acuity improves in a patient after treatment with AAV gene therapy by 5%, 10%, 15%, 20%, 25%, 30%, 35%, 40% 45%, 50%, 55%, 60% , 65%, 70%, 75%, 80%, 85%, 90%, 95% or 100%. In certain embodiments, a visual acuity assessment based on OKN determines that visual acuity does not further deteriorate in a patient after treatment with AAV gene therapy.

In certain embodiments, visual acuity is assessed in a patient presenting with Batten-CLN2-associated vision loss by measuring OKN before the patient has been treated with an AAV, preferably AAV8 or AAV9, encoding TPP1. Specifically, the patient presenting with Batten-CLN2-associated vision loss is at the age, and/or within the age range described above. In certain embodiments, visual acuity is assessed in a patient up to 5 years old presenting with Batten-CLN2-associated vision loss by measuring OKN after the patient has been treated with an AAV, preferably AAV8 or AAV9, encoding Tripeptidyl-Peptidase 1. In certain embodiments, a visual acuity assessment based on OKN determines that visual acuity does not decrease after treatment with AAV gene therapy. In certain embodiments, a visual acuity assessment based on OKN determines that visual acuity improves in a patient after treatment with AAV gene therapy by at least 5%, 10%, 15%, 20%, 25%, 30%, 35%, 40% 45%, 50%, 55%, 60% , 65%, 70%, 75%, 80%, 85%, 90%, 95% or at least 100%. In certain embodiments, a visual acuity assessment based on OKN determines that visual acuity does not further deteriorate in a patient after treatment with AAV gene therapy.

In certain embodiments, visual acuity is assessed in a patient presenting with Batten-CLN1-associated vision loss by measuring OKN before the patient has been treated with an AAV, preferably AAV8 or AAV9, encoding Palmitoyl-Protein Thioesterase 1 (PPT1). Specifically, the patient up to 5 years old presenting with Batten-CLN1-associated vision loss is at the age, and/or within the age range described above. In certain embodiments, visual acuity is assessed in a patient presenting with Batten-CLN1-associated vision loss by measuring OKN after the patient has been treated with an AAV, preferably AAV8 or AAV9, encoding PPT1. In certain embodiments, a visual acuity assessment based on OKN determines that visual acuity does not decrease after treatment with AAV gene therapy. In certain embodiments, a visual acuity assessment based on OKN determines that visual acuity improves in a patient after treatment with AAV gene therapy by 5%, 10%, 15%, 20%, 25%, 30%, 35%, 40% 45%, 50%, 55%, 60% , 65%, 70%, 75%, 80%, 85%, 90%, 95% or 100%. In certain embodiments, a visual acuity assessment based on OKN determines that visual acuity does not further deteriorate in a patient after treatment with AAV gene therapy.

In certain embodiments, visual acuity is assessed in a patient presenting with Batten-CLN1-associated vision loss by measuring OKN before the patient has been treated with an AAV, preferably AAV8 or AAV9, encoding PPT1. Specifically, the patient presenting with Batten-CLN1-associated vision loss is at the age, and/or within the age range described above. In certain embodiments, visual acuity is assessed in a patient up to 5 years old presenting with Batten-CLN1-associated vision loss by measuring OKN after the patient has been treated with an AAV, preferably AAV8 or AAV9, encoding PPT1. In certain embodiments, a visual acuity assessment based on OKN determines that visual acuity does not decrease after treatment with AAV gene therapy. In certain embodiments, a visual acuity assessment based on OKN determines that visual acuity improves in a patient after treatment with AAV gene therapy by at least 5%, 10%, 15%, 20%, 25%, 30%, 35%, 40% 45%, 50%, 55%, 60% , 65%, 70%, 75%, 80%, 85%, 90%, 95% or at least 100%. In certain embodiments, a visual acuity assessment based on OKN determines that visual acuity does not further deteriorate in a patient after treatment with AAV gene therapy.

In certain embodiments, visual acuity is assessed in a patient presenting with Batten-CLN3-associated vision loss by measuring OKN before the patient has been treated with an AAV, preferably AAV8 or AAV9, encoding Battenin (CLN3). Specifically, the patient presenting with Batten-CLN3-associated vision loss is at the age, and/or within the age range described above. In certain embodiments, visual acuity is assessed in a patient up to 5 years old presenting with Batten-CLN3-associated vision loss by measuring OKN after the patient has been treated with an AAV, preferably AAV8 or AAV9, encoding Battenin (CLN3). In certain embodiments, a visual acuity assessment based on OKN determines that visual acuity does not decrease after treatment with AAV gene therapy. In certain embodiments, a visual acuity assessment based on OKN determines that visual acuity improves in a patient after treatment with AAV gene therapy by 5%, 10%, 15%, 20%, 25%, 30%, 35%, 40% 45%, 50%, 55%, 60% , 65%, 70%, 75%, 80%, 85%, 90%, 95% or 100%. In certain embodiments, a visual acuity assessment based on OKN determines that visual acuity does not further deteriorate in a patient after treatment with AAV gene therapy.

In certain embodiments, visual acuity is assessed in a patient presenting with Batten-CLN3-associated vision loss by measuring OKN before the patient has been treated with an AAV, preferably AAV8 or AAV9, encoding Battenin (CLN3). Specifically, the patient presenting with Batten-CLN3-associated vision loss is at the age, and/or within the age range described above. In certain embodiments, visual acuity is assessed in a patient up to 5 years old presenting with Batten-CLN3-associated vision loss by measuring OKN after the patient has been treated with an AAV, preferably AAV8 or AAV9, encoding Battenin (CLN3). In certain embodiments, a visual acuity assessment based on OKN determines that visual acuity does not decrease after treatment with AAV gene therapy. In certain embodiments, a visual acuity assessment based on OKN determines that visual acuity improves in a patient after treatment with AAV gene therapy by at least 5%, 10%, 15%, 20%, 25%, 30%, 35%, 40% 45%, 50%, 55%, 60% , 65%, 70%, 75%, 80%, 85%, 90%, 95% or at least 100%. In certain embodiments, a visual acuity assessment based on OKN determines that visual acuity does not further deteriorate in a patient after treatment with AAV gene therapy.

In certain embodiments, visual acuity is assessed in a patient presenting with Batten-CLN6-associated vision loss by measuring OKN before the patient has been treated with an AAV, preferably AAV8 or AAV9, encoding CLN6 Transmembrane ER Protein (CLN6). Specifically, the patient up to 5 years old presenting with Batten-CLN6-associated vision loss is at the age, and/or within the age range described above. In certain embodiments, visual acuity is assessed in a patient presenting with Batten-CLN6-associated vision loss by measuring OKN after the patient has been treated with an AAV, preferably AAV8 or AAV9, encoding CLN6 Transmembrane ER Protein (CLN6). In certain embodiments, a visual acuity assessment based on OKN determines that visual acuity does not decrease after treatment with AAV gene therapy. In certain embodiments, a visual acuity assessment based on OKN determines that visual acuity improves in a patient after treatment with AAV gene therapy by 5%, 10%, 15%, 20%, 25%, 30%, 35%, 40% 45%, 50%, 55%, 60% , 65%, 70%, 75%, 80%, 85%, 90%, 95% or 100%. In certain embodiments, a visual acuity assessment based on OKN determines that visual acuity does not further deteriorate in a patient after treatment with AAV gene therapy.

In certain embodiments, visual acuity is assessed in a patient presenting with Batten-CLN6-associated vision loss by measuring OKN before the patient has been treated with an AAV, preferably AAV8 or AAV9, encoding CLN6 Transmembrane ER Protein (CLN6). Specifically, the patient up to 5 years old presenting with Batten-CLN6-associated vision loss is at the age, and/or within the age range described above. In certain embodiments, visual acuity is assessed in a patient presenting with Batten-CLN6-associated vision loss by measuring OKN after the patient has been treated with an AAV, preferably AAV8 or AAV9, encoding CLN6 Transmembrane ER Protein (CLN6). In certain embodiments, a visual acuity assessment based on OKN determines that visual acuity does not decrease after treatment with AAV gene therapy. In certain embodiments, a visual acuity assessment based on OKN determines that visual acuity improves in a patient after treatment with AAV gene therapy by at least 5%, 10%, 15%, 20%, 25%, 30%, 35%, 40% 45%, 50%, 55%, 60% , 65%, 70%, 75%, 80%, 85%, 90%, 95% or at least 100%. In certain embodiments, a visual acuity assessment based on OKN determines that visual acuity does not further deteriorate in a patient after treatment with AAV gene therapy.

In certain embodiments, visual acuity is assessed in a patient presenting with Batten-CLN7-associated vision loss by measuring OKN before the patient has been treated with an AAV, preferably AAV8 or AAV9, encoding Major Facilitator Superfamily Domain Containing 8 (MFSD8). Specifically, the patient up to 5 years old presenting with Batten-CLN7-associated vision loss is at the age, and/or within the age range described above. In certain embodiments, visual acuity is assessed in a patient presenting with Batten-CLN7-associated vision loss by measuring OKN after the patient has been treated with an AAV, preferably AAV8 or AAV9, encoding MFSD8. In certain embodiments, a visual acuity assessment based on OKN determines that visual acuity does not decrease after treatment with AAV gene therapy. In certain embodiments, a visual acuity assessment based on OKN determines that visual acuity improves in a patient after treatment with AAV gene therapy by 5%, 10%, 15%, 20%, 25%, 30%, 35%, 40% 45%, 50%, 55%, 60% , 65%, 70%, 75%, 80%, 85%, 90%, 95% or 100%. In certain embodiments, a visual acuity assessment based on OKN determines that visual acuity does not further deteriorate in a patient after treatment with AAV gene therapy.

In certain embodiments, visual acuity is assessed in a patient presenting with Batten-CLN7-associated vision loss by measuring OKN before the patient has been treated with an AAV, preferably AAV8 or AAV9, encoding MFSD8. Specifically, the patient presenting with Batten-CLN7-associated vision loss is at the age, and/or within the age range described above. In certain embodiments, visual acuity is assessed in a patient up to 5 years old presenting with Batten-CLN7-associated vision loss by measuring OKN after the patient has been treated with an AAV, preferably AAV8 or AAV9, encoding MFSD8. In certain embodiments, a visual acuity assessment based on OKN determines that visual acuity does not decrease after treatment with AAV gene therapy. In certain embodiments, a visual acuity assessment based on OKN determines that visual acuity improves in a patient after treatment with AAV gene therapy by at least 5%, 10%, 15%, 20%, 25%, 30%, 35%, 40% 45%, 50%, 55%, 60% , 65%, 70%, 75%, 80%, 85%, 90%, 95% or at least 100%. In certain embodiments, a visual acuity assessment based on OKN determines that visual acuity does not further deteriorate in a patient after treatment with AAV gene therapy.

Subretinal administration via vitrectomy is a surgical procedure performed by trained retinal surgeons that involves a vitrectomy with the subject under local anesthesia, and subretinal injection of the gene therapy into the retina (*see, e.g.,* Campochiaro et al., 2017, Hum Gen Ther 28(1):99-111, which is incorporated by reference herein in its entirety). Alternatively, subretinal administration can be performed without vitrectomy. In a specific embodiment, the subretinal administration without vitrectomy is performed via the suprachoroidal space using a suprachoroidal catheter which injects drug into the subretinal space, such as a subretinal drug delivery device that comprises a catheter which can be inserted and tunneled through the suprachoroidal space to the posterior pole, where a small needle injects into the subretinal space (*see, e.g.,* Baldassarre et al., 2017, Subretinal Delivery of Cells via the Suprachoroidal Space: Janssen Trial. In: Schwartz et al. (eds) Cellular Therapies for Retinal Disease, Springer, Cham; International Patent Application Publication No. WO 2016/040635 A1; each of which is incorporated by reference herein in its entirety). In another specific embodiment, the subretinal administration without vitrectomy is performed via peripheral injection. In other words, the recombinant vector can be delivered to the subretinal space by peripheral injection into the retina (*i.e.,* peripheral to the optic disc, fovea and macula located in the back of the eye) without performing a vitrectomy. This can be accomplished by transvitreal injection. Suprachoroidal administration procedures involve administration of a drug to the suprachoroidal space of the eye, and are normally performed using a suprachoroidal drug delivery device such as a microinjector with a microneedle (*see, e.g.,* Hariprasad, 2016, Retinal Physician 13: 20-23; Goldstein, 2014, Retina Today 9(5): 82-87; each of which is incorporated by reference herein in its entirety).

The suprachoroidal drug delivery devices that can be used to deposit the recombinant vector in the suprachoroidal space according to the invention described herein include, but are not limited to, suprachoroidal drug delivery devices manufactured by Clearside^{®} Biomedical, Inc. (*see,* for example, Hariprasad, 2016, Retinal Physician 13: 20-23) and MedOne suprachoroidal catheters. The subretinal drug delivery devices that can be used to deposit the recombinant vector in the subretinal space via the suprachoroidal space according to the invention described herein include, but are not limited to, subretinal drug delivery devices manufactured by Janssen Pharmaceuticals, Inc. (*see,* for example, International Patent Application Publication No. WO 2016/040635 A1) The subretinal drug delivery devices that can be used to deposit the recombinant vector in the subretinal space via the peripheral injection approach according to the invention described herein include, but are not limited to, sharp needles that can be inserted into the sclera via the superior or inferior side of the eye (*e.g.,* at the 2 or 10 o'clock position) and pass all the way through the vitreous to inject the retina on the other side, and trochars that can be inserted into the sclera to allow a subretinal cannula to be inserted into the eye and through the vitreous to the area of desired injection. In a specific embodiment, administration to the outer surface of the sclera is performed by a juxtascleral drug delivery device comprising a cannula whose tip can be inserted and kept in direct apposition to the scleral surface.

Suprachoroidal, subretinal, juxtascleral, intravitreal, subconjunctival, and/or intraretinal administration should result in delivery of the soluble therapeutic product to the retina, the vitreous humor, and/or the aqueous humor. The expression of the therapeutic product by retinal cells, *e.g.,* rod, cone, retinal pigment epithelial, horizontal, bipolar, amacrine, ganglion, and/or Müller cells, results in delivery and maintenance of the therapeutic product in the retina, the vitreous humor, and/or the aqueous humor. In specific embodiments, because the therapeutic product is continuously produced, maintenance of low concentrations can be effective. The concentration of the therapeutic product can be measured in patient samples of the vitreous humour and/or aqueous from the anterior chamber of the treated eye. Alternatively, vitreous humour concentrations can be estimated and/or monitored by measuring the patient's serum concentrations of the therapeutic product - the ratio of systemic to vitreal exposure to the therapeutic product is about 1:90,000. (*E.g.,* see, vitreous humor and serum concentrations of ranibizumab reported in Xu L, et al., 2013, Invest. Opthal. Vis. Sci. 54: 1616-1624, at p. 1621 and Table 5 at p. 1623, which is incorporated by reference herein in its entirety).

Pharmaceutical compositions suitable for suprachoroidal, subretinal, juxtascleral, intravitreal, subconjunctival, and/or intraretinal administration comprise a suspension of the recombinant vector in a formulation buffer comprising a physiologically compatible aqueous buffer, a surfactant and optional excipients.

The invention has several advantages over standard of care treatments that involve repeated ocular injections of high dose boluses of therapeutic products that dissipate over time resulting in peak and trough levels. Sustained expression of the therapeutic product, as opposed to injecting a therapeutic product repeatedly, allows for a more consistent levels of antibody to be present at the site of action, and is less risky and more convenient for patients, since fewer injections need to be made, resulting in fewer doctor visits. Consistent protein production may leads to better clinical outcomes as edema rebound in the retina is less likely to occur. Furthermore, in certain embodiments, therapeutic products expressed from recombinant vectors are post-translationally modified in a different manner than those that are directly injected because of the different microenvironment present during and after translation. Without being bound by any particular theory, this results in therapeutic products that have different diffusion, bioactivity, distribution, affinity, pharmacokinetic, and immunogenicity characteristics, such that the therapeutic products delivered to the site of action are "biobetters" in comparison with directly injected therapeutic products.

In addition, when the therapeutic products are antibodies, antibodies expressed from recombinant vectors *in vivo* are not likely to contain degradation products associated with antibodies produced by recombinant technologies, such as protein aggregation and protein oxidation. Aggregation is an issue associated with protein production and storage due to high protein concentration, surface interaction with manufacturing equipment and containers, and purification with certain buffer systems. These conditions, which promote aggregation, do not exist in antibody expression in gene therapy. Oxidation, such as methionine, tryptophan, and histidine oxidation, is also associated with protein production and storage, and is caused by stressed cell culture conditions, metal and air contact, and impurities in buffers and excipients. The proteins expressed from recombinant vectors in vivo may also oxidize in a stressed condition. However, humans, and many other organisms, are equipped with an antioxidation defense system, which not only reduces the oxidation stress, but sometimes also repairs and/or reverses the oxidation. Thus, proteins produced in vivo are not likely to be in an oxidized form. Both aggregation and oxidation could affect the potency, pharmacokinetics (clearance), and immunogenicity.

Unlike small molecule drugs, biologics usually comprise a mixture of many variants with different modifications or forms that have a different potency, pharmacokinetics, and safety profile. For therapeutic products that are post-translationally modified upon expression in cells of the eye, it is not essential that every molecule produced either in the gene therapy or protein therapy approach be fully post-translationally modified. Rather, the population of such therapeutic products that are produced should have sufficient post-translational modification (for example, from about 1% to about 10% of the population, from about 1% to about 20% of the population, from about 1% to about 50% of the population, or from about 10% to about 50% of the population) to demonstrate efficacy. The goal of gene therapy treatment provided herein is to slow or arrest the progression of the pathology of the eye, and to slow or prevent loss of vision with minimal intervention/invasive procedures. Efficacy may be monitored by measuring BCVA (Best-Corrected Visual Acuity), intraocular pressure, slit lamp biomicroscopy, indirect ophthalmoscopy, SD-OCT (SD-Optical Coherence Tomography), electroretinography (ERG). Signs of vision loss, infection, inflammation and other safety events, including retinal detachment may also be monitored. In certain embodiments, retinal thickness may be monitored to determine efficacy of the treatments provided herein. Without being bound by any particular theory, in certain embodiment, thickness of the retina may be used as a clinical readout, wherein the greater reduction in retinal thickness or the longer period of time before thickening of the retina, the more efficacious the treatment. Retinal thickness may be determined, for example, by SD-OCT. SD-OCT is a three-dimensional imaging technology which uses low-coherence interferometry to determine the echo time delay and magnitude of backscattered light reflected off an object of interest. OCT can be used to scan the layers of a tissue sample (*e.g.,* the retina) with 3 to 15 µm axial resolution, and SD-OCT improves axial resolution and scan speed over previous forms of the technology (Schuman, 2008, Trans. Am. Opthamol. Soc. 106:426-458). Retinal function may be determined, for example, by ERG. ERG is a non-invasive electrophysiologic test of retinal function, approved by the FDA for use in humans, which examines the light sensitive cells of the eye (the rods and cones), and their connecting ganglion cells, in particular, their response to a flash stimulation.

### 4.1 ILLUSTRATIVE EMBODIMENTS

### 4.1.1 Set 1

1. A method of subretinal administration without vitrectomy for treating a pathology of the eye, comprising administering to the subretinal space in the eye of a human subject in need of treatment a recombinant viral vector comprising a nucleotide sequence encoding a therapeutic product such that the therapeutic product is expressed and results in treatment of the pathology of the eye, wherein the method does not comprise performing a vitrectomy on the eye of said human patient.
2. The method of paragraph 1, wherein the administering step comprises administering to the subretinal space in the eye of said human subject the recombinant viral vector therapeutic product via the suprachoroidal space in the eye of said human subject.
3. The method of paragraph 2, wherein the administering step is by the use of a subretinal drug delivery device comprising a catheter that can be inserted and tunneled through the suprachoroidal space toward the posterior pole, where a small needle injects into the subretinal space.
4. The method of paragraph 3, wherein the administering step comprises inserting and tunneling the catheter of the subretinal drug delivery device through the suprachoroidal space.
5. A method of suprachoroidal administration for treating a pathology of the eye, comprising administering to the suprachoroidal space in the eye of a human subject in need of treatment a recombinant viral vector comprising a nucleotide sequence encoding a therapeutic product such that the therapeutic product is expressed and results in treatment of the pathology of the eye.
6. The method of paragraph 5, wherein the administering step is by injecting the recombinant viral vector into the suprachoroidal space using a suprachoroidal drug delivery device.
7. The method of paragraph 5 or 6, wherein the suprachoroidal drug delivery device is a microinjector.
8. A method of administration to the outer space of the sclera for treating a pathology of the eye, comprising administering to the outer surface of the sclera in the eye of a human subject in need of treatment a recombinant viral vector comprising a nucleotide sequence encoding a therapeutic product such that the therapeutic product is expressed and results in treatment of the pathology of the eye.
9. The method of paragraph 8, wherein the administering step is by the use of a juxtascleral drug delivery device that comprises a cannula whose tip can be inserted and kept in direct apposition to the scleral surface.
10. The method of paragraph 9, wherein the administering step comprises inserting and keeping the tip of the cannula in direct apposition to the scleral surface.
11. The method of any one of paragraphs 1-10, wherein the therapeutic product is not an anti-human vascular endothelial growth factor (hVEGF) antibody.
12. The method of any one of paragraphs 1-11, wherein the pathology of the eye is not associated with neovascular age-related macular degeneration (nAMD).
13. A method of subretinal administration accompanied by vitrectomy for treating a pathology of the eye, comprising administering to the subretinal space in the eye of a human subject in need of treatment a recombinant viral vector comprising a nucleotide sequence encoding a therapeutic product such that the therapeutic product is expressed and results in treatment of the pathology of the eye, wherein the method comprises performing a vitrectomy on the eye of said human patient, and wherein the therapeutic product is not anti-human vascular endothelial growth factor (hVEGF) antibody
14. The method of paragraph 13, wherein the vitrectomy is a partial vitrectomy.
15. A method of subretinal administration for treating a pathology of the eye, comprising administering to the subretinal space peripheral to the optic disc, fovea and macula located in the back of the eye of a human subject in need of treatment a recombinant viral vector comprising a nucleotide sequence encoding a therapeutic product such that the therapeutic product is expressed and results in treatment of the pathology of the eye, wherein the method does not comprise performing a vitrectomy on the eye of said human patient.
16. The method of paragraph 15, wherein the administering step is by transvitreal injection.
17. The method of paragraph 16, wherein the transvitreal injection comprises inserting a sharp needle into the sclera via the superior or inferior side of the eye and passing the sharp needle all the way through the vitreous to inject the recombinant viral vector to the subretinal space on the other side.
18. The method of paragraph 16, wherein the transvitreal injection comprises inserting a trochar into the sclera and inserting a cannula through the trochar and through the vitreous to inject the recombinant viral vector to the subretinal space on the other side
19. The method of any one of paragraphs 15-18, wherein the therapeutic product is an anti-hVEGF antibody.
20. The method of paragraph 19, wherein the anti-hVEGF antibody is an anti-hVEGF antigen-binding fragment.
21. The method of paragraph 20, wherein the anti-hVEGF antigen-binding fragment is a Fab, F(ab')₂, or single chain variable fragment (scFv).
22. The method of any one of paragraphs 19-21, wherein the anti-hVEGF antibody comprises a heavy chain comprising the amino acid sequence of SEQ ID NO:2 or SEQ ID NO:4, and a light chain comprising the amino acid sequence of SEQ ID NO:1, or SEQ ID NO:3.
23. The method of any one of paragraphs 19-21, wherein the anti-hVEGF antibody comprises light chain CDRs 1-3 of SEQ ID NOs:14-16 and heavy chain CDRs 1-3 of SEQ ID NOs:17-19 or SEQ ID NOs:20, 18, and 21.
24. The method of any one of paragraphs 19-23, wherein the pathology of the eye is associated with nAMD, dry age-related macular degeneration (dry AMD), retinal vein occlusion (RVO), diabetic macular edema (DME), or diabetic retinopathy (DR).
25. The method of any one of paragraphs 19-23, wherein the pathology of the eye is associated with nAMD.
26. The method of any one of paragraphs 1-11 and 13-18, wherein:
   (1) the pathology of the eye is associated with Batten-CLN1 and the therapeutic product is Palmitoyl-Protein Thioesterase 1 (PPT1);
   (2) the pathology of the eye is associated with Batten-CLN2 and the therapeutic product is Tripeptidyl-Peptidase 1 (TPP1);
   (3) the pathology of the eye is associated with Batten-CLN3 and the therapeutic product is Battenin (CLN3);
   (4) the pathology of the eye is associated with Batten-CLN6 and the therapeutic product is CLN6 Transmembrane ER Protein (CLN6);
   (5) the pathology of the eye is associated with Batten-CLN7 and the therapeutic product is Major Facilitator Superfamily Domain Containing 8 (MFSD8);
   (6) the pathology of the eye is associated with Usher's-Type 1 and the therapeutic product is Myosin VIIA (MYO7A);
   (7) the pathology of the eye is associated with Usher's-Type 1 and the therapeutic product is Cadherin Related 23 (CDH23);
   (8) the pathology of the eye is associated with Usher's-Type 2 and the therapeutic product is Protocadherin Related 15 (PCDH15);
   (9) the pathology of the eye is associated with Usher's-Type 2 and the therapeutic product is Usherin (USH2A);
   (10) the pathology of the eye is associated with Usher's-Type 3 and the therapeutic product is Clarin 1 (CLRN1);
   (11) the pathology of the eye is associated with Stargardt's and the therapeutic product is ATP Binding Cassette Subfamily A Member 4 (ABCA4);
   (12) the pathology of the eye is associated with Stargardt's and the therapeutic product is ELOVL Fatty Acid Elongase 4 (ELOVL4);
   (13) the pathology of the eye is associated with uveitis and the therapeutic product is an anti-Interleukin 6 (IL6) monoclonal antibody;
   (14) the pathology of the eye is associated with uveitis and the therapeutic product is an anti-TNF-alpha (TNF) monoclonal antibody;
   (15) the pathology of the eye is associated with diabetic macular edema (DME) and the therapeutic product is an anti-IL6 monoclonal antibody;
   (16) the pathology of the eye is associated with red-green color blindness and the therapeutic product is L opsin (OPN1LW);
   (17) the pathology of the eye is associated with red-green color blindness and the therapeutic product is M opsin (OPN1MW);
   (18) the pathology of the eye is associated with blue cone monochromacy and the therapeutic product is M opsin (OPN1MW);
   (19) the pathology of the eye is associated with Leber congenital amaurosis-1 (LCA 1) and the therapeutic product is Guanylate Cyclase 2D, Retinal (GUCY2D);
   (20) the pathology of the eye is associated with Leber congenital amaurosis-2 (LCA 2) and the therapeutic product is Retinoid Isomerohydrolase RPE65 (RPE65);
   (21) the pathology of the eye is associated with LCA 3 and the therapeutic product is Spermatogenesis Associated 7 (SPATA7);
   (22) the pathology of the eye is associated with Leber congenital amaurosis-4 (LCA 4) and the therapeutic product is Aryl Hydrocarbon Receptor Interacting Protein Like 1 (AIPL1);
   (23) the pathology of the eye is associated with Leber congenital amaurosis-5 (LCA 5) and the therapeutic product is Lebercilin (LCA5);
   (24) the pathology of the eye is associated with Leber congenital amaurosis-6 (LCA 6) and the therapeutic product is RPGR Interacting Protein 1 (RPGRIP1);
   (25) the pathology of the eye is associated with Leber congenital amaurosis-7 (LCA 7) and the therapeutic product is Cone-Rod Homeobox (CRX);
   (26) the pathology of the eye is associated with Leber congenital amaurosis-8 (LCA 8) and the therapeutic product is Crumbs Cell Polarity Complex Component 1 (CRB1);
   (27) the pathology of the eye is associated with Leber congenital amaurosis-9 (LCA 9) and the therapeutic product is Nicotinamide Nucleotide Adenylyltransferase 1 (NMNAT1);
   (28) the pathology of the eye is associated with Leber congenital amaurosis-10 (LCA 10) and the therapeutic product is Centrosomal Protein 290 (CEP290);
   (29) the pathology of the eye is associated with Leber congenital amaurosis-11 (LCA 11) and the therapeutic product is Inosine Monophosphate Dehydrogenase 1 (IMPDH1);
   (30) the pathology of the eye is associated with Leber congenital amaurosis-12 (LCA 12) and the therapeutic product is Retinal Degeneration 3, GUCY2D regulator (RD3) ;
   (31) the pathology of the eye is associated with Leber congenital amaurosis-13 (LCA 13) and the therapeutic product is Retinol Dehydrogenase 12 (RDH12);
   (32) the pathology of the eye is associated with Leber congenital amaurosis-14 (LCA 14) and the therapeutic product is Lecithin Retinol Acyltransferase (LRAT);
   (33) the pathology of the eye is associated with Leber congenital amaurosis-15 (LCA 15) and the therapeutic product is Tubby Like Protein 1 (TULP1);
   (34) the pathology of the eye is associated with Leber congenital amaurosis-16 (LCA 16) and the therapeutic product is Potassium Voltage-Gated Channel Subfamily J Member 13 (KCNJ13);
   (35) the pathology of the eye is associated with Leber's hereditary optic neuropathy (LHON) and the therapeutic product is Mitochondrially Encoded NADH Dehydrogenase 1 (MT-ND1);
   (36) the pathology of the eye is associated with LHON and the therapeutic product is Mitochondrially Encoded NADH Dehydrogenase 4 (MT-ND4);
   (37) the pathology of the eye is associated with LHON and the therapeutic product is Mitochondrially Encoded NADH Dehydrogenase 6 (MT-ND6);
   (38) the pathology of the eye is associated with neuromyelitis optica (NMO) and the therapeutic product is an anti-complement C5 monoclonal antibody;
   (39) the pathology of the eye is associated with NMO and the therapeutic product is an anti-IL6 monoclonal antibody;
   (40) the pathology of the eye is associated with uveitis and the therapeutic product is an anti-complement C5 monoclonal antibody;
   (41) the pathology of the eye is associated with uveitis and the therapeutic product is Angiotensin I Converting Enzyme (ACE);
   (42) the pathology of the eye is associated with uveitis and the therapeutic product is Interleukin 10 (IL10);
   (43) the pathology of the eye is associated with uveitis and the therapeutic product is an anti-TNF monoclonal antibody;
   (44) the pathology of the eye is associated with choroideremia and the therapeutic product is Rab Escort Protein 1 (CHM);
   (45) the pathology of the eye is associated with X-linked retinoschisis (XLRS) and the therapeutic product is Retinoschisin (RS1);
   (46) the pathology of the eye is associated with Bardet-Biedl syndrome 1 and the therapeutic product is Bardet-Biedl Syndrome 1 (BBS1);
   (47) the pathology of the eye is associated with Bardet-Biedl syndrome 2 and the therapeutic product is Bardet-Biedl Syndrome 2 (BBS2);
   (48) the pathology of the eye is associated with Bardet-Biedl syndrome 3 and the therapeutic product is ADP Ribosylation Factor Like GTPase 6 (ARL6);
   (49) the pathology of the eye is associated with Bardet-Biedl syndrome 4 and the therapeutic product is Bardet-Biedl Syndrome 4 (BBS4);
   (50) the pathology of the eye is associated with Bardet-Biedl syndrome 5 and the therapeutic product is Bardet-Biedl Syndrome 5 (BBS5);
   (51) the pathology of the eye is associated with Bardet-Biedl syndrome 6 and the therapeutic product is McKusick-Kaufman Syndrome (MKKS);
   (52) the pathology of the eye is associated with Bardet-Biedl syndrome 7 and the therapeutic product is Bardet-Biedl Syndrome 7 (BBS7);
   (53) the pathology of the eye is associated with Bardet-Biedl syndrome 8 and the therapeutic product is Tetratricopeptide Repeat Domain 8 (TTC8);
   (54) the pathology of the eye is associated with Bardet-Biedl syndrome 9 and the therapeutic product is Bardet-Biedl Syndrome 9 (BBS9);
   (55) the pathology of the eye is associated with Bardet-Biedl syndrome 10 and the therapeutic product is Bardet-Biedl Syndrome 10 (BBS10);
   (56) the pathology of the eye is associated with Bardet-Biedl syndrome 11 and the therapeutic product is Tripartite Motif Containing 32 (TRIM32);
   (57) the pathology of the eye is associated with Bardet-Biedl syndrome 12 and the therapeutic product is Bardet-Biedl Syndrome 12 (BBS12);
   (58) the pathology of the eye is associated with Bardet-Biedl syndrome 13 and the therapeutic product is MKS Transition Zone Complex Subunit 1 (MKS1);
   (59) the pathology of the eye is associated with Bardet-Biedl syndrome 14 and the therapeutic product is Centrosomal Protein 290 (CEP290);
   (60) the pathology of the eye is associated with Bardet-Biedl syndrome 15 and the therapeutic product is WD Repeat Containing Planar Cell Polarity Effector (WDPCP);
   (61) the pathology of the eye is associated with Bardet-Biedl syndrome 16 and the therapeutic product is Serologically Defined Colon Cancer Antigen 8 (SDCCAG8);
   (62) the pathology of the eye is associated with Bardet-Biedl syndrome 17 and the therapeutic product is Leucine Zipper Transcription Factor Like 1 (LZTFL1);
   (63) the pathology of the eye is associated with Bardet-Biedl syndrome 18 and the therapeutic product is BBSome Interacting Protein 1 (BBIP1);
   (64) the pathology of the eye is associated with Bardet-Biedl syndrome 19 and the therapeutic product is Intraflagellar Transport 27 (IFT27);
   (65) the pathology of the eye is associated with cone dystrophy and the therapeutic product is Guanylate Cyclase Activator 1A (GUCA1A);
   (66) the pathology of the eye is associated with optic atrophy and the therapeutic product is OPA1 Mitochondrial Dynamin Like GTPase (OPA1);
   (67) the pathology of the eye is associated with retinitis pigmentosa 1 and the therapeutic product is RP1 Axonemal Microtubule Associated (RP1);
   (68) the pathology of the eye is associated with retinitis pigmentosa 2 and the therapeutic product is RP2 Activator of ARL3 GTPase (RP2);
   (69) the pathology of the eye is associated with retinitis pigmentosa 7 and the therapeutic product is Peripherin 2 (PRPH2);
   (70) the pathology of the eye is associated with retinitis pigmentosa 11 and the therapeutic product is Pre-mRNA Processing Factor 31(PRPF31);
   (71) the pathology of the eye is associated with retinitis pigmentosa 12 and the therapeutic product is Crumbs Cell Polarity Complex Component 1 (CRB1);
   (72) the pathology of the eye is associated with retinitis pigmentosa 13 and the therapeutic product is Pre-mRNA Processing Factor 8 (PRPF8);
   (73) the pathology of the eye is associated with retinitis pigmentosa 25 and the therapeutic product is Eyes Shut Homolog (EYS);
   (74) the pathology of the eye is associated with retinitis pigmentosa 28 and the therapeutic product is FAM161 Centrosomal Protein A (FAM161A);
   (75) the pathology of the eye is associated with retinitis pigmentosa 37 and the therapeutic product is Nuclear Receptor Subfamily 2 Group E Member 3 (NR2E3);
   (76) the pathology of the eye is associated with retinitis pigmentosa 38 and the therapeutic product is MER Proto-Oncogene, Tyrosine Kinase (MERTK);
   (77) the pathology of the eye is associated with retinitis pigmentosa 40 and the therapeutic product is Phosphodiesterase 6B (PDE6B);
   (78) the pathology of the eye is associated with retinitis pigmentosa 41 and the therapeutic product is Prominin 1 (PROM1);
   (79) the pathology of the eye is associated with retinitis pigmentosa 43 and the therapeutic product is Phosphodiesterase 6A (PDE6A);
   (80) the pathology of the eye is associated with retinitis pigmentosa 56 and the therapeutic product is Interphotoreceptor Matrix Proteoglycan 2 (IMPG2);
   (81) the pathology of the eye is associated with petinitis pigmentosa 62 and the therapeutic product is Male Germ Cell Associated Kinase (MAK);
   (82) the pathology of the eye is associated with retinitis pigmentosa 80 and the therapeutic product is Intraflagellar Transport 140 (IFT140);
   (83) the pathology of the eye is associated with dry AMD and the therapeutic product is an anti-complement C5 monoclonal antibody;
   (84) the pathology of the eye is associated with dry AMD and the therapeutic product is an anti-membrane attack complex (MAC) monoclonal antibody;
   (85) the pathology of the eye is associated with dry AMD and the therapeutic product is HtrA Serine Peptidase 1 (HTRA1);
   (86) the pathology of the eye is associated with Best disease and the therapeutic product is Bestrophin 1 (BEST1);
   (87) the pathology of the eye is associated with dry AMD and the therapeutic product is a complement factor B antisense oligonucleotide;
   (88) the pathology of the eye is associated with dry AMD and the therapeutic product is an anti-beta-amyloid monoclonal antibody;
   (89) the pathology of the eye is associated with dry AMD and the therapeutic product is CD59 glycoprotein (CD59);
   (90) the pathology of the eye is associated with dry AMD and the therapeutic product is Channelrhodopsin-1 (ChR1);
   (91) the pathology of the eye is associated with dry AMD and the therapeutic product is Channelrhodopsin-2 (ChR2), the light-sensitive protein discovered in *Chlamydomonas reinhardtii;*
   (92) the pathology of the eye is associated with dry AMD and the therapeutic product is an anti-complement factor C5a aptamer;
   (93) the pathology of the eye is associated with dry AMD and the therapeutic product is anti-complement factor D monoclonal antibody;
   (94) the pathology of the eye is associated with age-related retinal ganglion cell (RGC) degeneration and the therapeutic product is DnaJ heat shock protein family (Hsp40) member C3 (DNAJC3);
   (95) the pathology of the eye is associated with blue cone monochromacy (BCM) and the therapeutic product is L opsin (OPN1LW);
   (96) the pathology of the eye is associated with glaucoma and the therapeutic product is beta-2 adrenoceptor siRNA;
   (97) the pathology of the eye is associated with glaucoma and the therapeutic product is Caspase-2 (CASP2);
   (98) the pathology of the eye is associated with glaucoma and the therapeutic product is Insulin Receptor Substrate 1 (IRS1);
   (99) the pathology of the eye is associated with glaucoma and the therapeutic product is HIF-1 Responsive Protein RTP801 (RTP801);
   (100) the pathology of the eye is associated with glaucoma and the therapeutic product is Transforming Growth Factor Beta 2 (TGFB2);
   (101) the pathology of the eye is associated with glaucoma and the therapeutic product is Brain Derived Neurotrophic Factor (BDNF);
   (102) the pathology of the eye is associated with glaucoma and the therapeutic product is Ciliary Neurotrophic Factor (CNTF);
   (103) the pathology of the eye is associated with glaucoma and the therapeutic product is Prostaglandin-Endoperoxide Synthase 2 (PTGS2);
   (104) the pathology of the eye is associated with glaucoma and the therapeutic product is Prostaglandin F Receptor (PTGFR);
   (105) the pathology of the eye is associated with glaucoma and the therapeutic product is a hyaluronidase;
   (106) the pathology of the eye is associated with glaucoma and the therapeutic product is Pigment Epithelium-Derived Factor (PEDF);
   (107) the pathology of the eye is associated with glaucoma and the therapeutic product is Vascular Endothelial Growth Factor (VEGF);
   (108) the pathology of the eye is associated with glaucoma and the therapeutic product is Placental Growth Factor (PGF);
   (109) the pathology of the eye is associated with glaucoma and the therapeutic product is Myocilin (MYOC);
   (110) the pathology of the eye is associated with NMO and the therapeutic product is an anti-complement C5 monoclonal antibody;
   (111) the pathology of the eye is associated with NMO and the therapeutic product is C-C Motif Chemokine Receptor 5 (CCR5) siRNA;
   (112) the pathology of the eye is associated with NMO and the therapeutic product is an anti-CD19 monoclonal antibody;
   (113) the pathology of the eye is associated with retinitis pigmentosa that is associated with rhodopsin mutations and the therapeutic product is Channelrhodopsin-1 (ChR1);
   (114) the pathology of the eye is associated with retinitis pigmentosa that is associated with rhodopsin mutations and the therapeutic product is Channelrhodopsin-2 (ChR2);
   (115) the pathology of the eye is associated with retinitis pigmentosa and the therapeutic product is Ciliary Neurotrophic Factor (CNTF);
   (116) the pathology of the eye is associated with autosomal recessive retinitis pigmentosa and the therapeutic product is Crumbs Cell Polarity Complex Component 1 (CRB1);
   (117) the pathology of the eye is associated with autosomal recessive retinitis pigmentosa and the therapeutic product is Crumbs Cell Polarity Complex Component 2 (CRB2);
   (118) the pathology of the eye is associated with retinitis pigmentosa and the therapeutic product is Histone Deacetylase 4 (HDAC4);
   (119) the pathology of the eye is associated with retinitis pigmentosa and the therapeutic product is Rhodopsin (RHO);
   (120) the pathology of the eye is associated with retinitis pigmentosa and the therapeutic product is Nerve Growth Factor (NGF);
   (121) the pathology of the eye is associated with retinitis pigmentosa and the therapeutic product is Nuclear Factor, Erythroid 2 Like 2 (NRF2);
   (122) the pathology of the eye is associated with retinitis pigmentosa and the therapeutic product is Pigment Epithelium-Derived Factor (PEDF);
   (123) the pathology of the eye is associated with retinitis pigmentosa and the therapeutic product is Glutathione S-Transferase PI 1 (GSTP1);
   (124) the pathology of the eye is associated with retinitis pigmentosa and the therapeutic product is Rod-Derived Cone Viability Factor (RDCVF);
   (125) the pathology of the eye is associated with retinitis pigmentosa and the therapeutic product is Rhodopsin (RHO);
   (126) the pathology of the eye is associated with retinitis pigmentosa and the therapeutic product is Retinaldehyde Binding Protein 1 (RLBP1);
   (127) the pathology of the eye is associated with Stargardt's disease and the therapeutic product is an anti-complement C5 aptamer;
   (128) the pathology of the eye is associated with uveitis and the therapeutic product is Double Homeobox 4 (DUX4);
   (129) the pathology of the eye is associated with uveitis and the therapeutic product is NLR Family Pyrin Domain Containing 3 (NLRP3);
   (130) the pathology of the eye is associated with uveitis and the therapeutic product is Spleen Associated Tyrosine Kinase (SYK);
   (131) the pathology of the eye is associated with uveitis and the therapeutic product is Adrenocorticotropic Hormone (ACTH);
   (132) the pathology of the eye is associated with uveitis and the therapeutic product is Caspase 1 (CASP1);
   (133) the pathology of the eye is associated with uveitis and the therapeutic product is anti-CD59 monoclonal antibody;
   (134) the pathology of the eye is associated with uveitis and the therapeutic product is an anti-complement C5 aptamer;
   (135) the pathology of the eye is associated with corneal neovascularization and the therapeutic product is Insulin Receptor Substrate 1 (IRS 1);
   (136) the pathology of the eye is associated with corneal neovascularization and the therapeutic product is NOTCH Regulated Ankyrin Repeat Protein (NRARP);
   (137) the pathology of the eye is associated with diabetic retinopathy and the therapeutic product is NOTCH Regulated Ankyrin Repeat Protein (NRARP);
   (138) the pathology of the eye is associated with diabetic retinopathy and the therapeutic product is Alpha-2-Antiplasmin (A2AP);
   (139) the pathology of the eye is associated with diabetic retinopathy and the therapeutic product is Plasminogen (PLG);
   (140) the pathology of the eye is associated with diabetic retinopathy and the therapeutic product is a growth hormone;
   (141) the pathology of the eye is associated with diabetic retinopathy and the therapeutic product is Insulin Like Growth Factor 1 (IGF1);
   (142) the pathology of the eye is associated with diabetic retinopathy and the therapeutic product is Interleukin 1 Beta (IL1B).
   (143) the pathology of the eye is associated with diabetic retinopathy and the therapeutic product is Angiotensin I Converting Enzyme 2 (ACE2);
   (144) the pathology of the eye is associated with diabetic retinopathy and the therapeutic product is IRS1;
   (145) the pathology of the eye is associated with diabetic retinopathy and the therapeutic product is an anti-integrin oligopeptide;
   (146) the pathology of the eye is associated with diabetic retinopathy and the therapeutic product is an anti-Placental Growth Factor (PGF) monoclonal antibody;
   (147) the pathology of the eye is associated with Graves' ophthalmopathy and the therapeutic product is an anti-CD40 monoclonal antibody;
   (148) the pathology of the eye is associated with Graves' ophthalmopathy and the therapeutic product is an anti-Insulin-Like Growth Factor 1 Receptor (IGF1R) monoclonal antibody;
   (149) the pathology of the eye is associated with Graves' ophthalmopathy and the therapeutic product is an anti-Insulin-Like Growth Factor 2 Receptor (IGF2R) monoclonal antibody;
   (150) the pathology of the eye is associated with DME and the therapeutic product is an anti-integrin oligopeptide;
   (151) the pathology of the eye is associated with DME and the therapeutic product is an anti-Placental Growth Factor (PGF) monoclonal antibody;
   (152) the pathology of the eye is associated with DME and the therapeutic product is RTP801 siRNA;
   (153) the pathology of the eye is associated with multiple sclerosis (MS)-associated vision loss and the therapeutic product is ND1;
   (154) the pathology of the eye is associated with myopia and the therapeutic product is Matrix Metalloproteinase 2 (MMP2) RNAi;
   (155) the pathology of the eye is associated with X-linked recessive ocular albinism and the therapeutic product is G-Protein Coupled Receptor 143 (GPR143);
   (156) the pathology of the eye is associated with oculocutaneous albinism type 1 and the therapeutic product is Tyrosinase (TYR);
   (157) the pathology of the eye is associated with optic neuritis and the therapeutic product is Caspase 2 (CASP2);
   (158) the pathology of the eye is associated with optic neuritis and the therapeutic product is an anti-Leucine Rich Repeat And Ig Domain Containing Protein 1 (LINGO1) monoclonal antibody;or
   (159) the pathology of the eye is associated with polypoidal choroidal vasculopathy and the therapeutic product is an anti-complement C5 aptamer.
27. The method of any one of paragraphs 1-11 and 15-18, wherein:
   (1) the pathology of the eye is associated with X-linked retinitis pigmentosa (XLRP) and the therapeutic product is Retinitis Pigmentosa GTPase Regulator (RPGR);
   (2) the pathology of the eye is associated with achromatopsia (ACHM) and the therapeutic product is Cyclic Nucleotide Gated Channel Beta 3 (CNGB3);
   (3) the pathology of the eye is associated with achromatopsia and the therapeutic product is Cyclic Nucleotide Gated Channel Alpha 3 (CNGA3); or
   (4) the pathology of the eye is associated with biallelic RPE65 mutation-associated retinal dystrophy and the therapeutic product is Retinoid Isomerohydrolase RPE65 (RPE65).
28. The method of any one of paragraphs 1-11 and 13-18, wherein:
   (1) the pathology of the eye is associated with Batten-CLN1 and the therapeutic product is Palmitoyl-Protein Thioesterase 1 (PPT1);
   (2) the pathology of the eye is associated with Batten-CLN2 and the therapeutic product is Tripeptidyl-Peptidase 1 (TPP1);
   (3) the pathology of the eye is associated with Batten-CLN3 and the therapeutic product is Battenin (CLN3);
   (4) the pathology of the eye is associated with uveitis and the therapeutic product is an anti-Interleukin 6 (IL6) monoclonal antibody;
   (5) the pathology of the eye is associated with uveitis and the therapeutic product is an anti-TNF-alpha (TNF) monoclonal antibody;
   (6) the pathology of the eye is associated with diabetic macular edema (DME) and the therapeutic product is an anti-IL6 monoclonal antibody;
   (7) the pathology of the eye is associated with red-green color blindness and the therapeutic product is L opsin (OPN1LW);
   (8) the pathology of the eye is associated with red-green color blindness and the therapeutic product is M opsin (OPN1MW);
   (9) the pathology of the eye is associated with blue cone monochromacy and the therapeutic product is M opsin (OPN1MW);
   (10) the pathology of the eye is associated with Leber congenital amaurosis-1 (LCA 1) and the therapeutic product is Guanylate Cyclase 2D, Retinal (GUCY2D);
   (11) the pathology of the eye is associated with Leber congenital amaurosis-2 (LCA 2) and the therapeutic product is Retinoid Isomerohydrolase RPE65 (RPE65);
   (12) the pathology of the eye is associated with Leber congenital amaurosis-7 (LCA 7) and the therapeutic product is Cone-Rod Homeobox (CRX);
   (13) the pathology of the eye is associated with Leber congenital amaurosis-11 (LCA 11) and the therapeutic product is Inosine Monophosphate Dehydrogenase 1 (IMPDH1);
   (14) the pathology of the eye is associated with Leber congenital amaurosis-12 (LCA 12) and the therapeutic product is Retinal Degeneration 3, GUCY2D regulator (RD3);
   (15) the pathology of the eye is associated with Leber congenital amaurosis-13 (LCA 13) and the therapeutic product is Retinol Dehydrogenase 12 (RDH12);
   (16) the pathology of the eye is associated with Leber congenital amaurosis-15 (LCA 15) and the therapeutic product is Tubby Like Protein 1 (TULP1);
   (17) the pathology of the eye is associated with Leber congenital amaurosis-16 (LCA 16) and the therapeutic product is Potassium Voltage-Gated Channel Subfamily J Member 13 (KCNJ13);
   (18) the pathology of the eye is associated with Leber's hereditary optic neuropathy (LHON) and the therapeutic product is Mitochondrially Encoded NADH Dehydrogenase 1 (MT-ND1);
   (19) the pathology of the eye is associated with LHON and the therapeutic product is Mitochondrially Encoded NADH Dehydrogenase 4 (MT-ND4);
   (20) the pathology of the eye is associated with LHON and the therapeutic product is Mitochondrially Encoded NADH Dehydrogenase 6 (MT-ND6);
   (21) the pathology of the eye is associated with neuromyelitis optica (NMO) and the therapeutic product is an anti-complement C5 monoclonal antibody;
   (22) the pathology of the eye is associated with NMO and the therapeutic product is an anti-IL6 monoclonal antibody;
   (23) the pathology of the eye is associated with uveitis and the therapeutic product is an anti-complement C5 monoclonal antibody;
   (24) the pathology of the eye is associated with uveitis and the therapeutic product is Angiotensin I Converting Enzyme (ACE);
   (25) the pathology of the eye is associated with uveitis and the therapeutic product is Interleukin 10 (IL10);
   (26) the pathology of the eye is associated with uveitis and the therapeutic product is an anti-TNF monoclonal antibody;
   (27) the pathology of the eye is associated with X-linked retinoschisis (XLRS) and the therapeutic product is Retinoschisin (RS1);
   (28) the pathology of the eye is associated with Bardet-Biedl syndrome 1 and the therapeutic product is Bardet-Biedl Syndrome 1 (BBS1);
   (29) the pathology of the eye is associated with Bardet-Biedl syndrome 3 and the therapeutic product is ADP Ribosylation Factor Like GTPase 6 (ARL6);
   (30) the pathology of the eye is associated with Bardet-Biedl syndrome 5 and the therapeutic product is Bardet-Biedl Syndrome 5 (BBS5);
   (31) the pathology of the eye is associated with Bardet-Biedl syndrome 6 and the therapeutic product is McKusick-Kaufman Syndrome (MKKS);
   (32) the pathology of the eye is associated with Bardet-Biedl syndrome 10 and the therapeutic product is Bardet-Biedl Syndrome 10 (BBS10);
   (33) the pathology of the eye is associated with Bardet-Biedl syndrome 11 and the therapeutic product is Tripartite Motif Containing 32 (TRIM32);
   (34) the pathology of the eye is associated with Bardet-Biedl syndrome 13 and the therapeutic product is MKS Transition Zone Complex Subunit 1 (MKS 1);
   (35) the pathology of the eye is associated with Bardet-Biedl syndrome 18 and the therapeutic product is BBSome Interacting Protein 1 (BBIP1);
   (36) the pathology of the eye is associated with Bardet-Biedl syndrome 19 and the therapeutic product is Intraflagellar Transport 27 (IFT27);
   (37) the pathology of the eye is associated with cone dystrophy and the therapeutic product is Guanylate Cyclase Activator 1A (GUCA1A);
   (38) the pathology of the eye is associated with retinitis pigmentosa 13 and the therapeutic product is Pre-mRNA Processing Factor 8 (PRPF8);
   (39) the pathology of the eye is associated with retinitis pigmentosa 37 and the therapeutic product is Nuclear Receptor Subfamily 2 Group E Member 3 (NR2E3); or
   (40) the pathology of the eye is associated with Best disease and the therapeutic product is Bestrophin 1 (BEST1).
29. The method of any one of paragraphs 1-11 and 15-18, wherein:
   (1) the pathology of the eye is associated with biallelic RPE65 mutation-associated retinal dystrophy and the therapeutic product is Retinoid Isomerohydrolase RPE65 (RPE65).
30. The method of any one of paragraphs 1-11 and 13-18, wherein:
   (1) the pathology of the eye is associated with Batten-CLN2 and the therapeutic product is Tripeptidyl-Peptidase 1 (TPP1);
   (2) the pathology of the eye is associated with Usher's-Type 1 and the therapeutic product is Myosin VIIA (MYO7A);
   (3) the pathology of the eye is associated with Usher's-Type 1 and the therapeutic product is Cadherin Related 23 (CDH23);
   (4) the pathology of the eye is associated with Usher's-Type 2 and the therapeutic product is Protocadherin Related 15 (PCDH15);
   (5) the pathology of the eye is associated with Usher's-Type 2 and the therapeutic product is Usherin (USH2A);
   (6) the pathology of the eye is associated with Usher's-Type 3 and the therapeutic product is Clarin 1 (CLRN1);
   (7) the pathology of the eye is associated with Stargardt's and the therapeutic product is ATP Binding Cassette Subfamily A Member 4 (ABCA4);
   (8) the pathology of the eye is associated with Stargardt's and the therapeutic product is ELOVL Fatty Acid Elongase 4 (ELOVL4);
   (9) the pathology of the eye is associated with red-green color blindness and the therapeutic product is L opsin (OPN1LW);
   (10) the pathology of the eye is associated with red-green color blindness and the therapeutic product is M opsin (OPN1MW);
   (11) the pathology of the eye is associated with blue cone monochromacy and the therapeutic product is M opsin (OPN1MW);
   (12) the pathology of the eye is associated with Leber congenital amaurosis-1 (LCA 1) and the therapeutic product is Guanylate Cyclase 2D, Retinal (GUCY2D);
   (13) the pathology of the eye is associated with Leber congenital amaurosis-2 (LCA 2) and the therapeutic product is Retinoid Isomerohydrolase RPE65 (RPE65);
   (14) the pathology of the eye is associated with Leber congenital amaurosis-4 (LCA 4) and the therapeutic product is Aryl Hydrocarbon Receptor Interacting Protein Like 1 (AIPL1);
   (15) the pathology of the eye is associated with Leber congenital amaurosis-7 (LCA 7) and the therapeutic product is Cone-Rod Homeobox (CRX);
   (16) the pathology of the eye is associated with Leber congenital amaurosis-8 (LCA 8) and the therapeutic product is Crumbs Cell Polarity Complex Component 1 (CRB1);
   (17) the pathology of the eye is associated with Leber congenital amaurosis-9 (LCA 9) and the therapeutic product is Nicotinamide Nucleotide Adenylyltransferase 1 (NMNAT1);
   (18) the pathology of the eye is associated with Leber congenital amaurosis-10 (LCA 10) and the therapeutic product is Centrosomal Protein 290 (CEP290);
   (19) the pathology of the eye is associated with Leber congenital amaurosis-11 (LCA 11) and the therapeutic product is Inosine Monophosphate Dehydrogenase 1 (IMPDH1);
   (20) the pathology of the eye is associated with Leber congenital amaurosis-15 (LCA 15) and the therapeutic product is Tubby Like Protein 1 (TLTLP1);
   (21) the pathology of the eye is associated with LHON and the therapeutic product is Mitochondrially Encoded NADH Dehydrogenase 4 (MT-ND4);
   (22) the pathology of the eye is associated with LHON and the therapeutic product is Mitochondrially Encoded NADH Dehydrogenase 6 (MT-ND6);
   (23) the pathology of the eye is associated with choroideremia and the therapeutic product is Rab Escort Protein 1 (CHM);
   (24) the pathology of the eye is associated with X-linked retinoschisis (XLRS) and the therapeutic product is Retinoschisin (RS1);
   (25) the pathology of the eye is associated with Bardet-Biedl syndrome 1 and the therapeutic product is Bardet-Biedl Syndrome 1 (BBS1);
   (26) the pathology of the eye is associated with Bardet-Biedl syndrome 6 and the therapeutic product is McKusick-Kaufman Syndrome (MKKS);
   (27) the pathology of the eye is associated with Bardet-Biedl syndrome 10 and the therapeutic product is Bardet-Biedl Syndrome 10 (BBS10);
   (28) the pathology of the eye is associated with cone dystrophy and the therapeutic product is Guanylate Cyclase Activator 1A (GUCA1A);
   (29) the pathology of the eye is associated with optic atrophy and the therapeutic product is OPA1 Mitochondrial Dynamin Like GTPase (OPA1);
   (30) the pathology of the eye is associated with retinitis pigmentosa 1 and the therapeutic product is RP1 Axonemal Microtubule Associated (RP1);
   (31) the pathology of the eye is associated with retinitis pigmentosa 2 and the therapeutic product is RP2 Activator of ARL3 GTPase (RP2);
   (32) the pathology of the eye is associated with retinitis pigmentosa 7 and the therapeutic product is Peripherin 2 (PRPH2);
   (33) the pathology of the eye is associated with retinitis pigmentosa 11 and the therapeutic product is Pre-mRNA Processing Factor 31(PRPF31);
   (34) the pathology of the eye is associated with retinitis pigmentosa 13 and the therapeutic product is Pre-mRNA Processing Factor 8 (PRPF8);
   (35) the pathology of the eye is associated with retinitis pigmentosa 37 and the therapeutic product is Nuclear Receptor Subfamily 2 Group E Member 3 (NR2E3);
   (36) the pathology of the eye is associated with retinitis pigmentosa 38 and the therapeutic product is MER Proto-Oncogene, Tyrosine Kinase (MERTK);
   (37) the pathology of the eye is associated with retinitis pigmentosa 40 and the therapeutic product is Phosphodiesterase 6B (PDE6B);
   (38) the pathology of the eye is associated with retinitis pigmentosa 41 and the therapeutic product is Prominin 1 (PROM1);
   (39) the pathology of the eye is associated with retinitis pigmentosa 56 and the therapeutic product is Interphotoreceptor Matrix Proteoglycan 2 (IMPG2);
   (40) the pathology of the eye is associated with petinitis pigmentosa 62 and the therapeutic product is Male Germ Cell Associated Kinase (MAK);
   (41) the pathology of the eye is associated with retinitis pigmentosa 80 and the therapeutic product is Intraflagellar Transport 140 (IFT140); or
   (42) the pathology of the eye is associated with Best disease and the therapeutic product is Bestrophin 1 (BEST1).
31. The method of any one of paragraphs 1-11 and 15-18, wherein:
   (1) the pathology of the eye is associated with X-linked retinitis pigmentosa (XLRP) and the therapeutic product is Retinitis Pigmentosa GTPase Regulator (RPGR);
   (2) the pathology of the eye is associated with achromatopsia and the therapeutic product is Cyclic Nucleotide Gated Channel Beta 3 (CNGB3); or
   (3) the pathology of the eye is associated with achromatopsia and the therapeutic product is Cyclic Nucleotide Gated Channel Alpha 3 (CNGA3).
32. The method of any one of paragraphs 1-31, wherein the recombinant viral vector further comprises a nucleotide sequence encoding a promoter or an enhancer-promoter, which nucleotide sequence encoding the promoter or enhancer-promoter is operably linked to the nucleotide sequence encoding the therapeutic product, and wherein the promoter or enhancer-promoter is:
   (1) a CAG promoter;
   (2) a CBA promoter;
   (3) a CMV promoter;
   (4) a PR1.7 promoter;
   (5) a Rhodopsin Kinase (GRK1) photoreceptor-specific enhancer-promoter;
   (6) an hCARp promoter;
   (7) an hRKp;
   (8) a cone photoreceptor specific human arrestin 3 (ARR3) promoter;
   (9) a rhodopsin promoter;or
   (10) a U6 promoter.
33. The method of any one of paragraphs 1-11 and 13-15, wherein the recombinant viral vector further comprises a nucleotide sequence encoding a cone-specific promoter, which nucleotide sequence encoding the cone-specific promoter is operably linked to the nucleotide sequence encoding the therapeutic product, and wherein:
   (1) the pathology of the eye is associated with red-green color blindness and the therapeutic product is L opsin (OPN1LW);
   (2) the pathology of the eye is associated with red-green color blindness and the therapeutic product is M opsin (OPN1MW);
   (3) the pathology of the eye is associated with blue cone monochromacy and the therapeutic product is M opsin (OPN1MW);
   (4) the pathology of the eye is associated with cone dystrophy and the therapeutic product is Guanylate Cyclase Activator 1A (GUCA1A); or
   (5) the pathology of the eye is associated with blue cone monochromacy (BCM) and the therapeutic product is L opsin (OPN1LW).
34. The method of any one of paragraphs 1-33, wherein the administering step delivers a therapeutically effective amount of the therapeutic product to the retina of said human subject.
35. The method of paragraph 34, wherein the therapeutically effective amount of the therapeutic product is produced by human retinal cells of said human subject.
36. The method of paragraph 34, wherein the therapeutically effective amount of the therapeutic product is produced by human photoreceptor cells, horizontal cells, bipolar cells, amacrine cells, retina ganglion cells, and/or retinal pigment epithelial cells in the external limiting membrane of said human subject.
37. The method of paragraph 36, wherein the human photoreceptor cells are cone cells and/or rod cells.
38. The method of paragraph 36, wherein the retina ganglion cells are midget cells, parasol cells, bistratified cells, giant retina ganglion cells, photosensitive ganglion cells, and/or Miiller glia.
39. The method of any one of paragraphs 1-38, wherein the recombinant viral vector is an rAAV vector.
40. The method of paragraph 39, wherein the recombinant viral vector is an rAAV8 vector.
41. The method of any one of paragraphs 1-40, which further comprises, after the administering step, a step of monitoring the post ocular injection thermal profile of the injected material in the eye using an infrared thermal camera.
42. The method of paragraph 41, wherein the infrared thermal camera is an FLIR T530 infrared thermal camera.
43. The method of any one of paragraphs 1-43, wherein the recombinant nucleotide expression vector is administered at a dose about 6.0× 10¹⁰ genome copies per eye.
44. The method of any one of paragraphs 1-43, wherein the recombinant nucleotide expression vector is administered at a dose about 1.6 × 10¹¹ genome copies per eye.
45. The method of any one of paragraphs 1-43, wherein the recombinant nucleotide expression vector is administered at a dose about 2.5 × 10¹¹ genome copies per eye.
46. The method of any one of paragraphs 1-43, wherein the recombinant nucleotide expression vector is administered at a dose about 5.0 × 10¹¹ genome copies per eye.
47. The method of any one of paragraphs 1-43, wherein the recombinant nucleotide expression vector is administered at a dose about 3.0 x 10¹² genome copies per eye.

### 4.1.2 Set 2

1. A method for treating a pathology of the eye, comprising administering to the subretinal space in the eye of a human subject in need of treatment a recombinant viral vector comprising a nucleotide sequence encoding a therapeutic product such that the therapeutic product is expressed and results in treatment of the pathology of the eye, wherein the method does not comprise performing a vitrectomy on the eye of said human patient.
2. The method of paragraph 1, wherein the administering step comprises administering to the subretinal space in the eye of said human subject the recombinant viral vector therapeutic product via the suprachoroidal space in the eye of said human subject.
3. The method of paragraph 2, wherein the administering step is by the use of a subretinal drug delivery device comprising a catheter that can be inserted and tunneled through the suprachoroidal space toward the posterior pole, where a small needle injects into the subretinal space.
4. The method of paragraph 3, wherein the administering step comprises inserting and tunneling the catheter of the subretinal drug delivery device through the suprachoroidal space.
5. A method for treating a pathology of the eye, comprising administering to the suprachoroidal space in the eye of a human subject in need of treatment a recombinant viral vector comprising a nucleotide sequence encoding a therapeutic product such that the therapeutic product is expressed and results in treatment of the pathology of the eye.
6. The method of paragraph 5, wherein the administering step is by injecting the recombinant viral vector into the suprachoroidal space using a suprachoroidal drug delivery device.
7. The method of paragraph 5 or 6, wherein the suprachoroidal drug delivery device is a microinjector.
8. A method for treating a pathology of the eye, comprising administering to the outer surface of the sclera in the eye of a human subject in need of treatment a recombinant viral vector comprising a nucleotide sequence encoding a therapeutic product such that the therapeutic product is expressed and results in treatment of the pathology of the eye.
9. The method of paragraph 8, wherein the administering step is by the use of a juxtascleral drug delivery device that comprises a cannula whose tip can be inserted and kept in direct apposition to the scleral surface.
10. The method of paragraph 9, wherein the administering step comprises inserting and keeping the tip of the cannula in direct apposition to the scleral surface.
11. The method of any one of paragraphs 1-10, wherein the therapeutic product is not an anti-human vascular endothelial growth factor (hVEGF) antibody.
12. The method of any one of paragraphs 1-11, wherein the pathology of the eye is not associated with neovascular age-related macular degeneration (nAMD).
13. A method for treating a pathology of the eye, comprising administering to the subretinal space in the eye of a human subject in need of treatment a recombinant viral vector comprising a nucleotide sequence encoding a therapeutic product such that the therapeutic product is expressed and results in treatment of the pathology of the eye, wherein the method comprises performing a vitrectomy on the eye of said human patient, and wherein the therapeutic product is not anti-human vascular endothelial growth factor (hVEGF) antibody
14. The method of paragraph 13, wherein the vitrectomy is a partial vitrectomy.
15. A method for treating a pathology of the eye, comprising administering to the subretinal space peripheral to the optic disc, fovea and macula located in the back of the eye of a human subject in need of treatment a recombinant viral vector comprising a nucleotide sequence encoding a therapeutic product such that the therapeutic product is expressed and results in treatment of the pathology of the eye, wherein the method does not comprise performing a vitrectomy on the eye of said human patient.
16. The method of paragraph 15, wherein the administering step is by transvitreal injection.
17. The method of paragraph 16, wherein the transvitreal injection comprises inserting a sharp needle into the sclera via the superior or inferior side of the eye and passing the sharp needle all the way through the vitreous to inject the recombinant viral vector to the subretinal space on the other side.
18. The method of paragraph 16, wherein the transvitreal injection comprises inserting a trochar into the sclera and inserting a cannula through the trochar and through the vitreous to inject the recombinant viral vector to the subretinal space on the other side
19. The method of any one of paragraphs 15-18, wherein the therapeutic product is an anti-hVEGF antibody.
20. The method of paragraph 19, wherein the anti-hVEGF antibody is an anti-hVEGF antigen-binding fragment.
21. The method of paragraph 20, wherein the anti-hVEGF antigen-binding fragment is a Fab, F(ab')₂, or single chain variable fragment (scFv).
22. The method of any one of paragraphs 19-21, wherein the anti-hVEGF antibody comprises a heavy chain comprising the amino acid sequence of SEQ ID NO:2 or SEQ ID NO:4, and a light chain comprising the amino acid sequence of SEQ ID NO: 1, or SEQ ID NO:3.
23. The method of any one of paragraphs 19-21, wherein the anti-hVEGF antibody comprises light chain CDRs 1-3 of SEQ ID NOs:14-16 and heavy chain CDRs 1-3 of SEQ ID NOs: 17-19 or SEQ ID NOs:20, 18, and 21.
24. The method of any one of paragraphs 19-23, wherein the pathology of the eye is associated with nAMD, dry age-related macular degeneration (dry AMD), retinal vein occlusion (RVO), diabetic macular edema (DME), or diabetic retinopathy (DR).
25. The method of any one of paragraphs 19-23, wherein the pathology of the eye is associated with nAMD.
26. The method of any one of paragraphs 1-11 and 13-18, wherein:
   (1) the pathology of the eye is associated with Batten-CLN1 and the therapeutic product is Palmitoyl-Protein Thioesterase 1 (PPT1);
   (2) the pathology of the eye is associated with Batten-CLN2 and the therapeutic product is Tripeptidyl-Peptidase 1 (TPP1);
   (3) the pathology of the eye is associated with Batten-CLN3 and the therapeutic product is Battenin (CLN3);
   (4) the pathology of the eye is associated with Batten-CLN6 and the therapeutic product is CLN6 Transmembrane ER Protein (CLN6);
   (5) the pathology of the eye is associated with Batten-CLN7 and the therapeutic product is Major Facilitator Superfamily Domain Containing 8 (MFSD8);
   (6) the pathology of the eye is associated with Usher's-Type 1 and the therapeutic product is Myosin VIIA (MYO7A);
   (7) the pathology of the eye is associated with Usher's-Type 1 and the therapeutic product is Cadherin Related 23 (CDH23);
   (8) the pathology of the eye is associated with Usher's-Type 2 and the therapeutic product is Protocadherin Related 15 (PCDH15);
   (9) the pathology of the eye is associated with Usher's-Type 2 and the therapeutic product is Usherin (USH2A);
   (10) the pathology of the eye is associated with Usher's-Type 3 and the therapeutic product is Clarin 1 (CLRN1);
   (11) the pathology of the eye is associated with Stargardt's and the therapeutic product is ATP Binding Cassette Subfamily A Member 4 (ABCA4);
   (12) the pathology of the eye is associated with Stargardt's and the therapeutic product is ELOVL Fatty Acid Elongase 4 (ELOVL4);
   (13) the pathology of the eye is associated with uveitis and the therapeutic product is an anti-Interleukin 6 (IL6) monoclonal antibody;
   (14) the pathology of the eye is associated with uveitis and the therapeutic product is an anti-TNF-alpha (TNF) monoclonal antibody;
   (15) the pathology of the eye is associated with diabetic macular edema (DME) and the therapeutic product is an anti-IL6 monoclonal antibody;
   (16) the pathology of the eye is associated with red-green color blindness and the therapeutic product is L opsin (OPN1LW);
   (17) the pathology of the eye is associated with red-green color blindness and the therapeutic product is M opsin (OPN1MW);
   (18) the pathology of the eye is associated with blue cone monochromacy and the therapeutic product is M opsin (OPN1MW);
   (19) the pathology of the eye is associated with Leber congenital amaurosis-1 (LCA 1) and the therapeutic product is Guanylate Cyclase 2D, Retinal (GUCY2D);
   (20) the pathology of the eye is associated with Leber congenital amaurosis-2 (LCA 2) and the therapeutic product is Retinoid Isomerohydrolase RPE65 (RPE65);
   (21) the pathology of the eye is associated with LCA 3 and the therapeutic product is Spermatogenesis Associated 7 (SPATA7);
   (22) the pathology of the eye is associated with Leber congenital amaurosis-4 (LCA 4) and the therapeutic product is Aryl Hydrocarbon Receptor Interacting Protein Like 1 (AIPL1);
   (23) the pathology of the eye is associated with Leber congenital amaurosis-5 (LCA 5) and the therapeutic product is Lebercilin (LCA5);
   (24) the pathology of the eye is associated with Leber congenital amaurosis-6 (LCA 6) and the therapeutic product is RPGR Interacting Protein 1 (RPGRIP1);
   (25) the pathology of the eye is associated with Leber congenital amaurosis-7 (LCA 7) and the therapeutic product is Cone-Rod Homeobox (CRX);
   (26) the pathology of the eye is associated with Leber congenital amaurosis-8 (LCA 8) and the therapeutic product is Crumbs Cell Polarity Complex Component 1 (CRB1);
   (27) the pathology of the eye is associated with Leber congenital amaurosis-9 (LCA 9) and the therapeutic product is Nicotinamide Nucleotide Adenylyltransferase 1 (NMNAT1);
   (28) the pathology of the eye is associated with Leber congenital amaurosis-10 (LCA 10) and the therapeutic product is Centrosomal Protein 290 (CEP290);
   (29) the pathology of the eye is associated with Leber congenital amaurosis-11 (LCA 11) and the therapeutic product is Inosine Monophosphate Dehydrogenase 1 (IMPDH1);
   (30) the pathology of the eye is associated with Leber congenital amaurosis-12 (LCA 12) and the therapeutic product is Retinal Degeneration 3, GUCY2D regulator (RD3) ;
   (31) the pathology of the eye is associated with Leber congenital amaurosis-13 (LCA 13) and the therapeutic product is Retinol Dehydrogenase 12 (RDH12);
   (32) the pathology of the eye is associated with Leber congenital amaurosis-14 (LCA 14) and the therapeutic product is Lecithin Retinol Acyltransferase (LRAT);
   (33) the pathology of the eye is associated with Leber congenital amaurosis-15 (LCA 15) and the therapeutic product is Tubby Like Protein 1 (TULP1);
   (34) the pathology of the eye is associated with Leber congenital amaurosis-16 (LCA 16) and the therapeutic product is Potassium Voltage-Gated Channel Subfamily J Member 13 (KCNJ13);
   (35) the pathology of the eye is associated with Leber's hereditary optic neuropathy (LHON) and the therapeutic product is Mitochondrially Encoded NADH Dehydrogenase 1 (MT-ND1);
   (36) the pathology of the eye is associated with LHON and the therapeutic product is Mitochondrially Encoded NADH Dehydrogenase 4 (MT-ND4);
   (37) the pathology of the eye is associated with LHON and the therapeutic product is Mitochondrially Encoded NADH Dehydrogenase 6 (MT-ND6);
   (38) the pathology of the eye is associated with neuromyelitis optica (NMO) and the therapeutic product is an anti-complement C5 monoclonal antibody;
   (39) the pathology of the eye is associated with NMO and the therapeutic product is an anti-IL6 monoclonal antibody;
   (40) the pathology of the eye is associated with uveitis and the therapeutic product is an anti-complement C5 monoclonal antibody;
   (41) the pathology of the eye is associated with uveitis and the therapeutic product is Angiotensin I Converting Enzyme (ACE);
   (42) the pathology of the eye is associated with uveitis and the therapeutic product is Interleukin 10 (IL10);
   (43) the pathology of the eye is associated with uveitis and the therapeutic product is an anti-TNF monoclonal antibody;
   (44) the pathology of the eye is associated with choroideremia and the therapeutic product is Rab Escort Protein 1 (CHM);
   (45) the pathology of the eye is associated with X-linked retinoschisis (XLRS) and the therapeutic product is Retinoschisin (RS1);
   (46) the pathology of the eye is associated with Bardet-Biedl syndrome 1 and the therapeutic product is Bardet-Biedl Syndrome 1 (BBS1);
   (47) the pathology of the eye is associated with Bardet-Biedl syndrome 2 and the therapeutic product is Bardet-Biedl Syndrome 2 (BBS2);
   (48) the pathology of the eye is associated with Bardet-Biedl syndrome 3 and the therapeutic product is ADP Ribosylation Factor Like GTPase 6 (ARL6);
   (49) the pathology of the eye is associated with Bardet-Biedl syndrome 4 and the therapeutic product is Bardet-Biedl Syndrome 4 (BBS4);
   (50) the pathology of the eye is associated with Bardet-Biedl syndrome 5 and the therapeutic product is Bardet-Biedl Syndrome 5 (BBS5);
   (51) the pathology of the eye is associated with Bardet-Biedl syndrome 6 and the therapeutic product is McKusick-Kaufman Syndrome (MKKS);
   (52) the pathology of the eye is associated with Bardet-Biedl syndrome 7 and the therapeutic product is Bardet-Biedl Syndrome 7 (BBS7);
   (53) the pathology of the eye is associated with Bardet-Biedl syndrome 8 and the therapeutic product is Tetratricopeptide Repeat Domain 8 (TTC8);
   (54) the pathology of the eye is associated with Bardet-Biedl syndrome 9 and the therapeutic product is Bardet-Biedl Syndrome 9 (BBS9);
   (55) the pathology of the eye is associated with Bardet-Biedl syndrome 10 and the therapeutic product is Bardet-Biedl Syndrome 10 (BBS10);
   (56) the pathology of the eye is associated with Bardet-Biedl syndrome 11 and the therapeutic product is Tripartite Motif Containing 32 (TRIM32);
   (57) the pathology of the eye is associated with Bardet-Biedl syndrome 12 and the therapeutic product is Bardet-Biedl Syndrome 12 (BBS 12);
   (58) the pathology of the eye is associated with Bardet-Biedl syndrome 13 and the therapeutic product is MKS Transition Zone Complex Subunit 1 (MKS 1);
   (59) the pathology of the eye is associated with Bardet-Biedl syndrome 14 and the therapeutic product is Centrosomal Protein 290 (CEP290);
   (60) the pathology of the eye is associated with Bardet-Biedl syndrome 15 and the therapeutic product is WD Repeat Containing Planar Cell Polarity Effector (WDPCP);
   (61) the pathology of the eye is associated with Bardet-Biedl syndrome 16 and the therapeutic product is Serologically Defined Colon Cancer Antigen 8 (SDCCAG8);
   (62) the pathology of the eye is associated with Bardet-Biedl syndrome 17 and the therapeutic product is Leucine Zipper Transcription Factor Like 1 (LZTFL1);
   (63) the pathology of the eye is associated with Bardet-Biedl syndrome 18 and the therapeutic product is BBSome Interacting Protein 1 (BBIP1);
   (64) the pathology of the eye is associated with Bardet-Biedl syndrome 19 and the therapeutic product is Intraflagellar Transport 27 (IFT27);
   (65) the pathology of the eye is associated with cone dystrophy and the therapeutic product is Guanylate Cyclase Activator 1A (GUCA1A);
   (66) the pathology of the eye is associated with optic atrophy and the therapeutic product is OPA1 Mitochondrial Dynamin Like GTPase (OPA1);
   (67) the pathology of the eye is associated with retinitis pigmentosa 1 and the therapeutic product is RP1 Axonemal Microtubule Associated (RP1);
   (68) the pathology of the eye is associated with retinitis pigmentosa 2 and the therapeutic product is RP2 Activator of ARL3 GTPase (RP2);
   (69) the pathology of the eye is associated with retinitis pigmentosa 7 and the therapeutic product is Peripherin 2 (PRPH2);
   (70) the pathology of the eye is associated with retinitis pigmentosa 11 and the therapeutic product is Pre-mRNA Processing Factor 31(PRPF31);
   (71) the pathology of the eye is associated with retinitis pigmentosa 12 and the therapeutic product is Crumbs Cell Polarity Complex Component 1 (CRB1);
   (72) the pathology of the eye is associated with retinitis pigmentosa 13 and the therapeutic product is Pre-mRNA Processing Factor 8 (PRPF8);
   (73) the pathology of the eye is associated with retinitis pigmentosa 25 and the therapeutic product is Eyes Shut Homolog (EYS);
   (74) the pathology of the eye is associated with retinitis pigmentosa 28 and the therapeutic product is FAM161 Centrosomal Protein A (FAM161A);
   (75) the pathology of the eye is associated with retinitis pigmentosa 37 and the therapeutic product is Nuclear Receptor Subfamily 2 Group E Member 3 (NR2E3);
   (76) the pathology of the eye is associated with retinitis pigmentosa 38 and the therapeutic product is MER Proto-Oncogene, Tyrosine Kinase (MERTK);
   (77) the pathology of the eye is associated with retinitis pigmentosa 40 and the therapeutic product is Phosphodiesterase 6B (PDE6B);
   (78) the pathology of the eye is associated with retinitis pigmentosa 41 and the therapeutic product is Prominin 1 (PROM1);
   (79) the pathology of the eye is associated with retinitis pigmentosa 43 and the therapeutic product is Phosphodiesterase 6A (PDE6A);
   (80) the pathology of the eye is associated with retinitis pigmentosa 56 and the therapeutic product is Interphotoreceptor Matrix Proteoglycan 2 (IMPG2);
   (81) the pathology of the eye is associated with petinitis pigmentosa 62 and the therapeutic product is Male Germ Cell Associated Kinase (MAK);
   (82) the pathology of the eye is associated with retinitis pigmentosa 80 and the therapeutic product is Intraflagellar Transport 140 (IFT140);
   (83) the pathology of the eye is associated with dry AMD and the therapeutic product is an anti-complement C5 monoclonal antibody;
   (84) the pathology of the eye is associated with dry AMD and the therapeutic product is an anti-membrane attack complex (MAC) monoclonal antibody;
   (85) the pathology of the eye is associated with dry AMD and the therapeutic product is HtrA Serine Peptidase 1 (HTRA1);
   (86) the pathology of the eye is associated with Best disease and the therapeutic product is Bestrophin 1 (BEST1);
   (87) the pathology of the eye is associated with dry AMD and the therapeutic product is a complement factor B antisense oligonucleotide;
   (88) the pathology of the eye is associated with dry AMD and the therapeutic product is an anti-beta-amyloid monoclonal antibody;
   (89) the pathology of the eye is associated with dry AMD and the therapeutic product is CD59 glycoprotein (CD59);
   (90) the pathology of the eye is associated with dry AMD and the therapeutic product is Channelrhodopsin-1 (ChR1);
   (91) the pathology of the eye is associated with dry AMD and the therapeutic product is Channelrhodopsin-2 (ChR2), the light-sensitive protein discovered in *Chlamydomonas reinhardtii;*
   (92) the pathology of the eye is associated with dry AMD and the therapeutic product is an anti-complement factor C5a aptamer;
   (93) the pathology of the eye is associated with dry AMD and the therapeutic product is anti-complement factor D monoclonal antibody;
   (94) the pathology of the eye is associated with age-related retinal ganglion cell (RGC) degeneration and the therapeutic product is DnaJ heat shock protein family (Hsp40) member C3 (DNAJC3);
   (95) the pathology of the eye is associated with blue cone monochromacy (BCM) and the therapeutic product is L opsin (OPN1LW);
   (96) the pathology of the eye is associated with glaucoma and the therapeutic product is beta-2 adrenoceptor siRNA;
   (97) the pathology of the eye is associated with glaucoma and the therapeutic product is Caspase-2 (CASP2);
   (98) the pathology of the eye is associated with glaucoma and the therapeutic product is Insulin Receptor Substrate 1 (IRS1);
   (99) the pathology of the eye is associated with glaucoma and the therapeutic product is HIF-1 Responsive Protein RTP801 (RTP801);
   (100) the pathology of the eye is associated with glaucoma and the therapeutic product is Transforming Growth Factor Beta 2 (TGFB2);
   (101) the pathology of the eye is associated with glaucoma and the therapeutic product is Brain Derived Neurotrophic Factor (BDNF);
   (102) the pathology of the eye is associated with glaucoma and the therapeutic product is Ciliary Neurotrophic Factor (CNTF);
   (103) the pathology of the eye is associated with glaucoma and the therapeutic product is Prostaglandin-Endoperoxide Synthase 2 (PTGS2);
   (104) the pathology of the eye is associated with glaucoma and the therapeutic product is Prostaglandin F Receptor (PTGFR);
   (105) the pathology of the eye is associated with glaucoma and the therapeutic product is a hyaluronidase;
   (106) the pathology of the eye is associated with glaucoma and the therapeutic product is Pigment Epithelium-Derived Factor (PEDF);
   (107) the pathology of the eye is associated with glaucoma and the therapeutic product is Vascular Endothelial Growth Factor (VEGF);
   (108) the pathology of the eye is associated with glaucoma and the therapeutic product is Placental Growth Factor (PGF);
   (109) the pathology of the eye is associated with glaucoma and the therapeutic product is Myocilin (MYOC);
   (110) the pathology of the eye is associated with NMO and the therapeutic product is an anti-complement C5 monoclonal antibody;
   (111) the pathology of the eye is associated with NMO and the therapeutic product is C-C Motif Chemokine Receptor 5 (CCR5) siRNA;
   (112) the pathology of the eye is associated with NMO and the therapeutic product is an anti-CD19 monoclonal antibody;
   (113) the pathology of the eye is associated with retinitis pigmentosa that is associated with rhodopsin mutations and the therapeutic product is Channelrhodopsin-1 (ChR1);
   (114) the pathology of the eye is associated with retinitis pigmentosa that is associated with rhodopsin mutations and the therapeutic product is Channelrhodopsin-2 (ChR2);
   (115) the pathology of the eye is associated with retinitis pigmentosa and the therapeutic product is Ciliary Neurotrophic Factor (CNTF);
   (116) the pathology of the eye is associated with autosomal recessive retinitis pigmentosa and the therapeutic product is Crumbs Cell Polarity Complex Component 1 (CRB1);
   (117) the pathology of the eye is associated with autosomal recessive retinitis pigmentosa and the therapeutic product is Crumbs Cell Polarity Complex Component 2 (CRB2);
   (118) the pathology of the eye is associated with retinitis pigmentosa and the therapeutic product is Histone Deacetylase 4 (HDAC4);
   (119) the pathology of the eye is associated with retinitis pigmentosa and the therapeutic product is Rhodopsin (RHO);
   (120) the pathology of the eye is associated with retinitis pigmentosa and the therapeutic product is Nerve Growth Factor (NGF);
   (121) the pathology of the eye is associated with retinitis pigmentosa and the therapeutic product is Nuclear Factor, Erythroid 2 Like 2 (NRF2);
   (122) the pathology of the eye is associated with retinitis pigmentosa and the therapeutic product is Pigment Epithelium-Derived Factor (PEDF);
   (123) the pathology of the eye is associated with retinitis pigmentosa and the therapeutic product is Glutathione S-Transferase PI 1 (GSTP1);
   (124) the pathology of the eye is associated with retinitis pigmentosa and the therapeutic product is Rod-Derived Cone Viability Factor (RDCVF);
   (125) the pathology of the eye is associated with retinitis pigmentosa and the therapeutic product is Rhodopsin (RHO);
   (126) the pathology of the eye is associated with retinitis pigmentosa and the therapeutic product is Retinaldehyde Binding Protein 1 (RLBP1);
   (127) the pathology of the eye is associated with Stargardt's disease and the therapeutic product is an anti-complement C5 aptamer;
   (128) the pathology of the eye is associated with uveitis and the therapeutic product is Double Homeobox 4 (DUX4);
   (129) the pathology of the eye is associated with uveitis and the therapeutic product is NLR Family Pyrin Domain Containing 3 (NLRP3);
   (130) the pathology of the eye is associated with uveitis and the therapeutic product is Spleen Associated Tyrosine Kinase (SYK);
   (131) the pathology of the eye is associated with uveitis and the therapeutic product is Adrenocorticotropic Hormone (ACTH);
   (132) the pathology of the eye is associated with uveitis and the therapeutic product is Caspase 1 (CASP1);
   (133) the pathology of the eye is associated with uveitis and the therapeutic product is anti-CD59 monoclonal antibody;
   (134) the pathology of the eye is associated with uveitis and the therapeutic product is an anti-complement C5 aptamer;
   (135) the pathology of the eye is associated with corneal neovascularization and the therapeutic product is Insulin Receptor Substrate 1 (IRS1);
   (136) the pathology of the eye is associated with corneal neovascularization and the therapeutic product is NOTCH Regulated Ankyrin Repeat Protein (NRARP);
   (137) the pathology of the eye is associated with diabetic retinopathy and the therapeutic product is NOTCH Regulated Ankyrin Repeat Protein (NRARP);
   (138) the pathology of the eye is associated with diabetic retinopathy and the therapeutic product is Alpha-2-Antiplasmin (A2AP);
   (139) the pathology of the eye is associated with diabetic retinopathy and the therapeutic product is Plasminogen (PLG);
   (140) the pathology of the eye is associated with diabetic retinopathy and the therapeutic product is a growth hormone;
   (141) the pathology of the eye is associated with diabetic retinopathy and the therapeutic product is Insulin Like Growth Factor 1 (IGF1);
   (142) the pathology of the eye is associated with diabetic retinopathy and the therapeutic product is Interleukin 1 Beta (IL1B).
   (143) the pathology of the eye is associated with diabetic retinopathy and the therapeutic product is Angiotensin I Converting Enzyme 2 (ACE2);
   (144) the pathology of the eye is associated with diabetic retinopathy and the therapeutic product is IRS1;
   (145) the pathology of the eye is associated with diabetic retinopathy and the therapeutic product is an anti-integrin oligopeptide;
   (146) the pathology of the eye is associated with diabetic retinopathy and the therapeutic product is an anti-Placental Growth Factor (PGF) monoclonal antibody;
   (147) the pathology of the eye is associated with Graves' ophthalmopathy and the therapeutic product is an anti-CD40 monoclonal antibody;
   (148) the pathology of the eye is associated with Graves' ophthalmopathy and the therapeutic product is an anti-Insulin-Like Growth Factor 1 Receptor (IGF1R) monoclonal antibody;
   (149) the pathology of the eye is associated with Graves' ophthalmopathy and the therapeutic product is an anti-Insulin-Like Growth Factor 2 Receptor (IGF2R) monoclonal antibody;
   (150) the pathology of the eye is associated with DME and the therapeutic product is an anti-integrin oligopeptide;
   (151) the pathology of the eye is associated with DME and the therapeutic product is an anti-Placental Growth Factor (PGF) monoclonal antibody;
   (152) the pathology of the eye is associated with DME and the therapeutic product is RTP801 siRNA;
   (153) the pathology of the eye is associated with multiple sclerosis (MS)-associated vision loss and the therapeutic product is ND1;
   (154) the pathology of the eye is associated with myopia and the therapeutic product is Matrix Metalloproteinase 2 (MMP2) RNAi;
   (155) the pathology of the eye is associated with X-linked recessive ocular albinism and the therapeutic product is G-Protein Coupled Receptor 143 (GPR143);
   (156) the pathology of the eye is associated with oculocutaneous albinism type 1 and the therapeutic product is Tyrosinase (TYR);
   (157) the pathology of the eye is associated with optic neuritis and the therapeutic product is Caspase 2 (CASP2);
   (158) the pathology of the eye is associated with optic neuritis and the therapeutic product is an anti-Leucine Rich Repeat And Ig Domain Containing Protein 1 (LINGO1) monoclonal antibody; or
   (159) the pathology of the eye is associated with polypoidal choroidal vasculopathy and the therapeutic product is an anti-complement C5 aptamer.
27. The method of any one of paragraphs 1-11 and 15-18, wherein:
   (1) the pathology of the eye is associated with X-linked retinitis pigmentosa (XLRP) and the therapeutic product is Retinitis Pigmentosa GTPase Regulator (RPGR);
   (2) the pathology of the eye is associated with achromatopsia (ACHM) and the therapeutic product is Cyclic Nucleotide Gated Channel Beta 3 (CNGB3);
   (3) the pathology of the eye is associated with achromatopsia and the therapeutic product is Cyclic Nucleotide Gated Channel Alpha 3 (CNGA3); or
   (4) the pathology of the eye is associated with biallelic RPE65 mutation-associated retinal dystrophy and the therapeutic product is Retinoid Isomerohydrolase RPE65 (RPE65).
28. The method of any one of paragraphs 1-11 and 13-18, wherein:
   (1) the pathology of the eye is associated with Batten-CLN1 and the therapeutic product is Palmitoyl-Protein Thioesterase 1 (PPT1);
   (2) the pathology of the eye is associated with Batten-CLN2 and the therapeutic product is Tripeptidyl-Peptidase 1 (TPP1);
   (3) the pathology of the eye is associated with Batten-CLN3 and the therapeutic product is Battenin (CLN3);
   (4) the pathology of the eye is associated with uveitis and the therapeutic product is an anti-Interleukin 6 (IL6) monoclonal antibody;
   (5) the pathology of the eye is associated with uveitis and the therapeutic product is an anti-TNF-alpha (TNF) monoclonal antibody;
   (6) the pathology of the eye is associated with diabetic macular edema (DME) and the therapeutic product is an anti-IL6 monoclonal antibody;
   (7) the pathology of the eye is associated with red-green color blindness and the therapeutic product is L opsin (OPN1LW);
   (8) the pathology of the eye is associated with red-green color blindness and the therapeutic product is M opsin (OPN1MW);
   (9) the pathology of the eye is associated with blue cone monochromacy and the therapeutic product is M opsin (OPN1MW);
   (10) the pathology of the eye is associated with Leber congenital amaurosis-1 (LCA 1) and the therapeutic product is Guanylate Cyclase 2D, Retinal (GUCY2D);
   (11) the pathology of the eye is associated with Leber congenital amaurosis-2 (LCA 2) and the therapeutic product is Retinoid Isomerohydrolase RPE65 (RPE65);
   (12) the pathology of the eye is associated with Leber congenital amaurosis-7 (LCA 7) and the therapeutic product is Cone-Rod Homeobox (CRX);
   (13) the pathology of the eye is associated with Leber congenital amaurosis-11 (LCA 11) and the therapeutic product is Inosine Monophosphate Dehydrogenase 1 (IMPDH1);
   (14) the pathology of the eye is associated with Leber congenital amaurosis-12 (LCA 12) and the therapeutic product is Retinal Degeneration 3, GUCY2D regulator (RD3);
   (15) the pathology of the eye is associated with Leber congenital amaurosis-13 (LCA 13) and the therapeutic product is Retinol Dehydrogenase 12 (RDH12);
   (16) the pathology of the eye is associated with Leber congenital amaurosis-15 (LCA 15) and the therapeutic product is Tubby Like Protein 1 (TULP1);
   (17) the pathology of the eye is associated with Leber congenital amaurosis-16 (LCA 16) and the therapeutic product is Potassium Voltage-Gated Channel Subfamily J Member 13 (KCNJ13);
   (18) the pathology of the eye is associated with Leber's hereditary optic neuropathy (LHON) and the therapeutic product is Mitochondrially Encoded NADH Dehydrogenase 1 (MT-ND1);
   (19) the pathology of the eye is associated with LHON and the therapeutic product is Mitochondrially Encoded NADH Dehydrogenase 4 (MT-ND4);
   (20) the pathology of the eye is associated with LHON and the therapeutic product is Mitochondrially Encoded NADH Dehydrogenase 6 (MT-ND6);
   (21) the pathology of the eye is associated with neuromyelitis optica (NMO) and the therapeutic product is an anti-complement C5 monoclonal antibody;
   (22) the pathology of the eye is associated with NMO and the therapeutic product is an anti-IL6 monoclonal antibody;
   (23) the pathology of the eye is associated with uveitis and the therapeutic product is an anti-complement C5 monoclonal antibody;
   (24) the pathology of the eye is associated with uveitis and the therapeutic product is Angiotensin I Converting Enzyme (ACE);
   (25) the pathology of the eye is associated with uveitis and the therapeutic product is Interleukin 10 (IL10);
   (26) the pathology of the eye is associated with uveitis and the therapeutic product is an anti-TNF monoclonal antibody;
   (27) the pathology of the eye is associated with X-linked retinoschisis (XLRS) and the therapeutic product is Retinoschisin (RS1);
   (28) the pathology of the eye is associated with Bardet-Biedl syndrome 1 and the therapeutic product is Bardet-Biedl Syndrome 1 (BBS1);
   (29) the pathology of the eye is associated with Bardet-Biedl syndrome 3 and the therapeutic product is ADP Ribosylation Factor Like GTPase 6 (ARL6);
   (30) the pathology of the eye is associated with Bardet-Biedl syndrome 5 and the therapeutic product is Bardet-Biedl Syndrome 5 (BBS5);
   (31) the pathology of the eye is associated with Bardet-Biedl syndrome 6 and the therapeutic product is McKusick-Kaufman Syndrome (MKKS);
   (32) the pathology of the eye is associated with Bardet-Biedl syndrome 10 and the therapeutic product is Bardet-Biedl Syndrome 10 (BBS10);
   (33) the pathology of the eye is associated with Bardet-Biedl syndrome 11 and the therapeutic product is Tripartite Motif Containing 32 (TRIM32);
   (34) the pathology of the eye is associated with Bardet-Biedl syndrome 13 and the therapeutic product is MKS Transition Zone Complex Subunit 1 (MKS1);
   (35) the pathology of the eye is associated with Bardet-Biedl syndrome 18 and the therapeutic product is BBSome Interacting Protein 1 (BBIP1);
   (36) the pathology of the eye is associated with Bardet-Biedl syndrome 19 and the therapeutic product is Intraflagellar Transport 27 (IFT27);
   (37) the pathology of the eye is associated with cone dystrophy and the therapeutic product is Guanylate Cyclase Activator 1A (GUCA1A);
   (38) the pathology of the eye is associated with retinitis pigmentosa 13 and the therapeutic product is Pre-mRNA Processing Factor 8 (PRPF8);
   (39) the pathology of the eye is associated with retinitis pigmentosa 37 and the therapeutic product is Nuclear Receptor Subfamily 2 Group E Member 3 (NR2E3); or
   (40) the pathology of the eye is associated with Best disease and the therapeutic product is Bestrophin 1 (BEST1).
29. The method of any one of paragraphs 1-11 and 15-18, wherein:
   (1) the pathology of the eye is associated with biallelic RPE65 mutation-associated retinal dystrophy and the therapeutic product is Retinoid Isomerohydrolase RPE65 (RPE65).
30. The method of any one of paragraphs 1-11 and 13-18, wherein:
   (1) the pathology of the eye is associated with Batten-CLN2 and the therapeutic product is Tripeptidyl-Peptidase 1 (TPP1);
   (2) the pathology of the eye is associated with Usher's-Type 1 and the therapeutic product is Myosin VIIA (MYO7A);
   (3) the pathology of the eye is associated with Usher's-Type 1 and the therapeutic product is Cadherin Related 23 (CDH23);
   (4) the pathology of the eye is associated with Usher's-Type 2 and the therapeutic product is Protocadherin Related 15 (PCDH15);
   (5) the pathology of the eye is associated with Usher's-Type 2 and the therapeutic product is Usherin (USH2A);
   (6) the pathology of the eye is associated with Usher's-Type 3 and the therapeutic product is Clarin 1 (CLRN1);
   (7) the pathology of the eye is associated with Stargardt's and the therapeutic product is ATP Binding Cassette Subfamily A Member 4 (ABCA4);
   (8) the pathology of the eye is associated with Stargardt's and the therapeutic product is ELOVL Fatty Acid Elongase 4 (ELOVL4);
   (9) the pathology of the eye is associated with red-green color blindness and the therapeutic product is L opsin (OPN1LW);
   (10) the pathology of the eye is associated with red-green color blindness and the therapeutic product is M opsin (OPN1MW);
   (11) the pathology of the eye is associated with blue cone monochromacy and the therapeutic product is M opsin (OPN1MW);
   (12) the pathology of the eye is associated with Leber congenital amaurosis-1 (LCA 1) and the therapeutic product is Guanylate Cyclase 2D, Retinal (GUCY2D);
   (13) the pathology of the eye is associated with Leber congenital amaurosis-2 (LCA 2) and the therapeutic product is Retinoid Isomerohydrolase RPE65 (RPE65);
   (14) the pathology of the eye is associated with Leber congenital amaurosis-4 (LCA 4) and the therapeutic product is Aryl Hydrocarbon Receptor Interacting Protein Like 1 (AIPL1);
   (15) the pathology of the eye is associated with Leber congenital amaurosis-7 (LCA 7) and the therapeutic product is Cone-Rod Homeobox (CRX);
   (16) the pathology of the eye is associated with Leber congenital amaurosis-8 (LCA 8) and the therapeutic product is Crumbs Cell Polarity Complex Component 1 (CRB1);
   (17) the pathology of the eye is associated with Leber congenital amaurosis-9 (LCA 9) and the therapeutic product is Nicotinamide Nucleotide Adenylyltransferase 1 (NMNAT1);
   (18) the pathology of the eye is associated with Leber congenital amaurosis-10 (LCA 10) and the therapeutic product is Centrosomal Protein 290 (CEP290);
   (19) the pathology of the eye is associated with Leber congenital amaurosis-11 (LCA 11) and the therapeutic product is Inosine Monophosphate Dehydrogenase 1 (IMPDH1);
   (20) the pathology of the eye is associated with Leber congenital amaurosis-15 (LCA 15) and the therapeutic product is Tubby Like Protein 1 (TULP1);
   (21) the pathology of the eye is associated with LHON and the therapeutic product is Mitochondrially Encoded NADH Dehydrogenase 4 (MT-ND4);
   (22) the pathology of the eye is associated with LHON and the therapeutic product is Mitochondrially Encoded NADH Dehydrogenase 6 (MT-ND6);
   (23) the pathology of the eye is associated with choroideremia and the therapeutic product is Rab Escort Protein 1 (CHM);
   (24) the pathology of the eye is associated with X-linked retinoschisis (XLRS) and the therapeutic product is Retinoschisin (RS1);
   (25) the pathology of the eye is associated with Bardet-Biedl syndrome 1 and the therapeutic product is Bardet-Biedl Syndrome 1 (BBS1);
   (26) the pathology of the eye is associated with Bardet-Biedl syndrome 6 and the therapeutic product is McKusick-Kaufman Syndrome (MKKS);
   (27) the pathology of the eye is associated with Bardet-Biedl syndrome 10 and the therapeutic product is Bardet-Biedl Syndrome 10 (BBS10);
   (28) the pathology of the eye is associated with cone dystrophy and the therapeutic product is Guanylate Cyclase Activator 1A (GUCA1A);
   (29) the pathology of the eye is associated with optic atrophy and the therapeutic product is OPA1 Mitochondrial Dynamin Like GTPase (OPA1);
   (30) the pathology of the eye is associated with retinitis pigmentosa 1 and the therapeutic product is RP1 Axonemal Microtubule Associated (RP1);
   (31) the pathology of the eye is associated with retinitis pigmentosa 2 and the therapeutic product is RP2 Activator of ARL3 GTPase (RP2);
   (32) the pathology of the eye is associated with retinitis pigmentosa 7 and the therapeutic product is Peripherin 2 (PRPH2);
   (33) the pathology of the eye is associated with retinitis pigmentosa 11 and the therapeutic product is Pre-mRNA Processing Factor 31(PRPF31);
   (34) the pathology of the eye is associated with retinitis pigmentosa 13 and the therapeutic product is Pre-mRNA Processing Factor 8 (PRPF8);
   (35) the pathology of the eye is associated with retinitis pigmentosa 37 and the therapeutic product is Nuclear Receptor Subfamily 2 Group E Member 3 (NR2E3);
   (36) the pathology of the eye is associated with retinitis pigmentosa 38 and the therapeutic product is MER Proto-Oncogene, Tyrosine Kinase (MERTK);
   (37) the pathology of the eye is associated with retinitis pigmentosa 40 and the therapeutic product is Phosphodiesterase 6B (PDE6B);
   (38) the pathology of the eye is associated with retinitis pigmentosa 41 and the therapeutic product is Prominin 1 (PROM1);
   (39) the pathology of the eye is associated with retinitis pigmentosa 56 and the therapeutic product is Interphotoreceptor Matrix Proteoglycan 2 (IMPG2);
   (40) the pathology of the eye is associated with petinitis pigmentosa 62 and the therapeutic product is Male Germ Cell Associated Kinase (MAK);
   (41) the pathology of the eye is associated with retinitis pigmentosa 80 and the therapeutic product is Intraflagellar Transport 140 (IFT140); or
   (42) the pathology of the eye is associated with Best disease and the therapeutic product is Bestrophin 1 (BEST1).
31. The method of any one of paragraphs 1-11 and 15-18, wherein:
   (1) the pathology of the eye is associated with X-linked retinitis pigmentosa (XLRP) and the therapeutic product is Retinitis Pigmentosa GTPase Regulator (RPGR);
   (2) the pathology of the eye is associated with achromatopsia and the therapeutic product is Cyclic Nucleotide Gated Channel Beta 3 (CNGB3); or
   (3) the pathology of the eye is associated with achromatopsia and the therapeutic product is Cyclic Nucleotide Gated Channel Alpha 3 (CNGA3).
32. The method of any one of paragraphs 1-31, wherein the recombinant viral vector further comprises a nucleotide sequence encoding a promoter or an enhancer-promoter, which nucleotide sequence encoding the promoter or enhancer-promoter is operably linked to the nucleotide sequence encoding the therapeutic product, and wherein the promoter or enhancer-promoter is:
   (1) a CAG promoter;
   (2) a CBA promoter;
   (3) a CMV promoter;
   (4) a PR1.7 promoter;
   (5) a Rhodopsin Kinase (GRK1) photoreceptor-specific enhancer-promoter;
   (6) an hCARp promoter;
   (7) an hRKp;
   (8) a cone photoreceptor specific human arrestin 3 (ARR3) promoter;
   (9) a rhodopsin promoter;or
   (10) a U6 promoter.
33. The method of any one of paragraphs 1-11 and 13-15, wherein the recombinant viral vector further comprises a nucleotide sequence encoding a cone-specific promoter, which nucleotide sequence encoding the cone-specific promoter is operably linked to the nucleotide sequence encoding the therapeutic product, and wherein:
   (1) the pathology of the eye is associated with red-green color blindness and the therapeutic product is L opsin (OPN1LW);
   (2) the pathology of the eye is associated with red-green color blindness and the therapeutic product is M opsin (OPN1MW);
   (3) the pathology of the eye is associated with blue cone monochromacy and the therapeutic product is M opsin (OPN1MW);
   (4) the pathology of the eye is associated with cone dystrophy and the therapeutic product is Guanylate Cyclase Activator 1A (GUCA1A); or
   (5) the pathology of the eye is associated with blue cone monochromacy (BCM) and the therapeutic product is L opsin (OPN1LW).
34. The method of any one of paragraphs 1-33, wherein the administering step delivers a therapeutically effective amount of the therapeutic product to the retina of said human subject.
35. The method of paragraph 34, wherein the therapeutically effective amount of the therapeutic product is produced by human retinal cells of said human subject.
36. The method of paragraph 34, wherein the therapeutically effective amount of the therapeutic product is produced by human photoreceptor cells, horizontal cells, bipolar cells, amacrine cells, retina ganglion cells, and/or retinal pigment epithelial cells in the external limiting membrane of said human subject.
37. The method of paragraph 36, wherein the human photoreceptor cells are cone cells and/or rod cells.
38. The method of paragraph 36, wherein the retina ganglion cells are midget cells, parasol cells, bistratified cells, giant retina ganglion cells, photosensitive ganglion cells, and/or Müller glia.
39. The method of any one of paragraphs 1-38, wherein the recombinant viral vector is an rAAV vector.
40. The method of paragraph 39, wherein the recombinant viral vector is an rAAV8 vector.
41. The method of any one of paragraphs 1-40, which further comprises, after the administering step, a step of monitoring the post ocular injection thermal profile of the injected material in the eye using an infrared thermal camera.
42. The method of paragraph 41, wherein the infrared thermal camera is an FLIR T530 infrared thermal camera.
43. The method of any one of paragraphs 1-43, wherein the recombinant nucleotide expression vector is administered at a dose about 6.0% 10¹⁰ genome copies per eye.
44. The method of any one of paragraphs 1-43, wherein the recombinant nucleotide expression vector is administered at a dose about 1.6 × 10¹¹ genome copies per eye.
45. The method of any one of paragraphs 1-43, wherein the recombinant nucleotide expression vector is administered at a dose about 2.5 × 10¹¹ genome copies per eye.
46. The method of any one of paragraphs 1-43, wherein the recombinant nucleotide expression vector is administered at a dose about 5.0 × 10¹¹ genome copies per eye.
47. The method of any one of paragraphs 1-43, wherein the recombinant nucleotide expression vector is administered at a dose about 3.0 x 10¹² genome copies per eye.

### 5. BRIEF DESCRIPTION OF THE DRAWINGS

**FIG. 1****.** A suprachoroidal drug delivery device manufactured by Clearside^{®} Biomedical, Inc.
**FIG. 2****.** A subretinal drug delivery device comprising a catheter that can be inserted and tunneled through the suprachoroidal space toward the posterior pole, where a small needle injects into the subretinal space, manufactured by Janssen Pharmaceuticals, Inc.
**FIG. 3****.** Diagram of the human eye with cross-sectional view.
**FIGs. 4A-4D****.** Illustration of the posterior juxtascleral depot procedure.
**FIG. 5****.** Schematic of AAV8-antiVEGFfab genome.
**FIG. 6****.** Use of an infrared thermal camera to monitor thermal profile post suprachoroidal injection.
**FIGs. 7A and 7B****.** A micro volume injector drug delivery device manufactured by Altaviz.

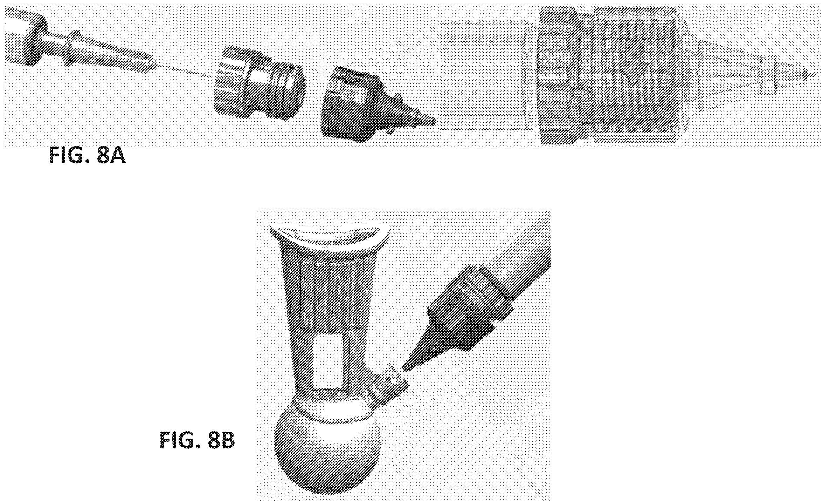

**FIGs. 8A and 8B****.** A drug delivery device manufactured by Visionisti OY. Specifically, FIG. 8A depicts the injection adapter, which is able to convert 30g short hypodermic needles into a suprachoroidal/subretinal needles. The device is able to control the length of the needle tip exposed from the distal tip of the adapter. Adjustments can be made at 10 µL. The device has the ability to adjust for suprachoroidal delivery and/or ab-externo subretinal delivery. FIG. 8B depicts a needle adaptor guide which is able to keep the lids open and hold the needle at the optimal angle and depth for delivery. The needle adapter is locked into the stabilizing device. The needle adapter is an all-in-one tool for standardized and optimized in-office suprachoroidal and/or subretinal injections.

### 6. DETAILED DESCRIPTION OF THE INVENTION

Provided herein are compositions and methods for the delivery of therapeutic products (such as therapeutic proteins (for example, antibodies), therapeutic RNAs (for example, shRNAs, siRNAs, and miRNAs), and therapeutic aptamers) to the retina/vitreal humour in the eyes of human subjects to treat pathologies of the eye, involving, for example, recombinant viral vectors such as recombinant adeno-associated virus (rAAV) vectors.

The therapeutic products can be, for example, therapeutic proteins (for example, antibodies), therapeutic RNAs (for example, shRNAs, siRNAs, and miRNAs), or therapeutic aptamers.

In a specific embodiment, the therapeutic products is a human protein or an antibody against a human protein. Antibodies include, but are not limited to, monoclonal antibodies, polyclonal antibodies, recombinantly produced antibodies, human antibodies, humanized antibodies, chimeric antibodies, synthetic antibodies, tetrameric antibodies comprising two heavy chain and two light chain molecules, antibody light chain monomers, antibody heavy chain monomers, antibody light chain dimers, antibody heavy chain dimers, antibody light chain-heavy chain pairs, intrabodies, heteroconjugate antibodies, monovalent antibodies, antigen-binding fragments of full-length antibodies, and fusion proteins of the above. Such antigen-binding fragments include, but are not limited to, single-domain antibodies (variable domain of heavy chain antibodies (VHHs) or nanobodies), Fabs, F(ab')₂s, and scFvs (single-chain variable fragments). In certain embodiment, the therapeutic product (for example, a therapeutic protein) is post-translationally modified. In a specific embodiment, the post-translational modification is specific to the cell type, to which the therapeutic product (for example, a therapeutic protein) is delivered using a specific route as described herein. Delivery may be accomplished via gene therapy - *e.g.,* by administering a recombinant viral vector or a recombinant DNA expression construct (collectively, a "recombinant vector") encoding an therapeutic product to the suprachoroidal space, subretinal space (with vitrectomy, or without vitrectomy (*e.g.,* with a catheter through the suprachoroidal space, or via peripheral injection), intraretinal space, vitreous cavity, and/or outer surface of the sclera (*i.e.,* juxtascleral administration) in the eye(s) of a human patient, to create a permanent depot in the eye that continuously supplies the therapeutic product (*e.g.,* a post-translationally modified therapeutic product).

### 6.1 METHODS FOR THE DELIVERY OF THERAPEUTIC PRODUCTS

In one aspect, provided herein is a method of subretinal administration without vitrectomy for treating a pathology of the eye, comprising administering to the subretinal space in the eye of a human subject in need of treatment a recombinant viral vector comprising a nucleotide sequence encoding a therapeutic product such that the therapeutic product is expressed and results in treatment of the pathology of the eye, wherein the method does not comprise performing a vitrectomy on the eye of said human patient. In certain embodiments, the administering step comprises administering to the subretinal space in the eye of said human subject the recombinant viral vector therapeutic product via the suprachoroidal space in the eye of said human subject. In certain embodiments, the administering step is by the use of a subretinal drug delivery device comprising a catheter that can be inserted and tunneled through the suprachoroidal space toward the posterior pole, where a small needle injects into the subretinal space. In certain embodiments, the administering step comprises inserting and tunneling the catheter of the subretinal drug delivery device through the suprachoroidal space.

In another aspect, provided herein is a method for treating a pathology of the eye, comprising administering to the subretinal space in the eye of a human subject in need of treatment a recombinant viral vector comprising a nucleotide sequence encoding a therapeutic product such that the therapeutic product is expressed and results in treatment of the pathology of the eye, wherein the method does not comprise performing a vitrectomy on the eye of said human patient. In certain embodiments, the administering step comprises administering to the subretinal space in the eye of said human subject the recombinant viral vector therapeutic product via the suprachoroidal space in the eye of said human subject. In certain embodiments, the administering step is by the use of a subretinal drug delivery device comprising a catheter that can be inserted and tunneled through the suprachoroidal space toward the posterior pole, where a small needle injects into the subretinal space. In certain embodiments, the administering step comprises inserting and tunneling the catheter of the subretinal drug delivery device through the suprachoroidal space.

In one aspect, provided herein is a method of subretinal administration with vitrectomy for treating a pathology of the eye, comprising administering to the subretinal space in the eye of a human subject in need of treatment a recombinant viral vector comprising a nucleotide sequence encoding a therapeutic product such that the therapeutic product is expressed and results in treatment of the pathology of the eye, wherein the method comprises performing a vitrectomy on the eye of said human patient. In certain embodiments, the vitrectomy is a partial vitrectomy.

In another aspect, provided herein is a method for treating a pathology of the eye, comprising administering to the subretinal space in the eye of a human subject in need of treatment a recombinant viral vector comprising a nucleotide sequence encoding a therapeutic product such that the therapeutic product is expressed and results in treatment of the pathology of the eye, wherein the method comprises performing a vitrectomy on the eye of said human patient. In certain embodiments, the vitrectomy is a partial vitrectomy.

In one aspect, provided herein is a method of suprachoroidal administration for treating a pathology of the eye, comprising administering to the suprachoroidal space in the eye of a human subject in need of treatment a recombinant viral vector comprising a nucleotide sequence encoding a therapeutic product such that the therapeutic product is expressed and results in treatment of the pathology of the eye. In certain embodiments, the administering step is by injecting the recombinant viral vector into the suprachoroidal space using a suprachoroidal drug delivery device. In certain embodiments, the suprachoroidal drug delivery device is a microinjector.

In another aspect, provided herein is a method for treating a pathology of the eye, comprising administering to the suprachoroidal space in the eye of a human subject in need of treatment a recombinant viral vector comprising a nucleotide sequence encoding a therapeutic product such that the therapeutic product is expressed and results in treatment of the pathology of the eye. In certain embodiments, the administering step is by injecting the recombinant viral vector into the suprachoroidal space using a suprachoroidal drug delivery device. In certain embodiments, the suprachoroidal drug delivery device is a microinjector.

In certain embodiments, delivery to the subretinal or suprachoroidal space can be performed using the methods and/or devices described and disclosed in International Publication Nos. WO 2016/042162, WO 2017/046358, WO 2017/158365, and WO 2017/158366, each of which is incorporated by reference in its entirety.

In one aspect, provided herein is a method of administration to the outer space of the sclera for treating a pathology of the eye, comprising administering to the outer surface of the sclera in the eye of a human subject in need of treatment a recombinant viral vector comprising a nucleotide sequence encoding a therapeutic product such that the therapeutic product is expressed and results in treatment of the pathology of the eye. In certain embodiments, the administering step is by the use of a juxtascleral drug delivery device that comprises a cannula whose tip can be inserted and kept in direct apposition to the scleral surface. In certain embodiments, the administering step comprises inserting and keeping the tip of the cannula in direct apposition to the scleral surface.

In another aspect, provided herein is a method for treating a pathology of the eye, comprising administering to the outer surface of the sclera in the eye of a human subject in need of treatment a recombinant viral vector comprising a nucleotide sequence encoding a therapeutic product such that the therapeutic product is expressed and results in treatment of the pathology of the eye. In certain embodiments, the administering step is by the use of a juxtascleral drug delivery device that comprises a cannula whose tip can be inserted and kept in direct apposition to the scleral surface. In certain embodiments, the administering step comprises inserting and keeping the tip of the cannula in direct apposition to the scleral surface

In one aspect, provided herein is a method of intravitreal administration for treating a pathology of the eye, comprising administering to the vitreous cavity in the eye of a human subject in need of treatment a recombinant viral vector comprising a nucleotide sequence encoding a therapeutic product such that the therapeutic product is expressed and results in treatment of the pathology of the eye. In certain embodiments, the administering step is by injecting the recombinant viral vector into the vitreous cavity using an intravitreal drug delivery device. In certain embodiments, the intravitreal drug delivery device is a microinjector. In another aspect, provided herein is a method for treating a pathology of the eye, comprising administering to the vitreous cavity in the eye of a human subject in need of treatment a recombinant viral vector comprising a nucleotide sequence encoding a therapeutic product such that the therapeutic product is expressed and results in treatment of the pathology of the eye. In certain embodiments, the administering step is by injecting the recombinant viral vector into the vitreous cavity using an intravitreal drug delivery device. In certain embodiments, the intravitreal drug delivery device is a microinjector.

In certain embodiments, the therapeutic product is not an anti-human vascular endothelial growth factor (hVEGF) antibody.

In certain embodiments, the pathology of the eye is not associated with neovascular age-related macular degeneration (nAMD) (also known as the "wet," neovascular form of AMD ("WAMD" or "wet AMD")).

In certain embodiments, the therapeutic product is an anti-hVEGF antibody.

In certain embodiments, the pathology of the eye is associated with nAMD.

In certain embodiments, the pathology of the eye is associated with nAMD and the therapeutic product is an anti-hVEGF antibody.

In one aspect, provided herein is a method of subretinal administration accompanied by vitrectomy for treating a pathology of the eye, comprising administering to the subretinal space in the eye of a human subject in need of treatment a recombinant viral vector comprising a nucleotide sequence encoding a therapeutic product such that the therapeutic product is expressed and results in treatment of the pathology of the eye, wherein the method comprises performing a vitrectomy on the eye of said human patient, and wherein the therapeutic product is not anti-human vascular endothelial growth factor (hVEGF) antibody. In certain embodiments, the pathology of the eye is an ocular disease or a disease involving multiple organs including the eye. In certain embodiments, the vitrectomy is a partial vitrectomy.

In another aspect, provided herein is a method for treating a pathology of the eye, comprising administering to the subretinal space in the eye of a human subject in need of treatment a recombinant viral vector comprising a nucleotide sequence encoding a therapeutic product such that the therapeutic product is expressed and results in treatment of the pathology of the eye, wherein the method comprises performing a vitrectomy on the eye of said human patient, and wherein the therapeutic product is not anti-human vascular endothelial growth factor (hVEGF) antibody. In certain embodiments, the pathology of the eye is an ocular disease or a disease involving multiple organs including the eye. In certain embodiments, the vitrectomy is a partial vitrectomy.

In one aspect, provided herein is a method of subretinal administration for treating a pathology of the eye, comprising administering to the subretinal space peripheral to the optic disc, fovea and macula located in the back of the eye of a human subject in need of treatment a recombinant viral vector comprising a nucleotide sequence encoding a therapeutic product such that the therapeutic product is expressed and results in treatment of the pathology of the eye, wherein the method does not comprise performing a vitrectomy on the eye of said human patient. In certain embodiments, the injecting step is by transvitreal injection. In certain embodiments, the method of transvitreal administration results in uniform expression of the therapeutic product throughout the eye (*e.g.* the expression level at the site of injection varies by less than 5%, 10%, 20%, 30%, 40%, or 50% as compared to the expression level at other areas of the eye). In certain embodiments, the transvitreal injection comprises inserting a sharp needle into the sclera via the superior or inferior side of the eye and passing the sharp needle all the way through the vitreous to inject the recombinant viral vector to the subretinal space on the other side. In certain embodiments, a needle is inserted at the 2 or 10 o'clock position. In certain embodiments, the transvitreal injection comprises inserting a trochar into the sclera and inserting a cannula through the trochar and through the vitreous to inject the recombinant viral vector to the subretinal space on the other side. In certain embodiments, the therapeutic product is an anti-hVEGF antibody. In certain embodiments, the anti-hVEGF antibody is an anti-hVEGF antigen-binding fragment. In certain embodiments, the anti-hVEGF antigen-binding fragment is a Fab, F(ab')₂, or single chain variable fragment (scFv). In certain embodiments, the anti-hVEGF antibody comprises a heavy chain comprising the amino acid sequence of SEQ ID NO:2 or SEQ ID NO:4, and a light chain comprising the amino acid sequence of SEQ ID NO: 1, or SEQ ID NO:3. In certain embodiments, wherein the anti-hVEGF antibody comprises light chain CDRs 1-3 of SEQ ID NOs: 14-16 and heavy chain CDRs 1-3 of SEQ ID NOs: 17-19 or SEQ ID NOs:20, 18, and 21. In certain embodiments, wherein the pathology of the eye is associated with nAMD, dry age-related macular degeneration (dry AMD), retinal vein occlusion (RVO), diabetic macular edema (DME), or diabetic retinopathy (DR). In certain embodiments, the pathology of the eye is associated with nAMD.

In another aspect, provided herein is a method for treating a pathology of the eye, comprising administering to the subretinal space peripheral to the optic disc, fovea and macula located in the back of the eye of a human subject in need of treatment a recombinant viral vector comprising a nucleotide sequence encoding a therapeutic product such that the therapeutic product is expressed and results in treatment of the pathology of the eye, wherein the method does not comprise performing a vitrectomy on the eye of said human patient. In certain embodiments, the injecting step is by transvitreal injection. In certain embodiments, the method of transvitreal administration results in uniform expression of the therapeutic product throughout the eye (*e.g.* the expression level at the site of injection varies by less than 5%, 10%, 20%, 30%, 40%, or 50% as compared to the expression level at other areas of the eye). In certain embodiments, the transvitreal injection comprises inserting a sharp needle into the sclera via the superior or inferior side of the eye and passing the sharp needle all the way through the vitreous to inject the recombinant viral vector to the subretinal space on the other side. In certain embodiments, a needle is inserted at the 2 or 10 o'clock position. In certain embodiments, the transvitreal injection comprises inserting a trochar into the sclera and inserting a cannula through the trochar and through the vitreous to inject the recombinant viral vector to the subretinal space on the other side. In certain embodiments, the therapeutic product is an anti-hVEGF antibody. In certain embodiments, the anti-hVEGF antibody is an anti-hVEGF antigen-binding fragment. In certain embodiments, the anti-hVEGF antigen-binding fragment is a Fab, F(ab')₂, or single chain variable fragment (scFv). In certain embodiments, the anti-hVEGF antibody comprises a heavy chain comprising the amino acid sequence of SEQ ID NO:2 or SEQ ID NO:4, and a light chain comprising the amino acid sequence of SEQ ID NO: 1, or SEQ ID NO:3. In certain embodiments, wherein the anti-hVEGF antibody comprises light chain CDRs 1-3 of SEQ ID NOs: 14-16 and heavy chain CDRs 1-3 of SEQ ID NOs: 17-19 or SEQ ID NOs:20, 18, and 21. In certain embodiments, wherein the pathology of the eye is associated with nAMD, dry age-related macular degeneration (dry AMD), retinal vein occlusion (RVO), diabetic macular edema (DME), or diabetic retinopathy (DR). In certain embodiments, the pathology of the eye is associated with nAMD.

In certain embodiments of the methods described herein, the administering step delivers a therapeutically effective amount of the therapeutic product to the retina of said human subject.

In certain embodiments of the methods described herein, the therapeutically effective amount of the therapeutic product is produced by human retinal cells of said human subject.

In certain embodiments of the methods described herein, the therapeutically effective amount of the therapeutic product is produced by human photoreceptor cells, horizontal cells, bipolar cells, amacrine cells, retina ganglion cells, and/or retinal pigment epithelial cells in the external limiting membrane of said human subject.

In certain embodiments of the methods described herein, the human photoreceptor cells are cone cells and/or rod cells.

In certain embodiments of the methods described herein, the retina ganglion cells are midget cells, parasol cells, bistratified cells, giant retina ganglion cells, photosensitive ganglion cells, and/or Müller glia.

In certain embodiments of the methods described herein, the recombinant viral vector is an rAAV vector (*e.g.,* an rAAV8, rAAV2, rAAV2tYF, or rAAV5 vector).

In certain embodiments of the methods described herein, wherein the recombinant viral vector is an rAAV8 vector.

In certain embodiments of the methods described herein, delivering to the eye comprises delivering to the retina, choroid, and/or vitreous humor of the eye.

### 6.1.1 POST-TRANSLATIONAL MODIFICATION

In certain embodiments, the therapeutic product (for example, a therapeutic protein) is post-translationally modified. In a specific embodiment, the post-translational modification is specific to the cell type, to which the therapeutic product (for example, a therapeutic protein) is delivered using a specific route as described herein.

In a specific embodiment, the post-translational modification is glycosylation. In another specific embodiment, the post-translational modification is tyrosine sulfation. In another specific embodiment, the post-translational modification is a phosphorylation. In another specific embodiment, the post-translational modification is a ADP-ribosylation. In another specific embodiment, the post-translational modification is a prenylation. In another specific embodiment, the post-translational modification is a myristoylation or palmitylation. In another specific embodiment, the post-translational modification is ubiquitination. In another specific embodiment, the post-translational modification is sentrinization. In another specific embodiment, the post-translational modification is a ubiquitination-like protein modification.

In a specific embodiment, the therapeutic product is post-translationally modified upon expression from the recombinant vector in a human immortalized retina-derived cell.

In a specific embodiment, the administration of the recombinant vector results in the formation of a depot that releases the therapeutic product containing a post-translational modification.

In a specific embodiment, the recombinant vector, when used to transduce a retina-derived cell in culture results in production of the therapeutic product containing a post-translational modification.

The post-translational modification can be detected by any method known in the art for detecting post-translational modifications, for example, western blot, chromatography, or flow cytometry.

In a specific embodiment, the post-translation can be detected by *in vivo* labeling of cellular substrate pools with radioactive substrate or substrate precursor molecules, which result in incorporation of radiolabeled moieties, including, but not limited to, phosphate, fatty acyl (*e.g.* myristoyl, or palmityl), sentrin, methyl, acetyl, hydroxyl, iodine, flavin, ubiquitin or ADP-ribosyls, to therapeutic product. Analysis of modified proteins is typically performed by electrophoresis and autoradiography, with specificity enhanced by immunoprecipitation of proteins of interest prior to electrophoresis.

In a specific embodiment, the post-translation can be detected by enzymatic incorporation of a labeled moiety (including, but not limited to, radioactive, luminescent, or fluorescent label) into a therapeutic product in vitro to estimate the state of modification in vivo.

In a specific embodiment, the post-translation can be detected by analyzing the alteration in electrophoretic mobility of modified therapeutic product (*e.g*., glycosylated or ubiquitinated) compared with unmodified therapeutic product.

In a specific embodiment, the post-translation can be detected by thin-layer chromatography of radiolabeled fatty acids extracted from the therapeutic product.

In a specific embodiment, the post-translation can be detected by partitioning of therapeutic product into detergent-rich or detergent layer by phase separation, and the effects of enzyme treatment of the therapeutic product on the partitioning between aqueous and detergent-rich environments.

In a specific embodiment, the post-translation can be detected by antibody recognition of the modified form of the protein, *e.g*., by western blot, or flow cytometry.

### 6.1.2 CONSTRUCTS AND FORMULATIONS

For use in the methods provided herein are recombinant viral vectors or other recombinant DNA expression constructs (collectively, "recombinant vectors") encoding an therapeutic product. The recombinant viral vectors and other DNA expression constructs provided herein include any suitable ones for delivery of therapeutic products (such as therapeutic proteins (for example, antibodies), therapeutic RNAs (for example, shRNAs, siRNAs, and miRNAs), and therapeutic aptamers)) to a target cell (*e.g.,* retinal pigment epithelial cells). The means of delivery of a therapeutic product include recombinant viral vectors, liposomes, other lipid-containing complexes, other macromolecular complexes, synthetic modified mRNA, unmodified mRNA, small molecules, non-biologically active molecules (*e.g*., gold particles), polymerized molecules (*e.g*., dendrimers), naked DNA, plasmids, phages, transposons, cosmids, or episomes. In some embodiments, the vector is a targeted vector, *e.g.,* a vector targeted to retinal pigment epithelial cells.

In some aspects, the disclosure provides for a nucleic acid for use, wherein the nucleic acid encodes a therapeutic product operatively linked to a promoter or enhancer-promoter described herein.

In certain embodiments, provided herein are recombinant vectors that comprise one or more nucleic acids (*e.g*. polynucleotides). The nucleic acids may comprise DNA, RNA, or a combination of DNA and RNA. In certain embodiments, the DNA comprises one or more of the sequences selected from the group consisting of promoter sequences, the sequence encoding the therapeutic product of interest, untranslated regions, and termination sequences. In certain embodiments, recombinant vectors provided herein comprise a promoter operably linked to the sequence encoding the therapeutic product of interest.

In certain embodiments, nucleic acids (*e.g*., polynucleotides) and nucleic acid sequences disclosed herein may be codon-optimized, for example, via any codon-optimization technique known to one of skill in the art (*see, e.g.,* review by Quax et al., 2015, Mol Cell 59:149-161).

### (a) mRNA

In certain embodiments, the recombinant vectors provided herein comprise modified mRNA encoding for the therapeutic product of interest. The synthesis of modified and unmodified mRNA for delivery of a therapeutic product to cells of the eye, for example, to retinal pigment epithelial cells, is taught, for example, in Hansson et al., J. Biol. Chem., 2015, 290(9):5661-5672, which is incorporated by reference herein in its entirety. In certain embodiments, provided herein is a modified mRNA encoding for a therapeutic product moiety.

### (b) shRNAs, siRNAs, and miRNAs

In certain embodiments, the recombinant vectors provided herein comprise a nucleotide sequence encoding for a therapeutic product that is an shRNA, siRNA, or miRNA.

### (c) Recombinant viral vectors

Recombinant viral vectors include recombinant adenovirus, adeno-associated virus (AAV, *e.g*., AAV1, AAV2, AAV2tYF, AAV3, AAV4, AAV5, AAV6, AAV7, AAV8, AAV9, AAV10, AAV11, and AAVrh10), lentivirus, helper-dependent adenovirus, herpes simplex virus, poxvirus, hemagglutinin virus of Japan (HVJ), alphavirus, vaccinia virus, and retrovirus vectors. Retroviral vectors include murine leukemia virus (MLV)- and human immunodeficiency virus (HIV)-based vectors. Alphavirus vectors include semliki forest virus (SFV) and sindbis virus (SIN). In certain embodiments, the recombinant viral vectors provided herein are altered such that they are replication-deficient in humans. In certain embodiments, the recombinant viral vectors are hybrid vectors, *e.g*., an AAV vector placed into a "helpless" adenoviral vector. In certain embodiments, provided herein are recombinant viral vectors comprising a viral capsid from a first virus and viral envelope proteins from a second virus. In specific embodiments, the second virus is vesicular stomatitus virus (VSV). In more specific embodiments, the envelope protein is VSV-G protein.

In certain embodiments, the recombinant viral vectors provided herein are HIV based viral vectors. In certain embodiments, HIV-based vectors provided herein comprise at least two polynucleotides, wherein the gag and pol genes are from an HIV genome and the env gene is from another virus.

In certain embodiments, the recombinant viral vectors provided herein are herpes simplex virus-based viral vectors. In certain embodiments, herpes simplex virus-based vectors provided herein are modified such that they do not comprise one or more immediately early (IE) genes, rendering them non-cytotoxic.

In certain embodiments, the recombinant viral vectors provided herein are MLV based viral vectors. In certain embodiments, MLV-based vectors provided herein comprise up to 8 kb of heterologous DNA in place of the viral genes.

In certain embodiments, the recombinant viral vectors provided herein are lentivirus-based viral vectors. In certain embodiments, lentiviral vectors provided herein are derived from human lentiviruses. In certain embodiments, lentiviral vectors provided herein are derived from non-human lentiviruses. In certain embodiments, lentiviral vectors provided herein are packaged into a lentiviral capsid. In certain embodiments, lentiviral vectors provided herein comprise one or more of the following elements: long terminal repeats, a primer binding site, a polypurine tract, att sites, and an encapsidation site.

In certain embodiments, the recombinant viral vectors provided herein are alphavirus-based viral vectors. In certain embodiments, alphavirus vectors provided herein are recombinant, replication-defective alphaviruses. In certain embodiments, alphavirus replicons in the alphavirus vectors provided herein are targeted to specific cell types by displaying a functional heterologous ligand on their virion surface.

In certain embodiments, the recombinant viral vectors provided herein are AAV based viral vectors. In preferred embodiments, the recombinant viral vectors provided herein are AAV8 based viral vectors. In certain embodiments, the AAV8 based viral vectors provided herein retain tropism for retinal cells. In certain embodiments, the AAV-based vectors provided herein encode the AAV rep gene (required for replication) and/or the AAV cap gene (required for synthesis of the capsid proteins). Multiple AAV serotypes have been identified. In certain embodiments, AAV-based vectors provided herein comprise components from one or more serotypes of AAV. In certain embodiments, AAV based vectors provided herein comprise capsid components from one or more of AAV1, AAV2, AAV2tYF, AAV3, AAV4, AAV5, AAV6, AAV7, AAV8, AAV9, AAV10, AAV11, or AAVrh10. In preferred embodiments, AAV based vectors provided herein comprise components from one or more of AAV8, AAV9, AAV10, AAV11, or AAVrh10 serotypes.

Provided in particular embodiments are AAV8 vectors comprising a viral genome comprising an expression cassette for expression of the therapeutic product, under the control of regulatory elements and flanked by ITRs and a viral capsid that has the amino acid sequence of the AAV8 capsid protein or is at least 95%, 96%, 97%, 98%, 99% or 99.9% identical to the amino acid sequence of the AAV8 capsid protein (SEQ ID NO: 48) while retaining the biological function of the AAV8 capsid. In certain embodiments, the encoded AAV8 capsid has the sequence of SEQ ID NO: 48 with 1, 2, 3, 4, 5, 6, 7, 8, 9, 10, 11, 12, 13, 14, 15, 16, 17, 18, 19, 20, 21, 22, 23, 24, 25, 26, 27, 28, 29 or 30 amino acid substitutions and retaining the biological function of the AAV8 capsid.

In certain embodiments, the AAV that is used in the methods described herein is Anc80 or Anc80L65, as described in Zinn et al., 2015, Cell Rep. 12(6): 1056-1068, which is incorporated by reference in its entirety. In certain embodiments, the AAV that is used in the methods described herein comprises one of the following amino acid insertions: LGETTRP or LALGETTRP, as described in United States Patent Nos. 9,193,956; 9458517; and 9,587,282 and US patent application publication no. 2016/0376323, each of which is incorporated herein by reference in its entirety. In certain embodiments, the AAV that is used in the methods described herein is AAV.7m8, as described in United States Patent Nos. 9,193,956; 9,458,517; and 9,587,282 and US patent application publication no. 2016/0376323, each of which is incorporated herein by reference in its entirety. In certain embodiments, the AAV that is used in the methods described herein is any AAV disclosed in United States Patent No. 9,585,971, such as AAV-PHP.B. In certain embodiments, the AAV that is used in the methods described herein is an AAV disclosed in any of the following patents and patent applications, each of which is incorporated herein by reference in its entirety: United States Patent Nos. 7,906,111; 8,524,446; 8,999,678; 8,628,966; 8,927,514; 8,734,809; US 9,284,357; 9,409,953; 9,169,299; 9,193,956; 9458517; and 9,587,282 US patent application publication nos. 2015/0374803; 2015/0126588; 2017/0067908; 2013/0224836; 2016/0215024; 2017/0051257; and International Patent Application Nos. PCT/US2015/034799; PCT/EP2015/053335.

AAV8-based viral vectors are used in certain embodiments of the methods described herein. Nucleic acid sequences of AAV based viral vectors and methods of making recombinant AAV and AAV capsids are taught, for example, in United States Patent No. 7,282,199 B2, United States Patent No. 7,790,449 B2, United States Patent No. 8,318,480 B2, United States Patent No. 8,962,332 B2 and International Patent Application No. PCT/EP2014/076466, each of which is incorporated herein by reference in its entirety. In one aspect, provided herein are AAV (e.g., AAV8)-based viral vectors encoding a therapeutic product.

In certain embodiments, a single-stranded AAV (ssAAV) may be used supra. In certain embodiments, a self-complementary vector, *e.g.,* scAAV, may be used (*see, e.g.,* Wu, 2007, Human Gene Therapy, 18(2):171-82, McCarty et al, 2001, Gene Therapy, Vol 8, Number 16, Pages 1248-1254; and U.S. Patent Nos. 6,596,535; 7,125,717; and 7,456,683, each of which is incorporated herein by reference in its entirety).

In certain embodiments, the recombinant viral vectors used in the methods described herein is a recombinant adenovirus vector.. The recombinant adenovirus can be a first generation vector, with an E1 deletion, with or without an E3 deletion, and with the expression cassette inserted into either deleted region. The recombinant adenovirus can be a second generation vector, which contains full or partial deletions of the E2 and E4 regions. A helper-dependent adenovirus retains only the adenovirus inverted terminal repeats and the packaging signal (phi). The therapeutic product is inserted between the packaging signal and the 3'ITR, with or without stuffer sequences to keep the genome close to wild-type size of approx. 36 kb. An exemplary protocol for production of adenoviral vectors may be found in Alba et al., 2005, "Gutless adenovirus: last generation adenovirus for gene therapy," Gene Therapy 12:S18-S27, which is incorporated by reference herein in its entirety.

In certain embodiments, the recombinant viral vectors used in the methods described herein are lentivirus based viral vectors. Four plasmids are used to make the construct: Gag/pol sequence containing plasmid, Rev sequence containing plasmids, Envelope protein containing plasmid (*i.e.* VSV-G), and Cis plasmid with the packaging elements and the therapeutic product containing plasmid.

For lentiviral vector production, the four plasmids are co-transfected into cells (*i.e.,* HEK293 based cells), whereby polyethylenimine or calcium phosphate can be used as transfection agents, among others. The lentivirus is then harvested in the supernatant (lentiviruses need to bud from the cells to be active, so no cell harvest needs/should be done). The supernatant is filtered (0.45 µm) and then magnesium chloride and benzonase added. Further downstream processes can vary widely, with using TFF and column chromatography being the most GMP compatible ones. Others use ultracentrifugation with/without column chromatography. Exemplary protocols for production of lentiviral vectors may be found in Lesch et al., 2011, "Production and purification of lentiviral vector generated in 293T suspension cells with baculoviral vectors," Gene Therapy 18:531-538, and Ausubel et al., 2012, "Production of CGMP-Grade Lentiviral Vectors," Bioprocess Int. 10(2):32-43, both of which are incorporated by reference herein in their entireties.

### (d) Promoters and Modifiers of Gene Expression

In certain embodiments, the recombinant vectors provided herein comprise components that modulate delivery or expression of the therapeutic product (*e.g.,* "expression control elements"). In certain embodiments, the recombinant vectors provided herein comprise components that modulate expression of the therapeutic product. In certain embodiments, the recombinant vectors provided herein comprise components that influence binding or targeting to cells. In certain embodiments, the recombinant vectors provided herein comprise components that influence the localization of the polynucleotide encoding the therapeutic product within the cell after uptake. In certain embodiments, the recombinant vectors provided herein comprise components that can be used as detectable or selectable markers, *e.g*., to detect or select for cells that have taken up the polynucleotide encoding the therapeutic product.

In certain embodiments, the recombinant vectors provided herein comprise one or more promoters. In certain embodiments, the promoter is a constitutive promoter. In certain embodiments, the promoter is an inducible promoter. Inducible promoters may be preferred so that expression of the therapeutic product may be turned on and off as desired for therapeutic efficacy. Such promoters include, for example, hypoxia-induced promoters and drug inducible promoters, such as promoters induced by rapamycin and related agents. Hypoxia-inducible promoters include promoters with HIF binding sites, *see,* for example, Schödel, et al., 2011, Blood 117(23):e207-e217 and Kenneth and Rocha, 2008, Biochem J. 414:19-29, each of which is incorporated by reference for teachings of hypoxia-inducible promoters. In addition, hypoxia-inducible promoters that may be used in the constructs include the erythropoietin promoter and N-WASP promoter (*see,* Tsuchiya, 1993, J. Biochem. 113:395 for disclosure of the erythropoietin promoter and Salvi, 2017, Biochemistry and Biophysics Reports 9:13-21 for disclosure of N-WASP promoter, both of which are incorporated by reference for the teachings of hypoxia-induced promoters). Alternatively, the recombinant vectors may contain drug inducible promoters, for example promoters inducible by administration of rapamycin and related analogs (*see,* for example, International Patent Application Publication Nos. WO94/18317, WO 96/20951, WO 96/41865, WO 99/10508, WO 99/10510, WO 99/36553, and WO 99/41258, and U.S. Patent No. US 7,067,526 (disclosing rapamycin analogs), which are incorporated by reference herein for their disclosure of drug inducible promoters). In certain embodiments the promoter is a hypoxia-inducible promoter. In certain embodiments, the promoter comprises a hypoxia-inducible factor (HIF) binding site. In certain embodiments, the promoter comprises a HIF-1α binding site. In certain embodiments, the promoter comprises a HIF-2α binding site. In certain embodiments, the HIF binding site comprises an RCGTG motif. For details regarding the location and sequence of HIF binding sites, *see, e.g.,* Schödel, et al., Blood, 2011, 117(23):e207-e217, which is incorporated by reference herein in its entirety. In certain embodiments, the promoter comprises a binding site for a hypoxia induced transcription factor other than a HIF transcription factor. In certain embodiments, the recombinant vectors provided herein comprise one or more IRES sites that is preferentially translated in hypoxia. For teachings regarding hypoxia-inducible gene expression and the factors involved therein, *see, e.g.,* Kenneth and Rocha, Biochem J., 2008, 414:19-29, which is incorporated by reference herein in its entirety.

In certain embodiments, the promoter is a CB7 promoter (see Dinculescu et al., 2005, Hum Gene Ther 16: 649-663, incorporated by reference herein in its entirety). In certain embodiments, the CB7 promoter includes other expression control elements that enhance expression of the therapeutic product driven by the vector, *e.g*.(1) a CAG promoter; (2) a CBA promoter; (3) a CMV promoter; (4) a 1.7-kb red cone opsin promoter (PR1.7 promoter); (5) a Rhodopsin Kinase (GRK1) photoreceptor-specific enhancer-promoter (Young et al., 2003, Retinal Cell Biology; 44:4076-4085); (6) an hCARp promoter, which is a human cone arrestin promoter; (7) an hRKp, which is a rhodopsin kinase promoter; (8) a cone photoreceptor specific human arrestin 3 (ARR3) promoter; (9) a rhodopsin promoter; and (10) a U6 promoter (in particular when the therapeutic product is a small RNA such as shRNA or siRNA).

In certain embodiments, the other expression control elements include chicken β-actin intron and/or rabbit β-globin polA signal. In certain embodiments, the promoter comprises a TATA box. In certain embodiments, the promoter comprises one or more elements. In certain embodiments, the one or more promoter elements may be inverted or moved relative to one another. In certain embodiments, the elements of the promoter are positioned to function cooperatively. In certain embodiments, the elements of the promoter are positioned to function independently. In certain embodiments, the recombinant vectors provided herein comprise one or more promoters selected from the group consisting of the human CMV immediate early gene promoter, the SV40 early promoter, the Rous sarcoma virus (RS) long terminal repeat, and rat insulin promoter. In certain embodiments, the recombinant vectors provided herein comprise one or more long terminal repeat (LTR) promoters selected from the group consisting of AAV, MLV, MMTV, SV40, RSV, HIV-1, and HIV-2 LTRs. In certain embodiments, the recombinant vectors provided herein comprise one or more tissue specific promoters (*e.g.,* a retinal pigment epithelial cell-specific promoter). In certain embodiments, the recombinant vectors provided herein comprise a RPE65 promoter. In certain embodiments, the recombinant vectors provided herein comprise a VMD2 promoter.

In certain embodiments, the recombinant vectors provided herein comprise one or more regulatory elements other than a promoter. In certain embodiments, the recombinant vectors provided herein comprise an enhancer. In certain embodiments, the recombinant vectors provided herein comprise a repressor. In certain embodiments, the recombinant vectors provided herein comprise an intron or a chimeric intron. In certain embodiments, the recombinant vectors provided herein comprise a polyadenylation sequence.

### (e) Signal Peptides

In certain embodiments wherein the therapeutic product is a therapeutic protein, the recombinant vectors provided herein comprise components that modulate protein delivery. In certain embodiments, the recombinant vectors provided herein comprise one or more signal peptides. Signal peptides may also be referred to herein as "leader sequences" or "leader peptides". In certain embodiments, the signal peptides allow for the therapeutic product to achieve the proper packaging (*e.g*. glycosylation) in the cell. In certain embodiments, the signal peptides allow for the therapeutic product to achieve the proper localization in the cell. In certain embodiments, the signal peptides allow for the therapeutic product to achieve secretion from the cell. Examples of signal peptides to be used in connection with the recombinant vectors and therapeutic products provided herein may be found in Table 1.

**Table 1. Signal peptides for use with the vectors provided herein.**

| **SEQ ID NO.** | **Signal Peptide** | **Sequence** |
|---|---|---|
| 5 | VEGF-A signal peptide | MNFLLSWVHW SLALLLYLHH AKWSQA |
| 6 | Fibulin-1 signal peptide | MERAAPSRRV PLPLLLLGGL ALLAAGVDA |
| 7 | Vitronectin signal peptide | MAPLRPLLIL ALLAWVALA |
| 8 | Complement Factor H signal peptide | MRLLAKIICLMLWAICVA |
| 9 | Opticin signal peptide | MRLLAFLSLL ALVLQETGT |
| 22 | Albumin signal peptide | MKWVTFISLLFLFSSAYS |
| 23 | Chymotrypsinogen signal peptide | MAFLWLLSCWALLGTTFG |
| 24 | Interleukin-2 signal peptide | MYRMQLLSCIALILALVTNS |
| 25 | Trypsinogen-2 signal peptide | MNLLLILTFVAAAVA |

### (f) Polycistronic Messages - IRES and F2A linkers

Internal ribosome entry sites. A single construct can be engineered to encode two peptides (for example, both the heavy and light chains of an antibody) separated by a cleavable linker or IRES so that the two peptides (for example, separate heavy and light chain polypeptides) are expressed by the transduced cells. In certain embodiments, the recombinant vectors provided herein provide polycistronic (*e.g.,* bicistronic) messages. For example, the recombinant vector can comprise a nucleotide sequence encoding two peptides (for example, the heavy and light chains of an antibody) separated by an internal ribosome entry site (IRES) elements (for example, the use of IRES elements to create bicistronic vectors *see, e.g.,* Gurtu et al., 1996, Biochem. Biophys. Res. Comm. 229(1):295-8, which is herein incorporated by reference in its entirety). IRES elements bypass the ribosome scanning model and begin translation at internal sites. The use of IRES in AAV is described, for example, in Furling et al., 2001, Gene Ther 8(11): 854-73, which is herein incorporated by reference in its entirety. In certain embodiments, the bicistronic message is contained within a recombinant vector with a restraint on the size of the polynucleotide(s) therein. In certain embodiments, the bicistronic message is contained within an AAV virus-based vector (*e.g.,* an AAV8-based vector).

Furin-F2A linkers. In other embodiments, the recombinant vectors provided herein comprise a nucleotide sequence encoding two peptides (for example, the heavy and light chains of an antibody) separated by a cleavable linker such as the self-cleaving furin/F2A (F/F2A) linkers (Fang et al., 2005, Nature Biotechnology 23: 584-590, and Fang, 2007, Mol Ther 15: 1153-9, each of which is incorporated by reference herein in its entirety).

For example, a furin-F2A linker may be incorporated into an expression cassette to separate the coding sequences of the two peptides (for example, the heavy and light chain coding sequences), resulting in a construct with the structure:
Leader - Peptide A (for example, Heavy chain of an antibody) - Furin site - F2A site - Leader - Peptide B (for example, Light chain of an antibody) - PolyA.

The F2A site, with the amino acid sequence LLNFDLLKLAGDVESNPGP (SEQ ID NO: 26) is self-processing, resulting in "cleavage" between the final G and P amino acid residues. Additional linkers that could be used include but are not limited to:
- T2A:(GSG) E G R G S L L T C G D V E E N P **G P** (SEQ ID NO: 27);
- P2A: (GSG) A T N F S L L K Q A G D V E E N P **G P** (SEQ ID NO: 28);
- E2A: (GSG) QC TNY ALL K L A G D V E S N P **G P** (SEQ ID NO: 29);
- F2A: (GSG) V K Q T L N F D L L K L A G D V E S N P **G P** (SEQ ID NO: 30).

A peptide bond is skipped when the ribosome encounters the F2A sequence in the open reading frame, resulting in the termination of translation, or continued translation of the downstream sequence (the second peptide). This self-processing sequence results in a string of additional amino acids at the end of the C-terminus of the first peptide. However, such additional amino acids are then cleaved by host cell Furin at the furin sites, located immediately prior to the F2A site and after the sequence of the first peptide, and further cleaved by carboxypeptidases. The resultant first peptide may have one, two, three, or more additional amino acids included at the C-terminus, or it may not have such additional amino acids, depending on the sequence of the Furin linker used and the carboxypeptidase that cleaves the linker in vivo (*See, e.g.,* Fang et al., 17 April 2005, Nature Biotechnol. Advance Online Publication; Fang et al., 2007, Molecular Therapy 15(6):1153-1159; Luke, 2012, Innovations in Biotechnology, Ch. 8, 161-186). Furin linkers that may be used comprise a series of four basic amino acids, for example, RKRR, RRRR, RRKR, or RKKR. Once this linker is cleaved by a carboxypeptidase, additional amino acids may remain, such that an additional zero, one, two, three or four amino acids may remain on the C-terminus of the first peptide, for example, R, RR, RK, RKR, RRR, RRK, RKK, RKRR, RRRR, RRKR, or RKKR. In certain embodiments, one the linker is cleaved by an carboxypeptidase, no additional amino acids remain. In certain embodiments, the furin linker has the sequence R-X-K/R-R, such that the additional amino acids on the C-terminus of the first peptide are R, RX, RXK, RXR, RXKR, or RXRR, where X is any amino acid, for example, alanine (A). In certain embodiments, no additional amino acids may remain on the C-terminus of the first peptide.

In certain embodiments, an expression cassette described herein is contained within a recombinant vector with a restraint on the size of the polynucleotide(s) therein. In certain embodiments, the expression cassette is contained within an AAV virus-based vector (e.g., an AAV8-based vector).

### (g) Untranslated regions

In certain embodiments wherein the therapeutic product is a therapeutic protein, the recombinant vectors provided herein comprise one or more untranslated regions (UTRs), *e.g.,* 3' and/or 5' UTRs. In certain embodiments, the UTRs are optimized for the desired level of protein expression. In certain embodiments, the UTRs are optimized for the half-life of the mRNA encoding the therapeutic protein. In certain embodiments, the UTRs are optimized for the stability of the mRNA encoding the therapeutic protein. In certain embodiments, the UTRs are optimized for the secondary structure of the mRNA encoding the therapeutic protein.

### (h) Inverted terminal repeats

In certain embodiments, the recombinant viral vectors provided herein comprise one or more inverted terminal repeat (ITR) sequences. ITR sequences may be used for packaging the recombinant therapeutic product expression cassette into the virion of the recombinant viral vector. In certain embodiments, the ITR is from an AAV, *e.g.,* AAV8 or AAV2 (*see, e.g.,* Yan et al., 2005, J. Virol., 79(1):364-379; United States Patent No. 7,282,199 B2, United States Patent No. 7,790,449 B2, United States Patent No. 8,318,480 B2, United States Patent No. 8,962,332 B2 and International Patent Application No. PCT/EP2014/076466, each of which is incorporated herein by reference in its entirety).

### (i) Therapeutic Product

The therapeutic products can be, for example, therapeutic proteins (for example, antibodies), therapeutic RNAs (for example, shRNAs, siRNAs, and miRNAs), or therapeutic aptamers. Antibodies include, but are not limited to, monoclonal antibodies, polyclonal antibodies, recombinantly produced antibodies, human antibodies, humanized antibodies, chimeric antibodies, synthetic antibodies, tetrameric antibodies comprising two heavy chain and two light chain molecules, antibody light chain monomers, antibody heavy chain monomers, antibody light chain dimers, antibody heavy chain dimers, antibody light chain-heavy chain pairs, intrabodies, heteroconjugate antibodies, monovalent antibodies, antigen-binding fragments of full-length antibodies, and fusion proteins of the above. Such antigen-binding fragments include, but are not limited to, single-domain antibodies (variable domain of heavy chain antibodies (VHHs) or nanobodies), Fabs, F(ab')2s, and scFvs (single-chain variable fragments).

In certain embodiments of the methods described herein, the therapeutic product is: (1) anti-human vascular endothelial growth factor (hVEGF) antibody or aptamer, (2) an anti-hVEGF antigen-binding fragment; (3) anti-hVEGF antigen-binding fragment is a Fab, F(ab')2, or single chain variable fragment (scFv); (4) Palmitoyl-Protein Thioesterase 1 (PPT1); (5) Tripeptidyl-Peptidase 1 (TPP1); (6) Battenin (CLN3); (7) CLN6 Transmembrane ER Protein (CLN6); (8) Major Facilitator Superfamily Domain Containing 8 (MFSD8); (9) Myosin VIIA (MYO7A); (1) Cadherin Related 23 (CDH23); (11)Protocadherin Related 15 (PCDH15); (12) Usherin (USH2A); (13) Clarin 1 (CLRN1); (14)ATP Binding Cassette Subfamily A Member 4 (ABCA4); (15) ELOVL Fatty Acid Elongase 4 (ELOVL4); anti-Interleukin 6 (IL6) monoclonal antibody/aptamer; (16) anti-TNF-alpha (TNF) monoclonal antibody or aptamers; (17) L opsin (OPN1LW); (18) M opsin (OPN1MW); (19) Guanylate Cyclase 2D, Retinal (GUCY2D); (20) Retinoid Isomerohydrolase RPE65 (RPE65); (21) Spermatogenesis Associated 7 (SPATA7); (22) Aryl Hydrocarbon Receptor Interacting Protein Like 1 (AIPL1); (23) Lebercilin (LCA5); (24) RPGR Interacting Protein 1 (RPGRIP1) ; (25) Cone-Rod Homeobox (CRX); (26) Crumbs Cell Polarity Complex Component 1 (CRB1); (27) Nicotinamide Nucleotide Adenylyltransferase 1 (NMNAT1); (28) Centrosomal Protein 290 (CEP290); (29) Inosine Monophosphate Dehydrogenase 1 (IMPDH1); (3) Retinal Degeneration 3, GUCY2D regulator (RD3); (31) Retinol Dehydrogenase 12 (RDH12); (32) Lecithin Retinol Acyltransferase (LRAT); (33)Tubby Like Protein 1 (TLTLP1); (34) Potassium Voltage-Gated Channel Subfamily J Member 13 (KCNJ13); (35) Mitochondrially Encoded NADH Dehydrogenase 1 (MT-ND1); (36) Mitochondrially Encoded NADH Dehydrogenase 4 (MT-ND4); (37) Mitochondrially Encoded NADH Dehydrogenase 6 (MT-ND6); (38) anti-complement monoclonal antibody or aptamers, wherein the anti-complement monoclonal antibody or aptamer is an anti-complement C1 antibody or aptamer, an anti-complement C1q monoclonal antibody or aptamer, an anti-complement C1s monoclonal antibody or aptamer, an anti-complement C2 monoclonal antibody or aptamer, an anti-complement C2a monoclonal antibody or aptamer, an anti-complement C2b monoclonal antibody or aptamer, an anti-complement C3 monoclonal antibody or aptamer, an anti-complement C3a monoclonal antibody or aptamer, an anti-complement C3b monoclonal antibody or aptamer, an anti-complement C4 monoclonal antibody or aptamer, an anti-complement C4a monoclonal antibody or aptamer, an anti-complement C4b monoclonal antibody or aptamer, an anti-complement C5 monoclonal antibody or aptamer, an anti-complement C5a monoclonal antibody or aptamer, an anti-complement C5b monoclonal antibody or aptamer, an anti-complement C6 monoclonal antibody or aptamer, an anti-complement C7 monoclonal antibody or aptamer, an anti-complement C8 monoclonal antibody or aptamer, or an anti-complement C9 monoclonal antibody or aptamer, or preferably an anti-complement C5 antibody and the pathology of the eye is associated with neuromyelitis optica (NMO); (39) an anti-IL6 monoclonal antibody or aptamer and the pathology of the eye is associated with NMO; (40) anti-complement monoclonal antibody or aptamer, wherein the anti-complement monoclonal antibody or aptamer is an anti-complement C1 monoclonal antibody or aptamer, an anti-complement C1q monoclonal antibody or aptamer, an anti-complement C1s monoclonal antibody or aptamer, an anti-complement C2 monoclonal antibody or aptamer, an anti-complement C2a monoclonal antibody or aptamer, an anti-complement C2b monoclonal antibody or aptamer, an anti-complement C3 monoclonal antibody or aptamer, an anti-complement C3a monoclonal antibody or aptamer, an anti-complement C3b monoclonal antibody or aptamer, an anti-complement C4 monoclonal antibody or aptamer, an anti-complement C4a monoclonal antibody or aptamer, an anti-complement C4b monoclonal antibody or aptamer, an anti-complement C5 monoclonal antibody or aptamer, an anti-complement C5a monoclonal antibody or aptamer, an anti-complement C5b monoclonal antibody or aptamer, an anti-complement C6 monoclonal antibody or aptamer, an anti-complement C7 monoclonal antibody or aptamer, an anti-complement C8 monoclonal antibody or aptamer, or an anti-complement C9 monoclonal antibody or aptamers, or preferably an anti-complement C5 antibody; (41) Angiotensin I Converting Enzyme (ACE); (42) Interleukin 10 (IL10); (43) anti-TNF monoclonal antibody; (43)Rab Escort Protein 1 (CHM); (44) Retinoschisin (RS1); (45) Bardet-Biedl Syndrome 1 (BBS1); (46) Bardet-Biedl Syndrome 2 (BBS2); (47) ADP Ribosylation Factor Like GTPase 6 (ARL6); (48) Bardet-Biedl Syndrome 4 (BBS4); (49) Bardet-Biedl Syndrome 5 (BBS5); (50) McKusick-Kaufman Syndrome (MKKS); (51) Bardet-Biedl Syndrome 7 (BBS7); (52) Tetratricopeptide Repeat Domain 8 (TTC8); (53) Bardet-Biedl Syndrome 9 (BBS9); (54) Bardet-Biedl Syndrome 10 (BBS10); (55) Tripartite Motif Containing 32 (TRIM32); (56) Bardet-Biedl Syndrome 12 (BBS12); (57) MKS Transition Zone Complex Subunit 1 (MKS1); (58)WD Repeat Containing Planar Cell Polarity Effector (WDPCP) ; (59) Serologically Defined Colon Cancer Antigen 8 (SDCCAG8); (6) Leucine Zipper Transcription Factor Like 1 (LZTFL1) ; (61) BBSome Interacting Protein 1 (BBIP1); (62) Intraflagellar Transport 27 (IFT27); (63) Guanylate Cyclase Activator 1A (GUCA1A); (64) OPA1 Mitochondrial Dynamin Like GTPase (OPA1);(65) RP1 Axonemal Microtubule Associated (RP1); (66) RP2 Activator of ARL3 GTPase (RP2); (67) Peripherin 2 (PRPH2); (68) Pre-mRNA Processing Factor 31(PRPF31); (69) Pre-mRNA Processing Factor 8 (PRPF8); (70) Eyes Shut Homolog (EYS); (71) FAM161 Centrosomal Protein A (FAM161A); (72) Nuclear Receptor Subfamily 2 Group E Member 3 (NR2E3); (73) MER Proto-Oncogene, Tyrosine Kinase (MERTK); (74) Phosphodiesterase 6B (PDE6B); (75) Prominin 1 (PROM1); (76) Phosphodiesterase 6A (PDE6A); (77) Interphotoreceptor Matrix Proteoglycan 2 (IMPG2); (78) Male Germ Cell Associated Kinase (MAK); (79) Intraflagellar Transport 140 (IFT140); (80) anti-membrane attack complex (MAC) monoclonal antibody; (81) HtrA Serine Peptidase 1 (HTRA1); (82) Bestrophin 1 (BEST1); (83) complement factor B antisense oligonucleotide; (84) anti-beta-amyloid monoclonal antibody; (85) CD59 glycoprotein (CD59); (86) Channelrhodopsin-1 (ChR1); (87) Channelrhodopsin-2 (ChR2), (88)anti-complement factor C5a aptamer or monoclonal antibody; (89) anti-complement factor D monoclonal antibody or aptamers; (90) DnaJ heat shock protein family (Hsp40) member C3 (DNAJC3); (91) beta-2 adrenoceptor siRNA; (92) Caspase-2 (CASP2); (93) Insulin Receptor Substrate 1 (IRS1); (94) HIF-1 Responsive Protein RTP801 (RTP801); (95) Transforming Growth Factor Beta 2 (TGFB2); (96) Brain Derived Neurotrophic Factor (BDNF); (97) Ciliary Neurotrophic Factor (CNTF); (98) Prostaglandin-Endoperoxide Synthase 2 (PTGS2); (99) Prostaglandin F Receptor (PTGFR); (100) hyaluronidase; (101) Pigment Epithelium-Derived Factor (PEDF); (102) Vascular Endothelial Growth Factor (VEGF); (103) Placental Growth Factor (PGF); (104) Myocilin (MYOC); (105) C-C Motif Chemokine Receptor 5 (CCR5) siRNA; (106) anti-CD19 monoclonal antibody or aptamers; (107) Crumbs Cell Polarity Complex Component 2 (CRB2); (108) Histone Deacetylase 4 (HDAC4); (109) Rhodopsin (RHO); (110) Nerve Growth Factor (NGF); (111) Nuclear Factor, Erythroid 2 Like 2 (NRF2); (112) Glutathione S-Transferase PI 1 (GSTP1); (113) Rod-Derived Cone Viability Factor (RDCVF); (114) Retinaldehyde Binding Protein 1 (RLBP1);(115) Double Homeobox 4 (DUX4); (116) NLR Family Pyrin Domain Containing 3 (NLRP3); (117) Spleen Associated Tyrosine Kinase (SYK); (118) Adrenocorticotropic Hormone (ACTH); (119) anti-CD59 monoclonal antibody or aptamers; (120) NOTCH Regulated Ankyrin Repeat Protein (NRARP); (121) Alpha-2-Antiplasmin (A2AP); (122) Plasminogen (PLG); (123) growth hormone; (124) Insulin Like Growth Factor 1 (IGF1) ; (125) Interleukin 1 Beta (IL1B); (126) Angiotensin I Converting Enzyme 2 (ACE2); (127) anti-integrin oligopeptide; (128) anti-Placental Growth Factor (PGF) monoclonal antibody or aptamer; (129) anti-Insulin-Like Growth Factor 1 Receptor (IGF1R) monoclonal antibody or aptamer; (130) anti-Insulin-Like Growth Factor 2 Receptor (IGF2R) monoclonal antibody or aptamer; (131) RTP801 siRNA; (132) Matrix Metalloproteinase 2 (MMP2) RNAi; (133) G-Protein Coupled Receptor 143 (GPR143); (134) Tyrosinase (TYR); (135) anti-Leucine Rich Repeat And Ig Domain Containing Protein 1 (LINGO1) monoclonal antibody or aptamers; (136) Retinitis Pigmentosa GTPase Regulator (RPGR); (137) Cyclic Nucleotide Gated Channel Beta 3 (CNGB3); (138) Cyclic Nucleotide Gated Channel Alpha 3 (CNGA3); (139) Retinoid Isomerohydrolase RPE65 (RPE65); (14) anti-TNF-alpha (TNF) monoclonal antibody; or (140) Interleukin 10 (IL10).

In certain embodiments of the methods described herein, (1) the pathology of the eye is associated with Batten-CLN1 and the therapeutic product is Palmitoyl-Protein Thioesterase 1 (PPT1); (2) the pathology of the eye is associated with Batten-CLN2 and the therapeutic product is Tripeptidyl-Peptidase 1 (TPP1); (3) the pathology of the eye is associated with Batten-CLN3 and the therapeutic product is Battenin (CLN3); (4) the pathology of the eye is associated with Batten-CLN6 and the therapeutic product is CLN6 Transmembrane ER Protein (CLN6); (5) the pathology of the eye is associated with Batten-CLN7 and the therapeutic product is Major Facilitator Superfamily Domain Containing 8 (MFSD8); (6) the pathology of the eye is associated with Usher's-Type 1 and the therapeutic product is Myosin VIIA (MYO7A); (7) the pathology of the eye is associated with Usher's-Type 1 and the therapeutic product is Cadherin Related 23 (CDH23); (8) the pathology of the eye is associated with Usher's-Type 2 and the therapeutic product is Protocadherin Related 15 (PCDH15); (9) the pathology of the eye is associated with Usher's-Type 2 and the therapeutic product is Usherin (USH2A); (10) the pathology of the eye is associated with Usher's-Type 3 and the therapeutic product is Clarin 1 (CLRN1); (11) the pathology of the eye is associated with Stargardt's and the therapeutic product is ATP Binding Cassette Subfamily A Member 4 (ABCA4); (12) the pathology of the eye is associated with Stargardt's and the therapeutic product is ELOVL Fatty Acid Elongase 4 (ELOVL4); (13) the pathology of the eye is associated with uveitis and the therapeutic product is an anti-Interleukin 6 (IL6) monoclonal antibody; (14) the pathology of the eye is associated with uveitis and the therapeutic product is an anti-TNF-alpha (TNF) monoclonal antibody; (15) the pathology of the eye is associated with diabetic macular edema (DME) and the therapeutic product is an anti-IL6 monoclonal antibody; (16) the pathology of the eye is associated with red-green color blindness and the therapeutic product is L opsin (OPN1LW); (17) the pathology of the eye is associated with red-green color blindness and the therapeutic product is M opsin (OPN1MW); (18) the pathology of the eye is associated with blue cone monochromacy and the therapeutic product is M opsin (OPN1MW); (19) the pathology of the eye is associated with Leber congenital amaurosis-1 (LCA 1) and the therapeutic product is Guanylate Cyclase 2D, Retinal (GUCY2D); (20) the pathology of the eye is associated with Leber congenital amaurosis-2 (LCA 2) and the therapeutic product is Retinoid Isomerohydrolase RPE65 (RPE65); (21) the pathology of the eye is associated with LCA 3 and the therapeutic product is Spermatogenesis Associated 7 (SPATA7); (22) the pathology of the eye is associated with Leber congenital amaurosis-4 (LCA 4) and the therapeutic product is Aryl Hydrocarbon Receptor Interacting Protein Like 1 (AIPL1); (23) the pathology of the eye is associated with Leber congenital amaurosis-5 (LCA 5) and the therapeutic product is Lebercilin (LCA5); (24) the pathology of the eye is associated with Leber congenital amaurosis-6 (LCA 6) and the therapeutic product is RPGR Interacting Protein 1 (RPGRIP1); (25) the pathology of the eye is associated with Leber congenital amaurosis-7 (LCA 7) and the therapeutic product is Cone-Rod Homeobox (CRX); (26) the pathology of the eye is associated with Leber congenital amaurosis-8 (LCA 8) and the therapeutic product is Crumbs Cell Polarity Complex Component 1 (CRB1) (also known as LCA8); (27) the pathology of the eye is associated with Leber congenital amaurosis-9 (LCA 9) and the therapeutic product is Nicotinamide Nucleotide Adenylyltransferase 1 (NMNAT1); (28) the pathology of the eye is associated with Leber congenital amaurosis-10 (LCA 10) and the therapeutic product is Centrosomal Protein 290 (CEP290); (29) the pathology of the eye is associated with Leber congenital amaurosis-11 (LCA 11) and the therapeutic product is Inosine Monophosphate Dehydrogenase 1 (IMPDH1); (30) the pathology of the eye is associated with Leber congenital amaurosis-12 (LCA 12) and the therapeutic product is Retinal Degeneration 3, GUCY2D regulator (RD3) ; (31) the pathology of the eye is associated with Leber congenital amaurosis-13 (LCA 13) and the therapeutic product is Retinol Dehydrogenase 12 (RDH12); (32) the pathology of the eye is associated with Leber congenital amaurosis-14 (LCA 14) and the therapeutic product is Lecithin Retinol Acyltransferase (LRAT); (33) the pathology of the eye is associated with Leber congenital amaurosis-15 (LCA 15) and the therapeutic product is Tubby Like Protein 1 (TULP1); (34) the pathology of the eye is associated with Leber congenital amaurosis-16 (LCA 16) and the therapeutic product is Potassium Voltage-Gated Channel Subfamily J Member 13 (KCNJ13); (35) the pathology of the eye is associated with Leber's hereditary optic neuropathy (LHON) and the therapeutic product is Mitochondrially Encoded NADH Dehydrogenase 1 (MT-ND1); (36) the pathology of the eye is associated with LHON and the therapeutic product is Mitochondrially Encoded NADH Dehydrogenase 4 (MT-ND4); (37) the pathology of the eye is associated with LHON and the therapeutic product is Mitochondrially Encoded NADH Dehydrogenase 6 (MT-ND6); (38) the pathology of the eye is associated with neuromyelitis optica (NMO) and the therapeutic product is an anti-complement antibody or an anti-complement aptamer, wherein the anti-complement monoclonal antibody or aptamer is an anti-complement C1 antibody or aptamer, an anti-complement C1q monoclonal antibody or aptamer, an anti-complement C1s monoclonal antibody or aptamer, an anti-complement C2 monoclonal antibody or aptamer, an anti-complement C2a monoclonal antibody or aptamer, an anti-complement C2b monoclonal antibody or aptamer, an anti-complement C3 monoclonal antibody or aptamer, an anti-complement C3a monoclonal antibody or aptamer, an anti-complement C3b monoclonal antibody or aptamer, an anti-complement C4 monoclonal antibody or aptamer, an anti-complement C4a monoclonal antibody or aptamer, an anti-complement C4b monoclonal antibody or aptamer, an anti-complement C5 monoclonal antibody or aptamer, an anti-complement C5a monoclonal antibody or aptamer, an anti-complement C5b monoclonal antibody or aptamer, an anti-complement C6 monoclonal antibody or aptamer, an anti-complement C7 monoclonal antibody or aptamer, an anti-complement C8 monoclonal antibody or aptamer, or an anti-complement C9 monoclonal antibody or aptamer, or preferably an anti-complement C5 antibody; (39) the pathology of the eye is associated with NMO and the therapeutic product is an anti-IL6 monoclonal antibody or aptamer; (40) the pathology of the eye is associated with uveitis and the therapeutic product is an anti-complement monoclonal antibody or aptamer, wherein the anti-complement monoclonal antibody or aptamer is an anti-complement C1 monoclonal antibody or aptamer, an anti-complement C1q monoclonal antibody or aptamer, an anti-complement C1s monoclonal antibody or aptamer, an anti-complement C2 monoclonal antibody or aptamer, an anti-complement C2a monoclonal antibody or aptamer, an anti-complement C2b monoclonal antibody or aptamer, an anti-complement C3 monoclonal antibody or aptamer, an anti-complement C3a monoclonal antibody or aptamer, an anti-complement C3b monoclonal antibody or aptamer, an anti-complement C4 monoclonal antibody or aptamer, an anti-complement C4a monoclonal antibody or aptamer, an anti-complement C4b monoclonal antibody or aptamer, an anti-complement C5 monoclonal antibody or aptamer, an anti-complement C5a monoclonal antibody or aptamer, an anti-complement C5b monoclonal antibody or aptamer, an anti-complement C6 monoclonal antibody or aptamer, an anti-complement C7 monoclonal antibody or aptamer, an anti-complement C8 monoclonal antibody or aptamer, or an anti-complement C9 monoclonal antibody or aptamers, or preferably an anti-complement C5 antibody; (41) the pathology of the eye is associated with uveitis and the therapeutic product is Angiotensin I Converting Enzyme (ACE); (42) the pathology of the eye is associated with uveitis and the therapeutic product is Interleukin 10 (IL10); (43) the pathology of the eye is associated with uveitis and the therapeutic product is an anti-TNF monoclonal antibody; (44) the pathology of the eye is associated with choroideremia and the therapeutic product is Rab Escort Protein 1 (CHM); (45) the pathology of the eye is associated with X-linked retinoschisis (XLRS) and the therapeutic product is Retinoschisin (RS1); (46) the pathology of the eye is associated with Bardet-Biedl syndrome 1 and the therapeutic product is Bardet-Biedl Syndrome 1 (BBS1); (47) the pathology of the eye is associated with Bardet-Biedl syndrome 2 and the therapeutic product is Bardet-Biedl Syndrome 2 (BBS2); (48) the pathology of the eye is associated with Bardet-Biedl syndrome 3 and the therapeutic product is ADP Ribosylation Factor Like GTPase 6 (ARL6) (also known as BBS3); (49) the pathology of the eye is associated with Bardet-Biedl syndrome 4 and the therapeutic product is Bardet-Biedl Syndrome 4 (BBS4); (50) the pathology of the eye is associated with Bardet-Biedl syndrome 5 and the therapeutic product is Bardet-Biedl Syndrome 5 (BBS5); (51) the pathology of the eye is associated with Bardet-Biedl syndrome 6 and the therapeutic product is McKusick-Kaufman Syndrome (MKKS), also known as BBS6; (52) the pathology of the eye is associated with Bardet-Biedl syndrome 7 and the therapeutic product is Bardet-Biedl Syndrome 7 (BBS7); (53) the pathology of the eye is associated with Bardet-Biedl syndrome 8 and the therapeutic product is Tetratricopeptide Repeat Domain 8 (TTC8), also known as BBS8; (54) the pathology of the eye is associated with Bardet-Biedl syndrome 9 and the therapeutic product is Bardet-Biedl Syndrome 9 (BBS9); (55) the pathology of the eye is associated with Bardet-Biedl syndrome 10 and the therapeutic product is Bardet-Biedl Syndrome 10 (BBS10); (56) the pathology of the eye is associated with Bardet-Biedl syndrome 11 and the therapeutic product is Tripartite Motif Containing 32 (TRIM32), also known as BBS11; (57) the pathology of the eye is associated with Bardet-Biedl syndrome 12 and the therapeutic product is Bardet-Biedl Syndrome 12 (BBS12); (58) the pathology of the eye is associated with Bardet-Biedl syndrome 13 and the therapeutic product is MKS Transition Zone Complex Subunit 1 (MKS1), also known as BBS13; (59) the pathology of the eye is associated with Bardet-Biedl syndrome 14 and the therapeutic product is Centrosomal Protein 290 (CEP290), also known as BBS14 and LCA10; (60) the pathology of the eye is associated with Bardet-Biedl syndrome 15 and the therapeutic product is WD Repeat Containing Planar Cell Polarity Effector (WDPCP), also known as BBS15; (61) the pathology of the eye is associated with Bardet-Biedl syndrome 16 and the therapeutic product is Serologically Defined Colon Cancer Antigen 8 (SDCCAG8), also known as BBS16; (62) the pathology of the eye is associated with Bardet-Biedl syndrome 17 and the therapeutic product is Leucine Zipper Transcription Factor Like 1 (LZTFL1), also known as BBS17; (63) the pathology of the eye is associated with Bardet-Biedl syndrome 18 and the therapeutic product is BBSome Interacting Protein 1 (BBIP1), also known as BBS18; (64) the pathology of the eye is associated with Bardet-Biedl syndrome 19 and the therapeutic product is Intraflagellar Transport 27 (IFT27), also known as BBS19; (65) the pathology of the eye is associated with cone dystrophy and the therapeutic product is Guanylate Cyclase Activator 1A (GUCA1A); (66) the pathology of the eye is associated with optic atrophy and the therapeutic product is OPA1 Mitochondrial Dynamin Like GTPase (OPA1); (67) the pathology of the eye is associated with retinitis pigmentosa 1 and the therapeutic product is RP1 Axonemal Microtubule Associated (RP1); (68) the pathology of the eye is associated with retinitis pigmentosa 2 and the therapeutic product is RP2 Activator of ARL3 GTPase (RP2); (69) the pathology of the eye is associated with retinitis pigmentosa 7 and the therapeutic product is Peripherin 2 (PRPH2); (70) the pathology of the eye is associated with retinitis pigmentosa 11 and the therapeutic product is Pre-mRNA Processing Factor 31(PRPF31); (71) the pathology of the eye is associated with retinitis pigmentosa 12 and the therapeutic product is Crumbs Cell Polarity Complex Component 1 (CRB1), also known as LCA8; (72) the pathology of the eye is associated with retinitis pigmentosa 13 and the therapeutic product is Pre-mRNA Processing Factor 8 (PRPF8); (73) the pathology of the eye is associated with retinitis pigmentosa 25 and the therapeutic product is Eyes Shut Homolog (EYS); (74) the pathology of the eye is associated with retinitis pigmentosa 28 and the therapeutic product is FAM161 Centrosomal Protein A (FAM161A); (75) the pathology of the eye is associated with retinitis pigmentosa 37 and the therapeutic product is Nuclear Receptor Subfamily 2 Group E Member 3 (NR2E3); (76) the pathology of the eye is associated with retinitis pigmentosa 38 and the therapeutic product is MER Proto-Oncogene, Tyrosine Kinase (MERTK); (77) the pathology of the eye is associated with retinitis pigmentosa 40 and the therapeutic product is Phosphodiesterase 6B (PDE6B); (78) the pathology of the eye is associated with retinitis pigmentosa 41 and the therapeutic product is Prominin 1 (PROM1); (79) the pathology of the eye is associated with retinitis pigmentosa 43 and the therapeutic product is Phosphodiesterase 6A (PDE6A); (80) the pathology of the eye is associated with retinitis pigmentosa 56 and the therapeutic product is Interphotoreceptor Matrix Proteoglycan 2 (IMPG2); (81) the pathology of the eye is associated with petinitis pigmentosa 62 and the therapeutic product is Male Germ Cell Associated Kinase (MAK); (82) the pathology of the eye is associated with retinitis pigmentosa 80 and the therapeutic product is Intraflagellar Transport 140 (IFT140); (83) the pathology of the eye is associated with dry AMD and the therapeutic product is an anti-complement monoclonal antibody or an anti-complement aptamer, wherein the anti-complement monoclonal antibody or an anti-complement aptamer is an anti-complement C1 monoclonal antibody or aptamer, an anti-complement C1q monoclonal antibody or aptamer, an anti-complement C1s monoclonal antibody or aptamer, an anti-complement C2 monoclonal antibody or aptamer, an anti-complement C2a monoclonal antibody or aptamer, an anti-complement C2b monoclonal antibody or aptamer, an anti-complement C3 monoclonal antibody or aptamer, an anti-complement C3a monoclonal antibody or aptamer, an anti-complement C3b monoclonal antibody or aptamer, an anti-complement C4 monoclonal antibody or aptamer, an anti-complement C4a monoclonal antibody or aptamer, an anti-complement C4b monoclonal antibody or aptamer, an anti-complement C5 monoclonal antibody or aptamer, an anti-complement C5a monoclonal antibody or aptamer, an anti-complement C5b monoclonal antibody or aptamer, an anti-complement C6 monoclonal antibody or aptamer, an anti-complement C7 monoclonal antibody or aptamer, an anti-complement C8 monoclonal antibody or aptamer, or an anti-complement C9 monoclonal antibody or aptamers, or preferably an anti-complement C5 antibody; (84) the pathology of the eye is associated with dry AMD and the therapeutic product is an anti-membrane attack complex (MAC) therapeutic product, preferably the anti-MAC therapeutic product is an anti-MAC monoclonal antibody, which is a monoclonal antibody against a human protein of the membrane attack complex, which is composed of four complement proteins C5b (SEQ ID NOs. 314-316), C6 (SEQ ID NO. 317), C7 (SEQ ID NO. 318), and C8 (SEQ ID NOs. 319-321); (85) the pathology of the eye is associated with dry AMD and the therapeutic product is HtrA Serine Peptidase 1 (HTRA1); (86) the pathology of the eye is associated with Best disease and the therapeutic product is Bestrophin 1 (BEST1); (87) the pathology of the eye is associated with dry AMD and the therapeutic product is a complement factor B antisense oligonucleotide; (88) the pathology of the eye is associated with dry AMD and the therapeutic product is an anti-beta-amyloid monoclonal antibody;(89) the pathology of the eye is associated with dry AMD and the therapeutic product is CD59 glycoprotein (CD59); (90) the pathology of the eye is associated with dry AMD and the therapeutic product is Channelrhodopsin-1 (ChR1), which includes the human homolog of ChR1; (91) the pathology of the eye is associated with dry AMD and the therapeutic product is Channelrhodopsin-2 (ChR2), which includes the human homolog of ChR2; (92) the pathology of the eye is associated with dry AMD and the therapeutic product is an anti-complement monoclonal antibody or an anti-complement aptamer, wherein the anti-complement monoclonal antibody or an anti-complement aptamer is an anti-complement C1 monoclonal antibody or aptamer, an anti-complement C1q monoclonal antibody or aptamer, an anti-complement C1s monoclonal antibody or aptamer, an anti-complement C2 monoclonal antibody or aptamer, an anti-complement C2a monoclonal antibody or aptamer, an anti-complement C2b monoclonal antibody or aptamer, an anti-complement C3 monoclonal antibody or aptamer, an anti-complement C3a monoclonal antibody or aptamer, an anti-complement C3b monoclonal antibody or aptamer, an anti-complement C4 monoclonal antibody or aptamer, an anti-complement C4a monoclonal antibody or aptamer, an anti-complement C4b monoclonal antibody or aptamer, an anti-complement C5 monoclonal antibody or aptamer, an anti-complement C5a monoclonal antibody or aptamer, an anti-complement C5b monoclonal antibody or aptamer, an anti-complement C6 monoclonal antibody or aptamer, an anti-complement C7 monoclonal antibody or aptamer, an anti-complement C8 monoclonal antibody or aptamer, or an anti-complement C9 monoclonal antibody or aptamers, or preferably an anti-complement C5 antibody; (93) the pathology of the eye is associated with dry AMD and the therapeutic product is anti-complement factor D therapeutic product, including but not limited to an anti-complement factor D monoclonal antibody, or an anti-complement factor D aptamer; (94) the pathology of the eye is associated with age-related retinal ganglion cell (RGC) degeneration and the therapeutic product is DnaJ heat shock protein family (Hsp40) member C3 (DNAJC3), also known as P58IPK; (95) the pathology of the eye is associated with blue cone monochromacy (BCM) and the therapeutic product is L opsin (OPN1LW); (96) the pathology of the eye is associated with glaucoma and the therapeutic product is beta-2 adrenoceptor siRNA; (97) the pathology of the eye is associated with glaucoma and the therapeutic product is Caspase-2 (CASP2); (98) the pathology of the eye is associated with glaucoma and the therapeutic product is Insulin Receptor Substrate 1 (IRS1); (99) the pathology of the eye is associated with glaucoma and the therapeutic product is HIF-1 Responsive Protein RTP801 (RTP801); (100) the pathology of the eye is associated with glaucoma and the therapeutic product is Transforming Growth Factor Beta 2 (TGFB2); (101) the pathology of the eye is associated with glaucoma and the therapeutic product is Brain Derived Neurotrophic Factor (BDNF); (102) the pathology of the eye is associated with glaucoma and the therapeutic product is Ciliary Neurotrophic Factor (CNTF); (103) the pathology of the eye is associated with glaucoma and the therapeutic product is Prostaglandin-Endoperoxide Synthase 2 (PTGS2); (104) the pathology of the eye is associated with glaucoma and the therapeutic product is Prostaglandin F Receptor (PTGFR) (when the pathology of the eye is associated with glaucoma, in a specific embodiment, a recombinant viral vector comprising a nucleotide sequence encoding PTGFR can be administered to the human subject in combination with a recombinant viral vector comprising a nucleotide sequence encoding PTGS2; in another specific embodiment, a recombinant viral vector comprising a nucleotide sequence encoding PTGFR and a nucleotide sequence encoding PTGS2 can be administered to the human subject); (105) the pathology of the eye is associated with glaucoma and the therapeutic product is a hyaluronidase, *e.g*. HYAL1, HYAL2, HYAL3, HYAL4, and HYAL5; (106) the pathology of the eye is associated with glaucoma and the therapeutic product is Pigment Epithelium-Derived Factor (PEDF); (107) the pathology of the eye is associated with glaucoma and the therapeutic product is Vascular Endothelial Growth Factor (VEGF); (108) the pathology of the eye is associated with glaucoma and the therapeutic product is Placental Growth Factor (PGF), wherein PGF can be used in combo with VEGF; (109) the pathology of the eye is associated with glaucoma (e.g., a congenital glaucoma or juvenile glaucoma) and the therapeutic product is Myocilin (MYOC); (110) the pathology of the eye is associated with NMO and the therapeutic product is an anti-complement C5 monoclonal antibody; (111) the pathology of the eye is associated with NMO and the therapeutic product is C-C Motif Chemokine Receptor 5 (CCR5) siRNA, CCR5 shRNA, siRNA or CCR5 miRNA (preferably, a CCR5 miRNA); (112) the pathology of the eye is associated with NMO and the therapeutic product is an anti-CD19 monoclonal antibody; (113) the pathology of the eye is associated with retinitis pigmentosa that is associated with rhodopsin mutations and the therapeutic product is Channelrhodopsin-1 (ChR1), which includes the human homolog of ChR1; (114) the pathology of the eye is associated with retinitis pigmentosa that is associated with rhodopsin mutations and the therapeutic product is Channelrhodopsin-2 (ChR2), which includes the human homolog of ChR2; (115) the pathology of the eye is associated with retinitis pigmentosa and the therapeutic product is Ciliary Neurotrophic Factor (CNTF); (116) the pathology of the eye is associated with autosomal recessive retinitis pigmentosa and the therapeutic product is Crumbs Cell Polarity Complex Component 1 (CRB1); (117) the pathology of the eye is associated with autosomal recessive retinitis pigmentosa and the therapeutic product is Crumbs Cell Polarity Complex Component 2 (CRB2); (118) the pathology of the eye is associated with retinitis pigmentosa and the therapeutic product is Histone Deacetylase 4 (HDAC4); (119) the pathology of the eye is associated with retinitis pigmentosa and the therapeutic product is Rhodopsin (RHO); (120) the pathology of the eye is associated with retinitis pigmentosa and the therapeutic product is Nerve Growth Factor (NGF); (121) the pathology of the eye is associated with retinitis pigmentosa and the therapeutic product is Nuclear Factor, Erythroid 2 Like 2 (NRF2); (122) the pathology of the eye is associated with retinitis pigmentosa and the therapeutic product is Pigment Epithelium-Derived Factor (PEDF); (123) the pathology of the eye is associated with retinitis pigmentosa and the therapeutic product is Glutathione S-Transferase PI 1 (GSTP1), also known as PI; (124) the pathology of the eye is associated with retinitis pigmentosa and the therapeutic product is Rod-Derived Cone Viability Factor (RDCVF); (125) the pathology of the eye is associated with retinitis pigmentosa and the therapeutic product is Rhodopsin (RHO); (126) the pathology of the eye is associated with retinitis pigmentosa and the therapeutic product is Retinaldehyde Binding Protein 1 (RLBP1); (127) the pathology of the eye is associated with Stargardt's disease and the therapeutic product is an anti-complement C5 aptamer; (128) the pathology of the eye is associated with uveitis and the therapeutic product is Double Homeobox 4 (DUX4); (129) the pathology of the eye is associated with uveitis and the therapeutic product is NLR Family Pyrin Domain Containing 3 (NLRP3); (130) the pathology of the eye is associated with uveitis and the therapeutic product is Spleen Associated Tyrosine Kinase (SYK); (131) the pathology of the eye is associated with uveitis and the therapeutic product is Adrenocorticotropic Hormone (ACTH); (132) the pathology of the eye is associated with uveitis and the therapeutic product is Caspase 1 (CASP1); (133) the pathology of the eye is associated with uveitis and the therapeutic product is anti-CD59 therapeutic product (such as an anti-CD59 therapeutic protein (for example, an anti-CD59 monoclonal antibody), or an anti-CD59 therapeutic RNA (for example, an anti-CD59 shRNA, anti-CD59 siRNA, or anti-CD59 miRNA), preferably an anti-CD59 monoclonal antibody); (134) the pathology of the eye is associated with uveitis and the therapeutic product is an anti-complement monoclonal antibody or an anti-complement aptamer, wherein the anti-complement monoclonal antibody or aptamer is an anti-complement C1 monoclonal antibody or aptamer, an anti-complement C1q monoclonal antibody or aptamer, an anti-complement C1s monoclonal antibody or aptamer, an anti-complement C2 monoclonal antibody or aptamer, an anti-complement C2a monoclonal antibody or aptamer, an anti-complement C2b monoclonal antibody or aptamer, an anti-complement C3 monoclonal antibody or aptamer, an anti-complement C3a monoclonal antibody or aptamer, an anti-complement C3b monoclonal antibody or aptamer, an anti-complement C4 monoclonal antibody or aptamer, an anti-complement C4a monoclonal antibody or aptamer, an anti-complement C4b monoclonal antibody or aptamer, an anti-complement C5 monoclonal antibody or aptamer, an anti-complement C5a monoclonal antibody or aptamer, an anti-complement C5b monoclonal antibody or aptamer, an anti-complement C6 monoclonal antibody or aptamer, an anti-complement C7 monoclonal antibody or aptamer, an anti-complement C8 monoclonal antibody or aptamer, or an anti-complement C9 monoclonal antibody or aptamers, or preferably an anti-complement C5 antibody; (135) the pathology of the eye is associated with corneal neovascularization and the therapeutic product is Insulin Receptor Substrate 1 (IRS1); (136) the pathology of the eye is associated with corneal neovascularization and the therapeutic product is NOTCH Regulated Ankyrin Repeat Protein (NRARP); (137) the pathology of the eye is associated with diabetic retinopathy and the therapeutic product is NOTCH Regulated Ankyrin Repeat Protein (NRARP); (138) the pathology of the eye is associated with diabetic retinopathy and the therapeutic product is Alpha-2-Antiplasmin (A2AP); (139) the pathology of the eye is associated with diabetic retinopathy and the therapeutic product is Plasminogen (PLG); (140) the pathology of the eye is associated with diabetic retinopathy and the therapeutic product can be a growth hormone; (141) the pathology of the eye is associated with diabetic retinopathy and the therapeutic product is Insulin Like Growth Factor 1 (IGF1), wherein IGF1 can be used in combo with growth hormone; (142) the pathology of the eye is associated with diabetic retinopathy and the therapeutic product is Interleukin 1 Beta (IL1B).(143) the pathology of the eye is associated with diabetic retinopathy and the therapeutic product is Angiotensin I Converting Enzyme 2 (ACE2), wherein ACE2 can be used in combo with IL1B; (144) the pathology of the eye is associated with diabetic retinopathy and the therapeutic product is IRS1; (145) the pathology of the eye is associated with diabetic retinopathy and the therapeutic product is an anti-integrin oligopeptide; (146) the pathology of the eye is associated with diabetic retinopathy and the therapeutic product is an anti-Placental Growth Factor (PGF) monoclonal antibody; (147) the pathology of the eye is associated with Graves' ophthalmopathy (also known as Graves' orbitopathy) and the therapeutic product is an anti-CD40 monoclonal antibody; (148) the pathology of the eye is associated with Graves' ophthalmopathy and the therapeutic product is an anti-Insulin-Like Growth Factor 1 Receptor (IGF1R) monoclonal antibody; (149) the pathology of the eye is associated with Graves' ophthalmopathy and the therapeutic product is an anti-Insulin-Like Growth Factor 2 Receptor (IGF2R) monoclonal antibody; (150) the pathology of the eye is associated with DME and the therapeutic product is an anti-integrin oligopeptide; (151) the pathology of the eye is associated with DME and the therapeutic product is an anti-Placental Growth Factor (PGF) monoclonal antibody; (152) the pathology of the eye is associated with DME and the therapeutic product is RTP801 siRNA; (153) the pathology of the eye is associated with multiple sclerosis (MS)-associated vision loss and the therapeutic product is ND1; (154) the pathology of the eye is associated with myopia and the therapeutic product is Matrix Metalloproteinase 2 (MMP2) RNAi; (155) the pathology of the eye is associated with X-linked recessive ocular albinism and the therapeutic product is G-Protein Coupled Receptor 143 (GPR143); (156) the pathology of the eye is associated with oculocutaneous albinism type 1 and the therapeutic product is Tyrosinase (TYR); (157) the pathology of the eye is associated with optic neuritis and the therapeutic product is Caspase 2 (CASP2); (158) the pathology of the eye is associated with optic neuritis and the therapeutic product is an anti-Leucine Rich Repeat And Ig Domain Containing Protein 1 (LINGO1) monoclonal antibody; or(159) the pathology of the eye is associated with polypoidal choroidal vasculopathy and the therapeutic product is anti-complement monoclonal antibody or an anti-complement aptamer, wherein the anti-complement monoclonal antibody or aptamer is an anti-complement C1 monoclonal antibody or aptamer, an anti-complement C1q monoclonal antibody/aptamer, an anti-complement C1s monoclonal antibody/aptamer, an anti-complement C2 monoclonal antibody/aptamer, an anti-complement C2a monoclonal antibody or aptamer, an anti-complement C2b monoclonal antibody or aptamer, an anti-complement C3 monoclonal antibody or aptamer, an anti-complement C3a monoclonal antibody or aptamer, an anti-complement C3b monoclonal antibody or aptamer, an anti-complement C4 monoclonal antibody or aptamer, an anti-complement C4a monoclonal antibody or aptamer, an anti-complement C4b monoclonal antibody or aptamer, an anti-complement C5 monoclonal antibody or aptamer, an anti-complement C5a monoclonal antibody or aptamer, an anti-complement C5b monoclonal antibody or aptamer, an anti-complement C6 monoclonal antibody or aptamer, an anti-complement C7 monoclonal antibody or aptamer, an anti-complement C8 monoclonal antibody or aptamer, or an anti-complement C9 monoclonal antibody or aptamers, or preferably an anti-complement C5 antibody.

In certain embodiments of the methods described herein, the pathology of the eye is associated with X-linked retinitis pigmentosa (XLRP) and the therapeutic product is Retinitis Pigmentosa GTPase Regulator (RPGR). In certain embodiments of any of the foregoing methods, the pathology of the eye is associated with achromatopsia (ACHM) and the therapeutic product is Cyclic Nucleotide Gated Channel Beta 3 (CNGB3). In certain embodiments of any of the foregoing methods, the pathology of the eye is associated with achromatopsia (for example, a CNGA3-linked achromatopsia) and the therapeutic product is Cyclic Nucleotide Gated Channel Alpha 3 (CNGA3). In certain embodiments of any of the foregoing methods, the pathology of the eye is associated with biallelic RPE65 mutation-associated retinal dystrophy and the therapeutic product is Retinoid Isomerohydrolase RPE65 (RPE65).

In certain embodiments of the methods described herein, (1) the pathology of the eye is associated with Batten-CLN1 and the therapeutic product is Palmitoyl-Protein Thioesterase 1 (PPT1); (2) the pathology of the eye is associated with Batten-CLN2 and the therapeutic product is Tripeptidyl-Peptidase 1 (TPP1); (3) the pathology of the eye is associated with Batten-CLN3 and the therapeutic product is Battenin (CLN3); (4) the pathology of the eye is associated with uveitis and the therapeutic product is an anti-Interleukin 6 (IL6) monoclonal antibody; (5) the pathology of the eye is associated with uveitis and the therapeutic product is an anti-TNF-alpha (TNF) monoclonal antibody; (6) the pathology of the eye is associated with diabetic macular edema (DME) and the therapeutic product is an anti-IL6 monoclonal antibody; (7) the pathology of the eye is associated with red-green color blindness and the therapeutic product is L opsin (OPN1LW); (8) the pathology of the eye is associated with red-green color blindness and the therapeutic product is M opsin (OPN1MW); (9) the pathology of the eye is associated with blue cone monochromacy and the therapeutic product is M opsin (OPN1MW); (10) the pathology of the eye is associated with Leber congenital amaurosis-1 (LCA 1) and the therapeutic product is Guanylate Cyclase 2D, Retinal (GUCY2D); (11) the pathology of the eye is associated with Leber congenital amaurosis-2 (LCA 2) and the therapeutic product is Retinoid Isomerohydrolase RPE65 (RPE65); (12) the pathology of the eye is associated with Leber congenital amaurosis-7 (LCA 7) and the therapeutic product is Cone-Rod Homeobox (CRX); (13) the pathology of the eye is associated with Leber congenital amaurosis-11 (LCA 11) and the therapeutic product is Inosine Monophosphate Dehydrogenase 1 (IMPDH1); (14) the pathology of the eye is associated with Leber congenital amaurosis-12 (LCA 12) and the therapeutic product is Retinal Degeneration 3, GUCY2D regulator (RD3); (15) the pathology of the eye is associated with Leber congenital amaurosis-13 (LCA 13) and the therapeutic product is Retinol Dehydrogenase 12 (RDH12); (16) the pathology of the eye is associated with Leber congenital amaurosis-15 (LCA 15) and the therapeutic product is Tubby Like Protein 1 (TULP1); (17) the pathology of the eye is associated with Leber congenital amaurosis-16 (LCA 16) and the therapeutic product is Potassium Voltage-Gated Channel Subfamily J Member 13 (KCNJ13); (18) the pathology of the eye is associated with Leber's hereditary optic neuropathy (LHON) and the therapeutic product is Mitochondrially Encoded NADH Dehydrogenase 1 (MT-ND1); (19) the pathology of the eye is associated with LHON and the therapeutic product is Mitochondrially Encoded NADH Dehydrogenase 4 (MT-ND4); (20) the pathology of the eye is associated with LHON and the therapeutic product is Mitochondrially Encoded NADH Dehydrogenase 6 (MT-ND6); (21) the pathology of the eye is associated with neuromyelitis optica (NMO) and the therapeutic product is an anti-complement monoclonal antibody or an anti-complement aptamer, wherein the anti-complement monoclonal antibody or aptamer is an anti-complement C1 monoclonal antibody or aptamer, an anti-complement C1q monoclonal antibody or aptamer, an anti-complement C1s monoclonal antibody or aptamer, an anti-complement C2 monoclonal antibody or aptamer, an anti-complement C2a monoclonal antibody or aptamer, an anti-complement C2b monoclonal antibody or aptamer, an anti-complement C3 monoclonal antibody or aptamer, an anti-complement C3a monoclonal antibody or aptamer, an anti-complement C3b monoclonal antibody or aptamer, an anti-complement C4 monoclonal antibody or aptamer, an anti-complement C4a monoclonal antibody or aptamer, an anti-complement C4b monoclonal antibody or aptamer, an anti-complement C5 monoclonal antibody or aptamer, an anti-complement C5a monoclonal antibody or aptamer, an anti-complement C5b monoclonal antibody or aptamer, an anti-complement C6 monoclonal antibody or aptamer, an anti-complement C7 monoclonal antibody or aptamer, an anti-complement C8 monoclonal antibody or aptamer, or an anti-complement C9 monoclonal antibody or aptamers, or preferably an anti-complement C5 antibody; (22) the pathology of the eye is associated with NMO and the therapeutic product is an anti-IL6 monoclonal antibody; (23) the pathology of the eye is associated with uveitis and the therapeutic product is an anti-complement C5 monoclonal antibody; (24) the pathology of the eye is associated with uveitis and the therapeutic product is Angiotensin I Converting Enzyme (ACE); (25) the pathology of the eye is associated with uveitis and the therapeutic product is Interleukin 10 (IL10); (26) the pathology of the eye is associated with uveitis and the therapeutic product is an anti-TNF monoclonal antibody; (27) the pathology of the eye is associated with X-linked retinoschisis (XLRS) and the therapeutic product is Retinoschisin (RS1); (28) the pathology of the eye is associated with Bardet-Biedl syndrome 1 and the therapeutic product is Bardet-Biedl Syndrome 1 (BBS1); (29) the pathology of the eye is associated with Bardet-Biedl syndrome 3 and the therapeutic product is ADP Ribosylation Factor Like GTPase 6 (ARL6); (30) the pathology of the eye is associated with Bardet-Biedl syndrome 5 and the therapeutic product is Bardet-Biedl Syndrome 5 (BBS5); (31) the pathology of the eye is associated with Bardet-Biedl syndrome 6 and the therapeutic product is McKusick-Kaufman Syndrome (MKKS); (32) the pathology of the eye is associated with Bardet-Biedl syndrome 10 and the therapeutic product is Bardet-Biedl Syndrome 10 (BBS10); (33) the pathology of the eye is associated with Bardet-Biedl syndrome 11 and the therapeutic product is Tripartite Motif Containing 32 (TRIM32); (34) the pathology of the eye is associated with Bardet-Biedl syndrome 13 and the therapeutic product is MKS Transition Zone Complex Subunit 1 (MKS1); (35) the pathology of the eye is associated with Bardet-Biedl syndrome 18 and the therapeutic product is BBSome Interacting Protein 1 (BBIP1); (36) the pathology of the eye is associated with Bardet-Biedl syndrome 19 and the therapeutic product is Intraflagellar Transport 27 (IFT27); (37) the pathology of the eye is associated with cone dystrophy and the therapeutic product is Guanylate Cyclase Activator 1A (GUCA1A); (38) the pathology of the eye is associated with retinitis pigmentosa 13 and the therapeutic product is Pre-mRNA Processing Factor 8 (PRPF8); (39) the pathology of the eye is associated with retinitis pigmentosa 37 and the therapeutic product is Nuclear Receptor Subfamily 2 Group E Member 3 (NR2E3); or (40) the pathology of the eye is associated with Best disease and the therapeutic product is Bestrophin 1 (BEST1).

In certain embodiments of the methods described herein, the pathology of the eye is associated with biallelic RPE65 mutation-associated retinal dystrophy and the therapeutic product is Retinoid Isomerohydrolase RPE65 (RPE65).

In certain embodiments of the methods described herein, (1) the pathology of the eye is associated with Batten-CLN2 and the therapeutic product is Tripeptidyl-Peptidase 1 (TPP1); (2) the pathology of the eye is associated with Usher's-Type 1 and the therapeutic product is Myosin VIIA (MYO7A); (3) the pathology of the eye is associated with Usher's-Type 1 and the therapeutic product is Cadherin Related 23 (CDH23); (4) the pathology of the eye is associated with Usher's-Type 2 and the therapeutic product is Protocadherin Related 15 (PCDH15); (5) the pathology of the eye is associated with Usher's-Type 2 and the therapeutic product is Usherin (USH2A); (6) the pathology of the eye is associated with Usher's-Type 3 and the therapeutic product is Clarin 1 (CLRN1); (7) the pathology of the eye is associated with Stargardt's and the therapeutic product is ATP Binding Cassette Subfamily A Member 4 (ABCA4); (8) the pathology of the eye is associated with Stargardt's and the therapeutic product is ELOVL Fatty Acid Elongase 4 (ELOVL4); (9) the pathology of the eye is associated with red-green color blindness and the therapeutic product is L opsin (OPN1LW); (10) the pathology of the eye is associated with red-green color blindness and the therapeutic product is M opsin (OPN1MW); (11) the pathology of the eye is associated with blue cone monochromacy and the therapeutic product is M opsin (OPN1MW); (12) the pathology of the eye is associated with Leber congenital amaurosis-1 (LCA 1) and the therapeutic product is Guanylate Cyclase 2D, Retinal (GUCY2D); (13) the pathology of the eye is associated with Leber congenital amaurosis-2 (LCA 2) and the therapeutic product is Retinoid Isomerohydrolase RPE65 (RPE65); (14) the pathology of the eye is associated with Leber congenital amaurosis-4 (LCA 4) and the therapeutic product is Aryl Hydrocarbon Receptor Interacting Protein Like 1 (AIPL1); (15) the pathology of the eye is associated with Leber congenital amaurosis-7 (LCA 7) and the therapeutic product is Cone-Rod Homeobox (CRX); (16) the pathology of the eye is associated with Leber congenital amaurosis-8 (LCA 8) and the therapeutic product is Crumbs Cell Polarity Complex Component 1 (CRB1); (17) the pathology of the eye is associated with Leber congenital amaurosis-9 (LCA 9) and the therapeutic product is Nicotinamide Nucleotide Adenylyltransferase 1 (NMNAT1); (18) the pathology of the eye is associated with Leber congenital amaurosis-10 (LCA 10) and the therapeutic product is Centrosomal Protein 290 (CEP290); (19) the pathology of the eye is associated with Leber congenital amaurosis-11 (LCA 11) and the therapeutic product is Inosine Monophosphate Dehydrogenase 1 (IMPDH1); (20) the pathology of the eye is associated with Leber congenital amaurosis-15 (LCA 15) and the therapeutic product is Tubby Like Protein 1 (TULP1); (21) the pathology of the eye is associated with LHON and the therapeutic product is Mitochondrially Encoded NADH Dehydrogenase 4 (MT-ND4); (22) the pathology of the eye is associated with LHON and the therapeutic product is Mitochondrially Encoded NADH Dehydrogenase 6 (MT-ND6); (23) the pathology of the eye is associated with choroideremia and the therapeutic product is Rab Escort Protein 1 (CHM); (24) the pathology of the eye is associated with X-linked retinoschisis (XLRS) and the therapeutic product is Retinoschisin (RS1); (25) the pathology of the eye is associated with Bardet-Biedl syndrome 1 and the therapeutic product is Bardet-Biedl Syndrome 1 (BBS1); (26) the pathology of the eye is associated with Bardet-Biedl syndrome 6 and the therapeutic product is McKusick-Kaufman Syndrome (MKKS); (27) the pathology of the eye is associated with Bardet-Biedl syndrome 10 and the therapeutic product is Bardet-Biedl Syndrome 10 (BBS10); (28) the pathology of the eye is associated with cone dystrophy and the therapeutic product is Guanylate Cyclase Activator 1A (GUCA1A); (29) the pathology of the eye is associated with optic atrophy and the therapeutic product is OPA1 Mitochondrial Dynamin Like GTPase (OPA1); (30) the pathology of the eye is associated with retinitis pigmentosa 1 and the therapeutic product is RP1 Axonemal Microtubule Associated (RP1); (31) the pathology of the eye is associated with retinitis pigmentosa 2 and the therapeutic product is RP2 Activator of ARL3 GTPase (RP2); (32) the pathology of the eye is associated with retinitis pigmentosa 7 and the therapeutic product is Peripherin 2 (PRPH2); (33) the pathology of the eye is associated with retinitis pigmentosa 11 and the therapeutic product is Pre-mRNA Processing Factor 31(PRPF31); (34) the pathology of the eye is associated with retinitis pigmentosa 13 and the therapeutic product is Pre-mRNA Processing Factor 8 (PRPF8); (35) the pathology of the eye is associated with retinitis pigmentosa 37 and the therapeutic product is Nuclear Receptor Subfamily 2 Group E Member 3 (NR2E3); (36) the pathology of the eye is associated with retinitis pigmentosa 38 and the therapeutic product is MER Proto-Oncogene, Tyrosine Kinase (MERTK); (37) the pathology of the eye is associated with retinitis pigmentosa 40 and the therapeutic product is Phosphodiesterase 6B (PDE6B); (38) the pathology of the eye is associated with retinitis pigmentosa 41 and the therapeutic product is Prominin 1 (PROM1); (39) the pathology of the eye is associated with retinitis pigmentosa 56 and the therapeutic product is Interphotoreceptor Matrix Proteoglycan 2 (IMPG2); (40) the pathology of the eye is associated with petinitis pigmentosa 62 and the therapeutic product is Male Germ Cell Associated Kinase (MAK); (41) the pathology of the eye is associated with retinitis pigmentosa 80 and the therapeutic product is Intraflagellar Transport 140 (IFT140); or (42) the pathology of the eye is associated with Best disease and the therapeutic product is Bestrophin 1 (BEST1).

In certain embodiments of the methods described herein, the pathology of the eye is associated with X-linked retinitis pigmentosa (XLRP) and the therapeutic product is Retinitis Pigmentosa GTPase Regulator (RPGR). In certain embodiments of any of the foregoing methods, the pathology of the eye is associated with achromatopsia and the therapeutic product is Cyclic Nucleotide Gated Channel Beta 3 (CNGB3); or achromatopsia (for example, a CNGA3-linked achromatopsia) and the therapeutic product is Cyclic Nucleotide Gated Channel Alpha 3 (CNGA3).

In certain embodiments of the methods described herein, the therapeutic product is a protein, or the therapeutic product is an antibody against a protein, which protein has at least 70%, 75%, 80%, 85% 90%, 91%, 92%, 93%, 94%, 95%, 96%, 97%, 98%, 99% or 100% sequence identity to an amino acid sequence selected from SEQ ID NOs: 52-321 provided in Section 7. In a specific embodiment of the methods described herein, the therapeutic product is a protein, or the therapeutic product is an antibody against a protein, which protein has 100% sequence identity to an amino acid sequence selected from SEQ ID NOs: 52-321 provided in Section 7.

### (j) Constructs

In certain embodiments of the methods described herein, the recombinant vectors provided herein comprise the following elements in the following order: a) a constitutive or a hypoxia-inducible promoter sequence, and b) a sequence encoding the therapeutic product. In certain embodiments, the sequence encoding the therapeutic product comprises multiple ORFs separated by IRES elements. In certain embodiments, the sequence encoding the therapeutic product comprises multiple subunits in one ORF separated by F/F2A sequences.

In certain embodiments of the methods described herein, the recombinant vectors provided herein comprise the following elements in the following order: a) a first ITR sequence, b) a first linker sequence, c) a constitutive or a hypoxia-inducible promoter sequence, d) a second linker sequence, e) an intron sequence, f) a third linker sequence, g) a first UTR sequence, h) a sequence encoding the therapeutic product, i) a second UTR sequence, j) a fourth linker sequence, k) a poly A sequence, l) a fifth linker sequence, and m) a second ITR sequence.

### (k) Manufacture and testing of vectors

The recombinant vectors (for example, recombinant viral vectors) provided herein may be manufactured using host cells. The recombinant vectors provided herein may be manufactured using mammalian host cells, for example, A549 , WEHI, 10T1/2, BHK, MDCK, COS1, COS7, BSC 1, BSC 40, BMT 10, VERO, W138, HeLa, 293, Saos, C2C12, L, HT1080, HepG2, primary fibroblast, hepatocyte, and myoblast cells. The recombinant vectors provided herein may be manufactured using host cells from human, monkey, mouse, rat, rabbit, or hamster.

For recombinant viral vectors, the host cells are stably transformed with the sequences encoding the therapeutic product and associated elements (*i.e.,* the vector genome), and the means of producing viruses in the host cells, for example, the replication and capsid genes (*e.g.,* the rep and cap genes of AAV). For a method of producing recombinant AAV vectors with AAV8 capsids, see Section IV of the Detailed Description of U.S. Patent No. 7,282,199 B2, which is incorporated herein by reference in its entirety. Genome copy titers of said vectors may be determined, for example, by TAQMAN^{®} analysis. Virions may be recovered, for example, by CsCl₂ sedimentation.

In vitro assays, *e.g*., cell culture assays, can be used to measure therapeutic product expression from a vector described herein, thus indicating, *e.g*., potency of the vector. For example, the PER.C6^{®} Cell Line (Lonza), a cell line derived from human embryonic retinal cells, or retinal pigment epithelial cells, *e.g.,* the retinal pigment epithelial cell line hTERT RPE-1 (available from ATCC^{®}), can be used to assess therapeutic product expression. Once expressed, characteristics of the expressed therapeutic product can be determined, including determination of the post-translational modification patterns, . In addition, benefits resulting from post-translational modification of the cell-expressed therapeutic product can be determined using assays known in the art.

### (l) Compositions

Compositions are described comprising a recombinant vector encoding a therapeutic product described herein and a suitable carrier. A suitable carrier (*e.g.,* for suprachoroidal, subretinal, juxtascleral, intravitreal, subconjunctival, and/or intraretinal administration) would be readily selected by one of skill in the art.

### 6.1.3 GENE THERAPY

Methods are described for the administration of a therapeutically effective amount of a recombinant vector (*i.e.,* a recombinant viral vector or a DNA expression construct) to human subjects having pathology of the eye. In particular, methods are described for the administration of a therapeutically effective amount of a recombinant vector (*i.e.,* a recombinant viral vector or a DNA expression construct) to human subjects via one of the following approaches: (1) subretinal administration without vitrectomy (for example, administration to subretinal space via the suprachoroidal space or via peripheral injection), (2) suprachoroidal administration, (3) administration to the outer space of the sclera (*i.e.,* juxtascleral administration); (4) subretinal administration accompanied by vitrectomy; (5) intravitreal administration, and (6) subconjunctival administration.

In certain embodiments, delivery to the subretinal or suprachoroidal space can be performed using the methods and/or devices described and disclosed in International Publication Nos. WO 2016/042162, WO 2017/046358, WO 2017/158365, and WO 2017/158366, each of which is incorporated by reference in its entirety.

### (a) Target Patient Populations

In certain embodiments of the methods described herein, the methods provided herein are for the administration to patients having a pathology of the eye associated with: (1) neovascular age-related macular degeneration (nAMD); (2) dry age-related macular degeneration (dry AMD); (3)retinal vein occlusion (RVO) diabetic macular edema (DME); (4) diabetic retinopathy (DR); (5) Batten-CLN1; (6) Batten-CLN2; (7) Batten-CLN3; (8) Batten-CLN6; (8) Batten-CLN7; (9) Usher's-Type 1; (10) Usher's-Type 2; (11) Usher's-Type 3; (12) Stargardt's disease; (13) uveitis; (14) red-green color blindness; (15)blue cone monochromacy; (16) Leber congenital amaurosis-1 (LCA 1); (17) Leber congenital amaurosis-2 (LCA 2); (18) ) Leber congenital amaurosis-3 (LCA 3); (19) Leber congenital amaurosis-4 (LCA 4); (20) Leber congenital amaurosis-5 (LCA 5); (21) Leber congenital amaurosis-6 (LCA 6); (22) Leber congenital amaurosis-7 (LCA 7); (23) Leber congenital amaurosis-8 (LCA 8); (24) Leber congenital amaurosis-9 (LCA 9); (25) Leber congenital amaurosis-10 (LCA 10); (26) Leber congenital amaurosis-11 (LCA 11); (27) Leber congenital amaurosis-12 (LCA 12); (28) Leber congenital amaurosis-13 (LCA 13); (29) Leber congenital amaurosis-14 (LCA 14); (30) Leber congenital amaurosis-15 (LCA 15); (30) Leber congenital amaurosis-16 (LCA 16); (31) Leber's hereditary optic neuropathy (LHON); (31) neuromyelitis optica (NMO); (32) choroideremia; (33) X-linked retinoschisis (XLRS); (34) Bardet-Biedl syndrome 1; (35) Bardet-Biedl syndrome 2; (36) Bardet-Biedl syndrome 3; (37) Bardet-Biedl syndrome 4; (38) Bardet-Biedl syndrome 5; (39) Bardet-Biedl syndrome 6; (40) Bardet-Biedl syndrome 7; (41) Bardet-Biedl syndrome 8; (42) Bardet-Biedl syndrome 9; (43) Bardet-Biedl syndrome 10; (44) Bardet-Biedl syndrome 11; (45) Bardet-Biedl syndrome 12; (46) Bardet-Biedl syndrome 13; (47) Bardet-Biedl syndrome 14; (48) Bardet-Biedl syndrome 15; (49) Bardet-Biedl syndrome 16 ; (50) Bardet-Biedl syndrome 17; (51) Bardet-Biedl syndrome 18 ; (52) Bardet-Biedl syndrome 19; (53) cone dystrophy; (54) optic atrophy; (55) retinitis pigmentosa 1; (56) retinitis pigmentosa 2; (57) retinitis pigmentosa 7; (58) retinitis pigmentosa 11; (58) retinitis pigmentosa 12; (59) retinitis pigmentosa 13; (60) retinitis pigmentosa 25; (61) retinitis pigmentosa 28; (62) retinitis pigmentosa 37; (63) retinitis pigmentosa 38; (64) retinitis pigmentosa 40; (65) retinitis pigmentosa 41 (66) retinitis pigmentosa 43; (67) retinitis pigmentosa 56; (68) petinitis pigmentosa 62; (69) retinitis pigmentosa 80; (70) age-related retinal ganglion cell (RGC) degeneration; (71) Best disease; (72 )glaucoma; (73) retinitis pigmentosa that is associated with rhodopsin mutations; (74) retinitis pigmentosa; (75) autosomal recessive retinitis pigmentosa; (76) corneal neovascularization; (77) diabetic retinopathy; (78) Graves' ophthalmopathy; (79) multiple sclerosis (MS)-associated vision loss; (80) myopia; (81) X-linked recessive ocular albinism; (82) oculocutaneous albinism type 1; (83) optic neuritis; (84) polypoidal choroidal vasculopathy; (85) X-linked retinitis pigmentosa (XLRP); (86) achromatopsia (ACHM); or (87) biallelic RPE65 mutation-associated retinal dystrophy.

In certain embodiments of the methods described herein, the human subject has a BCVA that is ≤20/20 and ≥20/400. In another specific embodiment, the human subject has a BCVA that is ≤20/63 and ≥20/400. [00152] In certain embodiments, the subject treated in accordance with the methods described herein is female. In certain embodiments, the subject treated in accordance with the methods described herein is male. In certain embodiments, the subject treated in accordance with the methods described herein is a child. In certain embodiments, the subject treated in accordance with the methods described herein is 1 month old, 2 months old, 3 months old, 4 months old, 5 months old, 6 months old, 7 months old, 8 months old, 9 months old, 10 months old, 11 months old, 1 year old, 1.5 years old, 2 years old, 2.5 years old, 3 years old, 3.5 years old, 4 years old, 4.5 years old, or 5 years old. In certain embodiments, the subject treated in accordance with the methods described herein is less than 1.5 months old, 2 months old, 3 months old, 4 months old, 5 months old, 6 months old, 7 months old, 8 months old, 9 months old, 10 months old, 11 months old, 1 year old, 1.5 years old, 2 years old, 2.5 years old, 3 years old, 3.5 years old, 4 years old, 4.5 years old, or less than 5 years old. In another specific embodiment, the subject treated in accordance with the methods described herein is 1-2 months old, 2-3 months old, 3-4 months old, 4-5 months old, 5-6 months old, 6-7 months old, 7-8 months old, 8-9 months old, 9-10 months old, 10-11 months old, 11 months to 1 year old, 1-1.5 years old, 1.5-2 years old, 2-2.5 years old, 2.5-3 years old, 3-3.5 years old, 3.5-4 years old, 4-4.5 years old, or 4.5-5 years old. In another specific embodiment, the subject treated in accordance with the methods described herein is 6 months to 5 years old.

### (b) Dosage and Mode of Administration

In certain embodiments of the method described herein, therapeutically effective doses of the recombinant vector are administered (1) to the subretinal space without vitrectomy *(e.g.,* via the suprachoroidal space or via peripheral injection), (2) to the suprachoroidal space, (3) to the outer space of the sclera (*i.e.,* juxtascleral administration), (4) to the subretinal space via vitrectomy, or (5) to the vitreous cavity, in a volume ranging from 50-100 µl or 100-500 µl, preferably 100-300 µl, and most preferably, 250 µl, depending on the administration method. In certain embodiments, therapeutically effective doses of the recombinant vector are administered suprachoroidally in a volume of 100 µl or less, for example, in a volume of 50-100 µl. In certain embodiments, therapeutically effective doses of the recombinant vector are administered to the outer surface of the sclera (*e.g*., by a posterior juxtascleral depot procedure) in a volume of 500 µl or less, for example, in a volume of 10-20 µl, 20-50 µl, 50-100 µl, 100-200 µl, 200-300 µl, 300-400 µl, or 400-500 µl. In certain embodiments, therapeutically effective doses of the recombinant vector are administered to the subretinal space via peripheral injection in a volume of 50-100 µl or 100-500 µl, preferably 100-300 µl, and most preferably, 250 µl.

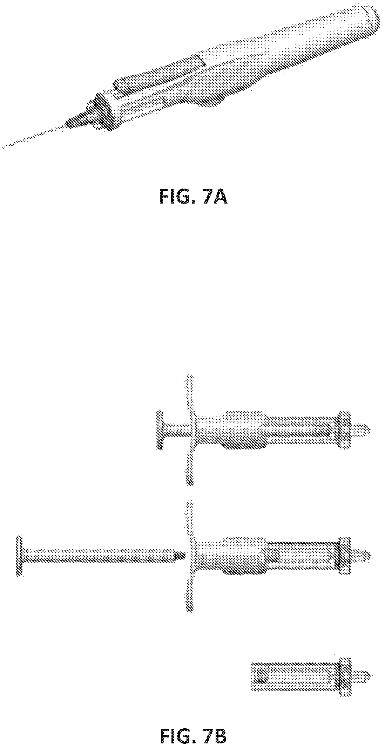

In certain embodiment, described herein is an micro volume injector delivery system, which is manufactured by Altaviz(see FIGs. 7A and 7B) *(see, e.g.* International Patent Application Publication No. WO 2013/177215, United States Patent Application Publication No. 2019/0175825, and United States Patent Application Publication No. 2019/0167906) that can be used for any administration route described herein for eye administration. The micro volume injector delivery system may include a gas-powered module providing high force delivery and improved precision, as described in United States Patent Application Publication No. 2019/0175825 and United States Patent Application Publication No. 2019/0167906. In addition, the micro volume injector delivery system may include a hydraulic drive for providing a consistent dose rate, and a low-force activation lever for controlling the gas-powered module and, in turn, the fluid delivery.

In certain embodiment, the micro volume injector delivery system can be used for micro volume injector is a micro volume injector with dose guidance and can be used with, for example, a suprachoroidal needle (for example, the Clearside^{®} needle), a subretinal needle, an intravitreal needle, a juxtascleral needle, a subconjunctival needle, and/or intraretinal needle. The benefits of using micro volume injector include: (a) more controlled delivery (for example, due to having precision injection flow rate control and dose guidance), (b) single surgeon, single hand, one finger operation; (c) pneumatic drive with 10 µL increment dosage; (d) divorced from the vitrectomy machine; (e) 400 µL syringe dose; (f) digitally guided delivery; (g) digitally recorded delivery; and (h) agnostic tip (for example, the MedOne 38g needle and the Dorc 41g needle can be used for subretinal delivery, while the Clearside^{®} needle and the Visionisti OY adaptor can be used for subretinal delivery).

In certain embodiments of the methods described herein, the recombinant vector is administered suprachoroidally (*e.g.,* by suprachoroidal injection). In a specific embodiment, suprachoroidal administration (*e.g.,* an injection into the suprachoroidal space) is performed using a suprachoroidal drug delivery device. Suprachoroidal drug delivery devices are often used in suprachoroidal administration procedures, which involve administration of a drug to the suprachoroidal space of the eye (*see, e.g.,* Hariprasad, 2016, Retinal Physician 13: 20-23; Goldstein, 2014, Retina Today 9(5): 82-87; Baldassarre *et al.,* 2017; each of which is incorporated by reference herein in its entirety). The suprachoroidal drug delivery devices that can be used to deposit the recombinant vector in the suprachoroidal space according to the invention described herein include, but are not limited to, suprachoroidal drug delivery devices manufactured by Clearside^{®} Biomedical, Inc. (*see,* for example, Hariprasad, 2016, Retinal Physician 13: 20-23) and MedOne suprachoroidal catheters. In another embodiment, the suprachoroidal drug delivery device that can be used in accordance with the methods described herein comprises the micro volume injector delivery system, which is manufactured by Altaviz (*see* FIGs. 7A and 7B ) (*see, e.g.* International Patent Application Publication No. WO 2013/177215, United States Patent Application Publication No. 2019/0175825, and United States Patent Application Publication No. 2019/0167906) that can be used for any administration route described herein for eye administration. The micro volume injector delivery system may include a gas-powered module providing high force delivery and improved precision, as described in United States Patent Application Publication No. 2019/0175825 and United States Patent Application Publication No. 2019/0167906. In addition, the micro volume injector delivery system may include a hydraulic drive for providing a consistent dose rate, and a low-force activation lever for controlling the gas-powered module and, in turn, the fluid delivery.

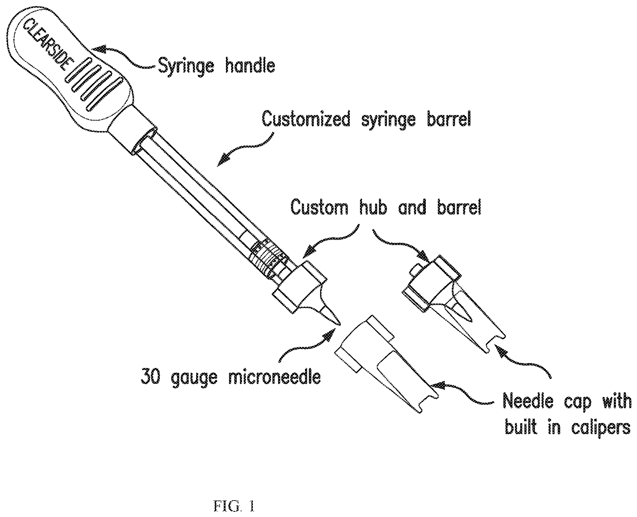

The micro volume injector is a micro volume injector with dose guidance and can be used with, for example, a suprachoroidal needle (for example, the Clearside^{®} needle) or a subretinal needle. The benefits of using micro volume injector include: (a) more controlled delivery (for example, due to having precision injection flow rate control and dose guidance), (b) single surgeon, single hand, one finger operation; (c) pneumatic drive with 10 µL increment dosage; (d) divorced from the vitrectomy machine; (e) 400 µL syringe dose; (f) digitally guided delivery; (g) digitally recorded delivery; and (h) agnostic tip (for example, the MedOne 38g needle and the Dorc 41g needle can be used for subretinal delivery, while the Clearside^{®} needle and the Visionisti OY adaptor can be used for suprachoroidal delivery). In another embodiment, the suprachoroidal drug delivery device that can be used in accordance with the methods described herein is a tool that comprises a normal length hypodermic needle with an adaptor (and preferably also a needle guide) manufactured by Visionisti OY, which adaptor turns the normal length hypodermic needle into a suprachoroidal needle by controlling the length of the needle tip exposing from the adapter (*see* FIG. 8) (*see,* for example, U.S. Design Patent No. D878,575; and International Patent Application. Publication No. WO/2016/083669) In a specific embodiment, the suprachoroidal drug delivery device is a syringe with a 1 millimeter 30 gauge needle *(see* FIG. 1). During an injection using this device, the needle pierces to the base of the sclera and fluid containing drug enters the suprachoroidal space, leading to expansion of the suprachoroidal space. As a result, there is tactile and visual feedback during the injection. Following the injection, the fluid flows posteriorly and absorbs dominantly in the choroid and retina. This results in the production of therapeutic product from all retinal cell layers and choroidal cells. Using this type of device and procedure allows for a quick and easy in-office procedure with low risk of complications. A max volume of 100 µl can be injected into the suprachoroidal space.

In certain embodiments of the methods described herein, the recombinant vector is administered subretinally via vitrectomy. Subretinal administration via vitrectomy is a surgical procedure performed by trained retinal surgeons that involves a vitrectomy with the subject under local anesthesia, and subretinal injection of the gene therapy into the retina (*see, e.g.,* Campochiaro et al., 2017, Hum Gen Ther 28(1):99-111, which is incorporated by reference herein in its entirety).

In certain embodiments of the methods described herein, the recombinant vector is administered subretinally without vitrectomy.

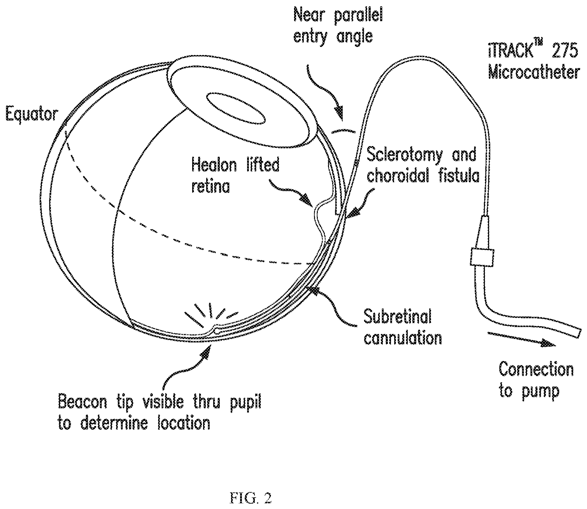

In certain embodiments of the methods described herein, the subretinal administration without vitrectomy is performed via the suprachoroidal space by use of a subretinal drug delivery device. In certain embodiments, the subretinal drug delivery device is a catheter which is inserted and tunneled through the suprachoroidal space around to the back of the eye during a surgical procedure to deliver drug to the subretinal space (*see* FIG. 2). This procedure allows the vitreous to remain intact and thus, there are fewer complication risks (less risk of gene therapy egress, and complications such as retinal detachments and macular holes), and without a vitrectomy, the resulting bleb may spread more diffusely allowing more of the surface area of the retina to be transduced with a smaller volume. The risk of induced cataract following this procedure is minimized, which is desirable for younger patients. Moreover, this procedure can deliver bleb under the fovea more safely than the standard transvitreal approach, which is desirable for patients with inherited retinal diseases effecting central vision where the target cells for transduction are in the macula. This procedure is also favorable for patients that have neutralizing antibodies (Nabs) to AAVs present in the systemic circulation which may impact other routes of delivery (such as suprachoroidal and intravitreal). Additionally, this method has shown to create blebs with less egress out the retinotomy site than the standard transvitreal approach. The subretinal drug delivery device originally manufactured by Janssen Pharmaceuticals, Inc. now by Orbit Biomedical Inc. (*see,* for example, Subretinal Delivery of Cells via the Suprachoroidal Space: Janssen Trial. In: Schwartz et al. (eds) Cellular Therapies for Retinal Disease, Springer, Cham; International Patent Application Publication No. WO 2016/040635 A1) can be used for such purpose.

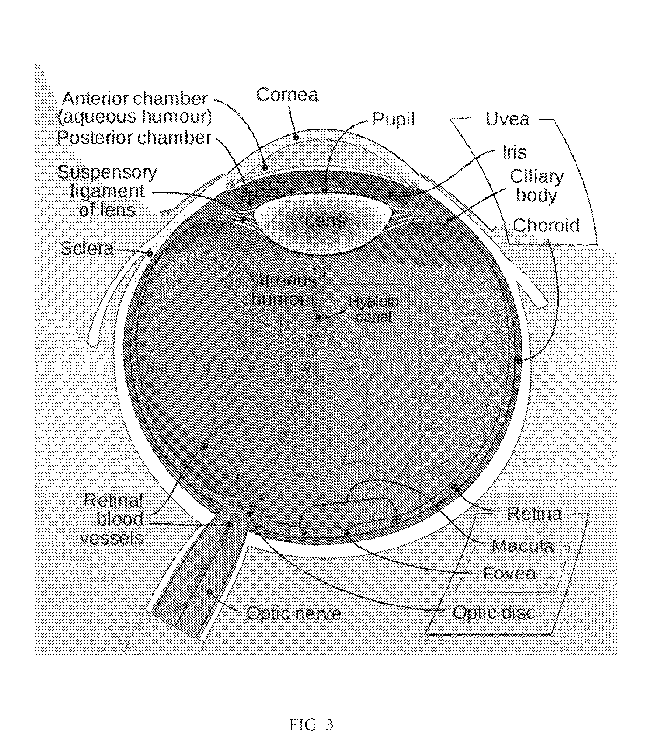

In another specific embodiment, the subretinal administration without vitrectomy is performed via peripheral injection into the retina (*i.e.,* peripheral to the optic disc, fovea and macula located in the back of the eye, *see* FIG. 3). This can be accomplished by transvitreal injection.

In one embodiment, a sharp needle is inserted into the sclera via the superior or inferior side of the eye (*e.g.,* at the 2 or 10 o'clock position) so that the needle passes all the way through the vitreous to inject the retina on the other side. In another embodiment, a trochar is inserted into the sclera to allow a subretinal cannula to be inserted into the eye. The cannula is inserted through the trochar and through the vitreous to the area of desired injection. In either embodiment, the recombinant vector is injected in the subretinal space, forming a bleb containing the recombinant vector on the opposite inner surface of the eye. Successful injection is confirmed by the appearance of a dome shaped retinal detachment/retinal bleb.

A self-illuminating lens may be used as a light source for the transvitreal administration (*see* e.g., Chalam et al., 2004, Ophthalmic Surgery and Lasers 35: 76-77, which is incorporated by reference herein in its entirety). Alternatively, one or more trochar(s) can be placed for light (or infusion) if desired. In yet another embodiment, an optic fiber chandelier can be utilized via a trocar for visualizing the subretinal injection.

One, two, or more peripheral injections can be performed to administer the recombinant vector. In this way, one, two, or more blebs containing recombinant vector can be made in the subretinal space peripheral to the optic disc, fovea and macula. Surprisingly, while administration of the recombinant vector is confined to the peripherally injected blebs, expression of the therapeutic product throughout the retina can be detected when using this approach.

In a specific embodiment, the intravitreal administration is performed with a intravitreal drug delivery device that comprises the micro volume injector delivery system, which is manufactured by Altaviz (see FIGs. 7A and 7B) (see, e.g. International Patent Application Publication No. WO 2013/177215) , United States Patent Application Publication No. 2019/0175825, and United States Patent Application Publication No. 2019/0167906) that can be used for any administration route described herein for eye administration. The micro volume injector delivery system may include a gas-powered module providing high force delivery and improved precision, as described in United States Patent Application Publication No. 2019/0175825 and United States Patent Application Publication No. 2019/0167906. In addition, the micro volume injector delivery system may include a hydraulic drive for providing a consistent dose rate, and a low-force activation lever for controlling the gas-powered module and, in turn, the fluid delivery. The micro volume injector is a micro volume injector with dose guidance and can be used with, for example, a intravitreal needle. The benefits of using micro volume injector include: (a) more controlled delivery (for example, due to having precision injection flow rate control and dose guidance), (b) single surgeon, single hand, one finger operation; (c) pneumatic drive with 10 µL increment dosage; (d) divorced from the vitrectomy machine; (e) 400 µL syringe dose; (f) digitally guided delivery; (g) digitally recorded delivery; and (h) agnostic tip (for example, the MedOne 38g needle and the Dorc 41g needle can be used for subretinal delivery, while the Clearside^{®} needle and the Visionisti OY adaptor can be used for subretinal delivery).

In certain embodiments, the peripheral injection results in uniform expression of the therapeutic product throughout the eye (*e.g.* the expression level at the site of injection varies by less than 5%, 10%, 20%, 30%, 40%, or 50% as compared to the expression level at other areas of the eye). The expression of the therapeutic product throughout the eye can be measured by any method known in the art for such a purpose, for example, by whole mount immunofluorescent staining of the eye or retina, or by immunofluorescent staining on frozen ocular sections.

In the event that a transvitreal injection results in loss of the recombinant vector in the vitreous instead of the subretinal space, an optional vitrectomy can be performed to remove the recombinant vector that was injected into the vitreous. A subretinal injection with vitrectomy can then be performed to deliver the 250 µl of recombinant vector into the subretinal space. Alternatively, if some of the injected recombinant vector is deposited into the vitreous and a vitrectomy is not performed to remove the recombinant vector from the vitreous, a catheter lined with immobilized (*e.g.,* covalently bound) anti-AAV antibodies (*e.g.,* anti AAV8 antibodies), can be inserted into the vitreous to capture and remove excess recombinant vector from the vitreous.

In a specific embodiment, the subretinal administration is performed with a subretinal drug delivery device that comprises the micro volume injector delivery system, which is manufactured by Altaviz (*see* FIGs. 7A and 7B) (*see, e.g.* International Patent Application Publication No. WO 2013/177215, United States Patent Application Publication No. 2019/0175825, and United States Patent Application Publication No. 2019/0167906) that can be used for any administration route described herein for eye administration. The micro volume injector delivery system may include a gas-powered module providing high force delivery and improved precision, as described in United States Patent Application Publication No. 2019/0175825 and United States Patent Application Publication No. 2019/0167906. In addition, the micro volume injector delivery system may include a hydraulic drive for providing a consistent dose rate, and a low-force activation lever for controlling the gas-powered module and, in turn, the fluid delivery. Micro volume injector is a micro volume injector with dose guidance and can be used with, for example, a subretinal needle. The benefits of using micro volume injector include: (a) more controlled delivery (for example, due to having precision injection flow rate control and dose guidance), (b) single surgeon, single hand, one finger operation; (c) pneumatic drive with 10 µL increment dosage; (d) divorced from the vitrectomy machine; (e) 400 µL syringe dose; (f) digitally guided delivery; (g) digitally recorded delivery; and (h) agnostic tip (for example, the MedOne 38g needle and the Dorc 41g needle can be used for subretinal delivery, while the Clearside^{®} needle and the Visionisti OY adaptor can be used for suprachoroidal delivery).

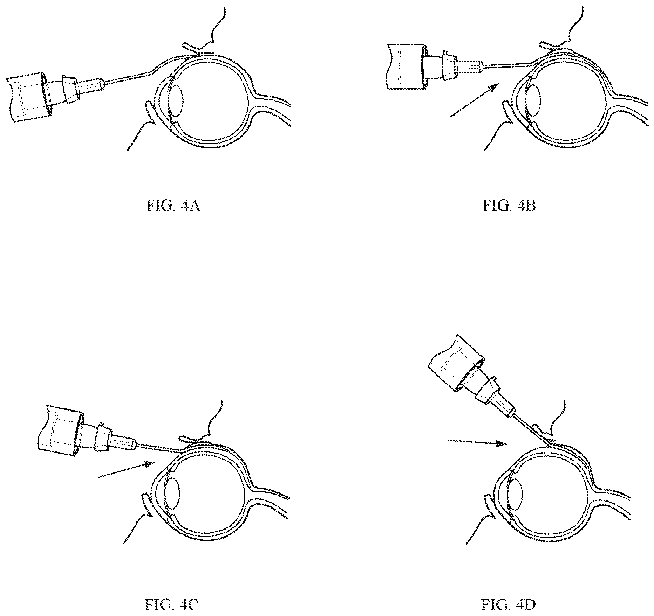

In certain embodiments, the recombinant vector is administered to the outer surface of the sclera (for example, by the use of a juxtascleral drug delivery device that comprises a cannula, whose tip can be inserted and kept in direct apposition to the scleral surface). In a specific embodiment, administration to the outer surface of the sclera is performed using a posterior juxtascleral depot procedure, which involves drug being drawn into a blunt-tipped curved cannula and then delivered in direct contact with the outer surface of the sclera without puncturing the eyeball. In particular, following the creation of a small incision to bare sclera, the cannula tip is inserted (*see* FIG. 4A). The curved portion of the cannula shaft is inserted, keeping the cannula tip in direct apposition to the scleral surface (*see* FIGS. 4B-4D). After complete insertion of the cannula (FIG. 4D), the drug is slowly injected while gentle pressure is maintained along the top and sides of the cannula shaft with sterile cotton swabs. This method of delivery avoids the risk of intraocular infection and retinal detachment, side effects commonly associated with injecting therapeutic agents directly into the eye.

In a specific embodiment, the juxtascleral administration is performed with a juxtascleral drug delivery device that comprises the micro volume injector delivery system, which is manufactured by Altaviz (*see* FIGs. 7A and 7B) (*see, e.g.* International Patent Application Publication No. WO 2013/177215 , United States Patent Application Publication No. 2019/0175825, and United States Patent Application Publication No. 2019/0167906) that can be used for any administration route described herein for eye administration. The micro volume injector delivery system may include a gas-powered module providing high force delivery and improved precision, as described in United States Patent Application Publication No. 2019/0175825 and United States Patent Application Publication No. 2019/0167906. In addition, the micro volume injector delivery system may include a hydraulic drive for providing a consistent dose rate, and a low-force activation lever for controlling the gas-powered module and, in turn, the fluid delivery. Micro Volume Injector is a micro volume injector with dose guidance and can be used with, for example, a juxtascleral needle. The benefits of using micro volume injector include: (a) more controlled delivery (for example, due to having precision injection flow rate control and dose guidance), (b) single surgeon, single hand, one finger operation; (c) pneumatic drive with 10 µL increment dosage; (d) divorced from the vitrectomy machine; (e) 400 µL syringe dose; (f) digitally guided delivery; (g) digitally recorded delivery; and (h) agnostic tip.

In certain embodiments, an infrared thermal camera can be used to detect changes in the thermal profile of the ocular surface after the administering of a solution which is cooler than body temperature to detect changes in the thermal profile of the ocular surface that allows for visualization of the spread of the solution, *e.g*., within the SCS, and can potentially determine whether the administration was successfully completed. This is because in certain embodiments the formulation containing the recombinant vector to be administered is initially frozen, brought to room temperature (68-72 °F), and thawed for a short period of time (*e.g.,* at least 30 minutes) before administration, and thus the formulation is colder than the human eye (about 92 °F) (and sometimes even colder than room temperature) at the time of injection. The drug product is typically used within 4 hours of thaw and the warmest the solution would be is room temperature. In a preferred embodiment, the procedure is videoed with infrared video.

Infrared thermal cameras can detect small changes in temperature. They capture infrared energy through a lens and convert the energy into an electronic signal. The infrared light is focused onto an infrared sensor array which converts the energy into a thermal image. The infrared thermal camera can be used for any method of administration to the eye, including any administration route described herein, for example, suprachoroidal administration, subretinal administration, subconjunctival administration, intravitreal administration, or administration with the use of a slow infusion catheter in to the suprachoroidal space. In a specific embodiment, the infrared thermal camera is an FLIR T530 infrared thermal camera. The FLIR T530 infrared thermal camera can capture slight temperature differences with an accuracy of ±3.6°F. The camera has an infrared resolution of 76,800 pixels. The camera also utilizes a 24° lens capturing a smaller field of view. A smaller field of view in combination with a high infrared resolution contributes to more detailed thermal profiles of what the operator is imaging. However, other infrared camera can be used that have different abilities and accuracy for capturing slight temperature changes, with different infrared resolutions, and/or with different degrees of lens.

In a specific embodiment, the infrared thermal camera is an FLIR T420 infrared thermal camera. In a specific embodiment, the infrared thermal camera is an FLIR T440 infrared thermal camera. In a specific embodiment, the infrared thermal camera is an Fluke Ti400 infrared thermal camera. In a specific embodiment, the infrared thermal camera is an FLIRE60 infrared thermal camera. In a specific embodiment, the infrared resolution of the infrared thermal camera is equal to or greater than 75,000 pixels. In a specific embodiment, the thermal sensitivity of the infrared thermal camera is equal to or smaller than 0.05 °C at 30 °C. In a specific embodiment, the field of view (FOV) of the infrared thermal camera is equal to or lower than 25° × 25°.

In certain embodiments, an iron filer is used with the infrared thermal camera to detect changes in the thermal profile of the ocular surface. In a preferred embodiment, the use of an iron filter is able to a generate pseudo-color image, wherein the warmest or high temperature parts are colored white, intermediate temperatures are reds and yellows, and the coolest or low temperature parts are black. In certain embodiments, other types of filters can also be used to generate pseudo-color images of the thermal profile.

The thermal profile for each administration method can be different. For example, in one embodiment, a successful suprachoroidal injection can be characterized by: (a) a slow, wide radial spread of the dark color, (b) very dark color at the beginning, and (c) a gradual change of injectate to lighter color, i.e., a temperature gradient noted by a lighter color. In one embodiment, an unsuccessful suprachoroidal injection can be characterized by: (a) no spread of the dark color, and (b) a minor change in color localized to the injection site without any distribution. In certain embodiments, the small localized temperature drop is result from cannula (low temperature) touching the ocular tissues (high temperature). In one embodiment, a successful intravitreal injection can be characterized by: (a) no spread of the dark color, (b) an initial change to very dark color localized to the injection site, and (c) a gradual and uniform change of the entire eye to darker color. In one embodiment, an extraocular efflux can be characterized by: (a) quick flowing streams on outside on the exterior surface of the eye, (b) very dark color at the beginning, and (c) a quick change to lighter color.

Because the therapeutic product is continuously produced (under the control of a constitutive promoter or induced by hypoxic conditions when using an hypoxia-inducible promoter), maintenance of lower concentrations can be effective. Vitreous humour concentrations can be measured directly in patient samples of fluid collected from the vitreous humour or the anterior chamber, or estimated and/or monitored by measuring the patient's serum concentrations of the therapeutic product - the ratio of systemic to vitreal exposure to the therapeutic product is about 1:90,000. (*E.g., see,* vitreous humor and serum concentrations of ranibizumab reported in Xu L, et al., 2013, Invest. Opthal. Vis. Sci. 54: 1616-1624, at p. 1621 and Table 5 at p. 1623, which is incorporated by reference herein in its entirety).

In certain embodiments, dosages are measured by genome copies per ml or the number of genome copies administered to the eye of the patient (*e.g*., administered suprachoroidally, subretinally, intravitreally, juxtasclerally, subconjunctivally, and/or intraretinally. In certain embodiments, 1 x 10⁹ genome copies per ml to 1 x10¹⁵ genome copies per ml are administered. In a specific embodiment, 1 x 10⁹ genome copies per ml to 1 x10¹⁰ genome copies per ml are administered. In another specific embodiment, 1 x 10¹⁰ genome copies per ml to 1 x10¹¹ genome copies per ml are administered. In another specific embodiment, 1 x 10¹⁰ to 5 x 10¹¹ genome copies are administered. In another specific embodiment, 1 x 10¹¹ genome copies per ml to 1 x10¹² genome copies per ml are administered. In another specific embodiment, 1 x 10¹² genome copies per ml to 1 x10¹³ genome copies per ml are administered. In another specific embodiment, 1 x 10¹³ genome copies per ml to 1 x10¹⁴ genome copies per ml are administered. In another specific embodiment, 1 x 10¹⁴ genome copies per ml to 1 x10¹⁵ genome copies per ml are administered. In another specific embodiment, about 1 x 10⁹ genome copies per ml are administered. In another specific embodiment, about 1 x 10¹⁰ genome copies per ml are administered. In another specific embodiment, about 1 x 10¹¹ genome copies per ml are administered. In another specific embodiment, about 1 x 10¹² genome copies per ml are administered. In another specific embodiment, about 1 x 10¹³ genome copies per ml are administered. In another specific embodiment, about 1 x 10¹⁴ genome copies per ml are administered. In another specific embodiment, about 1 x 10¹⁵ genome copies per ml are administered. In certain embodiments, 1 x 10⁹ to 1 x 10¹⁵ genome copies are administered. In a specific embodiment, 1 x 10⁹ to 1 x 10¹⁰ genome copies are administered. In another specific embodiment, 1 x 10¹⁰ to 1 x 10¹¹ genome copies are administered. In another specific embodiment, 1 x 10¹⁰ to 5 x 10¹¹ genome copies are administered. In another specific embodiment, 1 x 10¹¹ to 1 x 10¹² genome copies are administered. In another specific embodiment, 1 x 10¹² to 1 x 10¹³ genome copies are administered. In another specific embodiment, 1 x 10¹³ to 1 x 10¹⁴ genome copies are administered. In another specific embodiment, 1 x 10¹³ to 1 x 10¹⁴ genome copies are administered. In another specific embodiment, 1 x 10¹⁴ to 1 x 10¹⁵ genome copies are administered. In another specific embodiment, about 1 x 10⁹ genome copies are administered. In another specific embodiment, about 1 x 10¹⁰ genome copies are administered. In another specific embodiment, about 1 x 10¹¹ genome copies are administered. In another specific embodiment, about 1 x 10¹² genome copies are administered. In another specific embodiment, about 1 x 10¹³ genome copies are administered. In another specific embodiment, about 1 x 10¹⁴ genome copies are administered. In another specific embodiment, about 1 x 10¹⁵ genome copies are administered. In certain embodiments, about 3.0 × 10¹³ genome copies per eye are administered. In certain embodiments, up to 3.0 × 10¹³ genome copies per eye are administered.

In certain embodiments, about 6.0 × 10¹⁰ genome copies per eye are administered. In certain embodiments, about 1.6 × 10¹¹ genome copies per eye are administered. In certain embodiments, about 2.5 × 10¹¹ genome copies per eye are administered. In certain embodiments, about 5.0 × 10¹¹ genome copies per eye are administered. In certain embodiments, about 3 x 10¹² genome copies per eye are administered. In certain embodiments, about 1.0 × 10¹² genome copies per ml per eye are administered. In certain embodiments, about 2.5 × 10¹² genome copies per ml per eye are administered.

In certain embodiments, about 6.0 x 10¹⁰ genome copies per eye are administered by subretinal injection. In certain embodiments, about 1.6 x 10¹¹ genome copies per eye are administered by subretinal injection. In certain embodiments, about 2.5 × 10¹¹ genome copies per eye are administered by subretinal injection. In certain embodiments, about 3.0 × 10¹³ genome copies per eye are administered by subretinal injection. In certain embodiments, up to 3.0 × 10¹³ genome copies per eye are administered by subretinal injection.

In certain embodiments, about 2.5 × 10¹¹ genome copies per eye are administered by suprachoroidal injection. In certain embodiments, about 5.0 × 10¹¹ genome copies per eye are administered by suprachoroidal injection. In certain embodiments, about 3 x 10¹² genome copies per eye are administered by suprachoroidal injection. In certain embodiments, about 2.5 × 10¹¹ genome copies per eye are administered by a single suprachoroidal injection. In certain embodiments, about 5.0 × 10¹¹ genome copies per eye are administered by double suprachoroidal injections. In certain embodiments, about 3.0 × 10¹³ genome copies per eye are administered by suprachoroidal injection. In certain embodiments, up to 3.0 × 10¹³ genome copies per eye are administered by suprachoroidal injection. In certain embodiments, about 2.5 × 10¹² genome copies per ml per eye are administered by a single suprachoroidal injection in a volume of 100 µl. In certain embodiments, about 2.5 × 10¹² genome copies per ml per eye are administered by double suprachoroidal injections, wherein each injection is in a volume of 100 µl.

As used herein and unless otherwise specified, the term "about" means within plus or minus 10% of a given value or range. In certain embodiments, the term "about" encompasses the exact number recited.

### (c) Sampling and Monitoring of Efficacy

In certain embodiments, when the human subject has disease manifestations in both the CNS and the eye (for example, when the human subject has a Batten disease), the method provided herein comprises administering a recombinant vector described herein (*i.e*., a recombinant viral vector or a DNA expression construct) to the human subject via both a central nervous system (CNS) delivery route and an ocular delivery route (for example, an ocular delivery route described herein). In certain embodiments, the ocular delivery route is selected from one of the following: (1) subretinal administration without vitrectomy (for example, administration to subretinal space via the suprachoroidal space or via peripheral injection), (2) suprachoroidal administration, (3) administration to the outer space of the sclera (*i.e.,* juxtascleral administration); (4) subretinal administration accompanied by vitrectomy; (5) intravitreal administration, and (6) intravitreal administration. In certain embodiments, the CNS delivery route is selected from one of the following: intracerebroventricular (ICV) delivery, intracisternal (IC) delivery, or intrathecal-lumbar (IT-L) delivery.

Effects of the methods provided herein on visual deficits may be measured by BCVA (Best-Corrected Visual Acuity), intraocular pressure, slit lamp biomicroscopy, and/or indirect ophthalmoscopy.

In specific embodiments, effects of the methods provided herein on visual deficits may be measured by whether the human patient's eye that is treated by a method described herein achieves BCVA of greater than 43 letters post-treatment (*e.g.,* 46-50 weeks or 98-102 weeks post-treatment). A BCVA of 43 letters corresponds to 20/160 approximate Snellen equivalent. In a specific embodiment, the human patient's eye that is treated by a method described herein achieves BCVA of greater than 43 letters post-treatment (*e.g.,* 46-50 weeks or 98-102 weeks post-treatment).

In specific embodiments, effects of the methods provided herein on visual deficits may be measured by whether the human patient's eye that is treated by a method described herein achieves BCVA of greater than 84 letters post-treatment (*e.g.,* 46-50 weeks or 98-102 weeks post-treatment). A BCVA of 84 letters corresponds to 20/20 approximate Snellen equivalent. In a specific embodiment, the human patient's eye that is treated by a method described herein achieves BCVA of greater than 84 letters post-treatment (*e.g.,* 46-50 weeks or 98-102 weeks post-treatment).

Effects of the methods provided herein on physical changes to eye/retina may be measured by SD-OCT (SD-Optical Coherence Tomography).

Efficacy may be monitored as measured by electroretinography (ERG).

Effects of the methods provided herein may be monitored by measuring signs of vision loss, infection, inflammation and other safety events, including retinal detachment.

Retinal thickness may be monitored to determine efficacy of the methods provided herein. Without being bound by any particular theory, thickness of the retina may be used as a clinical readout, wherein the greater reduction in retinal thickness or the longer period of time before thickening of the retina, the more efficacious the treatment. Retinal function may be determined, for example, by ERG. ERG is a non-invasive electrophysiologic test of retinal function, approved by the FDA for use in humans, which examines the light sensitive cells of the eye (the rods and cones), and their connecting ganglion cells, in particular, their response to a flash stimulation. Retinal thickness may be determined, for example, by SD-OCT. SD-OCT is a three-dimensional imaging technology which uses low-coherence interferometry to determine the echo time delay and magnitude of backscattered light reflected off an object of interest. OCT can be used to scan the layers of a tissue sample (*e.g.,* the retina) with 3 to 15 µm axial resolution, and SD-OCT improves axial resolution and scan speed over previous forms of the technology (Schuman, 2008, Trans. Am. Opthamol. Soc. 106:426-458).

Effects of the methods provided herein may also be measured by a change from baseline in National Eye Institute Visual Functioning Questionnaire, the Rasch-scored version (NEI-VFQ-28-R) (composite score; activity limitation domain score; and socio-emotional functioning domain score). Effects of the methods provided herein may also be measured by a change from baseline in National Eye Institute Visual Functioning Questionnaire 25-item version (NEI-VFQ-25) (composite score and mental health subscale score). Effects of the methods provided herein may also be measured by a change from baseline in Macular Disease Treatment Satisfaction Questionnaire (MacTSQ) (composite score; safety, efficacy, and discomfort domain score; and information provision and convenience domain score).

In specific embodiments, the efficacy of a method described herein is reflected by an improvement in vision at about 4 weeks, 12 weeks, 6 months, 12 months, 24 months, 36 months, or at other desired timepoints. In a specific embodiment, the improvement in vision is characterized by an increase in BCVA, for example, an increase by 1 letter, 2 letters, 3 letters, 4 letters, 5 letters, 6 letters, 7 letters, 8 letters, 9 letters, 10 letters, 11 letters, or 12 letters, or more. In a specific embodiment, the improvement in vision is characterized by a 5%, 10%, 15%, 20%, 30%, 40%, 50% or more increase in visual acuity from baseline.

In specific embodiments, the efficacy of a method described herein is reflected by an reduction in central retinal thickness (CRT) at about 4 weeks, 12 weeks, 6 months, 12 months, 24 months, 36 months, or at other desired timepoint, for example, a 5%, 10%, 15%, 20%, 30%, 40%, 50% or more decrease in central retinal thickness from baseline.

In s specific embodiments, there is no inflammation in the eye after treatment or little inflammation in the eye after treatment (for example, an increase in the level of inflammation by 10%, 5%, 2%, 1% or less from baseline).

Effects of the methods provided herein on visual deficits may be measured by OptoKinetic Nystagmus (OKN).

Without being bound by theory, this visual acuity screening uses the principles of the OKN involuntary reflex to objectively assess whether a patient's eyes can follow a moving target. By using OKN, no verbal communication is needed between the tester and the patient. As such, OKN can be used to measure visual acuity in pre-verbal and/or non-verbal patients. In certain embodiments, OKN is used to measure visual acuity in patients that are 1 month old, 2 months old, 3 months old, 4 months old, 5 months old, 6 months old, 7 months old, 8 months old, 9 months old, 10 months old, 11 months old, 1 year old, 1.5 years old, 2 years old, 2.5 years old, 3 years old, 3.5 years old, 4 years old, 4.5 years old, or 5 years old. In certain embodiments, an iPad is used to measure visual acuity through detection of the OKN reflex when a patient is looking at movement on the iPad.

Without being bound by theory, this visual acuity screening uses the principles of the OKN involuntary reflex to objectively assess whether a patient's eyes can follow a moving target. By using OKN, no verbal communication is needed between the tester and the patient. As such, OKN can be used to measure visual acuity in pre-verbal and/or non-verbal patients. In certain embodiments, OKN is used to measure visual acuity in patients that are less than 1.5 months old, 2 months old, 3 months old, 4 months old, 5 months old, 6 months old, 7 months old, 8 months old, 9 months old, 10 months old, 11 months old, 1 year old, 1.5 years old, 2 years old, 2.5 years old, 3 years old, 3.5 years old, 4 years old, 4.5 years old, or 5 years old. In another specific embodiment, OKN is used to measure visual acuity in patients that are 1-2 months old, 2-3 months old, 3-4 months old, 4-5 months old, 5-6 months old, 6-7 months old, 7-8 months old, 8-9 months old, 9-10 months old, 10-11 months old, 11 months to 1 year old, 1-1.5 years old, 1.5-2 years old, 2-2.5 years old, 2.5-3 years old, 3-3.5 years old, 3.5-4 years old, 4-4.5 years old, or 4.5-5 years old. In another specific embodiment, OKN is used to measure visual acuity in patients that are 6 months to 5 years old. In certain embodiments, an iPad is used to measure visual acuity through detection of the OKN reflex when a patient is looking at movement on the iPad.

In certain embodiments, visual acuity is assessed in a patient presenting with Batten-CLN2-associated vision loss by measuring OKN before the patient has been treated with an AAV, preferably AAV8 or AAV9, encoding Tripeptidyl-Peptidase 1(TPP1). Specifically, the patient presenting with Batten-CLN2-associated vision loss is at the age, and/or within the age range described above. In certain embodiments, visual acuity assessed in a patient up to 5 years old presenting with Batten-CLN2-associated vision loss by measuring OKN after the patient has been treated with an AAV, preferably AAV8 or AAV9, encoding Tripeptidyl-Peptidase 1. In certain embodiments, a visual acuity assessment based on OKN determines that visual acuity does not decrease after treatment with AAV gene therapy. In certain embodiments, a visual acuity assessment based on OKN determines that visual acuity improves in a patient after treatment with AAV gene therapy by 5%, 10%, 15%, 20%, 25%, 30%, 35%, 40% 45%, 50%, 55%, 60% , 65%, 70%, 75%, 80%, 85%, 90%, 95% or 100%. In certain embodiments, a visual acuity assessment based on OKN determines that visual acuity does not further deteriorate in a patient after treatment with AAV gene therapy.

In certain embodiments, visual acuity is assessed in a patient presenting with Batten-CLN2-associated vision loss by measuring OKN before the patient has been treated with an AAV, preferably AAV8 or AAV9, encoding TPP1. Specifically, the patient presenting with Batten-CLN2-associated vision loss is at the age, and/or within the age range described above. In certain embodiments, visual acuity is assessed in a patient up to 5 years old presenting with Batten-CLN2-associated vision loss by measuring OKN after the patient has been treated with an AAV, preferably AAV8 or AAV9, encoding Tripeptidyl-Peptidase 1. In certain embodiments, a visual acuity assessment based on OKN determines that visual acuity does not decrease after treatment with AAV gene therapy. In certain embodiments, a visual acuity assessment based on OKN determines that visual acuity improves in a patient after treatment with AAV gene therapy by at least 5%, 10%, 15%, 20%, 25%, 30%, 35%, 40% 45%, 50%, 55%, 60% , 65%, 70%, 75%, 80%, 85%, 90%, 95% or at least 100%. In certain embodiments, a visual acuity assessment based on OKN determines that visual acuity does not further deteriorate in a patient after treatment with AAV gene therapy.

In certain embodiments, visual acuity is assessed in a patient presenting with Batten-CLN1-associated vision loss by measuring OKN before the patient has been treated with an AAV, preferably AAV8 or AAV9, encoding Palmitoyl-Protein Thioesterase 1 (PPT1). Specifically, the patient up to 5 years old presenting with Batten-CLN1-associated vision loss is at the age, and/or within the age range described above. In certain embodiments, visual acuity is assessed in a patient presenting with Batten-CLN1-associated vision loss by measuring OKN after the patient has been treated with an AAV, preferably AAV8 or AAV9, encoding PPT1. In certain embodiments, a visual acuity assessment based on OKN determines that visual acuity does not decrease after treatment with AAV gene therapy. In certain embodiments, a visual acuity assessment based on OKN determines that visual acuity improves in a patient after treatment with AAV gene therapy by 5%, 10%, 15%, 20%, 25%, 30%, 35%, 40% 45%, 50%, 55%, 60% , 65%, 70%, 75%, 80%, 85%, 90%, 95% or 100%. In certain embodiments, a visual acuity assessment based on OKN determines that visual acuity does not further deteriorate in a patient after treatment with AAV gene therapy.

In certain embodiments, visual acuity is assessed in a patient presenting with Batten-CLN1-associated vision loss by measuring OKN before the patient has been treated with an AAV, preferably AAV8 or AAV9, encoding PPT1. Specifically, the patient presenting with Batten-CLN1-associated vision loss is at the age, and/or within the age range described above. In certain embodiments, visual acuity is assessed in a patient up to 5 years old presenting with Batten-CLN1-associated vision loss by measuring OKN after the patient has been treated with an AAV, preferably AAV8 or AAV9, encoding PPT1. In certain embodiments, a visual acuity assessment based on OKN determines that visual acuity does not decrease after treatment with AAV gene therapy. In certain embodiments, a visual acuity assessment based on OKN determines that visual acuity improves in a patient after treatment with AAV gene therapy by at least 5%, 10%, 15%, 20%, 25%, 30%, 35%, 40% 45%, 50%, 55%, 60% , 65%, 70%, 75%, 80%, 85%, 90%, 95% or at least 100%. In certain embodiments, a visual acuity assessment based on OKN determines that visual acuity does not further deteriorate in a patient after treatment with AAV gene therapy.

In certain embodiments, visual acuity is assessed in a patient presenting with Batten-CLN3-associated vision loss by measuring OKN before the patient has been treated with an AAV, preferably AAV8 or AAV9, encoding Battenin (CLN3). Specifically, the patient presenting with Batten-CLN3-associated vision loss is at the age, and/or within the age range described above. In certain embodiments, visual acuity is assessed in a patient up to 5 years old presenting with Batten-CLN3-associated vision loss by measuring OKN after the patient has been treated with an AAV, preferably AAV8 or AAV9, encoding Battenin (CLN3). In certain embodiments, a visual acuity assessment based on OKN determines that visual acuity does not decrease after treatment with AAV gene therapy. In certain embodiments, a visual acuity assessment based on OKN determines that visual acuity improves in a patient after treatment with AAV gene therapy by 5%, 10%, 15%, 20%, 25%, 30%, 35%, 40% 45%, 50%, 55%, 60% , 65%, 70%, 75%, 80%, 85%, 90%, 95% or 100%. In certain embodiments, a visual acuity assessment based on OKN determines that visual acuity does not further deteriorate in a patient after treatment with AAV gene therapy.

In certain embodiments, visual acuity is assessed in a patient presenting with Batten-CLN3-associated vision loss by measuring OKN before the patient has been treated with an AAV, preferably AAV8 or AAV9, encoding Battenin (CLN3). Specifically, the patient presenting with Batten-CLN3-associated vision loss is at the age, and/or within the age range described above. In certain embodiments, visual acuity is assessed in a patient up to 5 years old presenting with Batten-CLN3-associated vision loss by measuring OKN after the patient has been treated with an AAV, preferably AAV8 or AAV9, encoding Battenin (CLN3). In certain embodiments, a visual acuity assessment based on OKN determines that visual acuity does not decrease after treatment with AAV gene therapy. In certain embodiments, a visual acuity assessment based on OKN determines that visual acuity improves in a patient after treatment with AAV gene therapy by at least 5%, 10%, 15%, 20%, 25%, 30%, 35%, 40% 45%, 50%, 55%, 60% , 65%, 70%, 75%, 80%, 85%, 90%, 95% or at least 100%. In certain embodiments, a visual acuity assessment based on OKN determines that visual acuity does not further deteriorate in a patient after treatment with AAV gene therapy.

In certain embodiments, visual acuity is assessed in a patient presenting with Batten-CLN6-associated vision loss by measuring OKN before the patient has been treated with an AAV, preferably AAV8 or AAV9, encoding CLN6 Transmembrane ER Protein (CLN6). Specifically, the patient up to 5 years old presenting with Batten-CLN6-associated vision loss is at the age, and/or within the age range described above. In certain embodiments, visual acuity is assessed in a patient presenting with Batten-CLN6-associated vision loss by measuring OKN after the patient has been treated with an AAV, preferably AAV8 or AAV9, encoding CLN6 Transmembrane ER Protein (CLN6). In certain embodiments, a visual acuity assessment based on OKN determines that visual acuity does not decrease after treatment with AAV gene therapy. In certain embodiments, a visual acuity assessment based on OKN determines that visual acuity improves in a patient after treatment with AAV gene therapy by 5%, 10%, 15%, 20%, 25%, 30%, 35%, 40% 45%, 50%, 55%, 60% , 65%, 70%, 75%, 80%, 85%, 90%, 95% or 100%. In certain embodiments, a visual acuity assessment based on OKN determines that visual acuity does not further deteriorate in a patient after treatment with AAV gene therapy.

In certain embodiments, visual acuity is assessed in a patient presenting with Batten-CLN6-associated vision loss by measuring OKN before the patient has been treated with an AAV, preferably AAV8 or AAV9, encoding CLN6 Transmembrane ER Protein (CLN6). Specifically, the patient up to 5 years old presenting with Batten-CLN6-associated vision loss is at the age, and/or within the age range described above. In certain embodiments, visual acuity is assessed in a patient presenting with Batten-CLN6-associated vision loss by measuring OKN after the patient has been treated with an AAV, preferably AAV8 or AAV9, encoding CLN6 Transmembrane ER Protein (CLN6). In certain embodiments, a visual acuity assessment based on OKN determines that visual acuity does not decrease after treatment with AAV gene therapy. In certain embodiments, a visual acuity assessment based on OKN determines that visual acuity improves in a patient after treatment with AAV gene therapy by at least 5%, 10%, 15%, 20%, 25%, 30%, 35%, 40% 45%, 50%, 55%, 60% , 65%, 70%, 75%, 80%, 85%, 90%, 95% or at least 100%. In certain embodiments, a visual acuity assessment based on OKN determines that visual acuity does not further deteriorate in a patient after treatment with AAV gene therapy.

In certain embodiments, visual acuity is assessed in a patient presenting with Batten-CLN7-associated vision loss by measuring OKN before the patient has been treated with an AAV, preferably AAV8 or AAV9, encoding Major Facilitator Superfamily Domain Containing 8 (MFSD8). Specifically, the patient up to 5 years old presenting with Batten-CLN7-associated vision loss is at the age, and/or within the age range described above. In certain embodiments, visual acuity is assessed in a patient presenting with Batten-CLN7-associated vision loss by measuring OKN after the patient has been treated with an AAV, preferably AAV8 or AAV9, encoding MFSD8. In certain embodiments, a visual acuity assessment based on OKN determines that visual acuity does not decrease after treatment with AAV gene therapy. In certain embodiments, a visual acuity assessment based on OKN determines that visual acuity improves in a patient after treatment with AAV gene therapy by 5%, 10%, 15%, 20%, 25%, 30%, 35%, 40% 45%, 50%, 55%, 60% , 65%, 70%, 75%, 80%, 85%, 90%, 95% or 100%. In certain embodiments, a visual acuity assessment based on OKN determines that visual acuity does not further deteriorate in a patient after treatment with AAV gene therapy.

In certain embodiments, visual acuity is assessed in a patient presenting with Batten-CLN7-associated vision loss by measuring OKN before the patient has been treated with an AAV, preferably AAV8 or AAV9, encoding MFSD8. Specifically, the patient presenting with Batten-CLN7-associated vision loss is at the age, and/or within the age range described above. In certain embodiments, visual acuity is assessed in a patient up to 5 years old presenting with Batten-CLN7-associated vision loss by measuring OKN after the patient has been treated with an AAV, preferably AAV8 or AAV9, encoding MFSD8. In certain embodiments, a visual acuity assessment based on OKN determines that visual acuity does not decrease after treatment with AAV gene therapy. In certain embodiments, a visual acuity assessment based on OKN determines that visual acuity improves in a patient after treatment with AAV gene therapy by at least 5%, 10%, 15%, 20%, 25%, 30%, 35%, 40% 45%, 50%, 55%, 60% , 65%, 70%, 75%, 80%, 85%, 90%, 95% or at least 100%. In certain embodiments, a visual acuity assessment based on OKN determines that visual acuity does not further deteriorate in a patient after treatment with AAV gene therapy.

If the human patient is a child, visual function can be assessed using an optokinetic nystagmus (OKN)-based approach or a modified OKN-based approach.

### 6.2 TREATMENT SYSTEM, DEVICE, OR APPARATUS TO BE USED FOR A TREATMENT METHOD DESCRIBED HEREIN

Also provided herein are treatment system, devices, and apparatuses to be used for a treatment method described herein, which may comprise one or more of the following: bottles, tubes, light source, microinjector, and foot pedal. In certain embodiments, the light source is a self-illuminating contact lens, which can be used to deposit vector in the back of the eye and in particular and to avoid damaging the optic disc, fovea and/or macula (*see, e.g.,* Chalam et al., 2004, Ophthalmic surgery and lasers. 35. 76-77, which is incorporated by reference herein in its entirety). In certain embodiments, a self-illuminating contact lens is utilized during peripheral injection for visualizing the subretinal injection (*see, e.g*., Chalam et al., 2004, Ophthalmic surgery and lasers. 35. 76-77, which is incorporated by reference herein in its entirety). In certain embodiments, an optic fiber chandelier is utilized via a second trocar for visualizing the subretinal injection.

### 6.3 DELIVERY OF ANTI-VEGF ANTIBODY OR ANTIGEN-BINDING FRAGMENT

In certain embodiments, the therapeutic product is a fully human post-translationally modified (HuPTM) antibody against VEGF. In a specific embodiment, the pathology of the eye is associated with an ocular disease caused by increased neovascularization, for example, nAMD (also known as "wet" AMD), dry AMD, retinal vein occlusion (RVO), diabetic macular edema (DME), or diabetic retinopathy (DR) (in particular, wet AMD). The embodiments/aspects described in other sections of this disclosure are incorporated herein in this section to the extent they are applicable to the delivery of anti-VEGF antibodies or antigen-binding fragments. Described below are certain additional embodiments applicable to the delivery of anti-VEGF antibodies or antigen-binding fragments.

In a preferred embodiment, the fully human post-translationally modified antibody against VEGF is a fully human post-translationally modified antigen-binding fragment of a monoclonal antibody (mAb) against VEGF ("HuPTMFabVEGFi"). In a further preferred embodiment, the HuPTMFabVEGFi is a fully human glycosylated antigen-binding fragment of an anti-VEGF mAb ("HuGlyFabVEGFi"). *See,* also, International Patent Application Publication No. WO/2017/180936 (International Patent Application No. PCT/US2017/027529, filed April 14, 2017), and International Patent Application Publication No. WO/2017/181021 (International Patent Application No. PCT/US2017/027650, filed April 14, 2017), each of which is incorporated by reference herein in its entirety, for compositions and methods that can be used according to the invention described herein. In an alternative embodiment, full-length mAbs can be used.

Subjects to whom such gene therapy is administered should be those responsive to anti-VEGF therapy. In particular embodiments, the methods encompass treating patients who have been diagnosed with wet AMD, dry AMD, retinal vein occlusion (RVO), diabetic macular edema (DME), or diabetic retinopathy (DR) (in particular, wet AMD) and identified as responsive to treatment with an anti-VEGF antibody. In more specific embodiments, the patients are responsive to treatment with an anti-VEGF antigen-binding fragment. In certain embodiments, the patients have been shown to be responsive to treatment with an anti-VEGF antigen-binding fragment injected intravitreally prior to treatment with gene therapy. In specific embodiments, the patients have previously been treated with LUCENTIS ^{®} (ranibizumab), EYLEA^{®} (aflibercept), and/or AVASTIN^{®} (bevacizumab), and have been found to be responsive to one or more of said LUCENTIS ^{®} (ranibizumab), EYLEA^{®} (aflibercept), and/or AVASTIN^{®} (bevacizumab).

Subjects to whom such recombinant viral vector or other DNA expression construct is delivered should be responsive to the anti-VEGF antigen-binding fragment encoded by the transgene in the recombinant viral vector or expression construct. To determine responsiveness, the anti-hVEGF antigen-binding fragment transgene product (*e.g.,* produced in cell culture, bioreactors, etc.) may be administered directly to the subject, such as by intravitreal injection.

The HuPTMFabVEGFi, *e.g*., HuGlyFabVEGFi, encoded by the transgene can include, but is not limited to an antigen-binding fragment of an antibody that binds to hVEGF, such as bevacizumab; an anti-hVEGF Fab moiety such as ranibizumab; or such bevacizumab or ranibizumab Fab moieties engineered to contain additional glycosylation sites on the Fab domain (*e.g.,* see Courtois et al., 2016, mAbs 8: 99-112 which is incorporated by reference herein in its entirety for it description of derivatives of bevacizumab that are hyperglycosylated on the Fab domain of the full length antibody).

The recombinant vector used for delivering the transgene should have a tropism for human retinal cells or photoreceptor cells. Such vectors can include non-replicating recombinant adeno-associated virus vectors ("rAAV"), particularly those bearing an AAV8 capsid are preferred. However, other recombinant viral vectors may be used, including but not limited to recombinant lentiviral vectors, vaccinia viral vectors, or non-viral expression vectors referred to as "naked DNA" constructs. Preferably, the HuPTMFabVEGFi, *e.g*., HuGlyFabVEGFi, transgene should be controlled by appropriate expression control elements, for example, the CB7 promoter (a chicken β-actin promoter and CMV enhancer), the RPE65 promoter, or opsin promoter to name a few, and can include other expression control elements that enhance expression of the transgene driven by the vector (*e.g*., introns such as the chicken β-actin intron, minute virus of mice (MVM) intron, human factor IX intron (*e.g.,* FIX truncated intron 1), β-globin splice donor/immunoglobulin heavy chain spice acceptor intron, adenovirus splice donor /immunoglobulin splice acceptor intron, SV40 late splice donor /splice acceptor (19S/16S) intron, and hybrid adenovirus splice donor/IgG splice acceptor intron and polyA signals such as the rabbit β-globin polyA signal, human growth hormone (hGH) polyA signal, SV40 late polyA signal, synthetic polyA (SPA) signal, and bovine growth hormone (bGH) polyA signal). *See, e.g.,* Powell and Rivera-Soto, 2015, Discov. Med., 19(102):49-57.

In preferred embodiments, gene therapy constructs are designed such that both the heavy and light chains are expressed. More specifically, the heavy and light chains should be expressed at about equal amounts, in other words, the heavy and light chains are expressed at approximately a 1:1 ratio of heavy chains to light chains. The coding sequences for the heavy and light chains can be engineered in a single construct in which the heavy and light chains are separated by a cleavable linker or IRES so that separate heavy and light chain polypeptides are expressed. *See, e.g.,* Section 6.1.2 for specific leader sequences and specific IRES, 2A, and other linker sequences that can be used with the methods and compositions provided herein.

Without being bound by theory, in certain embodiments, the methods and compositions provided herein for the delivery of anti-VEGF antibodies or antigen-binding fragments are based, in part, on the following principles:
(i) Human retinal cells are secretory cells that possess the cellular machinery for post-translational processing of secreted proteins - including glycosylation and tyrosine-O-sulfation, a robust process in retinal cells. (See, *e.g.,* Wang et al., 2013, Analytical Biochem. 427: 20-28 and Adamis et al., 1993, BBRC 193: 631-638 reporting the production of glycoproteins by retinal cells; and Kanan et al., 2009, Exp. Eye Res. 89: 559-567 and Kanan & Al-Ubaidi, 2015, Exp. Eye Res. 133: 126-131 reporting the production of tyrosine-sulfated glycoproteins secreted by retinal cells, each of which is incorporated by reference in its entirety for post-translational modifications made by human retinal cells).
(ii) Contrary to the state of the art understanding, anti-VEGF antigen-binding fragments, such as ranibizumab (and the Fab domain of full length anti-VEGF mAbs such as bevacizumab) do indeed possess N-linked glycosylation sites. For example, see FIG. 1 which identifies non-consensus asparaginal ("N") glycosylation sites in the C_{H} domain (TVSWN¹⁶⁵SGAL) and in the C_{L} domain (QSGN¹⁵⁸SQE), as well as glutamine ("Q") residues that are glycosylation sites in the V_{H} domain (Q¹¹⁵GT) and V_{L} domain (TFQ¹⁰⁰GT) of ranibizumab (and corresponding sites in the Fab of bevacizumab). (*See, e.g.,* Valliere-Douglass et al., 2009, J. Biol. Chem. 284: 32493-32506, and Valliere-Douglass et al., 2010, J. Biol. Chem. 285: 16012-16022, each of which is incorporated by reference in its entirety for the identification of N-linked glycosylation sites in antibodies).
(iii)While such non-canonical sites usually result in low level glycosylation (*e.g.,* about 1-5%) of the antibody population, the functional benefits may be significant in immunoprivileged organs, such as the eye (See, *e.g.,* van de Bovenkamp et al., 2016, J. Immunol. 196:1435-1441). For example, Fab glycosylation may affect the stability, half-life, and binding characteristics of an antibody. To determine the effects of Fab glycosylation on the affinity of the antibody for its target, any technique known to one of skill in the art may be used, for example, enzyme linked immunosorbent assay (ELISA), or surface plasmon resonance (SPR). To determine the effects of Fab glycosylation on the half-life of the antibody, any technique known to one of skill in the art may be used, for example, by measurement of the levels of radioactivity in the blood or organs (*e.g.,* the eye) in a subject to whom a radiolabelled antibody has been administered. To determine the effects of Fab glycosylation on the stability, for example, levels of aggregation or protein unfolding, of the antibody, any technique known to one of skill in the art may be used, for example, differential scanning calorimetry (DSC), high performance liquid chromatography (HPLC), *e.g.,* size exclusion high performance liquid chromatography (SEC-HPLC), capillary electrophoresis, mass spectrometry, or turbidity measurement. Provided herein, the HuPTMFabVEGFi, *e.g*., HuGlyFabVEGFi, transgene results in production of a Fab which is 0.5%, 1%, 2%, 3%, 4%, 5%, 6%, 7%, 8%, 9%, or 10% or more glycosylated at non-canonical sites. In certain embodiments, 0.5%, 1%, 2%, 3%, 4%, 5%, 6%, 7%, 8%, 9%, or 10% or more Fabs from a population of Fabs are glycosylated at non-canonical sites. In certain embodiments, 0.5%, 1%, 2%, 3%, 4%, 5%, 6%, 7%, 8%, 9%, or 10% or more non-canonical sites are glycosylated. In certain embodiments, the glycosylation of the Fab at these non-canonical sites is 25%, 50%, 100%, 200%, 300%, 400%, 500%, or more greater than the amount of glycosylation of these non-canonical sites in a Fab produced in HEK293 cells.
(iv)In addition to the glycosylation sites, anti-VEGF Fabs such as ranibizumab (and the Fab of bevacizumab) contain tyrosine ("Y") sulfation sites in or near the CDRs; see FIG. 1 which identifies tyrosine-O-sulfation sites in the V_{H} (EDTAVY⁹⁴Y⁹⁵) and V_{L} (EDFATY⁸⁶) domains of ranibizumab (and corresponding sites in the Fab of bevacizumab). (*See, e.g.,* Yang et al., 2015, Molecules 20:2138-2164, esp. at p. 2154 which is incorporated by reference in its entirety for the analysis of amino acids surrounding tyrosine residues subjected to protein tyrosine sulfation. The "rules" can be summarized as follows: Y residues with E or D within +5 to -5 position of Y, and where position -1 of Y is a neutral or acidic charged amino acid - but not a basic amino acid, *e.g.,* R, K, or H that abolishes sulfation). Human IgG antibodies can manifest a number of other post-translational modifications, such as N-terminal modifications, C-terminal modifications, degradation or oxidation of amino acid residues, cysteine related variants, and glycation (*See, e.g.,* Liu et al., 2014, mAbs 6(5):1145-1154).
(v) Glycosylation of anti-VEGF Fabs, such as ranibizumab or the Fab fragment of bevacizumab by human retinal cells will result in the addition of glycans that can improve stability, half-life and reduce unwanted aggregation and/or immunogenicity of the transgene product. (*See, e.g.,* Bovenkamp et al., 2016, J. Immunol. 196: 1435-1441 for a review of the emerging importance of Fab glycosylation). Significantly, glycans that can be added to HuPTMFabVEGFi, *e.g*., HuGlyFabVEGFi, provided herein, are highly processed complex-type biantennary N-glycans that contain 2,6-sialic acid (*e.g*., see FIG. 2 depicting the glycans that may be incorporated into HuPTMFabVEGFi, *e.g*., HuGlyFabVEGFi) and bisecting GlcNAc, but not NGNA (N-Glycolylneuraminic acid, Neu5Gc). Such glycans are not present in ranibizumab (which is made in *E. coli* and is not glycosylated at all) or in bevacizumab (which is made in CHO cells that do not have the 2,6-sialyltransferase required to make this post-translational modification, nor do CHO cells product bisecting GlcNAc, although they do add Neu5Gc (NGNA) as sialic acid not typical (and potentially immunogenic) to humans instead of Neu5Ac (NANA)). See, *e.g.,* Dumont et al., 2015, Crit. Rev. Biotechnol. (Early Online, published online September 18, 2015, pp. 1-13 at p. 5). Moreover, CHO cells can also produce an immunogenic glycan, the α-Gal antigen, which reacts with anti-α-Gal antibodies present in most individuals, and at high concentrations can trigger anaphylaxis. *See, e.g.,* Bosques, 2010, Nat Biotech 28: 1153-1156. The human glycosylation pattern of the HuPTMFabVEGFi, *e.g*., HuGlyFabVEGFi, provided herein, should reduce immunogenicity of the transgene product and improve efficacy.
(vi)Tyrosine-sulfation of anti-VEGF Fabs, such as ranibizumab or the Fab fragment of bevacizumab - a robust post-translational process in human retinal cells - could result in transgene products with increased avidity for VEGF. Indeed, tyrosine-sulfation of the Fab of therapeutic antibodies against other targets has been shown to dramatically increase avidity for antigen and activity. *(See, e.g.,* Loos et al., 2015, PNAS 112: 12675-12680, and Choe et al., 2003, Cell 114: 161-170). Such post-translational modifications are not present on ranibizumab (which is made in *E. coli* a host that does not possess the enzymes required for tyrosine-sulfation), and at best is under-represented in bevacizumab - a CHO cell product. Unlike human retinal cells, CHO cells are not secretory cells and have a limited capacity for post-translational tyrosine-sulfation. (*See, e.g.,* Mikkelsen & Ezban, 1991, Biochemistry 30: 1533-1537, esp. discussion at p. 1537).

For the foregoing reasons, the production of HuPTMFabVEGFi, *e.g*., HuGlyFabVEGFi, should result in a "biobetter" molecule for the treatment of wet AMD, dry AMD, retinal vein occlusion (RVO), diabetic macular edema (DME), or diabetic retinopathy (DR) (in particular, wet AMD) accomplished via gene therapy - *e.g.,* by administering a recombinant viral vector or a recombinant DNA expression construct encoding HuPTMFabVEGFi, *e.g*., HuGlyFabVEGFi, to the suprachoroidal space, subretinal space, or outer surface of the sclera in the eye(s) of patients (human subjects) diagnosed with wet AMD, dry AMD, retinal vein occlusion (RVO), diabetic macular edema (DME), or diabetic retinopathy (DR) (in particular, wet AMD), to create a permanent depot in the eye that continuously supplies the fully-human post-translationally modified, *e.g*., human-glycosylated, sulfated transgene product produced by transduced retinal cells. The cDNA construct for the FabVEGFi should include a signal peptide that ensures proper co- and post-translational processing (glycosylation and protein sulfation) by the transduced retinal cells. Such signal sequences used by retinal cells may include but are not limited to:
- MNFLLSWVHW SLALLLYLHH AKWSQA (VEGF-A signal peptide)
- MERAAPSRRV PLPLLLLGGL ALLAAGVDA (Fibulin-1 signal peptide)
- MAPLRPLLIL ALLAWVALA (Vitronectin signal peptide)
- MRLLAKIICLMLWAICVA (Complement Factor H signal peptide)
- MRLLAFLSLL ALVLQETGT (Opticin signal peptide)
- MKWVTFISLLFLFSSA YS (Albumin signal peptide)
- MAFLWLLSCWALLGTTFG (Chymotrypsinogen signal peptide)
- MYRMQLLSCIALILALVTNS (Interleukin-2 signal peptide)
- MNLLLILTFVAAAVA (Trypsinogen-2 signal peptide).
- *See, e.g.,* Stern et al., 2007, Trends Cell. Mol. Biol., 2:1-17 and Dalton & Barton, 2014, Protein Sci, 23: 517-525, each of which is incorporated by reference herein in its entirety for the signal peptides that can be used.

As an alternative, or an additional treatment to gene therapy, the HuPTMFabVEGFi product, *e.g*., HuGlyFabVEGFi glycoprotein, can be produced in human cell lines by recombinant DNA technology, and administered to patients diagnosed with wet AMD, dry AMD, retinal vein occlusion (RVO), diabetic macular edema (DME), or diabetic retinopathy (DR) (in particular, wet AMD) by intravitreal injection. The HuPTMFabVEGFi product, *e.g*., glycoprotein, may also be administered to patients with wet AMD, dry AMD, retinal vein occlusion (RVO), diabetic macular edema (DME), or diabetic retinopathy (DR) (in particular, wet AMD). Human cell lines that can be used for such recombinant glycoprotein production include but are not limited to human embryonic kidney 293 cells (HEK293), fibrosarcoma HT-1080, HKB-11, CAP, HuH-7, and retinal cell lines, PER.C6, or RPE to name a few (*e.g.,* see Dumont et al., 2015, Crit. Rev. Biotechnol. (Early Online, published online September 18, 2015, pp. 1-13) "Human cell lines for biopharmaceutical manufacturing: history, status, and future perspectives" which is incorporated by reference in its entirety for a review of the human cell lines that could be used for the recombinant production of the HuPTMFabVEGFi product, *e.g.,* HuGlyFabVEGFi glycoprotein). To ensure complete glycosylation, especially sialylation, and tyrosine-sulfation, the cell line used for production can be enhanced by engineering the host cells to co-express α-2,6-sialyltransferase (or both α-2,3- and α-2,6-sialyltransferases) and/or TPST-1 and TPST-2 enzymes responsible for tyrosine-O-sulfation in retinal cells.

Combinations of delivery of the HuPTMFabVEGFi, *e.g*., HuGlyFabVEGFi, to the eye/retina accompanied by delivery of other available treatments are encompassed by the methods provided herein. The additional treatments may be administered before, concurrently or subsequent to the gene therapy treatment. Available treatments for wet AMD, dry AMD, retinal vein occlusion (RVO), diabetic macular edema (DME), or diabetic retinopathy (DR) (in particular, wet AMD) that could be combined with the gene therapy provided herein include but are not limited to laser photocoagulation, photodynamic therapy with verteporfin, and intravitreal (IVT) injections with anti-VEGF agents, including but not limited to pegaptanib, ranibizumab, aflibercept, or bevacizumab. Additional treatments with anti-VEGF agents, such as biologics, may be referred to as "rescue" therapy.

### 6.3.1 N-GLYCOSYLATION, TYROSINE SULFATION, AND O-GLYCOSYLATION

The amino acid sequence (primary sequence) of the anti-VEGF antigen-binding fragment of a HuPTMFabVEGFi, *e.g*., HuGlyFabVEGFi, used in the methods described herein comprises at least one site at which N-glycosylation or tyrosine sulfation takes place. In certain embodiments, the amino acid sequence of the anti-VEGF antigen-binding fragment comprises at least one N-glycosylation site and at least one tyrosine sulfation site. Such sites are described in detail below. In certain embodiments, the amino acid sequence of the anti-VEGF antigen-binding fragment comprises at least one O-glycosylation site, which can be in addition to one or more N-glycosylation sites and/or tyrosine sulfation sites present in said amino acid sequence.

### (a) N-Glycosylation

### Reverse Glycosylation Sites

The canonical N-glycosylation sequence is known in the art to be Asn-X-Ser(or Thr), wherein X can be any amino acid except Pro. However, it recently has been demonstrated that asparagine (Asn) residues of human antibodies can be glycosylated in the context of a reverse consensus motif, Ser(or Thr)-X-Asn, wherein X can be any amino acid except Pro. See Valliere-Douglass et al., 2009, J. Biol. Chem. 284:32493-32506; and Valliere-Douglass et al., 2010, J. Biol. Chem. 285:16012-16022. As disclosed herein, and contrary to the state of the art understanding, anti-VEGF antigen-binding fragments for use in accordance with the methods described herein, *e.g*., ranibizumab, comprise several of such reverse consensus sequences. Accordingly, the methods described herein comprise use of anti-VEGF antigen-binding fragments that comprise at least one N-glycosylation site comprising the sequence Ser(or Thr)-X-Asn, wherein X can be any amino acid except Pro (also referred to herein as a "reverse N-glycosylation site").

In certain embodiments, the methods described herein comprise use of an anti-VEGF antigen-binding fragment that comprises one, two, three, four, five, six, seven, eight, nine, ten, or more than ten N-glycosylation sites comprising the sequence Ser(or Thr)-X-Asn, wherein X can be any amino acid except Pro. In certain embodiments, the methods described herein comprise use of an anti-VEGF antigen-binding fragment that comprises one, two, three, four, five, six, seven, eight, nine, ten, or more than ten reverse N-glycosylation sites, as well as one, two, three, four, five, six, seven, eight, nine, ten, or more than ten non-consensus N-glycosylation sites (as defined herein, below).

In a specific embodiment, the anti-VEGF antigen-binding fragment comprising one or more reverse N-glycosylation sites used in the methods described herein is ranibizumab, comprising a light chain and a heavy chain of SEQ ID NOs. 1 and 2, respectively. In another specific embodiment, the anti-VEGF antigen-binding fragment comprising one or more reverse N-glycosylation sites used in the methods comprises the Fab of bevacizumab, comprising a light chain and a heavy chain of SEQ ID NOs. 3 and 4, respectively.

### Non-Consensus Glycosylation Sites

In addition to reverse N-glycosylation sites, it recently has been demonstrated that glutamine (Gln) residues of human antibodies can be glycosylated in the context of a non-consensus motif, Gln-Gly-Thr. See Valliere-Douglass et al., 2010, J. Biol. Chem. 285:16012-16022. Surprisingly, anti-VEGF antigen-binding fragments for use in accordance with the methods described herein, *e.g.,* ranibizumab, comprise several of such non-consensus sequences. Accordingly, the methods described herein comprise use of anti-VEGF antigen-binding fragments that comprise at least one N-glycosylation site comprising the sequence Gln-Gly-Thr (also referred to herein as a "non-consensus N-glycosylation site").

In certain embodiments, the methods described herein comprise use of an anti-VEGF antigen-binding fragment that comprises one, two, three, four, five, six, seven, eight, nine, ten, or more than ten N-glycosylation sites comprising the sequence Gln-Gly-Thr.

In a specific embodiment, the anti-VEGF antigen-binding fragment comprising one or more non-consensus N-glycosylation sites used in the methods described herein is ranibizumab (comprising a light chain and a heavy chain of SEQ ID NOs. 1 and 2, respectively). In another specific embodiment, the anti-VEGF antigen-binding fragment comprising one or more non-consensus N-glycosylation sites used in the methods comprises the Fab of bevacizumab (comprising a light chain and a heavy chain of SEQ ID NOs. 3 and 4, respectively).

### Engineered N-Glycosylation Sites

In certain embodiments, a nucleic acid encoding an anti-VEGF antigen-binding fragment is modified to include 1, 2, 3, 4, 5, 6, 7, 8, 9, 10, or more N-glycosylation sites (including the canonical N-glycosylation consensus sequence, reverse N-glycosylation site, and non-consensus N-glycosylation sites) than would normally be associated with the HuGlyFabVEGFi (e.g., relative to the number of N-glycosylation sites associated with the anti-VEGF antigen-binding fragment in its unmodified state). In specific embodiments, introduction of glycosylation sites is accomplished by insertion of N-glycosylation sites (including the canonical N-glycosylation consensus sequence, reverse N-glycosylation site, and non-consensus N-glycosylation sites) anywhere in the primary structure of the antigen-binding fragment, so long as said introduction does not impact binding of the antigen-binding fragment to its antigen, VEGF. Introduction of glycosylation sites can be accomplished by, *e.g.,* adding new amino acids to the primary structure of the antigen-binding fragment, or the antibody from which the antigen-binding fragment is derived *(i.e.,* the glycosylation sites are added, in full or in part), or by mutating existing amino acids in the antigen-binding fragment, or the antibody from which the antigen-binding fragment is derived, in order to generate the N-glycosylation sites *(i.e.,* amino acids are not added to the antigen-binding fragment/antibody, but selected amino acids of the antigen-binding fragment/antibody are mutated so as to form N-glycosylation sites). Those of skill in the art will recognize that the amino acid sequence of a protein can be readily modified using approaches known in the art, e.g., recombinant approaches that include modification of the nucleic acid sequence encoding the protein.

In a specific embodiment, an anti-VEGF antigen-binding fragment used in the method described herein is modified such that, when expressed in retinal cells, it can be hyperglycosylated. See Courtois et al., 2016, mAbs 8:99-112 which is incorporated by reference herein in its entirety. In a specific embodiment, said anti-VEGF antigen-binding fragment is ranibizumab (comprising a light chain and a heavy chain of SEQ ID NOs. 1 and 2, respectively). In another specific embodiment, said anti-VEGF antigen-binding fragment comprises the Fab of bevacizumab (comprising a light chain and a heavy chain of SEQ ID NOs. 3 and 4, respectively).

### N-Glycosylation of anti-VEGF antigen-binding fragments

Unlike small molecule drugs, biologics usually comprise a mixture of many variants with different modifications or forms that have a different potency, pharmacokinetics, and safety profile. It is not essential that every molecule produced either in the gene therapy or protein therapy approach be fully glycosylated and sulfated. Rather, the population of glycoproteins produced should have sufficient glycosylation (including 2,6-sialylation) and sulfation to demonstrate efficacy. The goal of gene therapy treatment provided herein is to slow or arrest the progression of retinal degeneration, and to slow or prevent loss of vision with minimal intervention/invasive procedures.

In a specific embodiment, an anti-VEGF antigen-binding fragment, e.g., ranibizumab, used in accordance with the methods described herein, when expressed in a retinal cell, could be glycosylated at 100% of its N-glycosylation sites. However, one of skill in the art will appreciate that not every N-glycosylation site of an anti-VEGF antigen-binding fragment need be N-glycosylated in order for benefits of glycosylation to be attained. Rather, benefits of glycosylation can be realized when only a percentage of N-glycosylation sites are glycosylated, and/or when only a percentage of expressed antigen-binding fragments are glycosylated. Accordingly, in certain embodiments, an anti-VEGF antigen-binding fragment used in accordance with the methods described herein, when expressed in a retinal cell, is glycosylated at 10% - 20%, 20% - 30%, 30% - 40%, 40% - 50%, 50% - 60%, 60% - 70%, 70% - 80%, 80% - 90%, or 90% - 100% of it available N-glycosylation sites. In certain embodiments, when expressed in a retinal cell, 10% - 20%, 20% - 30%, 30% - 40%, 40% - 50%, 50% - 60%, 60% - 70%, 70% - 80%, 80% - 90%, or 90% - 100% of the an anti-VEGF antigen-binding fragments used in accordance with the methods described herein are glycosylated at least one of their available N-glycosylation sites.

In a specific embodiment, at least 10%, 20% 30%, 40%, 50%, 60%, 70%, 75%, 80%, 85%, 90%, 95%, or 99% of the N-glycosylation sites present in an anti-VEGF antigen-binding fragment used in accordance with the methods described herein are glycosylated at an Asn residue (or other relevant residue) present in an N-glycosylation site, when the anti-VEGF antigen-binding fragment is expressed in a retinal cell. That is, at least 50%, 60%, 70%, 75%, 80%, 85%, 90%, 95%, or 99% of the N-glycosylation sites of the resultant HuGlyFabVEGFi are glycosylated.

In another specific embodiment, at least 10%, 20% 30%, 40%, 50%, 60%, 70%, 75%, 80%, 85%, 90%, 95%, or 99% of the N-glycosylation sites present in an anti-VEGF antigen-binding fragment used in accordance with the methods described herein are glycosylated with an identical attached glycan linked to the Asn residue (or other relevant residue) present in an N-glycosylation site, when the anti-VEGF antigen-binding fragment is expressed in a retinal cell. That is, at least 50%, 60%, 70%, 75%, 80%, 85%, 90%, 95%, or 99% of the N-glycosylation sites of the resultant HuGlyFabVEGFi an identical attached glycan.

When an anti-VEGF antigen-binding fragment, e.g., ranibizumab, used in accordance with the methods described herein is expressed in a retinal cell, the N-glycosylation sites of the of the antigen-binding fragment can be glycosylated with various different glycans. N-glycans of antigen-binding fragments have been characterized in the art. For example, Bondt et al., 2014, Mol. & Cell. Proteomics 13.11:3029-3039 (incorporated by reference herein in its entirety for it disclosure of Fab-associated N-glycans) characterizes glycans associated with Fabs, and demonstrates that Fab and Fc portions of antibodies comprise distinct glycosylation patterns, with Fab glycans being high in galactosylation, sialylation, and bisection (e.g., with bisecting GlcNAc) but low in fucosylation with respect to Fc glycans. Like Bondt, Huang et al., 2006, Anal. Biochem. 349:197-207 (incorporated by reference herein in its entirety for it disclosure of Fab-associated N-glycans) found that most glycans of Fabs are sialylated. However, in the Fab of the antibody examined by Huang (which was produced in a murine cell background), the identified sialic residues were N-Glycolylneuraminic acid ("Neu5Gc" or "NeuGc") (which is not natural to humans) instead of N-acetylneuraminic acid ("Neu5Ac," the predominant human sialic acid). In addition, Song et al., 2014, Anal. Chem. 86:5661-5666 (incorporated by reference herein in its entirety for it disclosure of Fab-associated N-glycans) describes a library of N-glycans associated with commercially available antibodies.

Importantly, when the anti-VEGF antigen-binding fragments, *e.g*., ranibizumab, used in accordance with the methods described herein are expressed in human retinal cells, the need for *in vitro* production in prokaryotic host cells *(e.g., E. coli)* or eukaryotic host cells *(e.g.,* CHO cells) is circumvented. Instead, as a result of the methods described herein *(e.g.,* use of retinal cells to express anti-hVEGF antigen-binding fragments), N-glycosylation sites of the anti-VEGF antigen-binding fragments are advantageously decorated with glycans relevant to and beneficial to treatment of humans. Such an advantage is unattainable when CHO cells or *E. coli* are utilized in antibody/antigen-binding fragment production, because e.g., CHO cells (1) do not express 2,6 sialyltransferase and thus cannot add 2,6 sialic acid during N-glycosylation and (2) can add Neu5Gc as sialic acid instead of Neu5Ac; and because *E. coli* does not naturally contain components needed for N-glycosylation. Accordingly, in one embodiment, an anti-VEGF antigen-binding fragment expressed in a retinal cell to give rise to a HuGlyFabVEGFi used in the methods of treatment described herein is glycosylated in the manner in which a protein is N-glycosylated in human retinal cells, e.g., retinal pigment cells, but is not glycosylated in the manner in which proteins are glycosylated in CHO cells. In another embodiment, an anti-VEGF antigen-binding fragment expressed in a retinal cell to give rise to a HuGlyFabVEGFi used in the methods of treatment described herein is glycosylated in the manner in which a protein is N-glycosylated in human retinal cells, *e.g.,* retinal pigment cells, wherein such glycosylation is not naturally possible using a prokaryotic host cell, *e.g.,* using *E. coli.*

In certain embodiments, a HuGlyFabVEGFi, e.g., ranibizumab, used in accordance with the methods described herein comprises one, two, three, four, five or more distinct N-glycans associated with Fabs of human antibodies. In a specific embodiment, said N-glycans associated with Fabs of human antibodies are those described in Bondt et al., 2014, Mol. & Cell. Proteomics 13.11:3029-3039, Huang et al., 2006, Anal. Biochem. 349:197-207, and/or Song et al., 2014, Anal. Chem. 86:5661-5666. In certain embodiments, a HuGlyFabVEGFi, *e.g.,* ranibizumab, used in accordance with the methods described herein does not comprise detectable NeuGc and/or α-Gal antigen.

In a specific embodiment, the HuGlyFabVEGFi, *e.g.*, ranibizumab, used in accordance with the methods described herein are predominantly glycosylated with a glycan comprising 2,6-linked sialic acid. In certain embodiments, HuGlyFabVEGFi comprising 2,6-linked sialic acid is polysialylated, *i.e.,* contains more than one sialic acid. In certain embodiments, each N-glycosylation site of said HuGlyFabVEGFi comprises a glycan comprising 2,6-linked sialic acid, *i.e.,* 100% of the N-glycosylation site of said HuGlyFabVEGFi comprise a glycan comprising 2,6-linked sialic acid. In another specific embodiment, at least 20%, 30%, 40%, 50%, 60%, 70%, 75%, 80%, 85%, 90%, 95%, or 99% of the N-glycosylation sites of a HuGlyFabVEGFi used in accordance with the methods described herein are glycosylated with a glycan comprising 2,6-linked sialic acid. In another specific embodiment, at least 10% - 20%, 20% - 30%, 30% - 40%, 40% - 50%, 50% - 60%, 60% - 70%, 70% - 80%, 80% - 90%, or 90% - 99% of the N-glycosylation sites of a HuGlyFabVEGFi used in accordance with the methods described herein are glycosylated with a glycan comprising 2,6-linked sialic acid. In another specific embodiment, at least 20%, 30%, 40%, 50%, 60%, 70%, 75%, 80%, 85%, 90%, 95%, or 99% of the antigen-binding fragments expressed in a retinal cell in accordance with methods described herein *(i.e.,* the antigen-binding fragments that give rise to HuGlyFabVEGFi, e.g., ranibizumab) are glycosylated with a glycan comprising 2,6-linked sialic acid. In another specific embodiment, at least 10% - 20%, 20% - 30%, 30% - 40%, 40% - 50%, 50% - 60%, 60% - 70%, 70% - 80%, 80% - 90%, or 90% - 99% of the antigen-binding fragments expressed in a retinal cell in accordance with methods described herein *(i.e.,* the Fabs that give rise to HuGlyFabVEGFi, e.g., ranibizumab) are glycosylated with a glycan comprising 2,6-linked sialic acid. In another specific embodiment, said sialic acid is Neu5Ac. In accordance with such embodiments, when only a percentage of the N-glycosylation sites of a HuGlyFabVEGFi are 2,6 sialylated or polysialylated, the remaining N-glycosylation can comprise a distinct N-glycan, or no N-glycan at all *(i.e.,* remain non-glycosylated).

When a HuGlyFabVEGFi is 2,6 polysialylated, it comprises multiple sialic acid residues, *e.g.,* 2, 3, 4, 5, 6, 7, 8, 9, 10 or more than 10 sialic acid residues. In certain embodiments, when a HuGlyFabVEGFi is polysialylated, it comprises 2-5, 5-10, 10-20, 20-30, 30-40, or 40-50 sialic acid residues. In certain embodiments, when a HuGlyFabVEGFi is polysialylated, it comprises 2,6-linked (sialic acid)ⁿ, wherein n can be any number from 1-100.

In a specific embodiment, the HuGlyFabVEGFi, e.g., ranibizumab, used in accordance with the methods described herein are predominantly glycosylated with a glycan comprising a bisecting GlcNAc. In certain embodiments, each N-glycosylation site of said HuGlyFabVEGFi comprises a glycan comprising a bisecting GlcNAc, *i.e.,* 100% of the N-glycosylation site of said HuGlyFabVEGFi comprise a glycan comprising a bisecting GlcNAc. In another specific embodiment, at least 20%, 30%, 40%, 50%, 60%, 70%, 75%, 80%, 85%, 90%, 95%, or 99% of the N-glycosylation sites of a HuGlyFabVEGFi used in accordance with the methods described herein are glycosylated with a glycan comprising a bisecting GlcNAc. In another specific embodiment, at least 10% - 20%, 20% - 30%, 30% - 40%, 40% - 50%, 50% - 60%, 60% - 70%, 70% - 80%, 80% - 90%, or 90% - 99% of the N-glycosylation sites of a HuGlyFabVEGFi used in accordance with the methods described herein are glycosylated with a glycan comprising a bisecting GlcNAc. In another specific embodiment, at least 20%, 30%, 40%, 50%, 60%, 70%, 75%, 80%, 85%, 90%, 95%, or 99% of the antigen-binding fragments expressed in a retinal cell in accordance with methods described herein *(i.e.,* the antigen-binding fragments that give rise to HuGlyFabVEGFi, *e.g.*, ranibizumab) are glycosylated with a glycan comprising a bisecting GlcNAc. In another specific embodiment, at least 10% - 20%, 20% - 30%, 30% - 40%, 40% - 50%, 50% - 60%, 60% - 70%, 70% - 80%, 80% - 90%, or 90% - 99% of the antigen-binding fragments expressed in a retinal cell in accordance with methods described herein *(i.e.,* the antigen-binding fragments that give rise to HuGlyFabVEGFi, *e.g.,* ranibizumab) are glycosylated with a glycan comprising a bisecting GlcNAc.

In certain embodiments, the HuGlyFabVEGFi, *e.g.*, ranibizumab, used in accordance with the methods described herein are hyperglycosylated, *i.e.,* in addition to the N-glycosylation resultant from the naturally occurring N-glycosylation sites, said HuGlyFabVEGFi comprise glycans at N-glycosylation sites engineered to be present in the amino acid sequence of the antigen-binding fragment giving rise to HuGlyFabVEGFi. In certain embodiments, the HuGlyFabVEGFi, e.g., ranibizumab, used in accordance with the methods described herein is hyperglycosylated but does not comprise detectable NeuGc and/or α-Gal antigen.

Assays for determining the glycosylation pattern of antibodies, including antigen-binding fragments are known in the art. For example, hydrazinolysis can be used to analyze glycans. First, polysaccharides are released from their associated protein by incubation with hydrazine (the Ludger Liberate Hydrazinolysis Glycan Release Kit, Oxfordshire, UK can be used). The nucleophile hydrazine attacks the glycosidic bond between the polysaccharide and the carrier protein and allows release of the attached glycans. N-acetyl groups are lost during this treatment and have to be reconstituted by re-N-acetylation. Glycans may also be released using enzymes such as glycosidases or endoglycosidases, such as PNGase F and Endo H, which cleave cleanly and with fewer side reactions than hydrazines. The free glycans can be purified on carbon columns and subsequently labeled at the reducing end with the fluorophor 2-amino benzamide. The labeled polysaccharides can be separated on a GlycoSep-N column (GL Sciences) according to the HPLC protocol of Royle et al, Anal Biochem 2002, 304(1):70-90. The resulting fluorescence chromatogram indicates the polysaccharide length and number of repeating units. Structural information can be gathered by collecting individual peaks and subsequently performing MS/MS analysis. Thereby the monosaccharide composition and sequence of the repeating unit can be confirmed and additionally in homogeneity of the polysaccharide composition can be identified. Specific peaks of low or high molecular weight can be analyzed by MALDI-MS/MS and the result used to confirm the glycan sequence. Each peak in the chromatogram corresponds to a polymer, *e.g.,* glycan, consisting of a certain number of repeat units and fragments, *e.g.,* sugar residues, thereof. The chromatogram thus allows measurement of the polymer, *e.g.,* glycan, length distribution. The elution time is an indication for polymer length, while fluorescence intensity correlates with molar abundance for the respective polymer, *e.g.,* glycan. Other methods for assessing glycans associated with antigen-binding fragments include those described by Bondt et al., 2014, Mol. & Cell. Proteomics 13.11:3029-3039, Huang et al., 2006, Anal. Biochem. 349:197-207, and/or Song et al., 2014, Anal. Chem. 86:5661-5666.

Homogeneity or heterogeneity of the glycan patterns associated with antibodies (including antigen-binding fragments), as it relates to both glycan length or size and numbers glycans present across glycosylation sites, can be assessed using methods known in the art, *e.g.,* methods that measure glycan length or size and hydrodynamic radius. HPLC, such as Size exclusion, normal phase, reversed phase, and anion exchange HPLC, as well as capillary electrophoresis, allows the measurement of the hydrodynamic radius. Higher numbers of glycosylation sites in a protein lead to higher variation in hydrodynamic radius compared to a carrier with less glycosylation sites. However, when single glycan chains are analyzed, they may be more homogenous due to the more controlled length. Glycan length can be measured by hydrazinolysis, SDS PAGE, and capillary gel electrophoresis. In addition, homogeneity can also mean that certain glycosylation site usage patterns change to a broader/narrower range. These factors can be measured by Glycopeptide LC-MS/MS.

### Benefits of N-Glycosylation

N-glycosylation confers numerous benefits on the HuGlyFabVEGFi used in the methods described herein. Such benefits are unattainable by production of antigen-binding fragments in *E. coli,* because *E. coli* does not naturally possess components needed for N-glycosylation. Further, some benefits are unattainable through antibody production in, *e.g.,* CHO cells, because CHO cells lack components needed for addition of certain glycans *(e.g.,* 2,6 sialic acid and bisecting GlcNAc) and because CHO cells can add glycans, *e.g.,* Neu5Gc not typical to humans. *See, e.g.,* Song et al., 2014, Anal. Chem. 86:5661-5666. Accordingly, by virtue of the discovery set forth herein that anti-VEGF antigen-binding fragments, *e.g.,* ranibizumab, comprise non-canonical N-glycosylation sites (including both reverse and non-consensus glycosylation sites), a method of expressing such anti-VEGF antigen-binding fragments in a manner that results in their glycosylation (and thus improved benefits associated with the antigen-binding fragments) has been realized. In particular, expression of anti-VEGF antigen-binding fragments in human retinal cells results in the production of HuGlyFabVEGFi *(e.g.,* ranibizumab) comprising beneficial glycans that otherwise would not be associated with the antigen-binding fragments or their parent antibody.

While non-canonical glycosylation sites usually result in low level glycosylation *(e.g.,* 1-5%) of the antibody population, the functional benefits may be significant in immunoprivileged organs, such as the eye *(See, e.g.,* van de Bovenkamp et al., 2016, J. Immunol. 196:1435-1441). For example, Fab glycosylation may affect the stability, half-life, and binding characteristics of an antibody. To determine the effects of Fab glycosylation on the affinity of the antibody for its target, any technique known to one of skill in the art may be used, for example, enzyme linked immunosorbent assay (ELISA), or surface plasmon resonance (SPR). To determine the effects of Fab glycosylation on the half-life of the antibody, any technique known to one of skill in the art may be used, for example, by measurement of the levels of radioactivity in the blood or organs *(e.g.,* the eye) in a subject to whom a radiolabeled antibody has been administered. To determine the effects of Fab glycosylation on the stability, for example, levels of aggregation or protein unfolding, of the antibody, any technique known to one of skill in the art may be used, for example, differential scanning calorimetry (DSC), high performance liquid chromatography (HPLC), *e.g.,* size exclusion high performance liquid chromatography (SEC-HPLC), capillary electrophoresis, mass spectrometry, or turbidity measurement. Provided herein, the HuGlyFabVEGFi transgene results in production of an antigen-binding fragment which is 0.5%, 1%, 2%, 3%, 4%, 5%, 6%, 7%, 8%, 9%, or 10% or more glycosylated at non-canonical sites. In certain embodiments, 0.5%, 1%, 2%, 3%, 4%, 5%, 6%, 7%, 8%, 9%, or 10% or more antigen-binding fragments from a population of antigen-binding fragments are glycosylated at non-canonical sites. In certain embodiments, 0.5%, 1%, 2%, 3%, 4%, 5%, 6%, 7%, 8%, 9%, or 10% or more non-canonical sites are glycosylated. In certain embodiments, the glycosylation of the antigen-binding fragment at these non-canonical sites is 25%, 50%, 100%, 200%, 300%, 400%, 500%, or more greater than the amount of glycosylation of these non-canonical sites in an antigen-binding fragment produced in HEK293 cells.

The presence of sialic acid on HuGlyFabVEGFi used in the methods described herein can impact clearance rate of the HuGlyFabVEGFi, *e.g.,* the rate of clearance from the vitreous humour. Accordingly, sialic acid patterns of a HuGlyFabVEGFi can be used to generate a therapeutic having an optimized clearance rate. Method of assessing antigen-binding fragment clearance rate are known in the art. *See, e.g.,* Huang et al., 2006, Anal. Biochem. 349:197-207.

In another specific embodiment, a benefit conferred by N-glycosylation is reduced aggregation. Occupied N-glycosylation sites can mask aggregation prone amino acid residues, resulting in decreased aggregation. Such N-glycosylation sites can be native to an antigen-binding fragment used herein, or engineered into an antigen-binding fragment used herein, resulting in HuGlyFabVEGFi that is less prone to aggregation when expressed, *e.g.,* expressed in retinal cells. Methods of assessing aggregation of antibodies are known in the art. *See, e.g.,* Courtois et al., 2016, mAbs 8:99-112 which is incorporated by reference herein in its entirety.

In another specific embodiment, a benefit conferred by N-glycosylation is reduced immunogenicity. Such N-glycosylation sites can be native to an antigen-binding fragment used herein, or engineered into an antigen-binding fragment used herein, resulting in HuGlyFabVEGFi that is less prone to immunogenicity when expressed, *e.g.,* expressed in retinal cells.

In another specific embodiment, a benefit conferred by N-glycosylation is protein stability. N-glycosylation of proteins is well-known to confer stability on them, and methods of assessing protein stability resulting from N-glycosylation are known in the art. *See, e.g.,* Sola and Griebenow, 2009, J Pharm Sci., 98(4): 1223-1245.

In another specific embodiment, a benefit conferred by N-glycosylation is altered binding affinity. It is known in the art that the presence of N-glycosylation sites in the variable domains of an antibody can increase the affinity of the antibody for its antigen. *See, e.g.,* Bovenkamp et al., 2016, J. Immunol. 196:1435-1441. Assays for measuring antibody binding affinity are known in the art. *See, e.g.,* Wright et al., 1991, EMBO J. 10:2717-2723; and Leibiger et al., 1999, Biochem. J. 338:529-538.

### (b) Tyrosine Sulfation

Tyrosine sulfation occurs at tyrosine (Y) residues with glutamate (E) or aspartate (D) within +5 to -5 position of Y, and where position -1 of Y is a neutral or acidic charged amino acid, but not a basic amino acid, *e.g.,* arginine (R), lysine (K), or histidine (H) that abolishes sulfation. Surprisingly, anti-VEGF antigen-binding fragments for use in accordance with the methods described herein, *e.g.,* ranibizumab, comprise tyrosine sulfation sites (see Fig. 1). Accordingly, the methods described herein comprise use of anti-VEGF antigen-binding fragments, *e.g.,* HuPTMFabVEGFi , that comprise at least one tyrosine sulfation site, such the anti-VEGF antigen-binding fragments, when expressed in retinal cells, can be tyrosine sulfated.

Importantly, tyrosine-sulfated antigen-binding fragments, *e.g.,* ranibizumab, cannot be produced in *E. coli,* which naturally does not possess the enzymes required for tyrosine-sulfation. Further, CHO cells are deficient for tyrosine sulfation-they are not secretory cells and have a limited capacity for post-translational tyrosine-sulfation. *See, e.g.,* Mikkelsen & Ezban, 1991, Biochemistry 30: 1533-1537. Advantageously, the methods provided herein call for expression of anti-VEGF antigen-binding fragments, *e.g.,* HuPTMFabVEGFi , for example, ranibizumab, in retinal cells, which are secretory and do have capacity for tyrosine sulfation. See Kanan et al., 2009, Exp. Eye Res. 89: 559-567 and Kanan & Al-Ubaidi, 2015, Exp. Eye Res. 133: 126-131 reporting the production of tyrosine-sulfated glycoproteins secreted by retinal cells.

Tyrosine sulfation is advantageous for several reasons. For example, tyrosine-sulfation of the antigen-binding fragment of therapeutic antibodies against targets has been shown to dramatically increase avidity for antigen and activity. *See, e.g.,* Loos et al., 2015, PNAS 112: 12675-12680, and Choe et al., 2003, Cell 114: 161-170. Assays for detection tyrosine sulfation are known in the art. *See, e.g.,* Yang et al., 2015, Molecules 20:2138-2164.

### (c) O-Glycosylation

O-glycosylation comprises the addition of N-acetyl-galactosamine to serine or threonine residues by the enzyme. It has been demonstrated that amino acid residues present in the hinge region of antibodies can be O-glycosylated. In certain embodiments, the anti-VEGF antigen-binding fragments, *e.g.,* ranibizumab, used in accordance with the methods described herein comprise all or a portion of their hinge region, and thus are capable of being O-glycosylated when expressed in human retinal cells. The possibility of O-glycosylation confers another advantage to the HuPTMFabVEGFi, *e.g.,* HuGlyFabVEGFi, provided herein, as compared to, *e.g.,* antigen-binding fragments produced in *E. coli,* again because the *E. coli* naturally does not contain machinery equivalent to that used in human O-glycosylation. (Instead, O-glycosylation in *E. coli* has been demonstrated only when the bacteria is modified to contain specific O-glycosylation machinery. *See, e.g.,* Faridmoayer et al., 2007, J. Bacteriol. 189:8088-8098.) O-glycosylated HuPTMFabVEGFi, e.g., HuGlyFabVEGFi, by virtue of possessing glycans, shares advantageous characteristics with N-glycosylated HuGlyFabVEGFi (as discussed above).

### 6.3.2 CONSTRUCTS AND FORMULATIONS

In some aspects, the disclosure provides for a nucleic acid for use, wherein the nucleic acid encodes a HuPTMFabVEGFi, *e.g.,* HuGlyFabVEGFi operatively linked to a promoter selected from the group consisting of: cytomegalovirus (CMV) promoter, Rous sarcoma virus (RSV) promoter, MMT promoter, EF-1 alpha promoter, UB6 promoter, chicken beta-actin promoter, CAG promoter, RPE65 promoter and opsin promoter.

In a specific embodiment, the recombinant vectors described herein comprise the following components: (1) AAV2 inverted terminal repeats that flank the expression cassette; (2) Control elements, which include a) the CB7 promoter, comprising the CMV enhancer/chicken β-actin promoter, b) a chicken β-actin intron and c) a rabbit β-globin poly A signal; and (3) nucleic acid sequences coding for the heavy and light chains of anti-VEGF antigen-binding fragment, separated by a self-cleaving furin (F)/F2A linker, ensuring expression of equal amounts of the heavy and the light chain polypeptides.

The HuPTMFabVEGFi, *e.g.,* HuGlyFabVEGFi encoded by the transgene can include, but is not limited to an antigen-binding fragment of an antibody that binds to VEGF, such as bevacizumab; an anti-VEGF Fab moiety such as ranibizumab; or such bevacizumab or ranibizumab Fab moieties engineered to contain additional glycosylation sites on the Fab domain *(e.g.,* see Courtois et al., 2016, mAbs 8: 99-112 which is incorporated by reference herein in its entirety for it description of derivatives of bevacizumab that are hyperglycosylated on the Fab domain of the full length antibody).

In certain embodiments, the recombinant vectors provided herein encode an anti-VEGF antigen-binding fragment transgene. In specific embodiments, the anti-VEGF antigen-binding fragment transgene is controlled by appropriate expression control elements for expression in retinal cells: In certain embodiments, the anti-VEGF antigen-binding fragment transgene comprises bevacizumab Fab portion of the light and heavy chain cDNA sequences (SEQ ID NOs. 10 and 11, respectively). In certain embodiments, the anti-VEGF antigen-binding fragment transgene comprises ranibizumab light and heavy chain cDNA sequences (SEQ ID NOs. 12 and 13, respectively). In certain embodiments, the anti-VEGF antigen-binding fragment transgene encodes a bevacizumab Fab, comprising a light chain and a heavy chain of SEQ ID NOs: 3 and 4, respectively. In certain embodiments, the anti-VEGF antigen-binding fragment transgene encodes an antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 3. In certain embodiments, the anti-VEGF antigen-binding fragment transgene encodes an antigen-binding fragment comprising a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 4. In certain embodiments, the anti-VEGF antigen-binding fragment transgene encodes an antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 3 and a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 4. In certain embodiments, the anti-VEGF antigen-binding fragment transgene encodes a hyperglycosylated ranibizumab, comprising a light chain and a heavy chain of SEQ ID NOs: 1 and 2, respectively. In certain embodiments, the anti-VEGF antigen-binding fragment transgene encodes an antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 1. In certain embodiments, the anti-VEGF antigen-binding fragment transgene encodes an antigen-binding fragment comprising a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 2. In certain embodiments, the anti-VEGF antigen-binding fragment transgene encodes an antigen-binding fragment comprising a light chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 1 and a heavy chain comprising an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 2.

In certain embodiments, the anti-VEGF antigen-binding fragment transgene encodes a hyperglycosylated bevacizumab Fab, comprising a light chain and a heavy chain of SEQ ID NOs: 3 and 4, with one or more of the following mutations: L118N (heavy chain), E195N (light chain), or Q160N or Q160S (light chain). In certain embodiments, the anti-VEGF antigen-binding fragment transgene encodes a hyperglycosylated ranibizumab, comprising a light chain and a heavy chain of SEQ ID NOs: 1 and 2, with one or more of the following mutations: L118N (heavy chain), E195N (light chain), or Q160N or Q160S (light chain). The sequences of the antigen-binding fragment transgene cDNAs may be found, for example, in Table 2. In certain embodiments, the sequence of the antigen-binding fragment transgene cDNAs is obtained by replacing the signal sequence of SEQ ID NOs: 10 and 11 or SEQ ID NOs: 12 and 13 with one or more signal sequences listed in Table 1.

In certain embodiments, the anti-VEGF antigen-binding fragment transgene encodes an antigen-binding fragment and comprises the nucleotide sequences of the six bevacizumab CDRs. In certain embodiments, the anti-VEGF antigen-binding fragment transgene encodes an antigen-binding fragment and comprises the nucleotide sequences of the six ranibizumab CDRs. In certain embodiments, the anti-VEGF antigen-binding fragment transgene encodes an antigen-binding fragment comprising a heavy chain variable region comprising heavy chain CDRs 1-3 of ranibizumab (SEQ ID NOs: 20, 18, and 21). In certain embodiments, the anti-VEGF antigen-binding fragment transgene encodes an antigen-binding fragment comprising a light chain variable region comprising light chain CDRs 1-3 of ranibizumab (SEQ ID NOs: 14-16). In certain embodiments, the anti-VEGF antigen-binding fragment transgene encodes an antigen-binding fragment comprising a heavy chain variable region comprising heavy chain CDRs 1-3 of bevacizumab (SEQ ID NOs: 17-19). In certain embodiments, the anti-VEGF antigen-binding fragment transgene encodes an antigen-binding fragment comprising a light chain variable region comprising light chain CDRs 1-3 of bevacizumab (SEQ ID NOs: 14-16). In certain embodiments, the anti-VEGF antigen-binding fragment transgene encodes an antigen-binding fragment comprising a heavy chain variable region comprising heavy chain CDRs 1-3 of ranibizumab (SEQ ID NOs: 20, 18, and 21) and a light chain variable region comprising light chain CDRs 1-3 of ranibizumab (SEQ ID NOs: 14-16). In certain embodiments, the anti-VEGF antigen-binding fragment transgene encodes an antigen-binding fragment comprising a heavy chain variable region comprising heavy chain CDRs 1-3 of bevacizumab (SEQ ID NOs: 17-19) and a light chain variable region comprising light chain CDRs 1-3 of bevacizumab (SEQ ID NOs: 14-16).

In certain embodiments, the anti-VEGF antigen-binding fragment transgene encodes an antigen-binding fragment comprising a light chain variable region comprising light chain CDRs 1-3 of SEQ ID NOs: 14-16, wherein the second amino acid residue of the light chain CDR3 *(i.e.,* the second Q in QQYSTVPWTF (SEQ ID NO. 16)) does not carry one or more of the following chemical modifications: oxidation, acetylation, deamidation, and pyroglutamation (pyro Glu). In a specific embodiment, the anti-VEGF antigen-binding fragment transgene encodes an antigen-binding fragment comprising a light chain variable region comprising light chain CDRs 1-3 of SEQ ID NOs: 14-16, wherein the eighth and eleventh amino acid residues of the light chain CDR1 *(i.e.,* the two Ns in SASQDISNYLN (SEQ ID NO. 14) each carries one or more of the following chemical modifications: oxidation, acetylation, deamidation, and pyroglutamation (pyro Glu), and the second amino acid residue of the light chain CDR3 *(i.e.,* the second Q in QQYSTVPWTF (SEQ ID NO. 16)) does not carry one or more of the following chemical modifications: oxidation, acetylation, deamidation, and pyroglutamation (pyro Glu). In a specific embodiment, the anti-VEGF antigen-binding fragment transgene encodes an antigen-binding fragment comprising a light chain variable region comprising light chain CDRs 1-3 of SEQ ID NOs: 14-16, wherein the second amino acid residue of the light chain CDR3 *(i.e.,* the second Q in QQYSTVPWTF (SEQ ID NO. 16)) is not acetylated. In a specific embodiment, the anti-VEGF antigen-binding fragment transgene encodes an antigen-binding fragment comprising a light chain variable region comprising light chain CDRs 1-3 of SEQ ID NOs: 14-16, wherein the eighth and eleventh amino acid residues of the light chain CDR1 *(i.e.,* the two Ns in SASQDISNYLN (SEQ ID NO. 14) each carries one or more of the following chemical modifications: oxidation, acetylation, deamidation, and pyroglutamation (pyro Glu), and the second amino acid residue of the light chain CDR3 *(i.e.,* the second Q in QQYSTVPWTF (SEQ ID NO. 16)) is not acetylated. In a preferred embodiment, the chemical modification(s) or lack of chemical modification(s) (as the case may be) described herein is determined by mass spectrometry.

In certain embodiments, the anti-VEGF antigen-binding fragment transgene encodes an antigen-binding fragment comprising a heavy chain variable region comprising heavy chain CDRs 1-3 of SEQ ID NOs: 20, 18, and 21, wherein the last amino acid residue of the heavy chain CDR1 *(i.e.,* the N in GYDFTHYGMN (SEQ ID NO. 20)) does not carry one or more of the following chemical modifications: oxidation, acetylation, deamidation, and pyroglutamation (pyro Glu). In a specific embodiment, the anti-VEGF antigen-binding fragment transgene encodes an antigen-binding fragment comprising a heavy chain variable region comprising heavy chain CDRs 1-3 of SEQ ID NOs: 20, 18, and 21, wherein the ninth amino acid residue of the heavy chain CDR1 *(i.e.,* the M in GYDFTHYGMN (SEQ ID NO. 20)) carries one or more of the following chemical modifications: acetylation, deamidation, and pyroglutamation (pyro Glu), the third amino acid residue of the heavy chain CDR2 *(i.e.,* the N in WINTYTGEPTYAADFKR (SEQ ID NO. 18) carries one or more of the following chemical modifications: acetylation, deamidation, and pyroglutamation (pyro Glu), and the last amino acid residue of the heavy chain CDR1 *(i.e.,* the N in GYDFTHYGMN (SEQ ID NO. 20)) does not carry one or more of the following chemical modifications: oxidation, acetylation, deamidation, and pyroglutamation (pyro Glu). In a specific embodiment, the anti-VEGF antigen-binding fragment transgene encodes an antigen-binding fragment comprising a heavy chain variable region comprising heavy chain CDRs 1-3 of SEQ ID NOs: 20, 18, and 21, wherein the last amino acid residue of the heavy chain CDR1 *(i.e.,* the N in GYDFTHYGMN (SEQ ID NO. 20)) is not acetylated. In a specific embodiment, the anti-VEGF antigen-binding fragment transgene encodes an antigen-binding fragment comprising a heavy chain variable region comprising heavy chain CDRs 1-3 of SEQ ID NOs: 20, 18, and 21, wherein the ninth amino acid residue of the heavy chain CDR1 *(i.e.,* the M in GYDFTHYGMN (SEQ ID NO. 20)) carries one or more of the following chemical modifications: acetylation, deamidation, and pyroglutamation (pyro Glu), the third amino acid residue of the heavy chain CDR2 *(i.e.,* the N in WINTYTGEPTYAADFKR (SEQ ID NO. 18) carries one or more of the following chemical modifications: acetylation, deamidation, and pyroglutamation (pyro Glu), and the last amino acid residue of the heavy chain CDR1 *(i.e.,* the N in GYDFTHYGMN (SEQ ID NO. 20)) is not acetylated. In a preferred embodiment, the chemical modification(s) or lack of chemical modification(s) (as the case may be) described herein is determined by mass spectrometry.

In certain embodiments, the anti-VEGF antigen-binding fragment transgene encodes an antigen-binding fragment comprising a light chain variable region comprising light chain CDRs 1-3 of SEQ ID NOs: 14-16 and a heavy chain variable region comprising heavy chain CDRs 1-3 of SEQ ID NOs: 20, 18, and 21, wherein the second amino acid residue of the light chain CDR3 *(i.e.,* the second Q in QQYSTVPWTF (SEQ ID NO. 16)) does not carry one or more of the following chemical modifications: oxidation, acetylation, deamidation, and pyroglutamation (pyro Glu), and wherein the last amino acid residue of the heavy chain CDR1 *(i.e.,* the N in GYDFTHYGMN (SEQ ID NO. 20)) does not carry one or more of the following chemical modifications: oxidation, acetylation, deamidation, and pyroglutamation (pyro Glu). In a specific embodiment, the anti-VEGF antigen-binding fragment transgene encodes an antigen-binding fragment comprising a light chain variable region comprising light chain CDRs 1-3 of SEQ ID NOs: 14-16 and a heavy chain variable region comprising heavy chain CDRs 1-3 of SEQ ID NOs: 20, 18, and 21, wherein: (1) the ninth amino acid residue of the heavy chain CDR1 *(i.e.,* the M in GYDFTHYGMN (SEQ ID NO. 20)) carries one or more of the following chemical modifications: acetylation, deamidation, and pyroglutamation (pyro Glu), the third amino acid residue of the heavy chain CDR2 *(i.e.,* the N in WINTYTGEPTYAADFKR (SEQ ID NO. 18) carries one or more of the following chemical modifications: acetylation, deamidation, and pyroglutamation (pyro Glu), and the last amino acid residue of the heavy chain CDR1 *(i.e.,* the N in GYDFTHYGMN (SEQ ID NO. 20)) does not carry one or more of the following chemical modifications: oxidation, acetylation, deamidation, and pyroglutamation (pyro Glu); and (2) the eighth and eleventh amino acid residues of the light chain CDR1 *(i.e.,* the two Ns in SASQDISNYLN (SEQ ID NO. 14) each carries one or more of the following chemical modifications: oxidation, acetylation, deamidation, and pyroglutamation (pyro Glu), and the second amino acid residue of the light chain CDR3 *(i.e.,* the second Q in QQYSTVPWTF (SEQ ID NO. 16)) does not carry one or more of the following chemical modifications: oxidation, acetylation, deamidation, and pyroglutamation (pyro Glu). In a specific embodiment, the anti-VEGF antigen-binding fragment transgene encodes an antigen-binding fragment comprising a light chain variable region comprising light chain CDRs 1-3 of SEQ ID NOs: 14-16 and a heavy chain variable region comprising heavy chain CDRs 1-3 of SEQ ID NOs: 20, 18, and 21, wherein the second amino acid residue of the light chain CDR3 *(i.e.,* the second Q in QQYSTVPWTF (SEQ ID NO. 16)) is not acetylated, and wherein the last amino acid residue of the heavy chain CDR1 (i.e., the N in GYDFTHYGMN (SEQ ID NO. 20)) is not acetylated. In a specific embodiment, the antigen-binding fragment comprises a heavy chain CDR1 of SEQ ID NO. 20, wherein: (1) the ninth amino acid residue of the heavy chain CDR1 *(i.e.,* the M in GYDFTHYGMN (SEQ ID NO. 20)) carries one or more of the following chemical modifications: acetylation, deamidation, and pyroglutamation (pyro Glu), the third amino acid residue of the heavy chain CDR2 *(i.e.,* the N in WINTYTGEPTYAADFKR (SEQ ID NO. 18) carries one or more of the following chemical modifications: acetylation, deamidation, and pyroglutamation (pyro Glu), and the last amino acid residue of the heavy chain CDR1 *(i.e.,* the N in GYDFTHYGMN (SEQ ID NO. 20)) is not acetylated; and (2) the eighth and eleventh amino acid residues of the light chain CDR1 *(i.e.,* the two Ns in SASQDISNYLN (SEQ ID NO. 14) each carries one or more of the following chemical modifications: oxidation, acetylation, deamidation, and pyroglutamation (pyro Glu), and the second amino acid residue of the light chain CDR3 *(i.e.,* the second Q in QQYSTVPWTF (SEQ ID NO. 16)) is not acetylated. In a preferred embodiment, the chemical modification(s) or lack of chemical modification(s) (as the case may be) described herein is determined by mass spectrometry.

In certain aspects, also provided herein are anti-VEGF antigen-binding fragments comprising light chain CDRs 1-3 of SEQ ID NOs: 14-16 and heavy chain CDRs 1-3 of SEQ ID NOs: 20, 18, and 21, and transgenes encoding such antigen-VEGF antigen-binding fragments, wherein the second amino acid residue of the light chain CDR3 *(i.e.,* the second Q in QQYSTVPWTF (SEQ ID NO. 16)) does not carry one or more of the following chemical modifications: oxidation, acetylation, deamidation, and pyroglutamation (pyro Glu). In a specific embodiment, the antigen-binding fragment comprises light chain CDRs 1-3 of SEQ ID NOs: 14-16 and heavy chain CDRs 1-3 of SEQ ID NOs: 20, 18, and 21, wherein the eighth and eleventh amino acid residues of the light chain CDR1 *(i.e.,* the two Ns in SASQDISNYLN (SEQ ID NO. 14) each carries one or more of the following chemical modifications: oxidation, acetylation, deamidation, and pyroglutamation (pyro Glu), and the second amino acid residue of the light chain CDR3 *(i.e.,* the second Q in QQYSTVPWTF (SEQ ID NO. 16)) does not carry one or more of the following chemical modifications: oxidation, acetylation, deamidation, and pyroglutamation (pyro Glu). In a specific embodiment, the antigen-binding fragment comprises light chain CDRs 1-3 of SEQ ID NOs: 14-16 and heavy chain CDRs 1-3 of SEQ ID NOs: 20, 18, and 21, wherein the second amino acid residue of the light chain CDR3 *(i.e.,* the second Q in QQYSTVPWTF (SEQ ID NO. 16)) is not acetylated. In a specific embodiment, the antigen-binding fragment comprises light chain CDRs 1-3 of SEQ ID NOs: 14-16 and heavy chain CDRs 1-3 of SEQ ID NOs: 20, 18, and 21, wherein the eighth and eleventh amino acid residues of the light chain CDR1 *(i.e.,* the two Ns in SASQDISNYLN (SEQ ID NO. 14) each carries one or more of the following chemical modifications: oxidation, acetylation, deamidation, and pyroglutamation (pyro Glu), and the second amino acid residue of the light chain CDR3 *(i.e.,* the second Q in QQYSTVPWTF (SEQ ID NO. 16)) is not acetylated. The anti-VEGF antigen-binding fragments and transgenes provided herein can be used in any method according to the invention described herein. In a preferred embodiment, the chemical modification(s) or lack of chemical modification(s) (as the case may be) described herein is determined by mass spectrometry.

In certain aspects, also provided herein are anti-VEGF antigen-binding fragments comprising light chain CDRs 1-3 of SEQ ID NOs: 14-16 and heavy chain CDRs 1-3 of SEQ ID NOs: 20, 18, and 21, and transgenes encoding such antigen-VEGF antigen-binding fragments, wherein the last amino acid residue of the heavy chain CDR1 *(i.e.,* the N in GYDFTHYGMN (SEQ ID NO. 20)) does not carry one or more of the following chemical modifications: oxidation, acetylation, deamidation, and pyroglutamation (pyro Glu). In a specific embodiment, the antigen-binding fragment comprises light chain CDRs 1-3 of SEQ ID NOs: 14-16 and heavy chain CDRs 1-3 of SEQ ID NOs: 20, 18, and 21, wherein the ninth amino acid residue of the heavy chain CDR1 *(i.e.,* the M in GYDFTHYGMN (SEQ ID NO. 20)) carries one or more of the following chemical modifications: acetylation, deamidation, and pyroglutamation (pyro Glu), the third amino acid residue of the heavy chain CDR2 *(i.e.,* the N in WINTYTGEPTYAADFKR (SEQ ID NO. 18) carries one or more of the following chemical modifications: acetylation, deamidation, and pyroglutamation (pyro Glu), and the last amino acid residue of the heavy chain CDR1 *(i.e.,* the N in GYDFTHYGMN (SEQ ID NO. 20)) does not carry one or more of the following chemical modifications: oxidation, acetylation, deamidation, and pyroglutamation (pyro Glu). In a specific embodiment, the antigen-binding fragment comprises light chain CDRs 1-3 of SEQ ID NOs: 14-16 and heavy chain CDRs 1-3 of SEQ ID NOs: 20, 18, and 21, wherein the last amino acid residue of the heavy chain CDR1 *(i.e.,* the N in GYDFTHYGMN (SEQ ID NO. 20)) is not acetylated. In a specific embodiment, the antigen-binding fragment comprises light chain CDRs 1-3 of SEQ ID NOs: 14-16 and heavy chain CDRs 1-3 of SEQ ID NOs: 20, 18, and 21, wherein the ninth amino acid residue of the heavy chain CDR1 *(i.e.,* the M in GYDFTHYGMN (SEQ ID NO. 20)) carries one or more of the following chemical modifications: acetylation, deamidation, and pyroglutamation (pyro Glu), the third amino acid residue of the heavy chain CDR2 *(i.e.,* the N in WINTYTGEPTYAADFKR (SEQ ID NO. 18) carries one or more of the following chemical modifications: acetylation, deamidation, and pyroglutamation (pyro Glu), and the last amino acid residue of the heavy chain CDR1 *(i.e.,* the N in GYDFTHYGMN (SEQ ID NO. 20)) is not acetylated. The anti-VEGF antigen-binding fragments and transgenes provided herein can be used in any method according to the invention described herein. In a preferred embodiment, the chemical modification(s) or lack of chemical modification(s) (as the case may be) described herein is determined by mass spectrometry.

In certain aspects, also provided herein are anti-VEGF antigen-binding fragments comprising light chain CDRs 1-3 of SEQ ID NOs: 14-16 and heavy chain CDRs 1-3 of SEQ ID NOs: 20, 18, and 21, and transgenes encoding such antigen-VEGF antigen-binding fragments, wherein the last amino acid residue of the heavy chain CDR1 *(i.e.,* the N in GYDFTHYGMN (SEQ ID NO. 20)) does not carry one or more of the following chemical modifications: oxidation, acetylation, deamidation, and pyroglutamation (pyro Glu), and the second amino acid residue of the light chain CDR3 *(i.e.,* the second Q in QQYSTVPWTF (SEQ ID NO. 16)) does not carry one or more of the following chemical modifications: oxidation, acetylation, deamidation, and pyroglutamation (pyro Glu). In a specific embodiment, the antigen-binding fragment comprises light chain CDRs 1-3 of SEQ ID NOs: 14-16 and heavy chain CDRs 1-3 of SEQ ID NOs: 20, 18, and 21, wherein: (1) the ninth amino acid residue of the heavy chain CDR1 *(i.e.,* the M in GYDFTHYGMN (SEQ ID NO. 20)) carries one or more of the following chemical modifications: acetylation, deamidation, and pyroglutamation (pyro Glu), the third amino acid residue of the heavy chain CDR2 *(i.e.,* the N in WINTYTGEPTYAADFKR (SEQ ID NO. 18) carries one or more of the following chemical modifications: acetylation, deamidation, and pyroglutamation (pyro Glu), and the last amino acid residue of the heavy chain CDR1 *(i.e.,* the N in GYDFTHYGMN (SEQ ID NO. 20)) does not carry one or more of the following chemical modifications: oxidation, acetylation, deamidation, and pyroglutamation (pyro Glu); and (2) the eighth and eleventh amino acid residues of the light chain CDR1 *(i.e.,* the two Ns in SASQDISNYLN (SEQ ID NO. 14) each carries one or more of the following chemical modifications: oxidation, acetylation, deamidation, and pyroglutamation (pyro Glu), and the second amino acid residue of the light chain CDR3 *(i.e.,* the second Q in QQYSTVPWTF (SEQ ID NO. 16)) does not carry one or more of the following chemical modifications: oxidation, acetylation, deamidation, and pyroglutamation (pyro Glu). In a specific embodiment, the antigen-binding fragment comprises light chain CDRs 1-3 of SEQ ID NOs: 14-16 and heavy chain CDRs 1-3 of SEQ ID NOs: 20, 18, and 21, wherein the last amino acid residue of the heavy chain CDR1 *(i.e.,* the N in GYDFTHYGMN (SEQ ID NO. 20)) is not acetylated, and the second amino acid residue of the light chain CDR3 *(i.e.,* the second Q in QQYSTVPWTF (SEQ ID NO. 16)) is not acetylated. In a specific embodiment, the antigen-binding fragment comprises light chain CDRs 1-3 of SEQ ID NOs: 14-16 and heavy chain CDRs 1-3 of SEQ ID NOs: 20, 18, and 21, wherein: (1) the ninth amino acid residue of the heavy chain CDR1 *(i.e.,* the M in GYDFTHYGMN (SEQ ID NO. 20)) carries one or more of the following chemical modifications: acetylation, deamidation, and pyroglutamation (pyro Glu), the third amino acid residue of the heavy chain CDR2 *(i.e.,* the N in WINTYTGEPTYAADFKR (SEQ ID NO. 18) carries one or more of the following chemical modifications: acetylation, deamidation, and pyroglutamation (pyro Glu), and the last amino acid residue of the heavy chain CDR1 *(i.e.,* the N in GYDFTHYGMN (SEQ ID NO. 20)) is not acetylated; and (2) the eighth and eleventh amino acid residues of the light chain CDR1 *(i.e.,* the two Ns in SASQDISNYLN (SEQ ID NO. 14) each carries one or more of the following chemical modifications: oxidation, acetylation, deamidation, and pyroglutamation (pyro Glu), and the second amino acid residue of the light chain CDR3 *(i.e.,* the second Q in QQYSTVPWTF (SEQ ID NO. 16)) is not acetylated. The anti-VEGF antigen-binding fragments and transgenes provided herein can be used in any method according to the invention described herein. In a preferred embodiment, the chemical modification(s) or lack of chemical modification(s) (as the case may be) described herein is determined by mass spectrometry.

**Table 2. Exemplary anti-VEGF transgene and antibody sequences**

| **VEGF antigen-binding fragment (SEQ ID NO.)** | **Sequence** |
|---|---|
| bevacizumab cDNA (Light chain) (10) | gctagcgcca ccatgggctg gtcctgcatc atcctgttcc tggtggccac cgccaccggc gtgcactccg acatccagat gacccagtcc ccctcctccc tgtccgcctc cgtgggcgac cgggtgacca tcacctgctc cgcctcccag gacatctcca actacctgaa ctggtaccag cagaagcccg gcaaggcccc caaggtgctg atctacttca cctcctccct gcactccggc gtgccctccc ggttctccgg ctccggctcc ggcaccgact tcaccctgac catctcctcc ctgcagcccg aggacttcgc cacctactac tgccagcagt actccaccgt gccctggacc ttcggccagg gcaccaaggt ggagatcaag cggaccgtgg ccgccccctc cgtgttcatc ttccccccct ccgacgagca gctgaagtcc ggcaccgcct ccgtggtgtg cctgctgaac aacttctacc cccgggaggc caaggtgcag tggaaggtgg acaacgccct gcagtccggc aactcccagg agtccgtgac cgagcaggac tccaaggact ccacctactc cctgtcctcc accctgaccc tgtccaaggc cgactacgag aagcacaagg tgtacgcctg cgaggtgacc caccagggcc tgtcctcccc cgtgaccaag tccttcaacc ggggcgagtg ctgagcggcc gcctcgag |
| bevacizumab cDNA (Heavy chain) (11) | gctagcgcca ccatgggctg gtcctgcatc atcctgttcc tggtggccac cgccaccggc gtgcactccg aggtgcagct ggtggagtcc ggcggcggcc tggtgcagcc cggcggctcc ctgcggctgt cctgcgccgc ctccggctac accttcacca actacggcat gaactgggtg cggcaggccc ccggcaaggg cctggagtgg gtgggctgga tcaacaccta caccggcgag cccacctacg ccgccgactt caagcggcgg ttcaccttct ccctggacac ctccaagtcc accgcctacc tgcagatgaa ctccctgcgg gccgaggaca ccgccgtgta ctactgcgcc aagtaccccc actactacgg ctcctcccac tggtacttcg acgtgtgggg ccagggcacc ctggtgaccg tgtcctccgc ctccaccaag ggcccctccg tgttccccct ggccccctcc tccaagtcca cctccggcgg caccgccgcc ctgggctgcc tggtgaagga ctacttcccc gagcccgtga ccgtgtcctg gaactccggc gccctgacct ccggcgtgca caccttcccc gccgtgctgc agtcctccgg cctgtactcc ctgtcctccg tggtgaccgt gccctcctcc tccctgggca cccagaccta catctgcaac gtgaaccaca agccctccaa caccaaggtg gacaagaagg tggagcccaa gtcctgcgac aagacccaca cctgcccccc ctgccccgcc cccgagctgc tgggcggccc ctccgtgttc ctgttccccc ccaagcccaa ggacaccctg atgatctccc ggacccccga ggtgacctgc gtggtggtgg acgtgtccca cgaggacccc gaggtgaagt tcaactggta cgtggacggc gtggaggtgc acaacgccaa gaccaagccc cgggaggagc agtacaactc cacctaccgg gtggtgtccg tgctgaccgt gctgcaccag gactggctga acggcaagga gtacaagtgc aaggtgtcca acaaggccct gcccgccccc atcgagaaga ccatctccaa ggccaagggc cagccccggg agccccaggt gtacaccctg ccccctcccc gggaggagat gaccaagaac caggtgtccc tgacctgcct ggtgaagggc ttctacccct ccgacatcgc cgtggagtgg gagtccaacg gccagcccga gaacaactac aagaccaccc cccccgtgct ggactccgac ggctccttct tcctgtactc caagctgacc gtggacaagt cccggtggca gcagggcaac gtgttctcct gctccgtgat gcacgaggcc ctgcacaacc actacaccca gaagtccctg tccctgtccc ccggcaagtg agcggccgcc |
| bevacizumab Fab Amino Acid Sequence (Light chain) (3) | DIQMTQSPSSLSASVGDRVTITCSASQDISNYLNWYQQKPGKAPKVLIYFTSSLH SGVPSRFSGSGSGTDFTLTISSLQPEDFATYYCQQYSTVPWTFGQGTKVEIKRTV AAPSVFIFPPSDEQLKSGTASVVCLLNNFYPREAKVQWKVDNALQSGNSQESVTE QDSKDSTYSLSSTLTLSKADYEKHKVYACEVTHQGLSSPVTKSFNRGEC |
| bevacizumab Fab Amino Acid Sequence (Heavy chain) (4) | EVQLVESGGGLVQPGGSLRLSCAASGYTFTNYGMNWVRQAPGKGLEWVGWINTYT GEPTYAADFKRRFTFSLDTSKSTAYLQMNSLRAEDTAVYYCAKYPHYYGSSHWYF DVWGQGTLVTVSSASTKGPSVFPLAPSSKSTSGGTAALGCLVKDYFPEPVTVSWN SGALTSGVHTFPAVLQSSGLYSLSSVVTVPSSSLGTQTYICNVNHKPSNTKVDKK VEPKSCDKTHL |
| ranibizumab cDNA (Light chain comprising a signal sequence) (12) | gagctccatg gagtttttca aaaagacggc acttgccgca ctggttatgg gttttagtgg tgcagcattg gccgatatcc agctgaccca gagcccgagc agcctgagcg caagcgttgg tgatcgtgtt accattacct gtagcgcaag ccaggatatt agcaattatc tgaattggta tcagcagaaa ccgggtaaag caccgaaagt tctgatttat tttaccagca gcctgcatag cggtgttccg agccgtttta gcggtagcgg tagtggcacc gattttaccc tgaccattag cagcctgcag ccggaagatt ttgcaaccta ttattgtcag cagtatagca ccgttccgtg gacctttggt cagggcacca aagttgaaat taaacgtacc gttgcagcac cgagcgtttt tatttttccg cctagtgatg aacagctgaa aagcggcacc gcaagcgttg tttgtctgct gaataatttt tatccgcgtg aagcaaaagt gcagtggaaa gttgataatg cactgcagag cggtaatagc caagaaagcg ttaccgaaca ggatagcaaa gatagcacct atagcctgag cagcaccctg accctgagca aagcagatta tgaaaaacac aaagtgtatg cctgcgaagt tacccatcag ggtctgagca gtccggttac caaaagtttt aatcgtggcg aatgctaata gaagcttggt acc |
| ranibizumab cDNA (Heavy chain comprising a signal sequence) (13) | gagctcatat gaaatacctg ctgccgaccg ctgctgctgg tctgctgctc ctcgctgccc agccggcgat ggccgaagtt cagctggttg aaagcggtgg tggtctggtt cagcctggtg gtagcctgcg tctgagctgt gcagcaagcg gttatgattt tacccattat ggtatgaatt gggttcgtca ggcaccgggt aaaggtctgg aatgggttgg ttggattaat acctataccg gtgaaccgac ctatgcagca gattttaaac gtcgttttac ctttagcctg gataccagca aaagcaccgc atatctgcag atgaatagcc tgcgtgcaga agataccgca gtttattatt gtgccaaata tccgtattac tatggcacca gccactggta tttcgatgtt tggggtcagg gcaccctggt taccgttagc agcgcaagca ccaaaggtcc gagcgttttt ccgctggcac cgagcagcaa aagtaccagc ggtggcacag cagcactggg ttgtctggtt aaagattatt ttccggaacc ggttaccgtg agctggaata gcggtgcact gaccagcggt gttcatacct ttccggcagt tctgcagagc agcggtctgt atagcctgag cagcgttgtt accgttccga gcagcagcct gggcacccag acctatattt gtaatgttaa tcataaaccg agcaatacca aagtggataa aaaagttgag ccgaaaagct gcgataaaac ccatctgtaa tagggtacc |
| ranibizumab Fab Amino Acid Sequence (Light chain) (1) | DIQLTQSPSSLSASVGDRVTITCSASQDISNYLNWYQQKPGKAPKVLIYFTSSLH SGVPSRFSGSGSGTDFTLTISSLQPEDFATYYCQQYSTVPWTFGQGTKVEIKRTV AAPSVFIFPPSDEQLKSGTASVVCLLNNFYPREAKVQWKVDNALQSGNSQESVTE QDSKDSTYSLSSTLTLSKADYEKHKVYACEVTHQGLSSPVTKSFNRGEC |
| ranibizumab Fab Amino Acid Sequence (Heavy chain) (2) | EVQLVESGGGLVQPGGSLRLSCAASGYDFTHYGMNWVRQAPGKGLEWVGWINTYT GEPTYAADFKRRFTFSLDTSKSTAYLQMNSLRAEDTAVYYCAKYPYYYGTSHWYF DVWGQGTLVTVSSASTKGPSVFPLAPSSKSTSGGTAALGCLVKDYFPEPVTVSWN SGALTSGVHTFPAVLQSSGLYSLSSVVTVPSSSLGTQTYICNVNHKPSNTKVDKK VEPKSCDKTHL |
| bevacizumab Light Chain CDRs (14, 15, and 16) | SASQDISNYLN |
| | FTSSLHS |
| | QQYSTVPWT |
| bevacizumab Heavy Chain CDRs (17, 18, and 19) | GYTFTNYGMN |
| | WINTYTGEPTYAADFKR |
| | YPHYYGSSHWYFDV |
| ranibizumab Light Chain CDRs (14, 15, and 16) | SASQDISNYLN |
| | FTSSLHS |
| | QQYSTVPWT |
| ranibizumab Heavy Chain CDRs (20, 18, and 21) | GYDFTHYGMN |
| | WINTYTGEPTYAADFKR |
| | YPYYYGTSHWYFDV |

In certain embodiments, the recombinant vectors provided herein comprise the following elements in the following order: a) a constitutive or a hypoxia-inducible promoter sequence, and b) a sequence encoding the transgene *(e.g.,* an anti-VEGF antigen-binding fragment moiety). In certain embodiments, the sequence encoding the transgene comprises multiple ORFs separated by IRES elements. In certain embodiments, the ORFs encode the heavy and light chain domains of the anti-VEGF antigen-binding fragment. In certain embodiments, the sequence encoding the transgene comprises multiple subunits in one ORF separated by F/F2A sequences. In certain embodiments, the sequence comprising the transgene encodes the heavy and light chain domains of the anti-VEGF antigen-binding fragment separated by an F/F2A sequence. In certain embodiments, the viral vectors provided herein comprise the following elements in the following order: a) a constitutive or a hypoxia-inducible promoter sequence, and b) a sequence encoding the transgene *(e.g.,* an anti-VEGF antigen-binding fragment moiety), wherein the transgene comprises the signal peptide of VEGF (SEQ ID NO: 5), and wherein the transgene encodes a light chain and a heavy chain sequence separated by an IRES element. In certain embodiments, the recombinant vectors provided herein comprise the following elements in the following order: a) a constitutive or a hypoxia-inducible promoter sequence, and b) a sequence encoding the transgene *(e.g.,* an anti-VEGF antigen-binding fragment moiety), wherein the transgene comprises the signal peptide of VEGF (SEQ ID NO: 5), and wherein the transgene encodes a light chain and a heavy chain sequence separated by a cleavable F/F2A sequence.

In certain embodiments, the recombinant vectors provided herein comprise the following elements in the following order: a) a first ITR sequence, b) a first linker sequence, c) a constitutive or a hypoxia-inducible promoter sequence, d) a second linker sequence, e) an intron sequence, f) a third linker sequence, g) a first UTR sequence, h) a sequence encoding the transgene *(e.g.,* an anti-VEGF antigen-binding fragment moiety), i) a second UTR sequence, j) a fourth linker sequence, k) a poly A sequence, l) a fifth linker sequence, and m) a second ITR sequence.

In certain embodiments, the recombinant vectors provided herein comprise the following elements in the following order: a) a first ITR sequence, b) a first linker sequence, c) a constitutive or a hypoxia-inducible promoter sequence, d) a second linker sequence, e) an intron sequence, f) a third linker sequence, g) a first UTR sequence, h) a sequence encoding the transgene *(e.g.,* an anti-VEGF antigen-binding fragment moiety), i) a second UTR sequence, j) a fourth linker sequence, k) a poly A sequence, l) a fifth linker sequence, and m) a second ITR sequence, wherein the transgene comprises the signal peptide of VEGF (SEQ ID NO: 5), and wherein the transgene encodes a light chain and a heavy chain sequence separated by a cleavable F/F2A sequence.

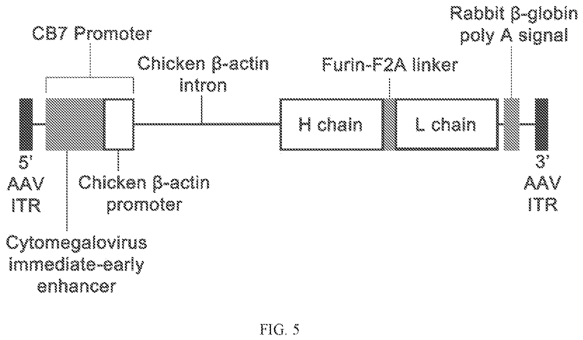

In a specific embodiment, the recombinant vector provided herein is Construct II, wherein the Construct II comprise the following components: (1) AAV2 inverted terminal repeats that flank the expression cassette; (2) control elements, which include a) the CB7 promoter, comprising the CMV enhancer/chicken β-actin promoter, b) a chicken β-actin intron and c) a rabbit β-globin poly A signal; and (3) nucleic acid sequences coding for the heavy and light chains of anti-VEGF antigen-binding fragment, separated by a self-cleaving furin (F)/F2A linker, ensuring expression of equal amounts of the heavy and the light chain polypeptides. In a specific embodiment, the construct described herein is illustrated in FIG. 5.

### 6.3.3 GENE THERAPY

### (a) Target Patient Populations

In certain embodiments, the methods provided herein are for the administration to patients diagnosed with an ocular disease (for example, wet AMD, dry AMD, retinal vein occlusion (RVO), diabetic macular edema (DME), or diabetic retinopathy (DR) (in particular, wet AMD)), in particular an ocular disease caused by increased neovascularization.

In certain embodiments, the methods provided herein are for the administration to patients diagnosed with severe AMD. In certain embodiments, the methods provided herein are for the administration to patients diagnosed with attenuated AMD.

In certain embodiments, the methods provided herein are for the administration to patients diagnosed with severe wet AMD. In certain embodiments, the methods provided herein are for the administration to patients diagnosed with attenuated wet AMD.

In certain embodiments, the methods provided herein are for the administration to patients diagnosed with severe diabetic retinopathy. In certain embodiments, the methods provided herein are for the administration to patients diagnosed with attenuated diabetic retinopathy.

In certain embodiments, the methods provided herein are for the administration to patients diagnosed with AMD who have been identified as responsive to treatment with an anti-VEGF antibody.

In certain embodiments, the methods provided herein are for the administration to patients diagnosed with AMD who have been identified as responsive to treatment with an anti-VEGF antigen-binding fragment.

In certain embodiments, the methods provided herein are for the administration to patients diagnosed with AMD who have been identified as responsive to treatment with an anti-VEGF antigen-binding fragment injected intravitreally prior to treatment with gene therapy.

In certain embodiments, the methods provided herein are for the administration to patients diagnosed with AMD who have been identified as responsive to treatment with LUCENTIS ^{®} (ranibizumab), EYLEA^{®} (aflibercept), and/or AVASTIN^{®} (bevacizumab).

In certain embodiments, a patient diagnosed with AMD is identified as responsive to treatment with an anti-VEGF antigen-binding fragment (e.g., ranibizumab) if the patient has improvement in fluid after intravitreal injection of the anti-VEGF antigen-binding fragment to the patient prior to treatment with gene therapy. In certain embodiments, a patient diagnosed with AMD is identified as responsive to treatment with an anti-VEGF antigen-binding fragment (e.g., ranibizumab) if the patient has improvement in fluid and has a central retinal thickness (CRT) < 400 µm after intravitreal injection of the anti-VEGF antigen-binding fragment to the patient prior to treatment with gene therapy. In some embodiments, the anti-VEGF antigen-binding fragment is intravitreally injected to the patient at 0.5 mg per month for two months prior to treatment with gene therapy. In other embodiments, the anti-VEGF antigen-binding fragment is intravitreally injected to the patient at 0.5 mg per month for three months prior to treatment with gene therapy. In a preferred embodiment, a patient has improvement in fluid if he or she has an improvement in inner retinal (parafovea 3 mm) fluid of > 50 µm or 30% relative to the level prior to the intravitreal injection of the anti-VEGF antigen-binding fragment, or has an improvement in center subfield thickness of > 50 µm or 30% as determined by the CRC relative to the level prior to the intravitreal injection of the anti-VEGF antigen-binding fragment.

In certain embodiments, the methods provided herein are for the administration to patients diagnosed with AMD who have disease other than fluid contributing to an increase in CRT *(i.e.,* pigment epithelial detachment (PED) or subretinal hyperreflective material (SHRM)) and who have < 75 µm of fluid (intraretinal or subretinal), as determined by the CRC.

In certain embodiments of the methods described herein, the patient has a BCVA in the eye to be treated that is ≤20/20 and ≥20/400 before treatment. In a specific embodiment, the patient has a BCVA in the eye to be treated that is ≤20/63 and ≥20/400 before treatment.

In certain embodiments of the methods described herein, the patient has an Early Treatment Diabetic Retinopathy Study (ETDRS) BCVA letter score between ≤78 and ≥44 in the eye to be treated before treatment.

In certain embodiments of the methods described herein, the patient is not concurrently having an anticoagulation therapy.

### (b) Dosage

In certain embodiments, doses that maintain a concentration of the therapeutic product at a Cₘᵢₙ of at least 0.330 µg/mL in the Vitreous humour, or 0.110 µg/mL in the Aqueous humour (the anterior chamber of the eye) for three months are desired; thereafter, Vitreous Cₘᵢₙ concentrations of the therapeutic product ranging from 1.70 to 6.60 µg/mL, and/or Aqueous Cₘᵢₙ concentrations ranging from 0.567 to 2.20 µg/mL should be maintained. However, because the therapeutic product is continuously produced (under the control of a constitutive promoter or induced by hypoxic conditions when using an hypoxia-inducible promoter), maintenance of lower concentrations can be effective. Vitreous humour concentrations can be measured directly in patient samples of fluid collected from the vitreous humour or the anterior chamber, or estimated and/or monitored by measuring the patient's serum concentrations of the therapeutic product - the ratio of systemic to vitreal exposure to the therapeutic product is about 1:90,000. *(E.g., see,* vitreous humor and serum concentrations of ranibizumab reported in Xu L, et al., 2013, Invest. Opthal. Vis. Sci. 54: 1616-1624, at p. 1621 and Table 5 at p. 1623, which is incorporated by reference herein in its entirety).

In certain embodiments, dosages are measured by genome copies per ml or the number of genome copies administered to the eye of the patient (e.g., administered suprachoroidally, subretinally, intravitreally, juxtasclerally, subconjunctivally, and/or intraretinally (e.g., by suprachoroidal injection, subretinal injection via the transvitreal approach (a surgical procedure), subretinal administration via the suprachoroidal space, or a posterior juxtascleral depot procedure)). In certain embodiments, 2.4 x 10¹¹ genome copies per ml to 1 x10¹³ genome copies per ml are administered. In a specific embodiment, 2.4 x 10¹¹ genome copies per ml to 5 x10¹¹ genome copies per ml are administered. In another specific embodiment, 5 x 10¹¹ genome copies per ml to 1 x10¹² genome copies per ml are administered. In another specific embodiment, 1 x 10¹² genome copies per ml to 5 x10¹² genome copies per ml are administered. In another specific embodiment, 5 x 10¹² genome copies per ml to 1 x10¹³ genome copies per ml are administered. In another specific embodiment, about 2.4 x 10¹¹ genome copies per ml are administered. In another specific embodiment, about 5 x 10¹¹ genome copies per ml are administered. In another specific embodiment, about 1 x 10¹² genome copies per ml are administered. In another specific embodiment, about 5 x 10¹² genome copies per ml are administered. In another specific embodiment, about 1 x 10¹³ genome copies per ml are administered. In certain embodiments, 1 x 10⁹ to 1 x 10¹² genome copies are administered. In specific embodiments, 3 x 10⁹ to 2.5 x 10¹¹ genome copies are administered. In specific embodiments, 1 x 10⁹ to 2.5 x 10¹¹ genome copies are administered. In specific embodiments, 1 x 10⁹ to 1 x 10¹¹ genome copies are administered. In specific embodiments, 1 x 10⁹ to 5 x 10⁹ genome copies are administered. In specific embodiments, 6 x 10⁹ to 3 x 10¹⁰ genome copies are administered. In specific embodiments, 4 x 10¹⁰ to 1 x 10¹¹ genome copies are administered. In specific embodiments, 2 x 10¹¹ to 1 x 10¹² genome copies are administered. In a specific embodiment, about 3 x 10⁹ genome copies are administered (which corresponds to about 1.2 x 10¹⁰ genome copies per ml in a volume of 250 µl). In another specific embodiment, about 1 x 10¹⁰ genome copies are administered (which corresponds to about 4 x 10¹⁰ genome copies per ml in a volume of 250 µl). In another specific embodiment, about 6 x 10¹⁰ genome copies are administered (which corresponds to about 2.4 x 10¹¹ genome copies per ml in a volume of 250 µl). In another specific embodiment, about 1.6 x 10¹¹ genome copies are administered (which corresponds to about 6.2 x 10¹¹ genome copies per ml in a volume of 250 µl). In another specific embodiment, about 1.55 x 10¹¹ genome copies are administered (which corresponds to about 6.2 x 10¹¹ genome copies per ml in a volume of 250 µl). In another specific embodiment, about 2.5 x 10¹¹ genome copies (which corresponds to about 1.0 x 10¹² genome copies per ml in a volume of 250 µl) are administered.

In certain embodiments, about 6.0 × 10¹⁰ genome copies per eye are administered. In certain embodiments, about 1.6 × 10¹¹ genome copies per eye are administered. In certain embodiments, about 2.5 × 10¹¹ genome copies per eye are administered. In certain embodiments, about 5.0 × 10¹¹ genome copies per eye are administered. In certain embodiments, about 3 x 10¹² genome copies per eye are administered. In certain embodiments, about 1.0 × 10¹² genome copies per ml per eye are administered. In certain embodiments, about 2.5 × 10¹² genome copies per ml per eye are administered. In certain embodiments, about 3.0 × 10¹³ genome copies per eye are administered. In certain embodiments, up to 3.0 × 10¹³ genome copies per eye are administered.

In certain embodiments, about 6.0 x 10¹⁰ genome copies per eye are administered by subretinal injection. In certain embodiments, about 1.6 x 10¹¹ genome copies per eye are administered by subretinal injection. In certain embodiments, about 2.5 × 10¹¹ genome copies per eye are administered by subretinal injection. In certain embodiments, about 3.0 × 10¹³ genome copies per eye are administered by subretinal injection. In certain embodiments, up to 3.0 × 10¹³ genome copies per eye are administered by subretinal injection.

In certain embodiments, about 2.5 × 10¹¹ genome copies per eye are administered by suprachoroidal injection. In certain embodiments, about 5.0 × 10¹¹ genome copies per eye are administered by suprachoroidal injection. In certain embodiments, about 3 x 10¹² genome copies per eye are administered by suprachoroidal injection. In certain embodiments, about 2.5 × 10¹¹ genome copies per eye are administered by a single suprachoroidal injection. In certain embodiments, about 5.0 × 10¹¹ genome copies per eye are administered by double suprachoroidal injections. In certain embodiments, about 3.0 × 10¹³ genome copies per eye are administered by suprachoroidal injection. In certain embodiments, up to 3.0 × 10¹³ genome copies per eye are administered suprachoroidal injection. In certain embodiments, about 2.5 × 10¹² genome copies per ml per eye are administered by a single suprachoroidal injection in a volume of 100 µl. In certain embodiments, about 2.5 × 10¹² genome copies per ml per eye are administered by double suprachoroidal injections, wherein each injection is in a volume of 100 µl.

As used herein and unless otherwise specified, the term "about" means within plus or minus 10% of a given value or range. In certain embodiments, the term "about" encompasses the exact number recited.

### (c) Sampling and Monitoring of Efficacy

Effects of the methods provided herein on visual deficits may be measured by BCVA (Best-Corrected Visual Acuity), intraocular pressure, slit lamp biomicroscopy, and/or indirect ophthalmoscopy.

In specific embodiments, effects of the methods provided herein on visual deficits may be measured by whether the human patient's eye that is treated by a method described herein achieves BCVA of greater than 43 letters post-treatment (e.g., 46-50 weeks or 98-102 weeks post-treatment). A BCVA of 43 letters corresponds to 20/160 approximate Snellen equivalent. In a specific embodiment, the human patient's eye that is treated by a method described herein achieves BCVA of greater than 43 letters post-treatment (e.g., 46-50 weeks or 98-102 weeks post-treatment).

In specific embodiments, effects of the methods provided herein on visual deficits may be measured by whether the human patient's eye that is treated by a method described herein achieves BCVA of greater than 84 letters post-treatment (e.g., 46-50 weeks or 98-102 weeks post-treatment). A BCVA of 84 letters corresponds to 20/20 approximate Snellen equivalent. In a specific embodiment, the human patient's eye that is treated by a method described herein achieves BCVA of greater than 84 letters post-treatment (e.g., 46-50 weeks or 98-102 weeks post-treatment).

Effects of the methods provided herein on physical changes to eye/retina may be measured by SD-OCT (SD-Optical Coherence Tomography).

Efficacy may be monitored as measured by electroretinography (ERG).

Effects of the methods provided herein may be monitored by measuring signs of vision loss, infection, inflammation and other safety events, including retinal detachment.

Retinal thickness may be monitored to determine efficacy of the methods provided herein. Without being bound by any particular theory, thickness of the retina may be used as a clinical readout, wherein the greater reduction in retinal thickness or the longer period of time before thickening of the retina, the more efficacious the treatment. Retinal function may be determined, for example, by ERG. ERG is a non-invasive electrophysiologic test of retinal function, approved by the FDA for use in humans, which examines the light sensitive cells of the eye (the rods and cones), and their connecting ganglion cells, in particular, their response to a flash stimulation. Retinal thickness may be determined, for example, by SD-OCT. SD-OCT is a three-dimensional imaging technology which uses low-coherence interferometry to determine the echo time delay and magnitude of backscattered light reflected off an object of interest. OCT can be used to scan the layers of a tissue sample *(e.g.,* the retina) with 3 to 15 µm axial resolution, and SD-OCT improves axial resolution and scan speed over previous forms of the technology (Schuman, 2008, Trans. Am. Opthamol. Soc. 106:426-458).

Effects of the methods provided herein may also be measured by a change from baseline in National Eye Institute Visual Functioning Questionnaire, the Rasch-scored version (NEI-VFQ-28-R) (composite score; activity limitation domain score; and socio-emotional functioning domain score). Effects of the methods provided herein may also be measured by a change from baseline in National Eye Institute Visual Functioning Questionnaire 25-item version (NEI-VFQ-25) (composite score and mental health subscale score). Effects of the methods provided herein may also be measured by a change from baseline in Macular Disease Treatment Satisfaction Questionnaire (MacTSQ) (composite score; safety, efficacy, and discomfort domain score; and information provision and convenience domain score).

In specific embodiments, the efficacy of a method described herein is reflected by an improvement in vision at about 4 weeks, 12 weeks, 6 months, 12 months, 24 months, 36 months, or at other desired timepoints. In a specific embodiment, the improvement in vision is characterized by an increase in BCVA, for example, an increase by 1 letter, 2 letters, 3 letters, 4 letters, 5 letters, 6 letters, 7 letters, 8 letters, 9 letters, 10 letters, 11 letters, or 12 letters, or more. In a specific embodiment, the improvement in vision is characterized by a 5%, 10%, 15%, 20%, 30%, 40%, 50% or more increase in visual acuity from baseline.

In specific embodiments, the efficacy of a method described herein is reflected by an reduction in central retinal thickness (CRT) at about 4 weeks, 12 weeks, 6 months, 12 months, 24 months, 36 months, or at other desired timepoint, for example, a 5%, 10%, 15%, 20%, 30%, 40%, 50% or more decrease in central retinal thickness from baseline.

In specific embodiments, there is no inflammation in the eye after treatment or little inflammation in the eye after treatment (for example, an increase in the level of inflammation by 10%, 5%, 2%, 1% or less from baseline).

If the human patient is a child, visual function can be assessed using an optokinetic nystagmus (OKN)-based approach or a modified OKN-based approach.

### 6.4 COMBINATION THERAPIES

The methods provided herein may be combined with one or more additional therapies. In one aspect, the methods provided herein are administered with laser photocoagulation. In one aspect, the methods provided herein are administered with photodynamic therapy with verteporfin.

In one aspect, the methods provided herein are administered with intravitreal (IVT) injections with the therapeutic product. In a specific embodiment wherein the therapeutic product is an anti-VEGF antibody or antigen-binding fragment, the methods provided herein are administered with IVT injections with anti-VEGF agents, including but not limited to HuPTMFabVEGFi, *e.g.,* HuGlyFabVEGFi produced in human cell lines (Dumont *et al.,* 2015, supra), or other anti-VEGF agents such as pegaptanib, ranibizumab, aflibercept, or bevacizumab.

The additional therapies may be administered before, concurrently or subsequent to the gene therapy treatment.

The efficacy of the gene therapy treatment may be indicated by the elimination of or reduction in the number of rescue treatments using standard of care. For example, when the therapeutic product is anti-VEGF antibody or antigen-binding fragment, the efficacy of the gene therapy treatment may be indicated by the elimination or reduction in the number of rescue treatments of intravitreal injections with anti-VEGF agents, including but not limited to HuPTMFabVEGFi, *e.g.,* HuGlyFabVEGFi produced in human cell lines, or other anti-VEGF agents such as pegaptanib, ranibizumab, aflibercept, or bevacizumab.

### 7. SEQUENCES

SEQ ID NO:1 
RANIBIZUMAB FAB AMINO ACID SEQUENCE (LIGHT CHAIN)
DIQLTQSPSSLSASVGDRVTITCSASQDISNYLNWYQQKPGKAPKVLIYFTSSLH
 
SGVPSRFSGSGSGTDFTLTISSLQPEDFATYYCQQYSTVPWTFGQGTKVEIKRTV
AAPSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC
SEQ ID NO:2 
RANIBIZUMAB FAB AMINO ACID SEQUENCE (HEAVY CHAIN)
EVQLVESGGGLVQPGGSLRLSCAASGYDFTHYGMNWVRQAPGKGLEWVGWINTYT
GEPTYAADFKRRFTFSLDTSKSTAYLQMNSLRAEDTAVYYCAKYPYYYGTSHWYF
DVWGQGTLVTVSSASTKGPSVFPLAPSSKSTSGGTAALGCLVKDYFPEPVTVSWN
SGALTSGVHTFPAVLQSSGLYSLSSVVTVPSSSLGTQTYICNVNHKPSNTKVDKK
VEPKSCDKTHL
SEQ ID NO:3 
BEVACIZUMAB FAB AMINO ACID SEQUENCE (LIGHT CHAIN)
DIQMTQSPSSLSASVGDRVTITCSASQDISNYLNWYQQKPGKAPKVLIYFTSSLH
SGVPSRFSGSGSGTDFTLTISSLQPEDFATYYCQQYSTVPWTFGQGTKVEIKRTV
AAPSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC
SEQ ID NO:4 
BEVACIZUMAB FAB AMINO ACID SEQUENCE (HEAVY CHAIN)
EVQLVESGGGLVQPGGSLRLSCAASGYTFTNYGMNWVRQAPGKGLEWVGWINTYT
GEPTYAADFKRRFTFSLDTSKSTAYLQMNSLRAEDTAVYYCAKYPHYYGSSHWYF
DVWGQGTLVTVSSASTKGPSVFPLAPSSKSTSGGTAALGCLVKDYFPEPVTVSWN
SGALTSGVHTFPAVLQSSGLYSLSSVVTVPSSSLGTQTYICNVNHKPSNTKVDKK
VEPKSCDKTHL
SEQ ID NO:5
   VEGF-A SIGNAL PEPTIDE
   MNFLLSWVHW SLALLLYLHH AKWSQA
SEQ ID NO:6
   FIBULIN-1 SIGNAL PEPTIDE
   MERAAPSRRV PLPLLLLGGL ALLAAGVDA
SEQ ID NO:7
   VITRONECTIN SIGNAL PEPTIDE
   MAPLRPLLIL ALLAWVALA
SEQ ID NO:8
   COMPLEMENT FACTOR H SIGNAL PEPTIDE
   MRLLAKIICLMLWAICVA
SEQ ID NO:9
   OPTICIN SIGNAL PEPTIDE
   MRLLAFLSLL ALVLQETGT
SEQ ID NO:10
   BEVACIZUMAB CDNA (LIGHT CHAIN) 
GCTAGCGCCA CCATGGGCTG GTCCTGCATC ATCCTGTTCC TGGTGGCCAC CGCCACCGGC GTGCACTCCG
ACATCCAGAT GACCCAGTCC CCCTCCTCCC TGTCCGCCTC CGTGGGCGAC CGGGTGACCA TCACCTGCTC
CGCCTCCCAG GACATCTCCA ACTACCTGAA CTGGTACCAG CAGAAGCCCG GCAAGGCCCC CAAGGTGCTG
ATCTACTTCA CCTCCTCCCT GCACTCCGGC GTGCCCTCCC GGTTCTCCGG CTCCGGCTCC GGCACCGACT
TCACCCTGAC CATCTCCTCC CTGCAGCCCG AGGACTTCGC CACCTACTAC TGCCAGCAGT ACTCCACCGT
GCCCTGGACC TTCGGCCAGG GCACCAAGGT GGAGATCAAG CGGACCGTGG CCGCCCCCTC CGTGTTCATC
TTCCCCCCCT CCGACGAGCA GCTGAAGTCC GGCACCGCCT CCGTGGTGTG CCTGCTGAAC AACTTCTACC
CCCGGGAGGC CAAGGTGCAG TGGAAGGTGG ACAACGCCCT GCAGTCCGGC AACTCCCAGG AGTCCGTGAC
 
CGAGCAGGAC TCCAAGGACT CCACCTACTC CCTGTCCTCC ACCCTGACCC TGTCCAAGGC CGACTACGAG
AAGCACAAGG TGTACGCCTG CGAGGTGACC CACCAGGGCC TGTCCTCCCC CGTGACCAAG TCCTTCAACC
GGGGCGAGTG CTGAGCGGCC GCCTCGAG
SEQ ID NO:11
   Bevacizumab cDNA (Heavy chain) 
gctagcgcca ccatgggctg gtcctgcatc atcctgttcc tggtggccac cgccaccggc gtgcactccg
aggtgcagct ggtggagtcc ggcggcggcc tggtgcagcc cggcggctcc ctgcggctgt cctgcgccgc
ctccggctac accttcacca actacggcat gaactgggtg cggcaggccc ccggcaaggg cctggagtgg
gtgggctgga tcaacaccta caccggcgag cccacctacg ccgccgactt caagcggcgg ttcaccttct
ccctggacac ctccaagtcc accgcctacc tgcagatgaa ctccctgcgg gccgaggaca ccgccgtgta
ctactgcgcc aagtaccccc actactacgg ctcctcccac tggtacttcg acgtgtgggg ccagggcacc
ctggtgaccg tgtcctccgc ctccaccaag ggcccctccg tgttccccct ggccccctcc tccaagtcca
cctccggcgg caccgccgcc ctgggctgcc tggtgaagga ctacttcccc gagcccgtga ccgtgtcctg
gaactccggc gccctgacct ccggcgtgca caccttcccc gccgtgctgc agtcctccgg cctgtactcc
ctgtcctccg tggtgaccgt gccctcctcc tccctgggca cccagaccta catctgcaac gtgaaccaca
agccctccaa caccaaggtg gacaagaagg tggagcccaa gtcctgcgac aagacccaca cctgcccccc
ctgccccgcc cccgagctgc tgggcggccc ctccgtgttc ctgttccccc ccaagcccaa ggacaccctg
atgatctccc ggacccccga ggtgacctgc gtggtggtgg acgtgtccca cgaggacccc gaggtgaagt
tcaactggta cgtggacggc gtggaggtgc acaacgccaa gaccaagccc cgggaggagc agtacaactc
cacctaccgg gtggtgtccg tgctgaccgt gctgcaccag gactggctga acggcaagga gtacaagtgc
aaggtgtcca acaaggccct gcccgccccc atcgagaaga ccatctccaa ggccaagggc cagccccggg
agccccaggt gtacaccctg cccccctccc gggaggagat gaccaagaac caggtgtccc tgacctgcct
ggtgaagggc ttctacccct ccgacatcgc cgtggagtgg gagtccaacg gccagcccga gaacaactac
aagaccaccc cccccgtgct ggactccgac ggctccttct tcctgtactc caagctgacc gtggacaagt
cccggtggca gcagggcaac gtgttctcct gctccgtgat gcacgaggcc ctgcacaacc actacaccca
gaagtccctg tccctgtccc ccggcaagtg agcggccgc
SEQ ID NO:12
   ranibizumab cDNA (Light chain comprising a signal sequence) 
gagctccatg gagtttttca aaaagacggc acttgccgca ctggttatgg gttttagtgg tgcagcattg
gccgatatcc agctgaccca gagcccgagc agcctgagcg caagcgttgg tgatcgtgtt accattacct
gtagcgcaag ccaggatatt agcaattatc tgaattggta tcagcagaaa ccgggtaaag caccgaaagt
tctgatttat tttaccagca gcctgcatag cggtgttccg agccgtttta gcggtagcgg tagtggcacc
gattttaccc tgaccattag cagcctgcag ccggaagatt ttgcaaccta ttattgtcag cagtatagca
ccgttccgtg gacctttggt cagggcacca aagttgaaat taaacgtacc gttgcagcac cgagcgtttt
tatttttccg cctagtgatg aacagctgaa aagcggcacc gcaagcgttg tttgtctgct gaataatttt
tatccgcgtg aagcaaaagt gcagtggaaa gttgataatg cactgcagag cggtaatagc caagaaagcg
ttaccgaaca ggatagcaaa gatagcacct atagcctgag
cagcaccctg accctgagca aagcagatta tgaaaaacac aaagtgtatg cctgcgaagt tacccatcag
ggtctgagca gtccggttac caaaagtttt aatcgtggcg aatgctaata gaagcttggt
SEQ ID NO:13
   ranibizumab cDNA (Heavy chain comprising a signal sequence) 
gagctcatat gaaatacctg ctgccgaccg ctgctgctgg tctgctgctc ctcgctgccc agccggcgat
ggccgaagtt cagctggttg aaagcggtgg tggtctggtt cagcctggtg gtagcctgcg tctgagctgt
gcagcaagcg gttatgattt tacccattat ggtatgaatt gggttcgtca ggcaccgggt aaaggtctgg
aatgggttgg ttggattaat acctataccg gtgaaccgac ctatgcagca gattttaaac gtcgttttac
ctttagcctg gataccagca
aaagcaccgc atatctgcag atgaatagcc tgcgtgcaga agataccgca gtttattatt gtgccaaata
tccgtattac tatggcacca gccactggta tttcgatgtt tggggtcagg gcaccctggt taccgttagc
agcgcaagca ccaaaggtcc gagcgttttt ccgctggcac cgagcagcaa aagtaccagc ggtggcacag
cagcactggg ttgtctggtt aaagattatt ttccggaacc ggttaccgtg agctggaata gcggtgcact
gaccagcggt gttcatacct
ttccggcagt tctgcagagc agcggtctgt atagcctgag cagcgttgtt accgttccga gcagcagcct
gggcacccag acctatattt gtaatgttaa tcataaaccg agcaatacca aagtggataa aaaagttgag
ccgaaaagct gcgataaaac ccatctgtaa tagggtacc
SEQ ID NO:14
   Bevacizumab and Ranibizumab Light Chain CDR1
   SASQDISNYLN
SEQ ID NO:15
   Bevacizumab and Ranibizumab Light Chain CDR2
   FTSSLHS
SEQ ID NO:16
   Bevacizumab and Ranibizumab Light Chain CDR3
   QQYSTVPWT
SEQ ID NO:17
   bevacizumab Heavy Chain CDR1
   GYTFTNYGMN
SEQ ID NO:18
   Bevacizumab and Ranibizumab Heavy Chain CDR2
   WINTYTGEPTYAADFKR
SEQ ID NO:19
   Bevacizumab Heavy Chain CDR3
   YPHYYGSSHWYFDV
SEQ ID NO:20
   ranibizumab Heavy Chain CDR1
   GYDFTHYGMN
SEQ ID NO:21
   ranibizumab Heavy Chain CDR1
   YPYYYGTSHWYFDV
SEQ ID NO:22
   Albumin signal peptide
   MKWVTFISLLFLFSSAYS
SEQ ID NO:23
   Chymotrypsinogen signal peptide
   MAFLWLLSCWALLGTTFG
SEQ ID NO:24
   Interleukin-2 signal peptide
   MYRMQLLSCIALILALVTNS
SEQ ID NO:25
   Trypsinogen-2 signal peptide
   MNLLLILTFVAAAVA
SEQ ID NO:26
   F2A site
   LLNFDLLKLAGDVESNPGP
SEQ ID NO:27
   T2A site
   (GSG)EGRGSLLTCGDVEENPGP
SEQ ID NO:28
   P2A site
   (GSG)ATNFSLLKQAGDVEENPGP
SEQ ID NO:29
   E2A site
   (GSG)QCTNYALLKLAGDVESNPGP
SEQ ID NO:30
   F2A site
   (GSG)VKQTLNFDLLKLAGDVESNPGP
SEQ ID NO:31
   Furin linker
   RKRR
SEQ ID NO:32
   Furin linker
   RRRR
SEQ ID NO:33
   Furin linker
   RRKR
SEQ ID NO:34
   Furin linker
   RKKR
SEQ ID NO:35
   Furin linker
   R-X-K/R-R
SEQ ID NO:36
   Furin linker
   RXKR
SEQ ID NO:37
   Furin linker
   RXRR
SEQ ID NO:38
   Ranibizumab Fab amino acid sequence (Light chain) 
MDIQLTQSPSSLSASVGDRVTITCSASQDISNYLNWYQQKPGKAPKVLIYFTSSLHSGVPSRFSGSGSGTDFTLTIS
SLQPEDFATYYCQQYSTVPWTFGQGTKVEIKRTVAAPSVFIFPPSDEQLKSGTASVVCLLNNFYPREAKVQWKVDNA
LQSGNSQESVTEQDSKDSTYSLSSTLTLSKADYEKHKVYACEVTHQGLSSPVTKSFNRGEC
SEQ ID NO:39
   Ranibizumab Fab amino acid sequence (Heavy chain) 
MEVQLVESGGGLVQPGGSLRLSCAASGYDFTHYGMNWVRQAPGKGLEWVGWINTYTGEPTYAADFKRRFTFSLDTSK
STAYLQMNSLRAEDTAVYYCAKYPYYYGTSHWYFDVWGQGTLVTVSSASTKGPSVFPLAPSSKSTSGGTAALGCLVK
DYFPEPVTVSWNSGALTSGVHTFPAVLQSSGLYSLSSVVTVPSSSLGTQTYICNVNHKPSNTKVDKKVEPKSCDKTH
LRKRR
SEQ ID NO:40
   Ranibizumab Fab amino acid sequence (Heavy chain) 
MEVQLVESGGGLVQPGGSLRLSCAASGYDFTHYGMNWVRQAPGKGLEWVGWINTYTGEPTYAADFKRRFTFSLDTSK
STAYLQMNSLRAEDTAVYYCAKYPYYYGTSHWYFDVWGQGTLVTVSSASTKGPSVFPLAPSSKSTSGGTAALGCLVK
 
DYFPEPVTVSWNSGALTSGVHTFPAVLQSSGLYSLSSVVTVPSSSLGTQTYICNVNHKPSNTKVDKKVEPKSCDKTH
L
SEQ ID NO:41
   AAV1 
MAADGYLPDWLEDNLSEGIREWWDLKPGAPKPKANQQKQDDGRGLVLPGYKYLGPFNGLDKGEPVNAADAAALEHDK
AYDQQLKAGDNPYLRYNHADAEFQERLQEDTSFGGNLGRAVFQAKKRVLEPLGLVEEGAKTAPGKKRPVEQSPQEPD
SSSGIGKTGQQPAKKRLNFGQTGDSESVPDPQPLGEPPATPAAVGPTTMASGGGAPMADNNEGADGVGNASGNWHCD
STWLGDRVITTSTRTWALPTYNNHLYKQISSASTGASNDNHYFGYSTPWGYFDFNRFHCHFSPRDWQRLINNNWGFR
PKRLNFKLFNIQVKEVTTNDGVTTIANNLTSTVQVFSDSEYQLPYVLGSAHQGCLPPFPADVFMIPQYGYLTLNNGS
QAVGRSSFYCLEYFPSQMLRTGNNFTFSYTFEEVPFHSSYAHSQSLDRLMNPLIDQYLYYLNRTQNQSGSAQNKDLL
FSRGSPAGMSVQPKNWLPGPCYRQQRVSKTKTDNNNSNFTWTGASKYNLNGRESIINPGTAMASHKDDEDKFFPMSG
VMIFGKESAGASNTALDNVMITDEEEIKATNPVATERFGTVAVNFQSSSTDPATGDVHAMGALPGMVWQDRDVYLQG
PIWAKIPHTDGHFHPSPLMGGFGLKNPPPQILIKNTPVPANPPAEFSATKFASFITQYSTGQVSVEIEWELQKENSK
RWNPEVQYTSNYAKSANVDFTVDNNGLYTEPRPIGTRYLTRPL
SEQ ID NO: 42
   AAV2
SEQ ID NO: 43
   AAV3-3 
MAADGYLPDWLEDNLSEGIREWWALKPGVPQPKANQQHQDNRRGLVLPGYKYLGPGNGLDKGEPVNEADAAALEHDK
AYDQQLKAGDNPYLKYNHADAEFQERLQEDTSFGGNLGRAVFQAKKRILEPLGLVEEAAKTAPGKKGAVDQSPQEPD
SSSGVGKSGKQPARKRLNFGQTGDSESVPDPQPLGEPPAAPTSLGSNTMASGGGAPMADNNEGADGVGNSSGNWHCD
SQWLGDRVITTSTRTWALPTYNNHLYKQISSQSGASNDNHYFGYSTPWGYFDFNRFHCHFSPRDWQRLINNNWGFRP
KKLSFKLFNIQVRGVTQNDGTTTIANNLTSTVQVFTDSEYQLPYVLGSAHQGCLPPFPADVFMVPQYGYLTLNNGSQ
AVGRSSFYCLEYFPSQMLRTGNNFQFSYTFEDVPFHSSYAHSQSLDRLMNPLIDQYLYYLNRTQGTTSGTTNQSRLL
FSQAGPQSMSLQARNWLPGPCYRQQRLSKTANDNNNSNFPWTAASKYHLNGRDSLVNPGPAMASHKDDEEKFFPMHG
NLIFGKEGTTASNAELDNVMITDEEEIRTTNPVATEQYGTVANNLQSSNTAPTTGTVNHQGALPGMVWQDRDVYLQG
PIWAKIPHTDGHFHPSPLMGGFGLKHPPPQIMIKNTPVPANPPTTFSPAKFASFITQYSTGQVSVEIEWELQKENSK
RWNPEIQYTSNYNKSVNVDFTVDTNGVYSEPRPIGTRYLTRNL
SEQ ID NO: 44
   AAV4-4 
MTDGYLPDWLEDNLSEGVREWWALQPGAPKPKANQQHQDNARGLVLPGYKYLGPGNGLDKGEPVNAADAAALEHDKA
YDQQLKAGDNPYLKYNHADAEFQQRLQGDTSFGGNLGRAVFQAKKRVLEPLGLVEQAGETAPGKKRPLIESPQQPDS
STGIGKKGKQPAKKKLVFEDETGAGDGPPEGSTSGAMSDDSEMRAAAGGAAVEGGQGADGVGNASGDWHCDSTWSEG
HVTTTSTRTWVLPTYNNHLYKRLGESLQSNTYNGFSTPWGYFDFNRFHCHFSPRDWQRLINNNWGMRPKAMRVKIFN
IQVKEVTTSNGETTVANNLTSTVQIFADSSYELPYVMDAGQEGSLPPFPNDVFMVPQYGYCGLVTGNTSQQQTDRNA
FYCLEYFPSQMLRTGNNFEITYSFEKVPFHSMYAHSQSLDRLMNPLIDQYLWGLQSTTTGTTLNAGTATTNFTKLRP
TNFSNFKKNWLPGPSIKQQGFSKTANQNYKIPATGSDSLIKYETHSTLDGRWSALTPGPPMATAGPADSKFSNSQLI
FAGPKQNGNTATVPGTLIFTSEEELAATNATDTDMWGNLPGGDQSNSNLPTVDRLTALGAVPGMVWQNRDIYYQGPI
WAKIPHTDGHFHPSPLIGGFGLKHPPPQIFIKNTPVPANPATTFSSTPVNSFITQYSTGQVSVQIDWEIQKERSKRW
NPEVQFTSNYGQQNSLLWAPDAAGKYTEPRAIGTRYLTHHL
SEQ ID NO: 45
   AAV5 
MSFVDHPPDWLEEVGEGLREFLGLEAGPPKPKPNQQHQDQARGLVLPGYNYLGPGNGLDRGEPVNRADEVAREHDIS
YNEQLEAGDNPYLKYNHADAEFQEKLADDTSFGGNLGKAVFQAKKRVLEPFGLVEEGAKTAPTGKRIDDHFPKRKKA
RTEEDSKPSTSSDAEAGPSGSQQLQIPAQPASSLGADTMSAGGGGPLGDNNQGADGVGNASGDWHCDSTWMGDRVVT
KSTRTWVLPSYNNHQYREIKSGSVDGSNANAYFGYSTPWGYFDFNRFHSHWSPRDWQRLINNYWGFRPRSLRVKIFN
IQVKEVTVQDSTTTIANNLTSTVQVFTDDDYQLPYVVGNGTEGCLPAFPPQVFTLPQYGYATLNRDNTENPTERSSF
FCLEYFPSKMLRTGNNFEFTYNFEEVPFHSSFAPSQNLFKLANPLVDQYLYRFVSTNNTGGVQFNKNLAGRYANTYK
NWFPGPMGRTQGWNLGSGVNRASVSAFATTNRMELEGASYQVPPQPNGMTNNLQGSNTYALENTMIFNSQPANPGTT
ATYLEGNMLITSESETQPVNRVAYNVGGQMATNNQSSTTAPATGTYNLQEIVPGSVWMERDVYLQGPIWAKIPETGA
HFHPSPAMGGFGLKHPPPMMLIKNTPVPGNITSFSDVPVSSFITQYSTGQVTVEMEWELKKENSKRWNPEIQYTNNY
NDPQFVDFAPDSTGEYRTTRPIGTRYLTRPL
SEQ ID NO: 46
   AAV6 
MAADGYLPDWLEDNLSEGIREWWDLKPGAPKPKANQQKQDDGRGLVLPGYKYLGPFNGLDKGEPVNAADAAALEHDK
AYDQQLKAGDNPYLRYNHADAEFQERLQEDTSFGGNLGRAVFQAKKRVLEPFGLVEEGAKTAPGKKRPVEQSPQEPD
SSSGIGKTGQQPAKKRLNFGQTGDSESVPDPQPLGEPPATPAAVGPTTMASGGGAPMADNNEGADGVGNASGNWHCD
STWLGDRVITTSTRTWALPTYNNHLYKQISSASTGASNDNHYFGYSTPWGYFDFNRFHCHFSPRDWQRLINNNWGFR
PKRLNFKLFNIQVKEVTTNDGVTTIANNLTSTVQVFSDSEYQLPYVLGSAHQGCLPPFPADVFMIPQYGYLTLNNGS
QAVGRSSFYCLEYFPSQMLRTGNNFTFSYTFEDVPFHSSYAHSQSLDRLMNPLIDQYLYYLNRTQNQSGSAQNKDLL
FSRGSPAGMSVQPKNWLPGPCYRQQRVSKTKTDNNNSNFTWTGASKYNLNGRESIINPGTAMASHKDDKDKFFPMSG
VMIFGKESAGASNTALDNVMITDEEEIKATNPVATERFGTVAVNLQSSSTDPATGDVHVMGALPGMVWQDRDVYLQG
PIWAKIPHTDGHFHPSPLMGGFGLKHPPPQILIKNTPVPANPPAEFSATKFASFITQYSTGQVSVEIEWELQKENSK
RWNPEVQYTSNYAKSANVDFTVDNNGLYTEPRPIGTRYLTRPL
SEQ ID NO: 47
   AAV7 
MAADGYLPDWLEDNLSEGIREWWDLKPGAPKPKANQQKQDNGRGLVLPGYKYLGPFNGLDKGEPVNAADAAALEHDK
AYDQQLKAGDNPYLRYNHADAEFQERLQEDTSFGGNLGRAVFQAKKRVLEPLGLVEEGAKTAPAKKRPVEPSPQRSP
DSSTGIGKKGQQPARKRLNFGQTGDSESVPDPQPLGEPPAAPSSVGSGTVAAGGGAPMADNNEGADGVGNASGNWHC
DSTWLGDRVITTSTRTWALPTYNNHLYKQISSETAGSTNDNTYFGYSTPWGYFDFNRFHCHFSPRDWQRLINNNWGF
RPKKLRFKLFNIQVKEVTTNDGVTTIANNLTSTIQVFSDSEYQLPYVLGSAHQGCLPPFPADVFMIPQYGYLTLNNG
SQSVGRSSFYCLEYFPSQMLRTGNNFEFSYSFEDVPFHSSYAHSQSLDRLMNPLIDQYLYYLARTQSNPGGTAGNRE
LQFYQGGPSTMAEQAKNWLPGPCFRQQRVSKTLDQNNNSNFAWTGATKYHLNGRNSLVNPGVAMATHKDDEDRFFPS
SGVLIFGKTGATNKTTLENVLMTNEEEIRPTNPVATEEYGIVSSNLQAANTAAQTQVVNNQGALPGMVWQNRDVYLQ
GPIWAKIPHTDGNFHPSPLMGGFGLKHPPPQILIKNTPVPANPPEVFTPAKFASFITQYSTGQVSVEIEWELQKENS
KRWNPEIQYTSNFEKQTGVDFAVDSQGVYSEPRPIGTRYLTRNL
SEQ ID NO: 48
   AAV8 
MAADGYLPDWLEDNLSEGIREWWALKPGAPKPKANQQKQDDGRGLVLPGYKYLGPFNGLDKGEPVNAADAAALEHDK
AYDQQLQAGDNPYLRYNHADAEFQERLQEDTSFGGNLGRAVFQAKKRVLEPLGLVEEGAKTAPGKKRPVEPSPQRSP
DSSTGIGKKGQQPARKRLNFGQTGDSESVPDPQPLGEPPAAPSGVGPNTMAAGGGAPMADNNEGADGVGSSSGNWHC
DSTWLGDRVITTSTRTWALPTYNNHLYKQISNGTSGGATNDNTYFGYSTPWGYFDFNRFHCHFSPRDWQRLINNNWG
FRPKRLSFKLFNIQVKEVTQNEGTKTIANNLTSTIQVFTDSEYQLPYVLGSAHQGCLPPFPADVFMIPQYGYLTLNN
GSQAVGRSSFYCLEYFPSQMLRTGNNFQFTYTFEDVPFHSSYAHSQSLDRLMNPLIDQYLYYLSRTQTTGGTANTQT
LGFSQGGPNTMANQAKNWLPGPCYRQQRVSTTTGQNNNSNFAWTAGTKYHLNGRNSLANPGIAMATHKDDEERFFPS
NGILIFGKQNAARDNADYSDVMLTSEEEIKTTNPVATEEYGIVADNLQQNTAPQIGTVNSQGALPGMVWQNRDVYL
QGPIWAKIPHTDGNFHPSPLMGGFGLKHPPPQILIKNTPVPADPPTTFNQSKLNSFITQYSTGQVSVEIEWELQKEN
SKRWNPEIQYTSNYYKSTSVDFAVNTEGVYSEPRPIGTRYLTRNL
SEQ ID NO: 49
   hu31 
MAADGYLPDWLEDTLSEGIRQWWKLKPGPPPPKPAERHKDDSRGLVLPGYKYLGPGNGLDKGEPVNAADAAALEHDK
AYDQQLKAGDNPYLKYNHADAEFQERLKEDTSFGGNLGRAVFQAKKRLLEPLGLVEEAAKTAPGKKRPVEQSPQEPD
SSAGIGKSGSQPAKKKLNFGQTGDTESVPDPQPIGEPPAAPSGVGSLTMASGGGAPVADNNEGADGVGSSSGNWHCD
SQWLGDRVITTSTRTWALPTYNNHLYKQISNSTSGGSSNDNAYFGYSTPWGYFDFNRFHCHFSPRDWQRLINNNWGF
RPKRLNFKLFNIQVKEVTDNNGVKTIANNLTSTVQVFTDSDYQLPYVLGSAHEGCLPPFPADVFMIPQYGYLTLNDG
 
GQAVGRSSFYCLEYFPSQMLRTGNNFQFSYEFENVPFHSSYAHSQSLDRLMNPLIDQYLYYLSKTINGSGQNQQTLK
FSVAGPSNMAVQGRNYIPGPSYRQQRVSTTVTQNNNSEFAWPGASSWALNGRNSLMNPGPAMASHKEGEDRFFPLSG
SLIFGKQGTGRDNVDADKVMITNEEEIKTTNPVATESYGQVATNHQSAQAQAQTGWVQNQGILPGMVWQDRDVYLQG
PIWAKIPHTDGNFHPSPLMGGFGMKHPPPQILIKNTPVPADPPTAFNKDKLNSFITQYSTGQVSVEIEWELQKENSK
RWNPEIQYTSNYYKSNNVEFAVSTEGVYSEPRPIGTRYLTRNL
SEQ ID NO: 50
   hu32 
MAADGYLPDWLEDTLSEGIRQWWKLKPGPPPPKPAERHKDDSRGLVLPGYKYLGPGNGLDKGEPVNAADAAALEHDK
AYDQQLKAGDNPYLKYNHADAEFQERLKEDTSFGGNLGRAVFQAKKRLLEPLGLVEEAAKTAPGKKRPVEQSPQEPD
SSAGIGKSGSQPAKKKLNFGQTGDTESVPDPQPIGEPPAAPSGVGSLTMASGGGAPVADNNEGADGVGSSSGNWHCD
SQWLGDRVITTSTRTWALPTYNNHLYKQISNSTSGGSSNDNAYFGYSTPWGYFDFNRFHCHFSPRDWQRLINNNWGF
RPKRLNFKLFNIQVKEVTDNNGVKTIANNLTSTVQVFTDSDYQLPYVLGSAHEGCLPPFPADVFMIPQYGYLTLNDG
SQAVGRSSFYCLEYFPSQMLRTGNNFQFSYEFENVPFHSSYAHSQSLDRLMNPLIDQYLYYLSKTINGSGQNQQTLK
FSVAGPSNMAVQGRNYIPGPSYRQQRVSTTVTQNNNSEFAWPGASSWALNGRNSLMNPGPAMASHKEGEDRFFPLSG
SLIFGKQGTGRDNVDADKVMITNEEEIKTTNPVATESYGQVATNHQSAQAQAQTGWVQNQGILPGMVWQDRDVYLQG
PIWAKIPHTDGNFHPSPLMGGFGMKHPPPQILIKNTPVPADPPTAFNKDKLNSFITQYSTGQVSVEIEWELQKENSK
RWNPEIQYTSNYYKSNNVEFAVNTEGVYSEPRPIGTRYLTRNL
SEQ ID NO: 51
   AAV9 
MAADGYLPDWLEDNLSEGIREWWALKPGAPQPKANQQHQDNARGLVLPGYKYLGPGNGLDKGEPVNAADAAALEHDK
AYDQQLKAGDNPYLKYNHADAEFQERLKEDTSFGGNLGRAVFQAKKRLLEPLGLVEEAAKTAPGKKRPVEQSPQEPD
SSAGIGKSGAQPAKKRLNFGQTGDTESVPDPQPIGEPPAAPSGVGSLTMASGGGAPVADNNEGADGVGSSSGNWHCD
SQWLGDRVITTSTRTWALPTYNNHLYKQISNSTSGGSSNDNAYFGYSTPWGYFDFNRFHCHFSPRDWQRLINNNWGF
RPKRLNFKLFNIQVKEVTDNNGVKTIANNLTSTVQVFTDSDYQLPYVLGSAHEGCLPPFPADVFMIPQYGYLTLNDG
SQAVGRSSFYCLEYFPSQMLRTGNNFQFSYEFENVPFHSSYAHSQSLDRLMNPLIDQYLYYLSKTINGSGQNQQTLK
FSVAGPSNMAVQGRNYIPGPSYRQQRVSTTVTQNNNSEFAWPGASSWALNGRNSLMNPGPAMASHKEGEDRFFPLSG
SLIFGKQGTGRDNVDADKVMITNEEEIKTTNPVATESYGQVATNHQSAQAQAQTGWVQNQGILPGMVWQDRDVYLQG
PIWAKIPHTDGNFHPSPLMGGFGMKHPPPQILIKNTPVPADPPTAFNKDKLNSFITQYSTGQVSVEIEWELQKENSK
RWNPEIQYTSNYYKSNNVEFAVNTEGVYSEPRPIGTRYLTRNL
SEQ ID NO:52
   SPERMATOGENESIS ASSOCIATED 7 (SPATA7);
   AAH90875.1 
1 MDGSRRVRAT SVLPRYGPPC LFKGHLSTKS NAAVDCSVPV SVSTSIKYAD QQRREKLKKE
61 LAQCEKEFKL TKTAMRANYK NNSKSLFNTL QKPSGEPQIE DDMLKEEMNG FSSFARSLVP
121 SSERLHLSLH KSSKVITNGP EKNSSSSPSS VDYAASGPRK LSSGALYGRR PRSTFPNSHR
181 FQLVISKAPS GDLLDKHSEL FSNKQLPFTP RTLKTEAKSF LSQYRYYTPA KRKKDFTDQR
241 IEAETQTELS FKSELGTAET KNMTDSEMNI KQASNCVTYD AKEKIAPLPL EGHDSTWDEI
301 KDDALQHSSP RAMCQYSLKP PSTRKIYSDE EELLYLSFIE DVTDEILKLG LFSNRFLERL
361 FERHIKQNKH LEEEKMRHLL HVLKVDLGCT SEENSVKQND VDMLNVFDFE KAGNSEPNEL
421 KNESEVTIQQ ERQQYQKALD MLLSAPKDEN EIFPSPTEFF MPIYKSKHSE GVIIQQVNDE
481 TNLETSTLDE NHPSISDSLT DRETSVNVIE GDSDPEKVEI SNGLCGLNTS PSQSVQFSSV
541 KGDNNHDMEL STLKIMEMSI EDCPLDV
SEQ ID NO:53
   LEBERCILIN (LCA5)
   NP_001116241.1 
1 
61 
121 
181 
241 
301 
 
361 
421 
481 
541 
601 
661 
SEQ ID NO:54
   RPGR INTERACTING PROTEIN 1 (RPGRIP1)
   CAD01135.1 
1 MSHLVDPTSG DLPVRDIDAI PLVLPASKGK NMKTQPPLSR MNREELEDSF FRLREDHMLV
61 KELSWKQQDE IKRLRTTLLR LTAAGRDLRV AEEAAPLSET ARRGQKAGWR QRLSMHQRPQ
121 MHRLQGHFHC VGPASPRRAQ PRVQVGHRQL HTAGAPVPEK PKRGPRDRLS YTAPPSFKEH
181 ATNENRGEVA SKPSELVSGS NSIISFSSVI SMAKPIGLCM PNSAHIMASN TMQVEEPPKS
241 PEKMWPKDEN FEQRSSLECA QKAAELRASI KEKVELIRLK KLLHERNASL VMTKAQLTEV
301 QEAYETLLQK NQGILSAAHE ALLKQVNELR AELKEESKKA VSLKSQLEDV SILQMTLKEF
361 QERVEDLEKE RKLLNDNYDK LLESMLDSSD SSSQPHWSNE LIAEQLQQQV SQLQDQLDAE
421 LEDKRKVLLE LSREKAQNED LKLEVTNILQ KHKQEVELLQ NAATISQPPD RQSEPATHPA
481 VLQENTQIEP SEPKNQEEKK LSQVLNELQV SHAETTLELE KTRDMLILQR KINVCYQEEL
541 EAMMTKADND NRDHKEKLER LTRLLDLKNN RIKQLEGILR SHDLPTSEQL KDVAYGTRPL
601 SLCLETLPAH GDEDKVDISL LHQGENLFEL HIHQAFLTSA ALAQAGDTQP TTFCTYSFYD
661 FETHCTPLSV GPQPLYDFTS QYVMETDSLF LHYLQEASAR LDIHQAMASE HSTLAAGWIC
721 FDRVLETVEK VHGLATLIGA GGEEFGVLEY WMRLRFPIKP SLQACNKRKK AQVYLSTDVL
781 GGRKAQEEEF RSESWEPQNE LWIEITKCCG LRSRWLGTQP SPYAVYRFFT FSDHDTAIIP
841 ASNNPYFRDQ ARFPVLVTSD LDHYLRREAL SIHVFDDEDL EPGSYLGRAR VPLLPLAKNE
901 SIKGDFNLTD PAEKPNGSIQ VQLDWKFPYI PPESFLKPEA QTKGKDTKDS SKISSEEEKA
961 SFPSQDQMAS PEVPIEAGQY RSKRKPPHGG ERKEKEHQVV SYSRRKHGKR IGVQGKNRME
1021 YLSLNILNGN TPQQVNYTEW KFSETNSFIG DGFKNQHEEE EMTLSHSALK QKEPLHPVND
1081 KESSEQGSEV SEAQTTDSDD VIVPPMSQKY PKADSEKMCI EIVSLAFYPE AEVMSDENIK
1141 QVYVEYKFYD LPLSETETPV SLRKPRAGEE IHFHFSKVID LDPQEQQGRR RFLFDMLNGQ
1201 DPDQGHLKFT VVSDPLDEEK KECEEVGYAY LQLWQILESG RDILEQELDI VSPEDLATPI
1261 GRLKVSLQAA AVLHAIYKEM TEDLFS
SEQ ID NO:55
   CONE-ROD HOMEOBOX (CRX)
   EAW57515.1 
1 
61 
121 
181 
241 
SEQ ID NO:56
   CRUMBS CELL POLARITY COMPLEX COMPONENT 1 (CRB1), HOMOLOG 1 ISOFORM 1 PRECURSOR
   NP_957705.1 
1 
61 
121 
181 
241 
301 
361 
421 
481 
541 
601 
 
 661 
 721 
 781 
 841 
 901 
 961 
1021 
1081 
1141 
1201 
1261 
1321 
1381 
SEQ ID NO:57
   CRUMBS CELL POLARITY COMPLEX COMPONENT 1 (CRB1), HOMOLOG 1 ISOFORM 2 PRECURSOR
   NP_001180569.1 
1 MALKNINYLL IFYLSFSLLI YIKNSFCNKN NTRCLSNSCQ NNSTCKDFSK DNDCSCSDTA
61 NNLDKDCDNM KDPCFSNPCQ GSATCVNTPG ERSFLCKCPP GYSGTICETT IGSCGKNSCQ
121 HGGICHQDPI YPVCICPAGY AGRFCEIDHD ECASSPCQNG AVCQDGIDGY SCFCVPGYQG
181 RHCDLEVDEC ASDPCKNEAT CLNEIGRYTC ICPHNYSGYT GAQCEIDLNE CNSNPCQSNG
241 ECVELSSEKQ YGRITGLPSS FSYHEASGYV CICQPGFTGI HCEEDVNECS SNPCQNGGTC
301 ENLPGNYTCH CPFDNLSRTF YGGRDCSDIL LGCTHQQCLN NGTCIPHFQD GQHGFSCLCP
361 SGYTGSLCEI ATTLSFEGDG FLWVKSGSVT TKGSVCNIAL RFQTVQPMAL LLFRSNRDVF
421 VKLELLSGYI HLSIQVNNQS KVLLFISHNT SDGEWHFVEV IFAEAVTLTL IDDSCKEKCI
481 AKAPTPLESD QSICAFQNSF LGGLPVGMTS NGVALLNFYN MPSTPSFVGC LQDIKIDWNH
541 ITLENISSGS SLNVKAGCVR KDWCESQPCQ SRGRCINLWL SYQCDCHRPY EGPNCLREYV
601 AGRFGQDDST GYVIFTLDES YGDTISLSMF VRTLQPSGLL LALENSTYQY IRVWLERGRL
661 AMLTPNSPKL VVKFVLNDGN VHLISLKIKP YKIELYQSSQ NLGFISASTW KIEKGDVIYI
721 GGLPDKQETE LNGGFFKGCI QDVRLNNQNL EFFPNPTNNA SLNPVLVNVT QGCAGDNSCK
781 SNPCHNGGVC HSRWDDFSCS CPALTSGKAC EEVQWCGFSP CPHGAQCQPV LQGFECIANA
841 VFNGQSGQIL FRSNGNITRE LTNITFGFRT RDANVIILHA EKEPEFLNIS IQDSRLFFQL
901 QSGNSFYMLS LTSLQSVNDG TWHEVTLSMT DPLSQTSRWQ MEVDNETPFV TSTIATGSLN
961 FLKDNTDIYV GDRAIDNIKG LQGCLSTIEI GGIYLSYFEN VHGFINKPQE EQFLKISTNS
1021 VVTGCLQLNV CNSNPCLHGG NCEDIYSSYH CSCPLGWSGK HCELNIDECF SNPCIHGNCS
1081 DRVAAYHCTC EPGYTGVNCE VDIDNCQSHQ CANGATCISH TNGYSCLCFG NFTGKFCRQS
1141 RLPSTVCGNE KTNLTCYNGG NCTEFQTELK CMCRPGFTGE WCEKDIDECA SDPCVNGGLC
1201 QDLLNKFQCL CDVAFAGERC EVDLADDLIS DIFTTIGSVT VALLLILLLA IVASVVTSNK
1261 RATQGTYSPS RQEKEGSRVE MWNLMPPPAM ERLI
SEQ ID NO:58
   NICOTINAMIDE NUCLEOTIDE ADENYLYLTRANSFERASE 1 (NMNAT1)
   Q9HAN9.1 
1 
61 
121 
181 
241 
SEQ ID NO:59
   NICOTINAMIDE NUCLEOTIDE ADENYLYLTRANSFERASE 1 (NMNAT1); ISOFORM CRA_A
   EAW71635.1 
1 
61 
121 
181 
 241 
SEQ ID NO:60
   NICOTINAMIDE NUCLEOTIDE ADENYLYLTRANSFERASE 1 (NMNAT1), ISOFORM 2
   NP 001284708.1 
1 MENSEKTEVV LLACGSFNPI TNMHLRLFEL AKDYMNGTGR YTVVKGIISP VGDAYKKKGL
61 IPAYHRVIMA ELATKNSKWV EVDTWESLQK EWKETLKVLR HHQEKLEASD CDHQQNSPTL
121 ERPGRKRKWT ETQDSSQKKS LEPKTKDGVS LYHPGWSAVA
SEQ ID NO:61
   NICOTINAMIDE NUCLEOTIDE ADENYLYLTRANSFERASE 1 (NMNAT1), ISOFORM 1
   NP 073624.2 
1 
61 
121 
181 
241 
SEQ ID NO:62
   CENTROSOMAL PROTEIN 290 (CEP290);
   NP 079390.3 
1 MPPNINWKEI MKVDPDDLPR QEELADNLLI SLSKVEVNEL KSEKQENVIH LFRITQSLMK
61 MKAQEVELAL EEVEKAGEEQ AKFENQLKTK VMKLENELEM AQQSAGGRDT RFLRNEICQL
121 EKQLEQKDRE LEDMEKELEK EKKVNEQLAL RNEEAENENS KLRRENKRLK KKNEQLCQDI
181 IDYQKQIDSQ KETLLSRRGE DSDYRSQLSK KNYELIQYLD EIQTLTEANE KIEVQNQEMR
241 KNLEESVQEM EKMTDEYNRM KAIVHQTDNV IDQLKKENDH YQLQVQELTD LLKSKNEEDD
301 PIMVAVNAKV EEWKLILSSK DDEIIEYQQM LHNLREKLKN AQLDADKSNV MALQQGIQER
361 DSQIKMLTEQ VEQYTKEMEK NTCIIEDLKN ELQRNKGAST LSQQTHMKIQ STLDILKEKT
421 KEAERTAELA EADAREKDKE LVEALKRLKD YESGVYGLED AVVEIKNCKN QIKIRDREIE
481 ILTKEINKLE LKISDFLDEN EALRERVGLE PKTMIDLTEF RNSKHLKQQQ YRAENQILLK
541 EIESLEEERL DLKKKIRQMA QERGKRSATS GLTTEDLNLT ENISQGDRIS ERKLDLLSLK
601 NMSEAQSKNE FLSRELIEKE RDLERSRTVI AKFQNKLKEL VEENKQLEEG MKEILQAIKE
661 MQKDPDVKGG ETSLIIPSLE RLVNAIESKN AEGIFDASLH LKAQVDQLTG RNEELRQELR
721 ESRKEAINYS QQLAKANLKI DHLEKETSLL RQSEGSNVVF KGIDLPDGIA PSSASIINSQ
781 NEYLIHLLQE LENKEKKLKN LEDSLEDYNR KFAVIRHQQS LLYKEYLSEK ETWKTESKTI
841 KEEKRKLEDQ VQQDAIKVKE YNNLLNALQM DSDEMKKILA ENSRKITVLQ VNEKSLIRQY
901 TTLVELERQL RKENEKQKNE LLSMEAEVCE KIGCLQRFKE MAIFKIAALQ KVVDNSVSLS
961 ELELANKQYN ELTAKYRDIL QKDNMLVQRT SNLEHLECEN ISLKEQVESI NKELEITKEK
1021 LHTIEQAWEQ ETKLGNESSM DKAKKSITNS DIVSISKKIT MLEMKELNER QRAEHCQKMY
1081 EHLRTSLKQM EERNFELETK FAELTKINLD AQKVEQMLRD ELADSVSKAV SDADRQRILE
1141 LEKNEMELKV EVSKLREISD IARRQVEILN AQQQSRDKEV ESLRMQLLDY QAQSDEKSLI
1201 AKLHQHNVSL QLSEATALGK LESITSKLQK MEAYNLRLEQ KLDEKEQALY YARLEGRNRA
1261 KHLRQTIQSL RRQFSGALPL AQQEKFSKTM IQLQNDKLKI MQEMKNSQQE HRNMENKTLE
1321 MELKLKGLEE LISTLKDTKG AQKVINWHMK IEELRLQELK LNRELVKDKE EIKYLNNIIS
1381 EYERTISSLE EEIVQQNKFH EERQMAWDQR EVDLERQLDI FDRQQNEILN AAQKFEEATG
1441 SIPDPSLPLP NQLEIALRKI KENIRIILET RATCKSLEEK LKEKESALRL AEQNILSRDK
1501 VINELRLRLP ATAEREKLIA ELGRKEMEPK SHHTLKIAHQ TIANMQARLN QKEEVLKKYQ
1561 RLLEKAREEQ REIVKKHEED LHILHHRLEL QADSSLNKFK QTAWDLMKQS PTPVPTNKHF
1621 IRLAEMEQTV AEQDDSLSSL LVKLKKVSQD LERQREITEL KVKEFENIKL QLQENHEDEV
1681 KKVKAEVEDL KYLLDQSQKE SQCLKSELQA QKEANSRAPT TTMRNLVERL KSQLALKEKQ
1741 QKALSRALLE LRAEMTAAAE ERIISATSQK EAHLNVQQIV DRHTRELKTQ VEDLNENLLK
1801 LKEALKTSKN RENSLTDNLN DLNNELQKKQ KAYNKILREK EEIDQENDEL KRQIKRLTSG
1861 LQGKPLTDNK QSLIEELQRK VKKLENQLEG KVEEVDLKPM KEKNAKEELI RWEEGKKWQA
1921 KIEGIRNKLK EKEGEVFTLT KQLNTLKDLF AKADKEKLTL QRKLKTTGMT VDQVLGIRAL
1981 ESEKELEELK KRNLDLENDI LYMRAHQALP RDSVVEDLHL QNRYLQEKLH ALEKQFSKDT
2041 YSKPSISGIE SDDHCQREQE LQKENLKLSS ENIELKFQLE QANKDLPRLK NQVRDLKEMC
2101 EFLKKEKAEV QRKLGHVRGS GRSGKTIPEL EKTIGLMKKV VEKVQRENEQ LKKASGILTS
 
2161 EKMANIEQEN EKLKAELEKL KAHLGHQLSM HYESKTKGTE KIIAENERLR KELKKETDAA
2221 EKLRIAKNNL EILNEKMTVQ LEETGKRLQF AESRGPQLEG ADSKSWKSIV VTRMYETKLK
2281 ELETDIAKKN QSITDLKQLV KEATEREQKV NKYNEDLEQQ IKILKHVPEG AETEQGLKRE
2341 LQVLRLANHQ LDKEKAELIH QIEANKDQSG AESTIPDADQ LKEKIKDLET QLKMSDLEKQ
2401 HLKEEIKKLK KELENFDPSF FEEIEDLKYN YKEEVKKNIL LEEKVKKLSE QLGVELTSPV
2461 AASEEFEDEE ESPVNFPIY
SEQ ID NO:63
   CENTROSOMAL PROTEIN 290 (CEP290), ISOFORM X1
   XP 011537059.1 
1 MPPNINWKEI MKVDPDDLPR QEELADNLLI SLSKVEVNEL KSEKQENVIH LFRITQSLMK
61 MKAQEVELAL EEVEKAGEEQ AKFENQLKTK VMKLENELEM AQQSAGGRDT RFLRNEICQL
121 EKQLEQKDRE LEDMEKELEK EKKVNEQLAL RNEEAENENS KLRRENKRLK KKNEQLCQDI
181 IDYQKQIDSQ KETLLSRRGE DSDYRSQLSK KNYELIQYLD EIQTLTEANE KIEVQNQEMR
241 KNLEESVQEM EKMTDEYNRM KAIVHQTDNV IDQLKKENDH YQLQVQELTD LLKSKNEEDD
301 PIMVAVNAKV EEWKLILSSK DDEIIEYQQM LHNLREKLKN AQLDADKSNV MALQQGIQER
361 DSQIKMLTEQ VEQYTKEMEK NTCIIEDLKN ELQRNKGAST LSQQTHMKIQ STLDILKEKT
421 KEAERTAELA EADAREKDKE LVEALKRLKD YESGVYGLED AVVEIKNCKN QIKIRDREIE
481 ILTKEINKLE LKISDFLDEN EALRERVGLE PKTMIDLTEF RNSKHLKQQQ YRAENQILLK
541 EIESLEEERL DLKKKIRQMA QERGKRSATS GLTTEDLNLT ENISQGDRIS ERKLDLLSLK
601 NMSEAQSKIR SSDKAELLHR RSSFNTPQSD QNETEENMTI GSLSRMLSEI HHSVESGMHP
661 FVPLTRLSSS MQVKENSTPE TITIREIFKA PCLQSSRNLE SLVSTFSRES HEEINDICLF
721 SDDCMKKVSR SHQALEKTSF VQKSNSSFHG LSTASDIMQK LSLRQKSAIF CQQIHENRAD
781 MDKSQVATLE EEQVHSQVKY ADINLKEDII KSEVPLQTEI LKNKLKVNLP DPVSITAQSK
841 LSQINSLENL IEQLRRELVF LRSQNEIIAQ EFLIKEAECR NADIELEHHR SQAEQNEFLS
901 RELIEKERDL ERSRTVIAKF QNKLKELVEE NKQLEEGMKE ILQAIKEMQK DPDVKGGETS
961 LIIPSLERLV NAIESKNAEG IFDASLHLKA QVDQLTGRNE ELRQELRESR KEAINYSQQL
1021 AKANLKIDHL EKETSLLRQS EGSNVVFKGI DLPDGIAPSS ASIINSQNEY LIHLLQELEN
1081 KEKKLKNLED SLEDYNRKFA VIRHQQSLLY KEYLSEKETW KTESKTIKEE KRKLEDQVQQ
1141 DAIKVKEYNN LLNALQMDSD EMKKILAENS RKITVLQVNE KSLIRQYTTL VELERQLRKE
1201 NEKQKNELLS MEAEVCEKIG CLQRFKEMAI FKIAALQKVV DNSVSLSELE LANKQYNELT
1261 AKYRDILQKD NMLVQRTSNL EHLECENISL KEQVESINKE LEITKEKLHT IEQAWEQETK
1321 LGNESSMDKA KKSITNSDIV SISKKITMLE MKELNERQRA EHCQKMYEHL RTSLKQMEER
1381 NFELETKFAE LTKINLDAQK VEQMLRDELA DSVSKAVSDA DRQRILELEK NEMELKVEVS
1441 KLREISDIAR RQVEILNAQQ QSRDKEVESL RMQLLDYQAQ SDEKSLIAKL HQHNVSLQLS
1501 EATALGKLES ITSKLQKMEA YNLRLEQKLD EKEQALYYAR LEGRNRAKHL RQTIQSLRRQ
1561 FSGALPLAQQ EKFSKTMIQL QNDKLKIMQE MKNSQQEHRN MENKTLEMEL KLKGLEELIS
1621 TLKDTKGAQK VINWHMKIEE LRLQELKLNR ELVKDKEEIK YLNNIISEYE RTISSLEEEI
1681 VQQNKFHEER QMAWDQREVD LERQLDIFDR QQNEILNAAQ KFEEATGSIP DPSLPLPNQL
1741 EIALRKIKEN IRIILETRAT CKSLEEKLKE KESALRLAEQ NILSRDKVIN ELRLRLPATA
1801 EREKLIAELG RKEMEPKSHH TLKIAHQTIA NMQARLNQKE EVLKKYQRLL EKAREEQREI
1861 VKKHEEDLHI LHHRLELQAD SSLNKFKQTA WDLMKQSPTP VPTNKHFIRL AEMEQTVAEQ
1921 DDSLSSLLVK LKKVSQDLER QREITELKVK EFENIKLQLQ ENHEDEVKKV KAEVEDLKYL
1981 LDQSQKESQC LKSELQAQKE ANSRAPTTTM RNLVERLKSQ LALKEKQQKA LSRALLELRA
2041 EMTAAAEERI ISATSQKEAH LNVQQIVDRH TRELKTQVED LNENLLKLKE ALKTSKNREN
2101 SLTDNLNDLN NELQKKQKAY NKILREKEEI DQENDELKRQ IKRLTSGLQG KPLTDNKQSL
2161 IEELQRKVKK LENQLEGKVE EVDLKPMKEK NAKEELIRWE EGKKWQAKIE GIRNKLKEKE
2221 GEVFTLTKQL NTLKDLFAKA DKEKLTLQRK LKTTGMTVDQ VLGIRALESE KELEELKKRN
2281 LDLENDILYM RAHQALPRDS VVEDLHLQNR YLQEKLHALE KQFSKDTYSK PSQNQISGIE
2341 SDDHCQREQE LQKENLKLSS ENIELKFQLE QANKDLPRLK NQVRDLKEMC EFLKKEKAEV
2401 QRKLGHVRGS GRSGKTIPEL EKTIGLMKKV VEKVQRENEQ LKKASGILTS EKMANIEQEN
2461 EKLKAELEKL KAHLGHQLSM HYESKTKGTE KIIAENERLR KELKKETDAA EKLRIAKNNL
2521 EILNEKMTVQ LEETGKRLQF AESRGPQLEG ADSKSWKSIV VTRMYETKLK ELETDIAKKN
2581 QSITDLKQLV KEATEREQKV NKYNEDLEQQ IKILKHVPEG AETEQGLKRE LQVLRLANHQ
2641 LDKEKAELIH QIEANKDQSG AESTIPDADQ LKEKIKDLET QLKMSDLEKQ HLKEEIKKLK
2701 KELENFDPSF FEEIEDLKYN YKEEVKKNIL LEEKVKKLSE QLGVELTSPV AASEEFEDEE
2761 ESPVNFPIY
SEQ ID NO:64
   CENTROSOMAL PROTEIN 290 (CEP290), ISOFORM X2
   XP_011537060.1 
1 MPPNINWKEI MKVDPDDLPR QEELADNLLI SLSKVEVNEL KSEKQENVIH LFRITQSLMK
61 MKAQEVELAL EEVEKAGEEQ AKFENQLKTK VMKLENELEM AQQSAGGRDT RFLRNEICQL
121 EKQLEQKDRE LEDMEKELEK EKKVNEQLAL RNEEAENENS KLRRENKRLK KKNEQLCQDI
181 IDYQKQIDSQ KETLLSRRGE DSDYRSQLSK KNYELIQYLD EIQTLTEANE KIEVQNQEMR
241 KNLEESVQEM EKMTDEYNRM KAIVHQTDNV IDQLKKENDH YQLQVQELTD LLKSKNEEDD
301 PIMVAVNAKV EEWKLILSSK DDEIIEYQQM LHNLREKLKN AQLDADKSNV MALQQGIQER
361 DSQIKMLTEQ VEQYTKEMEK NTCIIEDLKN ELQRNKGAST LSQQTHMKIQ STLDILKEKT
421 KEAERTAELA EADAREKDKE LVEALKRLKD YESGVYGLED AVVEIKNCKN QIKIRDREIE
481 ILTKEINKLE LKISDFLDEN EALRERVGLE PKTMIDLTEF RNSKHLKQQQ YRAENQILLK
541 EIESLEEERL DLKKKIRQMA QERGKRSATS GLTTEDLNLT ENISQGDRIS ERKLDLLSLK
601 NMSEAQSKIR SSDKAELLHR RSSFNTPQSD QNETEENMTI GSLSRMLSEI HHSVESGMHP
661 FVPLTRLSSS MQVKENSTPE TITIREIFKA PCLQSSRNLE SLVSTFSRES HEEINDICLF
721 SDDCMKKVSR SHQALEKTSF VQKSNSSFHG LSTASDIMQK LSLRQKSAIF CQQIHENRAD
781 MDKSQVATLE EEQVHSQVKY ADINLKEDII KSEVPLQTEI LKNKLKVNLP DPVSITAQSK
841 LSQINSLENL IEQLRRELVF LRSQNEIIAQ EFLIKEAECR NADIELEHHR SQAEQNEFLS
901 RELIEKERDL ERSRTVIAKF QNKLKELVEE NKQLEEGMKE ILQAIKEMQK DPDVKGGETS
961 LIIPSLERLV NAIESKNAEG IFDASLHLKA QVDQLTGRNE ELRQELRESR KEAINYSQQL
1021 AKANLKIDHL EKETSLLRQS EGSNVVFKGI DLPDGIAPSS ASIINSQNEY LIHLLQELEN
1081 KEKKLKNLED SLEDYNRKFA VIRHQQSLLY KEYLSEKETW KTESKTIKEE KRKLEDQVQQ
1141 DAIKVKEYNN LLNALQMDSD EMKKILAENS RKITVLQVNE KSLIRQYTTL VELERQLRKE
1201 NEKQKNELLS MEAEVCEKIG CLQRFKEMAI FKIAALQKVV DNSVSLSELE LANKQYNELT
1261 AKYRDILQKD NMLVQRTSNL EHLECENISL KEQVESINKE LEITKEKLHT IEQAWEQETK
1321 LGNESSMDKA KKSITNSDIV SISKKITMLE MKELNERQRA EHCQKMYEHL RTSLKQMEER
1381 NFELETKFAE LTKINLDAQK VEQMLRDELA DSVSKAVSDA DRQRILELEK NEMELKVEVS
1441 KLREISDIAR RQVEILNAQQ QSRDKEVESL RMQLLDYQAQ SDEKSLIAKL HQHNVSLQLS
1501 EATALGKLES ITSKLQKMEA YNLRLEQKLD EKEQALYYAR LEGRNRAKHL RQTIQSLRRQ
1561 FSGALPLAQQ EKFSKTMIQL QNDKLKIMQE MKNSQQEHRN MENKTLEMEL KLKGLEELIS
1621 TLKDTKGAQK VINWHMKIEE LRLQELKLNR ELVKDKEEIK YLNNIISEYE RTISSLEEEI
1681 VQQNKFHEER QMAWDQREVD LERQLDIFDR QQNEILNAAQ KFEEATGSIP DPSLPLPNQL
1741 EIALRKIKEN IRIILETRAT CKSLEEKLKE KESALRLAEQ NILSRDKVIN ELRLRLPATA
1801 EREKLIAELG RKEMEPKSHH TLKIAHQTIA NMQARLNQKE EVLKKYQRLL EKAREEQREI
1861 VKKHEEDLHI LHHRLELQAD SSLNKFKQTA WDLMKQSPTP VPTNKHFIRL AEMEQTVAEQ
1921 DDSLSSLLVK LKKVSQDLER QREITELKVK EFENIKLQLQ ENHEDEVKKV KAEVEDLKYL
1981 LDQSQKESQC LKSELQAQKE ANSRAPTTTM RNLVERLKSQ LALKEKQQKA LSRALLELRA
2041 EMTAAAEERI ISATSQKEAH LNVQQIVDRH TRELKTQVED LNENLLKLKE ALKTSKNREN
2101 SLTDNLNDLN NELQKKQKAY NKILREKEEI DQENDELKRQ IKRLTSGLQG KPLTDNKQSL
2161 IEELQRKVKK LENQLEGKVE EVDLKPMKEK NAKEELIRWE EGKKWQAKIE GIRNKLKEKE
2221 GEVFTLTKQL NTLKDLFAKA DKEKLTLQRK LKTTGMTVDQ VLGIRALESE KELEELKKRN
2281 LDLENDILYM RAHQALPRDS VVEDLHLQNR YLQEKLHALE KQFSKDTYSK PSNQISGIES
2341 DDHCQREQEL QKENLKLSSE NIELKFQLEQ ANKDLPRLKN QVRDLKEMCE FLKKEKAEVQ
2401 RKLGHVRGSG RSGKTIPELE KTIGLMKKVV EKVQRENEQL KKASGILTSE KMANIEQENE
2461 KLKAELEKLK AHLGHQLSMH YESKTKGTEK IIAENERLRK ELKKETDAAE KLRIAKNNLE
2521 ILNEKMTVQL EETGKRLQFA ESRGPQLEGA DSKSWKSIVV TRMYETKLKE LETDIAKKNQ
2581 SITDLKQLVK EATEREQKVN KYNEDLEQQI KILKHVPEGA ETEQGLKREL QVLRLANHQL
2641 DKEKAELIHQ IEANKDQSGA ESTIPDADQL KEKIKDLETQ LKMSDLEKQH LKEEIKKLKK
2701 ELENFDPSFF EEIEDLKYNY KEEVKKNILL EEKVKKLSEQ LGVELTSPVA ASEEFEDEEE
2761 SPVNFPIY
SEQ ID NO:65
   CENTROSOMAL PROTEIN 290 (CEP290), ISOFORM X3
   XP_011537061.1 
1 MPPNINWKEI MKVDPDDLPR QEELADNLLI SLSKVEVNEL KSEKQENVIH LFRITQSLMK
61 MKAQEVELAL EEVEKAGEEQ AKFENQLKTK VMKLENELEM AQQSAGGRDT RFLRNEICQL
 
121 EKQLEQKDRE LEDMEKELEK EKKVNEQLAL RNEEAENENS KLRRENKRLK KKNEQLCQDI
181 IDYQKQIDSQ KETLLSRRGE DSDYRSQLSK KNYELIQYLD EIQTLTEANE KIEVQNQEMR
241 KNLEESVQEM EKMTDEYNRM KAIVHQTDNV IDQLKKENDH YQLQVQELTD LLKSKNEEDD
301 PIMVAVNAKV EEWKLILSSK DDEIIEYQQM LHNLREKLKN AQLDADKSNV MALQQGIQER
361 DSQIKMLTEQ VEQYTKEMEK NTCIIEDLKN ELQRNKGAST LSQQTHMKIQ STLDILKEKT
421 KEAERTAELA EADAREKDKE LVEALKRLKD YESGVYGLED AVVEIKNCKN QIKIRDREIE
481 ILTKEINKLE LKISDFLDEN EALRERVGLE PKTMIDLTEF RNSKHLKQQQ YRAENQILLK
541 EIESLEEERL DLKKKIRQMA QERGKRSATS GLTTEDLNLT ENISQGDRIS ERKLDLLSLK
601 NMSEAQSKIR SSDKAELLHR RSSFNTPQSD QNETEENMTI GSLSRMLSEI HHSVESGMHP
661 FVPLTRLSSS MQVKENSTPE TITIREIFKA PCLQSSRNLE SLVSTFSRES HEEINDICLF
721 SDDCMKKVSR SHQALEKTSF VQKSNSSFHG LSTASDIMQK LSLRQKSAIF CQQIHENRAD
781 MDKSQVATLE EEQVHSQVKY ADINLKEDII KSEVPLQTEI LKNKLKVNLP DPVSITAQSK
841 LSQINSLENL IEQLRRELVF LRSQNEIIAQ EFLIKEAECR NADIELEHHR SQAEQNEFLS
901 RELIEKERDL ERSRTVIAKF QNKLKELVEE NKQLEEGMKE ILQAIKEMQK DPDVKGGETS
961 LIIPSLERLV NAIESKNAEG IFDASLHLKA QVDQLTGRNE ELRQELRESR KEAINYSQQL
1021 AKANLKIDHL EKETSLLRQS EGSNVVFKGI DLPDGIAPSS ASIINSQNEY LIHLLQELEN
1081 KEKKLKNLED SLEDYNRKFA VIRHQQSLLY KEYLSEKETW KTESKTIKEE KRKLEDQVQQ
1141 DAIKVKEYNN LLNALQMDSD EMKKILAENS RKITVLQVNE KSLIRQYTTL VELERQLRKE
1201 NEKQKNELLS MEAEVCEKIG CLQRFKEMAI FKIAALQKVV DNSVSLSELE LANKQYNELT
1261 AKYRDILQKD NMLVQRTSNL EHLECENISL KEQVESINKE LEITKEKLHT IEQAWEQETK
1321 LGNESSMDKA KKSITNSDIV SISKKITMLE MKELNERQRA EHCQKMYEHL RTSLKQMEER
1381 NFELETKFAE LTKINLDAQK VEQMLRDELA DSVSKAVSDA DRQRILELEK NEMELKVEVS
1441 KLREISDIAR RQVEILNAQQ QSRDKEVESL RMQLLDYQAQ SDEKSLIAKL HQHNVSLQLS
1501 EATALGKLES ITSKLQKMEA YNLRLEQKLD EKEQALYYAR LEGRNRAKHL RQTIQSLRRQ
1561 FSGALPLAQQ EKFSKTMIQL QNDKLKIMQE MKNSQQEHRN MENKTLEMEL KLKGLEELIS
1621 TLKDTKGAQK VINWHMKIEE LRLQELKLNR ELVKDKEEIK YLNNIISEYE RTISSLEEEI
1681 VQQNKFHEER QMAWDQREVD LERQLDIFDR QQNEILNAAQ KFEEATGSIP DPSLPLPNQL
1741 EIALRKIKEN IRIILETRAT CKSLEEKLKE KESALRLAEQ NILSRDKVIN ELRLRLPATA
1801 EREKLIAELG RKEMEPKSHH TLKIAHQTIA NMQARLNQKE EVLKKYQRLL EKAREEQREI
1861 VKKHEEDLHI LHHRLELQAD SSLNKFKQTA WDLMKQSPTP VPTNKHFIRL AEMEQTVAEQ
1921 DDSLSSLLVK LKKVSQDLER QREITELKVK EFENIKLQLQ ENHEDEVKKV KAEVEDLKYL
1981 LDQSQKESQC LKSELQAQKE ANSRAPTTTM RNLVERLKSQ LALKEKQQKA LSRALLELRA
2041 EMTAAAEERI ISATSQKEAH LNVQQIVDRH TRELKTQVED LNENLLKLKE ALKTSKNREN
2101 SLTDNLNDLN NELQKKQKAY NKILREKEEI DQENDELKRQ IKRLTSGLQG KPLTDNKQSL
2161 IEELQRKVKK LENQLEGKVE EVDLKPMKEK NAKEELIRWE EGKKWQAKIE GIRNKLKEKE
2221 GEVFTLTKQL NTLKDLFAKA DKEKLTLQRK LKTTGMTVDQ VLGIRALESE KELEELKKRN
2281 LDLENDILYM RAHQALPRDS VVEDLHLQNR YLQEKLHALE KQFSKDTYSK PSISGIESDD
2341 HCQREQELQK ENLKLSSENI ELKFQLEQAN KDLPRLKNQV RDLKEMCEFL KKEKAEVQRK
2401 LGHVRGSGRS GKTIPELEKT IGLMKKVVEK VQRENEQLKK ASGILTSEKM ANIEQENEKL
2461 KAELEKLKAH LGHQLSMHYE SKTKGTEKII AENERLRKEL KKETDAAEKL RIAKNNLEIL
2521 NEKMTVQLEE TGKRLQFAES RGPQLEGADS KSWKSIVVTR MYETKLKELE TDIAKKNQSI
2581 TDLKQLVKEA TEREQKVNKY NEDLEQQIKI LKHVPEGAET EQGLKRELQV LRLANHQLDK
2641 EKAELIHQIE ANKDQSGAES TIPDADQLKE KIKDLETQLK MSDLEKQHLK EEIKKLKKEL
2701 ENFDPSFFEE IEDLKYNYKE EVKKNILLEE KVKKLSEQLG VELTSPVAAS EEFEDEEESP
2761 VNFPIY
SEQ ID NO:66
   CENTROSOMAL PROTEIN 290 (CEP290), ISOFORM X4
   XP_011537062.1 
1 MPPNINWKEI MKVDPDDLPR QEELADNLLI SLSKVEVNEL KSEKQENVIH LFRITQSLMK
61 MKAQEVELAL EEVEKAGEEQ AKFENQLKTK VMKLENELEM AQQSAGGRDT RFLRNEICQL
121 EKQLEQKDRE LEDMEKELEK EKKVNEQLAL RNEEAENENS KLRRENKRLK KKNEQLCQDI
181 IDYQKQIDSQ KETLLSRRGE DSDYRSQLSK KNYELIQYLD EIQTLTEANE KIEVQNQEMR
241 KNLEESVQEM EKMTDEYNRM KAIVHQTDNV IDQLKKENDH YQLQVQELTD LLKSKNEEDD
301 PIMVAVNAKV EEWKLILSSK DDEIIEYQQM LHNLREKLKN AQLDADKSNV MALQQGIQER
361 DSQIKMLTEQ VEQYTKEMEK NTCIIEDLKN ELQRNKGAST LSQQTHMKIQ STLDILKEKT
421 KEAERTAELA EADAREKDKE LVEALKRLKD YESGVYGLED AVVEIKNCKN QIKIRDREIE
 
481 ILTKEINKLE LKISDFLDEN EALRERVGLE PKTMIDLTEF RNSKHLKQQQ YRAENQILLK
541 EIESLEEERL DLKKKIRQMA QERGKRSATS GLTTEDLNLT ENISQGDRIS ERKLDLLSLK
601 NMSEAQSKIR SSDKAELLHR RSSFNTPQSD QNETEENMTI GSLSRMLSEI HHSVESGMHP
661 FVPLTRLSSS MQVKENSTPE TITIREIFKA PCLQSSRNLE SLVSTFSRES HEEINDICLF
721 SDDCMKKVSR SHQALEKTSF VQKSNSSFHG LSTASDIMQK LSLRQKSAIF CQQIHENRAD
781 MDKSQVATLE EEQVHSQVKY ADINLKEDII KSEVPLQTEI LKNKLKVNLP DPVSITAQSK
841 LSQINSLENL IEQLRRELVF LRSQNEIIAQ EFLIKEAECR NADIELEHHR SQAEQNEFLS
901 RELIEKERDL ERSRTVIAKF QNKLKELVEE NKQLEEGMKE ILQAIKEMQK DPDVKGGETS
961 LIIPSLERLV NAIESKNAEG IFDASLHLKA QVDQLTGRNE ELRQELRESR KEAINYSQQL
1021 AKANLKIDHL EKETSLLRQS EGSNVVFKGI DLPDGIAPSS ASIINSQNEY LIHLLQELEN
1081 KEKKLKNLED SLEDYNRKFA VIRHQQSLLY KEYLSEKETW KTESKTIKEE KRKLEDQVQQ
1141 DAIKVKEYNN LLNALQMDSD EMKKILAENS RKITVLQVNE KSLIRQYTTL VELERQLRKE
1201 NEKQKNELLS MEAEVCEKIG CLQRFKEMAI FKIAALQKVV DNSVSLSELE LANKQYNELT
1261 AKYRDILQKD NMLVQRTSNL EHLECENISL KEQVESINKE LEITKEKLHT IEQAWEQETK
1321 LGNESSMDKA KKSITNSDIV SISKKITMLE MKELNERQRA EHCQKMYEHL RTSLKQMEER
1381 NFELETKFAE LTKINLDAQK VEQMLRDELA DSVSKAVSDA DRQRILELEK NEMELKVEVS
1441 KLREISDIAR RQVEILNAQQ QSRDKEVESL RMQLLDYQAQ SDEKSLIAKL HQHNVSLQLS
1501 EATALGKLES ITSKLQKMEA YNLRLEQKLD EKEQALYYAR LEGRNRAKHL RQTIQSLRRQ
1561 FSGALPLAQQ EKFSKTMIQL QNDKLKIMQE MKNSQQEHRN MENKTLEMEL KLKGLEELIS
1621 TLKDTKGAQK VINWHMKIEE LRLQELKLNR ELVKDKEEIK YLNNIISEYE RTISSLEEEI
1681 VQQNKFHEER QMAWDQREVD LERQLDIFDR QQNEILNAAQ KFEEATGSIP DPSLPLPNQL
1741 EIALRKIKEN IRIILETRAT CKSLEEKLKE KESALRLAEQ NILSRDKVIN ELRLRLPATA
1801 EREKLIAELG RKEMEPKSHH TLKIAHQTIA NMQARLNQKE EVLKKYQRLL EKAREEQREI
1861 VKKHEEDLHI LHHRLELQAD SSLNKFKQTA WDLMKQSPTP VPTNKHFIRL AEMEQTVAEQ
1921 DDSLSSLLVK LKKVSQDLER QREITELKVK EFENIKLQLQ ENHEDEVKKV KAEVEDLKYL
1981 LDQSQKESQC LKSELQAQKE ANSRAPTTTM RNLVERLKSQ LALKEKQQKA LSRALLELRA
2041 EMTAAAEERI ISATSQKEAH LNVQQIVDRH TRELKTQVED LNENLLKLKE ALKTSKNREN
2101 SLTDNLNDLN NELQKKQKAY NKILREKEEI DQENDELKRQ IKRLTSGLQG KPLTDNKQSL
2161 IEELQRKVKK LENQLEGKVE EVDLKPMKEK NAKEELIRWE EGKKWQAKIE GIRNKLKEKE
2221 GEVFTLTKQL NTLKDLFAKA DKEKLTLQRK LKTTGMTVDQ VLGIRALESE KELEELKKRN
2281 LDLENDILYM RAHQALPRDS VVEDLHLQNR YLQEKLHALE KQFSKDTYSK PSQNQISGIE
2341 SDDHCQREQE LQKENLKLSS ENIELKFQLE QANKDLPRLK NQVRDLKEMC EFLKKEKAEV
2401 QRKLGHVRGS GRSGKTIPEL EKTIGLMKKV VEKVQRENEQ LKKASGILTS EKMANIEQEN
2461 EKLKETDAAE KLRIAKNNLE ILNEKMTVQL EETGKRLQFA ESRGPQLEGA DSKSWKSIVV
2521 TRMYETKLKE LETDIAKKNQ SITDLKQLVK EATEREQKVN KYNEDLEQQI KILKHVPEGA
2581 ETEQGLKREL QVLRLANHQL DKEKAELIHQ IEANKDQSGA ESTIPDADQL KEKIKDLETQ
2641 LKMSDLEKQH LKEEIKKLKK ELENFDPSFF EEIEDLKYNY KEEVKKNILL EEKVKKLSEQ
2701 LGVELTSPVA ASEEFEDEEE SPVNFPIY
SEQ ID NO:67
   CENTROSOMAL PROTEIN 290 (CEP290), ISOFORM X5
   XP_016875469.1 
1 MPPNINWKEI MKVDPDDLPR QEELADNLLI SLSKVEVNEL KSEKQENVIH LFRITQSLMK
61 MKAQEVELAL EEVEKAGEEQ AKFENQLKTK VMKLENELEM AQQSAGGRDT RFLRNEICQL
121 EKQLEQKDRE LEDMEKELEK EKKVNEQLAL RNEEAENENS KLRRENKRLK KKNEQLCQDI
181 IDYQKQIDSQ KETLLSRRGE DSDYRSQLSK KNYELIQYLD EIQTLTEANE KIEVQNQEMR
241 KNLEESVQEM EKMTDEYNRM KAIVHQTDNV IDQLKKENDH YQLQVQELTD LLKSKNEEDD
301 PIMVAVNAKV EEWKLILSSK DDEIIEYQQM LHNLREKLKN AQLDADKSNV MALQQGIQER
361 DSQIKMLTEQ VEQYTKEMEK NTCIIEDLKN ELQRNKGAST LSQQTHMKIQ STLDILKEKT
421 KEAERTAELA EADAREKDKE LVEALKRLKD YESGVYGLED AVVEIKNCKN QIKIRDREIE
481 ILTKEINKLE LKISDFLDEN EALRERVGLE PKTMIDLTEF RNSKHLKQQQ YRAENQILLK
541 EIESLEEERL DLKKKIRQMA QERGKRSATS GLTTEDLNLT ENISQGDRIS ERKLDLLSLK
601 NMSEAQSKIR SSDKAELLHR RSSFNTPQSD QNETEENMTI GSLSRMLSEI HHSVESGMHP
661 FVPLTRLSSS MQVKENSTPE TITIREIFKA PCLQSSRNLE SLVSTFSRES HEEINDICLF
721 SDDCMKKVSR SHQALEKTSF VQKSNSSFHG LSTASDIMQK LSLRQKSAIF CQQIHENRAD
781 MDKSQVATLE EEQVHSQVKY ADINLKEDII KSEVPLQTEI LKNKLKVNLP DPVSITAQSK
841 LSQINSLENL IEQLRRELVF LRSQNEIIAQ EFLIKEAECR NADIELEHHR SQAEQNEFLS
 
901 RELIEKERDL ERSRTVIAKF QNKLKELVEE NKQLEEGMKE ILQAIKEMQK DPDVKGGETS
961 LIIPSLERLV NAIESKNAEG IFDASLHLKA QVDQLTGRNE ELRQELRESR KEAINYSQQL
1021 AKANLKIDHL EKETSLLRQS EGSNVVFKGI DLPDGIAPSS ASIINSQNEY LIHLLQELEN
1081 KEKKLKNLED SLEDYNRKFA VIRHQQSLLY KEYLSEKETW KTESKTIKEE KRKLEDQVQQ
1141 DAIKVKEYNN LLNALQMDSD EMKKILAENS RKITVLQVNE KSLIRQYTTL VELERQLRKE
1201 NEKQKNELLS MEAEVCEKIG CLQRFKEMAI FKIAALQKVV DNSVSLSELE LANKQYNELT
1261 AKYRDILQKD NMLVQRTSNL EHLECENISL KEQVESINKE LEITKEKLHT IEQAWEQETK
1321 LGNESSMDKA KKSITNSDIV SISKKITMLE MKELNERQRA EHCQKMYEHL RTSLKQMEER
1381 NFELETKFAE LTKINLDAQK VEQMLRDELA DSVSKAVSDA DRQRILELEK NEMELKVEVS
1441 KLREISDIAR RQVEILNAQQ QSRDKEVESL RMQLLDYQAQ SDEKSLIAKL HQHNVSLQLS
1501 EATALGKLES ITSKLQKMEA YNLRLEQKLD EKEQALYYAR LEGRNRAKHL RQTIQSLRRQ
1561 FSGALPLAQQ EKFSKTMIQL QNDKLKIMQE MKNSQQEHRN MENKTLEMEL KLKGLEELIS
1621 TLKDTKGAQK VINWHMKIEE LRLQELKLNR ELVKDKEEIK YLNNIISEYE RTISSLEEEI
1681 VQQNKFHEER QMAWDQREVD LERQLDIFDR QQNEILNAAQ KFEEATGSIP DPSLPLPNQL
1741 EIALRKIKEN IRIILETRAT CKSLEEKLKE KESALRLAEQ NILSRDKVIN ELRLRLPATA
1801 EREKLIAELG RKEMEPKSHH TLKIAHQTIA NMQARLNQKE EVLKKYQRLL EKAREEQREI
1861 VKKHEEDLHI LHHRLELQAD SSLNKFKQTA WDLMKQSPTP VPTNKHFIRL AEMEQTVAEQ
1921 DDSLSSLLVK LKKVSQDLER QREITELKVK EFENIKLQLQ ENHEDEVKKV KAEVEDLKYL
1981 LDQSQKESQC LKSELQAQKE ANSRAPTTTM RNLVERLKSQ LALKEKQQKA LSRALLELRA
2041 EMTAAAEERI ISATSQKEAH LNVQQIVDRH TRELKTQVED LNENLLKLKE ALKTSKNREN
2101 SLTDNLNDLN NELQKKQKAY NKILREKEEI DQENDELKRQ IKRLTSGLQG KPLTDNKQSL
2161 IEELQRKVKK LENQLEGKVE EVDLKPMKEK NAKEELIRWE EGKKWQAKIE GIRNKLKEKE
2221 GEVFTLTKQL NTLKDLFAKA DKEKLTLQRK LKTTGMTVDQ VLGIRALESE KELEELKKRN
2281 LDLENDILYM RAHQALPRDS VVEDLHLQNR YLQEKLHALE KQFSKDTYSK PSISGIESDD
2341 HCQREQELQK ENLKLSSENI ELKFQLEQAN KDLPRLKNQV RDLKEMCEFL KKEKAEVQRK
2401 LGHVRGSGRS GKTIPELEKT IGLMKKVVEK VQRENEQLKK ASGILTSEKM ANIEQENEKL
2461 KETDAAEKLR IAKNNLEILN EKMTVQLEET GKRLQFAESR GPQLEGADSK SWKSIVVTRM
2521 YETKLKELET DIAKKNQSIT DLKQLVKEAT EREQKVNKYN EDLEQQIKIL KHVPEGAETE
2581 QGLKRELQVL RLANHQLDKE KAELIHQIEA NKDQSGAEST IPDADQLKEK IKDLETQLKM
2641 SDLEKQHLKE EIKKLKKELE NFDPSFFEEI EDLKYNYKEE VKKNILLEEK VKKLSEQLGV
2701 ELTSPVAASE EFEDEEESPV NFPIY
SEQ ID NO:68
   CENTROSOMAL PROTEIN 290 (CEP290), ISOFORM X6
   XP_011537063.1 
1 MPPNINWKEI MKVDPDDLPR QEELADNLLI SLSKVEVNEL KSEKQENVIH LFRITQSLMK
61 MKAQEVELAL EEVEKAGEEQ AKFENQLKTK VMKLENELEM AQQSAGGRDT RFLRNEICQL
121 EKQLEQKDRE LEDMEKELEK EKKVNEQLAL RNEEAENENS KLRRENKRLK KKNEQLCQDI
181 IDYQKQIDSQ KETLLSRRGE DSDYRSQLSK KNYELIQYLD EIQTLTEANE KIEVQNQEMR
241 KNLEESVQEM EKMTDEYNRM KAIVHQTDNV IDQLKKENDH YQLQVQELTD LLKSKNEEDD
301 PIMVAVNAKV EEWKLILSSK DDEIIEYQQM LHNLREKLKN AQLDADKSNV MALQQGIQER
361 DSQIKMLTEQ VEQYTKEMEK NTCIIEDLKN ELQRNKGAST LSQQTHMKIQ STLDILKEKT
421 KEAERTAELA EADAREKDKE LVEALKRLKD YESGVYGLED AVVEIKNCKN QIKIRDREIE
481 ILTKEINKLE LKISDFLDEN EALRERVGLE PKTMIDLTEF RNSKHLKQQQ YRAENQILLK
541 EIESLEEERL DLKKKIRQMA QERGKRSATS GLTTEDLNLT ENISQGDRIS ERKLDLLSLK
601 NMSEAQSKIR SSDKAELLHR RSSFNTPQSD QNETEENMTI GSLSRMLSEI HHSVESGMHP
661 FVPLTRLSSS MQVKENSTPE TITIREIFKA PCLQSSRNLE SLVSTFSRES HEEINDICLF
721 SDDCMKKVSR SHQALEKTSF VQKSNSSFHG LSTASDIMQK LSLRQKSAIF CQQIHENRAD
781 MDKSQVATLE EEQVHSQVKY ADINLKEDII KSEVPLQTEI LKNKLKVNLP DPVSITAQSK
841 LSQINSLENL IEQLRRELVF LRSQNEIIAQ EFLIKEAECR NADIELEHHR SQAEQNEFLS
901 RELIEKERDL ERSRTVIAKF QNKLKELVEE NKQLEEGMKE ILQAIKEMQK DPDVKGGETS
961 LIIPSLERLV NAIESKNAEG IFDASLHLKA QVDQLTGRNE ELRQELRESR KEAINYSQQL
1021 AKANLKIDHL EKETSLLRQS EGSNVVFKGI DLPDGIAPSS ASIINSQNEY LIHLLQELEN
1081 KEKKLKNLED SLEDYNRKFA VIRHQQSLLY KEYLSEKETW KTESKTIKEE KRKLEDQVQQ
1141 DAIKVKEYNN LLNALQMDSD EMKKILAENS RKITVLQVNE KSLIRQYTTL VELERQLRKE
1201 NEKQKNELLS MEAEVCEKIG CLQRFKEMAI FKIAALQKVV DNSVSLSELE LANKQYNELT
1261 AKYRDILQKD NMLVQRTSNL EHLECENISL KEQVESINKE LEITKEKLHT IEQAWEQETK
 
1321 LGNESSMDKA KKSITNSDIV SISKKITMLE MKELNERQRA EHCQKMYEHL RTSLKQMEER
1381 NFELETKFAE LTKINLDAQK VEQMLRDELA DSVSKAVSDA DRQRILELEK NEMELKVEVS
1441 KLREISDIAR RQVEILNAQQ QSRDKEVESL RMQLLDYQAQ SDEKSLIAKL HQHNVSLQLS
1501 EATALGKLES ITSKLQKMEA YNLRLEQKLD EKEQALYYAR LEGRNRAKHL RQTIQSLRRQ
1561 FSGALPLAQQ EKFSKTMIQL QNDKLKIMQE MKNSQQEHRN MENKTLEMEL KLKGLEELIS
1621 TLKDTKGAQK VINWHMKIEE LRLQELKLNR ELVKDKEEIK YLNNIISEYE RTISSLEEEI
1681 VQQNKFHEER QMAWDQREVD LERQLDIFDR QQNEILNAAQ KFEEATGSIP DPSLPLPNQL
1741 EIALRKIKEN IRIILETRAT CKSLEEKLKE KESALRLAEQ NILSRDKVIN ELRLRLPATA
1801 EREKLIAELG RKEMEPKSHH TLKIAHQTIA NMQARLNQKE EVLKKYQRLL EKAREEQREI
1861 VKKHEEDLHI LHHRLELQAD SSLNKFKQTA WDLMKQSPTP VPTNKHFIRL AEMEQTVAEQ
1921 DDSLSSLLVK LKKVSQDLER QREITELKVK EFENIKLQLQ ENHEDEVKKV KAEVEDLKYL
1981 LDQSQKESQC LKSELQAQKE ANSRAPTTTM RNLVERLKSQ LALKEKQQKA LSRALLELRA
2041 EMTAAAEERI ISATSQKEAH LNVQQIVDRH TRELKTQVED LNENLLKLKE ALKTSKNREN
2101 SLTDNLNDLN NELQKKQKAY NKILREKEEI DQENDELKRQ IKRLTSGLQG KPLTDNKQSL
2161 IEELQRKVKK LENQLEGKVE EVDLKPMKEK NAKEELIRWE EGKKWQAKIE GIRNKLKEKE
2221 GEVFTLTKQL NTLKDLFAKA DKEKLTLQRK LKTTGMTVDQ VLGIRALESE KELEELKKRN
2281 LDLENDILYM RAHQALPRDS VVEDLHLQNR YLQEKLHALE KQFSKDTYSK PSQNQISGIE
2341 SDDHCQREQE LQKENLKLSS ENIELKFQLE QANKDLPRLK NQVRDLKEMC EFLKKEKAEV
2401 QRKLGHVRGA ELEKLKAHLG HQLSMHYESK TKGTEKIIAE NERLRKELKK ETDAAEKLRI
2461 AKNNLEILNE KMTVQLEETG KRLQFAESRG PQLEGADSKS WKSIVVTRMY ETKLKELETD
2521 IAKKNQSITD LKQLVKEATE REQKVNKYNE DLEQQIKILK HVPEGAETEQ GLKRELQVLR
2581 LANHQLDKEK AELIHQIEAN KDQSGAESTI PDADQLKEKI KDLETQLKMS DLEKQHLKEE
2641 IKKLKKELEN FDPSFFEEIE DLKYNYKEEV KKNILLEEKV KKLSEQLGVE LTSPVAASEE
2701 FEDEEESPVN FPIY
SEQ ID NO:69
   CENTROSOMAL PROTEIN 290 (CEP290), ISOFORM X7
   XP_016875470.1 
1 MPPNINWKEI MKVDPDDLPR QEELADNLLI SLSKVEVNEL KSEKQENVIH LFRITQSLMK
61 MKAQEVELAL EEVEKAGEEQ AKFENQLKTK VMKLENELEM AQQSAGGRDT RFLRNEICQL
121 EKQLEQKDRE LEDMEKELEK EKKVNEQLAL RNEEAENENS KLRRENKRLK KKNEQLCQDI
181 IDYQKQIDSQ KETLLSRRGE DSDYRSQLSK KNYELIQYLD EIQTLTEANE KIEVQNQEMR
241 KNLEESVQEM EKMTDEYNRM KAIVHQTDNV IDQLKKENDH YQLQVQELTD LLKSKNEEDD
301 PIMVAVNAKV EEWKLILSSK DDEIIEYQQM LHNLREKLKN AQLDADKSNV MALQQGIQER
361 DSQIKMLTEQ VEQYTKEMEK NTCIIEDLKN ELQRNKGAST LSQQTHMKIQ STLDILKEKT
421 KEAERTAELA EADAREKDKE LVEALKRLKD YESGVYGLED AVVEIKNCKN QIKIRDREIE
481 ILTKEINKLE LKISDFLDEN EALRERVGLE PKTMIDLTEF RNSKHLKQQQ YRAENQILLK
541 EIESLEEERL DLKKKIRQMA QERGKRSATS GLTTEDLNLT ENISQGDRIS ERKLDLLSLK
601 NMSEAQSKIR SSDKAELLHR RSSFNTPQSD QNETEENMTI GSLSRMLSEI HHSVESGMHP
661 FVPLTRLSSS MQVKENSTPE TITIREIFKA PCLQSSRNLE SLVSTFSRES HEEINDICLF
721 SDDCMKKVSR SHQALEKTSF VQKSNSSFHG LSTASDIMQK LSLRQKSAIF CQQIHENRAD
781 MDKSQVATLE EEQVHSQVKY ADINLKEDII KSEVPLQTEI LKNKLKVNLP DPVSITAQSK
841 LSQINSLENL IEQLRRELVF LRSQNEIIAQ EFLIKEAECR NADIELEHHR SQAEQNEFLS
901 RELIEKERDL ERSRTVIAKF QNKLKELVEE NKQLEEGMKE ILQAIKEMQK DPDVKGGETS
961 LIIPSLERLV NAIESKNAEG IFDASLHLKA QVDQLTGRNE ELRQELRESR KEAINYSQQL
1021 AKANLKIDHL EKETSLLRQS EGSNVVFKGI DLPDGIAPSS ASIINSQNEY LIHLLQELEN
1081 KEKKLKNLED SLEDYNRKFA VIRHQQSLLY KEYLSEKETW KTESKTIKEE KRKLEDQVQQ
1141 DAIKVKEYNN LLNALQMDSD EMKKILAENS RKITVLQVNE KSLIRQYTTL VELERQLRKE
1201 NEKQKNELLS MEAEVCEKIG CLQRFKEMAI FKIAALQKVV DNSVSLSELE LANKQYNELT
1261 AKYRDILQKD NMLVQRTSNL EHLECENISL KEQVESINKE LEITKEKLHT IEQAWEQETK
1321 LGNESSMDKA KKSITNSDIV SISKKITMLE MKELNERQRA EHCQKMYEHL RTSLKQMEER
1381 NFELETKFAE LTKINLDAQK VEQMLRDELA DSVSKAVSDA DRQRILELEK NEMELKVEVS
1441 KLREISDIAR RQVEILNAQQ QSRDKEVESL RMQLLDYQAQ SDEKSLIAKL HQHNVSLQLS
1501 EATALGKLES ITSKLQKMEA YNLRLEQKLD EKEQALYYAR LEGRNRAKHL RQTIQSLRRQ
1561 FSGALPLAQQ EKFSKTMIQL QNDKLKIMQE MKNSQQEHRN MENKTLEMEL KLKGLEELIS
1621 TLKDTKGAQK VINWHMKIEE LRLQELKLNR ELVKDKEEIK YLNNIISEYE RTISSLEEEI
1681 VQQNKFHEER QMAWDQREVD LERQLDIFDR QQNEILNAAQ KFEEATGSIP DPSLPLPNQL
 
1741 EIALRKIKEN IRIILETRAT CKSLEEKLKE KESALRLAEQ NILSRDKVIN ELRLRLPATA
1801 EREKLIAELG RKEMEPKSHH TLKIAHQTIA NMQARLNQKE EVLKKYQRLL EKAREEQREI
1861 VKKHEEDLHI LHHRLELQAD SSLNKFKQTA WDLMKQSPTP VPTNKHFIRL AEMEQTVAEQ
1921 DDSLSSLLVK LKKVSQDLER QREITELKVK EFENIKLQLQ ENHEDEVKKV KAEVEDLKYL
1981 LDQSQKESQC LKSELQAQKE ANSRAPTTTM RNLVERLKSQ LALKEKQQKA LSRALLELRA
2041 EMTAAAEERI ISATSQKEAH LNVQQIVDRH TRELKTQVED LNENLLKLKE ALKTSKNREN
2101 SLTDNLNDLN NELQKKQKAY NKILREKEEI DQENDELKRQ IKRLTSGLQG KPLTDNKQSL
2161 IEELQRKVKK LENQLEGKVE EVDLKPMKEK NAKEELIRWE EGKKWQAKIE GIRNKLKEKE
2221 GEVFTLTKQL NTLKDLFAKA DKEKLTLQRK LKTTGMTVDQ VLGIRALESE KELEELKKRN
2281 LDLENDILYM RAHQALPRDS VVEDLHLQNR YLQEKLHALE KQFSKDTYSK PSISGIESDD
2341 HCQREQELQK ENLKLSSENI ELKFQLEQAN KDLPRLKNQV RDLKEMCEFL KKEKAEVQRK
2401 LGHVRGAELE KLKAHLGHQL SMHYESKTKG TEKIIAENER LRKELKKETD AAEKLRIAKN
2461 NLEILNEKMT VQLEETGKRL QFAESRGPQL EGADSKSWKS IVVTRMYETK LKELETDIAK
2521 KNQSITDLKQ LVKEATEREQ KVNKYNEDLE QQIKILKHVP EGAETEQGLK RELQVLRLAN
2581 HQLDKEKAEL IHQIEANKDQ SGAESTIPDA DQLKEKIKDL ETQLKMSDLE KQHLKEEIKK
2641 LKKELENFDP SFFEEIEDLK YNYKEEVKKN ILLEEKVKKL SEQLGVELTS PVAASEEFED
2701 EEESPVNFPI Y
SEQ ID NO:70
   CENTROSOMAL PROTEIN 290 (CEP290), ISOFORM X8
   XP_016875471.1 
1 MPPNINWKEI MKVDPDDLPR QEELADNLLI SLSKVEVNEL KSEKQENVIH LFRITQSLMK
61 MKAQEVELAL EEVEKAGEEQ AKFENQLKTK VMKLENELEM AQQSAGGRDT RFLRNEICQL
121 EKQLEQKDRE LEDMEKELEK EKKVNEQLAL RNEEAENENS KLRRENKRLK KKNEQLCQDI
181 IDYQKQIDSQ KETLLSRRGE DSDYRSQLSK KNYELIQYLD EIQTLTEANE KIEVQNQEMR
241 KNLEESVQEM EKMTDEYNRM KAIVHQTDNV IDQLKKENDH YQLQVQELTD LLKSKNEEDD
301 PIMVAVNAKV EEWKLILSSK DDEIIEYQQM LHNLREKLKN AQLDADKSNV MALQQGIQER
361 DSQIKMLTEQ VEQYTKEMEK NTCIIEDLKN ELQRNKGAST LSQQTHMKIQ STLDILKEKT
421 KEAERTAELA EADAREKDKE LVEALKRLKD YESGVYGLED AVVEIKNCKN QIKIRDREIE
481 ILTKEINKLE LKISDFLDEN EALRERVGLE PKTMIDLTEF RNSKHLKQQQ YRAENQILLK
541 EIESLEEERL DLKKKIRQMA QERGKRSATS GLTTEDLNLT ENISQGDRIS ERKLDLLSLK
601 NMSEAQSKIR SSDKAELLHR RSSFNTPQSD QNETEENMTI GSLSRMLSEI HHSVESGMHP
661 FVPLTRLSSS MQVKENSTPE TITIREIFKA PCLQSSRNLE SLVSTFSRES HEEINDICLF
721 SDDCMKKVSR SHQALEKTSF VQKSNSSFHG LSTASDIMQK LSLRQKSAIF CQQIHENRAD
781 MDKSQVATLE EEQVHSQVKY ADINLKEDII KSEVPLQTEI LKNKLKVNLP DPVSITAQSK
841 LSQINSLENL IEQLRRELVF LRSQNEIIAQ EFLIKEAECR NADIELEHHR SQAEQNEFLS
901 RELIEKERDL ERSRTVIAKF QNKLKELVEE NKQLEEGMKE ILQAIKEMQK DPDVKGGETS
961 LIIPSLERLV NAIESKNAEG IFDASLHLKA QVDQLTGRNE ELRQELRESR KEAINYSQQL
1021 AKANLKIDHL EKETSLLRQS EGSNVVFKGI DLPDGIAPSS ASIINSQNEY LIHLLQELEN
1081 KEKKLKNLED SLEDYNRKFA VIRHQQSLLY KEYLSEKETW KTESKTIKEE KRKLEDQVQQ
1141 DAIKVKEYNN LLNALQMDSD EMKKILAENS RKITVLQVNE KSLIRQYTTL VELERQLRKE
1201 NEKQKNELLS MEAEVCEKIG CLQRFKEMAI FKIAALQKVV DNSVSLSELE LANKQYNELT
1261 AKYRDILQKD NMLVQRTSNL EHLECENISL KEQVESINKE LEITKEKLHT IEQAWEQETK
1321 LGNESSMDKA KKSITNSDIV SISKKITMLE MKELNERQRA EHCQKMYEHL RTSLKQMEER
1381 NFELETKFAE LTKINLDAQK VEQMLRDELA DSVSKAVSDA DRQRILELEK NEMELKVEVS
1441 KLREISDIAR RQVEILNAQQ QSRDKEVESL RMQLLDYQAQ SDEKSLIAKL HQHNVSLQLS
1501 EATALGKLES ITSKLQKMEA YNLRLEQKLD EKEQALYYAR LEGRNRAKHL RQTIQSLRRQ
1561 FSGALPLAQQ EKFSKTMIQL QNDKLKIMQE MKNSQQEHRN MENKTLEMEL KLKGLEELIS
1621 TLKDTKGAQK VINWHMKIEE LRLQELKLNR ELVKDKEEIK YLNNIISEYE RTISSLEEEI
1681 VQQNKFHEER QMAWDQREVD LERQLDIFDR QQNEILNAAQ KFEEATGSIP DPSLPLPNQL
1741 EIALRKIKEN IRIILETRAT CKSLEEKLKE KESALRLAEQ NILSRDKVIN ELRLRLPATA
1801 EREKLIAELG RKEMEPKSHH TLKIAHQTIA NMQARLNQKE EVLKKYQRLL EKAREEQREI
1861 VKKHEEDLHI LHHRLELQAD SSLNKFKQTA WDLMKQSPTP VPTNKHFIRL AEMEQTVAEQ
1921 DDSLSSLLVK LKKVSQDLER QREITELKVK EFENIKLQLQ ENHEDEVKKV KAEVEDLKYL
1981 LDQSQKESQC LKSELQAQKE ANSRAPTTTM RNLVERLKSQ LALKEKQQKA LSRALLELRA
2041 EMTAAAEERI ISATSQKEAH LNVQQIVDRH TRELKTQVED LNENLLKLKE ALKTSKNREN
2101 SLTDNLNDLN NELQKKQKAY NKILREKEEI DQENDELKRQ IKRLTSGLQG KPLTDNKQSL
 
2161 IEELQRKVKK LENQLEGKVE EVDLKPMKEK NAKEELIRWE EGKKWQAKIE GIRNKLKEKE
2221 GEVFTLTKQL NTLKDLFAKA DKEKLTLQRK LKTTGMTVDQ VLGIRALESE KELEELKKRN
2281 LDLENDILYM RAHQALPRDS VVEDLHLQNR YLQEKLHALE KQFSKDTYSK PSQNQISGIE
2341 SDDHCQREQE LQKENLKLSS ENIELKFQLE QANKDLPRLK NQVRDLKEMC EFLKKEKAEV
2401 QRKLGHVRGS GRSGKTIPEL EKTIGLMKKV VEKVQRENEQ LKKASGILTS EKMANIEQEN
2461 EKLKAELEKL KAHLGHQLSM HYESKTKGTE KIIAENERLR KELKKETDAA EKLRIAKNNL
2521 EILNEKMTVQ LEETGKRLQF AESRGPQLEG ADSKSWKSIV VTRMYETKLK ELETDIAKKN
2581 QSITDLKQLV KEATEREQKV NKYNEDLEQQ IS
SEQ ID NO:71
   CENTROSOMAL PROTEIN 290 (CEP290), ISOFORM X9
   XP_011537064.1 
1 MPPNINWKEI MKVDPDDLPR QEELADNLLI SLSKVEVNEL KSEKQENVIH LFRITQSLMK
61 MKAQEVELAL EEVEKAGEEQ AKFENQLKTK VMKLENELEM AQQSAGGRDT RFLRNEICQL
121 EKQLEQKDRE LEDMEKELEK EKKVNEQLAL RNEEAENENS KLRRENKRLK KKNEQLCQDI
181 IDYQKQIDSQ KETLLSRRGE DSDYRSQLSK KNYELIQYLD EIQTLTEANE KIEVQNQEMR
241 KNLEESVQEM EKMTDEYNRM KAIVHQTDNV IDQLKKENDH YQLQVQELTD LLKSKNEEDD
301 PIMVAVNAKV EEWKLILSSK DDEIIEYQQM LHNLREKLKN AQLDADKSNV MALQQGIQER
361 DSQIKMLTEQ VEQYTKEMEK NTCIIEDLKN ELQRNKGAST LSQQTHMKIQ STLDILKEKT
421 KEAERTAELA EADAREKDKE LVEALKRLKD YESGVYGLED AVVEIKNCKN QIKIRDREIE
481 ILTKEINKLE LKISDFLDEN EALRERVGLE PKTMIDLTEF RNSKHLKQQQ YRAENQILLK
541 EIESLEEERL DLKKKIRQMA QERGKRSATS GLTTEDLNLT ENISQGDRIS ERKLDLLSLK
601 NMSEAQSKNE IIAQEFLIKE AECRNADIEL EHHRSQAEQN EFLSRELIEK ERDLERSRTV
661 IAKFQNKLKE LVEENKQLEE GMKEILQAIK EMQKDPDVKG GETSLIIPSL ERLVNAIESK
721 NAEGIFDASL HLKAQVDQLT GRNEELRQEL RESRKEAINY SQQLAKANLK IDHLEKETSL
781 LRQSEGSNVV FKGIDLPDGI APSSASIINS QNEYLIHLLQ ELENKEKKLK NLEDSLEDYN
841 RKFAVIRHQQ SLLYKEYLSE KETWKTESKT IKEEKRKLED QVQQDAIKVK EYNNLLNALQ
901 MDSDEMKKIL AENSRKITVL QVNEKSLIRQ YTTLVELERQ LRKENEKQKN ELLSMEAEVC
961 EKIGCLQRFK EMAIFKIAAL QKVVDNSVSL SELELANKQY NELTAKYRDI LQKDNMLVQR
1021 TSNLEHLECE NISLKEQVES INKELEITKE KLHTIEQAWE QETKLGNESS MDKAKKSITN
1081 SDIVSISKKI TMLEMKELNE RQRAEHCQKM YEHLRTSLKQ MEERNFELET KFAELTKINL
1141 DAQKVEQMLR DELADSVSKA VSDADRQRIL ELEKNEMELK VEVSKLREIS DIARRQVEIL
1201 NAQQQSRDKE VESLRMQLLD YQAQSDEKSL IAKLHQHNVS LQLSEATALG KLESITSKLQ
1261 KMEAYNLRLE QKLDEKEQAL YYARLEGRNR AKHLRQTIQS LRRQFSGALP LAQQEKFSKT
1321 MIQLQNDKLK IMQEMKNSQQ EHRNMENKTL EMELKLKGLE ELISTLKDTK GAQKVINWHM
1381 KIEELRLQEL KLNRELVKDK EEIKYLNNII SEYERTISSL EEEIVQQNKF HEERQMAWDQ
1441 REVDLERQLD IFDRQQNEIL NAAQKFEEAT GSIPDPSLPL PNQLEIALRK IKENIRIILE
1501 TRATCKSLEE KLKEKESALR LAEQNILSRD KVINELRLRL PATAEREKLI AELGRKEMEP
1561 KSHHTLKIAH QTIANMQARL NQKEEVLKKY QRLLEKAREE QREIVKKHEE DLHILHHRLE
1621 LQADSSLNKF KQTAWDLMKQ SPTPVPTNKH FIRLAEMEQT VAEQDDSLSS LLVKLKKVSQ
1681 DLERQREITE LKVKEFENIK LQLQENHEDE VKKVKAEVED LKYLLDQSQK ESQCLKSELQ
1741 AQKEANSRAP TTTMRNLVER LKSQLALKEK QQKALSRALL ELRAEMTAAA EERIISATSQ
1801 KEAHLNVQQI VDRHTRELKT QVEDLNENLL KLKEALKTSK NRENSLTDNL NDLNNELQKK
1861 QKAYNKILRE KEEIDQENDE LKRQIKRLTS GLQGKPLTDN KQSLIEELQR KVKKLENQLE
1921 GKVEEVDLKP MKEKNAKEEL IRWEEGKKWQ AKIEGIRNKL KEKEGEVFTL TKQLNTLKDL
1981 FAKADKEKLT LQRKLKTTGM TVDQVLGIRA LESEKELEEL KKRNLDLEND ILYMRAHQAL
2041 PRDSVVEDLH LQNRYLQEKL HALEKQFSKD TYSKPSQNQI SGIESDDHCQ REQELQKENL
2101 KLSSENIELK FQLEQANKDL PRLKNQVRDL KEMCEFLKKE KAEVQRKLGH VRGSGRSGKT
2161 IPELEKTIGL MKKVVEKVQR ENEQLKKASG ILTSEKMANI EQENEKLKAE LEKLKAHLGH
2221 QLSMHYESKT KGTEKIIAEN ERLRKELKKE TDAAEKLRIA KNNLEILNEK MTVQLEETGK
2281 RLQFAESRGP QLEGADSKSW KSIVVTRMYE TKLKELETDI AKKNQSITDL KQLVKEATER
2341 EQKVNKYNED LEQQIKILKH VPEGAETEQG LKRELQVLRL ANHQLDKEKA ELIHQIEANK
2401 DQSGAESTIP DADQLKEKIK DLETQLKMSD LEKQHLKEEI KKLKKELENF DPSFFEEIED
2461 LKYNYKEEVK KNILLEEKVK KLSEQLGVEL TSPVAASEEF EDEEESPVNF PIY
SEQ ID NO:72
   CENTROSOMAL PROTEIN 290 (CEP290), ISOFORM X10
   XP_011537065.1 
1 MPPNINWKEI MKVDPDDLPR QEELADNLLI SLSKVEVNEL KSEKQENVIH LFRITQSLMK
61 MKAQEVELAL EEVEKAGEEQ AKFENQLKTK VMKLENELEM AQQSAGGRDT RFLRNEICQL
121 EKQLEQKDRE LEDMEKELEK EKKVNEQLAL RNEEAENENS KLRRENKRLK KKNEQLCQDI
181 IDYQKQIDSQ KETLLSRRGE DSDYRSQLSK KNYELIQYLD EIQTLTEANE KIEVQNQEMR
241 KNLEESVQEM EKMTDEYNRM KAIVHQTDNV IDQLKKENDH YQLQVQELTD LLKSKNEEDD
301 PIMVAVNAKV EEWKLILSSK DDEIIEYQQM LHNLREKLKN AQLDADKSNV MALQQGIQER
361 DSQIKMLTEQ VEQYTKEMEK NTCIIEDLKN ELQRNKGAST LSQQTHMKIQ STLDILKEKT
421 KEAERTAELA EADAREKDKE LVEALKRLKD YESGVYGLED AVVEIKNCKN QIKIRDREIE
481 ILTKEINKLE LKISDFLDEN EALRERVGLE PKTMIDLTEF RNSKHLKQQQ YRAENQILLK
541 EIESLEEERL DLKKKIRQMA QERGKRSATS GLTTEDLNLT ENISQGDRIS ERKLDLLSLK
601 NMSEAQSKNE FLSRELIEKE RDLERSRTVI AKFQNKLKEL VEENKQLEEG MKEILQAIKE
661 MQKDPDVKGG ETSLIIPSLE RLVNAIESKN AEGIFDASLH LKAQVDQLTG RNEELRQELR
721 ESRKEAINYS QQLAKANLKI DHLEKETSLL RQSEGSNVVF KGIDLPDGIA PSSASIINSQ
781 NEYLIHLLQE LENKEKKLKN LEDSLEDYNR KFAVIRHQQS LLYKEYLSEK ETWKTESKTI
841 KEEKRKLEDQ VQQDAIKVKE YNNLLNALQM DSDEMKKILA ENSRKITVLQ VNEKSLIRQY
901 TTLVELERQL RKENEKQKNE LLSMEAEVCE KIGCLQRFKE MAIFKIAALQ KVVDNSVSLS
961 ELELANKQYN ELTAKYRDIL QKDNMLVQRT SNLEHLECEN ISLKEQVESI NKELEITKEK
1021 LHTIEQAWEQ ETKLGNESSM DKAKKSITNS DIVSISKKIT MLEMKELNER QRAEHCQKMY
1081 EHLRTSLKQM EERNFELETK FAELTKINLD AQKVEQMLRD ELADSVSKAV SDADRQRILE
1141 LEKNEMELKV EVSKLREISD IARRQVEILN AQQQSRDKEV ESLRMQLLDY QAQSDEKSLI
1201 AKLHQHNVSL QLSEATALGK LESITSKLQK MEAYNLRLEQ KLDEKEQALY YARLEGRNRA
1261 KHLRQTIQSL RRQFSGALPL AQQEKFSKTM IQLQNDKLKI MQEMKNSQQE HRNMENKTLE
1321 MELKLKGLEE LISTLKDTKG AQKVINWHMK IEELRLQELK LNRELVKDKE EIKYLNNIIS
1381 EYERTISSLE EEIVQQNKFH EERQMAWDQR EVDLERQLDI FDRQQNEILN AAQKFEEATG
1441 SIPDPSLPLP NQLEIALRKI KENIRIILET RATCKSLEEK LKEKESALRL AEQNILSRDK
1501 VINELRLRLP ATAEREKLIA ELGRKEMEPK SHHTLKIAHQ TIANMQARLN QKEEVLKKYQ
1561 RLLEKAREEQ REIVKKHEED LHILHHRLEL QADSSLNKFK QTAWDLMKQS PTPVPTNKHF
1621 IRLAEMEQTV AEQDDSLSSL LVKLKKVSQD LERQREITEL KVKEFENIKL QLQENHEDEV
1681 KKVKAEVEDL KYLLDQSQKE SQCLKSELQA QKEANSRAPT TTMRNLVERL KSQLALKEKQ
1741 QKALSRALLE LRAEMTAAAE ERIISATSQK EAHLNVQQIV DRHTRELKTQ VEDLNENLLK
1801 LKEALKTSKN RENSLTDNLN DLNNELQKKQ KAYNKILREK EEIDQENDEL KRQIKRLTSG
1861 LQGKPLTDNK QSLIEELQRK VKKLENQLEG KVEEVDLKPM KEKNAKEELI RWEEGKKWQA
1921 KIEGIRNKLK EKEGEVFTLT KQLNTLKDLF AKADKEKLTL QRKLKTTGMT VDQVLGIRAL
1981 ESEKELEELK KRNLDLENDI LYMRAHQALP RDSVVEDLHL QNRYLQEKLH ALEKQFSKDT
2041 YSKPSQNQIS GIESDDHCQR EQELQKENLK LSSENIELKF QLEQANKDLP RLKNQVRDLK
2101 EMCEFLKKEK AEVQRKLGHV RGSGRSGKTI PELEKTIGLM KKVVEKVQRE NEQLKKASGI
2161 LTSEKMANIE QENEKLKAEL EKLKAHLGHQ LSMHYESKTK GTEKIIAENE RLRKELKKET
2221 DAAEKLRIAK NNLEILNEKM TVQLEETGKR LQFAESRGPQ LEGADSKSWK SIVVTRMYET
2281 KLKELETDIA KKNQSITDLK QLVKEATERE QKVNKYNEDL EQQIKILKHV PEGAETEQGL
2341 KRELQVLRLA NHQLDKEKAE LIHQIEANKD QSGAESTIPD ADQLKEKIKD LETQLKMSDL
2401 EKQHLKEEIK KLKKELENFD PSFFEEIEDL KYNYKEEVKK NILLEEKVKK LSEQLGVELT
2461 SPVAASEEFE DEEESPVNFP IY
SEQ ID NO: 73
   CENTROSOMAL PROTEIN 290 (CEP290), ISOFORM X11
   XP_016875472.1 
1 MPPNINWKEI MKVDPDDLPR QEELADNLLI SLSKVEVNEL KSEKQENVIH LFRITQSLMK
61 MKAQEVELAL EEVEKAGEEQ AKFENQLKTK VMKLENELEM AQQSAGGRDT RFLRNEICQL
121 EKQLEQKDRE LEDMEKELEK EKKVNEQLAL RNEEAENENS KLRRENEQLC QDIIDYQKQI
181 DSQKETLLSR RGEDSDYRSQ LSKKNYELIQ YLDEIQTLTE ANEKIEVQNQ EMRKNLEESV
241 QEMEKMTDEY NRMKAIVHQT DNVIDQLKKE NDHYQLQVQE LTDLLKSKNE EDDPIMVAVN
301 AKVEEWKLIL SSKDDEIIEY QQMLHNLREK LKNAQLDADK SNVMALQQGI QERDSQIKML
361 TEQVEQYTKE MEKNTCIIED LKNELQRNKG ASTLSQQTHM KIQSTLDILK EKTKEAERTA
421 ELAEADAREK DKELVEALKR LKDYESGVYG LEDAVVEIKN CKNQIKIRDR EIEILTKEIN
481 KLELKISDFL DENEALRERV GLEPKTMIDL TEFRNSKHLK QQQYRAENQI LLKEIESLEE
541 ERLDLKKKIR QMAQERGKRS ATSGLTTEDL NLTENISQGD RISERKLDLL SLKNMSEAQS
 
601 KNEFLSRELI EKERDLERSR TVIAKFQNKL KELVEENKQL EEGMKEILQA IKEMQKDPDV
661 KGGETSLIIP SLERLVNAIE SKNAEGIFDA SLHLKAQVDQ LTGRNEELRQ ELRESRKEAI
721 NYSQQLAKAN LKIDHLEKET SLLRQSEGSN VVFKGIDLPD GIAPSSASII NSQNEYLIHL
781 LQELENKEKK LKNLEDSLED YNRKFAVIRH QQSLLYKEYL SEKETWKTES KTIKEEKRKL
841 EDQVQQDAIK VKEYNNLLNA LQMDSDEMKK ILAENSRKIT VLQVNEKSLI RQYTTLVELE
901 RQLRKENEKQ KNELLSMEAE VCEKIGCLQR FKEMAIFKIA ALQKVVDNSV SLSELELANK
961 QYNELTAKYR DILQKDNMLV QRTSNLEHLE CENISLKEQV ESINKELEIT KEKLHTIEQA
1021 WEQETKLGNE SSMDKAKKSI TNSDIVSISK KITMLEMKEL NERQRAEHCQ KMYEHLRTSL
1081 KQMEERNFEL ETKFAELTKI NLDAQKVEQM LRDELADSVS KAVSDADRQR ILELEKNEME
1141 LKVEVSKLRE ISDIARRQVE ILNAQQQSRD KEVESLRMQL LDYQAQSDEK SLIAKLHQHN
1201 VSLQLSEATA LGKLESITSK LQKMEAYNLR LEQKLDEKEQ ALYYARLEGR NRAKHLRQTI
1261 QSLRRQFSGA LPLAQQEKFS KTMIQLQNDK LKIMQEMKNS QQEHRNMENK TLEMELKLKG
1321 LEELISTLKD TKGAQKVINW HMKIEELRLQ ELKLNRELVK DKEEIKYLNN IISEYERTIS
1381 SLEEEIVQQN KFHEERQMAW DQREVDLERQ LDIFDRQQNE ILNAAQKFEE ATGSIPDPSL
1441 PLPNQLEIAL RKIKENIRII LETRATCKSL EEKLKEKESA LRLAEQNILS RDKVINELRL
1501 RLPATAEREK LIAELGRKEM EPKSHHTLKI AHQTIANMQA RLNQKEEVLK KYQRLLEKAR
1561 EEQREIVKKH EEDLHILHHR LELQADSSLN KFKQTAWDLM KQSPTPVPTN KHFIRLAEME
1621 QTVAEQDDSL SSLLVKLKKV SQDLERQREI TELKVKEFEN IKLQLQENHE DEVKKVKAEV
1681 EDLKYLLDQS QKESQCLKSE LQAQKEANSR APTTTMRNLV ERLKSQLALK EKQQKALSRA
1741 LLELRAEMTA AAEERIISAT SQKEAHLNVQ QIVDRHTREL KTQVEDLNEN LLKLKEALKT
1801 SKNRENSLTD NLNDLNNELQ KKQKAYNKIL REKEEIDQEN DELKRQIKRL TSGLQGKPLT
1861 DNKQSLIEEL QRKVKKLENQ LEGKVEEVDL KPMKEKNAKE ELIRWEEGKK WQAKIEGIRN
1921 KLKEKEGEVF TLTKQLNTLK DLFAKADKEK LTLQRKLKTT GMTVDQVLGI RALESEKELE
1981 ELKKRNLDLE NDILYMRAHQ ALPRDSVVED LHLQNRYLQE KLHALEKQFS KDTYSKPSQN
2041 QISGIESDDH CQREQELQKE NLKLSSENIE LKFQLEQANK DLPRLKNQVR DLKEMCEFLK
2101 KEKAEVQRKL GHVRGSGRSG KTIPELEKTI GLMKKVVEKV QRENEQLKKA SGILTSEKMA
2161 NIEQENEKLK AELEKLKAHL GHQLSMHYES KTKGTEKIIA ENERLRKELK KETDAAEKLR
2221 IAKNNLEILN EKMTVQLEET GKRLQFAESR GPQLEGADSK SWKSIVVTRM YETKLKELET
2281 DIAKKNQSIT DLKQLVKEAT EREQKVNKYN EDLEQQIKIL KHVPEGAETE QGLKRELQVL
2341 RLANHQLDKE KAELIHQIEA NKDQSGAEST IPDADQLKEK IKDLETQLKM SDLEKQHLKE
2401 EIKKLKKELE NFDPSFFEEI EDLKYNYKEE VKKNILLEEK VKKLSEQLGV ELTSPVAASE
2461 EFEDEEESPV NFPIY
SEQ ID NO:74
   CENTROSOMAL PROTEIN 290 (CEP290), ISOFORM X12
   XP_011537066.1 
1 MPPNINWKEI MKVDPDDLPR QEELADNLLI SLSKVEVNEL KSEKQENVIH LFRITQSLMK
61 MKAQEVELAL EEVEKAGEEQ AKFENQLKTK VMKLENELEM AQQSAGGRDT RFLRNEICQL
121 EKQLEQKDRE LEDMEKELEK EKKVNEQLAL RNEEAENENS KLRRENKRLK KKNEQLCQDI
181 IDYQKQIDSQ KETLLSRRGE DSDYRSQLSK KNYELIQYLD EIQTLTEANE KIEVQNQEMR
241 KNLEESVQEM EKMTDEYNRM KAIVHQTDNV IDQLKKENDH YQLQVQELTD LLKSKNEEDD
301 PIMVAVNAKV EEWKLILSSK DDEIIEYQQM LHNLREKLKN AQLDADKSNV MALQQGIQER
361 DSQIKMLTEQ VEQYTKEMEK NTCIIEDLKN ELQRNKGAST LSQQTHMKIQ STLDILKEKT
421 KEAERTAELA EADAREKDKE LVEALKRLKD YESGVYGLED AVVEIKNCKN QIKIRDREIE
481 ILTKEINKLE LKISDFLDEN EALRERVGLE PKTMIDLTEF RNSKHLKQQQ YRAENQILLK
541 EIESLEEERL DLKKKIRQMA QERGKRSATS GLTTEDLNLT ENISQGDRIS ERKLDLLSLK
601 NMSEAQSKIR SSDKAELLHR RSSFNTPQSD QNETEENMTI GSLSRMLSEI HHSVESGMHP
661 FVPLTRLSSS MQVKENSTPE TITIREIFKA PCLQSSRNLE SLVSTFSRES HEEINDICLF
721 SDDCMKKVSR SHQALEKTSF VQKSNSSFHG LSTASDIMQK LSLRQKSAIF CQQIHENRAD
781 MDKSQVATLE EEQVHSQVKY ADINLKEDII KSEVPLQTEI LKNKLKVNLP DPVSITAQSK
841 LSQINSLENL IEQLRRELVF LRSQNEIIAQ EFLIKEAECR NADIELEHHR SQAEQNEFLS
901 RELIEKERDL ERSRTVIAKF QNKLKELVEE NKQLEEGMKE ILQAIKEMQK DPDVKGGETS
961 LIIPSLERLV NAIESKNAEG IFDASLHLKA QVDQLTGRNE ELRQELRESR KEAINYSQQL
1021 AKANLKIDHL EKETSLLRQS EGSNVVFKGI DLPDGIAPSS ASIINSQNEY LIHLLQELEN
1081 KEKKLKNLED SLEDYNRKFA VIRHQQSLLY KEYLSEKETW KTESKTIKEE KRKLEDQVQQ
1141 DAIKVKEYNN LLNALQMDSD EMKKILAENS RKITVLQVNE KSLIRQYTTL VELERQLRKE
1201 NEKQKNELLS MEAEVCEKIG CLQRFKEMAI FKIAALQKVV DNSVSLSELE LANKQYNELT
 
1261 AKYRDILQKD NMLVQRTSNL EHLECENISL KEQVESINKE LEITKEKLHT IEQAWEQETK
1321 LGNESSMDKA KKSITNSDIV SISKKITMLE MKELNERQRA EHCQKMYEHL RTSLKQMEER
1381 NFELETKFAE LTKINLDAQK VEQMLRDELA DSVSKAVSDA DRQRILELEK NEMELKVEVS
1441 KLREISDIAR RQVEILNAQQ QSRDKEVESL RMQLLDYQAQ SDEKSLIAKL HQHNVSLQLS
1501 EATALGKLES ITSKLQKMEA YNLRLEQKLD EKEQALYYAR LEGRNRAKHL RQTIQSLRRQ
1561 FSGALPLAQQ EKFSKTMIQL QNDKLKIMQE MKNSQQEHRN MENKTLEMEL KLKGLEELIS
1621 TLKDTKGAQK VINWHMKIEE LRLQELKLNR ELVKDKEEIK YLNNIISEYE RTISSLEEEI
1681 VQQNKFHEER QMAWDQREVD LERQLDIFDR QQNEILNAAQ KFEEATGSIP DPSLPLPNQL
1741 EIALRKIKEN IRIILETRAT CKSLEEKLKE KESALRLAEQ NILSRDKVIN ELRLRLPATA
1801 EREKLIAELG RKEMEPKSHH TLKIAHQTIA NMQARLNQKE EVLKKYQRLL EKAREEQREI
1861 VKKHEEDLHI LHHRLELQAD SSLNKFKQTA WDLMKQSPTP VPTNKHFIRL AEMEQTVAEQ
1921 DDSLSSLLVK LKKVSQDLER QREITELKVK EFENIKLQLQ ENHEDEVKKV KAEVEDLKYL
1981 LDQSQKESQC LKSELQAQKE ANSRAPTTTM RNLVERLKSQ LALKEKQQKA LSRALLELRA
2041 EMTAAAEERI ISATSQKEAH LNVQQIVDRH TRELKTQVED LNENLLKLKE ALKTSKNREN
2101 SLTDNLNDLN NELQKKQKAY NKILREKEEI DQENDELKRQ IKRLTSGLQG KPLTDNKQSL
2161 IEELQRKVKK LENQLEGKVE EVDLKPMKEK NAKEELIRWE EGKKWQAKIE GIRNKLKEKE
2221 GEVFTLTKQL NTLKDLFAKA DKEKLTLQRK LKTTGMTVDQ VLGIRALESE KELEELKKRN
2281 LDLENDILYM RAHQALPRDS VVEDLHLQNR YLQEKLHALE KQFSKDTYSK PSQNQDTSHS
2341 RIGFTLKSHF NLNTSVKTQS PNKVTF
SEQ ID NO:75
   CENTROSOMAL PROTEIN 290 (CEP290), ISOFORM X13
   XP_011537067.1 
1 MPPNINWKEI MKVDPDDLPR QEELADNLLI SLSKVEVNEL KSEKQENVIH LFRITQSLMK
61 MKAQEVELAL EEVEKAGEEQ AKFENQLKTK VMKLENELEM AQQSAGGRDT RFLRNEICQL
121 EKQLEQKDRE LEDMEKELEK EKKVNEQLAL RNEEAENENS KLRRENKRLK KKNEQLCQDI
181 IDYQKQIDSQ KETLLSRRGE DSDYRSQLSK KNYELIQYLD EIQTLTEANE KIEVQNQEMR
241 KNLEESVQEM EKMTDEYNRM KAIVHQTDNV IDQLKKENDH YQLQVQELTD LLKSKNEEDD
301 PIMVAVNAKV EEWKLILSSK DDEIIEYQQM LHNLREKLKN AQLDADKSNV MALQQGIQER
361 DSQIKMLTEQ VEQYTKEMEK NTCIIEDLKN ELQRNKGAST LSQQTHMKIQ STLDILKEKT
421 KEAERTAELA EADAREKDKE LVEALKRLKD YESGVYGLED AVVEIKNCKN QIKIRDREIE
481 ILTKEINKLE LKISDFLDEN EALRERVGLE PKTMIDLTEF RNSKHLKQQQ YRAENQILLK
541 EIESLEEERL DLKKKIRQMA QERGKRSATS GLTTEDLNLT ENISQGDRIS ERKLDLLSLK
601 NMSEAQSKIR SSDKAELLHR RSSFNTPQSD QNETEENMTI GSLSRMLSEI HHSVESGMHP
661 FVPLTRLSSS MQVKENSTPE TITIREIFKA PCLQSSRNLE SLVSTFSRES HEEINDICLF
721 SDDCMKKVSR SHQALEKTSF VQKSNSSFHG LSTASDIMQK LSLRQKSAIF CQQIHENRAD
781 MDKSQVATLE EEQVHSQVKY ADINLKEDII KSEVPLQTEI LKNKLKVNLP DPVSITAQSK
841 LSQINSLENL IEQLRRELVF LRSQNEIIAQ EFLIKEAECR NADIELEHHR SQAEQNEFLS
901 RELIEKERDL ERSRTVIAKF QNKLKELVEE NKQLEEGMKE ILQAIKEMQK DPDVKGGETS
961 LIIPSLERLV NAIESKNAEG IFDASLHLKA QVDQLTGRNE ELRQELRESR KEAINYSQQL
1021 AKANLKIDHL EKETSLLRQS EGSNVVFKGI DLPDGIAPSS ASIINSQNEY LIHLLQELEN
1081 KEKKLKNLED SLEDYNRKFA VIRHQQSLLY KEYLSEKETW KTESKTIKEE KRKLEDQVQQ
1141 DAIKVKEYNN LLNALQMDSD EMKKILAENS RKITVLQVNE KSLIRQYTTL VELERQLRKE
1201 NEKQKNELLS MEAEVCEKIG CLQRFKEMAI FKIAALQKVV DNSVSLSELE LANKQYNELT
1261 AKYRDILQKD NMLVQRTSNL EHLECENISL KEQVESINKE LEITKEKLHT IEQAWEQETK
1321 LGNESSMDKA KKSITNSDIV SISKKITMLE MKELNERQRA EHCQKMYEHL RTSLKQMEER
1381 NFELETKFAE LTKINLDAQK VEQMLRDELA DSVSKAVSDA DRQRILELEK NEMELKVEVS
1441 KLREISDIAR RQVEILNAQQ QSRDKEVESL RMQLLDYQAQ SDEKSLIAKL HQHNVSLQLS
1501 EATALGKLES ITSKLQKMEA YNLRLEQKLD EKEQALYYAR LEGRNRAKHL RQTIQSLRRQ
1561 FSGALPLAQQ EKFSKTMIQL QNDKLKIMQE MKNSQQEHRN MENKTLEMEL KLKGLEELIS
1621 TLKDTKGAQK VINWHMKIEE LRLQELKLNR ELVKDKEEIK YLNNIISEYE RTISSLEEEI
1681 VQQNKFHEER QMAWDQREVD LERQLDIFDR QQNEILNAAQ KFEEATGSIP DPSLPLPNQL
1741 EIALRKIKEN IRIILETRAT CKSLEEKLKE KESALRLAEQ NILSRDKVIN ELRLRLPATA
1801 EREKLIAELG RKEMEPKSHH TLKIAHQTIA NMQARLNQKE EVLKKYQRLL EKAREEQREI
1861 VKKHEEDLHI LHHRLELQAD SSLNKFKQTA WDLMKQSPTP VPTNKHFIRL AEMEQTVAEQ
1921 DDSLSSLLVK LKKVSQDLER QREITELKVK EFENIKLQLQ ENHEDEVKKV KAEVEDLKYL
1981 LDQSQKESQC LKSELQAQKE ANSRAPTTTM RNLVERLKSQ LALKEKQQKA LSRALLELRA
 
2041 EMTAAAEERI ISATSQKEAH LNVQQIVDRH TRELKTQVED LNENLLKLKE ALKTSKNREN
2101 SLTDNLNDLN NELQKKQKAY NKILREKEEI DQENDELKRQ IKRLTSGLQG KPLTDNKQSL
2161 IEELQRKVKK LENQLEGKVE EVDLKPMKEK NAKEELIRWE EGKKWQAKIE GIRNKLKEKE
2221 GEVFTLTKQL NTLKDLFAKA DKEKLTLQRK LKTTGMTVDQ VLGIRALESE KELEELKKRN
2281 LDLENDILYM RAHQALPRDS VVEDLHLQNR YLQEKLHALE KQFSKDTYSK PSNQDTSHSR
2341 IGFTLKSHFN LNTSVKTQSP NKVTF
SEQ ID NO:76
   CENTROSOMAL PROTEIN 290 (CEP290), ISOFORM X14
   XP_011537068.1 
1 MIDLTEFRNS KHLKQQQYRA ENQILLKEIE SLEEERLDLK KKIRQMAQER GKRSATSGLT
61 TEDLNLTENI SQGDRISERK LDLLSLKNMS EAQSKIRSSD KAELLHRRSS FNTPQSDQNE
121 TEENMTIGSL SRMLSEIHHS VESGMHPFVP LTRLSSSMQV KENSTPETIT IREIFKAPCL
181 QSSRNLESLV STFSRESHEE INDICLFSDD CMKKVSRSHQ ALEKTSFVQK SNSSFHGLST
241 ASDIMQKLSL RQKSAIFCQQ IHENRADMDK SQVATLEEEQ VHSQVKYADI NLKEDIIKSE
301 VPLQTEILKN KLKVNLPDPV SITAQSKLSQ INSLENLIEQ LRRELVFLRS QNEIIAQEFL
361 IKEAECRNAD IELEHHRSQA EQNEFLSREL IEKERDLERS RTVIAKFQNK LKELVEENKQ
421 LEEGMKEILQ AIKEMQKDPD VKGGETSLII PSLERLVNAI ESKNAEGIFD ASLHLKAQVD
481 QLTGRNEELR QELRESRKEA INYSQQLAKA NLKIDHLEKE TSLLRQSEGS NVVFKGIDLP
541 DGIAPSSASI INSQNEYLIH LLQELENKEK KLKNLEDSLE DYNRKFAVIR HQQSLLYKEY
601 LSEKETWKTE SKTIKEEKRK LEDQVQQDAI KVKEYNNLLN ALQMDSDEMK KILAENSRKI
661 TVLQVNEKSL IRQYTTLVEL ERQLRKENEK QKNELLSMEA EVCEKIGCLQ RFKEMAIFKI
721 AALQKVVDNS VSLSELELAN KQYNELTAKY RDILQKDNML VQRTSNLEHL ECENISLKEQ
781 VESINKELEI TKEKLHTIEQ AWEQETKLGN ESSMDKAKKS ITNSDIVSIS KKITMLEMKE
841 LNERQRAEHC QKMYEHLRTS LKQMEERNFE LETKFAELTK INLDAQKVEQ MLRDELADSV
901 SKAVSDADRQ RILELEKNEM ELKVEVSKLR EISDIARRQV EILNAQQQSR DKEVESLRMQ
961 LLDYQAQSDE KSLIAKLHQH NVSLQLSEAT ALGKLESITS KLQKMEAYNL RLEQKLDEKE
1021 QALYYARLEG RNRAKHLRQT IQSLRRQFSG ALPLAQQEKF SKTMIQLQND KLKIMQEMKN
1081 SQQEHRNMEN KTLEMELKLK GLEELISTLK DTKGAQKVIN WHMKIEELRL QELKLNRELV
1141 KDKEEIKYLN NIISEYERTI SSLEEEIVQQ NKFHEERQMA WDQREVDLER QLDIFDRQQN
1201 EILNAAQKFE EATGSIPDPS LPLPNQLEIA LRKIKENIRI ILETRATCKS LEEKLKEKES
1261 ALRLAEQNIL SRDKVINELR LRLPATAERE KLIAELGRKE MEPKSHHTLK IAHQTIANMQ
1321 ARLNQKEEVL KKYQRLLEKA REEQREIVKK HEEDLHILHH RLELQADSSL NKFKQTAWDL
1381 MKQSPTPVPT NKHFIRLAEM EQTVAEQDDS LSSLLVKLKK VSQDLERQRE ITELKVKEFE
1441 NIKLQLQENH EDEVKKVKAE VEDLKYLLDQ SQKESQCLKS ELQAQKEANS RAPTTTMRNL
1501 VERLKSQLAL KEKQQKALSR ALLELRAEMT AAAEERIISA TSQKEAHLNV QQIVDRHTRE
1561 LKTQVEDLNE NLLKLKEALK TSKNRENSLT DNLNDLNNEL QKKQKAYNKI LREKEEIDQE
1621 NDELKRQIKR LTSGLQGKPL TDNKQSLIEE LQRKVKKLEN QLEGKVEEVD LKPMKEKNAK
1681 EELIRWEEGK KWQAKIEGIR NKLKEKEGEV FTLTKQLNTL KDLFAKADKE KLTLQRKLKT
1741 TGMTVDQVLG IRALESEKEL EELKKRNLDL ENDILYMRAH QALPRDSVVE DLHLQNRYLQ
1801 EKLHALEKQF SKDTYSKPSQ NQISGIESDD HCQREQELQK ENLKLSSENI ELKFQLEQAN
1861 KDLPRLKNQV RDLKEMCEFL KKEKAEVQRK LGHVRGSGRS GKTIPELEKT IGLMKKVVEK
1921 VQRENEQLKK ASGILTSEKM ANIEQENEKL KAELEKLKAH LGHQLSMHYE SKTKGTEKII
1981 AENERLRKEL KKETDAAEKL RIAKNNLEIL NEKMTVQLEE TGKRLQFAES RGPQLEGADS
2041 KSWKSIVVTR MYETKLKELE TDIAKKNQSI TDLKQLVKEA TEREQKVNKY NEDLEQQIKI
2101 LKHVPEGAET EQGLKRELQV LRLANHQLDK EKAELIHQIE ANKDQSGAES TIPDADQLKE
2161 KIKDLETQLK MSDLEKQHLK EEIKKLKKEL ENFDPSFFEE IEDLKYNYKE EVKKNILLEE
2221 KVKKLSEQLG VELTSPVAAS EEFEDEEESP VNFPIY
SEQ ID NO:77
   INOSINE MONOPHOSPHATE DEHYDROGENASE 1 (IMPDH1)
   EAL24310.1 
1 
61 
121 
181 
241 
 
301 
361 
421 
481 
541 
SEQ ID NO:78
   INOSINE MONOPHOSPHATE DEHYDROGENASE 1 (IMPDH1)
   AAH33622.2 
1 MEGPLTPPPL QGGGAAAVPE PGARQHPGHE TAAQRYSARL LQAGYEPESM ADYLISGGTG
61 YVPEDGLTAQ QLFASADGLT YNDFLILPGF IDFIADEVDL TSALTRKITL KTPLISSPMD
121 TVTEADMAIA MALMGGIGFI HHNCTPEFQA NEVRKVKKFE QGFITDPVVL SPSHTVGDVL
181 EAKMRHGFSG IPITETGTMG SKLVGIVTSR DIDFLAEKDH TTLLSEVMTP RIELVVAPAG
241 VTLKEANEIL QRSKKGKLPI VNDCDELVAI IARTDLKKNR DYPLASKDSQ KQLLCGAAVG
301 TREDDKYRLD LLTQAGVDVI VLDSSQGNSV YQIAMVHYIK QKYPHLQVIG GNVVTAAQAK
361 NLIDAGVDGL RVGMGCGSIC ITQEVMACGR PQGTAVYKVA EYARRFGVPI IADGGIQTVG
421 HVVKALALGA STVMMGSLLA ATTEAPGEYF FSDGVRLKKY RGMGSLDAME KSSSSQKRYF
481 SEGDKVKIAQ GVSGSIQDKG SIQKFVPYLI AGIQHGCQDI GARSLSVLRS MMYSGELKFE
541 KRTMSAQIEG GVHGLHSYEK RLY
SEQ ID NO:79
   INOSINE MONOPHOSPHATE DEHYDROGENASE 1 (IMPDH1), ISOFORM CRA_A
   EAW83649.1 
1 MEGPLTPPPL QGGGAAAVPE PGARQHPGHE TAAQRYSARL LQAGYEPESM ADYLISGGTG
61 YVPEDGLTAQ QLFASADGLT YNDFLILPGF IDFIADEVDL TSALTRKITL KTPLISSPMD
121 TVTEADMAIA MALMGGIGFI HHNCTPEFQA NEVRKKFEQG FITDPVVLSP SHTVGDVLEA
181 KMRHGFSGIP ITETGTMGSK LVGIVTSRDI DFLAEKDHTT LLSEVMTPRI ELVVAPAGVT
241 LKEANEILQR SKKGKLPIVN DCDELVAIIA RTDLKKNRDY PLASKDSQKQ LLCGAAVGTR
301 EDDKYRLDLL TQAGVDVIVL DSSQGNSVYQ IAMVHYIKQK YPHLQVIGGN VVTAAQAKNL
361 IDAGVDGLRV GMGCGSICIT QEVMACGRPQ GTAVYKVAEY ARRFGVPIIA DGGIQTVGHV
421 VKALALGAST VMMGSLLAAT TEAPGEYFFS DGVRLKKYRG MGSLDAMEKS SSSQKRYFSE
481 GDKVKIAQGV SGSIQDKGSI QKFVPYLIAG IQHGCQDIGA RSLSVLRSMM YSGELKFEKR
541 TMSAQIEGGV HGLHSYEKRL Y
SEQ ID NO:80
   INOSINE MONOPHOSPHATE DEHYDROGENASE 1 (IMPDH1), ISOFORM CPA_B
   EAW83650.1 
1 MDRLRRASMA DYLISGGTGY VPEDGLTAQQ LFASADGLTY NDFLILPGFI DFIADEVDLT
61 SALTRKITLK TPLISSPMDT VTEADMAIAM AKFEQGFITD PVVLSPSHTV GDVLEAKMRH
121 GFSGIPITET GTMGSKLVGI VTSRDIDFLA EKDHTTLLSE VMTPRIELVV APAGVTLKEA
181 NEILQRSKKG KLPIVNDCDE LVAIIARTDL KKNRDYPLAS KDSQKQLLCG AAVGTREDDK
241 YRLDLLTQAG VDVIVLDSSQ GNSVYQIAMV HYIKQKYPHL QVIGGNVVTA AQAKNLIDAG
301 VDGLRVGMGC GSICITQEVM ACGRPQGTAV YKVAEYARRF GVPIIADGGI QTVGHVVKAL
361 ALGASTVMMG SLLAATTEAP GEYFFSDGVR LKKYRGMGSL DAMEKSSSSQ KRYFSEGDKV
421 KIAQGVSGSI QDKGSIQKFV PYLIAGIQHG CQDIGARSLS VLRSMMYSGE LKFEKRTMSA
481 QIEGGVHGLH SYEKRLY
SEQ ID NO:81
   INOSINE MONOPHOSPHATE DEHYDROGENASE 1 (IMPDH1), ISOFORM CPA_C
   EAW83651.1 
1 MRHGFSGIPI TETGTMGSKL VGIVTSRDID FLAEKDHTTL LSEVMTPRIE LVVAPAGVTL
61 KEANEILQRS KKGKLPIVND CDELVAIIAR TDLKKNRDYP LASKDSQKQL LCGAAVGTRE
121 DDKYRLDLLT QAGVDVIVLD SSQGNSVYQI AMVHYIKQKY PHLQVIGGNV VTAAQAKNLI
181 DAGVDGLRVG MGCGSICITQ EVMACGRPQG TAVYKVAEYA RRFGVPIIAD GGIQTVGHVV
241 KALALGASTV MMGSLLAATT EAPGEYFFSD GVRLKKYRGM GSLDAMEKSS SSQKRYFSEG
301 DKVKIAQGVS GSIQDKGSIQ KFVPYLIAGI QHGCQDIGAR SLSVLRSMMY SGELKFEKRT
361 MSAQIEGGVH GLHSYEKRLY
SEQ ID NO:82
   INOSINE MONOPHOSPHATE DEHYDROGENASE 1 (IMPDH1), ISOFORM CRA_D
   EAW83652.1 
1 MDRLRRASMA DYLISGGTGY VPEDGLTAQQ LFASADGLTY NDFLILPGFI DFIADEVDLT
61 SALTRKITLK TPLISSPMDT VTEADMAIAM ALMGGIGFIH HNCTPEFQAN EVRKVKKFEQ
121 GFITDPVVLS PSHTVGDVLE AKMRHGFSGI PITETGTMGS KLVGIVTSRD IDFLAEKDHT
181 TLLSEVMTPR IELVVAPAGV TLKEANEILQ RSKKGKLPIV NDCDELVAII ARTDLKKNRD
241 YPLASKDSQK QLLCGAAVGT REDDKYRLDL LTQAGVDVIV LDSSQGNSVY QIAMVHYIKQ
301 KYPHLQVIGG NVVTAAQAKN LIDAGVDGLR VGMGCGSICI TQEVMACGRP QGTAVYKVAE
361 YARRFGVPII ADGGIQTVGH VVKALALGAS TVMMGSLLAA TTEAPGEYFF SDGVRLKKYR
421 GMGSLDAMEK SSSSQKRYFS EGDKVKIAQG VSGSIQDKGS IQKFVPYLIA GIQHGCQDIG
481 ARSLSVLRSM MYSGELKFEK RTMSAQIEGG VHGLHSYEKR LY
SEQ ID NO:83
   RETINAL DEGENERATION 3, GUCY2D REGULATOR (RD3)
   NP_001158160.1 
1 MSLISWLRWN EAPSRLSTRS PAEMVLETLM MELTGQMREA ERQQRERSNA VRKVCTGVDY
61 SWLASTPRST YDLSPIERLQ LEDVCVKIHP SYCGPAILRF RQLLAEQEPE VQEVSQLFRS
121 VLQEVLERMK QEEEAHKLTR QWSLRPRGSL ATFKTRARIS PFASDIRTIS EDVERDTPPP
181 LRSWSMPEFR APKAD
SEQ ID NO:84
   RETINOL DEHYDROGENASE 12 (RDH12)
   Q96NR8.3 
1 
61 
121 
181 
241 
301 
SEQ ID NO:85
   RETINOL DEHYDROGENASE 12 (RDH12), ISOFORM 1, PARTIAL
   ALQ34323.1 
1 
61 
121 
181 
241 
301 
SEQ ID NO:86
   RETINOL DEHYDROGENASE 12 (RDH12), ISOFORM 4, PARTIAL
   ALQ34324.1 
1 MLVTLGLLTS FFSFLYMVAP SIRKFFAGGV CRTNVQLPGK VVVITGANTG IGKETARELA
61 SRGARVYIAC RDVLKGESAA SEIRVDTKNS QVLVRKLDLS DTKSIRAFAE GFLAEEKQLH
121 ILINNAGVMM CPYSKTADGF ETHLGVNHLG TGVTTYAVHP GVVRSELVRH SSLLCLLWRL
181 FSPFVKTARE GAQTSLHCAL AEGLEPLSGK YFSDCKRTWV SPRARNNKTA ERLWNVSCEL
241 LGIRWE
SEQ ID NO:87
   RETINOL DEHYDROGENASE 12 (RDH12), PRECURSOR
   NP_689656.2 
1 
61 
121 
 
181 
241 
301 
SEQ ID NO:88
   LECITHIN RETINOL ACYLTRANSFERASE (LRAT)
   AAD13529.1 
1 MKNPMLEVVS LLLEKLLLIS NFTLFSSGAA GKDKGRNSFY ETSSFHRGDV LEVPRTHLTH
61 YGIYLGDNRV AHMMPDILLA LTDDMGRTQK VVSNKRLILG VIVKVASIRV DTVEDFAYGA
121 NILVNHLDES LQKKALLNEE VARRAEKLLG FTPYSLLWNN CEHFVTYCRY GTPISPQSDK
181 FCETVKIIIR DQRSVLASAV LGLASIVCTG LVSYTTLPAI FIPFFLWMAG
SEQ ID NO:89
   TUBBY LIKE PROTEIN 1 (TULP1)
   AAB97966.1 
1 MPLRDETLRE VWASDSGHEE ESLSPEAPRR PKQRPAPAQR LRKKRTEAPE SPCPTGSKPR
61 KPGAGRRGRP REEPSPDPAQ ARAPQTVYAR FLRDPEAKKR DPRETFLVAR APDAEDEEEE
121 EEEDEEDEEE EAEEKKEKIL LPPKKPLREK SSADLKERRA KAQGPRGDLG SPDPPPKPLR
181 VRNKEAPAGE GTKMRKTKKK GSGEADKDPS GSPASARKSP AAMFLVGEGS PDKKALKKKG
241 TPKGARKEEE EEEEAATVIK NSNQKGKAKG KGKKKAKEER APSPPVEVDE PREFVLRPAP
301 QGRTVRCRLT RDKKGMDRGM YPSYFLHLDT EKKVFLLAGR KRKRSKTANY LISIDPTNLS
361 RGGENFIGKL RSNLLGNRFT VFDNGQNPQR GYSTNVASLR QELAAVIYET NVLGFRGPRR
421 MTVIIPGMSA ENERVPIRPR NASDGLLVRW QNKTLESLIE LHNKPPVWND DSGSYTLNFQ
481 GRVTQASVKN FQIVHADDPD YIVLQFGRVA EDAFTLDYRY PLCALQAFAI ALSSFDGKLA
541 CE
SEQ ID NO:90
   TUBBY LIKE PROTEIN 1 (TULP1), ISOFORM CRA_A
   EAX03839.1 
1 
61 
121 
181 
241 
301 
361 
421 
481 
SEQ ID NO:91
   TUBBY LIKE PROTEIN 1 (TULP1), ISOFORM CPA_B
   EAX03840.1 
1 
61 
121 
181 
241 
301 
361 
421 
481 
SEQ ID NO:92
   TUBBY LIKE PROTEIN 1 (TULP1), HOMOLOG ISOFORM A
   NP_003311.2 
1 MGARTPLPSF WVSFFAETGI LFPGGTPWPM GSQHSKQHRK PGPLKRGHRR DRRTTRRKYW
61 KEGREIARVL DDEGRNLRQQ KLDRQRALLE QKQKKKRQEP LMVQANADGR PRSRRARQSE
 
121 EQAPLVESYL SSSGSTSYQV QEADSLASVQ LGATRPTAPA SAKRTKAAAT AGGQGGAARK
181 EKKGKHKGTS GPAALAEDKS EAQGPVQILT VGQSDHAQDA GETAAGGGER PSGQDLRATM
241 QRKGISSSMS FDEDEEDEEE NSSSSSQLNS NTRPSSATSR KSVREAASAP SPTAPEQPVD
301 VEVQDLEEFA LRPAPQGITI KCRITRDKKG MDRGMYPTYF LHLDREDGKK VFLLAGRKRK
361 KSKTSNYLIS VDPTDLSRGG DSYIGKLRSN LMGTKFTVYD NGVNPQKASS STLESGTLRQ
421 ELAAVCYETN VLGFKGPRKM SVIVPGMNMV HERVSIRPRN EHETLLARWQ NKNTESIIEL
481 QNKTPVWNDD TQSYVLNFHG RVTQASVKNF QIIHGNDPDY IVMQFGRVAE DVFTMDYNYP
541 LCALQAFAIA LSSFDSKLAC E
SEQ ID NO:93
   TUBBY LIKE PROTEIN 1 (TULP1)
   NP_813977.1 
1 MTSKPHSDWI PYSVLDDEGR NLRQQKLDRQ RALLEQKQKK KRQEPLMVQA NADGRPRSRR
61 ARQSEEQAPL VESYLSSSGS TSYQVQEADS LASVQLGATR PTAPASAKRT KAAATAGGQG
121 GAARKEKKGK HKGTSGPAAL AEDKSEAQGP VQILTVGQSD HAQDAGETAA GGGERPSGQD
181 LRATMQRKGI SSSMSFDEDE EDEEENSSSS SQLNSNTRPS SATSRKSVRE AASAPSPTAP
241 EQPVDVEVQD LEEFALRPAP QGITIKCRIT RDKKGMDRGM YPTYFLHLDR EDGKKVFLLA
301 GRKRKKSKTS NYLISVDPTD LSRGGDSYIG KLRSNLMGTK FTVYDNGVNP QKASSSTLES
361 GTLRQELAAV CYETNVLGFK GPRKMSVIVP GMNMVHERVS IRPRNEHETL LARWQNKNTE
421 SIIELQNKTP VWNDDTQSYV LNFHGRVTQA SVKNFQIIHG NDPDYIVMQF GRVAEDVFTM
481 DYNYPLCALQ AFAIALSSFD SKLACE
SEQ ID NO:94
   POTASSIUM VOLTAGE-GATED CHANNEL SUBFAMILY J MEMBER 13 (KCNJ13)
   O60928.1 
1 
61 
121 
181 
241 
301 
SEQ ID NO:95
   POTASSIUM VOLTAGE-GATED CHANNEL SUBFAMILY J MEMBER 13 (KCNJ13)
   AAH37290.1 
1 MDSSNCKVIA PLLSQRYRRM VTKDGHSTLQ MDGAQRGLAY LRDAWGILMD MRWRWMMLVF
61 SASFVVHWLV FAVLWYVLAE MNGDLELDHD APPENHTICV KYITSFTAAF SFSLETQLTI
121 GYGTMFPSGD CPSAIALLAI QMLLGLMLEA FITGAFVAKI ARPKNRAFSI RFTDIAVVAH
181 MDGKPNLIFQ VANTRPSPLT SVRVSAVLYQ ERENGKLYQT SVDFHLDGIS SDECPFFIFP
241 LTYYHSITPS SPLATLLQHE NPSHFELVVF LSAMQEGTGE ICQRRTSYLQ SEIMLHHCFA
301 SLLTRGSKCE YQIKMENFDK TVPEFPTPLV SKSPNRTDLD IHINGQSIDN FQISETGLTE
SEQ ID NO:96
   MITOCHONDRIALLY ENCODED NADH DEHYDROGENASE 1 (MT-ND1)
   P03886.1 
1 MPMANLLLLI VPILIAMAFL MLTERKILGY MQLRKGPNVV GPYGLLQPFA DAMKLFTKEP
61 LKPATSTITL YITAPTLALT IALLLWTPLP MPNPLVNLNL GLLFILATSS LAVYSILWSG
121 WASNSNYALI GALRAVAQTI SYEVTLAIIL LSTLLMSGSF NLSTLITTQE HLWLLLPSWP
181 LAMMWFISTL AETNRTPFDL AEGESELVSG FNIEYAAGPF ALFFMAEYTN IIMMNTLTTT
241 IFLGTTYDAL SPELYTTYFV TKTLLLTSLF LWIRTAYPRF RYDQLMHLLW KNFLPLTLAL
301 LMWYVSMPIT ISSIPPQT
SEQ ID NO:97
   MITOCHONDRIALLY ENCODED NADH DEHYDROGENASE 4 (MT-ND4)
   ACT53103.1 
1 MLKLIVPTIM LLPLTWLSKK HMIWINTTTH SLIISIIPLL FFNQINNNLF SCSPTFSSDP
61 LTTPLLMLTT WLLPLTIMAS QRHLSSEPLS RKKLYLSMLI SLQISLIMTF TATELIMFYI
 
121 FFETTLIPTL AIITRWGNQP ERLNAGTYFL FYTLVGSLPL LIALIYTHNT LGSLNILLLT
181 LTAQELSNSW ANNLMWLAYT MAFMVKMPLY GLHLWLPKAH VEAPIAGSMV LAAVLLKLGG
241 YGMMRLTLIL NPLTKHMAYP FLVLSLWGMI MTSSICLRQT DLKSLIAYSS ISHMALVVTA
301 ILIQTPWSFT GAVILMIAHG LTSSLLFCLA NSNYERTHSR IMILSQGLQT LLPLMAFWWL
361 LASLANLALP PTINLLGELS VLVTTFSWSN ITLLLTGLNM LVTALYSLYM FTTTQWGSLT
421 HHINNMKPSF TRENTLMFMH LSPILLLSLN PDIITGFSS
SEQ ID NO:98
   MITOCHONDRIALLY ENCODED NADH DEHYDROGENASE 6 (MT-ND6)
   ACT53105.1 
1 MMYALFLLSV GLVMGFVGFS SKPSPIYGGL VLIVSGVVGC VIILNFGGGY MGLMVFLIYL
61 GGMMVVFGYT TAMAIEEYPE AWGSGVEVLV SVLVGLAMEV GLVLWVKEYD GVVVVVNFNS
121 VGSWMIYEGE GSGLIREDPI GAGALYDYGR WLVVVTGWTL FVGVYIVIEI ARGN
SEQ ID NO:99
   ANGIOTENSIN I CONVERTING ENZYME (ACE)
   P12821.1 
1 
61 
121 
181 
241 
301 
361 
421 
481 
541 
601 
661 
721 
781 
841 
901 
961 
1021 
1081 
1141 
1201 
1261 
SEQ ID NO:100
   ANGIOTENSIN I CONVERTING ENZYME (ACE), ISOFORM 1 PRECURSOR
   NP_000780.1 
1 
61 
121 
181 
241 
301 
361 
421 
481 
541 
601 
661 
721 
781 
 
841 
901 
961 
1021 
1081 
1141 
1201 
1261 
SEQ ID NO:101
   ANGIOTENSIN I CONVERTING ENZYME (ACE), ISOFORM 2 PRECURSOR
   NP_690043.1 
1 MGQGWATAGL PSLLFLLLCY GHPLLVPSQE ASQQVTVTHG TSSQATTSSQ TTTHQATAHQ
61 TSAQSPNLVT DEAEASKFVE EYDRTSQVVW NEYAEANWNY NTNITTETSK ILLQKNMQIA
121 NHTLKYGTQA RKFDVNQLQN TTIKRIIKKV QDLERAALPA QELEEYNKIL LDMETTYSVA
181 TVCHPNGSCL QLEPDLTNVM ATSRKYEDLL WAWEGWRDKA GRAILQFYPK YVELINQAAR
241 LNGYVDAGDS WRSMYETPSL EQDLERLFQE LQPLYLNLHA YVRRALHRHY GAQHINLEGP
301 IPAHLLGNMW AQTWSNIYDL VVPFPSAPSM DTTEAMLKQG WTPRRMFKEA DDFFTSLGLL
361 PVPPEFWNKS MLEKPTDGRE VVCHASAWDF YNGKDFRIKQ CTTVNLEDLV VAHHEMGHIQ
421 YFMQYKDLPV ALREGANPGF HEAIGDVLAL SVSTPKHLHS LNLLSSEGGS DEHDINFLMK
481 MALDKIAFIP FSYLVDQWRW RVFDGSITKE NYNQEWWSLR LKYQGLCPPV PRTQGDFDPG
541 AKFHIPSSVP YIRYFVSFII QFQFHEALCQ AAGHTGPLHK CDIYQSKEAG QRLATAMKLG
601 FSRPWPEAMQ LITGQPNMSA SAMLSYFKPL LDWLRTENEL HGEKLGWPQY NWTPNSARSE
661 GPLPDSGRVS FLGLDLDAQQ ARVGQWLLLF LGIALLVATL GLSQRLFSIR HRSLHRHSHG
721 PQFGSEVELR HS
SEQ ID NO:102
   INTERLEUKIN 10 (IL10)
   CAG46790.1 
  1 MHSSALLCCL VLLTGVRASP GQGTQSENSC THFPGNLPNM LRDLRDAFSR VKTFFQMKDQ
 61 LDNLLLKESL LEDFKGYLGC QALSEMIQFY LEEVMPQAEN QDPDIKAHVN SLGENLKTLR
121 LRLRRCHRFL PCENKSKAVE QVKNAFNKLQ EKGIYKAMSE FDIFINYIEA YMTMKIRN
SEQ ID NO:103
   RAB ESCORT PROTEIN 1 (CHM)
   EAW98559.1 
1 
61 
121 
181 
241 
301 
361 
421 
481 
541 
601 
SEQ ID NO:104
   RETINOSCHISIN (RS1)
   NP_000321.1 
  1 
 61 
121 
181 
SEQ ID NO:105
   RETINOSCHISIN (RS1), PARTIAL
   ABK40506.1
   1 VFYGNSDRTS TVQNLLRPPI ISRFIRLIPL GCHVRIAIRM ELLECVSKCA
SEQ ID NO:106
   BARDET-BIEDL SYNDROME 1 (BBS1)
   AAM92770.1 
1 
61 
121 
181 
241 
301 
361 
421 
481 
541 
SEQ ID NO:107
   BARDET-BIEDL SYNDROME 2 (BBS2)
   AAH14140.1 
1 MLLPVFTLKL RHKISPRMVA IGRYDGTHPC LAAATQTGKV FIHNPHTRNQ HVSASRVFQS
61 PLESDVSLLN INQAVSCLTA GVLNPELGYD ALLVGTQTNL LAYDVYNNSD LFYREVADGA
121 NVVVLGTLGD ISSPLAIIGG NCALQGFNHE GSDLFWTVTG DNVNSLALCD FDGDGKKELL
181 VGSEDFDIRV FKEDEIVAEM TETEIVTSLC PMYGSRFGYA LSNGTVGVYD KTSRYWRIKS
241 KNHAMSIHAF DLNSDGVNEL ITGWSNGKVD ARSDRTGEVI FKDNFSSAIA GVVEGDYRMD
301 GHIQLICCSV DGEIRGYLPG TAEMRGNLMD TSAEQDLIRE LSQKKQNLLL ELRNYEENAK
361 AELASPLNEA DGHRGIIPAN TRLHTTLSVS LGNETQTAHT ELRISTSNDT IIRAVLIFAE
421 GIFTGESHVV HPSIHNLSSS ICIPIVPPKD VPVDLHLKAF VGYRSSTQFH VFESTRQLPR
481 FSMYALTSLD PASEPISYVN FTIAERAQRV VVWLGQNFLL PEDTHIQNAP FQVCFTSLRN
541 GGHLHIKIKL SGEITINTDD IDLAGDIIQS MASFFAIEDL QVEADFPVYF EELRKVLVKV
601 DEYHSVHQKL SADMADHSNL IRSLLVGAED ARLMRDMKTM KSRYMELYDL NRDLLNGYKI
661 RCNNHTELLG NLKAVNQAIQ RAGRLRVGKP KNQVITACRD AIRSNNINTL FKIMRVGTAS
721 S
SEQ ID NO:108
   ADP RIBOSYLATION FACTOR LIKE GTPASE 6 (ARL6), ISOFORM BB3SL
   NP_001310442.1 
1 MGLLDRLSVL LGLKKKEVHV LCLGLDNSGK TTIINKLKPS NAQSQNILPT IGFSIEKFKS
61 SSLSFTVFDM SGQGRYRNLW EHYYKEGQAI IFVIDSSDRL RMVVAKEELD TLLNHPDIKH
121 RRIPILFFAN KMDLRDAVTS VKVSQLLCLE NIKDKPWHIC ASDAIKGEGL QEGVDWLQEK
181 TIQSDPDCED MKR
SEQ ID NO:109
   ADP RIBOSYLATION FACTOR LIKE GTPASE 6 (ARL6), ISOFORM 1
   NP_001265222.1 
1 
61 
121 
181 
SEQ ID NO:110
   ADP RIBOSYLATION FACTOR LIKE GTPASE 6 (ARL6), ISOFORM 2
   NP_001310443.1 
1 MGLLDRLSVL LGLKKKEVHV LCLGLDNSGK TTIINKLKPS NAQSQNILPT IGFSIEKFKS
61 SSLSFTVFDM SGQGRYRNLW EHYYKEGQAI IFVIDSSDRL RMVVAKEELD TLLNHPDIKH
 121 RRIPILFFAN KMDLRDAVTS VKVSQLLCLE NIKDKPWHI
SEQ ID NO:111
   BARDET-BIEDL SYNDROME 4 (BBS4)
   AAH27624.1 
1 MAEERVATRT QFPVSTESQK PRQKKAPEFP ILEKQNWLIH LHYIRKDYEA CKAVIKEQLQ
61 ETQGLCEYAI YVQALIFRLE GNIQESLELF QTCAVLSPQS ADNLKQVARS LFLLGKHKAA
121 IEVYNEAAKL NQKDWEISHN LGVCYIYLKQ FNKAQDQLHN ALNLNRHDLT YIMLGKIHLL
181 EGDLDKAIEV YKKAVEFSPE NTELLTTLGL LYLQLGIYQK AFEHLGNALT YDPTNYKAIL
241 AAGSMMQTHG DFDVALTKYR VVACAVPESP PLWNNIGMCF FGKKKYVAAI SCLKRANYLA
301 PFDWKILYNL GLVHLTMQQY ASAFHFLSAA INFQPKMGEL YMLLAVALTN LEDTENAKRA
361 YAEAVHLDKC NPLVNLNYAV LLYNQGEKKN ALVQYQEMEK KVSLLKDNSS LEFDSEMVEM
421 AQKLGAALQV GEALVWTKPV KDPKSKHQTT STSKPASFQQ PLGSNQALGQ AMSSAAAYRT
481 LPSGAGGTSQ FTKPPSLPLE PEPAVESSPT ETSEQIREK
SEQ ID NO:112
   BARDET-BIEDL SYNDROME 4 (BBS4), ISOFORM 1
   NP_149017.2 
  1 MAEERVATRT QFPVSTESQK PRQKKAPEFP ILEKQNWLIH LHYIRKDYEA CKAVIKEQLQ
 61 ETQGLCEYAI YVQALIFRLE GNIQESLELF QTCAVLSPQS ADNLKQVARS LFLLGKHKAA
121 IEVYNEAAKL NQKDWEISHN LGVCYIYLKQ FNKAQDQLHN ALNLNRHDLT YIMLGKIHLL
181 EGDLDKAIEV YKKAVEFSPE NTELLTTLGL LYLQLGIYQK AFEHLGNALT YDPTNYKAIL
241 AAGSMMQTHG DFDVALTKYR VVACAVPESP PLWNNIGMCF FGKKKYVAAI SCLKRANYLA
301 PFDWKILYNL GLVHLTMQQY ASAFHFLSAA INFQPKMGEL YMLLAVALTN LEDIENAKRA
361 YAEAVHLDKC NPLVNLNYAV LLYNQGEKKN ALAQYQEMEK KVSLLKDNSS LEFDSEMVEM
421 AQKLGAALQV GEALVWTKPV KDPKSKHQTT STSKPASFQQ PLGSNQALGQ AMSSAAAYRT
481 LPSGAGGTSQ FTKPPSLPLE PEPAVESSPT ETSEQIREK
SEQ ID NO:113
   BARDET-BIEDL SYNDROME 4 (BBS4), ISOFORM 2
   NP_001239607.1 
1 MLGKIHLLEG DLDKAIEVYK KAVEFSPENT ELLTTLGLLY LQLGIYQKAF EHLGNALTYD
61 PTNYKAILAA GSMMQTHGDF DVALTKYRVV ACAVPESPPL WNNIGMCFFG KKKYVAAISC
121 LKRANYLAPF DWKILYNLGL VHLTMQQYAS AFHFLSAAIN FQPKMGELYM LLAVALTNLE
181 DIENAKRAYA EAVHLDKCNP LVNLNYAVLL YNQGEKKNAL AQYQEMEKKV SLLKDNSSLE
241 FDSEMVEMAQ KLGAALQVGE ALVWTKPVKD PKSKHQTTST SKPASFQQPL GSNQALGQAM
301 SSAAAYRTLP SGAGGTSQFT KPPSLPLEPE PAVESSPTET SEQIREK
SEQ ID NO:114
   BARDET-BIEDL SYNDROME 4 (BBS4), ISOFORM 3
   NP_001307594.1 
1 
61 
121 
181 
241 
301 
361 
421 
481 
SEQ ID NO:115
   BARDET-BIEDL SYNDROME 5 (BBS5)
   NP_689597.1 
1 
61 
121 
 
181 
241 
301 
SEQ ID NO:116
   BARDET-BIEDL SYNDROME 5 (BBS5), ISOFORM 1
   AAT08182.1 
1 
61 
121 
181 
241 
301 
SEQ ID NO:117
   BARDET-BIEDL SYNDROME 5 (BBS5), ISOFORM 2
   AAT08183.1 
1 MSVLDALWED RDVRFDLSAQ QMKTRPGEVL IDCLDSIEDT KGNNGDRGRL LVTNLRILWH
61 SLALSRVNVS VGYNCILNIT TRTANSKLRG QTEALYILTK CNSTRFEFIF TNLVPGSPRL
121 FTSVMAVHRA YETSKMYRDF KLRSALIQNK QLRLLPQEHV YDKINGVWNL SSDQGNLGTF
181 FITNVRIVWH ANMNDSFNVS IPYLQISGGY VLGFKIDPVE KLQESVKEIN SLHKVYSASP
241 IFGVDYEMEE KPQPLEALTV EQIQDDVEID SDGHTDAFVA YFADGNKQQD REPVFSEELG
301 LAIEKLKDGF TLQGLWEVMS
SEQ ID NO:118
   MCKUSICK-KAUFMAN SYNDROME (MKKS)
   AAH28973.1 
1 
61 
121 
181 
241 
301 
361 
421 
481 
541 
SEQ ID NO:119
   MCKUSICK-KAUFMAN SYNDROME (MKKS), ISOFORM CRA_A
   EAX10343.1 
1 MSLRNLWRDY KVLVVMVPLV GLIHLGWYRI KSSPVFQIPK NDDIPEQDSL GLSNLQKSQI
61 QGK
SEQ ID NO:120
   MCKUSICK-KAUFMAN SYNDROME (MKKS), ISOFORM CPA_B
   EAX10344.1 
1 
61 
121 
181 
241 
301 
361 
421 
481 
541 
SEQ ID NO:121
   BARDET-BIEDL SYNDROME 7 (BBS7)
   AAH32691.1 
1 MDLILNRMDY LQVGVTSQKT MKLIPASRHR ATQKVVIGDH DGVVMCFGMK KGEAAAVFKT
61 LPGPKIARLE LGGVINTPQE KIFIAAASEI RGFTKRGKQF LSFETNLTES IKAMHISGSD
121 LFLSASYIYN HYCDCKDQHY YLSGDKINDV ICLPVERLSR ITPVLACQDR VLRVLQGSDV
181 MYAVEVPGPP TVLALHNGNG GDSGEDLLFG TSDGKLALIQ ITTSKPVRKW EIQNEKKRGG
241 ILCIDSFDIV GDGVKDLLVG RDDGMVEVYS FDNANEPVLR FDQMLSESVT SIQGGCVGKD
301 SYDEIVVSTY SGWVTGLTTE PIHKESGPGE ELKINQEMQN KISSLRNELE HLQYKVLQER
361 ENYQQSSQSS KAKSAVPSFG INDKFTLNKD DASYSLILEV QTAIDNVLIQ SDVPIDLLDV
421 DKNSAVVSFS SCDSESNDNF LLATYRCQAD TTRLELKIRS IEGQYGTLQA YVTPRIQPKT
481 CQVRQYHIKP LSLHQRTHFI DHDRPMNTLT LTGQFSFAEV HSWVVFCLPE VPEKPPAGEC
541 VTFYFQNTFL DTQLESTYRK GEGVFKSDNI STISILKDVL SKEATKRKIN LNISYEINEV
601 SVKHTLKLIH PKLEYQLLLA KKVQLIDALK ELQIHEGNTN FLIPEYHCIL EEADHLQEEY
661 KKQPAHLERL YG
SEQ ID NO:122
   BARDET-BIEDL SYNDROME 7 (BBS7), ISOFORM A
   NP_789794.1 
1 
61 
121 
181 
241 
301 
361 
421 
481 
541 
601 
661 
SEQ ID NO:123
   BARDET-BIEDL SYNDROME 7 (BBS7), ISOFORM B
   NP_060660.2 
1 MDLILNRMDY LQVGVTSQKT MKLIPASRHR ATQKVVIGDH DGVVMCFGMK KGEAAAVFKT
61 LPGPKIARLE LGGVINTPQE KIFIAAASEI RGFTKRGKQF LSFETNLTES IKAMHISGSD
121 LFLSASYIYN HYCDCKDQHY YLSGDKINDV ICLPVERLSR ITPVLACQDR VLRVLQGSDV
181 MYAVEVPGPP TVLALHNGNG GDSGEDLLFG TSDGKLALIQ ITTSKPVRKW EIQNEKKRGG
241 ILCIDSFDIV GDGVKDLLVG RDDGMVEVYS FDNANEPVLR FDQMLSESVT SIQGGCVGKD
301 SYDEIVVSTY SGWVTGLTTE PIHKESGPGE ELKINQEMQN KISSLRNELE HLQYKVLQER
361 ENYQQSSQSS KAKSAVPSFG INDKFTLNKD DASYSLILEV QTAIDNVLIQ SDVPIDLLDV
421 DKNSAVVSFS SCDSESNDNF LLATYRCQAD TTRLELKIRS IEGQYGTLQA YVTPRIQPKT
481 CQVRQYHIKP LSLHQRTHFI DHDRPMNTLT LTGQFSFAEV HSWVVFCLPE VPEKPPAGEC
541 VTFYFQNTFL DTQLESTYRK GEGVFKSDNI STISILKDVL SKEATKRKIN LNISYEINEV
601 SVKHTLKLIH PKLEYQLLLA KKVQLIDALK ELQIHEGNTN FLIPEYHCIL EEADHLQEEY
661 KKQPAHLERL YG
SEQ ID NO:124
   TETRATRICOPEPTIDE REPEAT DOMAIN 8 (TTC8)
   AAH95433.1 
1 MSSEMEPLLL AWSYFRRRKF QLCADLCTQM LEKSPYDQAA WILKARALTE MVYIDEIDVD
61 QEGIAEMMLD ENAIAQVPRP GTSLKLPGTN QTGGPSQAVR PITQAGRPIT GFLRPSTQSG
121 RPGTMEQAIR TPRTAYTARP ITSSSGRFVR LGTASMLTSP DGPFINLSRL NLTKYSQKPK
181 LAKALFEYIF HHENDVKTAL DLAALSTEHS QYKDWWWKVQ IGKCYYRLGM YREAEKQFKS
241 ALKQQEMVDT FLYLAKVYVS LDQPVTALNL FKQGLDKFPG EVTLLCGIAR IYEEMNNMSS
 
301 AAEYYKEVLK QDNTHVEAIA CIGSNHFYSD QPEIALRFYR RLLQMGIYNG QLFNNLGLCC
361 FYAQQYDMTL TSFERALSLA ENEEEAADVW YNLGHVAVGI GDTNLAHQCF RLALVNNNNH
421 AEAYNNLAVL EMRKGHVEQA RALLQTASSL APHMYEPHFN FATISDKIGD LQRSYVAAQK
481 SEAAFPDHVD TQHLIKQLRQ HFAML
SEQ ID NO:125
   TETRATRICOPEPTIDE REPEAT DOMAIN 8 (TTC8), ISOFORM A
   NP_653197.2 
1 MSSEMEPLLL AWSYFRRRKF QLCADLCTQM LEKSPYDQEP DPELPVHQAA WILKARALTE
61 MVYIDEIDVD QEGIAEMMLD ENAIAQVPRP GTSLKLPGTN QTGGPSQAVR PITQAGRPIT
121 GFLRPSTQSG RPGTMEQAIR TPRTAYTARP ITSSSGRFVR LGTASMLTSP DGPFINLSRL
181 NLTKYSQKPK LAKALFEYIF HHENDVKTAL DLAALSTEHS QYKDWWWKVQ IGKCYYRLGM
241 YREAEKQFKS ALKQQEMVDT FLYLAKVYVS LDQPVTALNL FKQGLDKFPG EVTLLCGIAR
301 IYEEMNNMSS AAEYYKEVLK QDNTHVEAIA CIGSNHFYSD QPEIALRFYR RLLQMGIYNG
361 QLFNNLGLCC FYAQQYDMTL TSFERALSLA ENEEEAADVW YNLGHVAVGI GDTNLAHQCF
421 RLALVNNNNH AEAYNNLAVL EMRKGHVEQA RALLQTASSL APHMYEPHFN FATISDKIGD
481 LQRSYVAAQK SEAAFPDHVD TQHLIKQLRQ HFAML
SEQ ID NO:126
   TETRATRICOPEPTIDE REPEAT DOMAIN 8 (TTC8), ISOFORM B
   NP_938051.1 
1 MSSEMEPLLL AWSYFRRRKF QLCADLCTQM LEKSPYDQAA WILKARALTE MVYIDEIDVD
61 QEGIAEMMLD ENAIAQVPRP GTSLKLPGTN QTGGPSQAVR PITQAGRPIT GFLRPSTQSG
121 RPGTMEQAIR TPRTAYTARP ITSSSGRFVR LGTASMLTSP DGPFINLSRL NLTKYSQKPK
181 LAKALFEYIF HHENDVKTAL DLAALSTEHS QYKDWWWKVQ IGKCYYRLGM YREAEKQFKS
241 ALKQQEMVDT FLYLAKVYVS LDQPVTALNL FKQGLDKFPG EVTLLCGIAR IYEEMNNMSS
301 AAEYYKEVLK QDNTHVEAIA CIGSNHFYSD QPEIALRFYR RLLQMGIYNG QLFNNLGLCC
361 FYAQQYDMTL TSFERALSLA ENEEEAADVW YNLGHVAVGI GDTNLAHQCF RLALVNNNNH
421 AEAYNNLAVL EMRKGHVEQA RALLQTASSL APHMYEPHFN FATISDKIGD LQRSYVAAQK
481 SEAAFPDHVD TQHLIKQLRQ HFAML
SEQ ID NO:127
   BARDET-BIEDL SYNDROME 9 (BBS9)
   AAI03832.1 
1 MSLFKARDWW STILGDKEEF DQGCLCLANV DNSGNGQDKI IVGSFMGYLR IFSPHPAKTG
61 DGAQAEDLLL EVDLRDPVLQ VEVGKFVSGT EMLHLAVLHS RKLCVYSVSG TLGNVEHGNQ
121 CQMKLMYEHN LQRTACNMTY GSFGGVKGRD LICIQSMDGM LMVFEQESYA FGRFLPGFLL
181 PGPLAYSSRT DSFLTVSSCQ QVESYKYQVL AFATDADKRQ ETEQQKLGSG KRLVVDWTLN
241 IGEQALDICI VSFNQSASSV FVLGERNFFC LKDNGQIRFM KKLDWSPSCF LPYCSVSEGT
301 INTLIGNHNN MLHIYQDVTL KWATQLPHIP VAVRVGCLHD LKGVIVTLSD DGHLQCSYLG
361 TDPSLFQAPN VQSRELNYDE LDVEMKELQK IIKDVNKSQG VWPMTEREDD LNVSVVVSPN
421 FDSVSQATDV EVGTDLVPSV TVKVTLQNRV ILQKAKLSVY VQPPLELTCD QFTFEFMTPD
481 LTRTVSFSVY LKRSYTPSEL EGNAVVSYSR PTDRNPDGIP RVIQCKFRLP LKLICLPGQP
541 SKTASHKITI DTNKSPVSLL SLFPGFASQS DDDQVNVMGF HFLGGARITV LASKTSQRYR
601 IQSEQFEDLW LITNELILRL QEYFEKQGVK DFACSFSGSI PLQEYFELID HHFELRINGE
661 KLEELLSERA VQFRAIQRRL LARFKDKTPA PLQHLDTLLD GTYKQVIALA DAVEENQGNL
721 FQSFTRLKSA THLVILLIAL WQKLSADQVA ILEAAFLPLQ EDTQELGWEE TVDAAISHLL
781 KTCLSKSSKE QALNLNSQLN IPKDTSQLKK HITLLCDRLS KGGRLCLSTD AAAPQTMVMP
841 GGCTTIPESD LEERSVEQDS TELFTNHRHL TAETPRPEVS PLQGVSE
SEQ ID NO:128
   BARDET-BIEDL SYNDROME 10 (BBS10)
   AAH26355.2 
1 
61 
121 
181 
 
241 
301 
361 
421 
481 
541 
601 
661 
721 
SEQ ID NO:129
   TRIPARTITE MOTIF CONTAINING 32 (TRIM32)
   AAH03154.1 
1 
61 
121 
181 
241 
301 
361 
421 
481 
541 
601 
SEQ ID NO:130
   TRIPARTITE MOTIF CONTAINING 32 (TRIM32), ISOFORM CRA_A
   EAW87447.1 
1 
61 
121 
181 
241 
301 
361 
421 
481 
541 
601 
SEQ ID NO:131
   BARDET-BIEDL SYNDROME 12 (BBS12)
   AAH55426.1 
1 MVMACRVVNK RRHMGLQQLS SFAETGRTFL GPLKSSKFII DEECHESVLI SSTVRLLESL
61 DLTSAVGQLL NEAVQAQNNT YRTGISTLLF LVGAWSSAVE ECLHLGVPIS IIVSVMSEGL
121 NFCSEEVVSL HVPVHNIFDC MDSTKTFSQL ETFSVSLCPF LQVPSDTDLI EELHGLKDVA
181 SQTLTISNLS GRPLRSYELF KPQTKVEADN NTSRTLKNSL LADTCCRQSI LIHSRHFNRT
241 DNTEGVSKPD GFQEHVTATH KTYRCNDLVE LAVGLSHGDH SSMKLVEEAV QLQYQNACVQ
301 QGNCTKPFMF DISRIFTCCL PGLPETSSCV CPGYITVVSV SNNPVIKELQ NQPVRIVLIE
361 GDLTENYRHL GFNKSANIKT VLDSMQLQED SSEELWANHV LQVLIQFKVN LVLVQGNVSE
421 RLIEKCINSK RLVIGSVNGS VMQAFAEAAG AVQVAYITQV NEDCVGNGVC VTFWRSSPLD
481 VVDRNNRIAI LLKTEGINLV TAVLTNPVTA QMQIKEDRFW TCAYRLYYAL KEEKVFLGGG
541 AVEFLCLSCL HILAEQSLKK ENHACSGWLH NTSSWLASSL AIYRPTVLKF LANGWQKYLS
601 TLLYNTANYS SEFEVSTYIQ HHLQNATDSG SPSSYILNEY SKLNSRIFNS DISNKLEQIP
661 RVYDVVTPKI EAWRRALDLV LLVLQTDSEI ITGHGHTQIN SQELTGFLFL
SEQ ID NO:132
   MKS TRANSITION ZONE COMPLEX SUBUNIT 1 (MKS1)
   NP_060247.2 
1 
61 
121 
181 
241 
301 
361 
421 
481 
541 
SEQ ID NO:133
   WD REPEAT CONTAINING PLANAR CELL POLARITY EFFECTOR (WDPCP), HOMOLOG ISOFORM 1
   NP_001036157.1 
1 MFSSLHSALL TDSFIILSFL AQNKLCFIQF TKKMESSDVN KRLEKLSALD YKIFYYEIPG
61 PINKTTERHL AINCVHDRVV CWWPLVNDDA WPWAPISSEK DRANLLLLGY AQGRLEVLSS
121 VRTEWDPLDV RFGTKQPYQV FTVEHSVSVD KEPMADSCIY ECIRNKIQCV SVTRIPLKSK
181 AISCCRNVTE DKLILGCEDS SLILYETHRR VTLLAQTELL PSLISCHPSG AILLVGSNQG
241 ELQIFDMALS PINIQLLAED RLPRETLQFS KLFDASSSLV QMQWIAPQVV SQKGEGSDIY
301 DLLFLRFERG PLGVLLFKLG VFTRGQLGLI DIIFQYIHCD EIYEAINILS SMNWDTLGHQ
361 CFISMSAIVN HLLRQKLTPE REAQLETSLG TFYAPTRPLL DSTILEYRDQ ISKYARRFFH
421 HLLRYQRFEK AFLLAVDVGA RDLFMDIHYL ALDKGELALA EVARKRASDI DAESITSGVE
481 LLGPLDRGDM LNEAFIGLSL APQGEDSFPD NLPPSCPTHR HILQQRILNG SSNRQIIDRR
541 NELEKDICSG FLMTNTCNAE DGELREDGRE QEIRDGGSLK MIHFGLV
SEQ ID NO:134
   WD REPEAT CONTAINING PLANAR CELL POLARITY EFFECTOR (WDPCP), HOMOLOG ISOFORM 2
   NP_056994.3 
1 MRREFCWDAY SKAAGSRASS PLPRQDRDSF CHQMSFCLTE LHLWSLKNTL HIADRDIGIY
61 QYYDKKDPPA TEHGNLEKKQ KLAESRDYPW TLKNRRPEKL RDSLKELEEL MQNSRCVLSK
121 WKNKYVCQLL FGSGVLVSLS LSGPQLEKVV IDRSLVGKLI SDTISDALLT DSFIILSFLA
181 QNKLCFIQFT KKMESSDVNK RLEKLSALDY KIFYYEIPGP INKTTERHLA INCVHDRVVC
241 WWPLVNDDAW PWAPISSEKD RANLLLLGYA QGRLEVLSSV RTEWDPLDVR FGTKQPYQVF
301 TVEHSVSVDK EPMADSCIYE CIRNKIQCVS VTRIPLKSKA ISCCRNVTED KLILGCEDSS
361 LILYETHRRV TLLAQTELLP SLISCHPSGA ILLVGSNQGE LQIFDMALSP INIQLLAEDR
421 LPRETLQFSK LFDASSSLVQ MQWIAPQVVS QKGEGSDIYD LLFLRFERGP LGVLLFKLGV
481 FTRGQLGLID IIFQYIHCDE IYEAINILSS MNWDTLGHQC FISMSAIVNH LLRQKLTPER
541 EAQLETSLGT FYAPTRPLLD STILEYRDQI SKYARRFFHH LLRYQRFEKA FLLAVDVGAR
601 DLFMDIHYLA LDKGELALAE VARKRASDID AESITSGVEL LGPLDRGDML NEAFIGLSLA
661 PQGEDSFPDN LPPSCPTHRH ILQQRILNGS SNRQIIDRRN ELEKDICSGF LMTNTCNAED
721 GELREDGREQ EIRDGGSLKM IHFGLV
SEQ ID NO:135
   WD REPEAT CONTAINING PLANAR CELL POLARITY EFFECTOR (WDPCP), HOMOLOG ISOFORM 3
   NP_001340973.1 
1 MDRDSFCHQM SFCLTELHLW SLKNTLHIAD RDIGIYQYYD KKDPPATEHG NLEKKQKLAE
61 SRDYPWTLKN RRPEKLRDSL KELEELMQNS RCVLSKWKNK YVCQLLFGSG VLVSLSLSGP
121 QLEKVVIDRS LVGKLISDTI SDALLTDSFI ILSFLAQNKL CFIQFTKKME SSDVNKRLEK
181 LSALDYKIFY YEIPGPINKT TERHLAINCV HDRVVCWWPL VNDDAWPWAP ISSEKDRANL
241 LLLGYAQGRL EVLSSVRTEW DPLDVRFGTK QPYQVFTVEH SVSVDKEPMA DSCIYECIRN
301 KIQCVSVTRI PLKSKAISCC RNVTEDKLIL GCEDSSLILY ETHRRVTLLA QTELLPSLIS
361 CHPSGAILLV GSNQGELQIF DMALSPINIQ LLAEDRLPRE TLQFSKLFDA SSSLVQMQWI
421 APQVVSQKGE GSDIYDLLFL RFERGPLGVL LFKLGVFTRG QLGLIDIIFQ YIHCDEIYEA
481 INILSSMNWD TLGHQCFISM SAIVNHLLRQ KLTPEREAQL ETSLGTFYAP TRPLLDSTIL
541 EYRDQISKYA RRFFHHLLRY QRFEKAFLLA VDVGARDLFM DIHYLALDKG ELALAEVARK
 
601 RASDIDAESI TSGVELLGPL DRGDMLNEAF IGLSLAPQGE DSFPDNLPPS CPTHRHILQQ
661 RILNGSSNRQ IIDRRNELEK DICSGFLMTN TCNAEDGELR EDGREQEIRD GGSLKMIHFG
721 LV
SEQ ID NO:136
   WD REPEAT CONTAINING PLANAR CELL POLARITY EFFECTOR (WDPCP), HOMOLOG ISOFORM 4
   NP_001340974.1 
1 MRREFCWDAY SKAAGSRASS PLPRQDRDSF CHQMSFCLTE LHLWSLKNTL HIADRDIGIY
61 QYYDKKDPPA TEHGNLEKKQ KLAESRDYPW TLKNRRPEKL RDSLKELEEL MQNSRCVLSK
121 WKNKYVCQLL FGSGVLVSLS LSGPQLEKVV IDRSLVGKLI SDTISDALLT DSFIILSFLA
181 QNKLCFIQFT KKMESSDVNK RLEKLSALDY KIFYYEIPGP INKTTERHLA INCVHDRVVC
241 WWPLVNDDAW PWAPISSEKD RANLLLLGYA QGRLEVLSSV RTEWDPLDVR FGTKQPYQVF
301 TVEHSVSVDK EPMADSCIYE CIRNKIQCVS VTRIPLKSKA ISCCRNVTED KLILGCEDSS
361 LILYETHRRV TLLAQTELLP SLISCHPSGA ILLVGSNQGE LQIFDMALSP INIQLLAEDR
421 LPRETLQFSK LFDASSSLVQ MQWIAPQVVS QKGEGSDIYD LLFLRFERGP LGVLLFKLGV
481 FTRGQLGLID IIFQYIHCDE IYEAINILSS MNWDTLGHQC FISMSAIVNH LLRQKLTPER
541 EAQLETSLGT FYAPTRPLLD STILEYRDQI SKYARRFFHH LLRWSLALSP RLECSDVIAV
601 HCHLHLLGSS DSSASASRVA GTTGMCHHTQ LIFVVFSRDG ISPCWPGWS
SEQ ID NO:137
   SEROLOGICALLY DEFINED COLON CANCER ANTIGEN 8 (SDCCAG8)
   Q86SQ7.1 
1 MAKSPENSTL EEILGQYQRS LREHASRSIH QLTCALKEGD VTIGEDAPNL SFSTSVGNED
61 ARTAWPELQQ SHAVNQLKDL LRQQADKESE VSPSRRRKMS PLRSLEHEET NMPTMHDLVH
121 TINDQSQYIH HLEAEVKFCK EELSGMKNKI QVVVLENEGL QQQLKSQRQE ETLREQTLLD
181 ASGNMHNSWI TTGEDSGVGE TSKRPFSHDN ADFGKAASAG EQLELEKLKL TYEEKCEIEE
241 SQLKFLRNDL AEYQRTCEDL KEQLKHKEFL LAANTCNRVG GLCLKCAQHE AVLSQTHTNV
301 HMQTIERLVK ERDDLMSALV SVRSSLADTQ QREASAYEQV KQVLQISEEA NFEKTKALIQ
361 CDQLRKELER QAERLEKELA SQQEKRAIEK DMMKKEITKE REYMGSKMLI LSQNIAQLEA
421 QVEKVTKEKI SAINQLEEIQ SQLASREMDV TKVCGEMRYQ LNKTNMEKDE AEKEHREFRA
481 KTNRDLEIKD QEIEKLRIEL DESKQHLEQE QQKAALAREE CLRLTELLGE SEHQLHLTRQ
541 EKDSIQQSFS KEAKAQALQA QQREQELTQK IQQMEAQHDK TENEQYLLLT SQNTFLTKLK
601 EECCTLAKKL EQISQKTRSE IAQLSQEKRY TYDKLGKLQR RNEELEEQCV QHGRVHETMK
661 QRLRQLDKHS QATAQQLVQL LSKQNQLLLE RQSLSEEVDR LRTQLPSMPQ SDC
SEQ ID NO:138
   LEUCINE ZIPPER TRANSCRIPTION FACTOR LIKE 1 (LZTFL1)
   CAB95836.1 
1 MAELGLNEHH QNEVINYMRF ARSKRGLRLK TVDSCFQDLK ESRLVEDTFT IDEVSEVLNG
61 LQAVVHSEVE SELINTAYTN VLLLRQLFAQ AEKWYLKLQT DISELENREL LEQVAEFEKA
121 EITSSNKKPI LDVTKPKLAP LNEGGTAELL NKEILRLQEE NEKLKSRLKT IEIQATNALD
181 EKSKLEKALQ DLQLDQGNQK DFIKAQDLSN LENTVAALKS EFQKTLNDKT ENQKSLEENL
241 ATAKHDLLRV QEQLHMAEKE LEKKFQQTAA YRNMKEILTK KNDQIKDLRK RLAQYEPED
SEQ ID NO:139
   BBSOME INTERACTING PROTEIN 1 (BBIP1), ISOFORM 1
   NP_001182233.1 
1 MLKAAAKRPE LSGLLKFNNY GILSESPLTS QRTTWLLYQS PSFIPGFAYP SRCLKTIGGV
61 YKQARKKHYI QQLRYGRSEV NVPGSSSKAR ATVCGRYNDN GAV
SEQ ID NO:140
   BBSOME INTERACTING PROTEIN 1 (BBIP1), ISOFORM 2
   NP_001182234.1 
1 MLKAAAKRPE LSGKNTISNN SDMAEVKSMF REVLPKQGPL FVEDIMTMVL CKPKLLPLKS
61 LTLEKLEKMH QAAQNTIRQQ EMAEKDQRQI TH
SEQ ID NO:141
   INTRAFLAGELLAR TRANSPORT 27 (IFT27), HOMOLOG ISOFORM 1
   NP_001349932.1 
1 MVKLAAKCIL AGDPAVGKTA LAQIFRSDGA HFQKSYTLTT GMDLVVKTVP VPDTGDSVEL
61 FIFDSAGKEL FSEMLDKLWE SPNVLCLVYD VTNEESFNNC SKWLEKARSQ APGISLPGVL
121 VGNKTDLAGR RAVDSAEARA WALGQGLECF ETSVKEMENF EAPFHCLAKQ FHQLYREKVE
181 VFRALA
SEQ ID NO:142
   INTRAFLAGELLAR TRANSPORT 27 (IFT27), HOMOLOG ISOFORM 2
   NP_006851.1 
1 
61 
121 
181 
SEQ ID NO:143
   GUANYLATE CYCLASE ACTIVATOR 1A (GUCA1A)
   EAX04084.1 
1 
61 
121 
181 
SEQ ID NO:144
   OPA1 MITOCHONDRIAL DYNAMIN LIKE GTPASE (OPA1);
   AAH58013.1 
1 MTEPKGKEHD DIFDKLKEAV KEESIKRHKW NDFAEDSLRV IQHNALEDRS ISDKQQWDAA
61 IYFMEEALQA RLKDTENAIE NMVGPDWKKR WLYWKNRTQE QCVHNETKNE LEKMLKCNEE
121 HPAYLASDEI TTVRKNLESR GVEVDPSLIK DTWHQVYRRH FLKTALNHCN LCRRGFYYYQ
181 RHFVDSELEC NDVVLFWRIQ RMLAITANTL RQQLTNTEVR RLEKNVKEVL EDFAEDGEKK
241 IKLLTGKRVQ LAEDLKKVRE IQEKLDAFIE ALHQEK
SEQ ID NO:145
   RP1 AXONEMAL MICROTUBULE ASSOCIATED (RP1)
   AAA20120.1 
1 MQKWFSAFDD AIIQRQWRAN PSRGGGGVSF TKEVDTNVAT GAPPRRQRVP GRACPWREPI
61 RGRRGARPGG GDAGGTPGET VRHCSAPEDP IFRFSSLHSY PFPGTIKSRD MSWKRHHLIP
121 ETFGVKRRRK RGPVESDPLR GEPGSARAAV SELMQLFPRG LFEDALPPIV LRSQVYSLVP
181 DRTVADRQLK ELQEQGEIRI VQLGFDLDAH GIIFTEDYRT RVLKACDGRP YAGAVQKFLA
241 SVLPACGDLS FQQDQMTQTF GFRDSEITHL VNAGVLTVRD AGSWWLAVPG AGRFIKYFVK
301 GRQAVLSMVR KAKYRELLLS ELLGRRAPVV VRLGLTYHVH DLIGAQLVDC ISTTSGTLLR
361 LPET
SEQ ID NO:146
   RP2 ACTIVATOR OF ARL3 GTPASE (RP2)
   ANZ79619.1 
1 
61 
121 
181 
241 
301 
SEQ ID NO:147
   PERIPHERIN 2 (PRPH2)
   NP_000313.2 1  
61 
121 
181 
241 
301 
SEQ ID NO:148
   PRE-MRNA PROCESSING FACTOR 31(PRPF31)
   AAI17390.1 
1 MSLADELLAD LEEAAEEEEG GSYGEEEEEP AIEDVQEETQ LDLSGDSVKT IAKLWDSKMF
61 AEIMMKIEEY ISKQAKASEV MGPVEAAPEY RVIVDANNLT VEIENELNII HKFIRDKYSK
121 RFPELESLVP NALDYIRTVK ELGNSLDKCK NNENLQQILT NATIMVVSVT ASTTQGQQLS
181 EEELERLEEA CDMALELNAS KHRIYEYVES RMSFIAPNLS IIIGASTAAK IMGVAGGLTN
241 LSKMPACNIM LLGAQRKTLS GFSSTSVLPH TGYIYHSDIV QSLPPDLRRK AARLVAAKCT
301 LAARVDSFHE STEGKVGYEL KDEIERKFDK WQEPPPVKQV KPLPAPLDGQ RKKRGGRRYR
361 KMKERLGLTE IRKQANRMSF GEIEEDAYQE DLGFSLGHLG KSGSGRVRQT QVNEATKARI
421 SKTLQRTLQK QSVVYGGKST IRDRSSGTAS SVAFTPLQGL EIVNPQAAEK KVAEANQKYF
481 SSMAEFLKVK GEKSGLMST
SEQ ID NO:149
   PRE-MRNA PROCESSING FACTOR 31(PRPF31), ISOFORM CRA_A
   EAW72190.1 
1 MSLADELLAD LEEAAEEEEG GSYGEEEEEP AIEDVQEETQ LDLSGDSVKT IAKLWDSKMF
61 AEIMMKIEEY ISKQAKASEV MGPVEAAPEY RVIVDANNLT VEIENELNII HKFIRDKYSK
121 RFPELESLVP NALDYIRTVK ELGNSLDKCK NNENLQQILT NATIMVVSVT ASTTQGQQLS
181 EEELERLEEA CDMALELNAS KHRIYEYVES RMSFIAPNLS IIIGASTAAK IMGVAGGLTN
241 LSKMPACNIM LLGAQRKTLS GFSSTSVLPH TGYIYHSDIV QSLPPDLRRK AARLVAAKCT
301 LAARVDSFHE STEGKVGYEL KDEIERKFDK WQEPPPVKQV KPLPAPLDGQ RKKRGGRRYR
361 KMKERLGLTE IRKQANRMSF GEIEEDAYQE DLGFSLGHLG KSGSGRVRQT QVNEATKARI
421 SKTLQRTLQK QSVVYGGKST IRDRSSGTAS SVAFTPLQGL EIVNPQAAEK KVAEANQKYF
481 SSMAEFLKVK GEKSGLMST
SEQ ID NO:150
   PRE-MRNA PROCESSING FACTOR 31(PRPF31), ISOFORM CRA_A
   EAW72191.1 
1 MFAEIMMKIE EYISKQAKAS EVMGPVEAAP EYRVIVDANN LTVEIENELN IIHKFIRDKY
61 SKRFPELESL VPNALDYIRT VKELGNSLDK CKNNENLQQI LTNATIMVVS VTASTTQGQQ
121 LSEEELERLE EACDMALELN ASKHRIYEYV ESRMSFIAPN LSIIIGASTA AKIMGVAGGL
181 TNLSKMPACN IMLLGAQRKT LSGFSSTSVL PHTGYIYHSD IVQSLPPDLR RKAARLVAAK
241 CTLAARVDSF HESTEGKVGY ELKDEIERKF DKWQEPPPVK QVKPLPAPLD GQRKKRGGRR
SEQ ID NO:151
   PRE-MRNA PROCESSING FACTOR 8 (PRPF8)
   AAH64370.1 
1 
61 
121 
181 
241 
301 
361 
421 
481 
541 
601 
661 
721 
 
781 
841 
901 
961 
1021 
1081 
1141 
1201 
1261 
1321 
1381 
1441 
1501 
1561 
1621 
1681 
1741 
1801 
1861 
1921 
1981 
2041 
2101 
2161 
2221 
2281 
SEQ ID NO:152
   PRE-MRNA PROCESSING FACTOR 8 (PRPFB), ISOFORM CRA_A
   EAW90588.1 
1 MAGVFPYRGP GNPVPGPLAP LPDYMSEEKL QEKARKWQQL QAKRYAEKRK FGFVDAQKED
61 MPPEHVRKII RDHGDMTNRK FRHDKRVYLG ALKYMPHAVL KLLENMPMPW EQIRDVPVLY
121 HITGAISFVN EIPWVIEPVY ISQWGSMWIM MRREKRDRRH FKRMRFPPFD DEEPPLDYAD
181 NILDVEPLEA IQLELDPEED APVLDWFYDH QPLRDSRKYV NGSTYQRWQF TLPMMSTLYR
241 LANQLLTDLV DDNYFYLFDL KAFFTSKALN MAIPGGPKFE PLVRDINLQD EDWNEFNDIN
301 KIIIRQPIRT EYKIAFPYLY NNLPHHVHLT WYHTPNVVFI KTEDPDLPAF YFDPLINPIS
361 HRHSVKSQEP LPDDDEEFEL PEFVEPFLKD TPLYTDNTAN GIALLWAPRP FNLRSGRTRR
421 ALDIPLVKNW YREHCPAGQP VKVRVSYQKL LKYYVLNALK HRPPKAQKKR YLFRSFKATK
481 FFQSTKLDWV EVGLQVCRQG YNMLNLLIHR KNLNYLHLDY NFNLKPVKTL TTKERKKSRF
541 GNAFHLCREV LRLTKLVVDS HVQYRLGNVD AFQLADGLQY IFAHVGQLTG MYRYKYKLMR
601 QIRMCKDLKH LIYYRFNTGP VGKGPGCGFW AAGWRVWLFF MRGITPLLER WLGNLLARQF
661 EGRHSKGVAK TVTKQRVESH FDLELRAAVM HDILDMMPEG IKQNKARTIL QHLSEAWRCW
721 KANIPWKVPG LPTPIENMIL RYVKAKADWW TNTAHYNRER IRRGATVDKT VCKKNLGRLT
781 RLYLKAEQER QHNYLKDGPY ITAEEAVAVY TTTVHWLESR RFSPIPFPPL SYKHDTKLLI
841 LALERLKEAY SVKSRLNQSQ REELGLIEQA YDNPHEALSR IKRHLLTQRA FKEVGIEFMD
901 LYSHLVPVYD VEPLEKITDA YLDQYLWYEA DKRRLFPPWI KPADTEPPPL LVYKWCQGIN
961 NLQDVWETSE GECNVMLESR FEKMYEKIDL TLLNRLLRLI VDHNIADYMT AKNNVVINYK
1021 DMNHTNSYGI IRGLQFASFI VQYYGLVMDL LVLGLHRASE MAGPPQMPND FLSFQDIATE
1081 AAHPIRLFCR YIDRIHIFFR FTADEARDLI QRYLTEHPDP NNENIVGYNN KKCWPRDARM
1141 RLMKHDVNLG RAVFWDIKNR LPRSVTTVQW ENSFVSVYSK DNPNLLFNMC GFECRILPKC
1201 RTSYEEFTHK DGVWNLQNEV TKERTAQCFL RVDDESMQRF HNRVRQILMA SGSTTFTKIV
1261 NKWNTALIGL MTYFREAVVN TQELLDLLVK CENKIQTRIK IGLNSKMPSR FPPVVFYTPK
1321 ELGGLGMLSM GHVLIPQSDL RWSKQTDVGI THFRSGMSHE EDQLIPNLYR YIQPWESEFI
1381 DSQRVWAEYA LKRQEAIAQN RRLTLEDLED SWDRGIPRIN TLFQKDRHTL AYDKGWRVRT
1441 DFKQYQVLKQ NPFWWTHQRH DGKLWNLNNY RTDMIQALGG VEGILEHTLF KGTYFPTWEG
1501 LFWEKASGFE ESMKWKKLTN AQRSGLNQIP NRRFTLWWSP TINRANVYVG FQVQLDLTGI
1561 FMHGKIPTLK ISLIQIFRAH LWQKIHESIV MDLCQVFDQE LDALEIETVQ KETIHPRKSY
 
1621 KMNSSCADIL LFASYKWNVS RPSLLADSKD VMDSTTTQKY WIDIQLRWGD YDSHDIERYA
1681 RAKFLDYTTD NMSIYPSPTG VLIAIDLAYN LHSAYGNWFP GSKPLIQQAM AKIMKANPAL
1741 YVLRERIRKG LQLYSSEPTE PYLSSQNYGE LFSNQIIWFV DDTNVYRVTI HKTFEGNLTT
1801 KPINGAIFIF NPRTGQLFLK IIHTSVWAGQ KRLGQLAKWK TAEEVAALIR SLPVEEQPKQ
1861 IIVTRKGMLD PLEVHLLDFP NIVIKGSELQ LPFQACLKVE KFGDLILKAT EPQMVLFNLY
1921 DDWLKTISSY TAFSRLILIL RALHVNNDRA KVILKPDKTT ITEPHHIWPT LTDEEWIKVE
1981 VQLKDLILAD YGKKNNVNVA SLTQSEIRDI ILGMEISAPS QQRQQIAEIE KQTKEQSQLT
2041 ATQTRTVNKH GDEIITSTTS NYETQTFSSK TEWRVRAISA ANLHLRTNHI YVSSDDIKET
2101 GYTYILPKNV LKKFICISDL RAQVSKWTQL GHSVCPTHFV PKTQT
SEQ ID NO:153
   PRE-MRNA PROCESSING FACTOR 8 (PRPF8), ISOFORM CPA_B
   EAW90589.1 
1 
61 
121 
181 
241 
301 
361 
421 
481 
541 
601 
661 
721 
781 
841 
901 
961 
1021 
1081 
1141 
1201 
1261 
1321 
1381 
1441 
1501 
1561 
1621 
1681 
1741 
1801 
1861 
1921 
1981 
2041 
2101 
2161 
2221 
2281 
SEQ ID NO:154
   PRE-MRNA PROCESSING FACTOR 8 (PRPF8), ISOFORM CPA_C
   EAW90590.1 1 
SEQ ID NO:155
   PRE-MRNA PROCESSING FACTOR 8 (PRPF8), ISOFORM CRA_D
   EAW90591.1 
1 MAGVFPYRGP GNPVPGPLAP LPDYMSEEKL QEKARKWQQL QAKRYAEKRK FGFVDAQKED
61 MPPEHVRKII RDHGDMTNRK FRHDKRVYLG ALKYMPHAVL KLLENMPMPW EQIRDVPVLY
121 HITGAISFVN EIPWVIEPVY ISQWGSMWIM MRREKRDRRH FKRMRFPPFD DEEPPLDYAD
181 NILDVEPLEA IQLELDPEED APVLDWFYDH QPLRDSRKYV NGSTYQRWQF TLPMMSTLYR
241 LANQLLTDLV DDNYFYLFDL KAFFTSKALN MAIPGGPKFE PLVRDINLQD EDWNEFNDIN
301 KIIIRQPIRT EYKIAFPYLY NNLPHHVHLT WYHTPNVVFI KTEDPDLPAF YFDPLINPIS
361 HRHSVKSQEP LPDDDEEFEL PEFVEPFLKD TPLYTDNTAN GIALLWAPRP FNLRSGRTRR
421 ALDIPLVKNW YREHCPAGQP VKVRVSYQKL LKYYVLNALK HRPPKAQKKR YLFRSFKATK
481 FFQSTKLDWV EVGLQVCRQG YNMLNLLIHR KNLNYLHLDY NFNLKPVKTL TTKERKKSRF
541 GNAFHLCREV LRLTKLVVDS HVQYRLGNVD AFQLADGLQY IFAHVGQLTG MYRYKYKLMR
601 QIRMCKDLKH LIYYRFNTGP VGKGPGCGFW AAGWRVWLFF MRGITPLLER WLGNLLARQF
661 EGRHSKGVAK TVTKQRVESH FDLELRAAVM HDILDMMPEG IKQNKARTIL QHLSEAWRCW
721 KANIPWKVPG LPTPIENMIL RYVKAKADWW TNTAHYNRER IRRGATVDKT VCKKNLGRLT
781 RLYLKAEQER QHNYLKDGPY ITAEEAVAVY TTTVHWLESR RFSPIPFPPL SYKHDTKLLI
841 LALERLKEAY SVKSRLNQSQ REELGLIEQA YDNPHEALSR IKRHLLTQRA FKEVGIEFMD
901 LYSHLVPVYD VEPLEKITDA YLDQYLWYEA DKRRLFPPWI KPADTEPPPL LVYKWCQGIN
961 NLQDVWETSE GECNVMLESR FEKMYEKIDL TLLNRLLRLI VDHNIADYMT AKNNVVINYK
1021 DMNHTNSYGI IRGLQFASFI VQYYGLVMDL LVLGLHRASE MAGPPQMPND FLSFQDIATE
1081 AAHPIRLFCR YIDRIHIFFR FTADEARDLI QRYLTEHPDP NNENIVGYNN KKCWPRDARM
1141 RLMKHDVNLG RAVFWDIKNR LPRSVTTVQW ENSFVSVYSK DNPNLLFNMC GFECRILPKC
1201 RTSYEEFTHK DGVWNLQNEV TKERTAQCFL RVDDESMQRF HNRVRQILMA SGSTTFTKIV
1261 NKWNTALIGL MTYFREAVVN TQELLDLLVK CENKIQTRIK IGLNSKMPSR FPPVVFYTPK
1321 ELGGLGMLSM GHVLIPQSDL RWSKQTDVGI THFRSGMSHE EDQLIPNLYR YIQPWESEFI
1381 DSQRVWAEYA LKRQEAIAQN RRLTLEDLED SWDRGIPRIN TLFQKDRHTL AYDKGWRVRT
1441 DFKQYQVLKQ NPFWWTHQRH DGKLWNLNNY RTDMIQALGG VEGILEHTLF KGTYFPTCEQ
1501 RSGKSDPEAR QDYYYRTTPH LAHSD
SEQ ID NO:156
   PRE-MRNA PROCESSING FACTOR 8 (PRPF8), ISOFORM CRA_E
   EAW90592.1 
  1 MEISAPSQQR QQIAEIEKQT KEQSQLTATQ TRTVNKHGDE IITSTTSNYE TQTFSSKTEW
 61 RVRAISAANL HLRTNHIYVS SDDIKETGYT YILPKNVLKK FICISDLRAQ VSKWTQLGHS
121 VCPTHFVPKT QT
SEQ ID NO:157
   PRE-MRNA PROCESSING FACTOR 8 (PRPF8), ISOFORM CRA_F
   EAW90593.1 
1 MAGVFPYRGP GNPVPGPLAP LPDYMSEEKL QEKARKWQQL QAKRYAEKRK FGFVDAQKED
61 MPPEHVRKII RDHGDMTNRK FRHDKRVYLG ALKYMPHAVL KLLENMPMPW EQIRDVPVLY
121 HITGAISFVN EIPWVIEPVY ISQWGSMWIM MRREKRDRRH FKRMRFPPFD DEEPPLDYAD
181 NILDVEPLEA IQLELDPEED APVLDWFYDH QPLRDSRKYV NGSTYQRWQF TLPMMSTLYR
241 LANQLLTDLV DDNYFYLFDL KAFFTSKALN MAIPGGPKFE PLVRDINLQD EDWNEFNDIN
301 KIIIRQPIRT EYKIAFPYLY NNLPHHVHLT WYHTPNVVFI KTEDPDLPAF YFDPLINPIS
361 HRHSVKSQEP LPDDDEEFEL PEFVEPFLKD TPLYTDNTAN GIALLWAPRP FNLRSGRTRR
421 ALDIPLVKNW YREHCPAGQP VKVRVSYQKL LKYYVLNALK HRPPKAQKKR YLFRSFKATK
481 FFQSTKLDWV EVGLQVCRQG YNMLNLLIHR KNLNYLHLDY NFNLKPVKTL TTKERKKSRF
541 GNAFHLCREV LRLTKLVVDS HVQYRLGNVD AFQLADGLQY IFAHVGQLTG MYRYKYKLMR
601 QIRMCKDLKH LIYYRFNTGP VGKGPGCGFW AAGWRVWLFF MRGITPLLER WLGNLLARQF
661 EGRHSKGVAK TVTKQRVESH FDLELRAAVM HDILDMMPEG IKQNKARTIL QHLSEAWRCW
721 KANIPWKVPG LPTPIENMIL RYVKAKADWW TNTAHYNRER IRRGATVDKT VCKKNLGRLT
781 RLYLKAEQER QHNYLKDGPY ITAEEAVAVY TTTVHWLESR RFSPIPFPPL SYKHDTKLLI
841 LALERLKEAY SVKSRLNQSQ REELGLIEQA YDNPHEALSR IKRHLLTQRA FKEVGIEFMD
 
901 LYSHLVPVYD VEPLEKITDA YLDQYLWYEA DKRRLFPPWI KPADTEPPPL LVYKWCQGIN
961 NLQDVWETSE GECNVMLESR FEKMYEKIDL TLLNRLLRLI VDHNIADYMT AKNNVVINYK
1021 DMNHTNSYGI IRGLQFASFI VQYYGLVMDL LVLGLHRASE MAGPPQMPND FLSFQDIATE
1081 AAHPIRLFCR YIDRIHIFFR FTADEARDLI QRYLTEHPDP NNENIVGYNN KKCWPRDARM
1141 RLMKHDVNLG RAVFWDIKNR LPRSVTTVQW ENSFVSVYSK DNPNLLFNMC GFECRILPKC
1201 RTSYEEFTHK DGVWNLQNEV TKERTAQCFL RVDDESMQRF HNRVRQILMA SGSTTFTKIV
1261 NKWNTALIGL MTYFREAVVN TQELLDLLVK CENKIQTRIK IGLNSKMPSR FPPVVFYTPK
1321 ELGGLGMLSM GHVLIPQSDL RWSKQTDVGI THFRSGMSHE EDQLIPNLYR YIQPWESEFI
1381 DSQRVWAEYA LKRQEAIAQN RRLTLEDLED SWDRGIPRIN TLFQKDRHTL AYDKGWRVRT
1441 DFKQYQVLKQ NPFWWTHQRH DGKLWNLNNY RTDMIQALGG VEGILEHTLF KGTYFPTWEG
1501 LFWEKASGFE ESMKWKKLTN AQRSGLNQIP NRRFTLWWSP TINRANVYVG FQVQLDLTGI
1561 FMHGKIPTLK ISLIQIFRAH LWQKIHESIV MDLCQVFDQE LDALEIETVQ KETIHPRKSY
1621 KMNSSCADIL LFASYKWNVS RPSLLADSKD VMDSTTTQKY WIDIQLRWGD YDSHDIERYA
1681 RAKFLDYTTD NMSIYPSPTG VLIAIDLAYN LHSAYGNWFP GSKPLIQQAM AKIMKANPAL
1741 YVLRERIRKG LQLYSSEPTE PYLSSQNYGE LFSNQIIWFV DDTNVYRVTI HKTFEGNLTT
1801 KPINGAIFIF NPRTGQLFLK IIHTSVWAGQ KRLGQLAKWK TAEEVAALIR SLPVEEQPKQ
1861 IIVTRKGMLD PLEVHLLDFP NIVIKGSELQ LPFQACLKVE KFGDLILKAT EPQMVLFNLY
1921 DDWLKTISSY TAFSRLILIL RALHVNNDRA KVILKPDKTT ITEPHHIWPT LTDEEWIKVE
1981 VQLKDLILAD YGKKNNVNVA SLTQSEIRDI ILGMEISAPS QQRQQIAEIE KQTKEQSQLT
2041 ATQTRTVNKH GDEIITSTTS NYETQTFSSK TEWRVRAISA ANLHLRTNHI YVSSDDIKET
2101 GYTYILPKNV LKKFICISDL RAQIAGYLYG VSPPDNPQVK EIRCIVMVPQ WGTHQTVHLP
2161 GQLPQHEYLK EMEPLGWIHT QPNESPQLSP QDVTTHAKIM ADNPSWDGEK TIIITCSFTP
2221 GSCTLTAYKL TPSGYEWGRQ NTDKGNNPKG YLPSHYERVQ MLLSDRFLGF FMVPAQSSWN
2281 YNFMGVRHDP NMKYELQLAN PKEFYHEVHR PSHFLNFALL QEGEVYSADR EDLYA
SEQ ID NO:158
   PRE-MRNA PROCESSING FACTOR 8 (PRPFB), ISOFORM CRA_G
   EAW90594.1 
1 MAGVFPYRGP GNPVPGPLAP LPDYMSEEKL QEKARKWQQL QAKRYAEKRK FGFVDAQKED
61 MPPEHVRKII RDHGDMTNRK FRHDKRVYLG ALKYMPHAVL KLLENMPMPW EQIRDVPVLY
121 HITGAISFVN EIPWVIEPVY ISQWGSMWIM MRREKRDRRH FKRMRFPPFD DEEPPLDYAD
181 NILDVEPLEA IQLELDPEED APVLDWFYDH QPLRDSRKYV NGSTYQRWQF TLPMMMSPPM
241 PRSWLTTHLG MARRPLSSHA ASRQAPVH
SEQ ID NO:159
   EYES SHUT HOMOLOG (EYS)
   CAR64275.1 
1 
61 
121 
181 
241 
301 
361 
421 
481 
541 
601 
661 
721 
781 
841 
901 
961 
1021 
1081 
1141 
 
1201 
1261 
1321 
1381 
1441 
1501 
1561 
1621 
1681 
1741 
1801 
1861 
1921 
1981 
2041 
2101 
2161 
2221 
2281 
2341 
2401 
2461 
2521 
2581 
2641 
2701 
2761 
2821 
2881 
2941 
3001 
3061 
3121 
SEQ ID NO:160
   FAM161 CENTROSOMAL PROTEIN A (FAM161A), ISOFORM 1
   NP_001188472.1 
1 MATSHRVAKL VASSLQTPVN PITGARVAQY EREDPLKALA AAEAILEDEE EEKVAQPAGA
61 SADLNTSFSG VDEHAPISYE DFVNFPDIHH SNEEYFKKVE ELKAAHIETM AKLEKMYQDK
121 LHLKEVQPVV IREDSLSDSS RSVSEKNSYH PVSLMTSFSE PDLGQSSSLY VSSSEEELPN
181 LEKEYPRKNR MMTYAKELIN NMWTDFCVED YIRCKDTGFH AAEKRRKKRK EWVPTITVPE
241 PFQMMIREQK KKEESMKSKS DIEMVHKALK KQEEDPEYKK KFRANPVPAS VFLPLYHDLV
301 KQKEERRRSL KEKSKEALLA SQKPFKFIAR EEQKRAAREK QLRDFLKYKK KTNRFKARPI
361 PRSTYGSTTN DKLKEEELYR NLRTQLRAQE HLQNSSPLPC RSACGCRNPR CPEQAVKLKC
421 KHKVRCPTPD FEDLPERYQK HLSEHKSPKL LTVCKPFDLH ASPHASIKRE KILADIEADE
481 ENLKETRWPY LSPRRKSPVR CAGVNPVPCN CNPPVPTVSS RGREQAVRRS LEEKKMLEEE
541 RNRILTKQKQ RMKELQKLLT TRAKAYDSHQ SLAQISKSRV KCLRKSEKER MREYQRELEE
601 REEKLKKRPL LFERVAQKNA RMAAEKHYSN TLKALGISDE FVSKKGQSGK VLEYFNNQET
661 KSVTEDKESF NEEEKIEERE NGEENYFIDT NSQDSYKEKD EANEESEEEK SVEESH
SEQ ID NO:161
   FAM161 CENTROSOMAL PROTEIN A (FAM161A), ISOFORM 2
   NP_115556.2 
1 MATSHRVAKL VASSLQTPVN PITGARVAQY EREDPLKALA AAEAILEDEE EEKVAQPAGA
61 SADLNTSFSG VDEHAPISYE DFVNFPDIHH SNEEYFKKVE ELKAAHIETM AKLEKMYQDK
121 LHLKEVQPVV IREDSLSDSS RSVSEKNSYH PVSLMTSFSE PDLGQSSSLY VSSSEEELPN
181 LEKEYPRKNR MMTYAKELIN NMWTDFCVED YIRCKDTGFH AAEKRRKKRK EWVPTITVPE
 
241 PFQMMIREQK KKEESMKSKS DIEMVHKALK KQEEDPEYKK KFRANPVPAS VFLPLYHDLV
301 KQKEERRRSL KEKSKEALLA SQKPFKFIAR EEQKRAAREK QLRDFLKYKK KTNRFKARPI
361 PRSTYGSTTN DKLKEEELYR NLRTQLRAQE HLQNSSPLPC RSACGCRNPR CPEQAVKLKC
421 KHKVRCPTPD FEDLPERYQK HLSEHKSPKL LTVCKPFDLH ASPHASIKRE KILADIEADE
481 ENLKETRWPY LSPRRKSPVR CAGVNPVPCN CNPPVPTVSS RGREQAVRKS EKERMREYQR
541 ELEEREEKLK KRPLLFERVA QKNARMAAEK HYSNTLKALG ISDEFVSKKG QSGKVLEYFN
601 NQETKSVTED KESFNEEEKI EERENGEENY FIDTNSQDSY KEKDEANEES EEEKSVEESH
SEQ ID NO:162
   MER PROTO-ONCOGENE, TYROSINE KINASE (MERTK);
   Q12866.2 
1 
61 
121 
181 
241 
301 
361 
421 
481 
541 
601 
661 
721 
781 
841 
901 
961 
SEQ ID NO:163
   PHOSPHODIESTERASE 6B (PDE6B)
   AAH00249.1 
1 MSLSEEQARS FLDQNPDFAR QYFGKKLSPE NVAAACEDGC PPDCDSLRDL CQVEESTALL
61 ELVQDMQESI NMERVVFKVL RRLCTLLQAD RCSLFMYRQR NGVAELATRL FSVQPDSVLE
121 DCLVPPDSEI VFPLDIGVVG HVAQTKKMVN VEDVAECPHF SSFADELTDY KTKNMLATPI
181 MNGKDVVAVI MAVNKLNGPF FTSEDEDVFL KYLNFATLYL KIYHLSYLHN CETRRGQVLL
241 WSANKVFEEL TDIERQFHKA FYTVRAYLNC ERYSVGLLDM TKEKEFFDVW SVLMGESQPY
301 SGPRTPDGRE IVFYKVIDYI LHGKEEIKVI PTPSADHWAL ASGLPSYVAE SGFICNIMNA
361 SADEMFKFQE GALDDSGWLI KNVLSMPIVN KKEEIVGVAT FYNRKDGKPF DEQDEVLMES
421 LTQFLGWSVM NTDTYDKMNK LENRKDIAQD MVLYHVKCDR DEIQLILPTR ARLGKEPADC
481 DEDELGEILK EELPGPTTFD IYEFHFSDLE CTELDLVKCG IQMYYELGVV RKFQIPQEVL
541 VRFLFSISKG YRRITYHNWR HGFNVAQTMF TLLMTGKLKS YYTDLEAFAM VTAGLCHDID
601 HRGTNNLYQM KSQNPLAKLH GSSILERHHL EFGKFLLSEE TLNIYQNLNR RQHDHVIHLM
661 DIAIIATDLA LYFKKRAMFQ KIVDESKNYQ DKKSWVEYLS LETTRKEIVM AMMMTACDLS
721 AITKPWEVQS KVALLVAAEF WEQGDLERTV LDQQPIPMMD RNKAAELPKL QVGFIDFVCT
781 FVYKEFSRFH EEILPMFDRL QNNRKEWKAL ADEYEAKVKA LEEKEEEERV AAKKGTEICN
841 GGPAPKSSTC CIL
SEQ ID NO:164
   PHOSPHODIESTERASE 6B (PDE6B), ISOFORM CRA_A
   EAW82661.1 
1 
61 
121 
181 
241 
301 
 
361 
421 
481 
541 
601 
661 
721 
781 
SEQ ID NO:165
   PHOSPHODIESTERASE 6B (PDE6B), ISOFORM CPA_B
   EAW82662.1 
1 
61 
121 
181 
241 
301 
361 
421 
481 
541 
601 
SEQ ID NO:166
   PROMININ 1 (PROM1)
   O43490.1 
1 
61 
121 
181 
241 
301 
361 
421 
481 
541 
601 
661 
721 
781 
841 
SEQ ID NO:167
   PROMININ 1 (PROM1), ISOFORM CRA_A
   EAW92750.1 
1 MALVLGSLLL LGLCGNSFSG GQPSSTDAPK AWNYELPATN YETQDSHKAG PIGILFELVH
61 IFLYVVQPRD FPEDTLRKFL QKAYESKIDY DKIVYYEAGI ILCCVLGLLF IILMPLVGYF
121 FCMCRCCNKC GGEMHQRQKE NGPFLRKCFA ISLLVICIII SIGIFYGFVA NHQVRTRIKR
181 SRKLADSNFK DLRTLLNETP EQIKYILAQY NTTKDKAFTD LNSINSVLGG GILDRLRPNI
241 IPVLDEIKSM ATAIKETKEA LENMNSTLKS LHQQSTQLSS SLTSVKTSLR SSLNDPLCLV
301 HPSSETCNSI RLSLSQLNSN PELRQLPPVD AELDNVNNVL RTDLDGLVQQ GYQSLNDIPD
361 RVQRQTTTVV AGIKRVLNSI GSDIDNVTQR LPIQDILSAF SVYVNNTESY IHRNLPTLEE
421 YDSYWWLGGL VICSLLTLIV IFYYLGLLCG VCGYDRHATP TTRGCVSNTG GVFLMVGVGL
481 SFLFCWILMI IVVLTFVFGA NVEKLICEPY TSKELFRVLD TPYLLNEDWE YYLSGKLFNK
541 SKMKLTFEQV YSDCKKNRGT YGTLHLQNSF NISEHLNINE HTGSISSELE SLKVNLNIFL
601 LGAAGRKNLQ DFAACGIDRM NYDSYLAQTG KSPAGVNLLS FAYDLEAKAN SLPPGNLRNS
 
661 LKRDAQTIKT IHQQRVLPIE QSLSTLYQSV KILQRTGNGL LERVTRILAS LDFAQNFITN
721 NTSSVIIEET KKYGRTIIGY FEHYLQWIEF SISEKVASCK PVATALDTAV DVFLCSYIID
781 PLNLFWFGIG KATVFLLPAL IFAVKLAKYY RRMDSEDVYD DVETIPMKNM ENGNNGYHKD
841 HVYGIHNPVM TSPSQH
SEQ ID NO:168
   PROMININ 1 (PROM1), ISOFORM CPA_B
   EAW92751.1 
1 MFHLEMACKS NHRETCVTPS DKFKREREIL REKCCSFKSG VVLTDANYGV QFNRVFCCIR
61 ININWSAANM SIIRLVSSVL K
SEQ ID NO:169
   PROMININ 1 (PROM1), ISOFORM CPA_C
   EAW92752.1 
1 
61 
121 
181 
241 
301 
361 
421 
481 
541 
601 
661 
721 
781 
841 
SEQ ID NO:170
   PHOSPHODIESTERASE 6A (PDE6A)
   AAH35909.1 
1 
61 
121 
181 
241 
301 
361 
421 
481 
541 
601 
661 
721 
781 
841 
SEQ ID NO:171
   PHOSPHODIESTERASE 6A (PDE6A), ISOFORM CRA_A
   EAW61756.1 
1 MVTAAFCHDI DHRGTNNLYQ MKSQNPLAKL HGSSILERHH LEFGKTLLRD ESLNIFQNLN
61 RRQHEHAIHM MDIAIIATDL ALYFKKRTMF QKIVDQSKTY ESEQEWTQYM MLEQTRKEIV
121 MAMMMTACDL SAITKPWEVQ SQVALLVAAE FWEQGDLERT VLQQNPIPMM DRNKADELPK
181 LQVGFIDFVC TFVYKEFSRF HEEITPMLDG ITNNRKEWKA LADEYDAKMK VQEEKKQKQQ
241 SAKSAAAGNQ PGGNPSPGGA TTSKSCCIQ
SEQ ID NO:172
   PHOSPHODIESTERASE 6A (PDE6A), ISOFORM CPA_B
   EAW61757.1 
1 
61 
121 
181 
241 
301 
361 
421 
481 
541 
601 
661 
721 
781 
841 
SEQ ID NO:173
   INTERPHOTORECEPTOR MATRIX PROTEOGLYCAN 2 (IMPG2)
   EAW79803.1 
1 
61 
121 
181 
241 
301 
361 
421 
481 
541 
601 
661 
721 
781 
841 
901 
961 
1021 
1081 
1141 
1201 
SEQ ID NO:174
   MALE GERM CELL ASSOCIATED KINASE (MAK)
   AAN16405.1 
1 MNRYTTMRQL GDGTYGSVLM GKSNESGELV AIKRMKRKFY SWDECMNLRE VKSLKKLNHA
61 NVIKLKEVIR ENDHLYFIFE YMKENLYQLM KDRNKLFPES VIRNIMYQIL QGLAFIHKHG
121 FFHRDMKPEN LLCMGPELVK IADFGLAREL RSQPPYTDYV STRWYRAPEV LLRSSVYSSP
181 IDVWAVGSIM AELYMLRPLF PGTSEVDEIF KICQVLGTPK KSDWPEGYQL ASSMNFRFPQ
241 CVPINLKTLI PNASNEAIQL MTEMLNWDPK KRPTASQALK HPYFQVGQVL GPSSNHLESK
301 QSLNKQLQPL ESKPSLVEVE PKPLPDIIDQ VVGQPQPKTS QQPLQPIQPP QNLSVQQPPK
361 QQSQEKPPQT LFPSIVKNMP TKPNGTLSHK SGRRRWGQTI FKSGDSWEEL EDYDFGASHS
421 KKPSMGVFKE KRKKDSPFRL PEPVPSGSNH STGENKSLPA VTSLKSDSEL STAPTSKQYY
481 LKQSRYLPGV NPKKVSLIAS GKEINPHTWS NQLFPKSLGP VGAELAFKRS NAGNLGSYAT
 
541 YNQSGYIPSF LKKEVQSAGQ RIHLAPLNAT ASEYTWNTKT GRGQFSGRTY NPTAKNLNIV
601 NRAQPIPSVH GRTDWVAKYG GHR
SEQ ID NO:175
   MALE GERM CELL ASSOCIATED KINASE (MAK); RETINAL-ENRICHED ISOFORM
   AEL29206.1 
1 
61 
121 
181 
241 
301 
361 
421 
481 
541 
601 
SEQ ID NO:176
   INTRAFLAGELLAR TRANSPORT 140 (IFT140)
   NP_055529.2 
1 
61 
121 
181 
241 
301 
361 
421 
481 
541 
601 
661 
721 
781 
841 
901 
961 
1021 
1081 
1141 
1201 
1261 
1321 
1381 
1441 
SEQ ID NO:177
   HTRA SERINE PEPTIDASE 1 (HTRA1)
   EAW49312.1 
1 
61 
121 
181 
241 
301 
361 
 421 
SEQ ID NO:178
   BESTROPHIN 1 (BEST1), ISOFORM 1
   NP_004174.1 
1 
61 
121 
181 
241 
301 
361 
421 
481 
541 
SEQ ID NO:179
   BESTROPHIN 1 (BEST1), ISOFORM 2
   NP_001132915.1 
1 MFEKLTLYCD SYIQLIPISF VLGFYVTLVV TRWWNQYENL PWPDRLMSLV SGFVEGKDEQ
61 GRLLRRTLIR YANLGNVLIL RSVSTAVYKR FPSAQHLVQA GFMTPAEHKQ LEKLSLPHNM
121 FWVPWVWFAN LSMKAWLGGR IRDPILLQSL LNEMNTLRTQ CGHLYAYDWI SIPLVYTQVV
181 TVAVYSFFLT CLVGRQFLNP AKAYPGHELD LVVPVFTFLQ FFFYVGWLKV AEQLINPFGE
241 DDDDFETNWI VDRNLQVSLL AVDEMHQDLP RMEPDMYWNK PEPQPPYTAA SAQFRRASFM
301 GSTFNISLNK EEMEFQPNQE DEEDAHAGII GRFLGLQSHD HHPPRANSRT KLLWPKRESL
361 LHEGLPKNHK AAKQNVRGQE DNKAWKLKAV DAFKSAPLYQ RPGYYSAPQT PLSPTPMFFP
421 LEPSAPSKLH SVTGIDTKDK SLKTVSSGAK KSFELLSESD GALMEHPEVS QVRRKTVEFN
481 LTDMPEIPEN HLKEPLEQSP TNIHTTLKDH MDPYWALENR SVLHLNQGHC IALCPTPASL
541 ALSLPFLHNF LGFHHCQSTL DLRPALAWGI YLATFTGILG KCSGPFLTSP WYHPEDFLGP
601 GEGR
SEQ ID NO:180
   BESTROPHIN 1 (BEST1), ISOFORM 3
   ALQ33849.1 
1 MFEKLTLYCD SYIQLIPISF VLGFYVTLVV TRWWNQYENL PWPDRLMSLV SGFVEGKDEQ
61 GRLLRRTLIR YANLGNVLIL RSVSTAVYKR FPSAQHLVQA GFMTPAEHKQ LEKLSLPHNM
121 FWVPWVWFAN LSMKAWLGGR IRDPILLQSL LNEMNTLRTQ CGHLYAYDWI SIPLVYTQVV
181 TVAVYSFFLT CLVGRQFLNP AKAYPGHELD LVVPVFTFLQ FFFYVGWLKV AEQLINPFGE
241 DDDDFETNWI VDRNLQVSLL AVDEMHQDLP RMEPDMYWNK PEPQPPYTAA SAQFRRASFM
301 GSTFNISLNK EEMEFQPNQE DEEDAHAGII GRFLGLQSHD HHPPRANSRT KLLWPKRESL
361 LHEGLPKNHK AAKQNVRGQE DNKAWKLKAV DAFKSAPLYQ RPGYYSAPQT PLSPTPMFFP
421 LEPSAPSKLH SVTGIDTKDK SLKTVSSGAK KSFELLSESD GALMEHPEVS QVRRKTVEFN
481 LTDMPEIPEN HLKEPLEQSP TNIHTTLKDH MDPYWALENR DEAHS
SEQ ID NO:181
   BESTROPHIN 1 (BEST1), ISOFORM 4
   NP_001287716.1 
1 MFEKLTLYCD SYIQLIPISF VLGFYVTLVV TRWWNQYENL PWPDRLMSLV SGFVEGKDEQ
61 GRLLRRTLIR YANLGNVLIL RSVSTAVYKR FPSAQHLVQA GFMTPAEHKQ LEKLSLPHNM
121 FWVPWVWFAN LSMKAWLGGR IRDPILLQSL LNEMNTLRTQ CGHLYAYDWI SIPLVYTQVA
181 EQLINPFGED DDDFETNWIV DRNLQVSLLA VDEMHQDLPR MEPDMYWNKP EPQPPYTAAS
241 AQFRRASFMG STFNISLNKE EMEFQPNQED EEDAHAGIIG RFLGLQSHDH HPPRANSRTK
301 LLWPKRESLL HEGLPKNHKA AKQNVRGQED NKAWKLKAVD AFKSAPLYQR PGYYSAPQTP
361 LSPTPMFFPL EPSAPSKLHS VTGIDTKDKS LKTVSSGAKK SFELLSESDG ALMEHPEVSQ
421 VRRKTVEFNL TDMPEIPENH LKEPLEQSPT NIHTTLKDHM DPYWALENRD EAHS
SEQ ID NO:182
   BESTROPHIN 1 (BEST1), ISOFORM 5
   NP_001350520.1 
1 MSLVSGFVEG KDEQGRLLRR TLIRYANLGN VLILRSVSTA VYKRFPSAQH LVQAGFMTPA
61 EHKQLEKLSL PHNMFWVPWV WFANLSMKAW LGGRIRDPIL LQSLLNEMNT LRTQCGHLYA
121 YDWISIPLVY TQVVTVAVYS FFLTCLVGRQ FLNPAKAYPG HELDLVVPVF TFLQFFFYVG
181 WLKVAEQLIN PFGEDDDDFE TNWIVDRNLQ VSLLAVDEMH QDLPRMEPDM YWNKPEPQPP
241 YTAASAQFRR ASFMGSTFNI SLNKEEMEFQ PNQEDEEDAH AGIIGRFLGL QSHDHHPPRA
301 NSRTKLLWPK RESLLHEGLP KNHKAAKQNV RGQEDNKAWK LKAVDAFKSA PLYQRPGYYS
361 APQTPLSPTP MFFPLEPSAP SKLHSVTGID TKDKSLKTVS SGAKKSFELL SESDGALMEH
421 PEVSQVRRKT VEFNLTDMPE IPENHLKEPL EQSPTNIHTT LKDHMDPYWA LENRSVLHLN
481 QGHCIALCPT PASLALSLPF LHNFLGFHHC QSTLDLRPAL AWGIYLATFT GILGKCSGPF
541 LTSPWYHPED FLGPGEGR
SEQ ID NO:183
   BESTROPHIN 1 (BEST1), ISOFORM 6
   NP_001350521.1 
1 MTITYTSQVA NARLGSFSRL LLCWRGSIYK LLYGEFLIFL LCYYIIRFIY RLALTEEQQL
61 MFEKLTLYCD SYIQLIPISF VLGFYVTLVV TRWWNQYENL PWPDRLMSLV SGFVEGKDEQ
121 GRLLRRTLIR YANLGNVLIL RSVSTAVYKR FPSAQHLVQA GFMTPAEHKQ LEKLSLPHNM
181 FWVPWVWFAN LSMKAWLGGR IRDPILLQSL LNEMNTLRTQ CGHLYAYDWI SIPLVYTQVV
241 TVAVYSFFLT CLVGRQFLNP AKAYPGHELD LVVPVFTFLQ FFFYVGWLKV GLSRALLGWR
301 HGQRGHGQQL PETRMQCQER KVSRVESSQA WWRTPVIPAT REAEAGESLE PGRRRLWWQS
361 SSSTPLERMM MILRPTGLST GICRCPCWLW MRCTRTCLGW SRTCTGISPS HSPPTQLLPP
421 SSVEPPLWAP PSTSA
SEQ ID NO:184
   BESTROPHIN 1 (BEST1), ISOFORM 7
   NP_001350522.1 
1 MHQDLPRMEP DMYWNKPEPQ PPYTAASAQF RRASFMGSTF NISLNKEEME FQPNQEDEED
61 AHAGIIGRFL GLQSHDHHPP RANSRTKLLW PKRESLLHEG LPKNHKAAKQ NVRGQEDNKA
121 WKLKAVDAFK SAPLYQRPGY YSAPQTPLSP TPMFFPLEPS APSKLHSVTG IDTKDKSLKT
181 VSSGAKKSFE LLSESDGALM EHPEVSQVRR KTVEFNLTDM PEIPENHLKE PLEQSPTNIH
241 TTLKDHMDPY WALENRSVLH LNQGHCIALC PTPASLALSL PFLHNFLGFH HCQSTLDLRP
301 ALAWGIYLAT FTGILGKCSG PFLTSPWYHP EDFLGPGEGR
SEQ ID NO:185
   BESTROPHIN 1 (BEST1), ISOFORM 8
   ALQ33852.1
   1 MFEKLTLYCD SYIQLIPISF VLGDEHLAYS VWTPVCLRLD
SEQ ID NO:186
   COMPLEMENT FACTOR B
   AAA16820.1 
1 MGSNLSPQLC LMPFILGLLS GGVTTTPWSL AQPQGSCSLE GVEIKGGSFR LLQEGQALEY
61 VCPSGFYPYP VQTRTCRSTG SWSTLKTQDQ KTVRKAECRA IHCPRPHDFE NGEYWPRSPY
121 YNVSDEISFH CYDGYTLRGS ANRTCQVNGR WSGQTAICDN GAGYCSNPGI PIGTRKVGSQ
181 YRLEDSVTYH CSRGLTLRGS QRRTCQEGGS WSGTEPSCQD SFMYDTPQEV AEAFLSSLTE
241 TIEGVDAEDG HGPGEQQKRK IVLDPSGSMN IYLVLDGSDS IGASNFTGAK KCLVNLIEKV
301 ASYGVKPRYG LVTYATYPKI WVKVSEADSS NADWVTKQLN EINYEDHKLK SGTNTKKALQ
361 AVYSMMSWPD DVPPEGWNRT RHVIILMTDG LHNMGGDPIT VIDEIRDLLY IGKDRKNPRE
421 DYLDVYVFGV GPLVNQVNIN ALASKKDNEQ HVFKVKDMEN LEDVFYQMID ESQSLSLCGM
481 VWEHRKGTDY HKQPWQAKIS VIRPSKGHES CMGAVVSEYF VLTAAHCFTV DDKEHSIKVS
541 VGGEKRDLEI EVVLFHPNYN INGKKEAGIP EFYDYDVALI KLKNKLKYGQ TIRPICLPCT
601 EGTTRALRLP PTTTCQQQKE ELLPAQDIKA LFVSEEEKKL TRKEVYIKNG DKKGSCERDA
661 QYAPGYDKVK DISEVVTPRF LCTGGVSPYA DPNTCRGDSG GPLIVHKRSR FIQVGVISWG
721 VVDVCKNQKR QKQVPAHARD FHINLFQVLP WLKEKLQDED LGFL
SEQ ID NO:187
   BETA-AMYLOID, PARTIAL
   AAB29908.1
   1 DAEFRHDSGY EVHHQKLVFF AEDVGSNKGA
SEQ ID NO:188
   BETA-AMYLOID, PARTIAL
   AAB26264.2 
1 GSGLTNIKTE EISEVKMDAE FRHDSGYEVH HQKLVFFAED VGSNKGAIIG LMVGGVVIAT
61 VIIITLVMLK KQYTSNHHGV VE
SEQ ID NO:189
   CD59 GLYCOPROTEIN (CD59)
   NP_001120697.1 
  1 MGIQGGSVLF GLLLVLAVFC HSGHSLQCYN CPNPTADCKT AVNCSSDFDA CLITKAGLQV
 61 YNKCWKFEHC NFNDVTTRLR ENELTYYCCK KDLCNFNEQL ENGGTSLSEK TVLLLVTPFL
121 AAAWSLHP
SEQ ID NO:190
   CHANNELRHODOPSIN-1 (CHR1) [VOLVOX CARTERI F. NAGARIENSIS]
   ABZ90901.1 
1 MDYPVARSLI VRYPTDLGNG TVCMPRGQCY CEGWLRSRGT SIEKTIAITL QWVVFALSVA
61 CLGWYAYQAW RATCGWEEVY VALIEMMKSI IEAFHEFDSP ATLWLSSGNG VVWMRYGEWL
121 LTCPVLLIHL SNLTGLKDDY SKRTMGLLVS DVGCIVWGAT SAMCTGWTKI LFFLISLSYG
181 MYTYFHAAKV YIEAFHTVPK GICRELVRVM AWTFFVAWGM FPVLFLLGTE GFGHISPYGS
241 AIGHSILDLI AKNMWGVLGN YLRVKIHEHI LLYGDIRKKQ KITIAGQEME VETLVAEEED
301 DTVKQSTAKY ASRDSFITMR NRMREKGLEV RASLDAGGGD SGMEAGGGGA AHAQPHMAKP
361 GTELGKTMSA SFTNGAATSL EPGRVILAVP DISMVDFFRE QFAQLPVPYE VVPALGAENT
421 VQLVQQAAML GGCDFVLMHP EFLRDRGPTG LLPQVKMMGQ RTAAFGWSQM GPMRDLIESS
481 GVGAWLEGPS FGSGISQAAL QQLVVKMQQA KRMAAMGSMM GGGMGNGMGM GMGMGMGMGM
541 GNGMGNGMGM GNGMGNGMGM GNGMGNGMGM GNGMGMGNGM GMGNGMGMGN GMGNGMGNGM
601 GMGNGMGNGM GNGMGNGMGN GMGNGMGMGN GMGMGNGMGN GMGNGMGNGM GNGMGMMTPG
661 AMGMGMGGMG NLAAAAGNAM YGGGGGGGGS TMGSGNAAMM TGLVMGGGNG VGAGPGGVVA
721 NLGSSALQPQ SQMMGGGNVV GMSSPQLQLQ QSSSMPLGGL APNRIGNNPL FGAAPSPLHS
781 QPGASPTGLS SPQLGMGAML PAGTSVGAGG GSVGPTETDM LQQLMTEINR LKDELGE
SEQ ID NO:191
   CHANNELRHODOPSIN-2 (CHR2) [VOLVOX CARTERI F. NAGARIENSIS
   ABZ90903.1 
1 MDHPVARSLI GSSYTNLNNG SIVIPSDACF CMKWLKSKGS PVALKMANAL QWAAFALSVI
61 ILIYYAYATW RTTCGWEEVY VCCVELTKVV IEFFHEFDEP GMLYLANGNR VLWLRYGEWL
121 LTCPVILIHL SNLTGLKDDY NKRTMRLLVS DVGTIVWGAT AAMSTGYIKV IFFLLGCMYG
181 ANTFFHAAKV YIESYHTVPK GLCRQLVRAM AWLFFVSWGM FPVLFLLGPE GFGHLSVYGS
241 TIGHTIIDLL SKNCWGLLGH FLRLKIHEHI LLYGDIRKVQ KIRVAGEELE VETLMTEEAP
301 DTVKKSTAQY ANRESFLTMR DKLKEKGFEV RASLDNSGID AVINHNNNYN NALANAAAAV
361 GKPGMELSKL DHVAANAAGM GGIADHVATT SGAISPGRVI LAVPDISMVD YFREQFAQLP
421 VQYEVVPALG ADNAVQLVVQ AAGLGGCDFV LLHPEFLRDK SSTSLPARLR SIGQRVAAFG
481 WSPVGPVRDL IESAGLDGWL EGPSFGLGIS LPNLASLVLR MQHARKMAAM LGGMGGMLGS
541 NLMSGSGGVG LMGAGSPGGG GGAMGVGMTG MGMVGTNAMG RGAVGNSVAN ASMGGGSAGM
601 GMGMMGMVGA GVGGQQQMGA NGMGPTSFQL GSNPLYNTAP SPLSSQPGGD ASAAAAAAAA
661 AAATGAASNS MNAMQAGGSV RNSGILAGGL GSMMGPPGAP AAPTAAATAA PAVTMGAPGG
721 GGAAASEAEM LQQLMAEINR LKSELGE
SEQ ID NO:192
   COMPLEMENT FACTOR C5, ISOFORM 1
   NP_001726.2 1 MGLLGILCFL IFLGKTWGQE QTYVISAPKI FRVGASENIV IQVYGYTEAF DATISIKSYP 
61 DKKFSYSSGH VHLSSENKFQ NSAILTIQPK QLPGGQNPVS YVYLEVVSKH FSKSKRMPIT
121 YDNGFLFIHT DKPVYTPDQS VKVRVYSLND DLKPAKRETV LTFIDPEGSE VDMVEEIDHI
181 GIISFPDFKI PSNPRYGMWT IKAKYKEDFS TTGTAYFEVK EYVLPHFSVS IEPEYNFIGY
241 KNFKNFEITI KARYFYNKVV TEADVYITFG IREDLKDDQK EMMQTAMQNT MLINGIAQVT
301 FDSETAVKEL SYYSLEDLNN KYLYIAVTVI ESTGGFSEEA EIPGIKYVLS PYKLNLVATP
361 LFLKPGIPYP IKVQVKDSLD QLVGGVPVTL NAQTIDVNQE TSDLDPSKSV TRVDDGVASF
421 VLNLPSGVTV LEFNVKTDAP DLPEENQARE GYRAIAYSSL SQSYLYIDWT DNHKALLVGE
481 HLNIIVTPKS PYIDKITHYN YLILSKGKII HFGTREKFSD ASYQSINIPV TQNMVPSSRL
541 LVYYIVTGEQ TAELVSDSVW LNIEEKCGNQ LQVHLSPDAD AYSPGQTVSL NMATGMDSWV
601 ALAAVDSAVY GVQRGAKKPL ERVFQFLEKS DLGCGAGGGL NNANVFHLAG LTFLTNANAD
661 DSQENDEPCK EILRPRRTLQ KKIEEIAAKY KHSVVKKCCY DGACVNNDET CEQRAARISL
721 GPRCIKAFTE CCVVASQLRA NISHKDMQLG RLHMKTLLPV SKPEIRSYFP ESWLWEVHLV
781 PRRKQLQFAL PDSLTTWEIQ GVGISNTGIC VADTVKAKVF KDVFLEMNIP YSVVRGEQIQ
841 LKGTVYNYRT SGMQFCVKMS AVEGICTSES PVIDHQGTKS SKCVRQKVEG SSSHLVTFTV
901 LPLEIGLHNI NFSLETWFGK EILVKTLRVV PEGVKRESYS GVTLDPRGIY GTISRRKEFP
961 YRIPLDLVPK TEIKRILSVK GLLVGEILSA VLSQEGINIL THLPKGSAEA ELMSVVPVFY
1021 VFHYLETGNH WNIFHSDPLI EKQKLKKKLK EGMLSIMSYR NADYSYSVWK GGSASTWLTA
1081 FALRVLGQVN KYVEQNQNSI CNSLLWLVEN YQLDNGSFKE NSQYQPIKLQ GTLPVEAREN
1141 SLYLTAFTVI GIRKAFDICP LVKIDTALIK ADNFLLENTL PAQSTFTLAI SAYALSLGDK
1201 THPQFRSIVS ALKREALVKG NPPIYRFWKD NLQHKDSSVP NTGTARMVET TAYALLTSLN
1261 LKDINYVNPV IKWLSEEQRY GGGFYSTQDT INAIEGLTEY SLLVKQLRLS MDIDVSYKHK
1321 GALHNYKMTD KNFLGRPVEV LLNDDLIVST GFGSGLATVH VTTVVHKTST SEEVCSFYLK
1381 IDTQDIEASH YRGYGNSDYK RIVACASYKP SREESSSGSS HAVMDISLPT GISANEEDLK
1441 ALVEGVDQLF TDYQIKDGHV ILQLNSIPSS DFLCVRFRIF ELFEVGFLSP ATFTVYEYHR
1501 PDKQCTMFYS TSNIKIQKVC EGAACKCVEA DCGQMQEELD LTISAETRKQ TACKPEIAYA
1561 YKVSITSITV ENVFVKYKAT LLDIYKTGEA VAEKDSEITF IKKVTCTNAE LVKGRQYLIM
1621 GKEALQIKYN FSFRYIYPLD SLTWIEYWPR DTTCSSCQAF LANLDEFAED IFLNGC
SEQ ID NO:193
   COMPLEMENT FACTOR C5, ISOFORM 2
   NP_001304092.1 
1 MPGSLGREAS GRAGPTGCGA FAFGLRCRYV ISAPKIFRVG ASENIVIQVY GYTEAFDATI
61 SIKSYPDKKF SYSSGHVHLS SENKFQNSAI LTIQPKQLPG GQNPVSYVYL EVVSKHFSKS
121 KRMPITYDNG FLFIHTDKPV YTPDQSVKVR VYSLNDDLKP AKRETVLTFI DPEGSEVDMV
181 EEIDHIGIIS FPDFKIPSNP RYGMWTIKAK YKEDFSTTGT AYFEVKEYVL PHFSVSIEPE
241 YNFIGYKNFK NFEITIKARY FYNKVVTEAD VYITFGIRED LKDDQKEMMQ TAMQNTMLIN
301 GIAQVTFDSE TAVKELSYYS LEDLNNKYLY IAVTVIESTG GFSEEAEIPG IKYVLSPYKL
361 NLVATPLFLK PGIPYPIKVQ VKDSLDQLVG GVPVTLNAQT IDVNQETSDL DPSKSVTRVD
421 DGVASFVLNL PSGVTVLEFN VKTDAPDLPE ENQAREGYRA IAYSSLSQSY LYIDWTDNHK
481 ALLVGEHLNI IVTPKSPYID KITHYNYLIL SKGKIIHFGT REKFSDASYQ SINIPVTQNM
541 VPSSRLLVYY IVTGEQTAEL VSDSVWLNIE EKCGNQLQVH LSPDADAYSP GQTVSLNMAT
601 GMDSWVALAA VDSAVYGVQR GAKKPLERVF QFLEKSDLGC GAGGGLNNAN VFHLAGLTFL
661 TNANADDSQE NDEPCKEILR PRRTLQKKIE EIAAKYKHSV VKKCCYDGAC VNNDETCEQR
721 AARISLGPRC IKAFTECCVV ASQLRANISH KDMQLGRLHM KTLLPVSKPE IRSYFPESWL
781 WEVHLVPRRK QLQFALPDSL TTWEIQGVGI SNTGICVADT VKAKVFKDVF LEMNIPYSVV
841 RGEQIQLKGT VYNYRTSGMQ FCVKMSAVEG ICTSESPVID HQGTKSSKCV RQKVEGSSSH
901 LVTFTVLPLE IGLHNINFSL ETWFGKEILV KTLRVVPEGV KRESYSGVTL DPRGIYGTIS
961 RRKEFPYRIP LDLVPKTEIK RILSVKGLLV GEILSAVLSQ EGINILTHLP KGSAEAELMS
1021 VVPVFYVFHY LETGNHWNIF HSDPLIEKQK LKKKLKEGML SIMSYRNADY SYSVWKGGSA
1081 STWLTAFALR VLGQVNKYVE QNQNSICNSL LWLVENYQLD NGSFKENSQY QPIKLQGTLP
1141 VEARENSLYL TAFTVIGIRK AFDICPLVKI DTALIKADNF LLENTLPAQS TFTLAISAYA
1201 LSLGDKTHPQ FRSIVSALKR EALVKGNPPI YRFWKDNLQH KDSSVPNTGT ARMVETTAYA
1261 LLTSLNLKDI NYVNPVIKWL SEEQRYGGGF YSTQDTINAI EGLTEYSLLV KQLRLSMDID
1321 VSYKHKGALH NYKMTDKNFL GRPVEVLLND DLIVSTGFGS GLATVHVTTV VHKTSTSEEV
1381 CSFYLKIDTQ DIEASHYRGY GNSDYKRIVA CASYKPSREE SSSGSSHAVM DISLPTGISA
1441 NEEDLKALVE GVDQLFTDYQ IKDGHVILQL NSIPSSDFLC VRFRIFELFE VGFLSPATFT
1501 VYEYHRPDKQ CTMFYSTSNI KIQKVCEGAA CKCVEADCGQ MQEELDLTIS AETRKQTACK
 
1561 PEIAYAYKVS ITSITVENVF VKYKATLLDI YKTGEAVAEK DSEITFIKKV TCTNAELVKG
1621 RQYLIMGKEA LQIKYNFSFR YIYPLDSLTW IEYWPRDTTC SSCQAFLANL DEFAEDIFLN
1681 GC
SEQ ID NO:194
   COMPLEMENT FACTOR C5, ISOFORM 2
   NP_001304093.1 
1 MGLLGILCFL IFLGKTWGQE QTYVISAPKI FRVGASENIV IQVYGYTEAF DATISIKSYP
61 DKKFSYSSGH VHLSSENKFQ NSAILTIQPK QLPGGQNPVS YVYLEVVSKH FSKSKRMPIT
121 YDNGFLFIHT DKPVYTPDQS VKVRVYSLND DLKPAKRETV LTFIDPEGSE VDMVEEIDHI
181 GIISFPDFKI PSNPRYGMWT IKAKYKEDFS TTGTAYFEVK EYVLPHFSVS IEPEYNFIGY
241 KNFKNFEITI KARYFYNKVV TEADVYITFG IREDLKDDQK EMMQTAMQNT MLINGIAQVT
301 FDSETAVKEL SYYSLEDLNN KYLYIAVTVI ESTGGFSEEA EIPGIKYVLS PYKLNLVATP
361 LFLKPGIPYP IKVQVKDSLD QLVGGVPVTL NAQTIDVNQE TSDLDPSKSV TRVDDGVASF
421 VLNLPSGVTV LEFNVKTDAP DLPEENQARE GYRAIAYSSL SQSYLYIDWT DNHKALLVGE
481 HLNIIVTPKS PYIDKITHYN YLILSKGKII HFGTREKFSD ASYQSINIPV TQNMVPSSRL
541 LVYYIVTGEQ TAELVSDSVW LNIEEKCGNQ LQVHLSPDAD AYSPGQTVSL NMATGMDSWV
601 ALAAVDSAVY GVQRGAKKPL ERVFQFLEKS DLGCGAGGGL NNANVFHLAG LTFLTNANAD
661 DSQENDEPCK EILRPRRTLQ KKIEEIAAKY KHSVVKKCCY DGACVNNDET CEQRAARISL
721 GPRCIKAFTE CCVVASQLRA NISHKDMQLG RLHMKTLLPV SKPEIRSYFP ESWLWEVHLV
781 PRRKQLQFAL PDSLTTWEIQ GVGISNTGIC VADTVKAKVF KDVFLEMNIP YSVVRGEQIQ
841 LKGTVYNYRT SGMQSLALSP RLECNGKISG HCKLRLPGSS DSPASASQVA GITGTHHHAQ
901 PT
SEQ ID NO:195
   COMPLEMENT FACTOR C5A
   3PVM A 
1 MGLLGILCFL IFLGKTWGQE QTYVISAPKI FRVGASENIV IQVYGYTEAF DATISIKSYP
61 DKKFSYSSGH VHLSSENKFQ NSAILTIQPK QLPGGQNPVS YVYLEVVSKH FSKSKRMPIT
121 YDNGFLFIHT DKPVYTPDQS VKVRVYSLND DLKPAKRETV LTFIDPEGSE VDMVEEIDHI
181 GIISFPDFKI PSNPRYGMWT IKAKYKEDFS TTGTAYFEVK EYVLPHFSVS IEPEYNFIGY
241 KNFKNFEITI KARYFYNKVV TEADVYITFG IREDLKDDQK EMMQTAMQNT MLINGIAQVT
301 FDSETAVKEL SYYSLEDLNN KYLYIAVTVI ESTGGFSEEA EIPGIKYVLS PYKLNLVATP
361 LFLKPGIPYP IKVQVKDSLD QLVGGVPVTL NAQTIDVNQE TSDLDPSKSV TRVDDGVASF
421 VLNLPSGVTV LEFNVKTDAP DLPEENQARE GYRAIAYSSL SQSYLYIDWT DNHKALLVGE
481 HLNIIVTPKS PYIDKITHYN YLILSKGKII HFGTREKFSD ASYQSINIPV TQNMVPSSRL
541 LVYYIVTGEQ TAELVSDSVW LNIEEKCGNQ LQVHLSPDAD AYSPGQTVSL NMATGMDSWV
601 ALAAVDSAVY GVQRGAKKPL ERVFQFLEKS DLGCGAGGGL NNANVFHLAG LTFLTNANAD
661 DSQENDEPCK EILRPRRTLQ KKIEEIAAKY KHSVVKKCCY DGACVNNDET CEQRAARISL
721 GPRCIKAFTE CCVVASQLRA NISHKDMQLG RLHMKTLLPV SKPEIRSYFP ESWLWEVHLV
781 PRRKQLQFAL PDSLTTWEIQ GVGISNTGIC VADTVKAKVF KDVFLEMNIP YSVVRGEQIQ
841 LKGTVYNYRT SGMQFCVKMS AVEGICTSES PVIDHQGTKS SKCVRQKVEG SSSHLVTFTV
901 LPLEIGLHNI NFSLETWFGK EILVKTLRVV PEGVKRESYS GVTLDPRGIY GTISRRKEFP
961 YRIPLDLVPK TEIKRILSVK GLLVGEILSA VLSQEGINIL THLPKGSAEA ELMSVVPVFY
1021 VFHYLETGNH WNIFHSDPLI EKQKLKKKLK EGMLSIMSYR NADYSYSVWK GGSASTWLTA
1081 FALRVLGQVN KYVEQNQNSI CNSLLWLVEN YQLDNGSFKE NSQYQPIKLQ GTLPVEAREN
1141 SLYLTAFTVI GIRKAFDICP LVKIDTALIK ADNFLLENTL PAQSTFTLAI SAYALSLGDK
1201 THPQFRSIVS ALKREALVKG NPPIYRFWKD NLQHKDSSVP NTGTARMVET TAYALLTSLN
1261 LKDINYVNPV IKWLSEEQRY GGGFYSTQDT INAIEGLTEY SLLVKQLRLS MDIDVSYKHK
1321 GALHNYKMTD KNFLGRPVEV LLNDDLIVST GFGSGLATVH VTTVVHKTST SEEVCSFYLK
1381 IDTQDIEASH YRGYGNSDYK RIVACASYKP SREESSSGSS HAVMDISLPT GISANEEDLK
1441 ALVEGVDQLF TDYQIKDGHV ILQLNSIPSS DFLCVRFRIF ELFEVGFLSP ATFTVYEYHR
1501 PDKQCTMFYS TSNIKIQKVC EGAACKCVEA DCGQMQEELD LTISAETRKQ TACKPEIAYA
1561 YKVSITSITV ENVFVKYKAT LLDIYKTGEA VAEKDSEITF IKKVTCTNAE LVKGRQYLIM
1621 GKEALQIKYN FSFRYIYPLD SLTWIEYWPR DTTCSSCQAF LANLDEFAED IFLNGC
SEQ ID NO:196
   COMPLEMENT FACTOR D, ISOFORM 1
   NP_001919.2 
1 MHSWERLAVL VLLGAAACAA PPRGRILGGR EAEAHARPYM ASVQLNGAHL CGGVLVAEQW
61 VLSAAHCLED AADGKVQVLL GAHSLSQPEP SKRLYDVLRA VPHPDSQPDT IDHDLLLLQL
121 SEKATLGPAV RPLPWQRVDR DVAPGTLCDV AGWGIVNHAG RRPDSLQHVL LPVLDRATCN
181 RRTHHDGAIT ERLMCAESNR RDSCKGDSGG PLVCGGVLEG VVTSGSRVCG NRKKPGIYTR
241 VASYAAWIDS VLA
SEQ ID NO:197
   COMPLEMENT FACTOR D, ISOFORM 2
   NP_001304264.1 
1 MHSWERLAVL VLLGAAACGE EAWAWAAPPR GRILGGREAE AHARPYMASV QLNGAHLCGG
61 VLVAEQWVLS AAHCLEDAAD GKVQVLLGAH SLSQPEPSKR LYDVLRAVPH PDSQPDTIDH
121 DLLLLQLSEK ATLGPAVRPL PWQRVDRDVA PGTLCDVAGW GIVNHAGRRP DSLQHVLLPV
181 LDRATCNRRT HHDGAITERL MCAESNRRDS CKGDSGGPLV CGGVLEGVVT SGSRVCGNRK
241 KPGIYTRVAS YAAWIDSVLA
SEQ ID NO:198
   DNAJ HEAT SHOCK PROTEIN FAMILY (HSP40) MEMBER C3 (DNAJC3), ALSO KNOWN AS P58IPK
   NP_006251.1 
1 MVAPGSVTSR LGSVFPFLLV LVDLQYEGAE CGVNADVEKH LELGKKLLAA GQLADALSQF
61 HAAVDGDPDN YIAYYRRATV FLAMGKSKAA LPDLTKVIQL KMDFTAARLQ RGHLLLKQGK
121 LDEAEDDFKK VLKSNPSENE EKEAQSQLIK SDEMQRLRSQ ALNAFGSGDY TAAIAFLDKI
181 LEVCVWDAEL RELRAECFIK EGEPRKAISD LKAASKLKND NTEAFYKIST LYYQLGDHEL
241 SLSEVRECLK LDQDHKRCFA HYKQVKKLNK LIESAEELIR DGRYTDATSK YESVMKTEPS
301 IAEYTVRSKE RICHCFSKDE KPVEAIRVCS EVLQMEPDNV NALKDRAEAY LIEEMYDEAI
361 QDYETAQEHN ENDQQIREGL EKAQRLLKQS QKRDYYKILG VKRNAKKQEI IKAYRKLALQ
421 WHPDNFQNEE EKKKAEKKFI DIAAAKEVLS DPEMRKKFDD GEDPLDAESQ QGGGGNPFHR
481 SWNSWQGFNP FSSGGPFRFK FHFN
SEQ ID NO:199
   DNAJ HEAT SHOCK PROTEIN FAMILY (HSP40) MEMBER C3 (DNAJC3), ALSO KNOWN AS P58IPK, ISOFORM X1
   XP_011519406.1 
1 MVAPGSVTSR LGSVFPFLLV LVDLQYEGAE CGVNADVEKH LELGKKLLAA GQLADALSQF
61 HAAVDGDPDN YIAYYRRATV FLAMGKSKAA LPDLTKVIQL KMDFTAARLQ RGHLLLKQGK
121 LDEAEDDFKK VVFPVPSLLG LQRSLLDDLY LLFWFFLMKK LKSNPSENEE KEAQSQLIKS
181 DEMQRLRSQA LNAFGSGDYT AAIAFLDKIL EVCVWDAELR ELRAECFIKE GEPRKAISDL
241 KAASKLKNDN TEAFYKISTL YYQLGDHELS LSEVRECLKL DQDHKRCFAH YKQVKKLNKL
301 IESAEELIRD GRYTDATSKY ESVMKTEPSI AEYTVRSKER ICHCFSKDEK PVEAIRVCSE
361 VLQMEPDNVN ALKDRAEAYL IEEMYDEAIQ DYETAQEHNE NDQQIREGLE KAQRLLKQSQ
421 KRDYYKILGV KRNAKKQEII KAYRKLALQW HPDNFQNEEE KKKAEKKFID IAAAKEVLSD
481 PEMRKKFDDG EDPLDAESQQ GGGGNPFHRS WNSWQGFNPF SSGGPFRFKF HFN
SEQ ID NO:200
   DNAJ HEAT SHOCK PROTEIN FAMILY (HSP40) MEMBER C3 (DNAJC3), ALSO KNOWN AS P58IPK, ISOFORM X2
   XP_011519407.1 
1 MGKSKAALPD LTKVIQLKMD FTAARLQRGH LLLKQGKLDE AEDDFKKVVF PVPSLLGLQR
61 SLLDDLYLLF WFFLMKKLKS NPSENEEKEA QSQLIKSDEM QRLRSQALNA FGSGDYTAAI
121 AFLDKILEVC VWDAELRELR AECFIKEGEP RKAISDLKAA SKLKNDNTEA FYKISTLYYQ
181 LGDHELSLSE VRECLKLDQD HKRCFAHYKQ VKKLNKLIES AEELIRDGRY TDATSKYESV
241 MKTEPSIAEY TVRSKERICH CFSKDEKPVE AIRVCSEVLQ MEPDNVNALK DRAEAYLIEE
301 MYDEAIQDYE TAQEHNENDQ QIREGLEKAQ RLLKQSQKRD YYKILGVKRN AKKQEIIKAY
361 RKLALQWHPD NFQNEEEKKK AEKKFIDIAA AKEVLSDPEM RKKFDDGEDP LDAESQQGGG
421 GNPFHRSWNS WQGFNPFSSG GPFRFKFHFN
SEQ ID NO:201
   DNAJ HEAT SHOCK PROTEIN FAMILY (HSP40) MEMBER C3 (DNAJC3), ALSO KNOWN AS P58IPK, ISOFORM X3
   XP_016876163.1 
1 MGKSKAALPD LTKVIQLKMD FTAARLQRGH LLLKQGKLDE AEDDFKKVLK SNPSENEEKE
61 AQSQLIKSDE MQRLRSQALN AFGSGDYTAA IAFLDKILEV CVWDAELREL RAECFIKEGE
121 PRKAISDLKA ASKLKNDNTE AFYKISTLYY QLGDHELSLS EVRECLKLDQ DHKRCFAHYK
181 QVKKLNKLIE SAEELIRDGR YTDATSKYES VMKTEPSIAE YTVRSKERIC HCFSKDEKPV
241 EAIRVCSEVL QMEPDNVNAL KDRAEAYLIE EMYDEAIQDY ETAQEHNEND QQIREGLEKA
301 QRLLKQSQKR DYYKILGVKR NAKKQEIIKA YRKLALQWHP DNFQNEEEKK KAEKKFIDIA
361 AAKEVLSDPE MRKKFDDGED PLDAESQQGG GGNPFHRSWN SWQGFNPFSS GGPFRFKFHF
421 N
SEQ ID NO:202
   DNAJ HEAT SHOCK PROTEIN FAMILY (HSP40) MEMBER C3 (DNAJC3), ALSO KNOWN AS P58IPK, ISOFORM X4
   XP_016876164.1 
1 MCFLHFFKKV LKSNPSENEE KEAQSQLIKS DEMQRLRSQA LNAFGSGDYT AAIAFLDKIL
61 EVCVWDAELR ELRAECFIKE GEPRKAISDL KAASKLKNDN TEAFYKISTL YYQLGDHELS
121 LSEVRECLKL DQDHKRCFAH YKQVKKLNKL IESAEELIRD GRYTDATSKY ESVMKTEPSI
181 AEYTVRSKER ICHCFSKDEK PVEAIRVCSE VLQMEPDNVN ALKDRAEAYL IEEMYDEAIQ
241 DYETAQEHNE NDQQIREGLE KAQRLLKQSQ KRDYYKILGV KRNAKKQEII KAYRKLALQW
301 HPDNFQNEEE KKKAEKKFID IAAAKEVLSD PEMRKKFDDG EDPLDAESQQ GGGGNPFHRS
361 WNSWQGFNPF SSGGPFRFKF HFN
SEQ ID NO:203
   BETA-2 ADRENOCEPTOR
   NP_000015.1 
1 MGQPGNGSAF LLAPNRSHAP DHDVTQQRDE VWVVGMGIVM SLIVLAIVFG NVLVITAIAK
61 FERLQTVTNY FITSLACADL VMGLAVVPFG AAHILMKMWT FGNFWCEFWT SIDVLCVTAS
121 IETLCVIAVD RYFAITSPFK YQSLLTKNKA RVIILMVWIV SGLTSFLPIQ MHWYRATHQE
181 AINCYANETC CDFFTNQAYA IASSIVSFYV PLVIMVFVYS RVFQEAKRQL QKIDKSEGRF
241 HVQNLSQVEQ DGRTGHGLRR SSKFCLKEHK ALKTLGIIMG TFTLCWLPFF IVNIVHVIQD
301 NLIRKEVYIL LNWIGYVNSG FNPLIYCRSP DFRIAFQELL CLRRSSLKAY GNGYSSNGNT
361 GEQSGYHVEQ EKENKLLCED LPGTEDFVGH QGTVPSDNID SQGRNCSTND SLL
SEQ ID NO:204
   CASPASE-2 (CASP2)
   AAX36439.1 
61 PKRGPQAFDA FCEALRETKQ GHLEDMLLTT LSGLQHVLPP LSCDYDLSLP FPVCESCPLY
121 KKLRLSTDTV EHSLDNKDGP VCLQVKPCTP EFYQTHFQLA YRLQSRPRGL ALVLSNVHFT
181 GEKELEFRSG GDVDHSTLVT LFKLLGYDVH VLCDQTAQEM QEKLQNFAQL PAHRVTDSCI
241 VALLSHGVEG AIYGVDGKLL QLQEVFQLFD NANCPSLQNK PKMFFIQACR GGAIGSLGHL
301 LLFTAATASL AL
SEQ ID NO:205
   INSULIN RECEPTOR SUBSTRATE 1 (IRS1)
   NP_005535.1 
1 MASPPESDGF SDVRKVGYLR KPKSMHKRFF VLRAASEAGG PARLEYYENE KKWRHKSSAP
61 KRSIPLESCF NINKRADSKN KHLVALYTRD EHFAIAADSE AEQDSWYQAL LQLHNRAKGH
121 HDGAAALGAG GGGGSCSGSS GLGEAGEDLS YGDVPPGPAF KEVWQVILKP KGLGQTKNLI
181 GIYRLCLTSK TISFVKLNSE AAAVVLQLMN IRRCGHSENF FFIEVGRSAV TGPGEFWMQV
241 DDSVVAQNMH ETILEAMRAM SDEFRPRSKS QSSSNCSNPI SVPLRRHHLN NPPPSQVGLT
301 RRSRTESITA TSPASMVGGK PGSFRVRASS DGEGTMSRPA SVDGSPVSPS TNRTHAHRHR
361 GSARLHPPLN HSRSIPMPAS RCSPSATSPV SLSSSSTSGH GSTSDCLFPR RSSASVSGSP
421 SDGGFISSDE YGSSPCDFRS SFRSVTPDSL GHTPPARGEE ELSNYICMGG KGPSTLTAPN
 
481 GHYILSRGGN GHRCTPGTGL GTSPALAGDE AASAADLDNR FRKRTHSAGT SPTITHQKTP
541 SQSSVASIEE YTEMMPAYPP GGGSGGRLPG HRHSAFVPTR SYPEEGLEMH PLERRGGHHR
601 PDSSTLHTDD GYMPMSPGVA PVPSGRKGSG DYMPMSPKSV SAPQQIINPI RRHPQRVDPN
661 GYMMMSPSGG CSPDIGGGPS SSSSSSNAVP SGTSYGKLWT NGVGGHHSHV LPHPKPPVES
721 SGGKLLPCTG DYMNMSPVGD SNTSSPSDCY YGPEDPQHKP VLSYYSLPRS FKHTQRPGEP
781 EEGARHQHLR LSTSSGRLLY AATADDSSSS TSSDSLGGGY CGARLEPSLP HPHHQVLQPH
841 LPRKVDTAAQ TNSRLARPTR LSLGDPKAST LPRAREQQQQ QQPLLHPPEP KSPGEYVNIE
901 FGSDQSGYLS GPVAFHSSPS VRCPSQLQPA PREEETGTEE YMKMDLGPGR RAAWQESTGV
961 EMGRLGPAPP GAASICRPTR AVPSSRGDYM TMQMSCPRQS YVDTSPAAPV SYADMRTGIA
1021 AEEVSLPRAT MAAASSSSAA SASPTGPQGA AELAAHSSLL GGPQGPGGMS AFTRVNLSPN
1081 RNQSAKVIRA DPQGCRRRHS SETFSSTPSA TRVGNTVPFG AGAAVGGGGG SSSSSEDVKR
1141 HSSASFENVW LRPGELGGAP KEPAKLCGAA GGLENGLNYI DLDLVKDFKQ CPQECTPEPQ
1201 PPPPPPPHQP LGSGESSSTR RSSEDLSAYA SISFQKQPED RQ
SEQ ID NO:206
   HIF-1 RESPONSIVE PROTEIN RTP801 (RTP801)
   Q9NX09.1 
1 MPSLWDRFSS SSTSSSPSSL PRTPTPDRPP RSAWGSATRE EGFDRSTSLE SSDCESLDSS
61 NSGFGPEEDT AYLDGVSLPD FELLSDPEDE HLCANLMQLL QESLAQARLG SRRPARLLMP
121 SQLVSQVGKE LLRLAYSEPC GLRGALLDVC VEQGKSCHSV GQLALDPSLV PTFQLTLVLR
181 LDSRLWPKIQ GLFSSANSPF LPGFSQSLTL STGFRVIKKK LYSSEQLLIE EC
SEQ ID NO:207
   TRANSFORMING GROWTH FACTOR BETA 2 (TGFB2)
   AAH99635.1 
1 
61 
121 
181 
241 
301 
361 
SEQ ID NO:208
   TRANSFORMING GROWTH FACTOR BETA 2 (TGFB2), ISOFORM CRA_A
   EAW93326.1 
1 MHYCVLSAFL ILHLVTVALS LSTCSTLDMD QFMRKRIEAI RGQILSKLKL TSPPEDYPEP
61 EEVPPEVISI YNSTRDLLQE KASRRAAACE RERSDEEYYA KEVYKIDMPP FFPSETVCPV
121 VTTPSGSVGS LCSRQSQVLC GYLDAIPPTF YRPYFRIVRF DVSAMEKNAS NLVKAEFRVF
181 RLQNPKARVP EQRIELYQIL KSKDLTSPTQ RYIDSKVVKT RAEGEWLSFD VTDAVHEWLH
241 HKDRNLGFKI SLHCPCCTFV PSNNYIIPNK SEELEARFAG IDGTSTYTSG DQKTIKSTRK
301 KNSGKTPHLL LMLLPSYRLE SQQTNRRKKR ALDAAYCFRN VQDNCCLRPL YIDFKRDLGW
361 KWIHEPKGYN ANFCAGACPY LWSSDTQHSR VLSLYNTINP EASASPCCVS QDLEPLTILY
421 YIGKTPKIEQ LSNMIVKSCK CS
SEQ ID NO:209
   TRANSFORMING GROWTH FACTOR BETA 2 (TGFB2), ISOFORM CPA_B
   EAW93327.1 
1 
61 
121 
181 
241 
301 
361 
SEQ ID NO:210
   BRAIN DERIVED NEUROTROPHIC FACTOR (BDNF)
   AAO15434.1 
1 MTILFLTMVI SYFGCMKAAP MKEANIRGQG GLAYPGVRTH GTLESVNGPK AGSRGLTSLA
61 DTFEHVIEEL LDEDQKVRPN EENNKDADLY TSRVMLSSQV PLEPPLLFLL EEYKNYLDAA
121 NMSMRVRRHS DPARRGELSV CDSISEWVTA ADKKTAVDMS GGTVTVLEKV PVSKGQLKQY
181 FYETKCNPMG YTKEGCRGID KRHWNSQCRT TQSYVRALTM DSKKRIGWRF IRIDTSCVCT
241 LTIKRGR
SEQ ID NO:211
   CILIARY NEUROTROPHIC FACTOR (CNTF)
   NP_000605.1 
1 MAFTEHSPLT PHRRDLCSRS IWLARKIRSD LTALTESYVK HQGLNKNINL DSADGMPVAS
61 TDQWSELTEA ERLQENLQAY RTFHVLLARL LEDQQVHFTP TEGDFHQAIH TLLLQVAAFA
121 YQIEELMILL EYKIPRNEAD GMPINVGDGG LFEKKLWGLK VLQELSQWTV RSIHDLRFIS
181 SHQTGIPARG SHYIANNKKM
SEQ ID NO:212
   PROSTAGLANDIN-ENDOPEROXIDE SYNTHASE 2 (PTGS2)
   BAA05698.1 
1 MLARALLLCA VLALSHTANP CCSHPCQNRG VCMSVGFDQY KCDCTRTGFY GENCSTPEFL
61 TRIKLFLKPT PNTVHYILTH FKGFWNVVNN IPFLRNAIMS YVLTSRSHLI DSPPTYNADY
121 GYKSWEAFSN LSYYTRALPP VPDDCPTPLG VKGKKQLPDS NEIVEKLLLR RKFIPDPQGS
181 NMMFAFFAQH FTHQFFKTDH KRGPAFTNGL GHGVDLNHIY GETLARQRKL RLFKDGKMKY
241 QIIDGEMYPP TVKDTQAEMI YPPQVPEHLR FAVGQEVFGL VPGLMMYATI WLREHNRVCD
301 VLKQEHPEWG DEQLFQTSRL ILIGETIKIV IEDYVQHLSG YHFKLKFDPE LLFNKQFQYQ
361 NRIAAEFNTL YHWHPLLPDT FQIHDQKYNY QQFIYNNSIL LEHGITQFVE SFTRQIAGRV
421 AGGRNVPPAV QKVSQASIDQ SRQMKYQSFN EYRKRFMLKP YESFEELTGE KEMSAELEAL
481 YGDIDAVELY PALLVEKPRP DAIFGETMVE VGAPFSLKGL MGNVICSPAY WKPSTFGGEV
541 GFQIINTASI QSLICNNVKG CPFTSFSVPD PELIKTVTIN ASSSRSGLDD INPTVLLKER
601 STEL
SEQ ID NO:213
   PROSTAGLANDIN F RECEPTOR (PTGFR)
   AAQ76788.1 
1 MSMNNSKQLV SPAAALLSNT TCQTENRLSV FFSVIFMTVG ILSNSLAIAI LMKAYQRFRQ
61 KSKASFLLLA SGLVITDFFG HLINGAIAVF VYASDKEWIR FDQSNVLCSI FGICMVFSGL
121 CPLLLGSVMA IERCIGVTKP IFHSTKITSK HVKMMLSGVC LFAVFIALLP ILGHRDYKIQ
181 ASRTWCFYNT EDIKDWEDRF YLLLFSFLGL LALGVSLLCN AITGITLLRV KFKSQQHRQG
241 RSHHLEMVIQ LLAIMCVSCI CWSPFLVTMA NIGINGNHSL ETCETTLFAL RMATWNQILD
301 PWVYILLRKA VLKNLYKLAS QCCGVHVISL HIWELSSIKN SLKVAAISES PVAEKSAST
SEQ ID NO:214
   PROSTAGLANDIN F RECEPTOR (PTGFR), ISOFORM CRA_A
   EAX06350.1 
1 MSMNNSKQLV SPAAALLSNT TCQTENRLSV FFSVIFMTVG ILSNSLAIAI LMKAYQRFRQ
61 KSKASFLLLA SGLVITDFFG HLINGAIAVF VYASDKEWIR FDQSNVLCSI FGICMVFSGL
121 CPLLLGSVMA IERCIGVTKP IFHSTKITSK HVKMMLSGVC LFAVFIALLP ILGHRDYKIQ
181 ASRTWCFYNT EDIKDWEDRF YLLLFSFLGL LALGVSLLCN AITGITLLRV KFKSQQHRQG
241 RSHHLEMVIQ LLAIMCVSCI CWSPFLVTMA NIGINGNHSL ETCETTLFAL RMATWNQILD
301 PWVYILLRKA VLKNLYKLAS QCCGVHVISL HIWELSSIKN SLKVAAISES PVAEKSAST
SEQ ID NO:215
   PROSTAGLANDIN F RECEPTOR (PTGFR), ISOFORM CPA_B
   EAX06351.1 
1 MSMNNSKQLV SPAAALLSNT TCQTENRLSV FFSVIFMTVG ILSNSLAIAI LMKAYQRFRQ
61 KSKASFLLLA SGLVITDFFG HLINGAIAVF VYASDKEWIR FDQSNVLCSI FGICMVFSGL
 
121 CPLLLGSVMA IERCIGVTKP IFHSTKITSK HVKMMLSGVC LFAVFIALLP ILGHRDYKIQ
181 ASRTWCFYNT EDIKDWEDRF YLLLFSFLGL LALGVSLLCN AITGITLLRV KFKSQQHRQG
241 RSHHLEMVIQ LLAIMCVSCI CWSPFLGYRI ILNGKEKYKV YEEQSDFLHR LQWPTLE
SEQ ID NO:216
   HYALURONIDASE
   AAC70915.1 
1 
61 
121 
181 
241 
301 
361 
421 
SEQ ID NO:217
   PIGMENT EPITHELIUM-DERIVED FACTOR (PEDF);
   P36955.4 
1 
61 
121 
181 
241 
301 
361 
SEQ ID NO:218
   PIGMENT EPITHELIUM-DERIVED FACTOR (PEDF); ISOFORM 1 PRECURSOR
   NP_001316832.1 
1 
61 
121 
181 
241 
301 
361 
SEQ ID NO:219
   PIGMENT EPITHELIUM-DERIVED FACTOR (PEDF); ISOFORM 2
   NP_001316834.1 
1 MKGKLARSTK EIPDEISILL LGVAHFKGQW VTKFDSRKTS LEDFYLDEER TVRVPMMSDP
61 KAVLRYGLDS DLSCKIAQLP LTGSMSIIFF LPLKVTQNLT LIEESLTSEF IHDIDRELKT
121 VQAVLTVPKL KLSYEGEVTK SLQEMKLQSL FDSPDFSKIT GKPIKLTQVE HRAGFEWNED
181 GAGTTPSPGL QPAHLTFPLD YHLNQPFIFV LRDTDTGALL FIGKILDPRG P
SEQ ID NO:220
   VASCULAR ENDOTHELIAL GROWTH FACTOR (VEGF)
   CAA44447.1 
1 MNFLLSWVHW SLALLLYLHH AKWSQAAPMA EGGGQNHHEV VKFMDVYQRS YCHPIETLVD
61 IFQEYPDEIE YIFKPSCVPL MRCGGCCNDE GLECVPTEES NITMQIMRIK PHQGQHIGEM
121 SFLQHNKCEC RPKKDRARQE NPCGPCSERR KHLFVQDPQT CKCSCKNTDS RCKARQLELN
181 ERTCRCDKPR R
SEQ ID NO:221
   PLACENTAL GROWTH FACTOR (PGF)
   AAH07789.1 
1 MPVMRLFPCF LQLLAGLALP AVPPQQWALS AGNGSSEVEV VPFQEVWGRS YCRALERLVD
61 VVSEYPSEVE HMFSPSCVSL LRCTGCCGDE NLHCVPVETA NVTMQLLKIR SGDRPSYVEL
121 TFSQHVRCEC RPLREKMKPE RRRPKGRGKR RREKQRPTDC HLCGDAVPRR
SEQ ID NO:222
   MYOCILIN (MYOC)
   BAA24532.1 
1 MPAVQLLLLA CLVWDVGART AQLRKANDQS GRCQYTFSVA SPNESSCPEQ SQAMSVIHNL
61 QRDSSTQRLD LEATKARLSS LESLLHQLTL DQAARPQETQ EGLQRELGTL RRERDQLETQ
121 TRELETAYSN LLRDKSVLEE EKKRLRQENE NLARRLESSS QEVARLRRGQ CPQTRDTARA
181 VPPGSREVST WNLDTLAFQE LKSELTEVPA SRILKESPSG YLRSGEGDTG CGELVWVGEP
241 LTLRTAETIT GKYGVWMRDP KPTYPYTQET TWRIDTVGTD VRQVFEYDLI SQFMQGYPSK
301 VHILPRPLES TGAVVYSGSL YFQGAESRTV IRYELNTETV KAEKEIPGAG YHGQFPYSWG
361 GYTDIDLAVD EAGLWVIYST DEAKGAIVLS KLNPENLELE QTWETNIRKQ SVANAFIICG
421 TLYTVSSYTS ADATVNFAYD TGTGISKTLT IPFKNRYKYS SMIDYNPLEK KLFAWDNLNM
481 VTYDIKLSKM
SEQ ID NO:223
   C-C MOTIF CHEMOKINE RECEPTOR 5 (CCR5)
   NP_001093638.1 
1 
61 
121 
181 
241 
301 
SEQ ID NO:224
   CD19
   AAB60697.1 
1 
61 
121 
181 
241 
301 
361 
421 
481 
541 
SEQ ID NO:225
   CRUMBS CELL POLARITY COMPLEX COMPONENT 2 (CRB2), PRECURSOR
   NP_775960.4 
1 
61 
121 
181 
241 
301 
361 
421 
481 
541 
601 
661 
721 
 
781 
841 
901 
961 
1021 
1081 
1141 
1201 
1261 
SEQ ID NO:226
   CRUMBS CELL POLARITY COMPLEX COMPONENT 2 (CRB2), ISOFORM X1
   XP_011516858.1 
1 MALARPGTPD PQALASVLLL LLWAPALSLL AGTVPSEPPS ACASDPCAPG TECQATESGG
61 YTCGPMEPRG CATQPCHHGA LCVPQGPDPT GFRCYCVPGF QGPRCELDID ECASRPCHHG
121 ATCRNLADRY ECHCPLGYAG VTCEMEVDEC ASAPCLHGGS CLDGVGSFRC VCAPGYGGTR
181 CQLDLDECQS QPCAHGGTCH DLVNGFRCDC AGTGYEGTHC EREVLECASA PCEHNASCLE
241 GLGSFRCLCW PGYSGELCEV DEDECASSPC QHGGRCLQRS DPALYGGVQA AFPGAFSFRH
301 AAGFLCHCPP GFEGADCGVE VDECASRPCL NGGHCQDLPN GFQCHCPDGY AGPTCEEDVD
361 ECLSDPCLHG GTCSDTVAGY ICRCPETWGG RDCSVQLTGC QGHTCPLAAT CIPIFESGVH
421 SYVCHCPPGT HGPFCGQNTT FSVMAGSPIQ ASVPAGGPLG LALRFRTTLP AGTLATRNDT
481 KESLELALVA ATLQATLWSY STTVLVLRLP DLALNDGHWH QVEVVLHLAT LELRLWHEGC
541 PARLCVASGP VALASTASAT PLPAGISSAQ LGDATFAGCL QDVRVDGHLL LPEDLGENVL
601 LGCERREQCR PLPCVHGGSC VDLWTHFRCD CARPHRGPTC ADEIPAATFG LGGAPSSASF
661 LLQELPGPNL TVSFLLRTRE SAGLLLQFAN DSAAGLTVFL SEGRIRAEVP GSPAVVLPGR
721 WDDGLRHLVM LSFGPDQLQD LGQHVHVGGR LLAADSQPWG GPFRGCLQDL RLDGCHLPFF
781 PLPLDNSSQP SELGGRQSWN LTAGCVSEDM CSPDPCFNGG TCLVTWNDFH CTCPANFTGP
841 TCAQQLWCPG QPCLPPATCV AEATFREGPP AAFSGHNASS GRLLGGLSLA FRTRDSEAWL
901 LRAAAGALEG VWLAVRNGSL AGGVRGGHGL PGAVLPIPGP RVADGAWHRV RLAMERPAAT
961 TSRWLLWLDG AATPVALRGL ASDLGFLQGP GAVRILLAEN FTGCLGRVAL GGLPLPLARP
1021 RPGAAPGARE HFASWPGTPA PILGCRGAPV CAPSPCLHDG ACRDLFDAFA CACGPGWEGP
1081 RCEAHVDPCH SAPCARGRCH THPDGRFECR CPPGFGGPRC RLPVPSKECS LNVTCLDGSP
1141 CEGGSPAANC SCLEGLAGQR CQVPTLPCEA NPCLNGGTCR AAGGVSECIC NARFSGQFCE
1201 VAKGLPLPLP FPLLEVAVPA ACACLLLLLL GLLSGILAAR KRRQSEGTYS PSQQEVAGAR
1261 LEMDSVLKVP PEERLI
SEQ ID NO:227
   CRUMBS CELL POLARITY COMPLEX COMPONENT 2 (CRB2), ISOFORM X2
   XP_011516860.1 
1 MEPRGCATQP CHHGALCVPQ GPDPTGFRCY CVPGFQGPRC ELDIDECASR PCHHGATCRN
61 LADRYECHCP LGYAGVTCEM EVDECASAPC LHGGSCLDGV GSFRCVCAPG YGGTRCQLDL
121 DECQSQPCAH GGTCHDLVNG FRCDCAGTGY EGTHCEREVL ECASAPCEHN ASCLEGLGSF
181 RCLCWPGYSG ELCEVDEDEC ASSPCQHGGR CLQRSDPALY GGVQAAFPGA FSFRHAAGFL
241 CHCPPGFEGA DCGVEVDECA SRPCLNGGHC QDLPNGFQCH CPDGYAGPTC EEDVDECLSD
301 PCLHGGTCSD TVAGYICRCP ETWGGRDCSV QLTGCQGHTC PLAATCIPIF ESGVHSYVCH
361 CPPGTHGPFC GQNTTFSVMA GSPIQASVPA GGPLGLALRF RTTLPAGTLA TRNDTKESLE
421 LALVAATLQA TLWSYSTTVL VLRLPDLALN DGHWHQVEVV LHLATLELRL WHEGCPARLC
481 VASGPVALAS TASATPLPAG ISSAQLGDAT FAGCLQDVRV DGHLLLPEDL GENVLLGCER
541 REQCRPLPCV HGGSCVDLWT HFRCDCARPH RGPTCADEIP AATFGLGGAP SSASFLLQEL
601 PGPNLTVSFL LRTRESAGLL LQFANDSAAG LTVFLSEGRI RAEVPGSPAV VLPGRWDDGL
661 RHLVMLSFGP DQLQDLGQHV HVGGRLLAAD SQPWGGPFRG CLQDLRLDGC HLPFFPLPLD
721 NSSQPSELGG RQSWNLTAGC VSEDMCSPDP CFNGGTCLVT WNDFHCTCPA NFTGPTCAQQ
781 LWCPGQPCLP PATCEEVPDG FVCVAEATFR EGPPAAFSGH NASSGRLLGG LSLAFRTRDS
841 EAWLLRAAAG ALEGVWLAVR NGSLAGGVRG GHGLPGAVLP IPGPRVADGA WHRVRLAMER
901 PAATTSRWLL WLDGAATPVA LRGLASDLGF LQGPGAVRIL LAENFTGCLG RVALGGLPLP
961 LARPRPGAAP GAREHFASWP GTPAPILGCR GAPVCAPSPC LHDGACRDLF DAFACACGPG
1021 WEGPRCEAHV DPCHSAPCAR GRCHTHPDGR FECRCPPGFG GPRCRLPVPS KECSLNVTCL
 
1081 DGSPCEGGSP AANCSCLEGL AGQRCQVPTL PCEANPCLNG GTCRAAGGVS ECICNARFSG
1141 QFCEVAKGLP LPLPFPLLEV AVPAACACLL LLLLGLLSGI LAARKRRQSE GTYSPSQQEV
1201 AGARLEMDSV LKVPPEERLI
SEQ ID NO:228
   HISTONE DEACETYLASE 4 (HDAC4)
   NP_006028.2 
   1 
  61 
 121 
 181 
 241 
 301 
 361 
 421 
 481 
 541 
 601 
 661 
 721 
 781 
 841 
 901 
 961 
1021 
1081 
SEQ ID NO:229
   HISTONE DEACETYLASE 4 (HDAC4) ISOFORM X2
   XP_006712940.1 
1 MNIDLCAFEI QKTSSPGYEV WFRKQYLAVD GDGLSGRDQP VELLNPARVN HMPSTVDVAT
61 ALPLQVAPSA VPMDLRLDHQ FSLPVAEPAL REQQLQQELL ALKQKQQIQR QILIAEFQRQ
121 HEQLSRQHEA QLHEHIKQQQ EMLAMKHQQE LLEHQRKLER HRQEQELEKQ HREQKLQQLK
181 NKEKGKESAV ASTEVKMKLQ EFVLNKKKAL AHRNLNHCIS SDPRYWYGKT QHSSLDQSSP
241 PQSGVSTSYN HPVLGMYDAK DDFPLRKTAS EPNLKLRSRL KQKVAERRSS PLLRRKDGPV
301 VTALKKRPLD VTDSACSSAP GSGPSSPNNS SGSVSAENGI APAVPSIPAE TSLAHRLVAR
361 EGSAAPLPLY TSPSLPNITL GLPATGPSAG TAGQQDAERL TLPALQQRLS LFPGTHLTPY
421 LSTSPLERDG GAAHSPLLQH MVLLEQPPAQ APLVTGLGAL PLHAQSLVGA DRVSPSIHKL
481 RQHRPLGRTQ SAPLPQNAQA LQHLVIQQQH QQFLEKHKQQ FQQQQLQMNK IIPKPSEPAR
541 QPESHPEETE EELREHQALL DEPYLDRLPG QKEAHAQAGV QVKQEPIESD EEEAEPPREV
601 EPGQRQPSEQ ELLFRQQALL LEQQRIHQLR NYQASMEAAG IPVSFGGHRP LSRAQSSPAS
661 ATFPVSVQEP PTKPRFTTGL VYDTLMLKHQ CTCGSSSSHP EHAGRIQSIW SRLQETGLRG
721 KCECIRGRKA TLEELQTVHS EAHTLLYGTN PLNRQKLDSK KLLGSLASVF VRLPCGGVGV
781 DSDTIWNEVH SAGAARLAVG CVVELVFKVA TGELKNGFAV VRPPGHHAEE STPMGFCYFN
841 SVAVAAKLLQ QRLSVSKILI VDWDVHHGNG TQQAFYSDPS VLYMSLHRYD DGNFFPGSGA
901 PDEVGTGPGV GFNVNMAFTG GLDPPMGDAE YLAAFRTVVM PIASEFAPDV VLVSSGFDAV
961 EGHPTPLGGY NLSARCFGYL TKQLMGLAGG RIVLALEGGH DLTAICDASE ACVSALLGNE
1021 LDPLPEKVLQ QRPNANAVRS MEKVMEIHSK YWRCLQRTTS TAGRSLIEAQ TCENEEAETV
1081 TAMASLSVGV KPAEKRPDEE PMEEEPPL
SEQ ID NO:230
   HISTONE DEACETYLASE 4 (HDAC4) ISOFORM X3
   XP_011510520.1 
1 MNIDLCAFEI QKTSSPGYEV WFRKQYLAVD GDGLSGRDQP VELLNPARVN HMPSTVDVAT
61 ALPLQVAPSA VPMDLRLDHQ FSLPVAEPAL REQQLQQELL ALKQKQQIQR QILIAEFQRQ
121 HEQLSRQHEA QLHEHIKQQQ EMLAMKHQQE LLEHQRKLER HRQEQELEKQ HREQKLQQLK
181 NKEKGKESAV ASTEVKMKLQ EFVLNKKKAL AHRNLNHCIS SDPRYWYGKT QHSSLDQSSP
 
241 PQSGVSTSYN HPVLGMYDAK DDFPLRKTAS EPNLKLRSRL KQKVAERRSS PLLRRKDGPV
301 VTALKKRPLD VTDSACSSAP GSGPSSPNNS SGSVSAENGI APAVPSIPAE TSLAHRLVAR
361 EGSAAPLPLY TSPSLPNITL GLPATGPSAG TAGQQDAERL TLPALQQRLS LFPGTHLTPY
421 LSTSPLERDG GAAHSPLLQH MVLLEQPPAQ APLVTDWYLS GLGALPLHAQ SLVGADRVSP
481 SIHKLRQHRP LGRTQSAPLP QNAQALQHLV IQQQHQQFLE KHKQQFQQQQ LQMNKIIPKP
541 SEPARQPESH PEETEEELRE HQALLDEPYL DRLPGQKEAH AQAGVQVKQE PIESDEEEAE
601 PPREVEPGQR QPSEQELLFR QQALLLEQQR IHQLRNYQAS MEAAGIPVSF GGHRPLSRAQ
661 SSPASATFPV SVQEPPTKPR FTTGLVYDTL MLKHQCTCGS SSSHPEHAGR IQSIWSRLQE
721 TGLRGKCECI RGRKATLEEL QTVHSEAHTL LYGTNPLNRQ KLDSSLASVF VRLPCGGVGV
781 DSDTIWNEVH SAGAARLAVG CVVELVFKVA TGELKNGFAV VRPPGHHAEE STPMGFCYFN
841 SVAVAAKLLQ QRLSVSKILI VDWDVHHGNG TQQAFYSDPS VLYMSLHRYD DGNFFPGSGA
901 PDEVGTGPGV GFNVNMAFTG GLDPPMGDAE YLAAFRTVVM PIASEFAPDV VLVSSGFDAV
961 EGHPTPLGGY NLSARCFGYL TKQLMGLAGG RIVLALEGGH DLTAICDASE ACVSALLGNE
1021 LDPLPEKVLQ QRPNANAVRS MEKVMEIHSK YWRCLQRTTS TAGRSLIEAQ TCENEEAETV
1081 TAMASLSVGV KPAEKRPDEE PMEEEPPL
SEQ ID NO:231
   HISTONE DEACETYLASE 4 (HDAC4) ISOFORM X4
   XP_011510521.1 
1 MNIDLCAFEI QKTSSPGYEV WFRKQYLAVD GDGLSGRDQP VELLNPARVN HMPSTVDVAT
61 ALPLQVAPSA VPMDLRLDHQ FSLPVAEPAL REQQLQQELL ALKQKQQIQR QILIAEFQRQ
121 HEQLSRQHEA QLHEHIKQQQ EMLAMKHQQE LLEHQRKLER HRQEQELEKQ HREQKLQQLK
181 NKEKGKESAV ASTEVKMKLQ EFVLNKKKAL AHRNLNHCIS SDPRYWYGKT QHSSLDQSSP
241 PQSGVSTSYN HPVLGMYDAK DDFPLRKTAS EPNLKLRSRL KQKVAERRSS PLLRRKDGPV
301 VTALKKRPLD VTDSACSSAP GSGPSSPNNS SGSVSAENGI APAVPSIPAE TSLAHRLVAR
361 EGSAAPLPLY TSPSLPNITL GLPATGPSAG TAGQQDAERL TLPALQQRLS LFPGTHLTPY
421 LSTSPLERDG GAAHSPLLQH MVLLEQPPAQ APLVTGLGAL PLHAQSLVGA DRVSPSIHKL
481 RQHRPLGRTQ SAPLPQNAQA LQHLVIQQQH QQFLEKHKQQ FQQQQLQMNK IIPKPSEPAR
541 QPESHPEETE EELREHQALL DEPYLDRLPG QKEAHAQAGV QVKQEPIESD EEEAEPPREV
601 EPGQRQPSEQ ELLFRQQALL LEQQRIHQLR NYQASMEAAG IPVSFGGHRP LSRAQSSPAS
661 ATFPVSVQEP PTKPRFTTGL VYDTLMLKHQ CTCGSSSSHP EHAGRIQSIW SRLQETGLRG
721 KCECIRGRKA TLEELQTVHS EAHTLLYGTN PLNRQKLDSS LASVFVRLPC GGVGVDSDTI
781 WNEVHSAGAA RLAVGCVVEL VFKVATGELK NGFAVVRPPG HHAEESTPMG FCYFNSVAVA
841 AKLLQQRLSV SKILIVDWDV HHGNGTQQAF YSDPSVLYMS LHRYDDGNFF PGSGAPDEVG
901 TGPGVGFNVN MAFTGGLDPP MGDAEYLAAF RTVVMPIASE FAPDVVLVSS GFDAVEGHPT
961 PLGGYNLSAR CFGYLTKQLM GLAGGRIVLA LEGGHDLTAI CDASEACVSA LLGNELDPLP
1021 EKVLQQRPNA NAVRSMEKVM EIHSKYWRCL QRTTSTAGRS LIEAQTCENE EAETVTAMAS
1081 LSVGVKPAEK RPDEEPMEEE PPL
SEQ ID NO:232
   HISTONE DEACETYLASE 4 (HDAC4) ISOFORM X5
   XP_011510522.1 
1 MRKLGPREDG LSGRDQPVEL LNPARVNHMP STVDVATALP LQVAPSAVPM DLRLDHQFSL
61 PVAEPALREQ QLQQELLALK QKQQIQRQIL IAEFQRQHEQ LSRQHEAQLH EHIKQQQEML
121 AMKHQQELLE HQRKLERHRQ EQELEKQHRE QKLQQLKNKE KGKESAVAST EVKMKLQEFV
181 LNKKKALAHR NLNHCISSDP RYWYGKTQHS SLDQSSPPQS GVSTSYNHPV LGMYDAKDDF
241 PLRKTASEPN LKLRSRLKQK VAERRSSPLL RRKDGPVVTA LKKRPLDVTD SACSSAPGSG
301 PSSPNNSSGS VSAENGIAPA VPSIPAETSL AHRLVAREGS AAPLPLYTSP SLPNITLGLP
361 ATGPSAGTAG QQDAERLTLP ALQQRLSLFP GTHLTPYLST SPLERDGGAA HSPLLQHMVL
421 LEQPPAQAPL VTDWYLSGLG ALPLHAQSLV GADRVSPSIH KLRQHRPLGR TQSAPLPQNA
481 QALQHLVIQQ QHQQFLEKHK QQFQQQQLQM NKIIPKPSEP ARQPESHPEE TEEELREHQA
541 LLDEPYLDRL PGQKEAHAQA GVQVKQEPIE SDEEEAEPPR EVEPGQRQPS EQELLFRQQA
601 LLLEQQRIHQ LRNYQASMEA AGIPVSFGGH RPLSRAQSSP ASATFPVSVQ EPPTKPRFTT
661 GLVYDTLMLK HQCTCGSSSS HPEHAGRIQS IWSRLQETGL RGKCECIRGR KATLEELQTV
721 HSEAHTLLYG TNPLNRQKLD SKKLLGSLAS VFVRLPCGGV GVDSDTIWNE VHSAGAARLA
781 VGCVVELVFK VATGELKNGF AVVRPPGHHA EESTPMGFCY FNSVAVAAKL LQQRLSVSKI
841 LIVDWDVHHG NGTQQAFYSD PSVLYMSLHR YDDGNFFPGS GAPDEVGTGP GVGFNVNMAF
 
 901 TGGLDPPMGD AEYLAAFRTV VMPIASEFAP DVVLVSSGFD AVEGHPTPLG GYNLSARCFG
 961 YLTKQLMGLA GGRIVLALEG GHDLTAICDA SEACVSALLG NELDPLPEKV LQQRPNANAV
1021 RSMEKVMEIH SKYWRCLQRT TSTAGRSLIE AQTCENEEAE TVTAMASLSV GVKPAEKRPD
1081 EEPMEEEPPL
SEQ ID NO:233
   HISTONE DEACETYLASE 4 (HDAC4) ISOFORM X6
   XP_011510523.1 
1 MSSQSHPDGL SGRDQPVELL NPARVNHMPS TVDVATALPL QVAPSAVPMD LRLDHQFSLP
61 VAEPALREQQ LQQELLALKQ KQQIQRQILI AEFQRQHEQL SRQHEAQLHE HIKQQQEMLA
121 MKHQQELLEH QRKLERHRQE QELEKQHREQ KLQQLKNKEK GKESAVASTE VKMKLQEFVL
181 NKKKALAHRN LNHCISSDPR YWYGKTQHSS LDQSSPPQSG VSTSYNHPVL GMYDAKDDFP
241 LRKTASEPNL KLRSRLKQKV AERRSSPLLR RKDGPVVTAL KKRPLDVTDS ACSSAPGSGP
301 SSPNNSSGSV SAENGIAPAV PSIPAETSLA HRLVAREGSA APLPLYTSPS LPNITLGLPA
361 TGPSAGTAGQ QDAERLTLPA LQQRLSLFPG THLTPYLSTS PLERDGGAAH SPLLQHMVLL
421 EQPPAQAPLV TDWYLSGLGA LPLHAQSLVG ADRVSPSIHK LRQHRPLGRT QSAPLPQNAQ
481 ALQHLVIQQQ HQQFLEKHKQ QFQQQQLQMN KIIPKPSEPA RQPESHPEET EEELREHQAL
541 LDEPYLDRLP GQKEAHAQAG VQVKQEPIES DEEEAEPPRE VEPGQRQPSE QELLFRQQAL
601 LLEQQRIHQL RNYQASMEAA GIPVSFGGHR PLSRAQSSPA SATFPVSVQE PPTKPRFTTG
661 LVYDTLMLKH QCTCGSSSSH PEHAGRIQSI WSRLQETGLR GKCECIRGRK ATLEELQTVH
721 SEAHTLLYGT NPLNRQKLDS KKLLGSLASV FVRLPCGGVG VDSDTIWNEV HSAGAARLAV
781 GCVVELVFKV ATGELKNGFA VVRPPGHHAE ESTPMGFCYF NSVAVAAKLL QQRLSVSKIL
841 IVDWDVHHGN GTQQAFYSDP SVLYMSLHRY DDGNFFPGSG APDEVGTGPG VGFNVNMAFT
901 GGLDPPMGDA EYLAAFRTVV MPIASEFAPD VVLVSSGFDA VEGHPTPLGG YNLSARCFGY
961 LTKQLMGLAG GRIVLALEGG HDLTAICDAS EACVSALLGN ELDPLPEKVL QQRPNANAVR
1021 SMEKVMEIHS KYWRCLQRTT STAGRSLIEA QTCENEEAET VTAMASLSVG VKPAEKRPDE
1081 EPMEEEPPL
SEQ ID NO:234
   HISTONE DEACETYLASE 4 (HDAC4) ISOFORM X7
   XP_011510526.1 
1 MFDGLSGRDQ PVELLNPARV NHMPSTVDVA TALPLQVAPS AVPMDLRLDH QFSLPVAEPA
61 LREQQLQQEL LALKQKQQIQ RQILIAEFQR QHEQLSRQHE AQLHEHIKQQ QEMLAMKHQQ
121 ELLEHQRKLE RHRQEQELEK QHREQKLQQL KNKEKGKESA VASTEVKMKL QEFVLNKKKA
181 LAHRNLNHCI SSDPRYWYGK TQHSSLDQSS PPQSGVSTSY NHPVLGMYDA KDDFPLRKTA
241 SEPNLKLRSR LKQKVAERRS SPLLRRKDGP VVTALKKRPL DVTDSACSSA PGSGPSSPNN
301 SSGSVSAENG IAPAVPSIPA ETSLAHRLVA REGSAAPLPL YTSPSLPNIT LGLPATGPSA
361 GTAGQQDAER LTLPALQQRL SLFPGTHLTP YLSTSPLERD GGAAHSPLLQ HMVLLEQPPA
421 QAPLVTDWYL SGLGALPLHA QSLVGADRVS PSIHKLRQHR PLGRTQSAPL PQNAQALQHL
481 VIQQQHQQFL EKHKQQFQQQ QLQMNKIIPK PSEPARQPES HPEETEEELR EHQALLDEPY
541 LDRLPGQKEA HAQAGVQVKQ EPIESDEEEA EPPREVEPGQ RQPSEQELLF RQQALLLEQQ
601 RIHQLRNYQA SMEAAGIPVS FGGHRPLSRA QSSPASATFP VSVQEPPTKP RFTTGLVYDT
661 LMLKHQCTCG SSSSHPEHAG RIQSIWSRLQ ETGLRGKCEC IRGRKATLEE LQTVHSEAHT
721 LLYGTNPLNR QKLDSKKLLG SLASVFVRLP CGGVGVDSDT IWNEVHSAGA ARLAVGCVVE
781 LVFKVATGEL KNGFAVVRPP GHHAEESTPM GFCYFNSVAV AAKLLQQRLS VSKILIVDWD
841 VHHGNGTQQA FYSDPSVLYM SLHRYDDGNF FPGSGAPDEV GTGPGVGFNV NMAFTGGLDP
901 PMGDAEYLAA FRTVVMPIAS EFAPDVVLVS SGFDAVEGHP TPLGGYNLSA RCFGYLTKQL
961 MGLAGGRIVL ALEGGHDLTA ICDASEACVS ALLGNELDPL PEKVLQQRPN ANAVRSMEKV
1021 MEIHSKYWRC LQRTTSTAGR SLIEAQTCEN EEAETVTAMA SLSVGVKPAE KRPDEEPMEE
1081 EPPL
SEQ ID NO:235
   HISTONE DEACETYLASE 4 (HDAC4) ISOFORM X8
   XP_011510527.1 
1 MFDGLSGRDQ PVELLNPARV NHMPSTVDVA TALPLQVAPS AVPMDLRLDH QFSLPVAEPA
61 LREQQLQQEL LALKQKQQIQ RQILIAEFQR QHEQLSRQHE AQLHEHIKQQ QEMLAMKHQQ
121 ELLEHQRKLE RHRQEQELEK QHREQKLQQL KNKEKGKESA VASTEVKMKL QEFVLNKKKA
 
181 LAHRNLNHCI SSDPRYWYGK TQHSSLDQSS PPQSGVSTSY NHPVLGMYDA KDDFPLRKTA
241 SEPNLKLRSR LKQKVAERRS SPLLRRKDGP VVTALKKRPL DVTDSACSSA PGSGPSSPNN
301 SSGSVSAENG IAPAVPSIPA ETSLAHRLVA REGSAAPLPL YTSPSLPNIT LGLPATGPSA
361 GTAGQQDAER LTLPALQQRL SLFPGTHLTP YLSTSPLERD GGAAHSPLLQ HMVLLEQPPA
421 QAPLVTGLGA LPLHAQSLVG ADRVSPSIHK LRQHRPLGRT QSAPLPQNAQ ALQHLVIQQQ
481 HQQFLEKHKQ QFQQQQLQMN KIIPKPSEPA RQPESHPEET EEELREHQAL LDEPYLDRLP
541 GQKEAHAQAG VQVKQEPIES DEEEAEPPRE VEPGQRQPSE QELLFRQQAL LLEQQRIHQL
601 RNYQASMEAA GIPVSFGGHR PLSRAQSSPA SATFPVSVQE PPTKPRFTTG LVYDTLMLKH
661 QCTCGSSSSH PEHAGRIQSI WSRLQETGLR GKCECIRGRK ATLEELQTVH SEAHTLLYGT
721 NPLNRQKLDS KKLLGSLASV FVRLPCGGVG VDSDTIWNEV HSAGAARLAV GCVVELVFKV
781 ATGELKNGFA VVRPPGHHAE ESTPMGFCYF NSVAVAAKLL QQRLSVSKIL IVDWDVHHGN
841 GTQQAFYSDP SVLYMSLHRY DDGNFFPGSG APDEVGTGPG VGFNVNMAFT GGLDPPMGDA
901 EYLAAFRTVV MPIASEFAPD VVLVSSGFDA VEGHPTPLGG YNLSARCFGY LTKQLMGLAG
961 GRIVLALEGG HDLTAICDAS EACVSALLGN ELDPLPEKVL QQRPNANAVR SMEKVMEIHS
1021 KYWRCLQRTT STAGRSLIEA QTCENEEAET VTAMASLSVG VKPAEKRPDE EPMEEEPPL
SEQ ID NO:236
   HISTONE DEACETYLASE 4 (HDAC4) ISOFORM X9
   XP_016860883.1 
1 MNIDLCAFEI QKTSSPGYEV WFRKQYLAVD GDGLSGRDQP VELLNPARVN HMPSTVDVAT
61 ALPLQVAPSA VPMDLRLDHQ FSLPVAEPAL REQQLQQELL ALKQKQQIQR QILIAEFQRQ
121 HEQLSRQHEA QLHEHIKQQQ EMLAMKHQQE LLEHQRKLER HRQEQELEKQ HREQKLQQLK
181 NKEKGKESAV ASTEVKMKLQ EFVLNKKKAL AHRNLNHCIS SDPRYWYGKT QHSSLDQSSP
241 PQSGVSTSYN HPVLGMYDAK DDFPLRKTDS ACSSAPGSGP SSPNNSSGSV SAENGIAPAV
301 PSIPAETSLA HRLVAREGSA APLPLYTSPS LPNITLGLPA TGPSAGTAGQ QDAERLTLPA
361 LQQRLSLFPG THLTPYLSTS PLERDGGAAH SPLLQHMVLL EQPPAQAPLV TDWYLSGLGA
421 LPLHAQSLVG ADRVSPSIHK LRQHRPLGRT QSAPLPQNAQ ALQHLVIQQQ HQQFLEKHKQ
481 QFQQQQLQMN KIIPKPSEPA RQPESHPEET EEELREHQAL LDEPYLDRLP GQKEAHAQAG
541 VQVKQEPIES DEEEAEPPRE VEPGQRQPSE QELLFRQQAL LLEQQRIHQL RNYQASMEAA
601 GIPVSFGGHR PLSRAQSSPA SATFPVSVQE PPTKPRFTTG LVYDTLMLKH QCTCGSSSSH
661 PEHAGRIQSI WSRLQETGLR GKCECIRGRK ATLEELQTVH SEAHTLLYGT NPLNRQKLDS
721 KKLLGSLASV FVRLPCGGVG VDSDTIWNEV HSAGAARLAV GCVVELVFKV ATGELKNGFA
781 VVRPPGHHAE ESTPMGFCYF NSVAVAAKLL QQRLSVSKIL IVDWDVHHGN GTQQAFYSDP
841 SVLYMSLHRY DDGNFFPGSG APDEVGTGPG VGFNVNMAFT GGLDPPMGDA EYLAAFRTVV
901 MPIASEFAPD VVLVSSGFDA VEGHPTPLGG YNLSARCFGY LTKQLMGLAG GRIVLALEGG
961 HDLTAICDAS EACVSALLGN ELDPLPEKVL QQRPNANAVR SMEKVMEIHS KYWRCLQRTT
1021 STAGRSLIEA QTCENEEAET VTAMASLSVG VKPAEKRPDE EPMEEEPPL
SEQ ID NO:237
   HISTONE DEACETYLASE 4 (HDAC4) ISOFORM X10
   XP_011510528.1 
1 MSSQSHPVDV ATALPLQVAP SAVPMDLRLD HQFSLPVAEP ALREQQLQQE LLALKQKQQI
61 QRQILIAEFQ RQHEQLSRQH EAQLHEHIKQ QQEMLAMKHQ QELLEHQRKL ERHRQEQELE
121 KQHREQKLQQ LKNKEKGKES AVASTEVKMK LQEFVLNKKK ALAHRNLNHC ISSDPRYWYG
181 KTQHSSLDQS SPPQSGVSTS YNHPVLGMYD AKDDFPLRKT ASEPNLKLRS RLKQKVAERR
241 SSPLLRRKDG PVVTALKKRP LDVTDSACSS APGSGPSSPN NSSGSVSAEN GIAPAVPSIP
301 AETSLAHRLV AREGSAAPLP LYTSPSLPNI TLGLPATGPS AGTAGQQDAE RLTLPALQQR
361 LSLFPGTHLT PYLSTSPLER DGGAAHSPLL QHMVLLEQPP AQAPLVTDWY LSGLGALPLH
421 AQSLVGADRV SPSIHKLRQH RPLGRTQSAP LPQNAQALQH LVIQQQHQQF LEKHKQQFQQ
481 QQLQMNKIIP KPSEPARQPE SHPEETEEEL REHQALLDEP YLDRLPGQKE AHAQAGVQVK
541 QEPIESDEEE AEPPREVEPG QRQPSEQELL FRQQALLLEQ QRIHQLRNYQ ASMEAAGIPV
601 SFGGHRPLSR AQSSPASATF PVSVQEPPTK PRFTTGLVYD TLMLKHQCTC GSSSSHPEHA
661 GRIQSIWSRL QETGLRGKCE CIRGRKATLE ELQTVHSEAH TLLYGTNPLN RQKLDSKKLL
721 GSLASVFVRL PCGGVGVDSD TIWNEVHSAG AARLAVGCVV ELVFKVATGE LKNGFAVVRP
781 PGHHAEESTP MGFCYFNSVA VAAKLLQQRL SVSKILIVDW DVHHGNGTQQ AFYSDPSVLY
841 MSLHRYDDGN FFPGSGAPDE VGTGPGVGFN VNMAFTGGLD PPMGDAEYLA AFRTVVMPIA
901 SEFAPDVVLV SSGFDAVEGH PTPLGGYNLS ARCFGYLTKQ LMGLAGGRIV LALEGGHDLT
 
 961 AICDASEACV SALLGNELDP LPEKVLQQRP NANAVRSMEK VMEIHSKYWR CLQRTTSTAG
1021 RSLIEAQTCE NEEAETVTAM ASLSVGVKPA EKRPDEEPME EEPPL
SEQ ID NO:238
   HISTONE DEACETYLASE 4 (HDAC4) ISOFORM X11
   XP_006712943.1 
1 MPSTVDVATA LPLQVAPSAV PMDLRLDHQF SLPVAEPALR EQQLQQELLA LKQKQQIQRQ
61 ILIAEFQRQH EQLSRQHEAQ LHEHIKQQQE MLAMKHQQEL LEHQRKLERH RQEQELEKQH
121 REQKLQQLKN KEKGKESAVA STEVKMKLQE FVLNKKKALA HRNLNHCISS DPRYWYGKTQ
181 HSSLDQSSPP QSGVSTSYNH PVLGMYDAKD DFPLRKTASE PNLKLRSRLK QKVAERRSSP
241 LLRRKDGPVV TALKKRPLDV TDSACSSAPG SGPSSPNNSS GSVSAENGIA PAVPSIPAET
301 SLAHRLVARE GSAAPLPLYT SPSLPNITLG LPATGPSAGT AGQQDAERLT LPALQQRLSL
361 FPGTHLTPYL STSPLERDGG AAHSPLLQHM VLLEQPPAQA PLVTDWYLSG LGALPLHAQS
421 LVGADRVSPS IHKLRQHRPL GRTQSAPLPQ NAQALQHLVI QQQHQQFLEK HKQQFQQQQL
481 QMNKIIPKPS EPARQPESHP EETEEELREH QALLDEPYLD RLPGQKEAHA QAGVQVKQEP
541 IESDEEEAEP PREVEPGQRQ PSEQELLFRQ QALLLEQQRI HQLRNYQASM EAAGIPVSFG
601 GHRPLSRAQS SPASATFPVS VQEPPTKPRF TTGLVYDTLM LKHQCTCGSS SSHPEHAGRI
661 QSIWSRLQET GLRGKCECIR GRKATLEELQ TVHSEAHTLL YGTNPLNRQK LDSKKLLGSL
721 ASVFVRLPCG GVGVDSDTIW NEVHSAGAAR LAVGCVVELV FKVATGELKN GFAVVRPPGH
781 HAEESTPMGF CYFNSVAVAA KLLQQRLSVS KILIVDWDVH HGNGTQQAFY SDPSVLYMSL
841 HRYDDGNFFP GSGAPDEVGT GPGVGFNVNM AFTGGLDPPM GDAEYLAAFR TVVMPIASEF
901 APDVVLVSSG FDAVEGHPTP LGGYNLSARC FGYLTKQLMG LAGGRIVLAL EGGHDLTAIC
961 DASEACVSAL LGNELDPLPE KVLQQRPNAN AVRSMEKVME IHSKYWRCLQ RTTSTAGRSL
1021 IEAQTCENEE AETVTAMASL SVGVKPAEKR PDEEPMEEEP PL
SEQ ID NO:239
   RHODOPSIN (RHO)
   NP_000530.1 
1 
61 
121 
181 
241 
301 
SEQ ID NO:240
   NERVE GROWTH FACTOR (NGF)
   CAA37703.1 
1 MSILFYVIFL AYLRGIQGNN MDQRSLPEDS LNSLIIKLIQ ADILKNKLSK QMVDVKENYQ
61 STLPKAEAPR EPERGGPAKS AFQPVIAMDT ELLRQQRRYN SPRVLLSDST PLEPPPLYLM
121 EDYVGSPVVA NRTSRRKRYA EHKSHRGEYS VCDSESLWVT DKSSAIDIRG HQVTVLGEIK
181 TGNSPVKQYF YETRCKEARP VKNGCRGIDD KHWNSQCKTS QTYVRALTSE NNKLVGWRWI
241 RIDTSCVCAL SRKIGRT
SEQ ID NO:241
   NUCLEAR FACTOR, ERYTHROID 2 LIKE 2 (NRF2)
   AAB32188.1 
1 MDLIDILWRQ DIDLGVSREV FDFSQRRKEY ELEKQKKLEK ERQEQLQKEQ EKAFFTQLQL
61 DEETGEFLPI QPAQHTQSET SGSANYSQVA HIPKSDALYF DDCMQLLAQT FPFVDDNEVS
121 SATFQSLVPD IPGHIESPVF IATNQAQSPE TSVAQVAPVD LDGMQQDIEQ VWEELLSIPE
181 LQCLNIENDK LVETTMVPSP EAKLTEVDNY HFYSSIPSME KEVGNCSPHF LNAFEDSFSS
241 ILSTEDPNQL TVNSLNSDAT VNTDFGDEFY SAFIAEPSIS NSMPSPATLS HSLSELLNGP
301 IDVSDLSLCK AFNQNHPEST AEFNDSDSGI SLNTSPSVAS PEHSVESSSY GDTLLGLSDS
361 EVEELDSAPG SVKQNGPKTP VHSSGDMVQP LSPSQGQSTH VHDAQCENTP EKELPVSPGH
421 RKTPFTKDKH SSRLEAHLTR DELRAKALHI PFPVEKIINL PVVDFNEMMS KEQFNEAQLA
481 LIRDIRRRGK NKVAAQNCRK RKLENIVELE QDLDHLKDEK EKLLKEKGEN DKSLHLLKKQ
541 LSTLYLEVFS MLRDEDGKPY SPSEYSLQQT RDGNVFLVPK SKKPDVKKN
SEQ ID NO:242
   GLUTATHIONE S-TRANSFERASE PI 1 (GSTP1)
   AAH10915.1 
  1 MPPYTVVYFP VRGRCAALRM LLADQGQSWK EEVVTVETWQ EGSLKASCLY GQLPKFQDGD
 61 LTLYQSNTIL RHLGRTLGLY GKDQQEAALV DMVNDGVEDL RCKYVSLIYT NYEAGKDDYV
121 KALPGQLKPF ETLLSQNQGG KTFIVGDQIS FADYNLLDLL LIHEVLAPGC LDAFPLLSAY
181 VGRLSARPKL KAFLASPEYV NLPINGNGKQ
SEQ ID NO:243
   ROD-DERIVED CONE VIABILITY FACTOR (RDCVF)
   NP_612463.1 
1 MASLFSGRIL IRNNSDQDEL DTEAEVSRRL ENRLVLLFFG AGACPQCQAF VPILKDFFVR
61 LTDEFYVLRA AQLALVYVSQ DSTEEQQDLF LKDMPKKWLF LPFEDDLRRD LGRQFSVERL
121 PAVVVLKPDG DVLTRDGADE IQRLGTACFA NWQEAAEVLD RNFQLPEDLE DQEPRSLTEC
181 LRRHKYRVEK AARGGRDPGG GGGEEGGAGG LF
SEQ ID NO:244
   RETINALDEHYDE BINDING PROTEIN 1 (RLBP1)
   EAX02038.1 
1 
61 
121 
181 
241 
301 
SEQ ID NO:245
   DOUBLE HOMEOBOX 4 (DUX4)
   AUA60624.1 
1 
61 
121 
181 
241 
301 
361 
421 
SEQ ID NO:246
   NLR FAMILY PYRIN DOMAIN CONTAINING 3 (NLRP3)
   AAI43360.1 
1 MKMASTRCKL ARYLEDLEDV DLKKFKMHLE DYPPQKGCIP LPRGQTEKAD HVDLATLMID
61 FNGEEKAWAM AVWIFAAINR RDLYEKAKRD EPKWGSDNAR VSNPTVICQE DSIEEEWMGL
121 LEYLSRISIC KMKKDYRKKY RKYVRSRFQC IEDRNARLGE SVSLNKRYTR LRLIKEHRSQ
181 QEREQELLAI GKTKTCESPV SPIKMELLFD PDDEHSEPVH TVVFQGAAGI GKTILARKMM
241 LDWASGTLYQ DRFDYLFYIH CREVSLVTQR SLGDLIMSCC PDPNPPIHKI VRKPSRILFL
301 MDGFDELQGA FDEHIGPLCT DWQKAERGDI LLSSLIRKKL LPEASLLITT RPVALEKLQH
361 LLDHPRHVEI LGFSEAKRKE YFFKYFSDEA QARAAFSLIQ ENEVLFTMCF IPLVCWIVCT
421 GLKQQMESGK SLAQTSKTTT AVYVFFLSSL LQPRGGSQEH GLCAHLWGLC SLAADGIWNQ
481 KILFEESDLR NHGLQKADVS AFLRMNLFQK EVDCEKFYSF IHMTFQEFFA AMYYLLEEEK
541 EGRTNVPGSR LKLPSRDVTV LLENYGKFEK GYLIFVVRFL FGLVNQERTS YLEKKLSCKI
601 SQQIRLELLK WIEVKAKAKK LQIQPSQLEL FYCLYEMQEE DFVQRAMDYF PKIEINLSTR
661 MDHMVSSFCI ENCHRVESLS LGFLHNMPKE EEEEEKEGRH LDMVQCVLPS SSHAACSHGL
721 VNSHLTSSFC RGLFSVLSTS QSLTELDLSD NSLGDPGMRV LCETLQHPGC NIRRLCNQKL
781 VELDLSDNAL GDFGIRLLCV GLKHLLCNLK KLWLVSCCLT SACCQDLASV LSTSHSLTRL
841 YVGENALGDS GVAILCEKAK NPQCNLQKLG LVNSGLTSVC CSALSSVLST NQNLTHLYLR
 
901 GNTLGDKGIK LLCEGLLHPD CKLQVLELDN CNLTSHCCWD LSTLLTSSQS LRKLSLGNND
961 LGDLGVMMFC EVLKQQSCLL QNLGLSEMYF NYETKSALET LQEEKPELTV VFEPSW
SEQ ID NO:247
   SPLEEN ASSOCIATED TYROSINE KINASE (SYK), ISOFORM SYK(S)
   NP_001167639.1 
1 MASSGMADSA NHLPFFFGNI TREEAEDYLV QGGMSDGLYL LRQSRNYLGG FALSVAHGRK
61 AHHYTIEREL NGTYAIAGGR THASPADLCH YHSQESDGLV CLLKKPFNRP QGVQPKTGPF
121 EDLKENLIRE YVKQTWNLQG QALEQAIISQ KPQLEKLIAT TAHEKMPWFH GKISREESEQ
181 IVLIGSKTNG KFLIRARDNN GSYALCLLHE GKVLHYRIDK DKTGKLSIPE GKKFDTLWQL
241 VEHYSYKADG LLRVLTVPCQ KIGTQGNVNF GGRPQLPGSH PASSPAQGNR QESTVSFNPY
301 EPELAPWAAD KGPQREALPM DTEVYESPYA DPEEIRPKEV YLDRKLLTLE DKELGSGNFG
361 TVKKGYYQMK KVVKTVAVKI LKNEANDPAL KDELLAEANV MQQLDNPYIV RMIGICEAES
421 WMLVMEMAEL GPLNKYLQQN RHVKDKNIIE LVHQVSMGMK YLEESNFVHR DLAARNVLLV
481 TQHYAKISDF GLSKALRADE NYYKAQTHGK WPVKWYAPEC INYYKFSSKS DVWSFGVLMW
541 EAFSYGQKPY RGMKGSEVTA MLEKGERMGC PAGCPREMYD LMNLCWTYDV ENRPGFAAVE
601 LRLRNYYYDV VN
SEQ ID NO:248
   SPLEEN ASSOCIATED TYROSINE KINASE (SYK), ISOFORM SYK(L)
   NP_003168.2 
1 
61 
121 
181 
241 
301 
361 
421 
481 
541 
601 
SEQ ID NO:249
   ADRENOCORTICOTROPIC HORMONE (ACTH), PREPROPROTEIN
   NP_000930.1 
1 MPRSCCSRSG ALLLALLLQA SMEVRGWCLE SSQCQDLTTE SNLLECIRAC KPDLSAETPM
61 FPGNGDEQPL TENPRKYVMG HFRWDRFGRR NSSSSGSSGA GQKREDVSAG EDCGPLPEGG
121 PEPRSDGAKP GPREGKRSYS MEHFRWGKPV GKKRRPVKVY PNGAEDESAE AFPLEFKREL
181 TGQRLREGDG PDGPADDGAG AQADLEHSLL VAAEKKDEGP YRMEHFRWGS PPKDKRYGGF
241 MTSEKSQTPL VTLFKNAIIK NAYKKGE
SEQ ID NO:250
   CASPASE 1 (CASP1), ISOFORM ALPHA PRECURSOR
   NP_001244047.1 
1 MADKVLKEKR KLFIRSMGEG TINGLLDELL QTRVLNKEEM EKVKRENATV MDKTRALIDS
61 VIPKGAQACQ ICITYICEED SYLAGTLGLS ADQTSGNYLN MQDSQGVLSS FPAPQAVQDN
121 PAMPTSSGSE GNVKLCSLEE AQRIWKQKSA EIYPIMDKSS RTRLALIICN EEFDSIPRRT
181 GAEVDITGMT MLLQNLGYSV DVKKNLTASD MTTELEAFAH RPEHKTSDST FLVFMSHGIR
241 EGICGKKHSE QVPDILQLNA IFNMLNTKNC PSLKDKPKVI IIQACRGDSP GVVWFKDSVG
301 VSGNLSLPTT EEFEDDAIKK AHIEKDFIAF CSSTPDNVSW RHPTMGSVFI GRLIEHMQEY
361 ACSCDVEEIF RKVRFSFEQP DGRAQMPTTE RVTLTRCFYL FPGH
SEQ ID NO:251
   CASPASE 1 (CASP1), ISOFORM BETA PRECURSOR
   NP_001244048.1 1 MADKVLKEKR KLFIRSMGEG TINGLLDELL QTRVLNKEEM EKVKRENATV MDKTRALIDS 
61 VIPKGAQACQ ICITYICEED SYLAGTLGLS AAPQAVQDNP AMPTSSGSEG NVKLCSLEEA
121 QRIWKQKSAE IYPIMDKSSR TRLALIICNE EFDSIPRRTG AEVDITGMTM LLQNLGYSVD
181 VKKNLTASDM TTELEAFAHR PEHKTSDSTF LVFMSHGIRE GICGKKHSEQ VPDILQLNAI
241 FNMLNTKNCP SLKDKPKVII IQACRGDSPG VVWFKDSVGV SGNLSLPTTE EFEDDAIKKA
301 HIEKDFIAFC SSTPDNVSWR HPTMGSVFIG RLIEHMQEYA CSCDVEEIFR KVRFSFEQPD
361 GRAQMPTTER VTLTRCFYLF PGH
SEQ ID NO:252
   CD59
   CAG46523.1 
  1 MGIQGGSVLF GLLLVLAVFC HSGHSLQCYN CPNPTADCKT AVNCSSDFDA CLITKAGLQV
 61 YNKCWKFEHC NFNDVTTRLR ENELTYYCCK KDLCNFNEQL ENGGTSLSEK TVLLLVTPFL
121 AAAWSLHP
SEQ ID NO:253
   NOTCH REGULATED ANKYRIN REPEAT PROTEIN (NRARP)
   NP_001004354.1 
1 MSQAELSTCS APQTQRIFQE AVRKGNTQEL QSLLQNMTNC EFNVNSFGPE GQTALHQSVI
61 DGNLELVKLL VKFGADIRLA NRDGWSALHI AAFGGHQDIV LYLITKAKYA ASGR
SEQ ID NO:254
   ALPHA-2-ANTIPLASMIN (A2AP), ISOFORM A PRECURSOR
   NP_000925.2 
1 MALLWGLLVL SWSCLQGPCS VFSPVSAMEP LGRQLTSGPN QEQVSPLTLL KLGNQEPGGQ
61 TALKSPPGVC SRDPTPEQTH RLARAMMAFT ADLFSLVAQT STCPNLILSP LSVALALSHL
121 ALGAQNHTLQ RLQQVLHAGS GPCLPHLLSR LCQDLGPGAF RLAARMYLQK GFPIKEDFLE
181 QSEQLFGAKP VSLTGKQEDD LANINQWVKE ATEGKIQEFL SGLPEDTVLL LLNAIHFQGF
241 WRNKFDPSLT QRDSFHLDEQ FTVPVEMMQA RTYPLRWFLL EQPEIQVAHF PFKNNMSFVV
301 LVPTHFEWNV SQVLANLSWD TLHPPLVWER PTKVRLPKLY LKHQMDLVAT LSQLGLQELF
361 QAPDLRGISE QSLVVSGVQH QSTLELSEVG VEAAAATSIA MSRMSLSSFS VNRPFLFFIF
421 EDTTGLPLFV GSVRNPNPSA PRELKEQQDS PGNKDFLQSL KGFPRGDKLF GPDLKLVPPM
481 EEDYPQFGSP K
SEQ ID NO:255
   ALPHA-2-ANTIPLASMIN (A2AP), ISOFORM B PRECURSOR
   NP_001159393.1 
1 MALLWGLLVL SWSCLQGPCS VFSPVSAMEP LGRQLTSGPN QEQVSPLTLL KLGNQVQPGA
61 QNHTLQRLQQ VLHAGSGPCL PHLLSRLCQD LGPGAFRLAA RMYLQKGFPI KEDFLEQSEQ
121 LFGAKPVSLT GKQEDDLANI NQWVKEATEG KIQEFLSGLP EDTVLLLLNA IHFQGFWRNK
181 FDPSLTQRDS FHLDEQFTVP VEMMQARTYP LRWFLLEQPE IQVAHFPFKN NMSFVVLVPT
241 HFEWNVSQVL ANLSWDTLHP PLVWERPTKV RLPKLYLKHQ MDLVATLSQL GLQELFQAPD
301 LRGISEQSLV VSGVQHQSTL ELSEVGVEAA AATSIAMSRM SLSSFSVNRP FLFFIFEDTT
361 GLPLFVGSVR NPNPSAPREL KEQQDSPGNK DFLQSLKGFP RGDKLFGPDL KLVPPMEEDY
421 PQFGSPK
SEQ ID NO:256
   PLASMINOGEN (PLG)
   AAA60113.1 
1 
61 
121 
181 
241 
301 
361 
421 
481 
 
541 
601 
661 
721 
781 
SEQ ID NO:257
   GROWTH HORMONE
   AAA98618.1 
  1 
 61 
121 
181 
SEQ ID NO:258
   INSULIN LIKE GROWTH FACTOR 1 (IGF1)
   CAG46659.1 
1 MGKISSLPTQ LFKCCFCDFL KVKMHTMSSS HLFYLALCLL TFTSSATAGP ETLCGAELVD
61 ALQFVCGDRG FYFNKPTGYG SSSRRAPQTG IVDECCFRSC DLRRLEMYCA PLKPAKSARS
121 VRAQRHTDMP KTQKEVHLKN ASRGSAGNKN YRM
SEQ ID NO:259
   INTERLEUKIN 1 BETA (IL1B)
   AAA74137.1 
1 MAEVPELASE MMAYYSGNED DLFFEADGPK QMKCSFQDLD LCPLDGGIQL RISDHHYSKG
61 FRQAASVVVA MDKLRKMLVP CPQTFQENDL STFFPFIFEE EPIFFDTWDN EAYVHDAPVR
121 SLNCTLRDSQ QKSLVMSGPY ELKALHLQGQ DMEQQVVFSM SFVQGEESND KIPVALGLKE
181 KNLYLSCVLK DDKPTLQLES VDPKNYPKKK MEKRFVFNKI EINNKLEFES AQFPNWYIST
241 SQAENMPVFL GGTKGGQDIT DFTMQFVSS
SEQ ID NO:260
   ANGIOTENSIN I CONVERTING ENZYME 2 (ACE2)
   ACT66268.1 
1 
61 
121 
181 
241 
301 
361 
421 
481 
541 
601 
661 
721 
781 
SEQ ID NO:261
   INTEGRIN ALPHA SUBUNIT PRECURSOR
   AAA51620.1 
  1 
 61 
121 
181 
241 
301 
 
361 
421 
481 
541 
601 
661 
721 
781 
841 
901 
961 
1021 
1081 
1141 
SEQ ID NO:262
   INTEGRIN ALPHA SUBUNIT PRECURSOR
   P05556.2 
1 MNLQPIFWIG LISSVCCVFA QTDENRCLKA NAKSCGECIQ AGPNCGWCTN STFLQEGMPT
61 SARCDDLEAL KKKGCPPDDI ENPRGSKDIK KNKNVTNRSK GTAEKLKPED ITQIQPQQLV
121 LRLRSGEPQT FTLKFKRAED YPIDLYYLMD LSYSMKDDLE NVKSLGTDLM NEMRRITSDF
181 RIGFGSFVEK TVMPYISTTP AKLRNPCTSE QNCTSPFSYK NVLSLTNKGE VFNELVGKQR
241 ISGNLDSPEG GFDAIMQVAV CGSLIGWRNV TRLLVFSTDA GFHFAGDGKL GGIVLPNDGQ
301 CHLENNMYTM SHYYDYPSIA HLVQKLSENN IQTIFAVTEE FQPVYKELKN LIPKSAVGTL
361 SANSSNVIQL IIDAYNSLSS EVILENGKLS EGVTISYKSY CKNGVNGTGE NGRKCSNISI
421 GDEVQFEISI TSNKCPKKDS DSFKIRPLGF TEEVEVILQY ICECECQSEG IPESPKCHEG
481 NGTFECGACR CNEGRVGRHC ECSTDEVNSE DMDAYCRKEN SSEICSNNGE CVCGQCVCRK
541 RDNTNEIYSG KFCECDNFNC DRSNGLICGG NGVCKCRVCE CNPNYTGSAC DCSLDTSTCE
601 ASNGQICNGR GICECGVCKC TDPKFQGQTC EMCQTCLGVC AEHKECVQCR AFNKGEKKDT
661 CTQECSYFNI TKVESRDKLP QPVQPDPVSH CKEKDVDDCW FYFTYSVNGN NEVMVHVVEN
721 PECPTGPDII PIVAGVVAGI VLIGLALLLI WKLLMIIHDR REFAKFEKEK MNAKWDTGEN
781 PIYKSAVTTV VNPKYEGK
SEQ ID NO:263
   CD40
   AAH64518.1 
  1 
 61 
121 
SEQ ID NO:264
   INSULIN-LIKE GROWTH FACTOR 1 RECEPTOR (IGF1R)
   AAI43722.1 
1 MKSGSGGGSP TSLWGLLFLS AALSLWPTSG EICGPGIDIR NDYQQLKRLE NCTVIEGYLH
61 ILLISKAEDY RSYRFPKLTV ITEYLLLFRV AGLESLGDLF PNLTVIRGWK LFYNYALVIF
121 EMTNLKDIGL YNLRNITRGA IRIEKNADLC YLSTVDWSLI LDAVSNNYIV GNKPPKECGD
181 LCPGTMEEKP MCEKTTINNE YNYRCWTTNR CQKMCPSTCG KRACTENNEC CHPECLGSCS
241 APDNDTACVA CRHYYYAGVC VPACPPNTYR FEGWRCVDRD FCANILSAES SDSEGFVIHD
301 GECMQECPSG FIRNGSQSMY CIPCEGPCPK VCEEEKKTKT IDSVTSAQML QGCTIFKGNL
361 LINIRRGNNI ASELENFMGL IEVVTGYVKI RHSHALVSLS FLKNLRLILG EEQLEGNYSF
421 YVLDNQNLQQ LWDWDHRNLT IKAGKMYFAF NPKLCVSEIY RMEEVTGTKG RQSKGDINTR
481 NNGERASCES DVLHFTSTTT SKNRIIITWH RYRPPDYRDL ISFTVYYKEA PFKNVTEYDG
541 QDACGSNSWN MVDVDLPPNK DVEPGILLHG LKPWTQYAVY VKAVTLTMVE NDHIRGAKSE
601 ILYIRTNASV PSIPLDVLSA SNSSSQLIVK WNPPSLPNGN LSYYIVRWQR QPQDGYLYRH
661 NYCSKDKIPI RKYADGTIDI EEVTENPKTE VCGGEKGPCC ACPKTEAEKQ AEKEEAEYRK
721 VFENFLHNSI FVPRPERKRR DVMQVANTTM SSRSRNTTAA DTYNITDPEE LETEYPFFES
781 RVDNKERTVI SNLRPFTLYR IDIHSCNHEA EKLGCSASNF VFARTMPAEG ADDIPGPVTW
 
841 EPRPENSIFL KWPEPENPNG LILMYEIKYG SQVEDQRECV SRQEYRKYGG AKLNRLNPGN
901 YTARIQATSL SGNGSWTDPV FFYVQAKRYE NFIHLIIALP VAVLLIVGGL VIMLYVFHRK
961 RNNSRLGNGV LYASVNPEYF SAADVYVPDE WEVAREKITM SRELGQGSFG MVYEGVAKGV
1021 VKDEPETRVA IKTVNEAASM RERIEFLNEA SVMKEFNCHH VVRLLGVVSQ GQPTLVIMEL
1081 MTRGDLKSYL RSLRPEMENN PVLAPPSLSK MIQMAGEIAD GMAYLNANKF VHRDLAARNC
1141 MVAEDFTVKI GDFGMTRDIY ETDYYRKGGK GLLPVRWMSP ESLKDGVFTT YSDVWSFGVV
1201 LWEIATLAEQ PYQGLSNEQV LRFVMEGGLL DKPDNCPDML FELMRMCWQY NPKMRPSFLE
1261 IISSIKEEME PGFREVSFYY SEENKLPEPE ELDLEPENME SVPLDPSASS SSLPLPDRHS
1321 GHKAENGPGP GVLVLRASFD ERQPYAHMNG GRKNERALPL PQSSTC
SEQ ID NO:265
   INSULIN-LIKE GROWTH FACTOR 2 RECEPTOR (IGF2R)
   AAK56918.1 
1 MGAAAGRSPH LGPAPARRPQ RSLLLLQLLL LVAAPGSTQA QAAPFPELCS YTWEAVDTKN
61 NVLYKINICG SVDIVQCGPS SAVCMHDLKT RTYHSVGDSV LRSATRSLLE FNTTVSCDQQ
121 GTNHRVQSSI AFLCGKTLGT PEFVTATECV HYFEWRTTAA CKKDIFKANK EVPCYVFDEE
181 LRKHDLNPLI KLSGAYLVDD SDPDTSLFIN VCRDIDTLRD PGSQLRACPP GTAACLVRGH
241 QAFDVGQPRD GLKLVRKDRL VLSYVREEAG KLDFCDGHSP AVTITFVCPS ERREGTIPKL
301 TAKSNCRYEI EWITEYACHR DYLESKTCSL SGEQQDVSID LTPLAQSGGS SYISDGKEYL
361 FYLNVCGETE IQFCNKKQAA VCQVKKSDTS QVKAAGRYHN QTLRYSDGDL TLIYFGGDEC
421 SSGFQRMSVI NFECNKTAGN DGKGTPVFTG EVDCTYFFTW DTEYACVKEK EDLLCGATDG
481 KKRYDLSALV RHAEPEQNWE AVDGSQTETE KKHFFINICH RVLQEGKARG CPEDAAVCAV
541 DKNGSKNLGK FISSPMKEKG NIQLSYSDGD DCGHGKKIKT NITLVCKPGD LESAPVLRTS
601 GEGGCFYEFE WHTAAACVLS KTEGENCTVF DSQAGFSFDL SPLTKKNGAY KVETKKYDFY
661 INVCGPVSVS PCQPDSGACQ VAKSDEKTWN LGLSNAKLSY YDGMIQLNYR GGTPYNNERH
721 TPRATLITFL CDRDAGVGFP EYQEEDNSTY NFRWYTSYAC PEEPLECVVT DPSTLEQYDL
781 SSLAKSEGGL GGNWYAMDNS GEHVTWRKYY INVCRPLNPV PGCNRYASAC QMKYEKDQGS
841 FTEVVSISNL GMAKTGPVVE DSGSLLLEYV NGSACTTSDG RQTTYTTRIH LVCSRGRLNS
901 HPIFSLNWEC VVSFLWNTEA ACPIQTTTDT DQACSIRDPN SGFVFNLNPL NSSQGYNVSG
961 IGKIFMFNVC GTMPVCGTIL GKPASGCEAE TQTEELKNWK PARPVGIEKS LQLSTEGFIT
1021 LTYKGPLSAK GTADAFIVRF VCNDDVYSGP LKFLHQDIDS GQGIRNTYFE FETALACVPS
1081 PVDCQVTDLA GNEYDLTGLS TVRKPWTAVD TSVDGRKRTF YLSVCNPLPY IPGCQGSAVG
1141 SCLVSEGNSW NLGVVQMSPQ AAANGSLSIM YVNGDKCGNQ RFSTRITFEC AQISGSPAFQ
1201 LQDGCEYVFI WRTVEACPVV RVEGDNCEVK DPRHGNLYDL KPLGLNDTIV SAGEYTYYFR
1261 VCGKLSSDVC PTSDKSKVVS SCQEKREPQG FHKVAGLLTQ KLTYENGLLK MNFTGGDTCH
1321 KVYQRSTAIF FYCDRGTQRP VFLKETSDCS YLFEWRTQYA CPPFDLTECS FKDGAGNSFD
1381 LSSLSRYSDN WEAITGTGDP EHYLINVCKS LAPQAGTEPC PPEAAACLLG GSKPVNLGRV
1441 RDGPQWRDGI IVLKYVDGDL CPDGIRKKST TIRFTCSESQ VNSRPMFISA VEDCEYTFAW
1501 PTATACPMKS NEHDDCQVTN PSTGHLFDLS SLSGRAGFTA AYSEKGLVYM SICGENENCP
1561 PGVGACFGQT RISVGKANKR LRYVDQVLQL VYKDGSPCPS KSGLSYKSVI SFVCRPEARP
1621 TNRPMLISLD KQTCTLFFSW HTPLACEQAT ECSVRNGSSI VDLSPLIHRT GGYEAYDESE
1681 DDASDTNPDF YINICQPLNP MHGVPCPAGA AVCKVPIDGP PIDIGRVAGP PILNPIANEI
1741 YLNFESSTPC LADKHFNYTS LIAFHCKRGV SMGTPKLLRT SECDFVFEWE TPVVCPDEVR
1801 MDGCTLTDEQ LLYSFNLSSL STSTFKVTRD SRTYSVGVCT FAVGPEQGGC KDGGVCLLSG
1861 TKGASFGRLQ SMKLDYRHQD EAVVLSYVNG DRCPPETDDG VPCVFPFIFN GKSYEECIIE
1921 SRAKLWCSTT ADYDRDHEWG FCRHSNSYRT SSIIFKCDED EDIGRPQVFS EVRGCDVTFE
1981 WKTKVVCPPK KLECKFVQKH KTYDLRLLSS LTGSWSLVHN GVSYYINLCQ KIYKGPLGCS
2041 ERASICRRTT TGDVQVLGLV HTQKLGVIGD KVVVTYSKGY PCGGNKTASS VIELTCTKTV
2101 GRPAFKRFDI DSCTYYFSWD SRAACAVKPQ EVQMVNGTIT NPINGKSFSL GDIYFKLFRA
2161 SGDMRTNGDN YLYEIQLSSI TSSRNPACSG ANICQVKPND QHFSRKVGTS DKTKYYLQDG
2221 DLDVVFASSS KCGKDKTKSV SSTIFFHCDP LVEDGIPEFS HETADCQYLF SWYTSAVCPL
2281 GVGFDSENPG DDGQMHKGLS ERSQAVGAVL SLLLVALTCC LLALLLYKKE RRETVISKLT
2341 TCCRRSSNVS YKYSKVNKEE ETDENETEWL MEEIQLPPPR QGKEGQENGH ITTKSVKALS
2401 SLHGDDQDSE DEVLTIPEVK VHSGRGAGAE SSHPVRNAQS NALQEREDDR VGLVRGEKAR
2461 KGKSSSAQQK TVSSTKLVSF HDDSDEDLLH I
SEQ ID NO:266
   RTP801
   AAL38424.1 
  1 MPSLWDRFSS SSTSSSPSSL PRTPTPDRPP RSAWGSATRE EGFDRSTSLE SSDCESLDSS
 61 NSGFGPEEDT AYLDGVSLPD FELLSDPEDE HLCANLMQLL QESLAQARLG SRRPARLLMP
121 SQLVSQVGKE LLRLAYSEPC GLRGALLDVC VEQGKSCHSV GQLALDPSLV PTFQLTLVLR
181 LDSRLWPKIQ GLFSSANSPF LPGFSQSLTL STGFRVIKKK LYSSEQLLIE EC
SEQ ID NO:267
   METALLOPROTEINASE 2 (MMP2)
   BAA12023 
1 MILLTFSTGR RLDFVHHSGV FFLQTLLWIL CATVCGTEQY FNVEVWLQKY GYLPPTDPRM
61 SVLRSAETMQ SALAAMQQFY GINMTGKVDR NTIDWMKKPR CGVPDQTRGS SKFHIRRKRY
121 ALTGQKWQHK HITYSIKNVT PKVGDPETRK AIRRAFDVWQ NVTPLTFEEV PYSELENGKR
181 DVDITIIFAS GFHGDSSPFD GEGGFLAHAY FPGPGIGGDT HFDSDEPWTL GNPNHDGNDL
241 FLVAVHELGH ALGLEHSNDP TAIMAPFYQY METDNFKLPN DDLQGIQKIY GPPDKIPPPT
301 RPLPTVPPHR SIPPADPRKN DRPKPPRPPT GRPSYPGAKP NICDGNFNTL AILRREMFVF
361 KDQWFWRVRN NRVMDGYPMQ ITYFWRGLPP SIDAVYENSD GNFVFFKVKG DTLSVIQDGW
421 LYKYHWKWIL EQRQSVPVLS RQTEKHKTYE ELSSITY
SEQ ID NO:268
   G-PROTEIN COUPLED RECEPTOR 143 (GPR143)
   NP_000264 
1 
61 
121 
181 
241 
301 
361 
SEQ ID NO:269
   G-PROTEIN COUPLED RECEPTOR 143 (GPR143)
   EAW98773.1 
1 MTQAGRRGPG TPEPRPRTQP MASPRLGTFC CPTRDAATQL VLSFQPRAFH ALCLGSGGLR
61 LALGLLQLLP GRRPAGPGSP ATSPPASVRI LRAAAACDLL GCLGMVIRST VWLGFPNFVD
121 SVSDMNHTEI WPAAFCVGSA MWIQLLYSAC FWWLFCYAVD AYLVIRRSAG LSTILLYHIM
181 AWGLATLLCV EGAAMLYYPS VSRCERGLDH AIPHYVTMYL PLLLVLVANP ILFQKTVTAV
241 ASLLKGRQGI YTENERRMGA VIKIRFFKIM LVLIICWLSN IINESLLFYL EMQTDINGGS
301 LKPVRTAAKT TWFIMGILNP AQGFLLSLAF YGWTGCSLGF QSPRKEIQWE SLTTSAAEGA
361 HPSPLMPHEN PASGKVSQVG GQTSDEALSM LSEGSDASTI EIHTASESCN KNEGDPALPT
421 HGDL
SEQ ID NO:270
   TYROSINASE (TYR)
   AAB60319.1 
1 
61 
121 
181 
241 
301 
361 
421 
481 
SEQ ID NO:271
   CASPASE 2 (CASP2)
   CAG46548.1 
1 MHPHHQETLK KNRVVLAKQL LLSELLEHLL EKDIITLEMR ELIQAKVGSF SQNVELLNLL
61 PKRGPQAFDA FCEALRETKQ GHLEDMLLTT LSGLQHVLPP LSCDYDLSLP FPVCESCPLY
121 KKLRLSTDTV EHSLDNKDGP VCLQVKPCTP EFYQTHFQLA YRLQSRPRGL ALVLSNVHFT
181 GEKELEFRSG GDVDHSTLVT LFKLLGYDVH VLCDQTAQEM QEKLQNFAQL PAHRVTDSCI
241 VALLSHGVEG AIYGVDGKLL QLQEVFQLFD NANCPSLQNK PKMFFIQACR GGAIGSLGHL
301 LLFTAATASL AL
SEQ ID NO:272
   LEUCINE RICH REPEAT AND IG DOMAIN CONTAINING PROTEIN 1 (LINGO1)
   AAH68558.1 
1 MLAGGVRSMP SPLLACWQPI LLLVLGSVLS GSATGCPPRY ECSAQDRAVL CHRKRFVAVP
61 EGIPTETRLL DLGKNRIKTL NQDEFASFPH LEELELNENI VSAVEPGAFN NLFNLRTLGL
121 RSNRLKLIPL GVFTGLSNLT KLDISENKIV ILLDYMFQDL YNLRSLEVGD NDLVYISHRA
181 FSGLNSLEQL TLEKCNLTSI PTEALSHLHG LIVLRLRHLN INAIRDYSFK RLYRLKVLEI
241 SHWPYLDTMT PNCLYGLNLT SLSITHCNLT AVPYLAVRHL VYLRFLNLSY NRISTIEGSM
301 LHELLRLQEI QLVGGQLAVV EPYAFRGLNY LRVLNVSGNQ LTTLEESVFH SVGNLETLIL
361 DSNPLACDCR LLWVFRRRWR LNFNRQQPTC ATPEFVQGKE FKDFPDVLLP NYFTCRRARI
421 RDRKAQQVFV DEGHTVQFVC RADGDPPPAI LWLSPRKHLV SAKSNGRLTV FPDGTLEVRY
481 AQVQDNGTYL CIAANAGGND SMPAHLHVRS YSPDWPHQPN KTFAFISNQP GEGEANSTRA
541 TVPFPFDIKT LIIATTMGFI SFLGVVLFCL VLLFLWSRGK GNTKHNIEIE YVPRKSDAGI
601 SSADAPRKFN MKMI
SEQ ID NO:273
   PALMITOYL-PROTEIN THIOESTERASE 1 (PPT1)
   AAH08426.1 
1 
61 
121 
181 
241 
301 
SEQ ID NO:274
   TRIPEPTIDYL-PEPTIDASE 1 (TPP1)
   NP_000382.3 
1 
61 
121 
181 
241 
301 
361 
421 
481 
541 
SEQ ID NO:275
   BATTENIN (CLN3)
   AAI11069.1 
1 MLSAAHDILS HKRTSGNQSH AVLLADILPT LVIKLLAPLG LHLLPYSPRV LVSGICAAGS
61 FVLVAFSHSV GTSLCGVVFA SISSGLGEVT FLSLTAFYPR AVISWWSSGT GGAGLLGALS
121 YLGLTQAGLS PQQTLLSMLG IPALLLASYF LLLTSPEAQD PGGEEEAESA ARQPLIRTEA
181 PESKPGSSSS LSLRERWTVF KGLLWYIVPL VVVYFAEYFI NQGLFELLFF WNTSLSHAQQ
241 YRWYQMLYQA GVFASRSSLR CCRIRFTWAL ALLQCLNLVF LLADVWFGFL PSIYLVFLII
301 LYEGLLGGAA YVNTFHNIAL ETSDEHREFA MAATCISDTL GISLSGLLAL PLHDFLCQLS
SEQ ID NO:276
   CLN6 TRANSMEMBRANE ER PROTEIN (CLN6)
   NP_060352.1 
1 
61 
121 
181 
241 
301 
SEQ ID NO:277
   MAJOR FACILITATOR SUPERFAMILY DOMAIN CONTAINING 8 (MFSD8)
   AAH29503.1 
1 
61 
121 
181 
241 
301 
361 
421 
481 
SEQ ID NO:278
   MYOSIN VIIA (MYO7A)
   AAB03679.1 
1 MVILQQGDHV WMDLRLGQEF DVPIGAVVKL CDSGQVQVVD DEDNEHWISP QNATHIKPMH
61 PTSVHGVEDM IRLGDLNEAG ILRNLLIRYR DHLIYTYTGS ILVAVNPYQL LSIYSPEHIR
121 QYTNKKIGEM PPHIFAIADN CYFNMKRNSR DQCCIISGES GAGKTESTKL ILQFLAAISG
181 QHSWIEQQVL EATPILEAFG NAKTIRNDNS SRFGKYIDIH FNKRGAIEGA KIEQYLLEKS
241 RVCRQALDER NYHVFYCMLE GMSEDQKKKL GLGQASDYNY LAMGNCITCE GRVDSQEYAN
301 IRSAMKVLMF TDTENWEISK LLAAILHLGN LQYEARTFEN LDACEVLFSP SLATAASLLE
361 VNPPDLMSCL TSRTLITRGE TVSTPLSREQ ALDVRDAFVK GIYGRLFVWI VDKINAAIYK
421 PPSQDVKNSR RSIGLLDIFG FENFAVNSFE QLCINFANEH LQQFFVRHVF KLEQEEYDLE
481 SIDWLHIEFT DNQDALDMIA NKPMNIISLI DEESKFPKGT DTTMLHKLNS QHKLNANYIP
541 PKNNHETQFG INHFAGIVYY ETQGFLEKNR DTLHGDIIQL VHSSRNKFIK QIFQADVAMG
601 AETRKRSPTL SSQFKRSLEL LMRTLGACQP FFVRCIKPNE FKKPMLFDRH LCVRQLRYSG
661 MMETIRIRRA GYPIRYSFVE FVERYRVLLP GVKPAYKQGD LRGTCQRMAE AVLGTHDDWQ
721 IGKTKIFLKD HHDMLLEVER DKAITDRVIL LQKVIRGFKD RSNFLKLKNA ATLIQRHWRG
781 HNCRKNYGLM RLGFLRLQAL HRSRKLHQQY RLARQRIIQF QARCRAYLVR KAFRHRLWAV
841 LTVQAYARGM IARRLHQRLR AEYLWRLEAE KMRLAEEEKL RKEMSAKKAK EEAERKHQER
901 LAQLAREDAE RELKEKEAAR RKKELLEQME RARHEPVNHS DMVDKMFGFL GTSGGLPGQE
961 GQAPSGFEDL ERGRREMVEE DLDAALPLPD EDEEDLSEYK FAKFAATYFQ GTTTHSYTRR
1021 PLKQPLLYHD DEGDQLAALA VWITILRFMG DLPEPKYHTA MSDGSEKIPV MTKIYETLGK
1081 KTYKRELQAL QGEGEAQLPE GQKKSSVRHK LVHLTLKKKS KLTEEVTKRL HDGESTVQGN
1141 SMLEDRPTSN LEKLHFIIGN GILRPALRDE IYCQISKQLT HNPSKSSYAR GWILVSLCVG
1201 CFAPSEKFVK YLRNFIHGGP PGYAPYCEER LRRTFVNGTR TQPPSWLELQ ATKSKKPIML
1261 PVTFMDGTTK TLLTDSATTA KELCNALADK ISLKDRFGFS LYIALFDKVS SLGSGSDHVM
1321 DAISQCEQYA KEQGAQERNA PWRLFFRKEV FTPWHSPSED NVATNLIYQQ VVRGVKFGEY
1381 RCEKEDDLAE LASQQYFVDY GSEMILERLL NLVPTYIPDR EITPLKTLEK WAQLAIAAHK
1441 KGIYAQRRTD AQKVKEDVVS YARFKWPLLF SRFYEAYKFS GPSLPKNDVI VAVNWTGVYF
1501 VDEQEQVLLE LSFPEIMAVS SSRECRVWLS LGCSDLGCAA PHSGWAGLTP AGPCSPCWSC
1561 RGAKTTAPSF TLATIKGDEY TFTSSNAEDI RDLVVTFLEG LRKRSKYVVA LQDNPNPAGE
1621 ESGFLSFAKG DLIILDHDTG EQVMNSGWAN GINERTKQRG DFPTDCVYVM PTVTMPPREI
1681 VALVTMTPDQ RQDVVRLLQL RTAEPEVRAK PYTLEEFSYD YFRPPPKHTL SRVMVSKARG
1741 KDRLWSHTRE PLKQALLKKL LGSEELSQEA CLAFIAVLKY MGDYPSKRTR SVNELTDQIF
1801 EGPLKAEPLK DEAYVQILKQ LTDNHIRYSE ERGWELLWLC TGLFPPSNIL LPHVQRFLQS
 
1861 RKHCPLAIDC LQRLQKALRN GSRKYPPHLV EVEAIQHKTT QIFHKVYFPD DTDEAFEVES
1921 STKAKDFCQN IATRLLLKSS EGFSLFVKIA DKVISVPEND FFFDFVRHLT DWIKKARPIK
1981 DGIVPSLTYQ VFFMKKLWTT TVPGKDPMAD SIFHYYQELP KYLRGYHKCT REEVLQLGAL
2041 IYRVKFEEDK SYFPSIPKLL RELVPQDLIR QVSPDDWKRS IVAYFNKHAG KSKEEAKLAF
2101 LKLIFKWPTF GSAFFEVKQT TEPNFPEILL IAINKYGVSL IDPKTKDILT THPFTKISNW
2161 SSGNTYFHIT IGNLVRGSKL LCETSLGYKM DDLLTSYISQ MLTAMSKQRG SRSGK
SEQ ID NO:279
   MYOSIN VIIA (MYO7A), ISOFORM CRA_A
   EAW75018.1 
1 MVILQQGDHV WMDLRLGQEF DVPIGAVVKL CDSGQVQVVD DEDNEHWISP QNATHIKPMH
61 PTSVHGVEDM IRLGDLNEAG ILRNLLIRYR DHLIYTYTGS ILVAVNPYQL LSIYSPEHIR
121 QYTNKKIGEM PPHIFAIADN CYFNMKRNSR DQCCIISGES GAGKTESTKL ILQFLAAISG
181 QHSWIEQQVL EATPILEAFG NAKTIRNDNS SRFGKYIDIH FNKRGAIEGA KIEQYLLEKS
241 RVCRQALDER NYHVFYCMLE GMSEDQKKKL GLGQASDYNY LAMGNCITCE GRVDSQEYAN
301 IRSAMKVLMF TDTENWEISK LLAAILHLGN LQYEARTFEN LDACEVLFSP SLATAASLLE
361 VNPPDLMSCL TSRTLITRGE TVSTPLSREQ ALDVRDAFVK GIYGRLFVWI VDKINAAIYK
421 PPSQDVKNSR RSIGLLDIFG FENFAVNSFE QLCINFANEH LQQFFVRHVF KLEQEEYDLE
481 SIDWLHIEFT DNQDALDMIA NKPMNIISLI DEESKFPKGT DTTMLHKLNS QHKLNANYIP
541 PKNNHETQFG INHFAGIVYY ETQGFLEKNR DTLHGDIIQL VHSSRNKFIK QIFQADVAMG
601 AETRKRSPTL SSQFKRSLEL LMRTLGACQP FFVRCIKPNE FKKPMLFDRH LCVRQLRYSG
661 MMETIRIRRA GYPIRYSFVE FVERYRVLLP GVKPAYKQGD LRGTCQRMAE AVLGTHDDWQ
721 IGKTKIFLKD HHDMLLEVER DKAITDRVIL LQKVIRGFKD RSNFLKLKNA ATLIQRHWRG
781 HNCRKNYGLM RLGFLRLQAL HRSRKLHQQY RLARQRIIQF QARCRAYLVR KAFRHRLWAV
841 LTVQAYARGM IARRLHQRLR AEYLWRLEAE KMRLAEEEKL RKEMSAKKAK EEAERKHQER
901 LAQLAREDAE RELKEKEAAR RKKELLEQME RARHEPVNHS DMVDKMFGFL GTSGGLPGQE
961 GQAPSGFEDL ERGRREMVEE DLDAALPLPD EDEEDLSEYK FAKFAATYFQ GTTTHSYTRR
1021 PLKQPLLYHD DEGDQLAALA VWITILRFMG DLPEPKYHTA MSDGSEKIPV MTKIYETLGK
1081 KTYKRELQAL QGEGEAQLPE GQKKSSVRHK LVHLTLKKKS KLTEEVTKRL HDGESTVQGN
1141 SMLEDRPTSN LEKLHFIIGN GILRPALRSV PGGGDTRA
SEQ ID NO:280
   MYOSIN VIIA (MYO7A), ISOFORM CRA_B
   EAW75019.1 
1 MVILQQGDHV WMDLRLGQEF DVPIGAVVKL CDSGQVQVVD DEDNEHWISP QNATHIKPMH
61 PTSVHGVEDM IRLGDLNEAG ILRNLLIRYR DHLIYTYTGS ILVAVNPYQL LSIYSPEHIR
121 QYTNKKIGEM PPHIFAIADN CYFNMKRNSR DQCCIISGES GAGKTESTKL ILQFLAAISG
181 QHSWIEQQVL EATPILEAFG NAKTIRNDNS SRFGKYIDIH FNKRGAIEGA KIEQYLLEKS
241 RVCRQALDER NYHVFYCMLE GMSEDQKKKL GLGQASDYNY LAMGNCITCE GRVDSQEYAN
301 IRSAMKVLMF TDTENWEISK LLAAILHLGN LQYEARTFEN LDACEVLFSP SLATAASLLE
361 VNPPDLMSCL TSRTLITRGE TVSTPLSREQ ALDVRDAFVK GIYGRLFVWI VDKINAAIYK
421 PPSQDVKNSR RSIGLLDIFG FENFAVNSFE QLCINFANEH LQQFFVRHVF KLEQEEYDLE
481 SIDWLHIEFT DNQDALDMIA NKPMNIISLI DEESKFPKGT DTTMLHKLNS QHKLNANYIP
541 PKNNHETQFG INHFAGIVYY ETQGFLEKNR DTLHGDIIQL VHSSRNKFIK QIFQADVAMG
601 AETRKRSPTL SSQFKRSLEL LMRTLGACQP FFVRCIKPNE FKKPMLFDRH LCVRQLRYSG
661 MMETIRIRRA GYPIRYSFVE FVERYRVLLP GVKPAYKQGD LRGTCQRMAE AVLGTHDDWQ
721 IGKTKIFLKD HHDMLLEVER DKAITDRVIL LQKVIRGFKD RSNFLKLKNA ATLIQRHWRG
781 HNCRKNYGLM RLGFLRLQAL HRSRKLHQQY RLARQRIIQF QARCRAYLVR KAFRHRLWAV
841 LTVQAYARGM IARRLHQRLR AEYLWRLEAE KMRLAEEEKL RKEMSAKKAK EEAERKHQER
901 LAQLAREDAE RELKEKEAAR RKKELLEQME RARHEPVNHS DMVDKMFGFL GTSGGLPGQE
961 GQAPSGFEDL ERGRREMVEE DLDAALPLPD EDEEDLSEYK FAKFAATYFQ GTTTHSYTRR
1021 PLKQPLLYHD DEGDQLAALA VWITILRFMG DLPEPKYHTA MSDGSEKIPV MTKIYETLGK
1081 KTYKRELQAL QGEGEAQLPE GQKKSSVRHK LVHLTLKKKS KLTEEVTKRL HDGESTVQGN
1141 SMLEDRPTSN LEKLHFIIGN GILRPALRDE IYCQISKQLT HNPSKSSYAR GWILVSLCVG
1201 CFAPSEKFVK YLRNFIHGGP PGYAPYCEER LRRTFVNGTR TQPPSWLELQ ATKSKKPIML
1261 PVTFMDGTTK TLLTDSATTA KELCNALADK ISLKDRFGFS LYIALFDKVS SLGSGSDHVM
1321 DAISQCEQYA KEQGAQERNA PWRLFFRKEV FTPWHSPSED NVATNLIYQQ VVRGVKFGEY
 
1381 RCEKEDDLAE LASQQYFVDY GSEMILERLL NLVPTYIPDR EITPLKTLEK WAQLAIAAHK
1441 KGIYAQRRTD AQKVKEDVVS YARFKWPLLF SRFYEAYKFS GPSLPKNDVI VAVNWTGVYF
1501 VDEQEQVLLE LSFPEIMAVS SSRECRVWLS LGCSDLGCAA PHSGWAGLTP AGPCSPCWSC
1561 RGAKTTAPSF TLATIKGDEY TFTSSNAEDI RDLVVTFLEG LRKRSKYVVA LQDNPNPAGE
1621 ESGFLSFAKG DLIILDHDTG EQVMNSGWAN GINERTKQRG DFPTDCVYVM PTVTMPPREI
1681 VALVTMTPDQ RQDVVRLLQL RTAEPEVRAK PYTLEEFSYD YFRPPPKHTL SRVMVSKARG
1741 KDRLWSHTRE PLKQALLKKL LGSEELSQEA CLAFIAVLKY MGDYPSKRTR SVNELTDQIF
1801 EGPLKAEPLK DEAYVQILKQ LTDNHIRYSE ERGWELLWLC TGLFPPSNIL LPHVQRFLQS
1861 RKHCPLAIDC LQRLQKALRN GSRKYPPHLV EVEAIQHKTT QIFHKVYFPD DTDEAFEVES
1921 STKAKDFCQN IATRLLLKSS EGFSLFVKIA DKVISVPEND FFFDFVRHLT DWIKKARPIK
1981 DGIVPSLTYQ VFFMKKLWTT TVPGKDPMAD SIFHYYQELP KYLRGYHKCT REEVLQLGAL
2041 IYRVKFEEDK SYFPSIPKLL RELVPQDLIR QVSPDDWKRS IVAYFNKHAG KSKEEAKLAF
2101 LKLIFKWPTF GSAFFEVKQT TEPNFPEILL IAINKYGVSL IDPKTKDILT THPFTKISNW
2161 SSGNTYFHIT IGNLVRGSKL LCETSLGYKM DDLLTSYISQ MLTAMSKQRG SRSGK
SEQ ID NO:281
   MYOSIN VIIA (MYO7A), ISOFORM CRA_C
   EAW75020.1 
1 
61 
121 
181 
241 
301 
361 
421 
481 
541 
601 
661 
721 
781 
841 
901 
961 
1021 
1081 
1141 
1201 
1261 
1321 
1381 
1441 
1501 
1561 
1621 
1681 
1741 
1801 
1861 
1921 
1981 
2041 
2101 
2161 
SEQ ID NO:282
   MYOSIN VIIA (MYO7A), ISOFORM CRA_D
   EAW75021.1 
1 MVILQQGDHV WMDLRLGQEF DVPIGAVVKL CDSGQVQVVD DEDNEHWISP QNATHIKPMH
61 PTSVHGVEDM IRLGDLNEAG ILRNLLIRYR DHLIYTYTGS ILVAVNPYQL LSIYSPEHIR
121 QYTNKKIGEM PPHIFAIADN CYFNMKRNSR DQCCIISGES GAGKTESTKL ILQFLAAISG
181 QHSWIEQQVL EATPILEAFG NAKTIRNDNS SRFGKYIDIH FNKRGAIEGA KIEQYLLEKS
241 RVCRQALDER NYHVFYCMLE GMSEDQKKKL GLGQASDYNY LAMGNCITCE GRVDSQEYAN
301 IRSAMKVLMF TDTENWEISK LLAAILHLGN LQYEARTFEN LDACEVLFSP SLATAASLLE
361 VNPPDLMSCL TSRTLITRGE TVSTPLSREQ ALDVRDAFVK GIYGRLFVWI VDKINAAIYK
421 PPSQDVKNSR RSIGLLDIFG FENFAVNSFE QLCINFANEH LQQFFVRHVF KLEQEEYDLE
481 SIDWLHIEFT DNQDALDMIA NKPMNIISLI DEESKFPKGT DTTMLHKLNS QHKLNANYIP
541 PKNNHETQFG INHFAGIVYY ETQGFLEKNR DTLHGDIIQL VHSSRNKFIK QIFQADVAMG
601 AETRKRSPTL SSQFKRSLEL LMRTLGACQP FFVRCIKPNE FKKPMLFDRH LCVRQLRYSG
661 MMETIRIRRA GYPIRYSFVE FVERYRVLLP GVKPAYKQGD LRGTCQRMAE AVLGTHDDWQ
721 IGKTKIFLKD HHDMLLEVER DKAITDRVIL LQKVIRGFKD RCVFPPAPPL LSPHTGVRVV
781 FGSPLLCPHE H
SEQ ID NO:283
   MYOSIN VIIA (MYO7A), ISOFORM CRA_E
   EAW75022.1 
1 
61 
121 
181 
241 
301 
361 
421 
481 
541 
601 
661 
721 
781 
841 
901 
961 
1021 
1081 
1141 
1201 
1261 
1321 
1381 
1441 
1501 
1561 
1621 
1681 
1741 
1801 
1861 
1921 
1981 
2041 
2101 
2161 
SEQ ID NO:284
   MYOSIN VIIA (MYO7A), ISOFORM CRA_F
   EAW75023.1 
1 
61 
121 
181 
241 
301 
361 
421 
481 
541 
601 
661 
721 
781 
841 
901 
961 
1021 
1081 
1141 
1201 
1261 
1321 
1381 
1441 
1501 
1561 
1621 
1681 
1741 
1801 
1861 
1921 
SEQ ID NO:285
   CADHERIN RELATED 23 (CDH23)
   AAG27034.2 
1 MGRHVATSCH VAWLLVLISG CWGQVNRLPF FTNHFFDTYL LISEDTPVGS SVTQLLAQDM
61 DNDPLVFGVS GEEASRFFAV EPDTGVVWLR QPLDRETKSE FTVEFSVSDH QGVITRKVNI
121 QVGDVNDNAP TFHNQPYSVR IPENTPVGTP IFIVNATDPD LGAGGSVLYS FQPPSQFFAI
181 DSARGIVTVI RELDYETTQA YQLTVNATDQ DKTRPLSTLA NLAIIITDVQ DMDPIFINLP
241 YSTNIYEHSP PGTTVRIITA IDQDKGRPRG IGYTIVSGNT NSIFALDYIS GVLTLNGLLD
301 RENPLYSHGF ILTVKGTELN DDRTPSDATV TTTFNILVID INDNAPEFNS SEYSVAITEL
361 AQVGFALPLF IQVVDKDENL GLNSMFEVYL VGNNSHHFII SPTSVQGKAD IRIRVAIPLD
421 YETVDRYDFD LFANESVPDH VGYAKVKITL INENDNRPIF SQPLYNISLY ENVTVGTSVL
481 TVLATDNDAG TFGEVSYFFS DDPDRFSLDK DTGLIMLIAR LDYELIQRFT LTIIARDGGG
541 EETTGRVRIN VLDVNDNVPT FQKDAYVGAL RENEPSVTQL VRLRATDEDS PPNNQITYSI
601 VSASAFGSYF DISLYEGYGV ISVSRPLDYE QISNGLIYLT VMAMDAGNPP LNSTVPVTIE
661 VFDENDNPPT FSKPAYFVSV VENIMAGATV LFLNATDLDR SREYGQESII YSLEGSTQFR
721 INARSGEITT TSLLDRETKS EYILIVRAVD GGVGHNQKTG IATVNITLLD INDNHPTWKD
781 APYYINLVEM TPPDSDVTTV VAVDPDLGEN GTLVYSIQPP NKFYSLNSTT GKIRTTHAML
841 DRENPDPHEA ELMRKIVVSV TDCGRPPLKA TSSATVFVNL LDLNDNDPTF QNLPFVAEVL
901 EGIPAGVSIY QVVAIDLDEG LNGLVSYRMP VGMPRMDFLI NSSSGVVVTT TELDRERIAE
 
961 YQLRVVASDA GTPTKSSTST LTIHVLDVND ETPTFFPAVY NVSVSEDVPR EFRVVWLNCT
1021 DNDVGLNAEL SYFITGGNVD GKFSVGYRDA VVRTVVGLDR ETTAAYMLIL EAIDNGPVGK
1081 RHTGTATVFV TVLDVNDNRP IFLQSSYEAS VPEDIPEGHS ILQLKATDAD EGEFGRVWYR
1141 ILHGNHGNNF RIHVSNGLLM RGPRPLDRER NSSHVLIVEA YNHDLGPMRS SVRVIVYVED
1201 INDEAPVFTQ QQYSRLGLRE TAGIGTSVIV VQATDRDSGD GGLVNYRILS GAEGKFEIDE
1261 STGLIITVNY LDYETKTSYM MNVSATDQAP PFNQGFCSVY ITLLNELDEA VQFSNASYEA
1321 AILENLALGT EIVRVQAYSI DNLNQITYRF DAYTSTQAKA LFKIDAITGV ITVQGLVDRE
1381 KGDFYTLTVV ADDGGPKVDS TVKVYITVLD ENDNSPRFDF TSDSAVSIPE DCPVGQRVAT
1441 VKAWDPDAGS NGQVVFSLAS GNIAGAFEIV TTNDSIGEVF VARPLDREEL DHYILQVVAS
1501 DRGTPPRKKD HILQVTILDI NDNPPVIESP FGYNVSVNEN VGGGTAVVQV RATDRDIGIN
1561 SVLSYYITEG NKDMTFRMDR ISGEIATRPA PPDRERQSFY HLVATVEDEG TPTLSATTHV
1621 YVTIVDENDN APMFQQPHYE VLLDEGPDTL NTSLITIQAL DLDEGPNGTV TYAIVAGNIV
1681 NTFRIDRHMG VITAAKELDY EISHGRYTLI VTATDQCPIL SHRLTSTTTV LVNVNDINDN
1741 VPTFPRDYEG PFEVTEGQPG PRVWTFLAHD RDSGPNGQVE YSIMDGDPLG EFVISPVEGV
1801 LRVRKDVELD RETIAFYNLT ICARDRGMPP LSSTMLVGIR VLDINDNDPV LLNLPMNITI
1861 SENSPVSSFV AHVLASDADS GCNARLTFNI TAGNRERAFF INATTGIVTV NRPLDRERIP
1921 EYKLTISVKD NPENPRIARR DYDLLLIFLS DENDNHPLFT KSTYQAEVME NSPAGTPLTV
1981 LNGPILALDA DQDIYAVVTY QLLGAQSGLF DINSSTGVVT VRSGVIIDRE AFSPPILELL
2041 LLAEDIGLLN STAHLLITIL DDNDNRPTFS PATLTVHLLE NCPPGFSVLQ VTATDEDSGL
2101 NGELVYRIEA GAQDRFLIHL VTGVIRVGNA TIDREEQESY RLTVVATDRG TVPLSGTAIV
2161 TILIDDINDS RPEFLNPIQT VSVLESAEPG TVIANITAID HDLNPKLEYH IVGIVAKDDT
2221 DRLVPNQEDA FAVNINTGSV MVKSPMNREL VATYEVTLSV IDNASDLPER SVSVPNAKLT
2281 VNVLDVNDNT PQFKPFGITY YMERILEGAT PGTTLIAVAA VDPDKGLNGL VTYTLLDLVP
2341 PGYVQLEDSS AGKVIANRTV DYEEVHWLNF TVRASDNGSP PRAAEIPVYL EIVDINDNNP
2401 IFDQPSYQEA VFEDVPVGTI ILTVTATDAD SGNFALIEYS LGDGESKFAI NPTTGDIYVL
2461 SSLDREKKDH YILTALAKDN PGDVASNRRE NSVQVVIQVL DVNDCRPQFS KPQFSTSVYE
2521 NEPAGTSVIT MMATDQDEGP NGELTYSLEG PGVEAFHVDM DSGLVTTQRP LQSYEKFSLT
2581 VVATDGGEPP LWGTTMLLVE VIDVNDNRPV FVRPPNGTIL HIREEIPLRS NVYEVYATDK
2641 DEGLNGAVRY SFLKTAGNRD WEFFIIDPIS GLIQTAQRLD RESQAVYSLI LVASDLGQPV
2701 PYETMQPLQV ALEDIDDNEP LFVRPPKGSP QYQLLTVPEH SPRGTLVGNV TGAVDADEGP
2761 NAIVYYFIAA GNEEKNFHLQ PDGCLLVLRD LDREREAIFS FIVKASSNRS WTPPRGPSPT
2821 LDLVADLTLQ EVRVVLEDIN DQPPRFTKAE YTAGVATDAK VGSELIQVLA LDADIGNNSL
2881 VFYSILAIHY FRALANDSED VGQVFTMGSM DGILRTFDLF MAYSPGYFVV DIVARDLAGH
2941 NDTAIIGIYI LRDDQRVKIV INEIPDRVRG FEEEFIHLLS NITGAIVNTD NVQFHVDKKG
3001 RVNFAQTELL IHVVNRDTNR ILDVDRVIQM IDENKEQLRN LFRNYNVLDV QPAISVRLPD
3061 DMSALQMAII VLAILLFLAA MLFVLMNWYY RTVHKRKLKA IVAGSAGNRG FIDIMDMPNT
3121 NKYSFDGANP VWLDPFCRNL ELAAQAEHED DLPENLSEIA DLWNSPTRTH GTFGREPAAV
3181 KPDDDRYLRA AIQEYDNIAK LGQIIREGPI KGSLLKVVLE DYLRLKKLFA QRMVQKASSC
3241 HSSISELIQT ELDEEPGDHS PGQGSLRFRH KPPVELKGPD GIHVVHGSTG TLLATDLNSL
3301 PEEDQKGLGR SLETLTAAEA TAFERNARTE SAKSTPLHKL RDVIMETPLE ITEL
SEQ ID NO:286
   PROTOCADHERIN RELATED 15 (PCDH15)
   AAK31581.1 
1 
61 
121 
181 
241 
301 
361 
421 
481 
541 
601 
661 
721 
 
781 
841 
901 
961 
1021 
1081 
1141 
1201 
1261 
1321 
1381 
1441 
1501 
1561 
1621 
1681 
1741 
1801 
1861 
1921 
SEQ ID NO:287
   PROTOCADHERIN RELATED 15 (PCDH15), ISOFORM CRA_A
   EAW54151.1 
1 MFRQFYLWTC LASGIILGSL FEICLGQYDD DCKLARGGPP ATIVAIDEES RNGTILVDNM
61 LIKGTAGGPD PTIELSLKDN VDYWVLMDPV KQMLFLNSTG RVLDRDPPMN IHSIVVQVQC
121 INKKVGTIIY HEVRIVVRDR NDNSPTFKHE SYYATVNELT PVGTTIFTGF SGDNGATDID
181 DGPNGQIEYV IQYNPDDPTS NDTFEIPLML TGNIVLRKRL NYEDKTRYFV IIQANDRAQN
241 LNERRTTTTT LTVDVLDGDD LGPMFLPCVL VPNTRDCRPL TYQAAIPELR TPEELNPIIV
301 TPPIQAIDQD RNIQPPSDRP GILYSILVGT PEDYPRFFHM HPRTAELSLL EPVNRDFHQK
361 FDLVIKAEQD NGHPLPAFAG LHIEILDENN QSPYFTMPSY QGYILESAPV GATISDSLNL
421 TSPLRIVALD KDIEDTKDPE LHLFLNDYTS VFTVTQTGIT RYLTLLQPVD REEQQTYTFS
481 ITAFDGVQES EPVIVNIQVM DANDNTPTFP EISYDVYVYT DMRPGDSVIQ LTAVDADEGS
541 NGEITYEILV GAQGDFIINK TTGLITIAPG VEMIVGRTYA LTVQAADNAP PAERRNSICT
601 VYIEVLPPNN QSPPRFPQLM YSLEISEAMR VGAVLLNLQA TDREGDSITY AIENGDPQRV
661 FNLSETTGIL TLGKALDRES TDRYILIITA SDGRPDGTST ATVNIVVTDV NDNAPVFDPY
721 LPRNLSVVEE EANAFVGQVK ATDPDAGING QVHYSLGNFN NLFRITSNGS IYTAVKLNRE
781 VRDYYELVVV ATDGAVHPRH STLTLAIKVL DIDDNSPVFT NSTYTVLVEE NLPAGTTILQ
841 IEAKDVDLGA NVSYRIRSPE VKHFFALHPF TGELSLLRSL DYEAFPDQEA SITFLVEAFD
901 IYGTMPPGIA TVTVIVKDMN DYPPVFSKRI YKGMVAPDAV KGTPITTVYA EDADPPGLPA
961 SRVRYRVDDV QFPYPASIFE VEEDSGRVIT RVNLNEEPTT IFKLVVVAFD DGEPVMSSSA
1021 TVKILVLHPG EIPRFTQEEY RPPPVSELAT KGTMVGVISA AAINQSIVYS IVSGNEEDTF
1081 GINNITGVIY VNGPLDYETR TSYVLRVQAD SLEVVLANLR VPSKSNTAKV YIEIQDENNH
1141 PPVFQKKFYI GGVSEDARMF TSVLRVKATD KDTGNYSVMA YRLIIPPIKE GKEGFVVETY
1201 TGLIKTAMLF HNMRRSYFKF QVIATDDYGK GLSGKADVLV SVVNQLDMQV IVSNVPPTLV
1261 EKKIEDLTEI LDRYVQEQIP GAKVVVESIG ARRHGDAFSL EDYTKCDLTV YAIDPQTNRA
1321 IDRNELFKFL DGKLLDINKD FQPYYGEGGR ILEIRTPEAV TSIKKRGESL GYTEGALLAL
1381 AFIIILCCIP AILVVLVSYR Q
SEQ ID NO:288
   PROTOCADHERIN RELATED 15 (PCDH15), ISOFORM X1
   XP_016872062.1 
1 MFRQFYLWTC LASGIILGSL FEICLGQYDD DWQYEDCKLA RGGPPATIVA IDEESRNGTI
61 LVDNMLIKGT AGGPDPTIEL SLKDNVDYWV LMDPVKQMLF LNSTGRVLDR DPPMNIHSIV
121 VQVQCINKKV GTIIYHEVRI VVRDRNDNSP TFKHESYYAT VNELTPVGTT IFTGFSGDNG
181 ATDIDDGPNG QIEYVIQYNP DDPTSNDTFE IPLMLTGNIV LRKRLNYEDK TRYFVIIQAN
241 DRAQNLNERR TTTTTLTVDV LDGDDLGPMF LPCVLVPNTR DCRPLTYQAA IPELRTPEEL
 
301 NPIIVTPPIQ AIDQDRNIQP PSDRPGILYS ILVGTPEDYP RFFHMHPRTA ELSLLEPVNR
361 DFHQKFDLVI KAEQDNGHPL PAFAGLHIEI LDENNQSPYF TMPSYQGYIL ESAPVGATIS
421 DSLNLTSPLR IVALDKDIED TKDPELHLFL NDYTSVFTVT QTGITRYLTL LQPVDREEQQ
481 TYTFSITAFD GVQESEPVIV NIQVMDANDN TPTFPEISYD VYVYTDMRPG DSVIQLTAVD
541 ADEGSNGEIT YEILVGAQGD FIINKTTGLI TIAPGVEMIV GRTYALTVQA ADNAPPAERR
601 NSICTVYIEV LPPNNQSPPR FPQLMYSLEI SEAMRVGAVL LNLQATDREG DSITYAIENG
661 DPQRVFNLSE TTGILTLGKA LDRESTDRYI LIITASDGRP DGTSTATVNI VVTDVNDNAP
721 VFDPYLPRNL SVVEEEANAF VGQVKATDPD AGINGQVHYS LGNFNNLFRI TSNGSIYTAV
781 KLNREVRDYY ELVVVATDGA VHPRHSTLTL AIKVLDIDDN SPVFTNSTYT VLVEENLPAG
841 TTILQIEAKD VDLGANVSYR IRSPEVKHFF ALHPFTGELS LLRSLDYEAF PDQEASITFL
901 VEAFDIYGTM PPGIATVTVI VKDMNDYPPV FSKRIYKGMV APDAVKGTPI TTVYAEDADP
961 PGLPASRVRY RVDDVQFPYP ASIFEVEEDS GRVITRVNLN EEPTTIFKLV VVAFDDGEPV
1021 MSSSATVKIL VLHPGEIPRF TQEEYRPPPV SELATKGTMV GVISAAAINQ SIVYSIVSGN
1081 EEDTFGINNI TGVIYVNGPL DYETRTSYVL RVQADSLEVV LANLRVPSKS NTAKVYIEIQ
1141 DENNHPPVFQ KKFYIGGVSE DARMFTSVLR VKATDKDTGN YSVMAYRLII PPIKEGKEGF
1201 VVETYTGLIK TAMLFHNMRR SYFKFQVIAT DDYGKGLSGK ADVLVSVVNQ LDMQVIVSNV
1261 PPTLVEKKIE DLTEILDRYV QEQIPGAKVV VESIGARRHG DAFSLEDYTK CDLTVYAIDP
1321 QTNRAIDRNE LFKFLDGKLL DINKDFQPYY GEGGRILEIR TPEAVTSIKK RGESLGYTEG
1381 ALLALAFIII LCCIPAILVV LVSYRQFKVR QAECTKTARI QAALPAAKPA VPAPAPVAAP
1441 PPPPPPPPGA HLYEELGDSS MHKYEMPQYG SRRRLLPPAG QEEYGEVVGE AEEEYEEEEE
1501 EPKKIKKPKV EIREPSEEEE VVVTIEKPPA AEPTYTTWKR ARIFPMIFKK VRGLADKRGI
1561 VDLEGEEWQR RLEEEDKDYL KLTLDQEEAT ESTVESEEES SSDYTEYSEE ESEFSESETT
1621 EEESESETPS EEEESSTPES EESESTESEG EKARKNIVLA RRRPMVEEVK EVKGRKEEPQ
1681 EEQKEPKMEE EEHSEEEESG PAPVEESTDP EAQDIPEEGS AESASVEGGV ESEEESESGS
1741 SSSSSESQSG GPWGYQVPAY DRSKNANQKK SPGANSEGYN TAL
SEQ ID NO:289
   USHERIN (USH2A), ISOFORM A
   NP_009054.5 
1 MNCPVLSLGS GFLFQVIEML IFAYFASISL TESRGLFPRL ENVGAFKKVS IVPTQAVCGL
61 PDRSTFCHSS AAAESIQFCT QRFCIQDCPY RSSHPTYTAL FSAGLSSCIT PDKNDLHPNA
121 HSNSASFIFG NHKSCFSSPP SPKLMASFTL AVWLKPEQQG VMCVIEKTVD GQIVFKLTIS
181 EKETMFYYRT VNGLQPPIKV MTLGRILVKK WIHLSVQVHQ TKISFFINGV EKDHTPFNAR
241 TLSGSITDFA SGTVQIGQSL NGLEQFVGRM QDFRLYQVAL TNREILEVFS GDLLRLHAQS
301 HCRCPGSHPR VHPLAQRYCI PNDAGDTADN RVSRLNPEAH PLSFVNDNDV GTSWVSNVFT
361 NITQLNQGVT ISVDLENGQY QVFYIIIQFF SPQPTEIRIQ RKKENSLDWE DWQYFARNCG
421 AFGMKNNGDL EKPDSVNCLQ LSNFTPYSRG NVTFSILTPG PNYRPGYNNF YNTPSLQEFV
481 KATQIRFHFH GQYYTTETAV NLRHRYYAVD EITISGRCQC HGHADNCDTT SQPYRCLCSQ
541 ESFTEGLHCD RCLPLYNDKP FRQGDQVYAF NCKPCQCNSH SKSCHYNISV DPFPFEHFRG
601 GGGVCDDCEH NTTGRNCELC KDYFFRQVGA DPSAIDVCKP CDCDTVGTRN GSILCDQIGG
661 QCNCKRHVSG RQCNQCQNGF YNLQELDPDG CSPCNCNTSG TVDGDITCHQ NSGQCKCKAN
721 VIGLRCDHCN FGFKFLRSFN DVGCEPCQCN LHGSVNKFCN PHSGQCECKK EAKGLQCDTC
781 RENFYGLDVT NCKACDCDTA GSLPGTVCNA KTGQCICKPN VEGRQCNKCL EGNFYLRQNN
841 SFLCLPCNCD KTGTINGSLL CNKSTGQCPC KLGVTGLRCN QCEPHRYNLT IDNFQHCQMC
901 ECDSLGTLPG TICDPISGQC LCVPNRQGRR CNQCQPGFYI SPGNATGCLP CSCHTTGAVN
961 HICNSLTGQC VCQDASIAGQ RCDQCKDHYF GFDPQTGRCQ PCNCHLSGAL NETCHLVTGQ
1021 CFCKQFVTGS KCDACVPSAS HLDVNNLLGC SKTPFQQPPP RGQVQSSSAI NLSWSPPDSP
1081 NAHWLTYSLL RDGFEIYTTE DQYPYSIQYF LDTDLLPYTK YSYYIETTNV HGSTRSVAVT
1141 YKTKPGVPEG NLTLSYIIPI GSDSVTLTWT TLSNQSGPIE KYILSCAPLA GGQPCVSYEG
1201 HETSATIWNL VPFAKYDFSV QACTSGGCLH SLPITVTTAQ APPQRLSPPK MQKISSTELH
1261 VEWSPPAELN GIIIRYELYM RRLRSTKETT SEESRVFQSS GWLSPHSFVE SANENALKPP
1321 QTMTTITGLE PYTKYEFRVL AVNMAGSVSS AWVSERTGES APVFMIPPSV FPLSSYSLNI
1381 SWEKPADNVT RGKVVGYDIN MLSEQSPQQS IPMAFSQLLH TAKSQELSYT VEGLKPYRIY
1441 EFTITLCNSV GCVTSASGAG QTLAAAPAQL RPPLVKGINS TTIHLKWFPP EELNGPSPIY
1501 QLERRESSLP ALMTTMMKGI RFIGNGYCKF PSSTHPVNTD FTGKCV
SEQ ID NO:290
   USHERIN (USH2A), ISOFORM B PRECURSOR
   NP_996816.2 
1 MNCPVLSLGS GFLFQVIEML IFAYFASISL TESRGLFPRL ENVGAFKKVS IVPTQAVCGL
61 PDRSTFCHSS AAAESIQFCT QRFCIQDCPY RSSHPTYTAL FSAGLSSCIT PDKNDLHPNA
121 HSNSASFIFG NHKSCFSSPP SPKLMASFTL AVWLKPEQQG VMCVIEKTVD GQIVFKLTIS
181 EKETMFYYRT VNGLQPPIKV MTLGRILVKK WIHLSVQVHQ TKISFFINGV EKDHTPFNAR
241 TLSGSITDFA SGTVQIGQSL NGLEQFVGRM QDFRLYQVAL TNREILEVFS GDLLRLHAQS
301 HCRCPGSHPR VHPLAQRYCI PNDAGDTADN RVSRLNPEAH PLSFVNDNDV GTSWVSNVFT
361 NITQLNQGVT ISVDLENGQY QVFYIIIQFF SPQPTEIRIQ RKKENSLDWE DWQYFARNCG
421 AFGMKNNGDL EKPDSVNCLQ LSNFTPYSRG NVTFSILTPG PNYRPGYNNF YNTPSLQEFV
481 KATQIRFHFH GQYYTTETAV NLRHRYYAVD EITISGRCQC HGHADNCDTT SQPYRCLCSQ
541 ESFTEGLHCD RCLPLYNDKP FRQGDQVYAF NCKPCQCNSH SKSCHYNISV DPFPFEHFRG
601 GGGVCDDCEH NTTGRNCELC KDYFFRQVGA DPSAIDVCKP CDCDTVGTRN GSILCDQIGG
661 QCNCKRHVSG RQCNQCQNGF YNLQELDPDG CSPCNCNTSG TVDGDITCHQ NSGQCKCKAN
721 VIGLRCDHCN FGFKFLRSFN DVGCEPCQCN LHGSVNKFCN PHSGQCECKK EAKGLQCDTC
781 RENFYGLDVT NCKACDCDTA GSLPGTVCNA KTGQCICKPN VEGRQCNKCL EGNFYLRQNN
841 SFLCLPCNCD KTGTINGSLL CNKSTGQCPC KLGVTGLRCN QCEPHRYNLT IDNFQHCQMC
901 ECDSLGTLPG TICDPISGQC LCVPNRQGRR CNQCQPGFYI SPGNATGCLP CSCHTTGAVN
961 HICNSLTGQC VCQDASIAGQ RCDQCKDHYF GFDPQTGRCQ PCNCHLSGAL NETCHLVTGQ
1021 CFCKQFVTGS KCDACVPSAS HLDVNNLLGC SKTPFQQPPP RGQVQSSSAI NLSWSPPDSP
1081 NAHWLTYSLL RDGFEIYTTE DQYPYSIQYF LDTDLLPYTK YSYYIETTNV HGSTRSVAVT
1141 YKTKPGVPEG NLTLSYIIPI GSDSVTLTWT TLSNQSGPIE KYILSCAPLA GGQPCVSYEG
1201 HETSATIWNL VPFAKYDFSV QACTSGGCLH SLPITVTTAQ APPQRLSPPK MQKISSTELH
1261 VEWSPPAELN GIIIRYELYM RRLRSTKETT SEESRVFQSS GWLSPHSFVE SANENALKPP
1321 QTMTTITGLE PYTKYEFRVL AVNMAGSVSS AWVSERTGES APVFMIPPSV FPLSSYSLNI
1381 SWEKPADNVT RGKVVGYDIN MLSEQSPQQS IPMAFSQLLH TAKSQELSYT VEGLKPYRIY
1441 EFTITLCNSV GCVTSASGAG QTLAAAPAQL RPPLVKGINS TTIHLKWFPP EELNGPSPIY
1501 QLERRESSLP ALMTTMMKGI RFIGNGYCKF PSSTHPVNTD FTGIKASFRT KVPEGLIVFA
1561 ASPGNQEEYF ALQLKKGRLY FLFDPQGSPV EVTTTNDHGK QYSDGKWHEI IAIRHQAFGQ
1621 ITLDGIYTGS SAILNGSTVI GDNTGVFLGG LPRSYTILRK DPEIIQKGFV GCLKDVHFMK
1681 NYNPSAIWEP LDWQSSEEQI NVYNSWEGCP ASLNEGAQFL GAGFLELHPY MFHGGMNFEI
1741 SFKFRTDQLN GLLLFVYNKD GPDFLAMELK SGILTFRLNT SLAFTQVDLL LGLSYCNGKW
1801 NKVIIKKEGS FISASVNGLM KHASESGDQP LVVNSPVYVG GIPQELLNSY QHLCLEQGFG
1861 GCMKDVKFTR GAVVNLASVS SGAVRVNLDG CLSTDSAVNC RGNDSILVYQ GKEQSVYEGG
1921 LQPFTEYLYR VIASHEGGSV YSDWSRGRTT GAAPQSVPTP SRVRSLNGYS IEVTWDEPVV
1981 RGVIEKYILK AYSEDSTRPP RMPSASAEFV NTSNLTGILT GLLPFKNYAV TLTACTLAGC
2041 TESSHALNIS TPQEAPQEVQ PPVAKSLPSS LLLSWNPPKK ANGIITQYCL YMDGRLIYSG
2101 SEENYTVTDL AVFTPHQFLL SACTHVGCTN SSWVLLYTAQ LPPEHVDSPV LTVLDSRTIH
2161 IQWKQPRKIS GILERYVLYM SNHTHDFTIW SVIYNSTELF QDHMLQYVLP GNKYLIKLGA
2221 CTGGGCTVSE ASEALTDEDI PEGVPAPKAH SYSPDSFNVS WTEPEYPNGV ITSYGLYLDG
2281 ILIHNSSELS YRAYGFAPWS LHSFRVQACT AKGCALGPLV ENRTLEAPPE GTVNVFVKTQ
2341 GSRKAHVRWE APFRPNGLLT HSVLFTGIFY VDPVGNNYTL LNVTKVMYSG EETNLWVLID
2401 GLVPFTNYTV QVNISNSQGS LITDPITIAM PPGAPDGVLP PRLSSATPTS LQVVWSTPAR
2461 NNAPGSPRYQ LQMRSGDSTH GFLELFSNPS ASLSYEVSDL QPYTEYMFRL VASNGFGSAH
2521 SSWIPFMTAE DKPGPVVPPI LLDVKSRMML VTWQHPRKSN GVITHYNIYL HGRLYLRTPG
2581 NVTNCTVMHL HPYTAYKFQV EACTSKGCSL SPESQTVWTL PGAPEGIPSP ELFSDTPTSV
2641 IISWQPPTHP NGLVENFTIE RRVKGKEEVT TLVTLPRSHS MRFIDKTSAL SPWTKYEYRV
2701 LMSTLHGGTN SSAWVEVTTR PSRPAGVQPP VVTVLEPDAV QVTWKPPLIQ NGDILSYEIH
2761 MPDPHITLTN VTSAVLSQKV THLIPFTNYS VTIVACSGGN GYLGGCTESL PTYVTTHPTV
2821 PQNVGPLSVI PLSESYVVIS WQPPSKPNGP NLRYELLRRK IQQPLASNPP EDLNRWHNIY
2881 SGTQWLYEDK GLSRFTTYEY MLFVHNSVGF TPSREVTVTT LAGLPERGAN LTASVLNHTA
2941 IDVRWAKPTV QDLQGEVEYY TLFWSSATSN DSLKILPDVN SHVIGHLKPN TEYWIFISVF
3001 NGVHSINSAG LHATTCDGEP QGMLPPEVVI INSTAVRVIW TSPSNPNGVV TEYSIYVNNK
3061 LYKTGMNVPG SFILRDLSPF TIYDIQVEVC TIYACVKSNG TQITTVEDTP SDIPTPTIRG
3121 ITSRSLQIDW VSPRKPNGII LGYDLLWKTW YPCAKTQKLV QDQSDELCKA VRCQKPESIC
3181 GHICYSSEAK VCCNGVLYNP KPGHRCCEEK YIPFVLNSTG VCCGGRIQEA QPNHQCCSGY
3241 YARILPGEVC CPDEQHNRVS VGIGDSCCGR MPYSTSGNQI CCAGRLHDGH GQKCCGRQIV
 
3301 SNDLECCGGE EGVVYNRLPG MFCCGQDYVN MSDTICCSAS SGESKAHIKK NDPVPVKCCE
3361 TELIPKSQKC CNGVGYNPLK YVCSDKISTG MMMKETKECR ILCPASMEAT EHCGRCDFNF
3421 TSHICTVIRG SHNSTGKASI EEMCSSAEET IHTGSVNTYS YTDVNLKPYM TYEYRISAWN
3481 SYGRGLSKAV RARTKEDVPQ GVSPPTWTKI DNLEDTIVLN WRKPIQSNGP IIYYILLRNG
3541 IERFRGTSLS FSDKEGIQPF QEYSYQLKAC TVAGCATSSK VVAATTQGVP ESILPPSITA
3601 LSAVALHLSW SVPEKSNGVI KEYQIRQVGK GLIHTDTTDR RQHTVTGLQP YTNYSFTLTA
3661 CTSAGCTSSE PFLGQTLQAA PEGVWVTPRH IIINSTTVEL YWSLPEKPNG LVSQYQLSRN
3721 GNLLFLGGSE EQNFTDKNLE PNSRYTYKLE VKTGGGSSAS DDYIVQTPMS TPEEIYPPYN
3781 ITVIGPYSIF VAWIPPGILI PEIPVEYNVL LNDGSVTPLA FSVGHHQSTL LENLTPFTQY
3841 EIRIQACQNG SCGVSSRMFV KTPEAAPMDL NSPVLKALGS ACIEIKWMPP EKPNGIIINY
3901 FIYRRPAGIE EESVLFVWSE GALEFMDEGD TLRPFTLYEY RVRACNSKGS VESLWSLTQT
3961 LEAPPQDFPA PWAQATSAHS VLLNWTKPES PNGIISHYRV VYQERPDDPT FNSPTVHAFT
4021 VKGTSHQAHL YGLEPFTTYR IGVVAANHAG EILSPWTLIQ TLESSPSGLR NFIVEQKENG
4081 RALLLQWSEP MRTNGVIKTY NIFSDGFLEY SGLNRQFLFR RLDPFTLYTL TLEACTRAGC
4141 AHSAPQPLWT DEAPPDSQLA PTVHSVKSTS VELSWSEPVN PNGKIIRYEV IRRCFEGKAW
4201 GNQTIQADEK IVFTEYNTER NTFMYNDTGL QPWTQCEYKI YTWNSAGHTC SSWNVVRTLQ
4261 APPEGLSPPV ISYVSMNPQK LLISWIPPEQ SNGIIQSYRL QRNEMLYPFS FDPVTFNYTD
4321 EELLPFSTYS YALQACTSGG CSTSKPTSIT TLEAAPSEVS PPDLWAVSAT QMNVCWSPPT
4381 VQNGKITKYL VRYDNKESLA GQGLCLLVSH LQPYSQYNFS LVACTNGGCT ASVSKSAWTM
4441 EALPENMDSP TLQVTGSESI EITWKPPRNP NGQIRSYELR RDGTIVYTGL ETRYRDFTLT
4501 PGVEYSYTVT ASNSQGGILS PLVKDRTSPS APSGMEPPKL QARGPQEILV NWDPPVRTNG
4561 DIINYTLFIR ELFERETKII HINTTHNSFG MQSYIVNQLK PFHRYEIRIQ ACTTLGCASS
4621 DWTFIQTPEI APLMQPPPHL EVQMAPGGFQ PTVSLLWTGP LQPNGKVLYY ELYRRQIATQ
4681 PRKSNPVLIY NGSSTSFIDS ELLPFTEYEY QVWAVNSAGK APSSWTWCRT GPAPPEGLRA
4741 PTFHVISSTQ AVVNISAPGK PNGIVSLYRL FSSSAHGAET VLSEGMATQQ TLHGLQAFTN
4801 YSIGVEACTC FNCCSKGPTA ELRTHPAPPS GLSSPQIGTL ASRTASFRWS PPMFPNGVIH
4861 SYELQFHVAC PPDSALPCTP SQIETKYTGL GQKASLGGLQ PYTTYKLRVV AHNEVGSTAS
4921 EWISFTTQKE LPQYRAPFSV DSNLSVVCVN WSDTFLLNGQ LKEYVLTDGG RRVYSGLDTT
4981 LYIPRTADKT FFFQVICTTD EGSVKTPLIQ YDTSTGLGLV LTTPGKKKGS RSKSTEFYSE
5041 LWFIVLMAML GLILLAIFLS LILQRKIHKE PYIRERPPLV PLQKRMSPLN VYPPGENHMG
5101 LADTKIPRSG TPVSIRSNRS ACVLRIPSQN QTSLTYSQGS LHRSVSQLMD IQDKKVLMDN
5161 SLWEAIMGHN SGLYVDEEDL MNAIKDFSSV TKERTTFTDT HL
SEQ ID NO:291
   USHERIN (USH2A), TYPE IIA
   AAC23748.2 
1 
61 
121 
181 
241 
301 
361 
421 
481 
541 
601 
661 
721 
781 
841 
901 
961 
1021 
1081 
1141 
1201 
 
1261 
1321 
1381 
1441 
1501 
SEQ ID NO:292
   CLARIN 1 (CLRN1)
   AAH74971.1 
1 MPSQQKKIIF CMAGVFSFAC ALGVVTALGT PLWIKATVLC KTGALLVNAS GQELDKFMGE
61 MQYGLFHGEG VRQCGLGARP FRFSFFPDLL KAIPVSIHVN VILFSAILIV LTMVGTAFFM
121 YNAFGKPFET LHGPLGLYLL SFISGSCGCL VMILFASEVK IHHLSEKIAN YKEGTYVYKT
181 QSEKYTTSFW VIFFCFFVHF LNGLLIRLAG FQFPFAKSKD AETTNVAADL MY
SEQ ID NO:293
   CLARIN 1 (CLRN1), ISOFORM A
   NP_777367.1 
1 MPSQQKKIIF CMAGVFSFAC ALGVVTALGT PLWIKATVLC KTGALLVNAS GQELDKFMGE
61 MQYGLFHGEG VRQCGLGARP FRFSFFPDLL KAIPVSIHVN VILFSAILIV LTMVGTAFFM
121 YNAFGKPFET LHGPLGLYLL SFISGSCGCL VMILFASEVK IHHLSEKIAN YKEGTYVYKT
181 QSEKYTTSFW VIFFCFFVHF LNGLLIRLAG FQFPFAKSKD AETTNVAADL MY
SEQ ID NO:294
   CLARIN 1 (CLRN1), ISOFORM D
   NP_001182723.1 
1 MPSQQKKIIF CMAGVFSFAC ALGVVTALGT PLWIKATVLC KTGALLVNAS GQELDKFMGE
61 MQYGLFHGEG VRQCGLGARP FRFSFFPDLL KAIPVSIHVN VILFSAILIV LTMVGTAFFM
121 YNAFGKPFET LHGPLGLYLL SFISVALWLP ATRHQAQGSC GCLVMILFAS EVKIHHLSEK
181 IANYKEGTYV YKTQSEKYTT SFWVIFFCFF VHFLNGLLIR LAGFQFPFAK SKDAETTNVA
241 ADLMY
SEQ ID NO:295
   CLARIN 1 (CLRN1), ISOFORM C
   NP_443721.1 
1 MQALQQQPVF PDLLKAIPVS IHVNVILFSA ILIVLTMVGT AFFMYNAFGK PFETLHGPLG
61 LYLLSFISGS CGCLVMILFA SEVKIHHLSE KIANYKEGTY VYKTQSEKYT TSFWLTKGHS
SEQ ID NO:296
   CLARIN 1 (CLRN1), ISOFORM E
   NP_001243748.1 
1 MPSQQKKIIF CMAGVFSFAC ALGVVTALGT PLWIKATVLC KTGALLVNAS GQELDKFMGE
61 MQYGLFHGEG VRQCGLGARP FRFSCYFLDP FMGLPTGVPH LLSLPCSTSC RREHTSERVQ
121 EPAGCFSAVR SKLHAGPAAA TSFSRFAQSN PSEHPRQCHS LLCHPYCVNH GGDSLLHVQC
181 FWKTF
SEQ ID NO:297
   ATP BINDING CASSETTE SUBFAMILY A MEMBER 4 (ABCA4)
   P78363.3 
1 
61 
121 
181 
241 
301 
361 
421 
481 
 
541 
601 
661 
721 
781 
841 
901 
961 
1021 
1081 
1141 
1201 
1261 
1321 
1381 
1441 
1501 
1561 
1621 
1681 
1741 
1801 
1861 
1921 
1981 
2041 
2101 
2161 
2221 
SEQ ID NO:298
   ATP BINDING CASSETTE SUBFAMILY A MEMBER 4 (ABCA4), ISOFORM CRA_A
   EAW73056.1 
1 
61 
121 
181 
241 
301 
361 
421 
481 
541 
601 
661 
721 
781 
841 
901 
961 
1021 
1081 
1141 
1201 
1261 
1321 
1381 
 
1441 
1501 
1561 
1621 
1681 
1741 
1801 
1861 
1921 
1981 
2041 
2101 
2161 
2221 
SEQ ID NO:299
   ATP BINDING CASSETTE SUBFAMILY A MEMBER 4 (ABCA4), ISOFORM CRA_B
   EAW73057.1 
1 
61 
121 
181 
241 
301 
361 
421 
481 
541 
601 
661 
721 
781 
841 
901 
961 
1021 
1081 
1141 
1201 
SEQ ID NO:300
   ATP BINDING CASSETTE SUBFAMILY A MEMBER 4 (ABCA4), ISOFORM CRA_C
   EAW73058.1 
1 
61 
121 
181 
241 
301 
361 
421 
481 
541 
601 
661 
721 
781 
 
841 
901 
961 
1021 
1081 
1141 
1201 
1261 
1321 
1381 
1441 
1501 
1561 
1621 
1681 
1741 
1801 
1861 
1921 
1981 
2041 
2101 
2161 
2221 
SEQ ID NO:301
   ELOVL FATTY ACID ELONGASE 4 (ELOVL4)
   NP_073563.1 
1 
61 
121 
181 
241 
301 
SEQ ID NO:302
   INTERLEUKIN 6 (IL6)
   AAC41704.1 
1 MNSFSTSAFG PVAFSLGLLL VLPAAFPAPV PPGEDSKDVA APHRQPLTSS ERIDKQIRYI
61 LDGISALRKE TCNKSNMCES SKEALAENNL NLPKMAEKDG CFQSGFNEET CLVKIITGLL
121 EFEVYLEYLQ NRFESSEEQA RAVQMSTKVL IQFLQKKAKN LDAITTPDPT TNASLLTKLQ
181 AQNQWLQDMT THLILRSFKE FLQSSLRALR QM
SEQ ID NO:303
   TNF-ALPHA (TNF)
   CAA26669.1 
  1 MSTESMIRDV ELAEEALPKK TGGPQGSRRC LFLSLFSFLI VAGATTLFCL LHFGVIGPQR
 61 EEFPRDLSLI SPLAQAVRSS SRTPSDKPVA HVVANPQAEG QLQWLNRRAN ALLANGVELR
121 DNQLVVPSEG LYLIYSQVLF KGQGCPSTHV LLTHTISRIA VSYQTKVNLL SAIKSPCQRE
181 TPEGAEAKPW YEPIYLGGVF QLEKGDRLSA EINRPDYLDF AESGQVYFGI IAL
SEQ ID NO:304
   L OPSIN (OPN1LW)
   NP_064445.2 
1 
61 
121 
 
181 
241 
301 
361 
SEQ ID NO:305
   M OPSIN (OPN1MW)
   NP_000504.1 
1 MAQQWSLQRL AGRHPQDSYE DSTQSSIFTY TNSNSTRGPF EGPNYHIAPR WVYHLTSVWM
61 IFVVIASVFT NGLVLAATMK FKKLRHPLNW ILVNLAVADL AETVIASTIS VVNQVYGYFV
121 LGHPMCVLEG YTVSLCGITG LWSLAIISWE RWMVVCKPFG NVRFDAKLAI VGIAFSWIWA
181 AVWTAPPIFG WSRYWPHGLK TSCGPDVFSG SSYPGVQSYM IVLMVTCCIT PLSIIVLCYL
241 QVWLAIRAVA KQQKESESTQ KAEKEVTRMV VVMVLAFCFC WGPYAFFACF AAANPGYPFH
301 PLMAALPAFF AKSATIYNPV IYVFMNRQFR NCILQLFGKK VDDGSELSSA SKTEVSSVSS
361 VSPA
SEQ ID NO:306
   GUANYLATE CYCLASE 2D, RETINAL (GUCY2D)
   Q02846.2 
1 
61 
121 
181 
241 
301 
361 
421 
481 
541 
601 
661 
721 
781 
841 
901 
961 
1021 
1081 
SEQ ID NO:307
   RETINOID ISOMEROHYDROLASE RPE65 (RPE65)
   NP_000320.1 
1 
61 
121 
181 
241 
301 
361 
421 
481 
SEQ ID NO:308
   RETINOID ISOMEROHYDROLASE RPE65 (RPE65), ISOFORM X1
   XP_016857516.1 
1 MTEKRIVITE FGTCAFPDPC KNIFSRFFSY FRGVEVTDNA LVNVYPVGED YYACTETNFI
61 TKINPETLET IKQVDLCNYV SVNGATAHPH IENDGTVYNI GNCFGKNFSI AYNIVKIPPL
 
121 QADKEDPISK SEIVVQFPCS DRFKPSYVHS FGLTPNYIVF VETPVKINLF KFLSSWSLWG
181 ANYMDCFESN ETMGVWLHIA DKKRKKYLNN KYRTSPFNLF HHINTYEDNG FLIVDLCCWK
241 GFEFVYNYLY LANLRENWEE VKKNARKAPQ PEVRRYVLPL NIDKADTGKN LVTLPNTTAT
301 AILCSDETIW LEPEVLFSGP RQAFEFPQIN YQKYCGKPYT YAYGLGLNHF VPDRLCKLNV
361 KTKETWVWQE PDSYPSEPIF VSHPDALEED DGVVLSVVVS PGAGQKPAYL LILNAKDLSE
421 VARAEVEINI PVTFHGLFKK S
SEQ ID NO:309
   ARYL HYDROCARBON RECEPTOR INTERACTING PROTEIN LIKE 1 (AIPL1)
   CAH25996.1 
1 MDAALLLNVE GVKKTILHGG TGELPNFITG SRVIFHFRTM KCDEERTVID DSRQVGQPMH
61 IIIGNMFKLE VWEILLTSMR VHEVAEFWCD TIHTGVYPIL SRSLRQMAQG KDPTEWHVHT
121 CGLANMFAYH TLGYEDLDEL QKEPQPLVFV IELLQVDAPS DYQRETWNLS NHEKMKAVPV
181 LHGEGNRLFK LGRYEEASSK YQEAIICLRN LQTKCLLKKE EYYEVLEHTS DILRHHPGIV
241 KAYYVRARAH AEVWNEAEAK ADLQKVLELE PSMQKAVRRE LRLLENRMAE KQEEERLRCR
301 NMLSQGATQP PAEPPTEPPA QSSTEPPAEP PTAPSAELSA GPPAEPATEP PPSPGHSLQH
SEQ ID NO:310
   ARYL HYDROCARBON RECEPTOR INTERACTING PROTEIN LIKE 1 (AIPL1)
   CAH25995.1 
1 MDAALLLNVE GVKKTILHGG TGELPNFITG SRVGQPMHII IGNMFKLEVW EILLTSMRVH
61 EVAEFWCDTI HTGVYPILSR SLRQMAQGKD PTEWHVHTCG LANMFAYHTL GYEDLDELQK
121 EPQPLVFVIE LLQVDAPSDY QRETWNLSNH EKMKAVPVLH GEGNRLFKLG RYEEASSKYQ
181 EAIICLRNLQ TKEKPWEVQW LKLEKMINTL ILNYCQCLLK KEEYYEVLEH TSDILRHHPG
241 IVKAYYVRAR AHAEVWNEAE AKADLQKVLE LEPSMQKAVR RELRLLENRM AEKQEEERLR
301 CRNMLSQGAT QPPAEPPTEP PAQSSTEPPA EPPTAPSAEL SAGPPAEPAT EPPPSPGHSL
361 QH
SEQ ID NO:311
   ARYL HYDROCARBON RECEPTOR INTERACTING PROTEIN LIKE 1 (AIPL1)
   CAG17883.1 
1 
61 
121 
181 
241 
301 
SEQ ID NO:312
   ARYL HYDROCARBON RECEPTOR INTERACTING PROTEIN LIKE 1 (AIPL1)
   CAG17882.1 
1 
61 
121 
181 
241 
301 
SEQ ID NO:313
   ARYL HYDROCARBON RECEPTOR INTERACTING PROTEIN LIKE 1 (AIPL1)
   AAH12055.1 
1 
61 
121 
181 
241 
 
301 
361 
SEQ ID NO:314
   C5B isoform 1
   NP_001726.2 
1 MGLLGILCFL IFLGKTWGQE QTYVISAPKI FRVGASENIV IQVYGYTEAF DATISIKSYP
61 DKKFSYSSGH VHLSSENKFQ NSAILTIQPK QLPGGQNPVS YVYLEVVSKH FSKSKRMPIT
121 YDNGFLFIHT DKPVYTPDQS VKVRVYSLND DLKPAKRETV LTFIDPEGSE VDMVEEIDHI
181 GIISFPDFKI PSNPRYGMWT IKAKYKEDFS TTGTAYFEVK EYVLPHFSVS IEPEYNFIGY
241 KNFKNFEITI KARYFYNKVV TEADVYITFG IREDLKDDQK EMMQTAMQNT MLINGIAQVT
301 FDSETAVKEL SYYSLEDLNN KYLYIAVTVI ESTGGFSEEA EIPGIKYVLS PYKLNLVATP
361 LFLKPGIPYP IKVQVKDSLD QLVGGVPVTL NAQTIDVNQE TSDLDPSKSV TRVDDGVASF
421 VLNLPSGVTV LEFNVKTDAP DLPEENQARE GYRAIAYSSL SQSYLYIDWT DNHKALLVGE
481 HLNIIVTPKS PYIDKITHYN YLILSKGKII HFGTREKFSD ASYQSINIPV TQNMVPSSRL
541 LVYYIVTGEQ TAELVSDSVW LNIEEKCGNQ LQVHLSPDAD AYSPGQTVSL NMATGMDSWV
601 ALAAVDSAVY GVQRGAKKPL ERVFQFLEKS DLGCGAGGGL NNANVFHLAG LTFLTNANAD
661 DSQENDEPCK EILRPRRTLQ KKIEEIAAKY KHSVVKKCCY DGACVNNDET CEQRAARISL
721 GPRCIKAFTE CCVVASQLRA NISHKDMQLG RLHMKTLLPV SKPEIRSYFP ESWLWEVHLV
781 PRRKQLQFAL PDSLTTWEIQ GVGISNTGIC VADTVKAKVF KDVFLEMNIP YSVVRGEQIQ
841 LKGTVYNYRT SGMQFCVKMS AVEGICTSES PVIDHQGTKS SKCVRQKVEG SSSHLVTFTV
901 LPLEIGLHNI NFSLETWFGK EILVKTLRVV PEGVKRESYS GVTLDPRGIY GTISRRKEFP
961 YRIPLDLVPK TEIKRILSVK GLLVGEILSA VLSQEGINIL THLPKGSAEA ELMSVVPVFY
1021 VFHYLETGNH WNIFHSDPLI EKQKLKKKLK EGMLSIMSYR NADYSYSVWK GGSASTWLTA
1081 FALRVLGQVN KYVEQNQNSI CNSLLWLVEN YQLDNGSFKE NSQYQPIKLQ GTLPVEAREN
1141 SLYLTAFTVI GIRKAFDICP LVKIDTALIK ADNFLLENTL PAQSTFTLAI SAYALSLGDK
1201 THPQFRSIVS ALKREALVKG NPPIYRFWKD NLQHKDSSVP NTGTARMVET TAYALLTSLN
1261 LKDINYVNPV IKWLSEEQRY GGGFYSTQDT INAIEGLTEY SLLVKQLRLS MDIDVSYKHK
1321 GALHNYKMTD KNFLGRPVEV LLNDDLIVST GFGSGLATVH VTTVVHKTST SEEVCSFYLK
1381 IDTQDIEASH YRGYGNSDYK RIVACASYKP SREESSSGSS HAVMDISLPT GISANEEDLK
1441 ALVEGVDQLF TDYQIKDGHV ILQLNSIPSS DFLCVRFRIF ELFEVGFLSP ATFTVYEYHR
1501 PDKQCTMFYS TSNIKIQKVC EGAACKCVEA DCGQMQEELD LTISAETRKQ TACKPEIAYA
1561 YKVSITSITV ENVFVKYKAT LLDIYKTGEA VAEKDSEITF IKKVTCTNAE LVKGRQYLIM
1621 GKEALQIKYN FSFRYIYPLD SLTWIEYWPR DTTCSSCQAF LANLDEFAED IFLNGC
SEQ ID NO:315
   C5B, isoform 2
   NP_001304092.1 
1 MPGSLGREAS GRAGPTGCGA FAFGLRCRYV ISAPKIFRVG ASENIVIQVY GYTEAFDATI
61 SIKSYPDKKF SYSSGHVHLS SENKFQNSAI LTIQPKQLPG GQNPVSYVYL EVVSKHFSKS
121 KRMPITYDNG FLFIHTDKPV YTPDQSVKVR VYSLNDDLKP AKRETVLTFI DPEGSEVDMV
181 EEIDHIGIIS FPDFKIPSNP RYGMWTIKAK YKEDFSTTGT AYFEVKEYVL PHFSVSIEPE
241 YNFIGYKNFK NFEITIKARY FYNKVVTEAD VYITFGIRED LKDDQKEMMQ TAMQNTMLIN
301 GIAQVTFDSE TAVKELSYYS LEDLNNKYLY IAVTVIESTG GFSEEAEIPG IKYVLSPYKL
361 NLVATPLFLK PGIPYPIKVQ VKDSLDQLVG GVPVTLNAQT IDVNQETSDL DPSKSVTRVD
421 DGVASFVLNL PSGVTVLEFN VKTDAPDLPE ENQAREGYRA IAYSSLSQSY LYIDWTDNHK
481 ALLVGEHLNI IVTPKSPYID KITHYNYLIL SKGKIIHFGT REKFSDASYQ SINIPVTQNM
541 VPSSRLLVYY IVTGEQTAEL VSDSVWLNIE EKCGNQLQVH LSPDADAYSP GQTVSLNMAT
601 GMDSWVALAA VDSAVYGVQR GAKKPLERVF QFLEKSDLGC GAGGGLNNAN VFHLAGLTFL
661 TNANADDSQE NDEPCKEILR PRRTLQKKIE EIAAKYKHSV VKKCCYDGAC VNNDETCEQR
721 AARISLGPRC IKAFTECCVV ASQLRANISH KDMQLGRLHM KTLLPVSKPE IRSYFPESWL
781 WEVHLVPRRK QLQFALPDSL TTWEIQGVGI SNTGICVADT VKAKVFKDVF LEMNIPYSVV
841 RGEQIQLKGT VYNYRTSGMQ FCVKMSAVEG ICTSESPVID HQGTKSSKCV RQKVEGSSSH
901 LVTFTVLPLE IGLHNINFSL ETWFGKEILV KTLRVVPEGV KRESYSGVTL DPRGIYGTIS
961 RRKEFPYRIP LDLVPKTEIK RILSVKGLLV GEILSAVLSQ EGINILTHLP KGSAEAELMS
1021 VVPVFYVFHY LETGNHWNIF HSDPLIEKQK LKKKLKEGML SIMSYRNADY SYSVWKGGSA
1081 STWLTAFALR VLGQVNKYVE QNQNSICNSL LWLVENYQLD NGSFKENSQY QPIKLQGTLP
 
1141 VEARENSLYL TAFTVIGIRK AFDICPLVKI DTALIKADNF LLENTLPAQS TFTLAISAYA
1201 LSLGDKTHPQ FRSIVSALKR EALVKGNPPI YRFWKDNLQH KDSSVPNTGT ARMVETTAYA
1261 LLTSLNLKDI NYVNPVIKWL SEEQRYGGGF YSTQDTINAI EGLTEYSLLV KQLRLSMDID
1321 VSYKHKGALH NYKMTDKNFL GRPVEVLLND DLIVSTGFGS GLATVHVTTV VHKTSTSEEV
1381 CSFYLKIDTQ DIEASHYRGY GNSDYKRIVA CASYKPSREE SSSGSSHAVM DISLPTGISA
1441 NEEDLKALVE GVDQLFTDYQ IKDGHVILQL NSIPSSDFLC VRFRIFELFE VGFLSPATFT
1501 VYEYHRPDKQ CTMFYSTSNI KIQKVCEGAA CKCVEADCGQ MQEELDLTIS AETRKQTACK
1561 PEIAYAYKVS ITSITVENVF VKYKATLLDI YKTGEAVAEK DSEITFIKKV TCTNAELVKG
1621 RQYLIMGKEA LQIKYNFSFR YIYPLDSLTW IEYWPRDTTC SSCQAFLANL DEFAEDIFLN
1681 GC
SEQ ID NO:316
   C5B, isoform 3
   NP_001304093.1 
1 MGLLGILCFL IFLGKTWGQE QTYVISAPKI FRVGASENIV IQVYGYTEAF DATISIKSYP
61 DKKFSYSSGH VHLSSENKFQ NSAILTIQPK QLPGGQNPVS YVYLEVVSKH FSKSKRMPIT
121 YDNGFLFIHT DKPVYTPDQS VKVRVYSLND DLKPAKRETV LTFIDPEGSE VDMVEEIDHI
181 GIISFPDFKI PSNPRYGMWT IKAKYKEDFS TTGTAYFEVK EYVLPHFSVS IEPEYNFIGY
241 KNFKNFEITI KARYFYNKVV TEADVYITFG IREDLKDDQK EMMQTAMQNT MLINGIAQVT
301 FDSETAVKEL SYYSLEDLNN KYLYIAVTVI ESTGGFSEEA EIPGIKYVLS PYKLNLVATP
361 LFLKPGIPYP IKVQVKDSLD QLVGGVPVTL NAQTIDVNQE TSDLDPSKSV TRVDDGVASF
421 VLNLPSGVTV LEFNVKTDAP DLPEENQARE GYRAIAYSSL SQSYLYIDWT DNHKALLVGE
481 HLNIIVTPKS PYIDKITHYN YLILSKGKII HFGTREKFSD ASYQSINIPV TQNMVPSSRL
541 LVYYIVTGEQ TAELVSDSVW LNIEEKCGNQ LQVHLSPDAD AYSPGQTVSL NMATGMDSWV
601 ALAAVDSAVY GVQRGAKKPL ERVFQFLEKS DLGCGAGGGL NNANVFHLAG LTFLTNANAD
661 DSQENDEPCK EILRPRRTLQ KKIEEIAAKY KHSVVKKCCY DGACVNNDET CEQRAARISL
721 GPRCIKAFTE CCVVASQLRA NISHKDMQLG RLHMKTLLPV SKPEIRSYFP ESWLWEVHLV
781 PRRKQLQFAL PDSLTTWEIQ GVGISNTGIC VADTVKAKVF KDVFLEMNIP YSVVRGEQIQ
841 LKGTVYNYRT SGMQSLALSP RLECNGKISG HCKLRLPGSS DSPASASQVA GITGTHHHAQ
901 PT
SEQ ID NO:317
   C6
   NP_001108603.2 
1 MARRSVLYFI LLNALINKGQ ACFCDHYAWT QWTSCSKTCN SGTQSRHRQI VVDKYYQENF
61 CEQICSKQET RECNWQRCPI NCLLGDFGPW SDCDPCIEKQ SKVRSVLRPS QFGGQPCTAP
121 LVAFQPCIPS KLCKIEEADC KNKFRCDSGR CIARKLECNG ENDCGDNSDE RDCGRTKAVC
181 TRKYNPIPSV QLMGNGFHFL AGEPRGEVLD NSFTGGICKT VKSSRTSNPY RVPANLENVG
241 FEVQTAEDDL KTDFYKDLTS LGHNENQQGS FSSQGGSSFS VPIFYSSKRS ENINHNSAFK
301 QAIQASHKKD SSFIRIHKVM KVLNFTTKAK DLHLSDVFLK ALNHLPLEYN SALYSRIFDD
361 FGTHYFTSGS LGGVYDLLYQ FSSEELKNSG LTEEEAKHCV RIETKKRVLF AKKTKVEHRC
421 TTNKLSEKHE GSFIQGAEKS ISLIRGGRSE YGAALAWEKG SSGLEEKTFS EWLESVKENP
481 AVIDFELAPI VDLVRNIPCA VTKRNNLRKA LQEYAAKFDP CQCAPCPNNG RPTLSGTECL
541 CVCQSGTYGE NCEKQSPDYK SNAVDGQWGC WSSWSTCDAT YKRSRTRECN NPAPQRGGKR
601 CEGEKRQEED CTFSIMENNG QPCINDDEEM KEVDLPEIEA DSGCPQPVPP ENGFIRNEKQ
661 LYLVGEDVEI SCLTGFETVG YQYFRCLPDG TWRQGDVECQ RTECIKPVVQ EVLTITPFQR
721 LYRIGESIEL TCPKGFVVAG PSRYTCQGNS WTPPISNSLT CEKDTLTKLK GHCQLGQKQS
781 GSECICMSPE EDCSHHSEDL CVFDTDSNDY FTSPACKFLA EKCLNNQQLH FLHIGSCQDG
841 RQLEWGLERT RLSSNSTKKE SCGYDTCYDW EKCSASTSKC VCLLPPQCFK GGNQLYCVKM
901 GSSTSEKTLN ICEVGTIRCA NRKMEILHPG KCLA
SEQ ID NO:318
   C7
   NP_000578.2 
  1 
 61 
121 
 
181 
241 
301 
361 
421 
481 
541 
601 
661 
721 
781 
841 
SEQ ID NO:319
   C8 ALPHA SUBUNIT NP_000553.1 
1 MFAVVFFILS LMTCQPGVTA QEKVNQRVRR AATPAAVTCQ LSNWSEWTDC FPCQDKKYRH
61 RSLLQPNKFG GTICSGDIWD QASCSSSTTC VRQAQCGQDF QCKETGRCLK RHLVCNGDQD
121 CLDGSDEDDC EDVRAIDEDC SQYEPIPGSQ KAALGYNILT QEDAQSVYDA SYYGGQCETV
181 YNGEWRELRY DSTCERLYYG DDEKYFRKPY NFLKYHFEAL ADTGISSEFY DNANDLLSKV
241 KKDKSDSFGV TIGIGPAGSP LLVGVGVSHS QDTSFLNELN KYNEKKFIFT RIFTKVQTAH
301 FKMRKDDIML DEGMLQSLME LPDQYNYGMY AKFINDYGTH YITSGSMGGI YEYILVIDKA
361 KMESLGITSR DITTCFGGSL GIQYEDKINV GGGLSGDHCK KFGGGKTERA RKAMAVEDII
421 SRVRGGSSGW SGGLAQNRST ITYRSWGRSL KYNPVVIDFE MQPIHEVLRH TSLGPLEAKR
481 QNLRRALDQY LMEFNACRCG PCFNNGVPIL EGTSCRCQCR LGSLGAACEQ TQTEGAKADG
541 SWSCWSSWSV CRAGIQERRR ECDNPAPQNG GASCPGRKVQ TQAC
SEQ ID NO:320
   C8 BETA SUBUNIT
   AAA51862.1 
1 MKNSRTWAWR APVELFLLCA ALGCLSLPGS RGERPHSFGS NAVNKSFAKS RQMRSVDVTL
61 MPIDCELSSW SSWTTCDPCQ KKRYRYAYLL QPSQFHGEPC NFSDKEVEDC VTNRPCGSQV
121 RCEGFVCAQT GRCVNRRLLC NGDNDCGDQS DEANCRRIYK KCQHEMDQYW GIGSLASGIN
181 LFTNSFEGPV LDHRYYAGGC SPHYILNTRF RKPYNVESYT PQTQGKYEFI LKEYESYSDF
241 ERNVTEKMAS KSGFSFGFKI PGIFELGISS QSDRGKHYIR RTKRFSHTKS VFLHARSDLE
301 VAHYKLKPRS LMLHYEFLQR VKRLPLEYSY GEYRDLFRDF GTHYITEAVL GGIYEYTLVM
361 NKEAMERGDY TLNNVHACAK NDFKIGGAIE EVYVSLGVSV GKCRGILNEI KDRNKRDTMV
421 EDLVVLVRGG ASEHITTLAY QELPTADLMQ EWGDAVQYNP AIIKVKVEPL YELVTATDFA
481 YSSTVRQNMK QALEEFQKEV SSCHCAPCQG NGVPVLKGSR CDCICPVGSQ GLACEVSYRK
541 NTPIDGKWNC WSNWSSCSGR RKTRQRQCNN PPPQNGGSPC SGPASETLDC S
SEQ ID NO:321
   C8 GAMMA SUBUNIT
   AAA51888.1

  1 MLPPGTATLL TLLLAAGSLG QKPQRPRRPA SPISTIQPKA NFDAQQFAGT WLLVAVGSAC
 61 RFLQEQGHRA EATTLHVAPQ GTAMAVSTFR KLDGICWQVR QLYGDTGVLG RFLLQARGAR
121 GAVHVVVAET DYQSFAVLYL ERAGQLSVKL YARSLPVSDS VLSGFEQRVQ EAHLTEDQIF
181 YFPKYGFCEA ADQFHVLDEV RR

### 8. EXAMPLES

### 8.1 EXAMPLE 1: Bevacizumab Fab cDNA-Based Vector

A bevacizumab Fab cDNA-based vector is constructed comprising a transgene comprising bevacizumab Fab portion of the light and heavy chain cDNA sequences (SEQ ID NOs. 10 and 11, respectively). The transgene also comprises nucleic acids comprising a signal peptide chosen from the group listed in Table 1. The nucleotide sequences encoding the light chain and heavy chain are separated by IRES elements or 2A cleavage sites to create a bicistronic vector. Optionally, the vector additionally comprises a hypoxia-inducible promoter.

### 8.2 EXAMPLE 2: Ranibizumab cDNA-Based Vector

A ranibizumab Fab cDNA-based vector is constructed comprising a transgene comprising ranibizumab Fab light and heavy chain cDNAs (the portions of SEQ ID NOs.12 and 13, respectively not encoding the signal peptide). The transgene also comprises nucleic acids comprising a signal peptide chosen from the group listed in Table 1. The nucleotide sequences encoding the light chain and heavy chain are separated by IRES elements or 2A cleavage sites to create a bicistronic vector. Optionally, the vector additionally comprises a hypoxia-inducible promoter.

### 8.3 EXAMPLE 3: Hyperglycosylated Bevacizumab Fab cDNA-Based Vector

A hyperglycosylated bevacizumab Fab cDNA-based vector is constructed comprising a transgene comprising bevacizumab Fab portion of the light and heavy chain cDNA sequences (SEQ ID NOs. 10 and 11, respectively) with mutations to the sequence encoding one or more of the following mutations: L118N (heavy chain), E195N (light chain), or Q160N or Q160S (light chain). The transgene also comprises nucleic acids comprising a signal peptide chosen from the group listed in Table 1. The nucleotide sequences encoding the light chain and heavy chain are separated by IRES elements or 2A cleavage sites to create a bicistronic vector. Optionally, the vector additionally comprises a hypoxia-inducible promoter.

### 8.4 EXAMPLE 4: Hyperglycosylated Ranibizumab cDNA-based vector

A hyperglycosylated ranibizumab Fab cDNA-based vector is constructed comprising a transgene comprising ranibizumab Fab light and heavy chain cDNAs (the portions of SEQ ID NOs.12 and 13, respectively not encoding the signal peptide), with mutations to the sequence encoding one or more of the following mutations: L118N (heavy chain), E195N (light chain), or Q160N or Q160S (light chain). The transgene also comprises nucleic acids comprising a signal peptide chosen from the group listed in Table 1. The nucleotide sequences encoding the light chain and heavy chain are separated by IRES elements or 2A cleavage sites to create a bicistronic vector. Optionally, the vector additionally comprises a hypoxia-inducible promoter.

### 8.5 EXAMPLE 5: Ranibizumab Based HuGlyFabVEGFi

A ranibizumab Fab cDNA-based vector (see Example 2) is expressed in the PER.C6^{®} Cell Line (Lonza) in the AAV8 background. The resultant product, ranibizumab-based HuGlyFabVEGFi is determined to be stably produced. N-glycosylation of the HuGlyFabVEGFi is confirmed by hydrazinolysis and MS/MS analysis. *See, e.g.,* Bondt *et al.,* Mol. & Cell. Proteomics 13.11:3029-3039. Based on glycan analysis, HuGlyFabVEGFi is confirmed to be N-glycosylated, with 2,6 sialic acid a predominant modification. Advantageous properties of the N-glycosylated HuGlyFabVEGFi are determined using methods known in the art. The HuGlyFabVEGFi can be found to have increased stability and increased affinity for its antigen (VEGF). See Sola and Griebenow, 2009, J Pharm Sci., 98(4): 1223-1245 for methods of assessing stability and Wright et al., 1991, EMBO J. 10:2717-2723 and Leibiger et al., 1999, Biochem. J. 338:529-538 for methods of assessing affinity.

### 8.6 EXAMPLE 6: Treatment of Wet AMD with Ranibizumab Based HuGlyFabVEGFi by Peripheral Injection

Based on determination of advantageous characteristics of ranibizumab-based HuGlyFabVEGFi (see Example 5), a ranibizumab Fab cDNA-based vector is deemed useful for treatment of wet AMD when expressed as a transgene. A subject presenting with wet AMD is administered AAV8 that encodes ranibizumab Fab at a dose sufficient to produce a concentration of the transgene product at a Cmin of at least 0.330 µg/mL in the Vitreous humour for three months. The administration is done by subretinal administration via peripheral injection into the retina (*i.e.,* peripheral to the optic disc, fovea and macula located in the back of the eye), which is accomplished by transvitreal injection. Following treatment, the subject is evaluated for improvement in symptoms of wet AMD.

### 8.7 EXAMPLE 7: Palmitoyl-Protein Thioesterase 1 cDNA-Based Vector

A Palmitoyl-Protein Thioesterase 1 (PPT1) cDNA-based vector is constructed comprising a transgene comprising the nucleotide sequences corresponding to the amino acid sequence of SEQ ID NO. 273. Optionally, the vector additionally comprises a hypoxia-inducible promoter.

### 8.8 EXAMPLE 8: Treatment of Batten-CLN1-Associated Vision Loss with Palmitoyl-Protein Thioesterase 1 cDNA-Based Vector by Peripheral Injection

A subject presenting with Batten-CLN1-associated vision loss is administered AAV8 or AAV9 that encodes Palmitoyl-Protein Thioesterase 1 at a dose sufficient to produce a therapeutically effective concentration of the transgene product in the vitreous humour for three months. The administration is done by subretinal administration via peripheral injection into the retina (*i.e.,* peripheral to the optic disc, fovea and macula located in the back of the eye), which is accomplished by transvitreal injection. Following treatment, the subject is evaluated for improvement in Batten-CLN1-associated vision loss.

Effects of the methods provided herein on visual deficits are measured by one or more visual acuity screenings, including OptoKinetic Nystagmus (OKN). OKN visual acuity screening uses the principles of the OKN involuntary reflex to objectively assess whether a patient's eyes can follow a moving target. By using OKN, no verbal communication is needed between the tester and the patient. As such, OKN is used to measure visual acuity in pre-verbal and/or non-verbal patients, including patients that are 1 month old, 2 months old, 3 months old, 4 months old, 5 months old, 6 months old, 7 months old, 8 months old, 9 months old, 10 months old, 11 months old, 1 year old, 1.5 years old, 2 years old, 2.5 years old, 3 years old, 3.5 years old, 4 years old, 4.5 years old, or 5 years old. The percentage change in OKN screening results before and after the said treatment is calculated.

### 8.9 EXAMPLE 9: Treatment of Batten-CLN1-Associated Vision Loss with Palmitoyl-Protein Thioesterase 1 cDNA-Based Vector by Suprachoroidal Injection

A subject presenting with Batten-CLN1-associated vision loss is administered AAV8 or AAV9 that encodes Palmitoyl-Protein Thioesterase 1 at a dose sufficient to produce a therapeutically effective concentration of the transgene product in the vitreous humour for three months. The administration is done by administration to the suprachoroidal space. Following treatment, the subject is evaluated for improvement in Batten-CLN1-associated vision loss.

Effects of the methods provided herein on visual deficits are measured by one or more visual acuity screenings, including OptoKinetic Nystagmus (OKN). OKN visual acuity screening uses the principles of the OKN involuntary reflex to objectively assess whether a patient's eyes can follow a moving target. By using OKN, no verbal communication is needed between the tester and the patient. As such, OKN is used to measure visual acuity in pre-verbal and/or non-verbal patients, including patients that are 1 month old, 2 months old, 3 months old, 4 months old, 5 months old, 6 months old, 7 months old, 8 months old, 9 months old, 10 months old, 11 months old, 1 year old, 1.5 years old, 2 years old, 2.5 years old, 3 years old, 3.5 years old, 4 years old, 4.5 years old, or 5 years old. The percentage change in OKN screening results before and after the said treatment is calculated.

### 8.10 EXAMPLE 10: Treatment of Batten-CLN1-Associated Vision Loss with Palmitoyl-Protein Thioesterase 1 cDNA-Based Vector by Subretinal Injection via Vitrectomy

A subject presenting with Batten-CLN1-associated vision loss is administered AAV8 or AAV9 that encodes Palmitoyl-Protein Thioesterase 1 at a dose sufficient to produce a therapeutically effective concentration of the transgene product in the vitreous humour for three months. The administration is done by administration to the subretinal space via vitrectomy. Following treatment, the subject is evaluated for improvement in Batten-CLN1-associated vision loss.

Effects of the methods provided herein on visual deficits are measured by one or more visual acuity screenings, including OptoKinetic Nystagmus (OKN). OKN visual acuity screening uses the principles of the OKN involuntary reflex to objectively assess whether a patient's eyes can follow a moving target. By using OKN, no verbal communication is needed between the tester and the patient. As such, OKN is used to measure visual acuity in pre-verbal and/or non-verbal patients, including patients that are 1 month old, 2 months old, 3 months old, 4 months old, 5 months old, 6 months old, 7 months old, 8 months old, 9 months old, 10 months old, 11 months old, 1 year old, 1.5 years old, 2 years old, 2.5 years old, 3 years old, 3.5 years old, 4 years old, 4.5 years old, or 5 years old. The percentage change in OKN screening results before and after the said treatment is calculated.

### 8.11 EXAMPLE 11: Treatment of Batten-CLN1-Associated Vision Loss with Palmitoyl-Protein Thioesterase 1 cDNA-Based Vector by Subretinal Administrate via the Suprachoroidal Space

A subject presenting with Batten-CLN1-associated vision loss is administered AAV8 or AAV9 that encodes Palmitoyl-Protein Thioesterase 1 at a dose sufficient to produce a therapeutically effective concentration of the transgene product in the vitreous humour for three months. The administration is done by administration to the subretinal space via the suprachoroidal space. Following treatment, the subject is evaluated for improvement in Batten-CLN1-associated vision loss.

Effects of the methods provided herein on visual deficits are measured by one or more visual acuity screenings, including OptoKinetic Nystagmus (OKN). OKN visual acuity screening uses the principles of the OKN involuntary reflex to objectively assess whether a patient's eyes can follow a moving target. By using OKN, no verbal communication is needed between the tester and the patient. As such, OKN is used to measure visual acuity in pre-verbal and/or non-verbal patients, including patients that are 1 month old, 2 months old, 3 months old, 4 months old, 5 months old, 6 months old, 7 months old, 8 months old, 9 months old, 10 months old, 11 months old, 1 year old, 1.5 years old, 2 years old, 2.5 years old, 3 years old, 3.5 years old, 4 years old, 4.5 years old, or 5 years old. The percentage change in OKN screening results before and after the said treatment is calculated.

### 8.12 EXAMPLE 12: Tripeptidyl-Peptidase 1 (TPP1) cDNA-Based Vector

A Tripeptidyl-Peptidase 1 (TPP1) cDNA-based vector is constructed comprising a transgene comprising the nucleotide sequences corresponding to the amino acid sequence of SEQ ID NO. 274. Optionally, the vector additionally comprises a hypoxia-inducible promoter.

### 8.13 EXAMPLE 13: Treatment of Batten-CLN2-Associated Vision Loss with Tripeptidyl-Peptidase 1 (TPP1) cDNA-Based Vector by Peripheral Injection

A subject presenting with Batten-CLN2-associated vision loss is administered AAV8 or AAV9 that encodes Tripeptidyl-Peptidase 1 at a dose sufficient to produce a therapeutically effective concentration of the transgene product in the vitreous humour for three months. The administration is done by subretinal administration via peripheral injection into the retina (*i.e.,* peripheral to the optic disc, fovea and macula located in the back of the eye), which is accomplished by transvitreal injection. Following treatment, the subject is evaluated for improvement in Batten-CLN2-associated vision loss.

Effects of the methods provided herein on visual deficits are measured by one or more visual acuity screenings, including OptoKinetic Nystagmus (OKN). OKN visual acuity screening uses the principles of the OKN involuntary reflex to objectively assess whether a patient's eyes can follow a moving target. By using OKN, no verbal communication is needed between the tester and the patient. As such, OKN is used to measure visual acuity in pre-verbal and/or non-verbal patients, including patients that are 1 month old, 2 months old, 3 months old, 4 months old, 5 months old, 6 months old, 7 months old, 8 months old, 9 months old, 10 months old, 11 months old, 1 year old, 1.5 years old, 2 years old, 2.5 years old, 3 years old, 3.5 years old, 4 years old, 4.5 years old, or 5 years old. The percentage change in OKN screening results before and after the said treatment is calculated.

### 8.14 EXAMPLE 14: Treatment of Batten-CLN2-Associated Vision Loss with Tripeptidyl-Peptidase 1 (TPP1) cDNA-Based Vector by Suprachoroidal Injection

A subject presenting with Batten-CLN2-associated vision loss is administered AAV8 or AAV9 that encodes Tripeptidyl-Peptidase 1 at a dose sufficient to produce a therapeutically effective concentration of the transgene product in the vitreous humour for three months. The administration is done by administration to the suprachoroidal space. Following treatment, the subject is evaluated for improvement in Batten-CLN2-associated vision loss.

Effects of the methods provided herein on visual deficits are measured by one or more visual acuity screenings, including OptoKinetic Nystagmus (OKN). OKN visual acuity screening uses the principles of the OKN involuntary reflex to objectively assess whether a patient's eyes can follow a moving target. By using OKN, no verbal communication is needed between the tester and the patient. As such, OKN is used to measure visual acuity in pre-verbal and/or non-verbal patients, including patients that are 1 month old, 2 months old, 3 months old, 4 months old, 5 months old, 6 months old, 7 months old, 8 months old, 9 months old, 10 months old, 11 months old, 1 year old, 1.5 years old, 2 years old, 2.5 years old, 3 years old, 3.5 years old, 4 years old, 4.5 years old, or 5 years old. The percentage change in OKN screening results before and after the said treatment is calculated.

### 8.15 EXAMPLE 15: Treatment of Batten-CLN2-Associated Vision Loss with Tripeptidyl-Peptidase 1 cDNA-Based Vector by Subretinal Injection via Vitrectomy

A subject presenting with Batten-CLN2-associated vision loss is administered AAV8 or AAV9 that encodes Tripeptidyl-Peptidase 1 at a dose sufficient to produce a therapeutically effective concentration of the transgene product in the vitreous humour for three months. The administration is done by administration to the subretinal space via vitrectomy. Following treatment, the subject is evaluated for improvement in Batten-CLN2-associated vision loss.

Effects of the methods provided herein on visual deficits are measured by one or more visual acuity screenings, including OptoKinetic Nystagmus (OKN). OKN visual acuity screening uses the principles of the OKN involuntary reflex to objectively assess whether a patient's eyes can follow a moving target. By using OKN, no verbal communication is needed between the tester and the patient. As such, OKN is used to measure visual acuity in pre-verbal and/or non-verbal patients, including patients that are 1 month old, 2 months old, 3 months old, 4 months old, 5 months old, 6 months old, 7 months old, 8 months old, 9 months old, 10 months old, 11 months old, 1 year old, 1.5 years old, 2 years old, 2.5 years old, 3 years old, 3.5 years old, 4 years old, 4.5 years old, or 5 years old. The percentage change in OKN screening results before and after the said treatment is calculated.

### 8.16 EXAMPLE 16: Treatment of Batten-CLN2-Associated Vision Loss with Tripeptidyl-Peptidase 1 cDNA-Based Vector by Subretinal Administrate via the Suprachoroidal Space

A subject presenting with Batten-CLN2-associated vision loss is administered AAV8 or AAV9 that encodes Tripeptidyl-Peptidase 1 at a dose sufficient to produce a therapeutically effective concentration of the transgene product in the vitreous humour for three months. The administration is done by administration to the subretinal space via the suprachoroidal space. Following treatment, the subject is evaluated for improvement in Batten-CLN2-associated vision loss.

Effects of the methods provided herein on visual deficits are measured by one or more visual acuity screenings, including OptoKinetic Nystagmus (OKN). OKN visual acuity screening uses the principles of the OKN involuntary reflex to objectively assess whether a patient's eyes can follow a moving target. By using OKN, no verbal communication is needed between the tester and the patient. As such, OKN is used to measure visual acuity in pre-verbal and/or non-verbal patients, including patients that are 1 month old, 2 months old, 3 months old, 4 months old, 5 months old, 6 months old, 7 months old, 8 months old, 9 months old, 10 months old, 11 months old, 1 year old, 1.5 years old, 2 years old, 2.5 years old, 3 years old, 3.5 years old, 4 years old, 4.5 years old, or 5 years old. The percentage change in OKN screening results before and after the said treatment is calculated.

### 8.17 EXAMPLE 17: A Randomized, Partially Masked, Controlled, Phase 2b Clinical Study to Evaluate the Safety and Efficacy of Construct II Gene Therapy in Participants with nAMD

### 8.17.1 Synopsis

### Primary Objectives.

To evaluate mean change in best-corrected visual acuity (BCVA) for Construct II compared with ranibizumab at Week 50.

### Secondary Objectives.

To evaluate the safety and tolerability of Construct II through Week 102. To evaluate the effect of Construct II on BCVA. To evaluate the effect of Construct II on central retinal thickness (CRT) as measured by spectral domain-optical coherence tomography (SD-OCT). To assess the need for supplemental anti-vascular endothelial growth factor (VEGF) therapy in the Construct II treatment arms. To assess aqueous protein concentrations of Construct II. To evaluate the immunogenicity of Construct II.

### Exploratory Objectives.

To evaluate changes over time in the area of geographic atrophy and to assess, in participants with no evidence at baseline, the incidence of new areas of geographic atrophy. To assess the proportion of participants with no fluid on SD-OCT. To assess aqueous VEGF-A concentrations. To evaluate visual function and treatment satisfaction using patient reported outcome (PRO) questionnaires

### Study Design.

This phase 2b partially masked, randomized, multicenter study will include 3 periods: an Active Run-in Period (*i.e.,* screening), a Treatment Period, and an Extension Period. Participants who receive Construct II will be asked to participate in a long-term follow-up study after completion of or early discontinuation from the current study and will sign a separate informed consent for the follow-up study at that time.

The Active Run-in Period, which will last up to 10 weeks, will begin when the participant signs the informed consent form and will end once the participant has been evaluated for eligibility and has received 3 monthly intravitreal injections of ranibizumab 0.5 mg. The Treatment Period will last up to 12 months, beginning when the participant is randomized to study treatment and ending at Week 50. The Extension Period will last up to 12 months, beginning after Week 50 and ending at Week 102.

At Screening Visit 1 (Week -10), participants who meet the inclusion/exclusion criteria will enter the study and receive a 0.5-mg intravitreal injection of ranibizumab in the study eye. At Screening Visit 2 (Week -6), participants will receive a second 0.5-mg intravitreal injection of ranibizumab in the study eye. One week later, at Screening Visit 3 (Week -5), participants' anatomic response on SD-OCT will be evaluated against prespecified response criteria. Participants not meeting response criteria will be exited from the study. If participants meet all inclusion criteria, at Screening Visit 4 (Week -2), participants will be randomized. Any participants who withdraw or become ineligible for randomization during the Screening Period and have an adverse event (AE) associated with the intravitreal ranibizumab injections will be followed until the AE resolves (up to 30 days postinjection). Participants who are identified at Screening Visit 4 as being eligible will receive a third 0.5- mg intravitreal injection of ranibizumab in the study eye. Once the Central Reading Center (CRC) has verified the CRT, participants will be randomized (1:1:1) using an interactive response technology system to receive a single dose of Construct II (Dose 1), a single dose of Construct II (Dose 2), or monthly intravitreal ranibizumab 0.5 mg; Construct II will be administered by subretinal delivery. Participants will be stratified by baseline (Screening Visit 4) BCVA score (> 58 letters vs ≤ 58 letters) in the randomization.

Participants randomized to the Construct II treatment arms will undergo the surgical procedure on Day 1 followed by visits on Day 2 and Day 8 to assess postoperative safety. At Week 2, participants will receive intravitreal ranibizumab to supplement any anti-VEGF that may have been removed during the vitrectomy surgery and to provide anti-VEGF therapy coverage while potential production of the gene therapy mediated protein escalates. The participants will then be seen at monthly intervals, beginning with Week 6, during which supplemental intravitreal ranibizumab 0.5-mg therapy may be administered if needed, as determined by the fully masked CRC evaluation of the SD-OCT data and the fully masked visual acuity assessor's evaluation of BCVA. Note that the SD-OCT and BCVA results from the masked assessors, together with predefined retreatment criteria, will inform the investigator's decision to provide supplemental anti-VEGF therapy.

Participants randomized to the ranibizumab control arm will have their first postrandomization visit at Week 2 and will receive intravitreal ranibizumab 0.5 mg. Following the Week 2 visit, the participants will have monthly (~28 day) study visits during which they will receive an intravitreal injection of ranibizumab 0.5 mg.

At the Week 50 primary endpoint, participants in the ranibizumab control arm will be offered the opportunity to receive Construct II treatment if they still meet key inclusion/exclusion criteria. The treating physician will determine if the participant is eligible and a good candidate for the procedure. Qualified participants will then be administered the highest tolerated dose evaluated in this protocol. Participants in the ranibizumab control arm who switch to Construct II following Week 50 will follow the same visit schedule as the one started on Day 1 for participants originally randomized to receive Construct II. Those participants who either choose not to have treatment with Construct II or are ineligible for treatment with Construct II will be discontinued from the study.

Throughout the study, participants will be evaluated through the assessment of ocular and nonocular AEs including serious adverse events (SAEs) and adverse events of special interest (AESIs) (ocular inflammation deemed by the investigator to be unrelated to the surgical/study procedure and is graded as 2+ or greater on the ocular inflammation grading scales, ocular infections [including endophthalmitis], retinal tears or detachment, retinal thinning, and new arterial thromboembolic events [nonfatal stroke, nonfatal myocardial infarction, or vascular death (including deaths of unknown cause)]), as well as assessments of clinical laboratory testing (chemistry, hematology, coagulation, urinalysis), ocular examinations and imaging (BCVA, intraocular pressure, slit-lamp biomicroscopy, indirect ophthalmoscopy, fluorescein angiography [FA], fundus autofluorescence [FAF], and SD- OCT), and vital signs. Note that AEs will be collected at all study visits. Immunogenicity to the vector and transgene product (TP) of Construct II will also be assessed. Patient reported outcomes will be collected using the supplemented National Eye Institute Visual Functioning Questionnaire 25-item version (NEI-VFQ-25) (also comprises the Rasch-scored version, NEI-VFQ-28-R) and Macular Disease Treatment Satisfaction Questionnaire (MacTSQ).

Planned safety monitoring of the study participants will be conducted on an ongoing basis. These include reviews conducted by the partially masked Medical Monitor and routine reviews conducted by the partially masked Sponsor's Internal Safety Committee. Separately, an Independent Data Monitoring Committee (IDMC) will also be established and will meet on a periodic basis to independently review the clinical data. If unmasked reviews are needed to understand a potential safety signal, these reviews will be conducted by the IDMC.

### Diagnosis and Main Criteria for Inclusion.

To be eligible for enrollment in this study, participants, aged ≥ 50 and ≤ 89 years, must have a diagnosis of subfoveal choroidal neovascularization secondary to age-related macular degeneration in the study eye. Optical coherence tomography documentation from a current image of center subfield fluid must be confirmed by the CRC. Participants must have a BCVA letter score in the study eye between ≤ 78 and ≥ 44 and be pseudophakic (status postcataract surgery) in the study eye. Participants also must be willing and able to provide written, signed informed consent for this study after the nature of the study has been explained, and prior to any research-related procedures being conducted.

### Investigational Product, Dosage, and Mode of Administration.

Construct II Dose 1: 1.6 × 10¹¹ GC/eye (6.2 × 10¹¹ GC/mL). Construct II Dose 2: 2.5 × 10¹¹ GC/eye (1.0 × 10¹² GC/mL). Construct II is administered via subretinal delivery (250 µL in a single dose).

### Duration of Treatment.

In the Construct II treatment arms: 1 day. In the ranibizumab control arm: 50 weeks

### Reference Therapy, Dosage and Mode of Administration.

Ranibizumab (LUCENTIS^{®}, Genentech) 0.5 mg (0.05 mL of 10 mg/mL solution) will be administered by intravitreal injection approximately every 28 days.

Intravitreal ranibizumab 0.5 mg will also be administered as supplemental anti-VEGF therapy in all treatment arms during the Run-in Period (Screening Visits 1, 2, and 4) and at Week 2. Participants in the Construct II arm will be evaluated for intravitreal ranibizumab 0.5 mg as supplemental anti-VEGF therapy starting at Week 6 according to retreatment criteria; participants in the ranibizumab control arm who switch to Construct II after Week 50 will receive intravitreal ranibizumab 0.5 mg at Week 54 and will be evaluated for intravitreal ranibizumab 0.5 mg as supplemental anti-VEGF therapy starting at Week 58 according to retreatment criteria.

### Criteria for Evaluation.

### Primary Endpoint:

Mean change from baseline in BCVA to Week 50 (as the average of Week 46 and Week 50) based on the Early Treatment Diabetic Retinopathy Study (ETDRS) score

### Secondary Endpoints:

Incidences of ocular and nonocular AEs over 50 weeks.

Incidences of ocular and nonocular AEs over 102 weeks.

Mean change from baseline in BCVA to Week 102 (as the average of Week 98 and Week 102).

Proportion of participants with BCVA of 43 letters (20/160 approximate Snellen equivalent) or worse at Week 50 (as the average of Week 46 and Week 50) and Week 102 (as the average of Week 98 and Week 102).

Proportion of participants with BCVA of 84 letters (20/20 approximate Snellen equivalent) or better at Week 50 (as the average of Week 46 and Week 50) and Week 102 (as the average of Week 98 and Week 102).

Proportion of participants (1) gaining or losing ≥ 15, ≥ 10, ≥ 5, or ≥ 0 letters; (2) maintaining vision (not losing ≥ 15 letters) compared with baseline as per BCVA at Week 50 (as the average of Week 46 and Week 50) and Week 102 (as the average of Week 98 and Week 102).

Mean change from baseline in BCVA to Week 50 (as the average of Week 46 and Week 50) for participants who received ≤ 2 supplemental anti-VEGF injections, 2 supplemental anti-VEGF injections, 1 supplemental anti-VEGF injection, or 0 supplemental anti-VEGF injections (Construct II randomized participants).

Mean change from Week 50 to Week 102 (as the average of Week 98 and Week 102) in BCVA (control arm participants who switch to Construct II).

Mean change from baseline in CRT as measured by SD-OCT to Week 50 (as the average of Week 46 and Week 50) and Week 102 (as the average of Week 98 and Week 102).

Mean change from Week 50 to Week 102 (as the average of Week 98 and Week 102) in CRT as measured by SD-OCT (control arm participants who switch to Construct II).

Proportion of participants who have a reduction of ≥ 50% in supplemental anti-VEGF injection rate through Week 50 and Week 102 compared with the prior 50 weeks preceding the first intravitreal ranibizumab injection received as part of the Active Run-in Period (Construct II randomized participants).

Mean reduction in supplemental anti VEGF injection rate through Week 50 and Week 102 compared with the prior 50 weeks preceding the first ranibizumab injection received as part of the Active Run-in Period (Construct II randomized participants).

Mean number of supplemental anti-VEGF injections in the Construct II arms through Week 50 and Week 102; Mean number of supplemental anti-VEGF injections after Week 50 through Week 102 relative to the prior 50 weeks in the study (control arm participants who switch to Construct II).

Time to first supplemental anti-VEGF injection after the Week 2 injection in the Construct II arms.

Proportion of participants in the Construct II arms who receive supplemental anti-VEGF injection after Week 2 through Week 26, after Week 26 through Week 50, after Week 50 through Week 74, after Week 74 through Week 102, after Week 2 through Week 50, and after Week 2 through Week 10

Aqueous Construct II TP concentrations at assessed time points; Immunogenicity measurements (serum neutralizing antibodies to AAV8 and serum antibodies to Construct II TP) at assessed time points.

### Exploratory Endpoints:

Mean change from baseline in area of geographic atrophy based on FAF at assessed time points.

Incidence of new area of geographic atrophy by FAF (in participants with no geographic atrophy at baseline).

Incidence of retinal thinning in the area of the bleb.

Proportion of participants with no fluid on SD-OCT.

VEGF-A concentrations (aqueous) at assessed time points.

Mean change from baseline in NEI-VFQ-28-R (composite score; activity limitation domain score; and socio-emotional functioning domain score) at assessed time points.

Mean change from baseline in NEI-VFQ-25 (composite score and mental health subscale score) at assessed time points.

Mean change from baseline in MacTSQ (composite score; safety, efficacy, and discomfort domain score; and information provision and convenience domain score) at assessed time points.

**Table 3. Objectives and Endpoints**

| | **Objectives** | **Endpoints** |
|---|---|---|
| **Primary** | | |
| Efficacy | • To evaluate mean change in BCVA for Construct II compared with ranibizumab at Week 50 | • Mean change from baseline in BCVA to Week 50 (as the average of Week 46 and Week 50) based on the ETDRS score |

| **Secondary** | | |
|---|---|---|
| Safety | • To evaluate the safety and tolerability of Construct II through Week 102 | Incidences of ocular and nonocular AEs over 50 weeks |
| | | • Incidences of ocular and nonocular AEs over 102 weeks |
| Efficacy | • To evaluate the effect of Construct II on BCVA | • Mean change from baseline in BCVA to Week 102 (as the average of Week 98 and Week 102) |
| | | • Proportion of participants with BCVA of 43 letters (20/160 approximate Snellen equivalent) or worse at Week 50 (as the average of Week 46 and Week 50) and Week 102 (as the average of Week 98 and Week 102) |
| | | • Proportion of participants with BCVA of 84 letters (20/20 approximate Snellen equivalent) or better at Week 50 (as the average of Week 46 and Week 50) and Week 102 (as the average of Week 98 and Week 102) |
| | | • Proportion of participants (1) gaining or losing ≥ 15, ≥ 10, ≥ 5, or ≥ 0 letters: (2) maintaining vision (not losing ≥ 15 letters) compared with baseline as per BCVA at Week 50 (as the average of Week 46 and Week 50) and Week 102 (as the average of Week 98 and Week 102) |
| | | • Mean change from baseline in BCVA to Week 50 (as the average of Week 46 and Week 50) for participants who received ≤ 2 supplemental anti-VEGF injections, 2 supplemental anti-VEGF injections, 1 supplemental anti-VEGF injection, or 0 supplemental anti- VEGF injections (Construct II randomized participants) |
| | | • Mean change from Week 50 to Week 102 (as the average of Week 98 and Week 102) in BCVA (control arm participants who switch to Construct II) |
| Efficacy | • To evaluate the effect of Construct II on CRT as measured by SD-OCT | • Mean change from baseline in CRT as measured by SD-OCT to Week 50 (as the average of Week 46 and Week 50) and Week 102 (as the average of Week 98 and Week 102) |
| | | • Mean change from Week 50 to Week 102 (as the average of Week 98 and Week 102) in CRT as measured by SD-OCT (control arm participants who switch to Construct II) |
| Efficacy | • To assess the need for supplemental anti-VEGF therapy in the Construct II treatment arms | • Proportion of participants who have a reduction of ≥ 50% in supplemental anti-VEGF injection rate through Week 50 and Week 102 compared with the prior 50 weeks preceding the first ranibizumab injection received as part of the Active Run-in Period (Construct II randomized participants) |
| | | • Mean reduction in supplemental anti-VEGF injection rate through Week 50 and Week 102 compared with the prior 50 weeks preceding the first ranibizumab injection received as part of the Active Run-in Period (Construct II randomized participants) |
| | | • Mean number of supplemental anti-VEGF injections in the Construct II arms through Week 50 and Week 102 |
| | | • Mean number of supplemental anti-VEGF injections after Week 50 through Week 102 relative to the prior 50 weeks in the study (control arm participants who switch to Construct II) |
| | | • Time to first supplemental anti-VEGF injection after the Week 2 injection in the Construct II arms |
| | | • Proportion of participants in the Construct II arm who receive supplemental anti-VEGF injection after Week 2 through Week 26, after Week 26 through Week 50, after Week 50 through Week 74, after Week 74 through Week 102, after Week 2 through Week 50, and after Week 2 through Week 102 |
| Pharmacod ynamics | • To assess aqueous protein concentrations of Construct II | • Aqueous Construct II TP concentrations at assessed time points |
| Immunoge nicity | • To evaluate the immunogenicity of Construct II | • Immunogenicity measurements (serum neutralizing antibodies to AAV8 and serum antibodies to Construct II TP) at assessed time points |
| Efficacy | • To evaluate changes over time in the area of geographic atrophy and to assess, in participants with no evidence at baseline, the incidence of new areas of geographic atrophy | • Mean change from baseline in area of geographic atrophy based on FAF at assessed time points |
| | | • Incidence of new area of geographic atrophy by FAF (in participants with no geographic atrophy at baseline) |
| | | • Incidence of retinal thinning in the area of the bleb |
| Efficacy | • To assess the proportion of participants with no fluid on SD-OCT | • Proportion of participants with no fluid on SD-OCT |
| Biomarkers | • To assess aqueous VEGF-A concentrations | • VEGF-A concentrations (aqueous) at assessed time points |
| PRO Questionna ires | • To evaluate visual function and treatment satisfaction using PRO questionnaires | • Mean change from baseline in NEI-VFQ-28-R (composite score; activity limitation domain score; and socio-emotional functioning domain score) at assessed time points |
| | | • Mean change from baseline in NEI-VFQ-25 (composite score and mental health subscale score) at assessed time points |
| | | • Mean change from baseline in MacTSQ (composite score; safety, efficacy, and discomfort domain score; and information provision and convenience domain score) at assessed time points |

| | | |
|---|---|---|
| AAV8 = adeno-associated virus serotype 8: AE = adverse event; BCVA = best-corrected visual acuity: CRT = central retinal thickness: ETDRS = Early Treatment Diabetic Retinopathy Study; FAF = fundus autofluorescence; MacTSQ = Macular Disease Treatment Satisfaction Questionnaire; NEI-VFQ-25 = National Eye Institute Visual Functioning Questionnaire 25-item Version; NEI-VFQ-28-R = National Eye Institute Visual Functioning Questionnaire 28-item Rasch-scored Version; PRO = patient reported outcome: SD-OCT = spectral domain-optical coherence tomography; TP = transgene product; VEGF = vascular endothelial growth factor | | |

### 8.17.2 Study Design

### Overall Study Design

This phase 2b partially masked, randomized, multicenter study will include 3 periods: an Active Run-in Period (*i.e.,* screening), a Treatment Period, and an Extension Period. Participants who receive Construct II will be asked to participate in a long-term follow-up study after completion of or early discontinuation from the current study and will sign a separate informed consent for the follow-up study at that time.

The Active Run-in Period, which will last up to 10 weeks, will begin when the participant signs the Informed consent form (ICF) and will end once the participant has been evaluated for eligibility and has received 3 monthly injections of intravitreal ranibizumab. The Treatment Period will last up to 12 months, beginning when the participant is randomized to study treatment and ending at Week 50. The Extension Period will last up to 12 months, beginning after Week 50 and ending at Week 102.

At Screening Visit 1 (Week -10), participants who meet the inclusion/exclusion criteria will enter the study and receive a 0.5-mg intravitreal injection of ranibizumab in the study eye. At Screening Visit 2 (Week -6), participants will receive a second 0.5-mg intravitreal injection of ranibizumab in the study eye. One week later, at Screening Visit 3 (Week -5), participants' anatomic response on SD-OCT will be evaluated against prespecified response criteria. Participants not meeting response criteria will be exited from the study. If participants meet all inclusion criteria, at Screening Visit 4 (Week -2), participants will be randomized. Any participants who withdraw or become ineligible for randomization during the Screening Period and have an AE associated with the intravitreal ranibizumab injections will be followed until the AE resolves (up to 30 days postinjection). Participants who are identified at Screening Visit 4 as being eligible will receive a third 0.5-mg intravitreal injection of ranibizumab in the study eye. Once the Central Reading Center (CRC) has verified the central retinal thickness (CRT), participants will be randomized (1:1:1) using an interactive response technology (IRT) system to receive a single dose of Construct II (Dose 1), a single dose of Construct II (Dose 2), or monthly intravitreal ranibizumab 0.5 mg; Construct II will be administered by subretinal delivery. Participants will be stratified by baseline (Screening Visit 4) best-corrected visual acuity (BCVA) score (> 58 letters vs ≤ 58 letters) in the randomization.

Participants randomized to the Construct II treatment arms will undergo the surgical procedure on Day 1 followed by visits on Day 2 and Day 8 to assess postoperative safety. At Week 2, participants will receive intravitreal ranibizumab to supplement any anti-VEGF that may have been removed during the vitrectomy surgery to provide anti-VEGF therapy coverage while potential production of the gene therapy mediated protein escalates. The participants will then be seen at monthly intervals, beginning with Week 6, during which supplemental intravitreal ranibizumab 0.5-mg therapy may be administered if needed, as determined by the fully masked CRC evaluation of the SD-OCT data and the fully masked VA assessor's evaluation of BCVA. Note that the SD-OCT and BCVA results, together with predefined retreatment criteria, will inform the investigator's decision to provide supplemental anti-VEGF therapy.

Participants randomized to the ranibizumab control arm will have their first postrandomization visit at Week 2 and will receive intravitreal ranibizumab 0.5 mg. Following the Week 2 visit, the participants will have monthly (~28 day) study visits during which they will receive an injection of ranibizumab 0.5 mg.

At the Week 50 primary endpoint, participants in the ranibizumab control arm will be offered the opportunity to receive Construct II treatment if they still meet key inclusion/exclusion criteria. The treating physician will determine if the participant is eligible and a good candidate for the procedure. Qualified participants will then be administered the highest tolerated dose evaluated in this protocol. Participants in the ranibizumab control arm who switch to Construct II following Week 50 will follow the same visit schedule as the one started on Day 1 for participants originally randomized to receive Construct II. Those participants who either choose not to have treatment with Construct II or are ineligible for treatment with Construct II will be discontinued from the study.

Throughout the study, participants will be evaluated through the assessment of ocular and nonocular AEs including serious adverse events (SAEs) and adverse events of special interest (AESIs) (ocular inflammation deemed by the investigator to be unrelated to the surgical/study procedure and is graded as 2+ or greater on the ocular inflammation grading scales (see Section 8.17.7), ocular infections [including endophthalmitis], retinal tears or detachment, retinal thinning, and new arterial thromboembolic events [nonfatal stroke, nonfatal myocardial infarction, or vascular death (including deaths of unknown cause)]), as well as assessments of clinical laboratory testing (chemistry, hematology, coagulation, urinalysis), ocular examinations and imaging (BCVA, IOP, slit-lamp biomicroscopy, indirect ophthalmoscopy, fluorescein angiography [FA], fundus autofluorescence [FAF], and SD-OCT), and vital signs. Note that AEs will be collected at all study visits. Immunogenicity to the vector and TP of Construct II will also be assessed. Patient reported outcomes (PROs) will be collected using the supplemented National Eye Institute Visual Functioning Questionnaire 25-item version (NEI-VFQ-25) (also comprises the Rasch-scored version, NEI-VFQ-28-R) and Macular Disease Treatment Satisfaction Questionnaire (MacTSQ).

Planned safety monitoring of the study participants will be conducted on an ongoing basis. These include reviews conducted by the partially masked Medical Monitor and routine reviews conducted by the partially masked Sponsor's Internal Safety Committee (ISC). Separately, an Independent Data Monitoring Committee (IDMC) will also be established and will meet on a periodic basis to independently review the clinical data. If unmasked reviews are needed to understand a potential safety signal, these reviews will be conducted by the IDMC.

### 8.17.3 Study Population

### (a) General Considerations

Approximately 300 participants with nAMD who meet the inclusion/exclusion criteria will be randomized. It is expected that up to 50 study centers in the United States will participate in this study. Prospective approval of protocol deviations to recruitment and enrollment criteria, also known as protocol waivers or exemptions, is not permitted.

### (b) Inclusion Criteria

Participants must meet all the following criteria in order to be eligible for this study:
1. Males or females aged ≥ 50 years and ≤ 89 years.
2. An Early Treatment Diabetic Retinopathy Study (ETDRS) BCVA letter score between ≤ 78 and ≥ 44 in the study eye at Screening Visit 1.
3. If both eyes are eligible, the study eye must be the participant's worse-seeing eye, as determined by the investigator prior to randomization.
4. Must have a diagnosis of subfoveal CNV secondary to AMD in the study eye, along with fluid within the parafovea (3-mm center of the macula, based on the early treatment diabetic retinopathy grid) at Screening Visit 1. CNV lesion characteristics as assessed by the CRC: lesion size needs to be less than 10-disc areas (typical disc area = 2.54 mm²).
5. Must be pseudophakic (at least 12 weeks postcataract surgery) in the study eye
6. Must be willing and able to comply with all study procedures and be available for the duration of the study.
7. Women must be postmenopausal (defined as being at least 12 consecutive months without menses) or surgically sterilized (ie, having a bilateral tubal ligation/bilateral salpingectomy, bilateral tubal occlusive procedure, hysterectomy, or bilateral oophorectomy). If not, women must have a negative serum pregnancy test at Screening Visit 1, have negative urine pregnancy test results at Screening Visit 4, and be willing to have additional pregnancy tests during the study.
8. Women of childbearing potential (and their male partners) must be willing to use a highly effective method of contraception and male participants engaged in a sexual relationship with a woman of childbearing potential must be willing to use condoms from Screening Visit 1 until 24 weeks after Construct II administration. For the purpose of this study, highly effective methods of contraception for women of childbearing potential include the following: combined hormonal contraception associated with inhibition of ovulation (oral, intravaginal, transdermal); progestogen-only hormonal contraception associated with inhibition of ovulation (oral, injecteable, implantable); intrauterine device; intrauterine hormone-releasing system; bilateral tubal occlusion; vasectomized partner; or sexual abstinence, when it is preferred and usual lifestyle of the participant.
9. Must be willing and able to provide written, signed informed consent.
10. Based on the Screening Visit 3 SD-OCT, participants must have improvement in fluid (see Response Criterion below) and have a CRT < 400 µm. Note that, if the participant has disease other than fluid contributing to an increase (ie, PED or SHRM) in CRT, they will be enrolled if they have < 75 µm of fluid (intraretinal or subretinal), as determined by the CRC. Response Criterion: Subjects must have an improvement in inner retinal (parafovea 3 mm) fluid relative to Screening Visit 1 of > 50 µm or 30%; or an improvement in center subfield thickness of > 50 µm or 30% as determined by the CRC.

### (c) Exclusion Criteria

Participants are excluded from the study if any of the following criteria apply:
1. CNV or macular edema in the study eye secondary to any causes other than AMD.
2. Subfoveal fibrosis or atrophy as determined by the CRC.
3. Participants who required > 10 anti-VEGF injections in the 12 months prior to the Screening Visit 1.
4. Any condition in the investigator's opinion that could limit VA improvement in the study eye.
5. Active or history of retinal detachment in the study eye.
6. Advanced glaucoma in the study eye defined as IOP of > 23 mmHg not controlled by 2 IOP-lowering medications or any invasive procedure to treat glaucoma (e.g., shunt, tube, or MIGS devices; selective laser trabeculectomy and argon laser trabeculoplasty are permitted).
7. Any condition in the study eye that, in the opinion of the investigator, may increase the risk to the participant, require either medical or surgical intervention during the course of the study to prevent or treat vision loss, or interfere with study procedures or assessments.
8. History of intraocular surgery in the study eye within 12 weeks prior to Screening Visit 1. Yttrium aluminum garnet capsulotomy is permitted if performed > 10 weeks prior to the Screening Visit 1.
9. History of intravitreal therapy in the study eye, such as intravitreal steroid injection or investigational product, other than anti-VEGF therapy, in the 6 months prior to Screening Visit 1.
10. Presence of any implant in the study eye at Screening Visit 1 (excluding intraocular lens).
11. History of malignancy or hematologic malignancy that may compromise the immune system requiring chemotherapy and/or radiation in the 5 years prior to Screening Visit 1. Localized basal cell carcinoma will be permitted.
12. Receipt of any investigational product within the 30 days of enrollment or 5 half-lives of the investigational product, whichever is longer.
13. Received gene therapy.
14. History of retinal toxicity caused by a therapy, or concomitant therapy with any drug that may affect VA or with known retinal toxicity, e.g., chloroquine or hydroxychloroquine.
15. Ocular or periocular infection in the study eye that may interfere with the surgical procedure.
16. Myocardial infarction, cerebrovascular accident, or transient ischemic attack within the past 6 months.
17. Uncontrolled hypertension (systolic blood pressure [BP] > 180 mmHg, diastolic BP > 100 mmHg) despite maximal medical treatment.
18. Any participant with the following laboratory values at Screening Visit 1 will be withdrawn from study:
   - Aspartate aminotransferase (AST)/alanine aminotransferase (ALT) > 2.5 × upper limit of normal (ULN).
   - Total bilirubin > 1.5 × ULN, unless the participant has a previously known history of Gilbert's syndrome and a fractionated bilirubin that shows conjugated bilirubin < 35% of total bilirubin.
   - Prothrombin time > 1.5 × ULN, unless the participant is anticoagulated. Participants who are anticoagulated will be monitored by local labs and managed per local practice to hold or bridge anticoagulant therapy for the study procedure; consultation with the Medical Monitor is also required.
   - Hemoglobin < 10 g/dL for male participants and < 9 g/dL for female participants.
   - Platelets < 100 × 103/µL.
   - Estimated glomerular filtration rate < 30 mL/min/1.73 m².
19. Any concomitant treatment that, in the opinion of the investigator, may interfere with ocular surgical procedure or healing process.
20. Known hypersensitivity to ranibizumab or any of its components.
21. Has a serious, chronic, or unstable medical or psychological condition that, in the opinion of the investigator or Sponsor, may compromise the participant's safety or ability to complete all assessments and follow-up in the study.
22. Currently taking anticoagulation therapy for which holding anticoagulation therapy for Construct II administration is not indicated or considered to be unsafe in the opinion of the treating investigator (ie, retinal surgeon), as well as the physician prescribing anticoagulation for the participant.

### Criteria for Participants in the Control Arm to Obtain Construct II After Week 50

### (a) Inclusion Criteria.

1. Study eye will be the eye that qualified at randomization.
2. Participant has a CRT < 400 µm of subretinal/intraretinal fluid or (in cases where a participant may have nonfluid elevation in the CRT, eg, pigment epithelial defect) < 75 µm of excess fluid, as confirmed by the masked CRC.
3. Women of childbearing potential (and their male partners) must be willing to use a highly effective method of contraception and male participants engaged in a sexual relationship with a woman of childbearing potential must be willing to use condoms from the surgical visit until 24 weeks after Construct II administration. For the purpose of this study, highly effective methods of contraception for women of childbearing potential include the following: combined hormonal contraception associated with inhibition of ovulation (oral, intravaginal, transdermal); progestogen-only hormonal contraception associated with inhibition of ovulation (oral, injecteable, implantable); intrauterine device; intrauterine hormone-releasing system; bilateral tubal occlusion; vasectomized partner; or sexual abstinence, when it is preferred and usual lifestyle of the participant.
4. Women of childbearing potential must have a negative urine pregnancy test at Week 52 and be willing to have additional pregnancy tests during the study.

### (b) Exclusion Criteria.

1. CNV or macular edema in the study eye secondary to any causes other than AMD.
2. Subfoveal fibrosis or atrophy as determined by the CRC, or any condition preventing VA improvement in the study eye.
3. Ocular or periocular infection in the study eye that may interfere with the surgical procedure.
4. Myocardial infarction, cerebrovascular accident, or transient ischemic attacks since randomization.
5. Uncontrolled hypertension (systolic BP >180 mmHg, diastolic BP > 100 mmHg) despite maximal medical treatment.
6. Any concomitant treatment that, in the opinion of the investigator, may interfere with ocular surgical procedure or healing process.
7. History of malignancy or hematologic malignancy that may compromise the immune system requiring chemotherapy and/or radiation in the past year. Localized basal cell carcinoma will be permitted.
8. Currently taking anticoagulation therapy for which holding anticoagulation therapy for Construct II administration is not indicated or considered to be unsafe in the opinion of the treating investigator as well as the physician prescribing anticoagulation for the participant.

### 8.17.4 Study Intervention

Study intervention is defined as any investigational intervention(s), marketed product(s), placebo, or medical device(s) intended to be administered to a study participant according to the study protocol.

### (a) Study Intervention(s) Administered

Eligible participants will be randomized 1:1:1 to receive a single dose of Construct II (Dose 1), a single dose of Construct II (Dose 2), or monthly intravitreal injections of ranibizumab.

Participants in either of the Construct II arms will receive Construct II on Day 1 via subretinal delivery in an operating room. During the study, participants in the Construct II arms will receive ranibizumab 0.5 mg, administered by intravitreal injection, on Screening Visits 1, 2, and 4, at Week 2, and then as needed every 4 weeks starting at Week 6.

Participants in the ranibizumab control arm will receive ranibizumab 0.5 mg, administered by intravitreal injection, on Screening Visits 1, 2, and 4, at Week 2, and then monthly (~28 days) thereafter.

**Table 4. Study Intervention(s) Administered**

| **Arm Name** | **Construct II Dose 1** | **Construct II Dose 2** | **Ranibizumab (LUCENTIS)** |
|---|---|---|---|
| **Type** | Gene therapy | | Drug |
| **Dose Formulation** | Solution | | |
| **Unit Dose Strength** | 6.2 × 10¹¹ GC/mL | 1.0 × 10¹² GC/mL | 10 mg/mL |
| **Dose Level(s)** | 250 µL (1.6 × 10¹¹ GC/eye) one-time dose | 250 µL (2.5 × 10¹¹ GC/eye) one-time dose | 0.5 mg (0.05 mL of 10 mg/mL solution) once a month (approximately every 28 days) |
| **Route of Administration** | Subretinal delivery | | Intravitreal injection |
| **Physical Description** | Construct II investigational product is supplied as a frozen, sterile, single-use solution of the AAV vector active ingredient in a formulation buffer. The solution appears clear to opalescent, colorless, and free of visible particulates at room temperature. | | LUCENTIS is supplied as a preservative-free, sterile solution in a single-use container designed to deliver 0.05 mL of 10 mg/mL LUCENTIS (0.5 mg dose prefilled syringe or vial) aqueous solution. The solution appears colorless to pale yellow. |
| **Manufacturer** | Advanced Bioscience Laboratories, Inc | | Genentech, Inc |
| **Packaging and Labeling** | Construct II will be supplied as a sterile, single-use solution in 2-mL Crystal Zenith^{®} vials sealed with latex free rubber stoppers and aluminum flip-off seals. Each vial will be labeled as required per country regulatory requirements. | | Study intervention will be obtained in commercial packaging, either the prefilled syringe (NDC 50242-080-03) or single-use 2-mL glass vial (NDC 50242-080-02) designed to deliver 0.05 mL of 10mg/mL ranibizumab solution. |

### 8.17.5 Ocular Inflammation Grading Scale

Ocular inflammation will be assessed during slit-lamp biomicroscopy and independent ophthalmoscopy and graded using the following scales. The standard practice for slit-lamp biomicroscopy and indirect ophthalmoscopy assessment should be used.

**Table 5. Grading Scale for Ocular Inflammation: Anterior Chamber Cells and Anterior Chamber Flare**

| **Anterior Chamber Cells** | |
|---|---|
| **Grade** | **Cells in Field (1 mm × 1 mm slit beam)** |
| 0 | None |
| +0.5 | 1-5 |
| +1 | 6 - 15 |
| +2 | 16-25 |
| +3 | 26 - 50 |
| +4 | > 50 |

| **Anterior Chamber Flare** | |
|---|---|
| **Grade** | **Description** |
| 0 | None |
| +1 | Trace |
| +2 | Moderate (iris and lens detail clear) |
| +3 | Marked (iris and lens detail hazy) |
| +4 | Intense (fibrin or plastic aqueous) |

| Source: Jabs et al., 2005, Am J Ophthalmol 140(3):509-516. Table 6. Grading Scale for Vitreous Haze | |
|---|---|
| **Grade** | **Amount of Vitreal Haze** |
| 0 | None |
| +0.5 | Trace |
| +1 | Clear optic disc and vessels; hazy nerve fiber layer |
| +2 | Hazy optic disc and vessels |
| +3 | Optic disk visible |
| +4 | Optic disc not visible |

| | |
|---|---|
| Source: Nussenblatt et al., 1985, Ophthalmology, 92(4):467-471. | |

### 8.18 EXAMPLE 18: A Phase 2, Randomized, Dose-escalation, Ranibizumab-controlled Study to Evaluate the Efficacy, Safety, and Tolerability of Construct II Gene Therapy Delivered via One or Two Suprachoroidal Space (SCS) Injections in Participants with Neovascular Age-Related Macular Degeneration (nAMD)

### 8.18.1 Synopsis

### (a) Objectives and Endpoints

**Table 7: Objectives and Endpoints**

| **Measure** | **Objectives** | **Endpoints** |
|---|---|---|
| **Primary** | | |
| Efficacy | To evaluate the mean change in BCVA for Construct II compared with ranibizumab monthly at Week 40 | Mean change from baseline in BCVA to Week 40 based on the ETDRS score |

| **Secondary** | | |
|---|---|---|
| Safety | To evaluate the safety and tolerability of Construct II | Incidences of overall and ocular AEs and SAEs through Week 52 |
| | | Vector shedding analysis in serum, urine, and tears |
| | To evaluate the effect of Construct II on CNV lesion growth and leakage as measured by FA | Mean change from baseline in CNV lesion size and leakage area based on FA at Week 40 and Week 52 |

| **Measure** | **Objectives** | **Endpoints** |
|---|---|---|
| Efficacy | To evaluate the effect of Construct II on BCVA | Mean change from baseline in BCVA to Week 52 |
| | | Proportion of participants (1) gaining or losing ≥ 15, ≥ 10, ≥ 5, or ≥ 0 letters; (2) maintaining vision (not losing ≥ 15 letters) compared with baseline as per BCVA at Week 40 and Week 52 |
| | | Mean change from baseline in BCVA to Week 40 and Week 52 for participants who received ≤ 2 supplemental anti-VEGF injections, 2 supplemental anti-VEGF injections, 1 supplemental anti-VEGF injection, or 0 supplemental anti-VEGF injections (Construct II randomized participants) |
| | To evaluate the effect of Construct II on CRT, as measured by SD-OCT | Mean change from baseline in CRT as measured by SD-OCT to Week 40 and Week 52 |
| | To assess the need for supplemental anti-VEGF therapy in participants who receive Construct II treatment | Annualized supplemental anti-VEGF injection rate through Week 40 and Week 52 |
| | | Proportion of participants who have a reduction of ≥ 50% in the annualized supplemental anti-VEGF injection rate through Week 40 and Week 52 compared with the prior 52 weeks preceding the first intravitreal ranibizumab injection received as part of the Screening Period (Construct II randomized participants) |
| | | Mean reduction in the annualized supplemental anti-VEGF injection rate through Week 40 and Week 52 compared with the prior 52 weeks preceding the first ranibizumab injection received as part of the Screening Period (Construct II randomized participants) |
| | | Time to first supplemental anti-VEGF injection |
| Pharmacodynamics | To evaluate the concentration of Construct II TP in aqueous humor | Mean change from baseline in aqueous Construct II TP concentrations over time |
| Immunogenicity | To evaluate the immunogenicity of Construct II | Immunogenicity measurements (AAV8: NAbs, TAbs, and ELISpot; Construct II protein: TAbs and ELISpot) |

| Exploratory | | |
|---|---|---|
| Efficacy | To evaluate the effect of Construct II on fluid accumulation as assessed by SD-OCT | Proportion of participants with no fluid on SD-OCT |
| | | Proportion of participants with stable fluid on SD-OCT within 30 µm of baseline |
| Safety | To assess changes in visual function by visual fields | Changes in visual field testing over time |
| | To evaluate the incidences of new areas of geographic atrophy, as assessed by FAF | Incidence of new area of geographic atrophy by FAF (in participants with no geographic atrophy at baseline) |
| Biomarker | To assess aqueous humor VEGF concentrations | VEGF-A concentrations (aqueous) at assessed time points |

| | | |
|---|---|---|
| AAV8 = adeno-associated virus serotype 8; AE = adverse event; BCVA = best-corrected visual acuity; CNV = choroidal neovascularization; CRT = central retinal thickness; ELISpot = enzyme-linked InununoSpot; ETDRS = Early Treatment Diabetic Retinopathy Study; FA = fluorescein angiography; FAF = fundus autofluorescence; NAbs = neutralizing antibodies; SAE = serious adverse event; SCS = suprachoroidal space; SD-OCT = spectral domain-optical coherence tomography; TAbs = total binding antibodies; TP = transgene product; VEGF = vascular endothelial growth factor | | |

### (b) Study Design

In this phase 2, randomized(3: 1), dose-escalation, ranibizumab-controlled, study, approximately 40 participants with nAMD will be enrolled into 2 dose cohorts. Within each dose cohort, participants will receive a one-time administration of Construct II in the SCS (n = 15 participants) or an intravitreal injection of ranibizumab 0.5 mg every 4 weeks up to Week 52 (n = 5 participants).

Participants who receive Construct II will strongly be encouraged to enroll in a long-term follow-up study after completion of the current study at Week 52 (or early discontinuation) and will sign a separate informed consent for the follow-up study at that time. Participants in the ranibizumab control arm will be offered an opportunity following the Week 52 visit to be included in a future Construct II dose cohort.

Screening will comprise 3 visits to select for eligible participants with qualifying AAV8 neutralizing antibodies (NAbs) titers (Visit 1) who demonstrate anatomic responsiveness to ranibizumab during a ranibizumab run-in phase (Visits 2 and 3). During Visit 1, participants who sign the informed consent form (ICF) will be evaluated for eligibility and will have serum samples collected to screen for pre-existing NAbs or will confirm NAb status from a NAb screening protocol. Participants who have negative or low (≤ 300) titer results for serum AAV8 NAbs will return to the study center to confirm the remaining inclusion/exclusion criteria. Participants continuing to meet eligibility criteria will receive a 0.5-mg intravitreal injection of ranibizumab in the study eye at Visit 2 (Day 1). At Visit 3 (Week 1), participants will be evaluated by spectral domain-optical coherence tomography (SD-OCT) to confirm their anatomic response to the screening anti-VEGF injection via comparison against their Day 1 SD-OCT assessment taken prior to the screening ranibizumab injection. Anatomic response will be determined by a central reading center (CRC) according to pre-specified criteria. Once the CRC has verified anatomic eligibility, 2 sentinel participants in each cohort will be randomized one to Construct II or ranibizumab control. Participants who do not have an anatomic response will be considered screen failures. For screen-failed participants, anyone who has an AE associated with the ranibizumab injections on Day 1 will be followed until the AE resolves (up to 30 days post injection).

At the Week 2 visit, Construct II randomized participants will receive either 1 or 2 injections of Construct II, depending on dose level, administered at the study center by SCS delivery using the Clearside SCS Microinjector^{™} investigational device; note that the Treatment Period of the study begins at the time of Construct II administration. All investigators will be trained on the SCS procedure. A detailed description of the procedure can be found in the SCS Administration Manual. Following Construct II administration to the sentinel participant who is randomized to Construct II, a 2-week observation period will be conducted for safety. The Sponsor's Internal Safety Committee (ISC) will review the safety data for this participant and, if there are no safety concerns, up to 18 additional participants (14 Construct II and 4 ranibizumab controls) may be randomized. If no safety review triggers (SRTs) are observed, then, following a 2-week observation period for the last dosed participant within the cohort, all available safety data will be evaluated by the Independent Data Monitoring Committee (IDMC). Additionally, if any event meets the criteria of a Stopping Rule, dosing of any new participants will be suspended until a complete review of all safety data has been performed. At any given IDMC meeting, whether planned or called for due to an SRT, the IDMC may recommend stopping the study, proceeding to the next dosing cohort, or proceeding to a lower dose (up to a half-log).

Participants randomized to Construct II will have 2 visits for post injection safety (1-day post procedure and 1-week post procedure). Starting 2 weeks after Construct II administration, participants will have monthly study visits and may receive intravitreal ranibizumab supplemental therapy if they meet predefined supplemental injection criteria. For participants in the Construct II treatment arms, immunogenicity to the vector (as assessed by AAV8 NAbs, AAV8 TAbs, antibodies to Construct II protein, and enzyme-linked ImmunoSpot [ELISpot]), VEGF-A concentrations, and anti-Construct II antibodies will be assessed throughout the study.

Participants randomized to the ranibizumab control arm will have their first post randomization visit at Week 4 and will receive intravitreal ranibizumab 0.5 mg. Following the Week 4 visit, the participants will have monthly (~ every 28 days) study visits during which they will receive an intravitreal injection of ranibizumab 0.5 mg.

Efficacy will be the primary focus of the initial 40 weeks (primary study period). Following completion of the primary study period, participants will continue to be assessed until Week 52. At the end of the Week 52 study visit, participants who received Construct II will be invited to enroll into a long-term follow-up study, while participants who were in the ranibizumab control arm, if eligible, will be offered an opportunity to be included in a future Construct II dose cohort. Participants will be evaluated for safety through the assessment of AEs, including SAEs and adverse events of special interest (AESIs) (ocular inflammation deemed by the investigator to be unrelated to the surgical/study procedure and graded as 2+ or greater on the ocular inflammation grading scales, ocular infections [including endophthalmitis], retinal tears or detachment, retinal thinning, and new arterial thromboembolic events [nonfatal stroke, nonfatal myocardial infarction, or vascular death (including deaths of unknown cause)]), as well as assessments of clinical laboratory tests (chemistry, hematology, coagulation, urinalysis), and ocular examinations and imaging (BCVA, IOP, slit-lamp biomicroscopy, indirect ophthalmoscopy, fluorescein angiography [FA], ultra-wide field Optos fundus auto fluorescence [FAF], ultra-wide field Optos color fundus photography [CFP], Humphrey visual field 120, ormicroperimetry, and SD-OCT). Note that AEs will be collected at all study visits. Participants who show evidence of new retinal hypo/hyper pigmentation changes as compared with baseline will be monitored using SD-OCT scans. Radial SD-OCT scans that transverse the margin of the hypo/hyper pigmentary area will be captured when possible.

Planned safety monitoring of the study participants will be conducted on an ongoing basis. The monitoring will include reviews conducted by the Medical Monitor and routine reviews conducted by the Sponsor's ISC. Separately, an IDMC will also be established and will meet on a periodic basis to independently review the clinical data.

### 8.18.2 Inclusion Criteria

### All Participants Entering the Study

Participants are eligible to be included in the study only if all of the following criteria apply:
1. Males or females, aged ≥ 50 years and ≤ 89 years.
2. Must have a diagnosis of subfoveal CNV secondary to AMD in the study eye, along with retinal fluid (either subretinal or intraretinal) within the parafovea (3-mm center of the macula, based on the early treatment diabetic retinopathy grid), as assessed by the CRC.
   - CNV lesion characteristics: lesion size needs to be less than 10-disc areas (typical disc area = 2.54 mm2).
3. May be phakic or pseudophakic.
4. Must have a negative or low serum titer result (≤ 300) for AAV8 NAbs.
5. BCVA between ≤ 20/25 and ≥ 20/125 (≤ 83 and ≥ 44 Early Treatment Diabetic Retinopathy Study [ETDRS] letters) in the study eye.
6. Based on the SD-OCT image obtained at Week 1, participants must have improvement in fluid (see Response Criterion below) and have a central retinal thickness (CRT) < 400 µm. Note that, if the participant has disease other than fluid contributing to an increase (ie, PED or SHRM) in CRT, they will be enrolled if they have < 75 µm of total fluid (intraretinal or subretinal), as determined by the CRC.
   - Response Criterion: Participants must have an improvement in inner retinal (parafovea 3 mm) fluid relative to Visit 2 of > 50 µm or 50%; or an improvement in center subfield thickness of > 50 µm or 50%, as determined by the CRC.
7. If both eyes are eligible, the study eye must be the participant's worse-seeing eye, as determined by the investigator.
8. Women must be postmenopausal (defined as being at least 12 consecutive months without menses) or surgically sterilized (*i.e.,* having a bilateral tubal ligation/bilateral salpingectomy, bilateral tubal occlusive procedure, hysterectomy, or bilateral oophorectomy). If not, women must have negative serum and urine pregnancy tests at Day 1 and be willing to undergo additional pregnancy testing during the study.
9. Women of childbearing potential (WOCBP) (and their male partners) must be willing to use a highly effective method of contraception (Section 8.5) and male participants engaged in a sexual relationship with a WOCBP must be willing to use condoms from Week 2 until 24 weeks after Construct II administration.
10. Must be willing and able to provide signed informed consent, comply with all study procedures, and be available for the duration of the study.

### 8.18.3 Exclusion Criteria

Participants are excluded from the study if any of the following criteria apply:
1. CNV or macular edema in the study eye secondary to any causes other than AMD.
2. Subfoveal fibrosis or atrophy, as determined by the CRC.
3. Participants who required > 10 anti-VEGF injections in the 12 months prior to Visit 2.
4. Participants who had a prior vitrectomy.
5. Any condition in the investigator's opinion that could limit VA improvement in the study eye.
6. Active or history of retinal detachment in the study eye.
7. Advanced glaucoma in the study eye, defined as IOP of > 23 mmHg not controlled by 2 IOP-lowering medications or any invasive procedure to treat glaucoma (eg, shunt, tube, or MIGS devices; however, selective laser trabeculectomy and argon laser trabeculoplasty are permitted).
8. Any condition in the study eye that, in the opinion of the investigator, may increase the risk to the participant, require either medical or surgical intervention during the course of the study to prevent or treat vision loss, or interfere with study procedures or assessments.
9. History of intravitreal therapy in the study eye, such as intravitreal steroid injection or investigational product, other than anti-VEGF therapy, in the 6 months prior to Visit 2.
10. Presence of an implant in the study eye at screening (excluding an intraocular lens).
11. History of malignancy requiring chemotherapy and/or radiation in the 5 years prior to screening. Localized basal cell carcinoma will be permitted.
12. Received any gene therapy.
13. History of therapy known to have caused retinal toxicity, or concomitant therapy with any drug that may affect VA or with known retinal toxicity, *e.g.*, chloroquine or hydroxychloroquine.
14. Any concomitant treatment that, in the opinion of the investigator, may interfere with the ocular procedure or healing process.
15. Known hypersensitivity to ranibizumab or any of its components or past hypersensitivity (in the investigator's opinion) to agents like Construct II.
16. Has a serious, chronic, or unstable medical or psychological condition that, in the opinion of the investigator, may compromise the participant's safety or ability to complete all assessments and follow-up in the study.
17. Any condition preventing visualization of the fundus or VA improvement in the study eye, *e.g.*, cataract, vitreous opacity, fibrosis, atrophy, or retinal epithelial tear in the center of the fovea.
18. History of intraocular surgery in the study eye within 12 weeks prior to Visit 2. Yttrium aluminum garnet capsulotomy is permitted if performed >10 weeks prior to Visit 2.
19. Receipt of any investigational product within 30 days of Visit 2 or 5 half-lives of the investigational product, whichever is longer.
20. Ocular or periocular infection in the study eye that may interfere with the administration of Construct II.
21. Myocardial infarction, cerebrovascular accident, or transient ischemic attacks within the 6 months prior to Visit 2.
22. Uncontrolled hypertension (systolic blood pressure [BP] >180 mmHg, diastolic BP > 100 mmHg) despite maximal medical treatment.
23. Any participant with the following laboratory values collected at Visit 2 and confirmed at Visit 3:
   - Aspartate aminotransferase (AST)/alanine aminotransferase (ALT) > 2.5 × upper limit of normal (ULN).
   - Total bilirubin > 1.5 × ULN, unless the participant has a previously known history of Gilbert's syndrome and a fractionated bilirubin that shows conjugated bilirubin < 35% of total bilirubin.
   - Prothrombin time > 1.5 × ULN, unless the participant is anticoagulated.
   - Hemoglobin < 10 g/dL for male participants and < 9 g/dL for female participants.
   - Platelets < 100 × 10³/µL.
   - Estimated glomerular filtration rate < 30 mL/min/1.73 m².

### 8.18.4 Study Intervention(s) Administered

Eligible participants will be assigned either to receive Construct II (Dose 1 or Dose 2) or ranibizumab in the study eye. Information regarding Construct II and ranibizumab follows.

**Table 8. Information regarding Construct II and ranibizumab**

| **Arm Name** | **Construct II Dose 1** | **Construct II Dose 2** | **Ranibizumab (LUCENTIS)** |
|---|---|---|---|
| **Type** | Gene therapy (AAV8.CB7.CI.amd42.RBG) | | Drug (control treatment arm and run-in/rescue) |
| **Dose Formulation** | Solution | | |
| **Unit Dose Strength** | 1.0 × 10¹² GC/mL | 2.5 × 10¹² GC/mL | 10 mg/mL |
| **Dose Level(s)** | 100 µL (2.5 × 10¹¹ GC/eye) delivered via a single SCS injection | 100 µL (5.0 × 10¹¹ GC/eye) delivered via 2 SCS injections at the same visit | 0.5 mg (0.05 mL of 10 mg/mL solution) once at Visit 2 or as rescue starting 2 weeks post Construct II administration, provided according to rescue criteria |
| **Route of Administration** | Suprachoroidal space injection(s) in the study eye using the Clearside SCS Microinjector^{™} investigational device | | Intravitreal injection in the study eye |
| **Physical Description** | Construct II investigational product is supplied as a frozen, sterile, single-use solution of the AAV vector active ingredient (AAV8.CB7.CI.amd42.RBG) in a formulation buffer. The solution appears clear to opalescent, colorless, and free of visible particulates at room temperature. | | LUCENTIS is supplied as a preservative-free, sterile solution in a single-use container designed to deliver 0.05 mL of 10 mg/mL LUCENTIS (0.5-mg dose prefilled syringe or vial) aqueous solution. The solution appears colorless to pale yellow. |
| **Manufacturer** | Advanced BioScience Laboratories, Inc | | Genentech, Inc |
| **Packaging and Labeling** | Construct II will be supplied as a sterile, single-use solution in 2-mL Crystal Zenith^{®} vials sealed with latex-free rubber stoppers and aluminum flip-off seals. Each vial will be labeled as required per country regulatory requirements. | | Study intervention will be obtained in commercial packaging either the prefilled syringe (NDC 50242-080-03) or single-use 2-mL glass vial (NDC 50242-080-02) designed to deliver 0.05 mL of 10 mg/mL ranibizumab solution. |

### 8.19 EXAMPLE 19: Treatment of Batten-CLN2-Associated Vision Loss with Tripeptidyl-Peptidase 1 (TPP1) cDNA-Based Vector in Non-human Primates

A nonclinical toxicology study in non-human primates was initiated to evaluate Tripeptidyl-Peptidase 1 (TPP1) cDNA-based vector by two different routes of administration - subretinal and suprachoroidal. All animals were sacrificed and tissues are being analyzed

In this study, groups of cynomolgus monkeys (5 animals/group) were administered TPP1 cDNA-based vector via subretinal (SR) injection at doses of 0 (vehicle), 1 × 10¹⁰, 1 × 10¹¹, 1×10¹² or 1×10¹³ GC/eye (100 µL). Additional groups (5 animals/group) were administered TPP1 cDNA-based vector via injection into the suprachoroidal space (SCS) using a microneedle at a dose of 0 (vehicle) or 1×10¹² GC/eye (two 50 µL injections at superior temporal or inferior nasal quadrants). All treated groups were administered TPP1 cDNA-based vector in both eyes. Control animals received an injection of vehicle into via either the SCS (OS) or the SR route (OD). Animals were euthanized either 4 weeks (2 animals/group) or 3 months (3 animals/group) after administration of the TPP1 cDNA-based vector. Endpoints included in this study were: clinical observations, body weights, ophthalmic procedures (ophthalmoscopy, intraocular pressure, optical coherence tomography, fundus ocular photography and full field electroretinography), TPP1 (aqueous and vitreous [terminal only] humor; serum), anti-AAV antibodies (nAbs), anti-transgene product antibodies (ATPA), biodistribution, organ weights, immunohistochemistry (anti-TPP1 in the eye), macroscopic and microscopic examination.

### 8.20 EXAMPLE 20: Treatment of Batten-CLN2-Associated Vision Loss with Tripeptidyl-Peptidase 1 (TPP1) cDNA-Based Vector in Human Subject

A subject presenting with Batten-CLN2-associated vision loss is administered AAV9 that encodes Tripeptidyl-Peptidase 1 at a dose (*e.g.,* 1 x 10¹⁰ to 5 x 10¹¹ genome copies per eye) sufficient to produce a therapeutically effective concentration of the transgene product in the vitreous humour for three months. The administration is done by a dual route of administration that involves both a central nervous system (CNS) delivery (*e.g*., intracerebroventricular (ICV), intracisternal (IC), or intrathecal-lumbar (IT-L) delivery) and an ocular delivery (*e.g*., suprachoroidal, subretinal, juxtascleral, or intravitreal delivery). Following treatment, the subject is evaluated for improvement in Batten-CLN2-associated vision loss.

### 8.21 EXAMPLE 21: Use of an Infrared Thermal Camera to Monitor Injection in Pigs

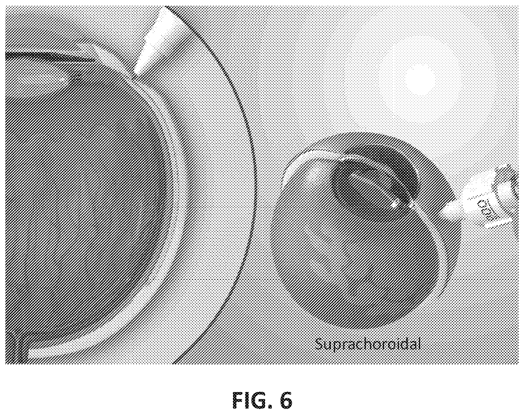

The FLIR T530 infrared thermal camera was used to characterize post ocular injection thermal profiles in live pigs. Alternatively, an FLIR T420, FLIR T440, Fluke Ti400, or FLIRE60 infrared thermal camera is used. Suprachoroidal (FIG. 6), unsuccessful suprachoroidal, intravitreal, and extraocular efflux injections of room temperature saline (68-72 °F ) were assessed in the study. Dose volume was 100 µL for every injection with the solution from the refrigerator to room temperature for injection.

Infrared camera lens to ocular surface distance was established at approximately 1 ft. The manual temperature range on the camera for viewing was set to ~80-90 °F. Imaging operator held the camera and set the center screen cursor aimed at the injection site during video recordings. Pigs received a retrobulbar injection of saline to proptose the eye for better visibility, and eye lids were cut and retracted back to expose the sclera at the site of injection. The iron filter was used during thermal video recordings.

A successful suprachoroidal injection was characterized by: (a) a slow, wide radial spread of the dark color, (b) very dark color at the beginning, and (c) a gradual change of injectate to lighter color, i.e., a temperature gradient noted by a lighter color. An unsuccessful suprachoroidal injection was characterized by: (a) no spread of the dark color, and (b) a minor change in color localized to the injection site. A successful intravitreal injection was characterized by: (a) no spread of the dark color, (b) an initial change to very dark color localized to the injection site, and (c) a gradual and uniform change of the entire eye to darker color occurring after the injection developing with time. Extraocular efflux was characterized by: (a) quick flowing streams on outside exterior of the eye, (b) very dark color at the beginning, and (c) a quick change to lighter color.

### 8.22 EXAMPLE 22: Use of an Infrared Thermal Camera to Monitor Injection in Human Patients

A subject presenting with wet AMD is administered AAV8 that encodes ranibizumab Fab (*e.g.,* by subretinal administration, suprachoroidal administration, or intravitreal administration) at a dose sufficient to produce a concentration of the transgene product at a Cmin of at least 0.330 µg/mL in the Vitreous humour for three months. The FLIR T530 infrared thermal camera is used to evaluate the injection during the procedure and is available to evaluate after the injection to confirm either that the administration is successfully completed or misdose of the administration. Alternatively, an FLIR T420, FLIR T440, Fluke Ti400, or FLIRE60 infrared thermal camera is used. Following treatment, the subject is evaluated clinically for signs of clinical effect and improvement in signs and symptoms of wet AMD.

### 8.23 EXAMPLE 23: Treatment of Batten-CLN2-Associated Vision Loss with Tripeptidyl-Peptidase 1 (TPP1) cDNA-Based Vector in Human Subject by Suprachoroidal Administration

A subject presenting with Batten-CLN2-associated vision loss is administered AAV9 that encodes Tripeptidyl-Peptidase 1 at a dose (*e.g.,* 1 x 10¹⁰ to 5 x 10¹¹ genome copies per eye) sufficient to produce a therapeutically effective concentration of the transgene product in the vitreous humour for three months. The TPP1 cDNA-based vector is administered by suprachoroidal administration. Following treatment, the subject is evaluated for improvement in Batten-CLN2-associated vision loss.

### 8.24 EXAMPLE 24: Treatment of Batten-CLN2-Associated Vision Loss with Tripeptidyl-Peptidase 1 (TPP1) cDNA-Based Vector in Human Subject by Subretinal Administration

A subject presenting with Batten-CLN2-associated vision loss is administered AAV9 that encodes Tripeptidyl-Peptidase 1 at a dose *(e.g.,* 1 x 10¹⁰ to 5 x 10¹¹ genome copies per eye) sufficient to produce a therapeutically effective concentration of the transgene product in the vitreous humour for three months. The TPP1 cDNA-based vector is administered by subretinal administration. Following treatment, the subject is evaluated for improvement in Batten-CLN2-associated vision loss.

### EQUIVALENTS

Although the invention is described in detail with reference to specific embodiments thereof, it will be understood that variations which are functionally equivalent are within the scope of this invention. Indeed, various modifications of the invention in addition to those shown and described herein will become apparent to those skilled in the art from the foregoing description and accompanying drawings. Such modifications are intended to fall within the scope of the appended claims. Those skilled in the art will recognize, or be able to ascertain using no more than routine experimentation, many equivalents to the specific embodiments of the invention described herein. Such equivalents are intended to be encompassed by the following claims.

All publications, patents and patent applications mentioned in this specification are herein incorporated by reference into the specification to the same extent as if each individual publication, patent or patent application was specifically and individually indicated to be incorporated herein by reference in their entireties.
Also disclosed herein are *inter alia* the following items (said items are not claims).
1. A method of subretinal administration without vitrectomy for treating a pathology of the eye, comprising administering to the subretinal space in the eye of a human subject in need of treatment a recombinant viral vector comprising a nucleotide sequence encoding a therapeutic product such that the therapeutic product is expressed and results in treatment of the pathology of the eye, wherein the method does not comprise performing a vitrectomy on the eye of said human patient.
2. The method of item 1, wherein the administering step comprises administering to the subretinal space in the eye of said human subject the recombinant viral vector therapeutic product via the suprachoroidal space in the eye of said human subject.
3. The method of item 2, wherein the administering step is by the use of a subretinal drug delivery device comprising a catheter that can be inserted and tunneled through the suprachoroidal space toward the posterior pole, where a small needle injects into the subretinal space.
4. The method of item 3, wherein the administering step comprises inserting and tunneling
   the catheter of the subretinal drug delivery device through the suprachoroidal space.
5. A method of suprachoroidal administration for treating a pathology of the eye, comprising administering to the suprachoroidal space in the eye of a human subject in need of treatment a recombinant viral vector comprising a nucleotide sequence encoding a therapeutic product such that the therapeutic product is expressed and results in treatment of the pathology of the eye.
6. The method of item 5, wherein the administering step is by injecting the recombinant viral vector into the suprachoroidal space using a suprachoroidal drug delivery device.
7. The method of item 5 or 6, wherein the suprachoroidal drug delivery device is a microinjector.
8. A method of administration to the outer space of the sclera for treating a pathology of the eye, comprising administering to the outer surface of the sclera in the eye of a human subject in need of treatment a recombinant viral vector comprising a nucleotide sequence encoding a therapeutic product such that the therapeutic product is expressed and results in treatment of the pathology of the eye.
9. The method of item 8, wherein the administering step is by the use of a juxtascleral drug delivery device that comprises a cannula whose tip can be inserted and kept in direct apposition to the scleral surface.
10. The method of item 9, wherein the administering step comprises inserting and keeping the tip of the cannula in direct apposition to the scleral surface.
11. The method of any one of items 1-10, wherein the therapeutic product is not an anti-human vascular endothelial growth factor (hVEGF) antibody.
12. The method of any one of items 1-11, wherein the pathology of the eye is not associated with neovascular age-related macular degeneration (nAMD).
13. A method of subretinal administration accompanied by vitrectomy for treating a pathology of the eye, comprising administering to the subretinal space in the eye of a human subject in need of treatment a recombinant viral vector comprising a nucleotide sequence encoding a therapeutic product such that the therapeutic product is expressed and results in treatment of the pathology of the eye, wherein the method comprises performing a vitrectomy on the eye of said human patient, and wherein the therapeutic product is not anti-human vascular endothelial growth factor (hVEGF) antibody.
14. The method of item 13, wherein the vitrectomy is a partial vitrectomy.
15. A method of subretinal administration for treating a pathology of the eye, comprising administering to the subretinal space peripheral to the optic disc, fovea and macula located in the back of the eye of a human subject in need of treatment a recombinant viral vector comprising a nucleotide sequence encoding a therapeutic product such that the therapeutic product is expressed and results in treatment of the pathology of the eye, wherein the method does not comprise performing a vitrectomy on the eye of said human patient.
16. The method of item 15, wherein the administering step is by transvitreal injection.
17. The method of item 16, wherein the transvitreal injection comprises inserting a sharp needle into the sclera via the superior or inferior side of the eye and passing the sharp needle all the way through the vitreous to inject the recombinant viral vector to the subretinal space on the other side.
18. The method of item 16, wherein the transvitreal injection comprises inserting a trochar into the sclera and inserting a cannula through the trochar and through the vitreous to inject the recombinant viral vector to the subretinal space on the other side.
19. The method of any one of items 15-18, wherein the therapeutic product is an anti-hVEGF antibody.
20. The method of item 19, wherein the anti-hVEGF antibody is an anti-hVEGF antigen-binding fragment.
21. The method of item 20, wherein the anti-hVEGF antigen-binding fragment is a Fab, F(ab')₂, or single chain variable fragment (scFv).
22. The method of any one of items 19-21, wherein the anti-hVEGF antibody comprises a heavy chain comprising the amino acid sequence of SEQ ID NO:2 or SEQ ID NO:4, and a light chain comprising the amino acid sequence of SEQ ID NO:1, or SEQ ID NO:3.
23. The method of any one of items 19-21, wherein the anti-hVEGF antibody comprises light chain CDRs 1-3 of SEQ ID NOs:14-16 and heavy chain CDRs 1-3 of SEQ ID NOs:17-19 or SEQ ID NOs:20, 18, and 21.
24. The method of any one of items 19-23, wherein the pathology of the eye is associated with nAMD, dry age-related macular degeneration (dry AMD), retinal vein occlusion (RVO), diabetic macular edema (DME), or diabetic retinopathy (DR).
25. The method of any one of items 19-23, wherein the pathology of the eye is associated with nAMD.
26. The method of any one of items 1-11 and 13-18, wherein:
   (1) the pathology of the eye is associated with Batten-CLN1 and the therapeutic product is Palmitoyl-Protein Thioesterase 1 (PPT1);
   (2) the pathology of the eye is associated with Batten-CLN2 and the therapeutic product is Tripeptidyl-Peptidase 1 (TPP1);
   (3) the pathology of the eye is associated with Batten-CLN3 and the therapeutic product is Battenin (CLN3);
   (4) the pathology of the eye is associated with Batten-CLN6 and the therapeutic product is CLN6 Transmembrane ER Protein (CLN6);
   (5) the pathology of the eye is associated with Batten-CLN7 and the therapeutic product is Major Facilitator Superfamily Domain Containing 8 (MFSD8);
   (6) the pathology of the eye is associated with Usher's-Type 1 and the therapeutic product is Myosin VIIA (MYO7A);
   (7) the pathology of the eye is associated with Usher's-Type 1 and the therapeutic product is Cadherin Related 23 (CDH23);
   (8) the pathology of the eye is associated with Usher's-Type 2 and the therapeutic product is Protocadherin Related 15 (PCDH15);
   (9) the pathology of the eye is associated with Usher's-Type 2 and the therapeutic product is Usherin (USH2A);
   (10) the pathology of the eye is associated with Usher's-Type 3 and the therapeutic product is Clarin 1 (CLRN1);
   (11) the pathology of the eye is associated with Stargardt's and the therapeutic product is ATP Binding Cassette Subfamily A Member 4 (ABCA4);
   (12) the pathology of the eye is associated with Stargardt's and the therapeutic product is ELOVL Fatty Acid Elongase 4 (ELOVL4);
   (13) the pathology of the eye is associated with uveitis and the therapeutic product is an anti-Interleukin 6 (IL6) monoclonal antibody;
   (14) the pathology of the eye is associated with uveitis and the therapeutic product is an anti-TNF-alpha (TNF) monoclonal antibody;
   (15) the pathology of the eye is associated with diabetic macular edema (DME) and the therapeutic product is an anti-IL6 monoclonal antibody;
   (16) the pathology of the eye is associated with red-green color blindness and the therapeutic product is L opsin (OPN1LW);
   (17) the pathology of the eye is associated with red-green color blindness and the therapeutic product is M opsin (OPN1MW);
   (18) the pathology of the eye is associated with blue cone monochromacy and the therapeutic product is M opsin (OPN1MW);
   (19) the pathology of the eye is associated with Leber congenital amaurosis-1 (LCA 1) and the therapeutic product is Guanylate Cyclase 2D, Retinal (GUCY2D);
   (20) the pathology of the eye is associated with Leber congenital amaurosis-2 (LCA 2) and the therapeutic product is Retinoid Isomerohydrolase RPE65 (RPE65);
   (21) the pathology of the eye is associated with LCA 3 and the therapeutic product is Spermatogenesis Associated 7 (SPATA7);
   (22) the pathology of the eye is associated with Leber congenital amaurosis-4 (LCA 4) and the therapeutic product is Aryl Hydrocarbon Receptor Interacting Protein Like 1 (AIPL1);
   (23) the pathology of the eye is associated with Leber congenital amaurosis-5 (LCA 5) and the therapeutic product is Lebercilin (LCA5);
   (24) the pathology of the eye is associated with Leber congenital amaurosis-6 (LCA 6) and the therapeutic product is RPGR Interacting Protein 1 (RPGRIP1);
   (25) the pathology of the eye is associated with Leber congenital amaurosis-7 (LCA 7) and the therapeutic product is Cone-Rod Homeobox (CRX);
   (26) the pathology of the eye is associated with Leber congenital amaurosis-8 (LCA 8) and the therapeutic product is Crumbs Cell Polarity Complex Component 1 (CRB1);
   (27) the pathology of the eye is associated with Leber congenital amaurosis-9 (LCA 9) and the therapeutic product is Nicotinamide Nucleotide Adenylyltransferase 1 (NMNAT1);
   (28) the pathology of the eye is associated with Leber congenital amaurosis-10 (LCA 10) and the therapeutic product is Centrosomal Protein 290 (CEP290);
   (29) the pathology of the eye is associated with Leber congenital amaurosis-11 (LCA 11) and the therapeutic product is Inosine Monophosphate Dehydrogenase 1 (IMPDH1);
   (30) the pathology of the eye is associated with Leber congenital amaurosis-12 (LCA 12) and the therapeutic product is Retinal Degeneration 3, GUCY2D regulator (RD3) ;
   (31) the pathology of the eye is associated with Leber congenital amaurosis-13 (LCA 13) and the therapeutic product is Retinol Dehydrogenase 12 (RDH12);
   (32) the pathology of the eye is associated with Leber congenital amaurosis-14 (LCA 14) and the therapeutic product is Lecithin Retinol Acyltransferase (LRAT);
   (33) the pathology of the eye is associated with Leber congenital amaurosis-15 (LCA 15) and the therapeutic product is Tubby Like Protein 1 (TULP1);
   (34) the pathology of the eye is associated with Leber congenital amaurosis-16 (LCA 16) and the therapeutic product is Potassium Voltage-Gated Channel Subfamily J Member 13 (KCNJ13);
   (35) the pathology of the eye is associated with Leber's hereditary optic neuropathy (LHON) and the therapeutic product is Mitochondrially Encoded NADH Dehydrogenase 1 (MT-ND1);
   (36) the pathology of the eye is associated with LHON and the therapeutic product is Mitochondrially Encoded NADH Dehydrogenase 4 (MT-ND4);
   (37) the pathology of the eye is associated with LHON and the therapeutic product is Mitochondrially Encoded NADH Dehydrogenase 6 (MT-ND6);
   (38) the pathology of the eye is associated with neuromyelitis optica (NMO) and the therapeutic product is an anti-complement C5 monoclonal antibody;
   (39) the pathology of the eye is associated with NMO and the therapeutic product is an anti-IL6 monoclonal antibody;
   (40) the pathology of the eye is associated with uveitis and the therapeutic product is an anti-complement C5 monoclonal antibody;
   (41) the pathology of the eye is associated with uveitis and the therapeutic product is Angiotensin I Converting Enzyme (ACE);
   (42) the pathology of the eye is associated with uveitis and the therapeutic product is Interleukin 10 (IL10);
   (43) the pathology of the eye is associated with uveitis and the therapeutic product is an anti-TNF monoclonal antibody;
   (44) the pathology of the eye is associated with choroideremia and the therapeutic product is Rab Escort Protein 1 (CHM);
   (45) the pathology of the eye is associated with X-linked retinoschisis (XLRS) and the therapeutic product is Retinoschisin (RS1);
   (46) the pathology of the eye is associated with Bardet-Biedl syndrome 1 and the therapeutic product is Bardet-Biedl Syndrome 1 (BBS1);
   (47) the pathology of the eye is associated with Bardet-Biedl syndrome 2 and the therapeutic product is Bardet-Biedl Syndrome 2 (BBS2);
   (48) the pathology of the eye is associated with Bardet-Biedl syndrome 3 and the therapeutic product is ADP Ribosylation Factor Like GTPase 6 (ARL6);
   (49) the pathology of the eye is associated with Bardet-Biedl syndrome 4 and the therapeutic product is Bardet-Biedl Syndrome 4 (BBS4);
   (50) the pathology of the eye is associated with Bardet-Biedl syndrome 5 and the therapeutic product is Bardet-Biedl Syndrome 5 (BBS5);
   (51) the pathology of the eye is associated with Bardet-Biedl syndrome 6 and the therapeutic product is McKusick-Kaufman Syndrome (MKKS);
   (52) the pathology of the eye is associated with Bardet-Biedl syndrome 7 and the therapeutic product is Bardet-Biedl Syndrome 7 (BBS7);
   (53) the pathology of the eye is associated with Bardet-Biedl syndrome 8 and the therapeutic product is Tetratricopeptide Repeat Domain 8 (TTC8);
   (54) the pathology of the eye is associated with Bardet-Biedl syndrome 9 and the therapeutic product is Bardet-Biedl Syndrome 9 (BBS9);
   (55) the pathology of the eye is associated with Bardet-Biedl syndrome 10 and the therapeutic product is Bardet-Biedl Syndrome 10 (BBS10);
   (56) the pathology of the eye is associated with Bardet-Biedl syndrome 11 and the therapeutic product is Tripartite Motif Containing 32 (TRIM32);
   (57) the pathology of the eye is associated with Bardet-Biedl syndrome 12 and the therapeutic product is Bardet-Biedl Syndrome 12 (BBS 12);
   (58) the pathology of the eye is associated with Bardet-Biedl syndrome 13 and the therapeutic product is MKS Transition Zone Complex Subunit 1 (MKS 1);
   (59) the pathology of the eye is associated with Bardet-Biedl syndrome 14 and the therapeutic product is Centrosomal Protein 290 (CEP290);
   (60) the pathology of the eye is associated with Bardet-Biedl syndrome 15 and the therapeutic product is WD Repeat Containing Planar Cell Polarity Effector (WDPCP);
   (61) the pathology of the eye is associated with Bardet-Biedl syndrome 16 and the therapeutic product is Serologically Defined Colon Cancer Antigen 8 (SDCCAG8);
   (62) the pathology of the eye is associated with Bardet-Biedl syndrome 17 and the therapeutic product is Leucine Zipper Transcription Factor Like 1 (LZTFL1);
   (63) the pathology of the eye is associated with Bardet-Biedl syndrome 18 and the therapeutic product is BBSome Interacting Protein 1 (BBIP1);
   (64) the pathology of the eye is associated with Bardet-Biedl syndrome 19 and the therapeutic product is Intraflagellar Transport 27 (IFT27);
   (65) the pathology of the eye is associated with cone dystrophy and the therapeutic product is Guanylate Cyclase Activator 1A (GUCA1A);
   (66) the pathology of the eye is associated with optic atrophy and the therapeutic product is OPA1 Mitochondrial Dynamin Like GTPase (OPA1);
   (67) the pathology of the eye is associated with retinitis pigmentosa 1 and the therapeutic product is RP1 Axonemal Microtubule Associated (RP1);
   (68) the pathology of the eye is associated with retinitis pigmentosa 2 and the therapeutic product is RP2 Activator of ARL3 GTPase (RP2);
   (69) the pathology of the eye is associated with retinitis pigmentosa 7 and the therapeutic product is Peripherin 2 (PRPH2);
   (70) the pathology of the eye is associated with retinitis pigmentosa 11 and the therapeutic product is Pre-mRNA Processing Factor 31(PRPF31);
   (71) the pathology of the eye is associated with retinitis pigmentosa 12 and the therapeutic product is Crumbs Cell Polarity Complex Component 1 (CRB1);
   (72) the pathology of the eye is associated with retinitis pigmentosa 13 and the therapeutic product is Pre-mRNA Processing Factor 8 (PRPF8);
   (73) the pathology of the eye is associated with retinitis pigmentosa 25 and the therapeutic product is Eyes Shut Homolog (EYS);
   (74) the pathology of the eye is associated with retinitis pigmentosa 28 and the therapeutic product is FAM161 Centrosomal Protein A (FAM161A);
   (75) the pathology of the eye is associated with retinitis pigmentosa 37 and the therapeutic product is Nuclear Receptor Subfamily 2 Group E Member 3 (NR2E3);
   (76) the pathology of the eye is associated with retinitis pigmentosa 38 and the therapeutic product is MER Proto-Oncogene, Tyrosine Kinase (MERTK);
   (77) the pathology of the eye is associated with retinitis pigmentosa 40 and the therapeutic product is Phosphodiesterase 6B (PDE6B);
   (78) the pathology of the eye is associated with retinitis pigmentosa 41 and the therapeutic product is Prominin 1 (PROM1);
   (79) the pathology of the eye is associated with retinitis pigmentosa 43 and the therapeutic product is Phosphodiesterase 6A (PDE6A);
   (80) the pathology of the eye is associated with retinitis pigmentosa 56 and the therapeutic product is Interphotoreceptor Matrix Proteoglycan 2 (IMPG2);
   (81) the pathology of the eye is associated with petinitis pigmentosa 62 and the therapeutic product is Male Germ Cell Associated Kinase (MAK);
   (82) the pathology of the eye is associated with retinitis pigmentosa 80 and the therapeutic product is Intraflagellar Transport 140 (IFT140);
   (83) the pathology of the eye is associated with dry AMD and the therapeutic product is an anti-complement C5 monoclonal antibody;
   (84) the pathology of the eye is associated with dry AMD and the therapeutic product is an anti-membrane attack complex (MAC) monoclonal antibody;
   (85) the pathology of the eye is associated with dry AMD and the therapeutic product is HtrA Serine Peptidase 1 (HTRA1);
   (86) the pathology of the eye is associated with Best disease and the therapeutic product is Bestrophin 1 (BEST1);
   (87) the pathology of the eye is associated with dry AMD and the therapeutic product is a complement factor B antisense oligonucleotide;
   (88) the pathology of the eye is associated with dry AMD and the therapeutic product is an anti-beta-amyloid monoclonal antibody;
   (89) the pathology of the eye is associated with dry AMD and the therapeutic product is CD59 glycoprotein (CD59);
   (90) the pathology of the eye is associated with dry AMD and the therapeutic product is Channelrhodopsin-1 (ChR1);
   (91) the pathology of the eye is associated with dry AMD and the therapeutic product is Channelrhodopsin-2 (ChR2), the light-sensitive protein discovered in *Chlamydomonas reinhardtii;*
   (92) the pathology of the eye is associated with dry AMD and the therapeutic product is an anti-complement factor C5a aptamer;
   (93) the pathology of the eye is associated with dry AMD and the therapeutic product is anti-complement factor D monoclonal antibody;
   (94) the pathology of the eye is associated with age-related retinal ganglion cell (RGC) degeneration and the therapeutic product is DnaJ heat shock protein family (Hsp40) member C3 (DNAJC3);
   (95) the pathology of the eye is associated with blue cone monochromacy (BCM) and the therapeutic product is L opsin (OPN1LW);
   (96) the pathology of the eye is associated with glaucoma and the therapeutic product is beta-2 adrenoceptor siRNA;
   (97) the pathology of the eye is associated with glaucoma and the therapeutic product is Caspase-2 (CASP2);
   (98) the pathology of the eye is associated with glaucoma and the therapeutic product is Insulin Receptor Substrate 1 (IRS1);
   (99) the pathology of the eye is associated with glaucoma and the therapeutic product is HIF-1 Responsive Protein RTP801 (RTP801);
   (100) the pathology of the eye is associated with glaucoma and the therapeutic product is Transforming Growth Factor Beta 2 (TGFB2);
   (101) the pathology of the eye is associated with glaucoma and the therapeutic product is Brain Derived Neurotrophic Factor (BDNF);
   (102) the pathology of the eye is associated with glaucoma and the therapeutic product is Ciliary Neurotrophic Factor (CNTF);
   (103) the pathology of the eye is associated with glaucoma and the therapeutic product is Prostaglandin-Endoperoxide Synthase 2 (PTGS2);
   (104) the pathology of the eye is associated with glaucoma and the therapeutic product is Prostaglandin F Receptor (PTGFR);
   (105) the pathology of the eye is associated with glaucoma and the therapeutic product is a hyaluronidase;
   (106) the pathology of the eye is associated with glaucoma and the therapeutic product is Pigment Epithelium-Derived Factor (PEDF);
   (107) the pathology of the eye is associated with glaucoma and the therapeutic product is Vascular Endothelial Growth Factor (VEGF);
   (108) the pathology of the eye is associated with glaucoma and the therapeutic product is Placental Growth Factor (PGF);
   (109) the pathology of the eye is associated with glaucoma and the therapeutic product is Myocilin (MYOC);
   (110) the pathology of the eye is associated with NMO and the therapeutic product is an anti-complement C5 monoclonal antibody;
   (111) the pathology of the eye is associated with NMO and the therapeutic product is C-C Motif Chemokine Receptor 5 (CCR5) siRNA;
   (112) the pathology of the eye is associated with NMO and the therapeutic product is an anti-CD19 monoclonal antibody;
   (113) the pathology of the eye is associated with retinitis pigmentosa that is associated with rhodopsin mutations and the therapeutic product is Channelrhodopsin-1 (ChR1);
   (114) the pathology of the eye is associated with retinitis pigmentosa that is associated with rhodopsin mutations and the therapeutic product is Channelrhodopsin-2 (ChR2);
   (115) the pathology of the eye is associated with retinitis pigmentosa and the therapeutic product is Ciliary Neurotrophic Factor (CNTF);
   (116) the pathology of the eye is associated with autosomal recessive retinitis pigmentosa and the therapeutic product is Crumbs Cell Polarity Complex Component 1 (CRB1);
   (117) the pathology of the eye is associated with autosomal recessive retinitis pigmentosa and the therapeutic product is Crumbs Cell Polarity Complex Component 2 (CRB2);
   (118) the pathology of the eye is associated with retinitis pigmentosa and the therapeutic product is Histone Deacetylase 4 (HDAC4);
   (119) the pathology of the eye is associated with retinitis pigmentosa and the therapeutic product is Rhodopsin (RHO);
   (120) the pathology of the eye is associated with retinitis pigmentosa and the therapeutic product is Nerve Growth Factor (NGF);
   (121) the pathology of the eye is associated with retinitis pigmentosa and the therapeutic product is Nuclear Factor, Erythroid 2 Like 2 (NRF2);
   (122) the pathology of the eye is associated with retinitis pigmentosa and the therapeutic product is Pigment Epithelium-Derived Factor (PEDF);
   (123) the pathology of the eye is associated with retinitis pigmentosa and the therapeutic product is Glutathione S-Transferase PI 1 (GSTP1);
   (124) the pathology of the eye is associated with retinitis pigmentosa and the therapeutic product is Rod-Derived Cone Viability Factor (RDCVF);
   (125) the pathology of the eye is associated with retinitis pigmentosa and the therapeutic product is Rhodopsin (RHO);
   (126) the pathology of the eye is associated with retinitis pigmentosa and the therapeutic product is Retinaldehyde Binding Protein 1 (RLBP1);
   (127) the pathology of the eye is associated with Stargardt's disease and the therapeutic product is an anti-complement C5 aptamer;
   (128) the pathology of the eye is associated with uveitis and the therapeutic product is Double Homeobox 4 (DUX4);
   (129) the pathology of the eye is associated with uveitis and the therapeutic product is NLR Family Pyrin Domain Containing 3 (NLRP3);
   (130) the pathology of the eye is associated with uveitis and the therapeutic product is Spleen Associated Tyrosine Kinase (SYK);
   (131) the pathology of the eye is associated with uveitis and the therapeutic product is Adrenocorticotropic Hormone (ACTH);
   (132) the pathology of the eye is associated with uveitis and the therapeutic product is Caspase 1 (CASP1);
   (133) the pathology of the eye is associated with uveitis and the therapeutic product is anti-CD59 monoclonal antibody;
   (134) the pathology of the eye is associated with uveitis and the therapeutic product is an anti-complement C5 aptamer;
   (135) the pathology of the eye is associated with corneal neovascularization and the therapeutic product is Insulin Receptor Substrate 1 (IRS1);
   (136) the pathology of the eye is associated with corneal neovascularization and the therapeutic product is NOTCH Regulated Ankyrin Repeat Protein (NRARP);
   (137) the pathology of the eye is associated with diabetic retinopathy and the therapeutic product is NOTCH Regulated Ankyrin Repeat Protein (NRARP);
   (138) the pathology of the eye is associated with diabetic retinopathy and the therapeutic product is Alpha-2-Antiplasmin (A2AP);
   (139) the pathology of the eye is associated with diabetic retinopathy and the therapeutic product is Plasminogen (PLG);
   (140) the pathology of the eye is associated with diabetic retinopathy and the therapeutic product is a growth hormone;
   (141) the pathology of the eye is associated with diabetic retinopathy and the therapeutic product is Insulin Like Growth Factor 1 (IGF1);
   (142) the pathology of the eye is associated with diabetic retinopathy and the therapeutic product is Interleukin 1 Beta (IL1B).
   (143) the pathology of the eye is associated with diabetic retinopathy and the therapeutic product is Angiotensin I Converting Enzyme 2 (ACE2);
   (144) the pathology of the eye is associated with diabetic retinopathy and the therapeutic product is IRS1;
   (145) the pathology of the eye is associated with diabetic retinopathy and the therapeutic product is an anti-integrin oligopeptide;
   (146) the pathology of the eye is associated with diabetic retinopathy and the therapeutic product is an anti-Placental Growth Factor (PGF) monoclonal antibody;
   (147) the pathology of the eye is associated with Graves' ophthalmopathy and the therapeutic product is an anti-CD40 monoclonal antibody;
   (148) the pathology of the eye is associated with Graves' ophthalmopathy and the therapeutic product is an anti-Insulin-Like Growth Factor 1 Receptor (IGF1R) monoclonal antibody;
   (149) the pathology of the eye is associated with Graves' ophthalmopathy and the therapeutic product is an anti-Insulin-Like Growth Factor 2 Receptor (IGF2R) monoclonal antibody;
   (150) the pathology of the eye is associated with DME and the therapeutic product is an anti-integrin oligopeptide;
   (151) the pathology of the eye is associated with DME and the therapeutic product is an anti-Placental Growth Factor (PGF) monoclonal antibody;
   (152) the pathology of the eye is associated with DME and the therapeutic product is RTP801 siRNA;
   (153) the pathology of the eye is associated with multiple sclerosis (MS)-associated vision loss and the therapeutic product is ND1;
   (154) the pathology of the eye is associated with myopia and the therapeutic product is Matrix Metalloproteinase 2 (MMP2) RNAi;
   (155) the pathology of the eye is associated with X-linked recessive ocular albinism and the therapeutic product is G-Protein Coupled Receptor 143 (GPR143);
   (156) the pathology of the eye is associated with oculocutaneous albinism type 1 and the therapeutic product is Tyrosinase (TYR);
   (157) the pathology of the eye is associated with optic neuritis and the therapeutic product is Caspase 2 (CASP2);
   (158) the pathology of the eye is associated with optic neuritis and the therapeutic product is an anti-Leucine Rich Repeat And Ig Domain Containing Protein 1 (LINGO1) monoclonal antibody;or
   (159) the pathology of the eye is associated with polypoidal choroidal vasculopathy and the therapeutic product is an anti-complement C5 aptamer.
27. The method of any one of items 1-11 and 15-18, wherein: (1) the pathology of the eye is associated with X-linked retinitis pigmentosa (XLRP) and the therapeutic product is Retinitis Pigmentosa GTPase Regulator (RPGR);
   (2) the pathology of the eye is associated with achromatopsia (ACHM) and the therapeutic product is Cyclic Nucleotide Gated Channel Beta 3 (CNGB3);
   (3) the pathology of the eye is associated with achromatopsia and the therapeutic product is Cyclic Nucleotide Gated Channel Alpha 3 (CNGA3); or
   (4) the pathology of the eye is associated with biallelic RPE65 mutation-associated retinal dystrophy and the therapeutic product is Retinoid Isomerohydrolase RPE65 (RPE65).
28. The method of any one of items 1-11 and 13-18, wherein:
   (1) the pathology of the eye is associated with Batten-CLN1 and the therapeutic product is Palmitoyl-Protein Thioesterase 1 (PPT1);
   (2) the pathology of the eye is associated with Batten-CLN2 and the therapeutic product is Tripeptidyl-Peptidase 1 (TPP1);
   (3) the pathology of the eye is associated with Batten-CLN3 and the therapeutic product is Battenin (CLN3);
   (4) the pathology of the eye is associated with uveitis and the therapeutic product is an anti-Interleukin 6 (IL6) monoclonal antibody;
   (5) the pathology of the eye is associated with uveitis and the therapeutic product is an anti-TNF-alpha (TNF) monoclonal antibody;
   (6) the pathology of the eye is associated with diabetic macular edema (DME) and the therapeutic product is an anti-IL6 monoclonal antibody;
   (7) the pathology of the eye is associated with red-green color blindness and the therapeutic product is L opsin (OPN1LW);
   (8) the pathology of the eye is associated with red-green color blindness and the therapeutic product is M opsin (OPN1MW);
   (9) the pathology of the eye is associated with blue cone monochromacy and the therapeutic product is M opsin (OPN1MW);
   (10) the pathology of the eye is associated with Leber congenital amaurosis-1 (LCA 1) and the therapeutic product is Guanylate Cyclase 2D, Retinal (GUCY2D);
   (11) the pathology of the eye is associated with Leber congenital amaurosis-2 (LCA 2) and the therapeutic product is Retinoid Isomerohydrolase RPE65 (RPE65);
   (12) the pathology of the eye is associated with Leber congenital amaurosis-7 (LCA 7) and the therapeutic product is Cone-Rod Homeobox (CRX);
   (13) the pathology of the eye is associated with Leber congenital amaurosis-11 (LCA 11) and the therapeutic product is Inosine Monophosphate Dehydrogenase 1 (IMPDH1);
   (14) the pathology of the eye is associated with Leber congenital amaurosis-12 (LCA 12) and the therapeutic product is Retinal Degeneration 3, GUCY2D regulator (RD3);
   (15) the pathology of the eye is associated with Leber congenital amaurosis-13 (LCA 13) and the therapeutic product is Retinol Dehydrogenase 12 (RDH12);
   (16) the pathology of the eye is associated with Leber congenital amaurosis-15 (LCA 15) and the therapeutic product is Tubby Like Protein 1 (TULP1);
   (17) the pathology of the eye is associated with Leber congenital amaurosis-16 (LCA 16) and the therapeutic product is Potassium Voltage-Gated Channel Subfamily J Member 13 (KCNJ13);
   (18) the pathology of the eye is associated with Leber's hereditary optic neuropathy (LHON) and the therapeutic product is Mitochondrially Encoded NADH Dehydrogenase 1 (MT-ND1);
   (19) the pathology of the eye is associated with LHON and the therapeutic product is Mitochondrially Encoded NADH Dehydrogenase 4 (MT-ND4);
   (20) the pathology of the eye is associated with LHON and the therapeutic product is Mitochondrially Encoded NADH Dehydrogenase 6 (MT-ND6);
   (21) the pathology of the eye is associated with neuromyelitis optica (NMO) and the therapeutic product is an anti-complement C5 monoclonal antibody;
   (22) the pathology of the eye is associated with NMO and the therapeutic product is an anti-IL6 monoclonal antibody;
   (23) the pathology of the eye is associated with uveitis and the therapeutic product is an anti-complement C5 monoclonal antibody;
   (24) the pathology of the eye is associated with uveitis and the therapeutic product is Angiotensin I Converting Enzyme (ACE);
   (25) the pathology of the eye is associated with uveitis and the therapeutic product is Interleukin 10 (IL10);
   (26) the pathology of the eye is associated with uveitis and the therapeutic product is an anti-TNF monoclonal antibody;
   (27) the pathology of the eye is associated with X-linked retinoschisis (XLRS) and the therapeutic product is Retinoschisin (RS1);
   (28) the pathology of the eye is associated with Bardet-Biedl syndrome 1 and the therapeutic product is Bardet-Biedl Syndrome 1 (BBS 1);
   (29) the pathology of the eye is associated with Bardet-Biedl syndrome 3 and the therapeutic product is ADP Ribosylation Factor Like GTPase 6 (ARL6);
   (30) the pathology of the eye is associated with Bardet-Biedl syndrome 5 and the therapeutic product is Bardet-Biedl Syndrome 5 (BBS5);
   (31) the pathology of the eye is associated with Bardet-Biedl syndrome 6 and the therapeutic product is McKusick-Kaufman Syndrome (MKKS);
   (32) the pathology of the eye is associated with Bardet-Biedl syndrome 10 and the therapeutic product is Bardet-Biedl Syndrome 10 (BBS10);
   (33) the pathology of the eye is associated with Bardet-Biedl syndrome 11 and the therapeutic product is Tripartite Motif Containing 32 (TRIM32);
   (34) the pathology of the eye is associated with Bardet-Biedl syndrome 13 and the therapeutic product is MKS Transition Zone Complex Subunit 1 (MKS 1);
   (35) the pathology of the eye is associated with Bardet-Biedl syndrome 18 and the therapeutic product is BBSome Interacting Protein 1 (BBIP1);
   (36) the pathology of the eye is associated with Bardet-Biedl syndrome 19 and the therapeutic product is Intraflagellar Transport 27 (IFT27);
   (37) the pathology of the eye is associated with cone dystrophy and the therapeutic product is Guanylate Cyclase Activator 1A (GUCA1A);
   (38) the pathology of the eye is associated with retinitis pigmentosa 13 and the therapeutic product is Pre-mRNA Processing Factor 8 (PRPF8);
   (39) the pathology of the eye is associated with retinitis pigmentosa 37 and the therapeutic product is Nuclear Receptor Subfamily 2 Group E Member 3 (NR2E3); or
   (40) the pathology of the eye is associated with Best disease and the therapeutic product is Bestrophin 1 (BEST1).
29. The method of any one of items 1-11 and 15-18, wherein:
   (1) the pathology of the eye is associated with biallelic RPE65 mutation-associated retinal dystrophy and the therapeutic product is Retinoid Isomerohydrolase RPE65 (RPE65).
30. The method of any one of items 1-11 and 13-18, wherein:
   (1) the pathology of the eye is associated with Batten-CLN2 and the therapeutic product is Tripeptidyl-Peptidase 1 (TPP1);
   (2) the pathology of the eye is associated with Usher's-Type 1 and the therapeutic product is Myosin VIIA (MYO7A);
   (3) the pathology of the eye is associated with Usher's-Type 1 and the therapeutic product is Cadherin Related 23 (CDH23);
   (4) the pathology of the eye is associated with Usher's-Type 2 and the therapeutic product is Protocadherin Related 15 (PCDH15);
   (5) the pathology of the eye is associated with Usher's-Type 2 and the therapeutic product is Usherin (USH2A);
   (6) the pathology of the eye is associated with Usher's-Type 3 and the therapeutic product is Clarin 1 (CLRN1);
   (7) the pathology of the eye is associated with Stargardt's and the therapeutic product is ATP Binding Cassette Subfamily A Member 4 (ABCA4);
   (8) the pathology of the eye is associated with Stargardt's and the therapeutic product is ELOVL Fatty Acid Elongase 4 (ELOVL4);
   (9) the pathology of the eye is associated with red-green color blindness and the therapeutic product is L opsin (OPN1LW);
   (10) the pathology of the eye is associated with red-green color blindness and the therapeutic product is M opsin (OPN1MW);
   (11) the pathology of the eye is associated with blue cone monochromacy and the therapeutic product is M opsin (OPN1MW);
   (12) the pathology of the eye is associated with Leber congenital amaurosis-1 (LCA 1) and the therapeutic product is Guanylate Cyclase 2D, Retinal (GUCY2D);
   (13) the pathology of the eye is associated with Leber congenital amaurosis-2 (LCA 2) and the therapeutic product is Retinoid Isomerohydrolase RPE65 (RPE65);
   (14) the pathology of the eye is associated with Leber congenital amaurosis-4 (LCA 4) and the therapeutic product is Aryl Hydrocarbon Receptor Interacting Protein Like 1 (AIPL1);
   (15) the pathology of the eye is associated with Leber congenital amaurosis-7 (LCA 7) and the therapeutic product is Cone-Rod Homeobox (CRX);
   (16) the pathology of the eye is associated with Leber congenital amaurosis-8 (LCA 8) and the therapeutic product is Crumbs Cell Polarity Complex Component 1 (CRB1);
   (17) the pathology of the eye is associated with Leber congenital amaurosis-9 (LCA 9) and the therapeutic product is Nicotinamide Nucleotide Adenylyltransferase 1 (NMNAT1);
   (18) the pathology of the eye is associated with Leber congenital amaurosis-10 (LCA 10) and the therapeutic product is Centrosomal Protein 290 (CEP290);
   (19) the pathology of the eye is associated with Leber congenital amaurosis-11 (LCA 11) and the therapeutic product is Inosine Monophosphate Dehydrogenase 1 (IMPDH1);
   (20) the pathology of the eye is associated with Leber congenital amaurosis-15 (LCA 15) and the therapeutic product is Tubby Like Protein 1 (TULP1);
   (21) the pathology of the eye is associated with LHON and the therapeutic product is Mitochondrially Encoded NADH Dehydrogenase 4 (MT-ND4);
   (22) the pathology of the eye is associated with LHON and the therapeutic product is Mitochondrially Encoded NADH Dehydrogenase 6 (MT-ND6);
   (23) the pathology of the eye is associated with choroideremia and the therapeutic product is Rab Escort Protein 1 (CHM);
   (24) the pathology of the eye is associated with X-linked retinoschisis (XLRS) and the therapeutic product is Retinoschisin (RS1);
   (25) the pathology of the eye is associated with Bardet-Biedl syndrome 1 and the therapeutic product is Bardet-Biedl Syndrome 1 (BBS1);
   (26) the pathology of the eye is associated with Bardet-Biedl syndrome 6 and the therapeutic product is McKusick-Kaufman Syndrome (MKKS);
   (27) the pathology of the eye is associated with Bardet-Biedl syndrome 10 and the therapeutic product is Bardet-Biedl Syndrome 10 (BBS10);
   (28) the pathology of the eye is associated with cone dystrophy and the therapeutic product is Guanylate Cyclase Activator 1A (GUCA1A);
   (29) the pathology of the eye is associated with optic atrophy and the therapeutic product is OPA1 Mitochondrial Dynamin Like GTPase (OPA1);
   (30) the pathology of the eye is associated with retinitis pigmentosa 1 and the therapeutic product is RP1 Axonemal Microtubule Associated (RP1);
   (31) the pathology of the eye is associated with retinitis pigmentosa 2 and the therapeutic product is RP2 Activator of ARL3 GTPase (RP2);
   (32) the pathology of the eye is associated with retinitis pigmentosa 7 and the therapeutic product is Peripherin 2 (PRPH2);
   (33) the pathology of the eye is associated with retinitis pigmentosa 11 and the therapeutic product is Pre-mRNA Processing Factor 31(PRPF31);
   (34) the pathology of the eye is associated with retinitis pigmentosa 13 and the therapeutic product is Pre-mRNA Processing Factor 8 (PRPF8);
   (35) the pathology of the eye is associated with retinitis pigmentosa 37 and the therapeutic product is Nuclear Receptor Subfamily 2 Group E Member 3 (NR2E3);
   (36) the pathology of the eye is associated with retinitis pigmentosa 38 and the therapeutic product is MER Proto-Oncogene, Tyrosine Kinase (MERTK);
   (37) the pathology of the eye is associated with retinitis pigmentosa 40 and the therapeutic product is Phosphodiesterase 6B (PDE6B);
   (38) the pathology of the eye is associated with retinitis pigmentosa 41 and the therapeutic product is Prominin 1 (PROM1);
   (39) the pathology of the eye is associated with retinitis pigmentosa 56 and the therapeutic product is Interphotoreceptor Matrix Proteoglycan 2 (IMPG2);
   (40) the pathology of the eye is associated with petinitis pigmentosa 62 and the therapeutic product is Male Germ Cell Associated Kinase (MAK);
   (41) the pathology of the eye is associated with retinitis pigmentosa 80 and the therapeutic product is Intraflagellar Transport 140 (IFT140); or
   (42) the pathology of the eye is associated with Best disease and the therapeutic product is Bestrophin 1 (BEST1).
31. The method of any one of items 1-11 and 15-18, wherein:
   (1) the pathology of the eye is associated with X-linked retinitis pigmentosa (XLRP) and the therapeutic product is Retinitis Pigmentosa GTPase Regulator (RPGR);
   (2) the pathology of the eye is associated with achromatopsia and the therapeutic product is Cyclic Nucleotide Gated Channel Beta 3 (CNGB3); or
   (3) the pathology of the eye is associated with achromatopsia and the therapeutic product is Cyclic Nucleotide Gated Channel Alpha 3 (CNGA3).
32. The method of any one of items 1-31, wherein the recombinant viral vector further comprises a nucleotide sequence encoding a promoter or an enhancer-promoter, which nucleotide sequence encoding the promoter or enhancer-promoter is operably linked to the nucleotide sequence encoding the therapeutic product, and wherein the promoter or enhancer-promoter is:
   (1) a CAG promoter;
   (2) a CBA promoter;
   (3) a CMV promoter;
   (4) a PR1.7 promoter;
   (5) a Rhodopsin Kinase (GRK1) photoreceptor-specific enhancer-promoter;
   (6) an hCARp promoter;
   (7) an hRKp;
   (8) a cone photoreceptor specific human arrestin 3 (ARR3) promoter;
   (9) a rhodopsin promoter;or
   (10) a U6 promoter.
33. The method of any one of items 1-11 and 13-15, wherein the recombinant viral vector further comprises a nucleotide sequence encoding a cone-specific promoter, which nucleotide sequence encoding the cone-specific promoter is operably linked to the nucleotide sequence encoding the therapeutic product, and wherein:
   (1) the pathology of the eye is associated with red-green color blindness and the therapeutic product is L opsin (OPN1LW);
   (2) the pathology of the eye is associated with red-green color blindness and the therapeutic product is M opsin (OPN1MW);
   (3) the pathology of the eye is associated with blue cone monochromacy and the therapeutic product is M opsin (OPN1MW);
   (4) the pathology of the eye is associated with cone dystrophy and the therapeutic product is Guanylate Cyclase Activator 1A (GUCA1A); or
   (5) the pathology of the eye is associated with blue cone monochromacy (BCM) and the therapeutic product is L opsin (OPN1LW).
34. The method of any one of items 1-33, wherein the administering step delivers a therapeutically effective amount of the therapeutic product to the retina of said human subject.
35. The method of item 34, wherein the therapeutically effective amount of the therapeutic product is produced by human retinal cells of said human subject.
36. The method of item 34, wherein the therapeutically effective amount of the therapeutic product is produced by human photoreceptor cells, horizontal cells, bipolar cells, amacrine cells, retina ganglion cells, and/or retinal pigment epithelial cells in the external limiting membrane of said human subject.
37. The method of item 36, wherein the human photoreceptor cells are cone cells and/ or rod cells.
38. The method of item 36, wherein the retina ganglion cells are midget cells, parasol cells, bistratified cells, giant retina ganglion cells, photosensitive ganglion cells, and/or Müller glia.
39. The method of any one of items 1-38, wherein the recombinant viral vector is an rAAV vector.
40. The method of item 39, wherein the recombinant viral vector is an rAAV8 vector.
41. The method of any one of items 1-40, which further comprises, after the administering step, a step of monitoring the post ocular injection thermal profile of the injected material in the eye using an infrared thermal camera.
42. The method of item 41, wherein the infrared thermal camera is an FLIR T530 infrared thermal camera.
43. The method of any one of items 1-43, wherein the recombinant nucleotide expression vector is administered at a dose about 6.0× 10¹⁰ genome copies per eye.
44. The method of any one of items 1-43, wherein the recombinant nucleotide expression vector is administered at a dose about 1.6 × 10¹¹ genome copies per eye.
45. The method of any one of items 1-43, wherein the recombinant nucleotide expression vector is administered at a dose about 2.5 × 10¹¹ genome copies per eye.
46. The method of any one of items 1-43, wherein the recombinant nucleotide expression vector is administered at a dose about 5.0 × 10¹¹ genome copies per eye.
47. The method of any one of items 1-43, wherein the recombinant nucleotide expression vector is administered at a dose about 3.0 x 10¹² genome copies per eye.

## Claims

1. A recombinant adeno-associated virus (rAAV) vector for use in a method for treating Batten-CLN2-associated vision loss, wherein the method comprises:
(a) administering the rAAV vector to a subretinal space in an eye of a human subject in need thereof;
(b) administering the rAAV vector to a subretinal space via a suprachoroidal space in an eye of a human subject in need thereof; or
(c) administering the rAAV vector to a suprachoroidal space in an eye of a human subject in need thereof;
wherein the rAAV vector comprises an expression cassette encoding Tripeptidyl-Peptidase 1 at a dose sufficient to produce a therapeutically effective concentration of a transgene product in a vitreous humour in the eye.

2. The rAAV vector for use of claim 1, wherein the administering comprises an injection, a peripheral injection, an intravitreal injection, or a transvitreal injection.

3. The rAAV vector for use of claim 2, wherein the transvitreal injection comprises: (i) inserting a sharp needle into a sclera via the superior or inferior side of the eye and passing the sharp needle all the way through the vitreous to inject the rAAV vector to the subretinal space on the other side; or (ii) inserting a trochar into the sclera and inserting a cannula through the trochar and through the vitreous to inject the rAAV vector to the subretinal space on the other side.

4. The rAAV vector for use of claim 2, wherein the intravitreal injection comprises using an intravitreal drug delivery device.

5. The rAAV vector for use of claim 1, wherein the administering comprises injecting the rAAV vector into the suprachoroidal space using a suprachoroidal drug delivery device.

6. The rAAV vector for use of claim 1, wherein the method further comprises a vitrectomy.

7. The rAAV vector for use of any one of claims 1 to 6, wherein the rAAV vector is administered at a dose of about 1.0 x 10^9 genome copies per eye to about 1.0 x 10^15 genome copies per eye.

8. The rAAV vector for use of any one of claims 1 to 6, wherein the rAAV vector is administered at a dose of about 1.0 x 10^10 genome copies per eye to about 6.0 x 10^10 genome copies per eye.

9. The rAAV vector for use of any one of claims 1 to 6, wherein the rAAV vector is administered at a dose of about 1.0 x 10^9 genome copies per mL to about 1.0 x 10^15 genome copies per mL.

10. The rAAV vector for use of any one of claims 1 to 6, wherein the rAAV vector is administered at a dose of about 1.0 x 10^11 genome copies per mL to about 1.0 x 10^12 genome copies per mL.

11. The rAAV vector for use of any one of claims 1 to 10, wherein the therapeutically effective concentration is produced by retinal cells of the human subject in need thereof.

12. The rAAV vector for use of any one of claims 1 to 11, wherein the therapeutically effective concentration is produced by photoreceptor cells, horizontal cells, bipolar cells, amacrine cells, retina ganglion cells and/or retinal pigment epithelial cells in the external limiting membrane of the human subject in need thereof.

13. The rAAV vector for use of any one of claims 1 to 12, wherein the method further comprises evaluating the human subject for improvement in Batten-CLN2-associated vision loss.

14. The rAAV vector for use of any one of claims 1 to 13, wherein the method further comprises administering the rAAV via a central nervous system (CNS) delivery route.

15. The rAAV vector for use of any one of claims 1 to 14, wherein the rAAV is rAAV9.
